(19) Europäisches Patentamt
European Patent Office
Office européen des brevets

(11) EP 1 352 906 A1

(12) # EUROPEAN PATENT APPLICATION
published in accordance with Art. 158(3) EPC

(43) Date of publication:
**15.10.2003 Bulletin 2003/42**

(21) Application number: **02716313.8**

(22) Date of filing: **17.01.2002**

(51) Int Cl.[7]: **C07D 401/04**, C07D 401/14, C07D 451/02, C07D 453/02, C07D 471/04, A61K 31/437, A61K 31/4375, A61K 31/439, A61K 31/4439, A61K 31/444, A61K 31/4545, A61K 31/496, A61K 31/506, A61K 31/5377, A61K 31/541, A61P 1/16

(86) International application number:
**PCT/JP02/00290**

(87) International publication number:
**WO 02/057254 (25.07.2002 Gazette 2002/30)**

(84) Designated Contracting States:
**AT BE CH CY DE DK ES FI FR GB GR IE IT LI LU MC NL PT SE TR**

(30) Priority: **18.01.2001 JP 2001009629**

(71) Applicant: **Sankyo Company, Limited**
**Tokyo 103-8426 (JP)**

(72) Inventors:
- **SHIMOZATO, Takaichi,**
  **c/o SANKYO COMPANY, LIMITED**
  **Tokyo 140-8710 (JP)**
- **SHIMADA, Yoko,**
  **c/o SANKYO COMPANY, LIMITED**
  **Tokyo 140-8710 (JP)**
- **KIMURA, Tomio,**
  **c/o SANKYO COMPANY, LIMITED**
  **Tokyo 140-8710 (JP)**

(74) Representative:
**Gibson, Christian John Robert et al**
**MARKS & CLERK,**
**57/60 Lincoln's Inn Fields**
**London WC2A 3LS (GB)**

(54) **COMPOSITIONS FOR PREVENTION OR TREATMENT OF HEPATOPATHY**

(57) [Object]

The object of the present invention is to provide a composition for the prophylaxis or treatment of hepatopathy.

[Solution]

A composition containing as an active ingredient a compound of formula (I), or a pharmacologically acceptable salt, ester or other derivative thereof for the prophylaxis or treatment of hepatopathy:

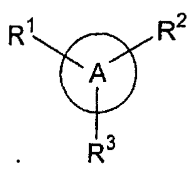

(I)

[wherein: A is a pyrrole ring; $R^1$ is an optionally substituted aryl or heteroaryl group; $R^2$ is an optionally substituted heteroaryl group; $R^3$ represents a group of the formula $-X-R^4$, wherein X is a single bond or an optionally substituted alkylene, alkenylene or alkynylene group, and $R^4$ is a substituted cycloalkyl group, a substituted aryl group, an optionally substituted heterocyclyl group, an optionally substituted heteroaryl group, or $-NR^aR^b$, wherein each of $R^a$ and $R^b$ is a hydrogen atom or an alkyl, alkenyl, alkynyl, aralkyl or alkylsulfonyl group; PROVIDED THAT said substituents $R^1$ and $R^3$ are bonded to the two atoms of said pyrrole ring which are adjacent to the atom of the pyrrole ring to which said substituent $R^2$ is bonded].

EP 1 352 906 A1

## Description

[Technical field]

The present invention relates to compositions containing as an active ingredient heteroaryl-substituted pyrrole derivatives for the prophylaxis or treatment of hepatopathy.

[Background art]

It is disclosed in WO 00/66124 that compounds having two or three aromatic groups on a pyrrole ring exhibit excellent activity for the prophylaxis or treatment of hepatopathy.
There is, however, a need for further compounds having improved activity, pharmacokinetics and safety.
As a result of earnest research over the course of many years on the synthesis of pyrrole derivatives and their pharmacological effects, the inventors of the present invention found that compounds having a 1H-pyrrole ring in which three consecutive atoms on the 1H-pyrrole ring are substituted have excellent activity for the prophylaxis or treatment of hepatopathy, thereby leading to completion of the present invention.

[Disclosure of the invention]

The present invention relates to

(1) compositions containing as an active ingredient a compound of the following formula (I), or a pharmacologically acceptable salt, ester or other derivative thereof, for the prophylaxis or treatment of hepatopathy:

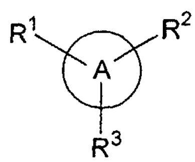

(I)

wherein:

A represents a pyrrole ring;

$R^1$ represents an aryl group; an aryl group which is substituted with group(s) selected from Substituent group $\alpha$, Substituent group $\beta$, Substituent group $\gamma$ and Substituent group $\delta$; a heteroaryl group; or a heteroaryl group which is substituted with group(s) selected from Substituent group $\alpha$, Substituent group $\beta$, Substituent group $\gamma$ and Substituent group $\delta$;

$R^2$ represents a heteroaryl group having at least one ring nitrogen atom; or a heteroaryl group having at least one ring nitrogen atom, wherein said heteroaryl group is substituted with group(s) selected from Substituent group $\alpha$, Substituent group $\beta$, Substituent group $\gamma$ and Substituent group $\delta$;

$R^3$ represents a group of the formula $-X-R^4$,

wherein:

X represents a single bond; a lower alkylene group; a lower alkylene group which is substituted with group(s) selected from Substituent group $\alpha$; a lower alkenylene group; a lower alkenylene group which is substituted with group(s) selected from Substituent group $\alpha$; a lower alkynylene group; or a lower alkynylene group which is substituted with group(s) selected from Substituent group $\alpha$; and
$R^4$ represents a cycloalkyl group which may be substituted with group(s) selected from Substituent group $\alpha$ and Substituent group $\delta$ and which is substituted with group(s) selected from Substituent group $\beta$ and Sub-

stituent group γ; an aryl group which may be substituted with group(s) selected from Substituent group α and Substituent group δ and which is substituted with group(s) selected from Substituent group β and Substituent group γ; a heterocyclyl group which may be substituted with group(s) selected from Substituent group α and Substituent group δ and which is substituted with group(s) selected from Substituent group β and Substituent group γ; a heterocyclyl group having at least one nitrogen atom, said heterocyclyl group being optionally substituted with group(s) selected from Substituent group α, Substituent group δ and Substituent group ε; a heteroaryl group which may be substituted with group(s) selected from Substituent group α and Substituent group δ and which is substituted with group(s) selected from Substituent group β and Substituent group γ; a heteroaryl group having at least one nitrogen atom, said heteroaryl group being optionally substituted with group(s) selected from Substituent group α and Substituent group δ; or a group of formula $-NR^aR^b$ (wherein $R^a$ and $R^b$ are the same or different from each other and each represents a hydrogen atom, a lower alkyl group, a lower alkenyl group, a lower alkynyl group, an aralkyl group or a lower alkylsulfonyl group); PROVIDED THAT said substituents $R^1$ and $R^3$ are bonded to the atoms of said pyrrole ring which are adjacent to the atom of the pyrrole ring to which said substituent $R^2$ is bonded;

[Substituent group α]
a hydroxyl group, a nitro group, a cyano group, a halogen atom, a lower alkoxy group, a halogeno lower alkoxy group, an alkylthio group and a halogeno lower alkylthio group;
[Substituent group β]
a group of formula $-NR^cR^d$ (wherein $R^c$ and $R^d$ are the same or different from each other and each represents a hydrogen atom, a lower alkyl group, a lower alkenyl group, a lower alkynyl group, an aralkyl group or a lower alkylsulfonyl group, or $R^c$ and $R^d$, together with the nitrogen atom to which $R^c$ and $R^d$ are bonded, form a heterocyclyl group);
[Substituent group γ]
a lower alkyl group which is substituted with a group of formula $-NR^cR^d$ (wherein $R^c$ and $R^d$ are as defined above);
[Substituent group δ]
a lower alkyl group, a lower alkenyl group, a lower alkynyl group, an aralkyl group, a cycloalkyl group, a lower alkyl group which is substituted with group(s) selected from Substituent group α, a lower alkenyl group which is substituted with group(s) selected from Substituent group α, and an alkynyl group which is substituted with group(s) selected from Substituent group α; and
[Substituent group ε]
an oxo group, a hydroxyimino group, a lower alkoxyimino group, a lower alkylene group, a lower alkylenedioxy group, a lower alkylsulfinyl group, a lower alkylsulfonyl group, an aryl group and an aryl group which is substituted with group(s) selected from Substituent group α, Substituent group β, Substituent group γ and Substituent group δ.

Of these compositions, preferred are:

(2) compositions comprising a compound of formula (I) or a pharmacologically acceptable salt, ester or other derivative thereof, wherein $R^1$ is an aryl group; or an aryl group which is substituted with group(s) selected from Substituent group α, Substituent group β, Substituent group γ and Substituent group δ,

(3) compositions comprising a compound of formula (I) or a pharmacologically acceptable salt, ester or other derivative thereof, wherein $R^1$ is a phenyl or naphthyl group; or a phenyl or naphthyl group which is substituted with group(s) selected from Substituent group α, Substituent group β, Substituent group γ and Substituent group δ,

(4) compositions comprising a compound of formula (I) or a pharmacologically acceptable salt, ester or other derivative thereof, wherein $R^1$ is a phenyl group; or a phenyl group which is substituted with group(s) selected from Substituent group $\alpha^1$, Substituent group $\beta^1$, Substituent group $\gamma^1$ and Substituent group $\delta^1$,

(5) compositions comprising a compound of formula (I) or a pharmacologically acceptable salt, ester or other derivative thereof, wherein $R^1$ is a phenyl group; or a phenyl group which is substituted with group(s) selected from the substituent groups below,
(the substituent groups: a halogen atom, a halogeno lower alkyl group and a halogeno lower alkoxy group),

(6) compositions comprising a compound of formula (I) or a pharmacologically acceptable salt, ester or other derivative thereof, wherein $R^1$ is a phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl or 3-trifluoromethylphenyl group,

(7) compositions comprising a compound of formula (I) or a pharmacologically acceptable salt, ester or other

derivative thereof, wherein $R^2$ is a 5- or 6-membered aromatic heterocyclic group containing one or two nitrogen atoms; or a 5- or 6-membered aromatic heterocyclic group containing one or two nitrogen atoms, said group being substituted with group(s) selected from Substituent group α, Substituent group β, Substituent group γ and Substituent group δ,

(8) compositions comprising a compound of formula (I) or a pharmacologically acceptable salt, ester or other derivative thereof, wherein $R^2$ is a pyridyl or pyrimidinyl group; or a pyridyl or pyrimidinyl group which is substituted with group(s) selected from Substituent group α, Substituent group β, Substituent group γ and Substituent group δ,

(9) compositions comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted with group(s) selected from Substituent group α, Substituent group β, Substituent group γ and Substituent group δ,

(10) compositions comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted at the 2-position thereof with a group selected from Substituent group α, Substituent group β, Substituent group γ and Substituent group δ,

(11) compositions comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted at the 2-position thereof with a group selected from the substituent groups below,
(the substituent groups: methoxy, amino, methylamino, benzylamino and α-methylbenzylamino),

(12) compositions comprising a compound of formula (I) or a pharmacologically acceptable salt, ester or other derivative thereof, wherein $R^4$ is a cycloalkyl group having from 3 to 7 carbon atoms, which may be substituted with group(s) selected from Substituent group α and Substituent group δ and which is substituted with group(s) selected from Substituent group β and Substituent group γ; a phenyl or naphthyl group which may be substituted with group(s) selected from Substituent group α and Substituent group δ and which is substituted with group(s) selected from Substituent group β and Substituent group γ; a 4-to 12-membered non-aromatic heterocyclic group containing one or two heteroatom(s) selected from the group consisting of sulfur atoms, oxygen atoms and nitrogen atoms, which may be substituted with group(s) selected from Substituent group α and Substituent group δ and which is substituted with group(s) selected from Substituent group β and Substituent group γ; a 4-to 12-membered non-aromatic heterocyclic group containing one nitrogen atom and optionally one further heteroatom selected from the group consisting of oxygen atom, sulfur atom and nitrogen atom, which may be substituted with group(s) selected from Substituent group α, Substituent group δ and Substituent group ε; a 5- or 6-membered aromatic heterocyclic group containing one or two heteroatom(s) selected from the group consisting of sulfur atoms, oxygen atoms and nitrogen atoms, which may be substituted with group(s) selected from Substituent group α and Substituent group δ and which is substituted with group(s) selected from Substituent group β and Substituent group γ; a 5- or 6-membered aromatic heterocyclic group containing one or two nitrogen atoms, said group optionally being substituted with group(s) selected from Substituent group α and Substituent group δ; and a group of formula $-NR^aR^b$ wherein $R^a$ and $R^b$ are as defined above,

(13) compositions comprising a compound of formula (I) or a pharmacologically acceptable salt, ester or other derivative thereof, wherein X is a single bond; an alkylene group having from 1 to 6 carbon atoms; an alkylene group having from 1 to 6 carbon atoms which is substituted with group(s) selected from Substituent group α; an alkenylene group having from 2 to 6 carbon atoms; an alkenylene group having from 2 to 6 carbon atoms which is substituted with group(s) selected from Substituent group α; an alkynylene group having from 2 to 6 carbon atoms; or an alkynylene group having from 2 to 6 carbon atoms which is substituted with group(s) selected from Substituent group α;

(14) compositions comprising a compound of formula (I) or a pharmacologically acceptable salt, ester or other derivative thereof, wherein X is a single bond; an alkylene group having from 1 to 4 carbon atoms; an alkylene group having from 1 to 4 carbon atoms which is substituted with group(s) selected from Substituent group α; an alkenylene group having from 2 to 4 carbon atoms; an alkenylene group having from 2 to 4 carbon atoms which is substituted with group(s) selected from Substituent group α; an alkynylene group having from 2 to 4 carbon atoms; or an alkynylene group having from 2 to 4 carbon atoms which is substituted with group(s) selected from Substituent group α;

(15) compositions comprising a compound of formula (I) or a pharmacologically acceptable salt, ester or other derivative thereof, wherein X is a single bond; an alkenylene group having from 2 to 4 carbon atoms; or an alkenylene group having from 2 to 4 carbon atoms which is substituted with group(s) selected from Substituent group α;

(16) compositions comprising a compound of formula (I) or a pharmacologically acceptable salt, ester or other derivative thereof, wherein $R^3$ is a group of the following formula (II):

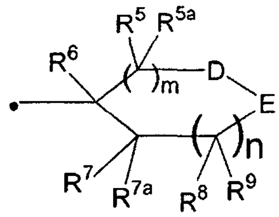
(II)

wherein:

m represents 0 or 1;
n represents 1 or 2;
$R^5$, $R^{5a}$, $R^6$, $R^7$, $R^{7a}$, $R^8$ and $R^9$ are the same or different from each other and each represents a group selected from a hydrogen atom, Substituent group α, Substituent group β, Substituent group γ, Substituent group δ and Substituent group ε;

one of D and E represents a group of formula >$NR^{10}$ (wherein $R^{10}$ is selected from a hydrogen atom, Substituent group γ and Substituent group δ), and the other represents a group of formula >$CR^{11}R^{12}$ (wherein each of $R^{11}$ and $R^{12}$ is selected from a hydrogen atom, Substituent group α, Substituent group β, Substituent group γ and Substituent group δ), or

$R^6$ may form, together with $R^5$ or $R^7$, a single bond, and/or $R^{10}$ and $R^{11}$ together may form a lower alkylene group; or a lower alkylene group which is substituted with group(s) selected from Substituent group α, Substituent group β, Substituent group γ, Substituent group δ and Substituent group ε; provided that

when one of $R^5$ and $R^{5a}$ represents an oxo group, a hydroxyimino group, a lower alkoxyimino group, a lower alkylene group or a lower alkylenedioxy group, the other is absent;
when one of $R^7$ and $R^{7a}$ represents an oxo group, a hydroxyimino group, a lower alkoxyimino group, a lower alkylene group or a lower alkylenedioxy group, the other is absent;
when one of $R^8$ and $R^9$ represents an oxo group, a hydroxyimino group, a lower alkoxyimino group, a lower alkylene group or a lower alkylenedioxy group, the other is absent;

(17) compositions comprising a compound of formula (II) or a pharmacologically acceptable salt, ester or other derivative thereof, wherein m is 1 and n is 1;

(18) compositions comprising a compound of formula (II) or a pharmacologically acceptable salt, ester or other derivative thereof, wherein E represents a group of formula >$NR^{10}$ and D represents a group of formula >$CR^{11}R^{12}$;

(19) compositions comprising a compound of formula (II) or a pharmacologically acceptable salt, ester or other derivative thereof, wherein
one of $R^5$ and $R^7$ together with $R^6$ represents a single bond and the other represents a group selected from a hydrogen atom,
Substituent group α, Substituent group γ, Substituent group δ and Substituent group ε,
$R^{5a}$, $R^{7a}$, $R^8$ and $R^9$ are the same or different from each other and each represents a group selected from a hydrogen atom,
Substituent group α, Substituent group γ, Substituent group δ and Substituent group ε,
$R^{12}$ may be the same or different from each other and each represents a group selected from a hydrogen atom, Substituent group α, Substituent group γ and Substituent group δ, and $R^{10}$ and $R^{11}$ are the same or different from each other and each represents a group selected from a hydrogen atom, Substituent group α, Substituent group γ and Substituent group δ, or $R^{10}$ and

$R^{11}$ together form a lower alkylene group or a lower alkylene group which is substituted with group(s) selected from Substituent group α, Substituent group β, Substituent group γ, Substituent group δ and Substituent group ε;

(20) compositions comprising a compound of formula (II) or a pharmacologically acceptable salt, ester or other derivative thereof, wherein $R^{10}$ and $R^{11}$ together form a straight chain alkylene group having 3 or 4 carbon atoms or a straight chain alkylene group having 3 or 4 carbon atoms which is substituted with group(s) selected from Substituent group α, Substituent group β, Substituent group γ, Substituent group δ and Substituent group ε;

(21) compositions comprising a compound of formula (II) or a pharmacologically acceptable salt, ester or other derivative thereof, wherein each of $R^{5a}$, $R^7$, $R^{7a}$, $R^8$ and $R^9$ is a hydrogen atom, and $R^{10}$ and $R^{11}$ together form a straight chain alkylene group having 3 or 4 carbon atoms or a straight chain alkylene group having 3 or 4 carbon atoms which is substituted with group(s) selected from Substituent group α, Substituent group β, Substituent group γ, Substituent group δ and Substituent group ε; and

(22) compositions comprising a compound of formula (I),
wherein said compound of formula (I) is a compound of formula (I-1) or (I-3) below:

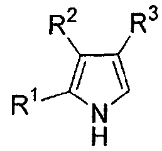

(I-1)

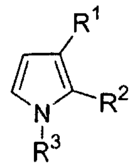

(I-3)

or a pharmacologically acceptable salt, ester or other derivative thereof.

In the above, Substituent group $\alpha^1$, Substituent group $\beta^1$, Substituent group $\gamma^1$ and Substituent group $\delta^1$ each represent as follows;

[Substituent group $\alpha^1$]
a halogen atom, a lower alkoxy group and a halogeno lower alkoxy group;

[Substituent group $\beta^1$]
a group of formula $NR^cR^d$ (wherein one of $R^c$ and $R^d$ is a hydrogen atom or a lower alkyl group and the other is a hydrogen atom, a lower alkyl group or an aralkyl group);

[Substituent group $\gamma^1$]
an amino lower alkyl group, a lower alkyl-amino lower alkyl group, di(lower alkyl)-amino lower alkyl group and an aralkyl-amino lower alkyl group; and

[Substituent group $\delta^1$]
a lower alkyl group, a halogeno lower alkyl group, a hydroxy lower alkyl group and a nitro lower alkyl group.

Further, in the compositions of the above (1), compositions satisfying a combination of requirements optionally selected from the five groups consisting of (2) to (6); (7) to (11); (12); (13) and (15); (16) to (21); and (22) are preferred.

Of these, particularly preferred compounds include:

(23) those comprising compounds selected from the following compounds or pharmacologically acceptable salts or esters or other derivatives thereof:

4-(3-aminopropyl)-2-(4-fluorophenyl)-3-(pyridin-4-yl)-1*H*-pyrrole,
4-(3-acetylaminopropyl)-2-(4-fluorophenyl)-3-(pyridin-4-yl)-1*H*-pyrrole,
2-(4-fluorophenyl)-4-(3-methylaminopropyl)-3-(pyridin-4-yl)-1*H*-pyrrole,
[5-(4-fluorophenyl)-4-(pyridin-4-yl)-1*H*-pyrrol-3-yl]-(pyridin-4-yl)methanol,
4-(1-acetyl-1,2,3,6-tetrahydropyridin-4-yl)-2-(4-fluorophenyl)-3-(pyridin-4-yl)-1*H*-pyrrole,
2-(4-fluorophenyl)-3-(pyridin-4-yl)-4-(1,2,3,6-tetrahydropyridin-4-yl)-1*H*-pyrrole,
2-(4-fluorophenyl)-4-(piperidin-4-yl)-3-(pyridin-4-yl)-1*H*-pyrrole,
3-(4-fluorophenyl)-1-(piperidin-4-yl)-2-(pyridin-4-yl)-1*H*-pyrrole,
1-(1-acetylpiperidin-4-yl)-3-(4-fluorophenyl)-2-(pyridin-4-yl)-1*H*-pyrrole,
3-(4-fluorophenyl)-1-[1-(2-nitroethyl)piperidin-4-yl]-2-(pyridin-4-yl)-1*H*-pyrrole,
3-(4-fluorophenyl)-1-[3-(morpholin-1-yl)propyl]-2-(pyridin-4-yl)-1*H*-pyrrole,
3-(4-fluorophenyl)-1-(piperidin-3-yl)-2-(pyridin-4-yl)-1*H*-pyrrole,

1-(azetidin-3-yl)-3-(4-fluorophenyl)-2-(pyridin-4-yl)-1*H*-pyrrole,
4-(3-dimethylaminopropyl)-2-(4-fluorophenyl)-3-(pyridin-4-yl)-1*H*-pyrrole,
2-(4-fluorophenyl)-4-[3-(piperidin-1-yl)propyl]-3-(pyridin-4-yl)-1*H*-pyrrole,
2-(4-fluorophenyl)-4-[3-(1-methylpiperazin-4-yl)propyl]-3-(pyridin-4-yl)-1*H*-pyrrole,
2-(4-fluorophenyl)-4-(1-methyl-1,2,3,6-tetrahydropyridin-4-yl)-3-(pyridin-4-yl)-1*H*-pyrrole,
2-(4-fluorophenyl)-4- (1-methylpiperidin-4-yl)-3-(pyridin-4-yl)-1*H*-pyrrole,
3-(4-fluorophenyl)-2-(pyridin-4-yl)-1-(2,2,6,6-tetramethylpiperidin-4-yl)-1*H*-pyrrole,
2-(4-fluorophenyl)-3-(pyridin-4-yl)-4-(quinuclidin-2-en-3-yl)-1*H*-pyrrole,
2-(4-fluorophenyl)-3-(pyridin-4-yl)-4-(quinuclidin-3-yl)-1*H*-pyrrole,
2-(4-fluorophenyl)-4-(4-hydroxypiperidin-4-yl)-3-(pyridin-4-yl)-1*H*-pyrrole,
3-(4-fluorophenyl)-2-(pyridin-4-yl)-1-(quinuclidin-3-yl)-1*H*-pyrrole,
1-(4-aminocyclohexyl)-3-(4-fluorophenyl)-2-(pyridin-4-yl)-1*H*-pyrrole,
3-(4-fluorophenyl)-2-(2-methylaminopyrimidin-4-yl)-1-(piperidin-4-yl)-1*H*-pyrrole,
2-(4-fluorophenyl)-4-(8-methyl-8-azabicyclo[3.2.1]oct-2-en-3-yl)-3-(pyridin-4-yl)-1*H*-pyrrole,
2-(4-fluorophenyl)-4-(8-methyl-8-azabicyclo[3.2.1]octan-3-yl)-3-(pyridin-4-yl)-1*H*-pyrrole,
4-(8-azabicyclo[3.2.1]oct-2-en-3-yl)-2-(4-fluorophenyl)-3-(pyridin-4-yl)-1*H*-pyrrole,
2-(4-fluorophenyl)-3-(pyridin-4-yl)-4-(1,2,3,6-tetrahydro-2,2,6,6-tetramethylpyridin-4-yl)-1*H*-pyrrole,
2-(4-fluorophenyl)-3-(pyridin-4-yl)-4-(2,2,6,6-tetramethylpiperidin-4-yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
4-(1-ethylpiperidin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(3-dimethylamino-1-propen-1-yl)-2-(4-fluorophenyl)-3-(pyridin-4-yl)-1*H*-pyrrole,
4-(4-aminobutyl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
1-(3-dimethylaminopropyl)-3-(4-fluorophenyl)-2-(pyridin-4-yl)-1*H*-pyrrole,
2-(4-fluorophenyl)-4-(1,2,3,5,6,7,8,8a-octahydroindolizin-7-yl)-3-(pyridin-4-yl)-1*H*-pyrrole,
4-(6-allyl-1-methyl-1,2,3,6-tetrahydropyridin-4-yl)-2-(4-fluorophenyl)-3-(pyridin-4-yl)-1*H*-pyrrole,
4-(2-allyl-1-methyl-1,2,3,6-tetrahydropyridin-4-yl)-2-(4-fluorophenyl)-3-(pyridin-4-yl)-1*H*-pyrrole,
4-(6-benzyl-1-methyl-1,2,3,6-tetrahydropyridin-4-yl)-2-(4-fluorophenyl)-3-(pyridin-4-yl)-1*H*-pyrrole,
4-(2-benzyl-1-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(4-fluorophenyl)-4-(1,3,4,6,9,9a-hexahydro-2*H*-quinolizin-8-yl)-3-(pyridin-4-yl)-1*H*-pyrrole,
2-(4-fluorophenyl)-4-(1,2,3,5,8,8a-hexahydroindolizin-7-yl)-3-(2-methylaminopyrimidin-4-yl)-1*H*-pyrrole,
2-(4-fluorophenyl)-4-(1,2,3,5,6,8a-hexahydroindolizin-7-yl)-3-(2-methylaminopyrimidin-4-yl) -1*H*-pyrrole,
2-(3,4-difluorophenyl)-4-(1,2,3,5,8,8a-hexahydroindolizin-7-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-3-(pyridin-4-yl)-2-(3-trifluoromethylphenyl)-1*H*-pyrrole,
4-(1,2,3,5,6,8a-hexahydroindolizin-7-yl)-3-(pyridin-4-yl)-2-(3-trifluoromethylphenyl)-1*H*-pyrrole,
2-(3-fluorophenyl)-4-(1,2,3,5,8,8a-hexahydroindolizin-7-yl)-3-(pyridin-4-yl)-1*H*-pyrrole,
2-(3-fluorophenyl)-4-(1,2,3,5,6,8a-hexahydroindolizin-7-yl)-3-(pyridin-4-yl)-1*H*-pyrrole,
2-(3-chlorophenyl)-4-(1,2,3,5,8,8a-hexahydroindolizin-7-yl)-3-(pyridin-4-yl)-1*H*-pyrrole,
2-(3-chlorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-2-phenyl-3-(pyridin-4-yl)-1*H*-pyrrole, and
4-(1,2,3,5,6,8a-hexahydroindolizin-7-yl)-2-phenyl-3-(pyridin-4-yl)-1*H*-pyrrole.

In the above (23), still particularly preferred compositions include:

(24) those containing compounds selected from the following compounds or pharmacologically acceptable salts or esters or other derivatives thereof:

2-(4-fluorophenyl)-3-(pyridin-4-yl)-4-(1,2,3,6-tetrahydropyridin-4-yl)-1*H*-pyrrole,
3-(4-fluorophenyl)-1-(piperidin-4-yl)-2-(pyridin-4-yl)-1*H*-pyrrole,
3-(4-fluorophenyl)-1-[3-(morpholin-1-yl)propyl]-2-(pyridin-4-yl)-1*H*-pyrrole,
4-(8-azabicyclo[3.2.1]oct-2-en-3-yl)-2-(4-fluorophenyl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole, and
4-(1,2,3,5,6,8a-hexahydroindolizin-7-yl)-3-(pyridin-4-yl)-2-(3-trifluoromethylphenyl)-1*H*-pyrrole.

The present invention also provides a method for the prophylaxis or treatment of hepatopathy which comprises administering to a warm blooded animal suffering therefrom an effective amount of a compound of formula (I) selected from any one of (1) to (24) or a pharmacologically acceptable salt, ester or other derivative thereof.

The present invention also provides the use of a compound of formula (I) selected from any one of (1) to (24) or a pharmacologically acceptable salt, ester or other derivative thereof in the manufacture of a medicament for the prophylaxis or treatment of hepatopathy.

The compounds of formula (I) are selected from compounds of the following formulae (I-1) to (I-5):

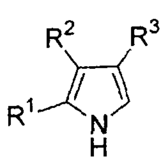
(I-1)

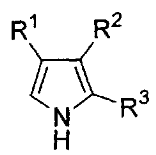
(I-2)

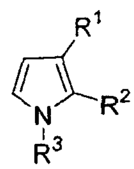
(I-3)

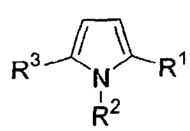
(I-4)

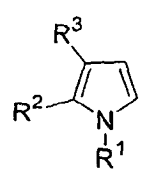
(I-5)

wherein $R^1$, $R^2$ and $R^3$ are as defined above.

In the above formula (I),

where $R^1$ and Substituent group ε represent an aryl group or an aryl group which is substituted with group(s) selected from Substituent group α, Substituent group β, Substituent group γ and Substituent group δ, or where $R^4$ represents an aryl group which may be substituted with group(s) selected from Substituent group α and Substituent group δ, and which is substituted with group(s) selected from Substituent group β and Substituent group γ, said aryl group is an aromatic hydrocarbon group having from 6 to 14 carbon atoms, and examples include phenyl, naphthyl, phenanthryl and anthracenyl groups. Of these, we prefer phenyl and naphthyl groups, most preferably phenyl group.

The aryl group defined and exemplified above may be fused with a cycloalkyl group having from 3 to 10 carbon atoms. Examples of such a fused ring group include 5-indanyl group.

Where $R^1$ and Substituent group ε represent an aryl group which is substituted with group(s) selected from Substituent group α, Substituent group β, Substituent group γ and Substituent group δ, it is preferably an aryl group substituted with 1 to 4 groups selected from Substituent group α, Substituent group β, Substituent group γ and Substituent group δ, and more preferably it is an aryl group substituted with 1 to 3 groups selected from Substituent group α, Substituent group β , Substituent group γ and Substituent group δ.

Examples of such substituted aryl groups include 4-fluorophenyl, 3-fluorophenyl, 4-chlorophenyl, 3-chlorophenyl, 3,4-difluorophenyl, 3,4-dichlorophenyl, 3,4,5-trifluorophenyl, 3-chloro-4-fluorophenyl, 3-difluoromethoxyphenyl, 3-trifluoromethoxyphenyl and 3-trifluoromethylphenyl groups.

Where $R^4$ represents an aryl group which may be substituted with group(s) selected from Substituent group α and Substituent group δ, and which is substituted with group(s) selected from Substituent group β and Substituent group γ, it is preferably an aryl group which may be substituted with one or two groups selected from Substituent group α and Substituent group δ, and which is substituted with a group selected from Substituent group β and Substituent group γ.

Examples of such substituted aryl groups include 2-aminophenyl, 3-amino-phenyl, 4-aminophenyl, 2-aminomethylphenyl, 3-aminomethylphenyl, 4-amino-methylphenyl, 2-(2-aminoethyl)phenyl, 3-(2-aminoethyl)phenyl, 4-(2-aminoethyl)-phenyl, 2-methylaminophenyl, 3-methylaminophenyl, 4-methylaminophenyl, 2-(dimethylamino)phenyl, 3-(dimethylamino)phenyl, 4-(dimethylamino)phenyl, 2-methylaminomethylphenyl, 3-methylaminomethylphenyl, 4-methylamino-methylphenyl, 2-(dimethylaminomethyl)phenyl, 3-(dimethylaminomethyl)phenyl, 4-(dimethylaminomethyl)phenyl, 3-amino-4-fluorophenyl, 3-amino-5-fluorophenyl, 2-aminomethyl-4-fluorophenyl, 3-amino-5-chlorophenyl, 2-aminomethyl-3-chlorophenyl, 3-amino-5-difluoromethoxyphenyl and 2-aminomethyl-3-trifluoromethoxyphenyl groups.

Where $R^1$ represents a heteroaryl group or a heteroaryl group which is substituted with group(s) selected from Substituent group α, Substituent group β, Substituent group γ and Substituent group δ, or where $R^4$ represents a heteroaryl group which may be substituted with group(s) selected from Substituent group α and Substituent group δ and which is substituted with group(s) selected from Substituent group β and Substituent group γ, said heteroaryl group is a 5- to 7-membered aromatic heterocyclic group containing from 1 to 3 heteroatoms selected from the group consisting of sulfur atoms, oxygen atoms and nitrogen atoms. Examples of such heteroaryl groups include furyl, thienyl, pyrrolyl, pyrazolyl, imidazolyl, oxazolyl, isoxazolyl, thiazolyl, isothiazolyl, triazolyl, tetrazolyl, thiadiazolyl, pyridyl, pyridazinyl, pyrimidinyl and pyrazinyl groups.

We prefer a 5- or 6-membered aromatic heterocyclic group containing one or two heteroatoms selected from the group consisting of sulfur atoms, oxygen atoms and nitrogen atoms, examples of which include furyl, thienyl, pyrrolyl, pyrazolyl, imidazolyl, oxazolyl, isoxazolyl, thiazolyl, isothiazolyl, pyranyl, pyridyl, pyridazinyl, pyrimidinyl and pyrazinyl groups.

Where $R^1$ represents a heteroaryl group, furyl, thienyl, pyridyl and pyrimidinyl groups are more preferred.

Where $R^4$ represents a heteroaryl group, 5- or 6-membered aromatic heterocyclic groups containing at least one nitrogen atom and optionally containing a further heteroatom selected from the group consisting of sulfur atom, oxygen atom and nitrogen atom are preferred, examples of which include pyrrolyl, pyrazolyl, imidazolyl, oxazolyl, isoxazolyl, thiazolyl, isothiazolyl, pyridyl, pyridazinyl, pyrimidinyl and pyrazinyl groups. Of these, 5- or 6-membered aromatic heterocyclic groups containing one or two nitrogen atoms, such as imidazolyl, pyridyl, pyridazinyl, pyrimidinyl, pyrazinyl, pyridyl and pyrimidinyl groups are preferred, and pyridyl and pyrimidinyl groups are particularly preferred.

The heteroaryl groups defined and exemplified above may be fused with another cyclic group (a cyclic group such as an aryl group or a cycloalkyl group having from 3 to 10 carbon atoms).

Examples of such fused heteroaryl groups include indolyl, benzofuranyl, benzothienyl, quinolyl, isoquinolyl, quinazolyl, tetrahydroquinolyl and tetrahydroisoquinolyl groups.

Where $R^1$ represents a heteroaryl group which is substituted with group(s) selected from Substituent group α, Substituent group β, Substituent group γ and Substituent group δ, said heteroaryl group is preferably a heteroaryl group substituted with from 1 to 3 group(s) selected from Substituent group α, Substituent group β, Substituent group γ and Substituent group δ, and more preferably it is a heteroaryl group substituted with one or two group(s) selected from Substituent group α, Substituent group β, Substituent group γ and Substituent group δ.

Examples of such substituted heteroaryl groups include 5-fluoro-2-furyl, 4-chloro-2-thienyl, 5-difluoromethoxy-3-furyl, 5-trifluoromethoxy-3-thienyl and 5-fluoro-2-oxazolyl groups.

Where $R^4$ represents a heteroaryl group which may be substituted with group(s) selected from Substituent group α and Substituent group δ and which is substituted with group(s) selected from Substituent group β and Substituent group γ, it is preferably a heteroaryl group which may be substituted with a group selected from Substituent group α and Substituent group δ and which is substituted with a group selected from Substituent group β and Substituent group γ.

Examples of such substituted heteroaryl groups include 5-amino-2-furyl, 5-aminomethyl-2-furyl, 5-methylaminomethyl-2-furyl, 5-dimethylaminomethyl-2-furyl, 5-amino-2-thienyl, 5-aminomethyl-2-thienyl, 5-methylaminomethyl-2-thienyl, 5-dimethylaminomethyl-2-thienyl, 5-amino-2-oxazolyl, 5-aminomethyl-2-oxazolyl, 2-amino-4-pyridyl, 2-amino-4-pyrimidinyl, 2-methylamino-4-pyridyl, 2-methyl-amino-4-pyrimidinyl, 2-benzylamino-4-pyridyl, 2-benzylamino-4-pyrimidinyl, 2-(α-methylbenzylamino)-4-pyridyl, 2-(α-methylbenzylamino)-4-pyrimidinyl, 5-amino-4-fluoro-2-furyl, 5-aminomethyl-4-fluoro-2-furyl, 5-amino-4-fluoro-2-thienyl, 5-aminomethyl-4-fluoro-2-thienyl, 4-amino-5-difluoromethoxy-2-furyl, 4-aminomethyl-5-difluoromethoxy-2-furyl, 4-amino-5-difluoromethoxy-2-thienyl and 4-aminomethyl-5-difluoromethoxy-2-thienyl groups.

Where $R^2$ represents a heteroaryl group having at least one ring nitrogen atom, or a heteroaryl group having at least one ring nitrogen atom, wherein said heteroaryl group is substituted with group(s) selected from Substituent group α, Substituent group β, Substituent group γ and Substituent group δ, or $R^4$ represents a heteroaryl group having at least one ring nitrogen atom, said heteroaryl group being optionally substituted with group(s) selected from Substituent group α and Substituent group δ, said heteroaryl group having at least one nitrogen atom is a 5- to 7-membered aromatic heterocyclic group containing at least one nitrogen atom and optionally containing one or two further heteroatoms selected from the group consisting of sulfur atoms, oxygen atoms and nitrogen atoms. Examples of such groups include pyrrolyl, pyrazolyl, imidazolyl, oxazolyl, isoxazolyl, thiazolyl, isothiazolyl, triazolyl, tetrazolyl, thiadiazolyl, pyranyl, pyridyl, pyridazinyl, pyrimidinyl and pyrazinyl groups. Of these, we prefer 5- or 6-membered aromatic heterocyclic groups containing one nitrogen atom and optionally containing one further heteroatom selected from the group consisting of sulfur atom, oxygen atom and nitrogen atom, examples of which include pyrrolyl, pyrazolyl, imidazolyl, oxazolyl, isoxazolyl, thiazolyl, isothiazolyl, pyridyl, pyridazinyl, pyrimidinyl and pyrazinyl groups. 5- or 6-membered aromatic heterocyclic groups containing one or two nitrogen atoms, such as imidazolyl, pyridyl, pyridazinyl, pyrimidinyl, pyrazinyl, pyridyl and pyrimidinyl groups, are more preferred and pyridyl and pyrimidinyl groups are particularly preferred.

Where $R^2$ represents a heteroaryl group having at least one ring nitrogen atom, 4-pyridyl and 4-pyrimidinyl groups are most preferred.

Where $R^2$ represents a heteroaryl group having at least one ring nitrogen atom, wherein said heteroaryl group is substituted with groups selected from Substituent group α, Substituent group β, Substituent group γ and Substituent group δ, said heteroaryl group is preferably a group substituted with 1 to 3 group(s) selected from Substituent group α, Substituent group β, Substituent group γ and Substituent group δ, more preferably it is a heteroaryl group substituted with one or two group(s) selected from Substituent group α, Substituent group β, Substituent group γ and Substituent group δ, still more preferably it is a heteroaryl group substituted with one group selected from Substituent group α, Substituent group β, Substituent group γ and Substituent group δ, and particularly preferably it is a 4-pyridyl or 4-pyrimidinyl group which is substituted with one group selected from Substituent group α, Substituent group β, Substituent group γ and Substituent group δ at the 2-position of said group. Most preferably, said heteroaryl group is a 4-pyridyl or 4-pyrimidinyl group which is substituted at the 2-position with one group selected from Substituent group β and Substituent group γ. Examples of such substituted heteroaryl groups include 2-amino-4-pyridyl, 2-amino-4-pyrimidinyl, 2-methylamino-4-pyridyl, 2-methylamino-4-pyrimidinyl, 2-methoxy-4-pyridyl, 2-methoxy-4-pyrimidinyl, 2-benzylamino-4-pyridyl, 2-benzylamino-4-pyrimidinyl, 2-(α-methylbenzylamino)-4-pyridyl and 2-(α-methylbenzylamino)-4-pyrimidinyl groups.

Where $R^4$ represents a heteroaryl group having at least one nitrogen atom, said heteroaryl group being optionally substituted with group(s) selected from Substituent group α and Substituent group δ, it is preferably a heteroaryl group being optionally substituted with 1 or 2 group(s) selected from Substituent group α and Substituent group δ, and more preferably it is a heteroaryl group being optionally substituted with a group selected from Substituent group α and Substituent group δ.

Examples of such substituted heteroaryl groups include 2-methyl-4-pyridyl, 2-ethyl-4-pyridyl, 2-benzyl-4-pyridyl, 2-phenethyl-4-pyridyl,2-fluoro-4-pyridyl, 2,6-difluoro-4-pyridyl and 2,3,5,6-tetrafluoro-4-pyridyl groups.

Where X represents a lower alkylene group or a lower alkylene group which is substituted with group(s) selected from Substituent group α, or where Substituent group ε represents a lower alkylene group, said lower alkylene group is a straight or branched chain alkylene group having from 1 to 6 carbon atoms, examples of which include methylene, ethylene, trimethylene, propylene, tetramethylene, 1-methyltrimethylene, 2-methyltrimethylene, 1,1-dimethylethylene, pentamethylene, 1, 1-dimethyltrimethylene and hexamethylene groups. Of these, a straight or branched chain alkylene group having from 1 to 4 carbon atoms is preferred.

Where X defined above represents a lower alkylene group, a straight or branched chain alkylene group having from 1 to 3 carbon atoms is more preferred and methylene, ethylene and trimethylene groups are most preferred.

Where Substituent group ε defined above represents a lower alkylene group, ethylene, trimethylene and tetramethylene groups are more preferred.

Where X represents a lower alkenylene group or a lower alkenylene group which is substituted with group(s) selected from Substituent group α, said lower alkenylene group is a straight or branched chain alkenylene group having from 2 to 6 carbon atoms, examples of which include vinylene, 1-methylvinylene, propenylene, 1-butenylene, 2-butenylene, 1-pentenylene and 2-pentenylene groups. Of these, a straight or branched chain alkenylene group having from 2 to 4 carbon atoms is preferred, vinylene, propenylene and butenylene groups are more preferred, and vinylene and propenylene groups are most preferred.

Where X represents a lower alkynylene group or a lower alkynylene group which is substituted with group(s) selected from Substituent group α, said lower alkynylene group is a straight or branched chain alkynylene group having from 2 to 6 carbon atoms. Of these, a straight or branched alkynylene group having from 2 to 4 carbon atoms is preferred, ethynylene, propynylene, 1-butynylene and 2-butynylene groups are more preferred, and ethynylene and propynylene groups are most preferred.

Where the Substituent group δ represents a cycloalkyl group, or where $R^4$ represents a cycloalkyl group which may be substituted with group(s) selected from Substituent group α and Substituent group δ and which is substituted with group(s) selected from Substituent group β and Substituent group γ, said cycloalkyl group has from 3 to 7 carbon atoms, examples of which include cyclopropyl, cyclobutyl, cyclopentyl, cyclohexyl and cycloheptanyl groups. Of these, a cycloalkyl group having from 3 to 6 carbon atoms is preferred.

Where $R^4$ represents a heterocyclyl group which may be substituted with group(s) selected from Substituent group α and Substituent group δ and which is substituted with group(s) selected from Substituent group β and Substituent group γ, said heterocyclyl group is a non-aromatic heterocyclic group having from 4 to 14 ring atoms in one or more rings, containing 1 to 3 heteroatoms selected from the group consisting of sulfur atoms, oxygen atoms and nitrogen atoms. It is preferably a 4-to 12-membered non-aromatic heterocyclic group (preferably a 4- to 10-membered non-aromatic heterocyclic group) containing one or two heteroatoms selected from the group consisting of sulfur atoms, oxygen atoms and nitrogen atoms, and more preferably it is a 4- to 12-membered non-aromatic heterocyclic group (preferably a 4- to 10-membered non-aromatic heterocyclic group) containing one nitrogen atom and optionally containing one further heteroatom selected from the group consisting of oxygen atom, sulfur atom and nitrogen atom. Examples of such a group include azetidinyl, pyrrolidinyl, pyrrolinyl, imidazolidinyl, imidazolinyl, pyrazolidinyl, pyrazolinyl, oxazolidinyl, thiazolidinyl, piperidyl, tetrahydropyridyl, dihydropyridyl, piperazinyl, morpholinyl, thiomorpholinyl, homopiperidyl, quinuclidinyl, quinuclidienyl, octahydroindolizinyl, hexahydroindolizinyl, octahydroquinolizinyl, hexahydroquinolizinyl, 8-azabicyclo[3.2.1]octanyl, 8-azabicyclo[3.2.1]octenyl, 9-azabicyclo[3.3.1]nonanyl and 9-azabicyclo [3.3.1]nonenyl groups, of which piperidyl, tetrahydropyridyl, homopiperidyl, quinuclidinyl, quinuclidienyl, octahydroindolizinyl, hexahydroindolizinyl, octahydroquinolizinyl, hexahydroquinolizinyl, 8-azabicyclo[3.2.1]octanyl, 8-azabicyclo [3.2.1]octenyl/ 9-azabicyclo[3.3.1]nonanyl and 9-azabicyclo[3.3.1]nonenyl groups are preferred, tetrahydropyridyl, quinuclidienyl, hexahydroindolizinyl, hexahydroquinolizinyl, 8-azabicyclo[3.2.1]octenyl and 9-azabicyclo[3.3.1]nonenyl groups are more preferred, and tetrahydropyridyl, hexahydroindolizinyl and hexahydroquinolizinyl groups are most preferred.

The heterocyclyl group defined and exemplified above may be fused with aryl groups or heteroaryl groups. Examples of such a fused heterocyclyl group include tetrahydroquinolinyl, tetrahydroisoquinolinyl, chromanyl, indolinyl and isoindolinyl groups.

Where $R^4$ represents a heterocyclyl group having at least one ring nitrogen atom in which said heterocyclyl group may be substituted with group(s) selected from Substituent group α and Substituent group δ, said heterocyclyl group having at least one nitrogen atom has the same meaning as "a 4- to 12-membered non-aromatic heterocyclic group containing one nitrogen atom and optionally containing one further heteroatom selected from the group consisting of oxygen atoms, sulfur atoms and nitrogen atoms" in the definition of heterocyclyls as defined above. Preferably, it is a 4- to 10-membered non-aromatic heterocyclic group containing one nitrogen atom and optionally containing one further heteroatom selected from the group consisting of oxygen atom, sulfur atom and nitrogen atom. Examples of such a group include azetidinyl, pyrrolidinyl, pyrrolinyl, imidazolidinyl, imidazolinyl, pyrazolidinyl, pyrazolinyl, oxazolidinyl, thiazolidinyl, piperidyl, tetrahydropyridyl, dihydropyridyl, piperazinyl, morpholinyl, thiomorpholinyl, homopiperidyl, quinuclidinyl, quinuclidienyl, octahydroindolizinyl, hexahydroindolizinyl, octahydroquinolizinyl, hexahydroquinolizinyl, 8-azabicyclo-[3.2.1]octanyl, 8-azabicyclo[3.2.1]octenyl, 9-azabicyclo[3.3.1]nonanyl and 9-azabicyclo[3.3.1]nonenyl groups, of which piperidyl, tetrahydropyridyl, homopiperidyl, quinuclidinyl, quinuclidienyl, octahydroindolizinyl, hexahydroindolizinyl, octahydroquinolizinyl, hexahydroquinolizinyl, 8-azabicyclo[3.2.1]octanyl, 8-azabicyclo[3.2.1]octenyl, 9-azabicyclo[3.3.1]nonanyl and 9-azabicyclo[3.3.1]nonenyl groups are preferred, tetrahydropyridyl, quinuclidienyl, hexahydroindolizinyl, hexahydroquinolizinyl, 8-azabicyclo[3.2.1]octenyl and 9-azabicyclo[3.3.1]nonenyl groups are more preferred, and tetrahydropyridyl, hexahydroindolizinyl and hexahydroquinolizinyl groups are most preferred.

The heterocyclyl group having at least one ring nitrogen atom defined and exemplified above may be fused with another cyclic group selected from the group consisting of aryl groups and heteroaryl groups. Examples of such fused heterocyclyl groups include tetrahydroquinolinyl, tetrahydroisoquinolinyl, indolinyl and isoindolinyl groups.

The lower alkyl groups in the definitions of substituents $R^a$, $R^b$, $R^c$, $R^d$ and Substituent group δ, the lower alkyl group of the lower alkyl group substituted with a group of formula $-NR^cR^d$ ($R^c$ and $R^d$ are as defined above) in the definition of Substituent group γ, and the lower alkyl group which is substituted with group(s) selected from Substituent group α in the definition of Substituent group δ, are straight or branched chain alkyl groups having from 1 to 6 carbon atoms. Examples of said lower alkyl groups include methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl and 2-ethylbutyl groups. Alkyl groups having from 1 to 4 carbon atoms are preferred, methyl, ethyl and propyl groups are more preferred, and methyl and ethyl groups are most preferred.

The lower alkenyl groups in the definitions of substituents $R^a$, $R^b$, $R^c$, $R^d$ and Substituent group δ, and the lower alkenyl group which is substituted with group(s) selected from Substituent group α in the definition of Substituent group δ, are straight or branched chain alkenyl groups having from 2 to 6 carbon atoms. Examples of said lower alkenyl

groups include vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl and 5-hexenyl groups. Alkenyl groups having from 2 to 4 carbon atoms are preferred, and alkenyl groups having 2 or 3 carbon atoms are most preferred.

The lower alkynyl groups in the definitions of substituents $R^a$, $R^b$, $R^c$, $R^d$ and Substituent group $\delta$, and the lower alkynyl group which is substituted with group(s) selected from Substituent group $\alpha$ in the definition of Substituent group $\delta$, are straight or branched alkynyl groups having from 2 to 6 carbon atoms. Examples of said lower alkynyl groups include ethynyl, 2-propynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl and 5-hexynyl groups. Alkynyl groups having from 2 to 4 carbon atoms are preferred, and alkynyl groups having 2 or 3 carbon atoms are most preferred.

The aralkyl groups in the definitions of $R^a$, $R^b$, $R^c$, $R^d$ and Substituent group $\delta$ are lower alkyl groups as defined above which are substituted with at least one aryl group as defined above. Examples of said aralkyl group include benzyl, indenylmethyl, phenanthrenylmethyl, anthracenylmethyl, $\alpha$-naphthylmethyl, $\beta$-naphthylmethyl, diphenylmethyl, triphenylmethyl, $\alpha$-naphthyldiphenylmethyl, 9-anthrylmethyl, piperonyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl and 6-naphthylhexyl groups, of which benzyl, phenanthrenylmethyl, anthracenylmethyl, $\alpha$-naphthylmethyl, $\beta$-naphthylmethyl, diphenylmethyl, triphenylmethyl, 9-anthrylmethyl, piperonyl, 1-phenethyl, 2-phenethyl, 1-phenylpropyl, 2-phenylpropyl, 3-phenylpropyl, 1-phenylbutyl, 2-phenylbutyl, 3-phenylbutyl and 4-phenylbutyl are preferred.

The aryl moiety of the aralkyl groups defined and exemplified above may be substituted with from 1 to 3 group (s) selected from Substituent group $\alpha$ defined above, Substituent group $\beta$ defined above, Substituent group $\gamma$ defined above and Substituent group $\delta$ defined above. Examples of said substituted aralkyl groups include aralkyl groups substituted with at least one halogen atom, examples of which include 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 3,5-difluorobenzyl, 2,5-difluorophenethyl, 2,6-difluorobenzyl, 2,4-difluorophenethyl, 3,5-dibromobenzyl, 2,5-dibromophenethyl, 2,6-dichloro-benzyl, 2,4-dichlorophenethyl, 2,3,6-trifluorobenzyl, 2,3,4-trifluorophenethyl, 3,4,5-trifluorobenzyl, 2,5,6-trifluorophenethyl, 2,4,6-trifluorobenzyl, 2,3,6-tribromophenethyl, 2,3,4-tribromobenzyl, 3,4,5-tribromophenethyl, 2,5,6-trichlorobenzyl, 2,4,6-trichlorophenethyl, 1-fluoro-2-naphthylmethyl, 2-fluoro-1-naphthylethyl, 3-fluoro-1-naphthylmethyl, 1-chloro-2-naphthylethyl, 2-chloro-1-naphthylmethyl, 3-bromo-1-naphthylethyl, 3,8-difluoro-1-naphthylmethyl, 2,3-difluoro-1-naphthylethyl, 4,8-difluoro-1-naphthylmethyl, 5,6-difluoro-1-naphthylethyl, 3,8-dichloro-1-naphthylmethyl, 2,3-dichloro-1-naphthylethyl, 4,8-dibromo-1-naphthylmethyl, 5, 6-dibromo-1-naphthylethyl, 2,3,6-trifluoro-1-naphthylmethyl, 2,3,4-trifluoro-1-naphthylethyl, 3,4,5-trifluoro-1-naphthylmethyl, 4,5,6-trifluoro-1-naphthylethyl, 2,4,8-trifluoro-1-naphthylmethyl, bis(2-fluorophenyl)methyl, 3-fluorophenylphenylmethyl, bis(4-fluorophenyl)methyl, 4-fluorophenylphenylmethyl, bis(2-chlorophenyl)methyl, bis(3-chlorophenyl)methyl, bis(4-chloro-phenyl)methyl, 4-chlorophenylphenylmethyl, 2-bromophenylphenylmethyl, 3-bromophenylphenylmethyl, bis(4-bromophenyl)methyl, bis(3,5-difluorophenyl)methyl, bis(2,5-difluorophenyl)methyl, bis(2,6-difluorophenyl)methyl, 2,4-difluorophenyl-phenylmethyl, bis(3,5-dibromophenyl)methyl, 2,5-dibromophenylphenylmethyl, 2,6-dichlorophenylphenylmethyl, bis(2,4-dichlorophenyl)methyl and bis(2,3,6-trifluorophenyl)methyl groups; aralkyl groups substituted with at least one halogeno lower alkyl group, examples of which include 2-trifluoromethylbenzyl, 3-trifluoromethylphenethyl, 4-trifluoromethylbenzyl, 2-trichloromethylphenethyl, 3-dichloromethylbenzyl, 4-trichloromethylphenethyl, 2-tribromomethylbenzyl, 3-dibromomethylphenethyl, 4-dibromomethylbenzyl, 3,5-bistrifluoromethylphenethyl, 2,5-bistrifluoromethylbenzyl, 2,6-bistrifluoromethyl-phenethyl, 2,4-bistrifluoromethylbenzyl, 3,5-bistribromomethylphenethyl, 2,5-bisdibromomethylbenzyl, 2,6-bisdichloromethylphenethyl, 2,4-bisdichloromethyl-benzyl, 2,3,6-tristrifluoromethylphenethyl, 2,3,4-tristrifluoromethylbenzyl, 3,4,5-tristrifluoromethylphenethyl, 2,5,6-tristrifluoromethylbenzyl, 2,4,6-tristrifluoro-methylphenethyl, 2,3,6-tristribromomethylbenzyl, 2,3,4-trisdibromomethylphenethyl, 3,4,5-tristribromomethylbenzyl, 2,5,6-trisdichloromethylphenethyl, 2,4,6-trisdichloromethylbenzyl, 1-trifluromethyl-2-naphthylethyl, 2-trifluoromethyl-1-naphthylmethyl, 3-trifluoromethyl-1-naphthylethyl, 1-trichloromethyl-2-naphthylmethyl, 2-dichloromethyl-1-naphthylethyl, 3-tribromomethyl-1-naphthylmethyl, 3,8-bistrifluoromethyl-1-naphthylethyl, 2,3-bistrifluoromethyl-1-naphthylmethyl, 4,8-bistrifluoromethyl-1-naphthylethyl, 5,6-bistrifluoromethyl-1-naphthylmethyl, 3,8-bistrichloromethyl-1-naphthylethyl, 2,3-bisdichloromethyl-1-naphthylmethyl, 4,8-bisdibromomethyl-1-naphthylethyl, 5,6-bistribromomethyl-1-naphthylmethyl, 2,3,6-tristrifluoromethyl-1-naphthylethyl, 2,3,4-tristrifluoromethyl-1-naphthylmethyl, 3,4,5-tristrifluoromethyl-1-naphthylethyl, 4,5,6-tristrifluoromethyl-1-naphthylmethyl, 2,4,8-tristrifluoromethyl-1-naphthylmethyl, bis (4-trifluoromethylphenyl)methyl, 4-trifluoromethylphenylphenylmethyl, bis(2-trichloromethylphenyl)methyl, bis(3-trichloromethylphenyl)-methyl, bis (4-trichloromethylphenyl)methyl, 2-tribromomethylphenylphenylmethyl, 3-tribromomethylphenylphenylmethyl, bis(4-tribromomethylphenyl)methyl, bis(3,5-bistrifluoromethylphenyl)methyl, bis(2,5-bistrifluoromethylphenyl)methyl, bis(2,6-bistrifluoromethylphenyl)methyl, 2,4-bistrifluoromethylphenylphenylmethyl, bis(3,5-bistribromomethylphenyl)methyl, 2,5-bistribromomethylphenylphenylmethyl, 2, 6-bistrichloromethylphenylphenylmethyl, bis(2,4-bistrichloromethylphenyl)methyl and bis(2,3,6-tristrifluoromethylphenyl)methyl groups; aralkyl groups substituted with at least one lower alkyl group, examples of which include 2-methylbenzyl, 3-methylbenzyl, 4-methylbenzyl, 2-methylphenethyl, 4-methyl-phenethyl, 2-ethylbenzyl, 3-propylphenethyl, 4-ethylbenzyl, 2-butylphenethyl, 3-pentylbenzyl, 4-pentylphenethyl, 3,5-dimethylbenzyl, 2,5-dimethylphenethyl, 2,6-dimethylbenzyl, 2,4-dimethylphenethyl, 3,5-dibutylbenzyl, 2,5-dipentylphenethyl, 2,6-dipropylbenzyl, 2,4-dipropylphenethyl, 2,3,6-trimethylbenzyl, 2,3,4-trimethylphenethyl, 3,4,5-trimethylbenzyl, 2,4,6-trimethylbenzyl, 2,5,6-trimethylphenethyl, 2,3,6-tributylphenethyl, 2,3,4-tripentylbenzyl, 3,4,5-tributylphenethyl, 2,5,6-tripropylbenzyl, 2,4,6-tripropylphenethyl, 1-methyl-2-naphthylmethyl, 2-methyl-1-naphthylethyl, 3-methyl-1-naphthylmethyl, 1-ethyl-2-naphthylethyl, 2-propyl-1-naphthylmethyl, 3-butyl-1-naphthylethyl, 3,8-dimethyl-1-naphthylmethyl, 2,3-dimethyl-1-naphthylethyl, 4,8-dimethyl-1-naphthylmethyl, 5,6-dimethyl-1-naphthylethyl, 3,8-diethyl-1-naphthylmethyl, 2,3-dipropyl-1-naphthylmethyl, 4,8-dipentyl-1-naphthylethyl, 5,6-dibutyl-1-naphthylmethyl, 2,3,6-trimethyl-1-naphthylmethyl, 2,3,4-trimethyl-1-naphthylethyl, 3,4,5-trimethyl-1-naphthylmethyl, 4,5,6-trimethyl-1-naphthylmethyl, 2,4,8-trimethyl-1-naphthylmethyl, bis (2-methyl-phenyl)methyl, 3-methylphenylphenylmethyl, bis(4-methylphenyl)methyl, 4-methylphenylphenylmethyl, bis(2-ethylphenyl)methyl, bis(3-ethylphenyl)methyl, bis(4-ethylphenyl)methyl, 2-propylphenylphenylmethyl, 3-propylphenylphenylmethyl, bis(4-propylphenyl)methyl, bis(3,5-dimethylphenyl)methyl, bis(2,5-dimethylphenyl)-methyl, bis(2,6-dimethylphenyl)methyl, 2,4-dimethylphenylphenylmethyl, bis(3,5-dipropylphenyl)methyl, 2,5-dipropylphenylphenylmethyl, 2,6-diethylphenylphenylmethyl, bis (2,4-diethylphenyl)methyl and bis(2,3,6-trimethylphenyl)methyl groups; aralkyl groups substituted with at least one lower alkoxy group, examples of which include 2-methoxybenzyl, 3-methoxybenzyl, 4-methoxy-benzyl, 3-methoxyphenethyl, 2-ethoxyphenethyl, 3-propoxybenzyl, 4-ethoxy-phenethyl, 2-butoxybenzyl, 3-pentoxyphenethyl, 4-pentoxybenzyl, 3,5-dimethoxy-phenethyl, 2,5-dimethoxybenzyl, 2,6-dimethoxyphenethyl, 2,4-dimethoxybenzyl, 3,5-dibutoxyphenethyl, 2,5-dipentoxybenzyl, 2,6-dipropoxyphenethyl, 2,4-dipropoxy-benzyl, 2,3,6-trimethoxyphenethyl, 2,3,4-trimethoxybenzyl, 3,4,5-trimethoxy-phenethyl, 2,5,6-trimethoxybenzyl, 2,4,6-trimethoxyphenethyl, 2,3,6-tributoxybenzyl, 2,3,4-tripentoxyphenethyl, 3,4,5-tributoxybenzyl, 2,5,6-tripropoxyphenethyl, 2,4,6-tripropoxybenzyl, 1-methoxy-2-naphthylmethyl, 2-methoxy-1-naphthylmethyl, 3-methoxy-1-naphthylethyl, 1-ethoxy-2-naphthylmethyl, 2-propoxy-1-naphthyl-methyl, 3-butoxy-1-naphthylethyl, 3,8-dimethoxy-1-naphthylmethyl, 2,3-dimethoxy-1-naphthylmethyl, 4,8-dimethoxy-1-naphthylethyl, 5,6-dimethoxy-1-naphthylmethyl, 3,8-diethoxy-1-naphthylmethyl, 2,3-dipropoxy-1-naphthylethyl, 4,8-dipentoxy-1-naphthylmethyl, 5,6-dibutoxy-1-naphthylmethyl, 2,3,6-trimethoxy-1-naphthylethyl, 2,3,4-trimethoxy-1-naphthylmethyl, 3,4,5-trimethoxy-1-naphthylmethyl, 4,5,6-trimethoxy-1-naphthylethyl, 2,4,8-trimethoxy-1-naphthylmethyl, bis(2-methoxyphenyl)methyl, 3-methoxyphenylphenylmethyl, bis(4-methoxyphenyl)-methyl, 4-methoxyphenylphenylmethyl, bis(2-ethoxyphenyl)methyl, bis(3-ethoxyphenyl)methyl, bis(4-ethoxyphenyl)methyl, 2-propoxyphenylphenylmethyl, 3-propoxyphenylphenylmethyl, bis(4-propoxyphenyl)methyl, bis(3,5-dimethoxyphenyl)methyl, bis(2,5-dimethoxyphenyl)methyl, bis(2,6-dimethoxyphenyl)methyl, 2,4-dimethoxyphenylphenylmethyl, bis (3,5-dipropoxyphenyl)methyl, 2,5-dipropoxy-phenylphenylmethyl, 2,6-diethoxyphenylphenylmethyl, bis(2,4-diethoxyphenyl)methyl and bis(2,3,6-trimethoxyphenyl)methyl groups; aralkyl groups substituted with at least one amino group, examples of which include 2-aminophenethyl, 3-aminobenzyl, 4-aminophenethyl, 3,5-diaminobenzyl, 2,5-diaminophenethyl, 2,6-diaminobenzyl,. 2,4-diaminophenethyl, 2,3,6-triaminobenzyl, 2,3,4-triaminophenethyl, 3,4,5-triaminobenzyl, 2,5,6-triaminophenethyl, 2,4,6-triaminobenzyl, 1-amino-2-naphthylmethyl, 2-amino-1-naphthylethyl, 3-amino-1-naphthylmethyl, 3,8-diamino-1-naphthylmethyl, 2,3-diamino-1-naphthylethyl, 4, 8-diamino-1-naphthylmethyl, 5,6-diamino-1-naphthylmethyl, 2,3,6-triamino-1-naphthylethyl, 2,3,4-triamino-1-naphthylmethyl, 3,4,5-triamino-1-naphthylmethyl, 4,5,6-triamino-1-naphthylethyl, 2,4,8-triamino-1-naphthylmethyl, bis(2-aminophenyl)methyl, 3-amino-phenylphenylmethyl, bis(4-aminophenyl)methyl, 4-aminophenylphenylmethyl, bis(3,5-diaminophenyl)methyl, bis (2,5-diaminophenyl)methyl, bis(2,6-diamino-phenyl)methyl, 2,4-diaminophenylphenylmethyl and bis(2,3,6-triaminophenyl)-methyl groups; aralkyl groups substituted with at least one nitro group, examples of which include 2-nitrophenethyl, 3-nitrobenzyl, 4-nitrobenzyl, 4-nitrophenethyl, 3,5-dinitrobenzyl, 2,5-dinitrophenethyl, 2,6-dinitrobenzyl, 2,4-dinitrophenethyl, 2,3,6-trinitrobenzyl, 2,3,4-trinitrophenethyl, 3,4,5-trinitrobenzyl, 2,5,6-trinitrophenethyl, 2,4,6-trinitrobenzyl, 1-nitro-2-naphthylmethyl, 2-nitro-1-naphthylethyl, 3-nitro-1-naphthylmethyl, 3,8-dinitro-1-naphthylmethyl, 2,3-dinitro-1-naphthylethyl., 4,8-dinitro-1-naphthylmethyl, 5,6-dinitro-1-naphthylmethyl, 2,3,6-trinitro-1-naphthyl-ethyl, 2,3,4-trinitro-1-naphthylmethyl, 3,4,5-trinitro-1-naphthylmethyl, 4,5,6-trinitro-1-naphthylethyl, 2,4,8-trinitro-1-naphthylmethyl, bis(2-nitrophenyl)methyl, 3-nitro-phenylphenylmethyl, bis(4-nitrophenyl)methyl, 4-nitrophenylphenylmethyl, bis (3,5-dinitrophenyl)methyl, bis(2,5-dinitrophenyl)methyl, bis(2,6-dinitrophenyl)methyl, 2,4-dinitrophenylphenylmethyl and bis(2,3,6-trinitrophenyl)methyl groups; and aralkyl groups substituted with at least one cyano group, examples of which include 2-cyanophenethyl, 3-cyanobenzyl, 4-cyanobenzyl, 4-cyanobenzyldiphenylmethyl, 4-cyanophenethyl,

3,5-dicyanobenzyl, 2,5-dicyanophenethyl, 2,6-dicyanobenzyl, 2,4-dicyanophenethyl, 2,3,6-tricyanobenzyl, 2,3,4-tricyanophenethyl, 3,4,5-tricyanobenzyl, 2,5,6-tricyanophenethyl, 2,4,6-tricyanobenzyl, 1-cyano-2-naphthylmethyl, 3-cyano-1-naphthylmethyl, 3,8-dicyano-1-naphthylmethyl/ 2,3-dicyano-1-naphthyl-ethyl, 4,8-dicyano-1-naphthylmethyl, 5,6-dicyano-1-naphthylmethyl, 2,3,6-tricyano-1-naphthylethyl, 2,3,4-tricyano-1-naphthylmethyl, 3,4,5-tricyano-1-naphthylmethyl, 4,5,6-tricyano-1-naphthylethyl, 2,4,8-tricyano-1-naphthylmethyl, bis(2-cyanophenyl)-methyl, 3-cyanophenylphenylmethyl, bis(4-cyanophenyl)methyl, 4-cyanophenyl-phenylmethyl, bis(3,5-dicyanophenyl)methyl, bis (2,5-dicyanophenyl)methyl, bis(2,6-dicyanophenyl)methyl, 2,4-dicyanophenylphenylmethyl and bis (2,3, 6-tricyanophenyl) methyl groups.

Of the above, unsubstituted aralkyl groups and aralkyl groups substituted with at least one substituent selected from the group consisting of halogen atoms, lower alkyl groups and lower alkoxy groups are preferred, unsubstituted aralkyl groups and aralkyl groups substituted with at least one substituent selected from the group consisting of halogen atoms and lower alkyl groups are more preferred, and unsubstituted aralkyl groups are most preferred.

Where substituents $R^a$, $R^b$, $R^c$, $R^d$ and Substituent ε represent a lower alkylsulfonyl group, this is a group in which a lower alkyl group, defined and exemplified above, is bonded to a sulfonyl group ($-SO_2-$). The lower alkylsulfonyl group is preferably a straight or branched chain alkylsulfonyl group having from 1 to 4 carbon atoms, more preferably a methylsulfonyl, ethylsulfonyl, propylsulfonyl, isopropylsulfonyl or butylsulfonyl group, and most preferably a methylsulfonyl, ethylsulfonyl or propylsulfonyl group.

Where substituents $R^c$ and $R^d$ together with the nitrogen atom to which they are bonded form a heterocyclyl group, said heterocyclyl group is a heterocyclyl group having at least one nitrogen atom as defined above and further bonding to another group at a ring nitrogen atom. Examples of said heterocyclyl group include 1-azetidinyl, 1-pyrrolidinyl, 1-pyrrolinyl, 1-imidazolidinyl, 1-imidazolinyl, 1-pyrazolidinyl, 1-pyrazolinyl, 3-oxazolidinyl, 3-thiazolidinyl, 1-piperidyl, tetrahydropyridin-1-yl, dihydropyridin-1-yl, 1-piperazinyl, 4-morpholinyl, 4-thiomorpholinyl, 1-homopiperidyl, 8-azabicyclo[3.2.1]octan-8-yl, 8-azabicyclo[3.2.1]octen-8-yl, 9-azabicyclo[3.3.1]nonan-9-yl and 9-azabicyclo[3.3.1] nonen-9-yl groups.

The said heterocyclyl group as defined and exemplified above may be fused with another cyclic group selected from the group consisting of aryl groups and heteroaryl groups. Examples of such fused heterocyclyl groups include tetrahydroquinolin-1-yl and tetrahydroisoquinolin-2-yl.

The halogen atoms in the definition of Substituent group α include fluorine, chlorine, bromine and iodine atoms, of which fluorine and chlorine atoms are preferred.

Where the substituent in the definition of Substituent group α is a lower alkoxy group, this is a group in which an oxygen atom is bonded to a lower alkyl group as defined and exemplified above. The alkoxy group is preferably a straight or branched chain alkoxy group having 1 to 4 carbon atoms, more preferably a methoxy, ethoxy, propoxy, isopropoxy or butoxy group, and particularly preferably a methoxy, ethoxy or propoxy group.

Where the substituent in the definition of Substituent group α is a halogeno lower alkoxy group, this is a group in which one or more hydrogen atoms in a lower alkoxy group as defined above is substituted with halogen atom(s) as exemplified above. The halogeno lower alkoxy group preferably has from 1 to 4 carbon atoms, and is more preferably selected from the group consisting of difluoromethoxy, trifluoromethoxy and 2,2,2-trifluoromethoxy groups. Difluoromethoxy group is most preferred.

Where the substituent in the definition of Substituent group α is a lower alkylthio group, this is a group in which a sulfur atom is bonded to a lower alkyl group as defined and exemplified above. The lower alkylthio group is preferably a straight or branched chain alkylthio group having from 1 to 4 carbon atoms, more preferably a methylthio, ethylthio, propylthio, isopropylthio or butylthio group, and particularly preferably a methylthio, ethylthio or propylthio group.

Where the substituent in the definition of Substituent group α is a halogeno lower alkylthio group, this is a group in which one or more hydrogen atoms in a lower alkylthio group as defined above is substituted with halogen atom(s) as exemplified above. The halogeno lower alkylthio group preferably has from 1 to 4 carbon atoms, and is more preferably selected from the group consisting of difluoromethylthio, trifluoromethylthio and 2,2,2-trifluoroethylthio groups.

Where the substituent in the definition of Substituent group ε is a lower alkoxyimino group, this is a group in which the hydrogen atom in a hydroxyimino group as defined above is substituted with one lower alkyl group as exemplified above. The lower alkoxyimino group preferably has from 1 to 4 carbon atoms, and is more preferably selected from the group consisting of methoxyimino, ethoxyimino and propoxyimino groups.

Where the substituent in the definition of Substituent group ε is a lower alkylenedioxy group, this is a group in which the alkylene moiety is a straight or branched chain alkylene having from 1 to 6 carbon atoms, such as methylene, ethylene, trimethylene, propylene, tetramethylene, 1-methyltrimethylene, 2-methyltrimethylene, 1,1-dimethylethylene, pentamethylene, 1,1-dimethyltrimethylene and hexamethylene groups. The alkylenedioxy group is preferably a straight or branched chain alkylenedioxy group having from 1 to 4 carbon atoms, more preferably a methylenedioxy, ethylenedioxy, trimethylenedioxy, propylenedioxy or tetramethylenedioxy group.

Where Substituent ε represents a lower alkylsulfinyl group, this is a group in which a sulfinyl group (-SO-) is

bonded to a lower alkyl group as defined and exemplified above. The lower alkylsulfinyl group is preferably a straight or branched chain alkylsulfinyl group having from 1 to 4 carbon atoms, more preferably a methylsulfinyl, ethylsulfinyl, propylsulfinyl, isopropylsulfinyl or butylsulfinyl group, and most preferably a methylsulfinyl, ethylsulfinyl or propylsulfinyl group.

A preferred group of substituents of Substituent group $\alpha$ is Substituent group $\alpha^1$ which comprises a halogen atom, a lower alkoxy group and a halogeno lower alkoxy group.

A preferred group of substituents of Substituent group $\beta$ is Substituent group $\beta^1$ which comprises a group of formula $-NR^cR^d$ (wherein one of $R^c$ and $R^d$ is a hydrogen atom or a lower alkyl group and the other is a hydrogen atom, a lower alkyl group or an aralkyl group).

A preferred group of substituents of Substituent group $\gamma$ is Substituent group $\gamma^1$ which comprises an amino lower alkyl group, a lower alkyl-amino lower alkyl group, a di(lower alkyl)-amino lower alkyl group and an aralkyl-amino lower alkyl group.

Where the substituent in the definition of Substituent group $\gamma^1$ is an amino lower alkyl group, a lower alkyl-amino lower alkyl group, a di(lower alkyl)-amino lower alkyl group or an aralkyl-amino lower alkyl group, this is a group in which one or more hydrogen atoms of a lower alkyl group as defined above is substituted with a group of formula $-NR^cR^d$ as in Substituent group $\beta^1$ (wherein one of $R^c$ and $R^d$ is a hydrogen atom or a lower alkyl group and the other is a hydrogen atom, a lower alkyl group or an aralkyl group).

Of these, groups in which the alkyl moiety has from 1 to 4 carbon atoms are preferred. Aminomethyl, 2-aminoethyl, 3-aminopropyl, methylaminomethyl, 2-(methylamino)ethyl, 3-(methylamino)propyl, ethylaminomethyl, 2-(ethylamino)ethyl, 3-(ethylamino)propyl, dimethylaminomethyl, 2-(dimethylamino)ethyl, 3-(dimethylamino)propyl, diethylaminomethyl, 2-(diethylamino)ethyl, 3-(diethylamino)propyl, benzylaminomethyl, 2-(benzylamino)ethyl and 3-(benzylamino)propyl groups are more preferred.

A preferred group of substituents of Substituent group $\delta$ is Substituent group $\delta^1$ which comprises a lower alkyl group, a halogeno lower alkyl group, a hydroxy lower alkyl group and a nitro lower alkyl group.

The halogeno lower alkyl group in the definition of Substituent group $\delta^1$ above is a lower alkyl group as defined above wherein one or more hydrogen atoms are substituted with halogen atom(s).

Halogeno alkyl groups having from 1 to 4 carbon atoms are preferred, trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl and 2,2-dibromoethyl groups are more preferred, trifluoromethyl, trichloromethyl, difluoromethyl and fluoromethyl groups are still more preferred, and trifluoromethyl group is most preferred.

The hydroxy lower alkyl group in the definition of Substituent group $\delta^1$ above is a lower alkyl group as defined above wherein one or more hydrogen atoms are substituted with hydroxy group(s). Hydroxyalkyl groups having from 1 to 4 carbon atoms are preferred, and hydroxymethyl, 2-hydroxyethyl and 3-hydroxypropyl group are most preferred.

The nitro lower alkyl group in the definition of Substituent group $\delta^1$ above is a lower alkyl group as defined above wherein one or more hydrogen atoms are substituted with nitro group(s). Nitroalkyl groups having from 1 to 4 carbon atoms are preferred, and nitromethyl, 2-nitroethyl and 3-nitropropyl are most preferred.

Where $R^3$ is a group of formula $-X-R^4$ wherein X is a single bond and $R^4$ is a heterocyclyl group which may be substituted with group(s) selected from Substituent group $\alpha$ and Substituent group $\delta$, and which is substituted with group(s) selected from Substituent group $\beta$ and Substituent group $\gamma$ or it is a heteroaryl group having at least one nitrogen atom, said heteroaryl group being optionally substituted with group(s) selected from Substituent group $\alpha$ and Substituent group $\delta$, the group $R^3$ is preferably a group of the following formula (II):

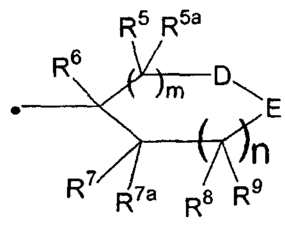

(II)

wherein:

m represents 0 or 1;
n represents 1 or 2;
$R^5$, $R^{5a}$, $R^6$, $R^7$, $R^{7a}$, $R^8$ and $R^9$ are the same or different from each other and each represents a group selected from a hydrogen atom, Substituent group $\alpha$, Substituent group $\beta$, Substituent group $\gamma$, Substituent group $\delta$ and

Substituent group ε;

one of D and E represents a group of formula $>NR^{10}$ (wherein $R^{10}$ is selected from a hydrogen atom, Substituent group γ and Substituent group δ), and the other represents a group of formula $>CR^{11}R^{12}$ (wherein each of $R^{11}$ and $R^{12}$ is selected from a hydrogen atom, Substituent group α, Substituent group β, Substituent group γ and Substituent group δ), or

$R^6$ may form, together with $R^5$ or $R^7$, a single bond, and/or $R^{10}$ and $R^{11}$ together may form a lower alkylene group; or a lower alkylene group which is substituted with group(s) selected from Substituent group α, Substituent group β, Substituent group γ, Substituent group δ and Substituent group ε;

provided that

when one of $R^5$ and $R^{5a}$ represents an oxo group, a hydroxyimino group, a lower alkoxyimino group, a lower alkylene group or a lower alkylenedioxy group, the other is absent;

when one of $R^7$ and $R^{7a}$ represents an oxo group, a hydroxyimino group, a lower alkoxyimino group, a lower alkylene group or a lower alkylenedioxy group, the other is absent;

when one of $R^8$ and $R^9$ represents an oxo group, a hydroxyimino group, a lower alkoxyimino group, a lower alkylene group or a lower alkylenedioxy group, the other is absent.

Preferably, one of $R^5$ and $R^7$ forms a single bond together with $R^6$, and the other is selected from the group consisting of a hydrogen atom, Substituent group α, Substituent group γ and Substituent group δ, more preferably selected from a hydrogen atom, a lower alkyl group and an aralkyl group, and most preferably selected from a hydrogen atom, an alkyl group having from 1 to 4 carbon atoms, a benzyl group and a phenethyl group.

Each of $R^8$, $R^9$, $R^{10}$, $R^{11}$ and $R^{12}$ is independently selected from a hydrogen atom, Substituent group α, Substituent group β, Substituent group γ, Substituent group δ, preferably selected from a hydrogen atom, Substituent group γ and Substituent group δ, more preferably selected from a hydrogen atom, a lower alkyl group and an aralkyl group, and most preferably selected from a hydrogen atom, an alkyl group having from 1 to 4 carbon atoms, a benzyl group and a phenethyl group.

The lower alkylene group which is formed by $R^{10}$ and $R^{11}$ together, and the lower alkylene group which is substituted with group(s) selected from Substituent group α, Substituent group β, Substituent group γ, Substituent group δ and Substituent group ε, is a straight or branched chain alkylene group having from 1 to 6 carbon atoms, examples of which include methylene, ethylene, trimethylene, propylene, tetramethylene, 1-methyltrimethylene, 2-methyltrimethylene, 1,1-dimethylethylene, pentamethylene, 1,1-dimethyltrimethylene, 2,2-dimethyltrimethylene, 1,2-dimethyltrimethylene and hexamethylene groups. A straight or branched chain alkylene group having from 3 to 6 carbon atoms is preferred, a straight or branched chain alkylene group having 3 or 4 carbon atoms is more preferred, and a straight chain alkylene group having 3 or 4 carbon atoms is most preferred.

Preferred examples of the above group of formula (II) include:

(a) a group wherein m is 1 and n is 1;

(b) a group wherein one of $R^5$ and $R^7$ together with $R^6$ represents a single bond and the other is selected from a hydrogen atom, Substituent group α, Substituent group γ, Substituent group δ and Substituent group ε;

(c) a group wherein $R^{5a}$, $R^{7a}$ $R^8$ and $R^9$ are the same or different from each other and each is selected from a hydrogen atom, Substituent group α, Substituent group γ, Substituent group δ and Substituent group ε;

(d) a group wherein $R^{10}$, $R^{11}$ and $R^{12}$ are the same or different from each other and each is selected from a hydrogen atom, Substituent group α, Substituent group γ and Substituent group δ;

(e) a group wherein $R^{10}$ and $R^{11}$ together form a straight chain alkylene group having 3 or 4 carbon atoms, or a straight chain alkylene group having 3 or 4 carbon atoms which is substituted with group(s) selected from Substituent group α, Substituent group β, Substituent group γ, Substituent group δ and Substituent group ε; and

(f) a group wherein each of $R^{5a}$, $R^7$, $R^{7a}$, $R^8$ and $R^9$ is a hydrogen atom, and $R^{10}$ and $R^{11}$ together form a straight chain alkylene group having 3 or 4 carbon atoms or a straight chain alkylene group having 3 or 4 carbon atoms which is substituted with group(s) selected from Substituent group α, Substituent group β, Substituent group γ, Substituent group δ and Substituent group ε.

Of these, more preferred examples of the above group include:

(g) a group wherein m is 1, n is 1, D represents a group of formula $>CR^{11}R^{12}$ and E represents a group of formula $>NR^{10}$; and

(h) a group wherein m is 1, n is 1, D represents a group of formula $>CR^{11}R^{12}$, E represents a group of formula $>NR^{10}$, and at least one of the substituents $R^8$, $R^9$, $R^{11}$ and $R^{12}$ is a substituent selected from Substituent group α, Substituent group γ and Substituent group δ.

A preferred group of the above group of formula (II) wherein $R^{10}$ and $R^{11}$ together form a lower alkylene group or a lower alkylene group which is substituted with group(s) selected from Substituent group α, Substituent group β, Substituent group γ, Substituent group δ and Substituent group ε is represented by a group selected from groups of following formulae (IIa), (IIb) and (IIc):

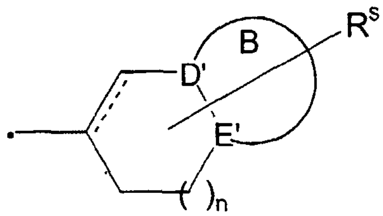
(IIa)

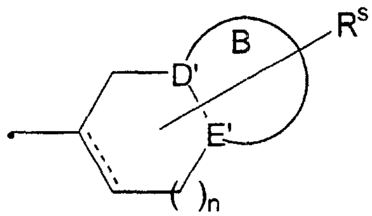
(IIb)

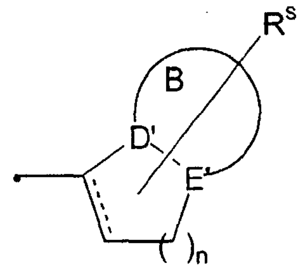
(IIc)

wherein:

a bond including a dotted line represents a single bond or a double bond;
n has the same meaning as defined above;

one of D' and E' represents a nitrogen atom, and the other represents a group of formula >$CR^{12}$- (wherein $R^{12}$ has the same meaning as defined above);

B represents D'; E'; or a 4- to 7-membered heterocyclic group having from 2 to 5 carbon atoms;
$R^s$ represents one to three groups selected from a hydrogen atom, Substituent group α, Substituent group β, Substituent group γ, Substituent group δ and Substituent group ε.

The group B represents a cyclic group corresponding to a cyclic group which is formed by $R^{10}$ and $R^{11}$ together, and

$R^s$ represents a group corresponding to $R^5$, $R^{5a}$, $R^6$, $R^7$, $R7^a$, $R^8$, $R^9$, $R^{12}$ or a substituent on the cyclic group B.

Preferred examples of the above group include:

(i) a group selected from groups of formulae (IIa), (IIb) and (IIc) wherein the bond including a dotted line is a double bond;
(j) a group selected from groups of formulae (IIa), (IIb) and (IIc) wherein n is 1;
(k) a group selected from groups of formulae (IIa), (IIb) and (IIe) wherein D' represents a group of formula >$C(R^{12})$- and E represents a nitrogen atom;
(l) a group selected from groups of formulae (IIa), (IIb) and (IIc) wherein B represents D'; E'; or a 5- or 6-membered heterocyclic group having 3 or 4 carbon atoms; and
(m) a group represented by the formula (IIa).

In the definition of $R^3$, we also prefer a group of formula -X-$R^4$ wherein X is a lower alkenylene group or a lower alkenylene group which is substituted with group(s) selected from Substituent group α, and $R^4$ is a heterocyclyl group which may be substituted with group(s) selected from Substituent group α and Substituent group δ and which is substituted with group(s) selected from Substituent group β and Substituent group γ, or is a heterocyclyl group having at least one nitrogen atom, which may be substituted with group(s) selected from Substituent group α, Substituent group δ and Substituent group ε.

In this case, preferred examples of such a heterocyclyl group for $R^4$ include 2-pyrrolidinyl, 1-methyl-2-pyrrolidinyl, 1-ethyl-2-pyrrolidinyl, 1-propyl-2-pyrrolidinyl, 2-piperidinyl, 1-methyl-2-piperidinyl, 1-ethyl-2-piperidinyl and 1-propyl-2-piperidinyl groups.

"The esters and other derivatives" are compounds in which a functional group (for example, a hydroxyl group, an amino group, an imino group or a sulfonamide group) of said compound is modified by the addition of a protecting group using conventional techniques well-known in the art.

"Esters and other derivatives" are compounds of formula (I) in which a functional group (for example, a hydroxyl group, an amino group, an imino group or a sulfonamide group) of said compound of formula (I) is modified by the addition of a protecting group using conventional techniques well-known in the art.

In the case where a compound of the present invention includes, for example, a hydroxy group therein, such "esters and other derivatives" can be obtained by protecting said hydroxy group with a general protecting group or a protecting group which is capable of being removed by a metabolic process (e.g. hydrolysis) *in vivo.*

The general protecting group referred to above is a protecting group which is removable by a chemical process such as hydrogenolysis, hydrolysis, electrolysis or photolysis. Preferred examples of such a general protecting group include the following:

aliphatic acyl groups (preferably lower aliphatic acyl groups having from 1 to 6 carbon atoms), examples of which include alkanoyl groups such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl groups,
halogenated alkylcarbonyl groups such as chloroacetyl, dichloroacetyl, trichloroacetyl and trifluoroacetyl groups,
lower alkoxyalkylcarbonyl groups such as methoxyacetyl group, and
unsaturated alkylcarbonyl groups such as acryloyl, propioloyl, methacryloyl, crotonoyl, isocrotonoyl and (E)-2-methyl-2-butenoyl groups;
aromatic acyl groups, examples of which include
arylcarbonyl groups such as benzoyl, α-naphthoyl and β-naphthoyl groups,
halogenated arylcarbonyl groups such as 2-bromobenzoyl, 4-chlorobenzoyl and 2,4,6-trifluorobenzoyl groups,
lower alkylated arylcarbonyl groups such as 2,4,6-trimethylbenzoyl and 4-toluoyl groups,
lower alkoxylated arylcarbonyl groups such as 4-anisoyl group,
nitrated arylcarbonyl groups such as 4-nitrobenzoyl and 2-nitrobenzoyl groups,
lower alkoxycarbonylated arylcarbonyl groups such as 2-(methoxycarbonyl)benzoyl group, and
arylated arylcarbonyl groups such as 4-phenylbenzoyl group;
alkoxycarbonyl groups, examples of which include
lower alkoxycarbonyl groups such as methoxycarbonyl, ethoxycarbonyl, propoxycarbonyl, butoxycarbonyl, s-butoxycarbonyl, t-butoxycarbonyl and isobutoxycarbonyl groups, and
lower alkoxycarbonyl groups which are substituted with halogen atom(s) or tri(lower alkyl)silyl group(s) such as 2,2,2-trichloroethoxycarbonyl and 2-trimethylsilylethoxycarbonyl groups;
tetrahydropyranyl or tetrahydrothiopyranyl groups such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl groups;
tetrahydrofuranyl or tetrahydrothiofuranyl groups such as tetrahydrofuran-2-yl and tetrahydrothiofuran-2-yl groups;
silyl groups, examples of which include
tri(lower alkyl)silyl groups such as trimethylsilyl, triethylsilyl, isopropyldimethylsilyl, t-butyldimethylsilyl, methyldiisopropylsilyl, methyl-di-t-butylsilyl and triisopropylsilyl groups, and
tri(lower alkyl)silyl groups in which 1 or 2 lower alkyl groups are replaced by aryl group(s) such as diphenylmethylsilyl, diphenylbutylsilyl, diphenylisopropylsilyl and phenyldiisopropylsilyl groups;
alkoxymethyl groups, examples of which include
lower alkoxymethyl groups such as methoxymethyl, 1,1-dimethyl-1-methoxymethyl, ethoxymethyl, propoxymethyl, isopropoxymethyl, butoxymethyl and t-butoxymethyl groups,
lower alkoxylated lower alkoxymethyl groups such as 2-methoxyethoxymethyl group, and
halogeno lower alkoxymethyl groups such as 2,2,2-trichloroethoxymethyl and bis(2-chloroethoxy)methyl groups;
substituted ethyl groups, examples of which include
lower alkoxylated ethyl groups such as 1-ethoxyethyl and 1-(isopropoxy)ethyl groups, and
halogenated ethyl groups such as 2,2,2-trichloroethyl group;
aralkyl groups, examples of which include
lower alkyl groups which are substituted with from 1 to 3 aryl groups, examples of which include benzyl, α-naphthylmethyl, β-naphthylmethyl, diphenylmethyl, triphenylmethyl, α-naphthyldiphenylmethyl and 9-anthrylmethyl groups, and
lower alkyl groups which are substituted with from 1 to 3 aryl groups in which said aryl moiety is substituted with substituent(s) selected from the group consisting of lower alkyl groups, lower alkoxy groups, nitro group, halogen atoms and cyano group, examples of which include 4-methylbenzyl, 2,4,6-trimethylbenzyl, 3,4,5-trimethyl-benzyl, 4-methoxybenzyl, 4-methoxyphenydiphenylmethyl, 2-nitrobenzyl, 4-nitro-benzyl, 4-chlorobenzyl, 4-bromobenzyl and 4-cyanobenzyl groups;
"alkenyloxycarbonyl groups", examples of which include vinyloxycarbonyl and allyloxycarbonyl groups; and

aralkyloxycarbonyl groups in which the aryl moiety thereof may optionally be substituted with one or two substituents selected from lower alkoxy groups and nitro group, examples of which include benzyloxycarbonyl, 4-methoxybenzyloxy-carbonyl, 3,4-dimethoxybenzyloxycarbonyl, 2-nitrobenzyloxycarbonyl and 4-nitro-benzyloxycarbonyl groups.

The protecting group which is capable of being removed by a metabolic process (e.g. hydrolysis) *in vivo* is one which, on administration to the body of a live mammal, is removable by a metabolic process (e.g. hydrolysis) to give the original compound or a salt thereof. Whether the compound under investigation is such a derivative or not can be determined by detecting the original compound or a pharmacologically acceptable salt thereof. The compound under investigation is intravenously administered to an experimental animal such as a rat or mouse and the body fluids of the animal are thereafter studied. If the original compound or a pharmacologically acceptable salt thereof can be detected in the body fluids, the compound under investigation is judged to be a pharmacologically acceptable ester or other derivative of the original compound.

Preferred examples of such a protecting group which is capable of being removed by a metabolic process (e.g. hydrolysis) *in vivo* include the following:

1-(acyloxy)lower alkyl groups, examples of which include

1-(lower aliphatic acyloxy)lower alkyl groups, examples of which include formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups,

1-(cycloalkylcarbonyloxy)lower alkyl groups, examples of which include cyclopentylcarbonyloxymethyl, cyclohexylcarbonyloxy-methyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentyl-carbonyloxybutyl and 1-cyclohexylcarbonyloxybutyl groups, and

1-(aromatic acyloxy)lower alkyl groups, examples of which include benzoyloxymethyl group;

carbonyloxyalkyl groups, examples of which include

(lower alkoxycarbonyloxy)alkyl groups, examples of which include methoxycarbonyloxymethyl, ethoxy-carbonyloxymethyl, propoxycarbonyloxymethyl, isopropoxycarbonyloxymethyl, butoxycarbonyloxymethyl, isobutoxycarbonyloxymethyl, pentyloxycarbonyloxy-methyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups, and

oxodioxolenylmethyl groups, examples of which include (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)-methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups;

phthalidyl groups, examples of which include phthalidyl, dimethylphthalidyl and dimethoxyphthalidyl groups;

lower aliphatic acyl groups as defined and exemplified above;

aromatic acyl groups as defined and exemplified above;

half-ester salt residues of succinic acid;

phosphate ester salt residues;

ester-forming residues of an amino acid;

carbamoyl groups;

carbamoyl groups which are substituted with one or two lower alkyl groups; and

1-(acyloxy)alkoxycarbonyl groups, examples of which include pivaloyloxymethyloxycarbonyl group.

Of the above protecting groups carbonyloxyalkyl groups are preferred.

In the case where the compound of formula (I) of the present invention has an amino group, an imino group and/or a sulfonamide group, the compound can be converted to a derivative by modifying said functional groups.

Examples of such derivatives include an amide derivative in which an aliphatic acyl group as defined and exemplified above or an aromatic acyl group as defined and exemplified above is bonded to a nitrogen atom of an amino group, imino group and/or sulfonamide group present in said compound of formula (I).

Where the compound of formula (I) of the present invention has a basic group, such as an amino group, the compound can be converted to a salt by reacting it with an acid, and in the case where the compound of formula (I) of the present invention has an acidic group, such as a sulfonamide group, the compound can be converted to a salt by reacting it with a base.

Preferred examples of the salts formed with a basic group present in the compound of formula (I) of the present invention include inorganic acid salts such as hydrohalogenated acid salts (e.g. hydrochloride, hydrobromide and hydroiodide), nitrate, perchlorate, sulfate and phosphate; organic acid salts such as lower alkanesulfonates (e.g. methanesulfonate, trifluoromethanesulfonate and ethanesulfonate), arylsulfonates (e.g. benzenesulfonate or p-toluenesulfonate), organic acid salts such as acetate, malate, fumarate, succinate, citrate, ascorbate, tartrate, oxalate and maleate; and amino acid salts such as glycine salt, lysine salt, arginine salt, ornithine salt, glutamate and aspartate.

Preferred examples of the salts formed with an acidic group present in the compound of formula (I) of the present invention include metal salts such as alkali metal salts (e.g. sodium salt, potassium salt and lithium salt), alkali earth metal salts (e.g. calcium salt and magnesium salt), aluminum salt and iron salt; amine salts such as inorganic amine salts (e.g. ammonium salt) and organic amine salts (e.g. t-octylamine salt, dibenzylamine salt, morpholine salt, glucosamine salt, phenylglycinealkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt and tris(hydroxymethyl)aminomethane salt; and amino acid salts such as glycine salt, lysine salt, arginine salt, ornithine salt, glutamate and aspartate.

The compound of formula (I) of the present invention can sometimes take up water upon exposure to the atmosphere or when recrystallized to absorb water or to form a hydrate and such hydrates are also included within the scope of the present invention.

The compounds of formula (I) of the present invention can sometimes exist in the form of geometrical isomers (cis and trans isomers) and, where said compounds contain one or more asymmetric centres, optical isomers. For the compounds of the present invention, each of said isomers and mixtures of said isomers are depicted by a single formula, i.e. the formula (I). Accordingly, the present invention covers both the individual isomers and mixtures thereof in any proportion, including racemic mixtures.

Specific examples of compounds of formula (I) of the present invention include the following compounds described in Table 1 and Table 2.

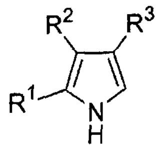

(I-1)

Table 1

| Compound No. | $R^1$ | $R^2$ | $R^3$ |
|---|---|---|---|
| 1-1 | Ph | 4-Pyr | $H_2N-CH_2$ |
| 1-2 | Ph | 4-Pyr | $H_2N-(CH_2)_2$ |
| 1-3 | Ph | 4-Pyr | $H_2N-(CH_2)_3$ |
| 1-4 | Ph | 4-Pyr | $H_2N-(CH_2)_4$ |
| 1-5 | Ph | 4-Pyr | $MeNH-CH_2$ |
| 1-6 | Ph | 4-Pyr | $MeNH-(CH_2)_2$ |
| 1-7 | Ph | 4-Pyr | $MeNH-(CH_2)_3$ |
| 1-8 | Ph | 4-Pyr | $MeNH-(CH_2)_4$ |
| 1-9 | Ph | 4-Pyr | $EtNH-CH_2$ |
| 1-10 | Ph | 4-Pyr | $EtNH-(CH_2)_2$ |
| 1-11 | Ph | 4-Pyr | $EtNH-(CH_2)_3$ |
| 1-12 | Ph | 4-Pyr | $EtNH-(CH_2)_4$ |
| 1-13 | Ph | 4-Pyr | $Me_2N-CH_2$ |
| 1-14 | Ph | 4-Pyr | $Me_2N-(CH_2)_2$ |
| 1-15 | Ph | 4-Pyr | $Me_2N-(CH_2)_3$ |
| 1-16 | Ph | 4-Pyr | $Me_2N-(CH_2)_4$ |
| 1-17 | Ph | 4-Pyr | $1-Azt-CH_2$ |
| 1-18 | Ph | 4-Pyr | $1-Azt-(CH_2)_2$ |
| 1-19 | Ph | 4-Pyr | $1-Azt-(CH_2)_3$ |
| 1-20 | Ph | 4-Pyr | $1-Azt-(CH_2)_4$ |
| 1-21 | Ph | 4-Pyr | $1-Pyrd-CH_2$ |
| 1-22 | Ph | 4-Pyr | $1-Pyrd-(CH_2)_2$ |
| 1-23 | Ph | 4-Pyr | $1-Pyrd-(CH_2)_3$ |
| 1-24 | Ph | 4-Pyr | $1-Pyrd-(CH_2)_4$ |
| 1-25 | Ph | 4-Pyr | $1-Pip-CH_2$ |
| 1-26 | Ph | 4-Pyr | $1-Pip-(CH_2)_2$ |
| 1-27 | Ph | 4-Pyr | $1-Pip-(CH_2)_3$ |
| 1-28 | Ph | 4-Pyr | $1-Pip-(CH_2)_4$ |
| 1-29 | Ph | 4-Pyr | $1-Mor-CH_2$ |
| 1-30 | Ph | 4-Pyr | $1-Mor-(CH_2)_2$ |

Table 1 (continued)

| Compound No. | $R^1$ | $R^2$ | $R^3$ |
|---|---|---|---|
| 1-31 | Ph | 4-Pyr | 1-Mor-$(CH_2)_3$ |
| 1-32 | Ph | 4-Pyr | 1-Mor-$(CH_2)_4$ |
| 1-33 | Ph | 4-Pyr | 1-Tmor-$CH_2$ |
| 1-34 | Ph | 4-Pyr | 1-Tmor-$(CH_2)_2$ |
| 1-35 | Ph | 4-Pyr | 1-Tmor-$(CH_2)_3$ |
| 1-36 | Ph | 4-Pyr | 1-Tmor-$(CH_2)_4$ |
| 1-37 | Ph | 4-Pyr | 1-Piz-$CH_2$ |
| 1-38 | Ph | 4-Pyr | 1-Piz-$(CH_2)_2$ |
| 1-39 | Ph | 4-Pyr | 1-Piz-$(CH_2)_3$ |
| 1-40 | Ph | 4-Pyr | 9-Me-1-Piz-$(CH_2)_3$ |
| 1-41 | Ph | 4-Pyr | 1-Piz-$(CH_2)_4$ |
| 1-42 | Ph | 4-Pyr | 3-Azt |
| 1-43 | Ph | 4-Pyr | 1-Me-3-Azt |
| 1-44 | Ph | 4-Pyr | 1-Bn-3-Azt |
| 1-45 | Ph | 4-Pyr | 3-Pyrd |
| 1-46 | Ph | 4-Pyr | 1-Me-3-Pyrd |
| 1-47 | Ph | 4-Pyr | 3-Pip |
| 1-48 | Ph | 4-Pyr | 4-Pip |
| 1-49 | Ph | 4-Pyr | 4-(3,4-deH-Pip) |
| 1-50 | Ph | 4-Pyr | 1-Me-4-Pip |
| 1-51 | Ph | 4-Pyr | 1-Me-4-(3,4-deH-Pip) |
| 1-52 | Ph | 4-Pyr | 1-Et-4-Pip |
| 1-53 | Ph | 4-Pyr | 1-Bn-4-Pip |
| 1-54 | Ph | 4-Pyr | 3-Hpip |
| 1-55 | Ph | 4-Pyr | 1-Me-3-Hpip |
| 1-56 | Ph | 4-Pyr | 4-Hpip |
| 1-57 | Ph | 4-Pyr | 1-Me-4-Hpip |
| 1-58 | Ph | 4-Pyr | 2-Mor |
| 1-59 | Ph | 4-Pyr | 1-Me-2-Mor |
| 1-60 | Ph | 4-Pyr | 2-Tmor |
| 1-61 | Ph | 4-Pyr | 1-Me-2-Tmor |
| 1-62 | Ph | 4-Pyr | 1-Piz |
| 1-63 | Ph | 4-Pyr | 4-Me-1-Piz |
| 1-64 | Ph | 4-Pyr | 2-Piz |
| 1-65 | Ph | 4-Pyr | 4-Pyr |
| 1-66 | Ph | 4-Pyr | 3-Pyr |
| 1-67 | Ph | 4-Pyr | 2-Pyr |
| 1-68 | Ph | 4-Pyr | 4-Pym |

Table 1 (continued)

| Compound No. | $R^1$ | $R^2$ | $R^3$ |
|---|---|---|---|
| 1-69 | Ph | 4-Pyr | 5-Pym |
| 1-70 | Ph | 4-Pyr | 2-Pym |
| 1-71 | Ph | 4-Pyr | 3-Azt-$CH_2$ |
| 1-72 | Ph | 4-Pyr | 1-Me-3-Azt-$CH_2$ |
| 1-73 | Ph | 4-Pyr | 3-pyrd-$CH_2$ |
| 1-74 | Ph | 4-Pyr | 1-Me-3-Pyrd-$CH_2$ |
| 1-75 | Ph | 4-Pyr | 4-Pip-$CH_2$ |
| 1-76 | Ph | 4-Pyr | 1-Me-4-Pip-$CH_2$ |
| 1-77 | Ph | 4-Pyr | 3-Hpip-$CH_2$ |
| 1-78 | Ph | 4-Pyr | 1-Me-3-Hpip-$CH_2$ |
| 1-79 | Ph | 4-Pyr | 4-Hpip-$CH_2$ |
| 1-80 | Ph | 4-Pyr | 1-Me-4-Hpip-$CH_2$ |
| 1-81 | Ph | 4-Pyr | 2-Mor-$CH_2$ |
| 1-82 | Ph | 4-Pyr | 1-Me-2-Mor-$CH_2$ |
| 1-83 | Ph | 4-Pyr | 2-Tmor-$CH_2$ |
| 1-84 | Ph | 4-Pyr | 1-Me-2-Tmor-$CH_2$ |
| 1-85 | Ph | 4-Pyr | 1-Piz-$CH_2$ |
| 1-86 | Ph | 4-Pyr | 4-Me-1-Piz-$CH_2$ |
| 1-87 | Ph | 4-Pyr | 2-Piz-$CH_2$ |
| 1-88 | Ph | 4-Pyr | 4-Pyr-$CH_2$ |
| 1-89 | Ph | 4-Pyr | 3-Pyr-$CH_2$ |
| 1-90 | Ph | 4-Pyr | 2-Pyr-$CH_2$ |
| 1-91 | Ph | 4-Pyr | 4-Pym-$CH_2$ |
| 1-92 | Ph | 4-Pyr | 5-Pym-$CH_2$ |
| 1-93 | Ph | 4-Pyr | 2-Pym-$CH_2$ |
| 1-94 | Ph | 4-Pyr | $H_2N-CH_2CH=CH$ |
| 1-95 | 4-F-Ph | 4-Pyr | $H_2N-CH_2$ |
| 1-96 | 4-F-Ph | 4-Pyr | $H_2N-(CH_2)_2$ |
| 1-97 | 4-F-Ph | 4-Pyr | $H_2N-(CH_2)_3$ |
| 1-98 | 4-F-Ph | 4-Pyr | $H_2N-(CH_2)_4$ |
| 1-99 | 4-F-Ph | 4-Pyr | MeNH-$CH_2$ |
| 1-100 | 4-F-Ph | 4-Pyr | MeNH-$(CH_2)_2$ |
| 1-101 | 4-F-Ph | 4-Pyr | MeNH-$(CH_2)_3$ |
| 1-102 | 4-F-Ph | 4-Pyr | MeNH-$(CH_2)_4$ |
| 1-103 | 4-F-Ph | 4-Pyr | EtNH-$CH_2$ |
| 1-104 | 4-F-Ph | 4-Pyr | EtNH-$(CH_2)_2$ |
| 1-105 | 4-F-Ph | 4-Pyr | EtNH-$(CH_2)_3$ |
| 1-106 | 4-F-Ph | 4-Pyr | EtNH-$(CH_2)_4$ |

Table 1 (continued)

| Compound No. | $R^1$ | $R^2$ | $R^3$ |
|---|---|---|---|
| 1-107 | 4-F-Ph | 4-Pyr | $Me_2N-CH_2$ |
| 1-108 | 4-F-Ph | 4-Pyr | $Me_2N-(CH_2)_2$ |
| 1-109 | 4-F-Ph | 4-Pyr | $Me_2N-(CH_2)_3$ |
| 1-110 | 4-F-Ph | 4-Pyr | $Me_2N-(CH_2)_4$ |
| 1-111 | 4-F-Ph | 4-Pyr | 1-Azt-$CH_2$ |
| 1-112 | 4-F-Ph | 4-Pyr | 1-Azt-$(CH_2)_2$ |
| 1-113 | 4-F-Ph | 4-Pyr | 1-Azt-$(CH_2)_3$ |
| 1-114 | 4-F-Ph | 4-Pyr | 1-Azt-$(CH_2)_4$ |
| 1-115 | 4-F-Ph | 4-Pyr | 1-pyrd-$CH_2$ |
| 1-116 | 4-F-Ph | 4-Pyr | 1-Pyrd-$(CH_2)_2$ |
| 1-117 | 4-F-Ph | 4-Pyr | 1-Pyrd-$(CH_2)_3$ |
| 1-118 | 4-F-Ph | 4-Pyr | 1-Pyrd-$(CH_2)_4$ |
| 1-119 | 4-F-Ph | 4-Pyr | 1-Pip-$CH_2$ |
| 1-120 | 4-F-Ph | 4-Pyr | 1-Pip-$(CH_2)_2$ |
| 1-121 | 4-F-Ph | 4-Pyr | 1-Pip-$(CH_2)_3$ |
| 1-122 | 4-F-Ph | 4-Pyr | 1-Pip-$(CH_2)_4$ |
| 1-123 | 4-F-Ph | 4-Pyr | 1-Mor-$CH_2$ |
| 1-124 | 4-F-Ph | 4-Pyr | 1-Mor-$(CH_2)_2$ |
| 1-125 | 4-F-Ph | 4-Pyr | 1-Mor-$(CH_2)_3$ |
| 1-126 | 4-F-Ph | 4-Pyr | 1-Mor-$(CH_2)_4$ |
| 1-127 | 4-F-Ph | 4-Pyr | 1-Tmor-$CH_2$ |
| 1-128 | 4-F-Ph | 4-Pyr | 1-Tmor-$(CH_2)_2$ |
| 1-129 | 4-F-Ph | 4-Pyr | 1-Tmor-$(CH_2)_3$ |
| 1-130 | 4-F-Ph | 4-Pyr | 1-Tmor-$(CH_2)_4$ |
| 1-131 | 4-F-Ph | 4-Pyr | 1-Piz-$CH_2$ |
| 1-132 | 4-F-Ph | 4-Pyr | 1-Piz-$(CH_2)_2$ |
| 1-133 | 4-F-Ph | 4-Pyr | 1-Piz-$(CH_2)_3$ |
| 1-134 | 4-F-Ph | 4-Pyr | 4-Me-1-Piz-$(CH_2)_3$ |
| 1-135 | 4-F-Ph | 4-Pyr | 4-Bn-1-Piz-$(CH_2)_3$ |
| 1-136 | 4-F-Ph | 4-Pyr | 3-Azt |
| 1-137 | 4-F-Ph | 4-Pyr | 1-Me-3-Azt |
| 1-138 | 4-F-Ph | 4-Pyr | 1-Bn-3-Azt |
| 1-139 | 4-F-Ph | 4-Pyr | 3-Pyrd |
| 1-140 | 4-F-Ph | 4-Pyr | 1-Me-3-Pyrd |
| 1-141 | 4-F-Ph | 4-Pyr | 3-Pip |
| 1-142 | 4-F-Ph | 4-Pyr | 4-Pip |
| 1-143 | 4-F-Ph | 4-Pyr | 4-(3,4-deH-Pip) |
| 1-144 | 4-F-Ph | 4-Pyr | 1-Me-4-Pip |

Table 1 (continued)

| Compound No. | $R^1$ | $R^2$ | $R^3$ |
|---|---|---|---|
| 1-145 | 4-F-Ph | 4-Pyr | 1-Me-4-(3,4-deH-Pip) |
| 1-146 | 4-F-Ph | 4-Pyr | 1-Et-4-Pip |
| 1-147 | 4-F-Ph | 4-Pyr | 1-Bn-4-Pip |
| 1-148 | 4-F-Ph | 4-Pyr | 3-Hpip |
| 1-149 | 4-F-Ph | 4-Pyr | 1-Me-3-Hpip |
| 1-150 | 4-F-Ph | 4-Pyr | 4-Hpip |
| 1-151 | 4-F-Ph | 4-Pyr | 1-Me-4-Hpip |
| 1-152 | 4-F-Ph | 4-Pyr | 2-Mor |
| 1-153 | 4-F-Ph | 4-Pyr | 1-Me-2-Mor |
| 1-154 | 4-F-Ph | 4-Pyr | 2-Tmor |
| 1-155 | 4-F-Ph | 4-Pyr | 1-Me-2-Tmor |
| 1-156 | 4-F-Ph | 4-Pyr | 1-Piz |
| 1-157 | 4-F-Ph | 4-Pyr | 4-Me-1-Piz |
| 1-158 | 4-F-Ph | 4-Pyr | 2-Piz |
| 1-159 | 4-F-Ph | 4-Pyr | 4-Pyr |
| 1-160 | 4-F-Ph | 4-Pyr | 3-Pyr |
| 1-161 | 4-F-Ph | 4-Pyr | 2-Pyr |
| 1-162 | 4-F-Ph | 4-Pyr | 4-Pym |
| 1-163 | 4-F-Ph | 4-Pyr | 5-Pym |
| 1-164 | 4-F-Ph | 4-Pyr | 2-Pym |
| 1-165 | 4-F-Ph | 4-Pyr | 3-Azt-$CH_2$ |
| 1-166 | 4-F-Ph | 4-Pyr | 1-Me-3-Azt-$CH_2$ |
| 1-167 | 4-F-Ph | 4-Pyr | 3-Pyrd-$CH_2$ |
| 1-168 | 4-F-Ph | 4-Pyr | 1-Me-3-Pyrd-$CH_2$ |
| 1-169 | 4-F-Ph | 4-Pyr | 4-Pip-$CH_2$ |
| 1-170 | 4-F-Ph | 4-Pyr | 1-Me-4-Pip-$CH_2$ |
| 1-171 | 4-F-Ph | 4-Pyr | 3-Hpip-$CH_2$ |
| 1-172 | 4-F-Ph | 4-Pyr | 1-Me-3-Hpip-$CH_2$ |
| 1-173 | 4-F-Ph | 4-Pyr | 4-Hpip-$CH_2$ |
| 1-174 | 4-F-Ph | 4-Pyr | 1-Me-4-Hpip-$CH_2$ |
| 1-175 | 4-F-Ph | 4-Pyr | 2-Mor-$CH_2$ |
| 1-176 | 4-F-Ph | 4-Pyr | 1-Me-2-Mor-$CH_2$ |
| 1-177 | 4-F-Ph | 4-Pyr | 2-Tmor-$CH_2$ |
| 1-178 | 4-F-Ph | 4-Pyr | 1-Me-2-Tmor-$CH_2$ |
| 1-179 | 4-F-Ph | 4-Pyr | 1-Piz-$CH_2$ |
| 1-180 | 4-F-Ph | 4-Pyr | 4-Me-1-Piz-$CH_2$ |
| 1-181 | 4-F-Ph | 4-Pyr | 2-Piz-$CH_2$ |
| 1-182 | 4-F-Ph | 4-Pyr | 4-Pyr-$CH_2$ |

Table 1 (continued)

| Compound No. | $R^1$ | $R^2$ | $R^3$ |
|---|---|---|---|
| 1-183 | 4-F-Ph | 4-Pyr | 3-Pyr-$CH_2$ |
| 1-184 | 4-F-Ph | 4-Pyr | 2-Pyr-$CH_2$ |
| 1-185 | 4-F-Ph | 4-Pyr | 4-Pym-$CH_2$ |
| 1-186 | 4-F-Ph | 4-Pyr | 5-Pym-$CH_2$ |
| 1-187 | 4-F-Ph | 4-Pyr | 2-Pym-$CH_2$ |
| 1-188 | 3-F-Ph | 4-Pyr | $H_2N$-$CH_2$ |
| 1-189 | 3-F-Ph | 4-Pyr | $H_2N$-$(CH_2)_2$ |
| 1-190 | 3-F-Ph | 4-Pyr | $H_2N$-$(CH_2)_3$ |
| 1-191 | 3-F-Ph | 4-Pyr | $H_2N$-$(CH_2)_4$ |
| 1-192 | 3-F-Ph | 4-Pyr | MeNH-$CH_2$ |
| 1-193 | 3-F-Ph | 4-Pyr | MeNH-$(CH_2)_2$ |
| 1-194 | 3-F-Ph | 4-Pyr | MeNH-$(CH_2)_3$ |
| 1-195 | 3-F-Ph | 4-Pyr | MeNH-$(CH_2)_4$ |
| 1-196 | 3-F-Ph | 4-Pyr | EtNH-$CH_2$ |
| 1-197 | 3-F-Ph | 4-Pyr | EtNH-$(CH_2)_2$ |
| 1-198 | 3-F-Ph | 4-Pyr | EtNH-$(CH_2)_3$ |
| 1-199 | 3-F-Ph | 4-Pyr | EtNH-$(CH_2)_4$ |
| 1-200 | 3-F-Ph | 4-Pyr | $Me_2N$-$CH_2$ |
| 1-201 | 3-F-Ph | 4-Pyr | $Me_2N$-$(CH_2)_2$ |
| 1-202 | 3-F-Ph | 4-Pyr | $Me_2N$-$(CH_2)_3$ |
| 1-203 | 3-F-Ph | 4-Pyr | $Me_2N$-$(CH_2)_4$ |
| 1-204 | 3-F-Ph | 4-Pyr | 1-Azt-$CH_2$ |
| 1-205 | 3-F-Ph | 4-Pyr | 1-Azt-$(CH_2)_2$ |
| 1-206 | 3-F-Ph | 4-Pyr | 1-Azt-$(CH_2)_3$ |
| 1-207 | 3-F-Ph | 4-Pyr | 1-Azt-$(CH_2)_4$ |
| 1-208 | 3-F-Ph | 4-Pyr | 1-Pyrd-$CH_2$ |
| 1-209 | 3-F-Ph | 4-Pyr | 1-Pyrd-$(CH_2)_2$ |
| 1-210 | 3-F-Ph | 4-Pyr | 1-Pyrd-$(CH_2)_3$ |
| 1-211 | 3-F-Ph | 4-Pyr | 1-Pyrd-$(CH_2)_4$ |
| 1-212 | 3-F-Ph | 4-Pyr | 1-Pip-$CH_2$ |
| 1-213 | 3-F-Ph | 4-Pyr | 1-Pip-$(CH_2)_2$ |
| 1-214 | 3-F-Ph | 4-Pyr | 1-Pip-$(CH_2)_3$ |
| 1-215 | 3-F-Ph | 4-Pyr | 1-Pip-$(CH_2)_4$ |
| 1-216 | 3-F-Ph | 4-Pyr | 1-Mor-$CH_2$ |
| 1-217 | 3-F-Ph | 4-Pyr | 1-Mor-$(CH_2)_2$ |
| 1-218 | 3-F-Ph | 4-Pyr | 1-Mor-$(CH_2)_3$ |
| 1-219 | 3-F-Ph | 4-Pyr | 1-Mor-$(CH_2)_4$ |
| 1-220 | 3-F-Ph | 4-Pyr | 1-Tmor-$CH_2$ |

Table 1 (continued)

| Compound No. | $R^1$ | $R^2$ | $R^3$ |
|---|---|---|---|
| 1-221 | 3-F-Ph | 4-Pyr | 1-Tmor-$(CH_2)_2$ |
| 1-222 | 3-F-Ph | 4-Pyr | 1-Tmor-$(CH_2)_3$ |
| 1-223 | 3-F-Ph | 4-Pyr | 1-Tmor-$(CH_2)_4$ |
| 1-224 | 3-F-Ph | 4-Pyr | 1-Piz-$CH_2$ |
| 1-225 | 3-F-Ph | 4-Pyr | 1-Piz-$(CH_2)_2$ |
| 1-226 | 3-F-Ph | 4-Pyr | 1-Piz-$(CH_2)_3$ |
| 1-227 | 3-F-Ph | 4-Pyr | 4-Me-1-Piz-$(CH_2)_3$ |
| 1-228 | 3-F-Ph | 4-Pyr | 1-Piz-$(CH_2)_4$ |
| 1-229 | 3-F-Ph | 4-Pyr | 3-Azt |
| 1-230 | 3-F-Ph | 4-Pyr | 1-Me-3-Azt |
| 1-231 | 3-F-Ph | 4-Pyr | 1-Bn-3-Azt |
| 1-232 | 3-F-Ph | 4-Pyr | 3-Pyrd |
| 1-233 | 3-F-Ph | 4-Pyr | 1-Me-3-Pyrd |
| 1-234 | 3-F-Ph | 4-Pyr | 3-Pip |
| 1-235 | 3-F-Ph | 4-Pyr | 4-Pip |
| 1-236 | 3-F-Ph | 4-Pyr | 4-(3,4-deH-Pip) |
| 1-237 | 3-F-Ph | 4-Pyr | 1-Me-4-Pip |
| 1-238 | 3-F-Ph | 4-Pyr | 1-Me-4-(3,4-deH-Pip) |
| 1-239 | 3-F-Ph | 4-Pyr | 1-Et-4-Pip |
| 1-240 | 3-F-Ph | 4-Pyr | 1-Bn-4-Pip |
| 1-241 | 3-F-Ph | 4-Pyr | 3-Hpip |
| 1-242 | 3-F-Ph | 4-Pyr | 1-Me-3-Hpip |
| 1-243 | 3-F-Ph | 4-Pyr | 4-Hpip |
| 1-244 | 3-F-Ph | 4-Pyr | 1-Me-4-Hpip |
| 1-245 | 3-F-Ph | 4-Pyr | 2-Mor |
| 1-246 | 3-F-Ph | 4-Pyr | 1-Me-2-Mor |
| 1-247 | 3-F-Ph | 4-Pyr | 2-Tmor |
| 1-248 | 3-F-Ph | 4-Pyr | 1-Me-2-Tmor |
| 1-249 | 3-F-Ph | 4-Pyr | 1-Piz |
| 1-250 | 3-F-Ph | 4-Pyr | 4-Me-1-Piz |
| 1-251 | 3-F-Ph | 4-Pyr | 2-Piz |
| 1-252 | 3-F-Ph | 4-Pyr | 4-Pyr |
| 1-253 | 3-F-Ph | 4-Pyr | 3-Pyr |
| 1-254 | 3-F-Ph | 4-Pyr | 2-Pyr |
| 1-255 | 3-F-Ph | 4-Pyr | 4-Pym |
| 1-256 | 3-F-Ph | 4-Pyr | 5-Pym |
| 1-257 | 3-F-Ph | 4-Pyr | 2-Pym |
| 1-258 | 3-F-Ph | 4-Pyr | 3-Azt-$CH_2$ |

Table 1 (continued)

| Compound No. | $R^1$ | $R^2$ | $R^3$ |
|---|---|---|---|
| 1-259 | 3-F-Ph | 4-Pyr | 1-Me-3-Azt-$CH_2$ |
| 1-260 | 3-F-Ph | 4-Pyr | 3-Pyrd-$CH_2$ |
| 1-261 | 3-F-Ph | 4-Pyr | 1-Me-3-Pyrd-$CH_2$ |
| 1-262 | 3-F-Ph | 4-Pyr | 4-Pip-$CH_2$ |
| 1-263 | 3-F-Ph | 4-Pyr | 1-Me-4-Pip-$CH_2$ |
| 1-264 | 3-F-Ph | 4-Pyr | 3-Hpip-$CH_2$ |
| 1-265 | 3-F-Ph | 4-Pyr | 1-Me-3-Hpip-$CH_2$ |
| 1-266 | 3-F-Ph | 4-Pyr | 4-Hpip-$CH_2$ |
| 1-267 | 3-F-Ph | 4-Pyr | 1-Me-4-Hpip-$CH_2$ |
| 1-268 | 3-F-Ph | 4-Pyr | 2-Mor-$CH_2$ |
| 1-269 | 3-F-Ph | 4-Pyr | 1-Me-2-Mor-$CH_2$ |
| 1-270 | 3-F-Ph | 4-Pyr | 2-Tmor-$CH_2$ |
| 1-271 | 3-F-Ph | 4-Pyr | 1-Me-2-Tmor-$CH_2$ |
| 1-272 | 3-F-Ph | 4-Pyr | 1-Piz-$CH_2$ |
| 1-273 | 3-F-Ph | 4-Pyr | 4-Me-1-Piz-$CH_2$ |
| 1-274 | 3-F-Ph | 4-Pyr | 2-Piz-$CH_2$ |
| 1-275 | 3-F-Ph | 4-Pyr | 4-Pyr-$CH_2$ |
| 1-276 | 3-F-Ph | 4-Pyr | 3-Pyr-$CH_2$ |
| 1-277 | 3-F-Ph | 4-Pyr | 2-Pyr-$CH_2$ |
| 1-278 | 3-F-Ph | 4-Pyr | 4-Pym-$CH_2$ |
| 1-279 | 3-F-Ph | 4-Pyr | 5-Pym-$CH_2$ |
| 1-280 | 3-F-Ph | 4-Pyr | 2-Pym-$CH_2$ |
| 1-281 | 3,4-diF-Ph | 4-Pyr | $H_2N$-$CH_2$ |
| 1-282 | 3,4-diF-Ph | 4-Pyr | $H_2N$-$(CH_2)_2$ |
| 1-283 | 3,4-diF-Ph | 4-Pyr | $H_2N$-$(CH_2)_3$ |
| 1-284 | 3,4-diF-Ph | 4-Pyr | $H_2N$-$(CH_2)_4$ |
| 1-285 | 3,4-diF-Ph | 4-Pyr | MeNH-$CH_2$ |
| 1-286 | 3,4-diF-Ph | 4-Pyr | MeNH-$(CH_2)_2$ |
| 1-287 | 3,4-diF-Ph | 4-Pyr | MeNH-$(CH_2)_3$ |
| 1-288 | 3,4-diF-Ph | 4-Pyr | MeNH-$(CH_2)_4$ |
| 1-289 | 3,4-diF-Ph | 4-Pyr | EtNH-$CH_2$ |
| 1-290 | 3,4-diF-Ph | 4-Pyr | EtNH-$(CH_2)_2$ |
| 1-291 | 3,4-diF-Ph | 4-Pyr | EtNH-$(CH_2)_3$ |
| 1-292 | 3,4-diF-Ph | 4-Pyr | EtNH-$(CH_2)_4$ |
| 1-293 | 3,4-diF-Ph | 4-Pyr | $Me_2N$-$CH_2$ |
| 1-294 | 3,4-diF-Ph | 4-Pyr | $Me_2N$-$(CH_2)_2$ |
| 1-295 | 3,4-diF-Ph | 4-Pyr | $Me_2N$-$(CH_2)_3$ |
| 1-296 | 3,4-diF-Ph | 4-Pyr | $Me_2N$-$(CH_2)_4$ |

Table 1 (continued)

| Compound No. | $R^1$ | $R^2$ | $R^3$ |
|---|---|---|---|
| 1-297 | 3,4-diF-Ph | 4-Pyr | 1-Azt-$CH_2$ |
| 1-298 | 3,4-diF-Ph | 4-Pyr | 1-Azt-$(CH_2)_2$ |
| 1-299 | 3,4-diF-Ph | 4-Pyr | 1-Azt-$(CH_2)_3$ |
| 1-300 | 3,4-diF-Ph | 4-Pyr | 1-Azt-$(CH_2)_4$ |
| 1-301 | 3,4-diF-Ph | 4-Pyr | 1-Pyrd-$CH_2$ |
| 1-302 | 3,4-diF-Ph | 4-Pyr | 1-Pyrd-$(CH_2)_2$ |
| 1-303 | 3,4-diF-Ph | 4-Pyr | 1-Pyrd-$(CH_2)_3$ |
| 1-304 | 3,4-diF-Ph | 4-Pyr | 1-Pyrd-$(CH_2)_4$ |
| 1-305 | 3,4-diF-Ph | 4-Pyr | 1-Pip-$CH_2$ |
| 1-306 | 3,4-diF-Ph | 4-Pyr | 1-Pip-$(CH_2)_2$ |
| 1-307 | 3,4-diF-Ph | 4-Pyr | 1-Pip-$(CH_2)_3$ |
| 1-308 | 3,4-diF-Ph | 4-Pyr | 1-Pip-$(CH_2)_4$ |
| 1-309 | 3,4-diF-Ph | 4-Pyr | 1-Mor-$CH_2$ |
| 1-310 | 3,4-diF-Ph | 4-Pyr | 1-Mor-$(CH_2)_2$ |
| 1-311 | 3,4-diF-Ph | 4-Pyr | 1-Mor-$(CH_2)_3$ |
| 1-312 | 3,4-diF-Ph | 4-Pyr | 1-Mor-$(CH_2)_4$ |
| 1-313 | 3,4-diF-Ph | 4-Pyr | 1-Tmor-$CH_2$ |
| 1-314 | 3,4-diF-Ph | 4-Pyr | 1-Tmor-$(CH_2)_2$ |
| 1-315 | 3,4-diF-Ph | 4-Pyr | 1-Tmor-$(CH_2)_3$ |
| 1-316 | 3,4-diF-Ph | 4-Pyr | 1-Tmor-$(CH_2)_4$ |
| 1-317 | 3,4-diF-Ph | 4-Pyr | 1-Piz-$CH_2$ |
| 1-318 | 3,4-diF-Ph | 4-Pyr | 1-Piz-$(CH_2)_2$ |
| 1-319 | 3,4-diF-Ph | 4-Pyr | 1-Piz-$(CH_2)_3$ |
| 1-320 | 3,4-diF-Ph | 4-Pyr | 4-Me-1-Piz-$(CH_2)_3$ |
| 1-321 | 3,4-diF-Ph | 4-Pyr | 1-Piz-$(CH_2)_4$ |
| 1-322 | 3,4-diF-Ph | 4-Pyr | 3-Azt |
| 1-323 | 3,4-diF-Ph | 4-Pyr | 1-Me-3-Azt |
| 1-324 | 3,4-diF-Ph | 4-Pyr | 1-Bn-3-Azt |
| 1-325 | 3,4-diF-Ph | 4-Pyr | 3-Pyrd |
| 1-326 | 3,4-diF-Ph | 4-Pyr | 1-Me-3-Pyrd |
| 1-327 | 3,4-diF-Ph | 4-Pyr | 3-Pip |
| 1-328 | 3,4-diF-Ph | 4-Pyr | 4-Pip |
| 1-329 | 3,4-diF-Ph | 4-Pyr | 4-(3,4-deH-Pip) |
| 1-330 | 3,4-diF-Ph | 4-Pyr | 1-Me-4-Pip |
| 1-331 | 3,4-diF-Ph | 4-Pyr | 1-Me-4-(3,4-deH-Pip) |
| 1-332 | 3,4-diF-Ph | 4-Pyr | 1-Et-4-Pip |
| 1-333 | 3,4-diF-Ph | 4-Pyr | 1-Bn-4-Pip |
| 1-334 | 3,4-diF-Ph | 4-Pyr | 3-Hpip |

Table 1 (continued)

| Compound No. | $R^1$ | $R^2$ | $R^3$ |
|---|---|---|---|
| 1-335 | 3,4-diF-Ph | 4-Pyr | 1-Me-3-Hpip |
| 1-336 | 3,4-diF-Ph | 4-Pyr | 4-Hpip |
| 1-337 | 3,4-diF-Ph | 4-Pyr | 1-Me-4-Hpip |
| 1-338 | 3,4-diF-Ph | 4-Pyr | 2-Mor |
| 1-339 | 3,4-diF-Ph | 4-Pyr | 1-Me-2-Mor |
| 1-340 | 3,4-diF-Ph | 4-Pyr | 2-Tmor |
| 1-341 | 3,4-diF-Ph | 4-Pyr | 1-Me-2-Tmor |
| 1-342 | 3,4-diF-Ph | 4-Pyr | 1-Piz |
| 1-343 | 3,4-diF-Ph | 4-Pyr | 4-Me-1-Piz |
| 1-344 | 3,4-diF-Ph | 4-Pyr | 2-Piz |
| 1-345 | 3,4-diF-Ph | 4-Pyr | 4-Pyr |
| 1-346 | 3,4-diF-Ph | 4-Pyr | 3-Pyr |
| 1-347 | 3,4-diF-Ph | 4-Pyr | 2-Pyr |
| 1-348 | 3,4-diF-Ph | 4-Pyr | 4-Pym |
| 1-349 | 3,4-diF-Ph | 4-Pyr | 5-Pym |
| 1-350 | 3,4-diF-Ph | 4-Pyr | 2-Pym |
| 1-351 | 3,4-diF-Ph | 4-Pyr | 3-Azt-$CH_2$ |
| 1-352 | 3,4-diF-Ph | 4-Pyr | 1-Me-3-Azt-$CH_2$ |
| 1-353 | 3,4-diF-Ph | 4-Pyr | 3-Pyrd-$CH_2$ |
| 1-354 | 3,4-diF-Ph | 4-Pyr | 1-Me-3-Pyrd-$CH_2$ |
| 1-355 | 3,4-diF-Ph | 4-Pyr | 4-Pip-$CH_2$ |
| 1-356 | 3,4-diF-Ph | 4-Pyr | 1-Me-4-Pip-$CH_2$ |
| 1-357 | 3,4-diF-Ph | 4-Pyr | 3-Hpip-$CH_2$ |
| 1-358 | 3,4-diF-Ph | 4-Pyr | 1-Me-3-Hpip-$CH_2$ |
| 1-359 | 3,4-diF-Ph | 4-Pyr | 4-Hpip-$CH_2$ |
| 1-360 | 3,4-diF-Ph | 4-Pyr | 1-Me-4-Hpip-$CH_2$ |
| 1-361 | 3,4-diF-Ph | 4-Pyr | 2-Mor-$CH_2$ |
| 1-362 | 3,4-diF-Ph | 4-Pyr | 1-Me-2-Mor-$CH_2$ |
| 1-363 | 3,4-diF-Ph | 4-Pyr | 2-Tmor-$CH_2$ |
| 1-364 | 3,4-diF-Ph | 4-Pyr | 1-Me-2-Tmor-$CH_2$ |
| 1-365 | 3,4-diF-Ph | 4-Pyr | 1-Piz-$CH_2$ |
| 1-366 | 3,4-diF-Ph | 4-Pyr | 4-Me-1-Piz-$CH_2$ |
| 1-367 | 3,4-diF-Ph | 4-Pyr | 2-Piz-$CH_2$ |
| 1-368 | 3,4-diF-Ph | 4-Pyr | 4-Pyr-$CH_2$ |
| 1-369 | 3,4-diF-Ph | 4-Pyr | 3-Pyr-$CH_2$ |
| 1-370 | 3,4-diF-Ph | 4-Pyr | 2-Pyr-$CH_2$ |
| 1-371 | 3,4-diF-Ph | 4-Pyr | 4-Pym-$CH_2$ |
| 1-372 | 3,4-diF-Ph | 4-Pyr | 5-Pym-$CH_2$ |

Table 1 (continued)

| Compound No. | $R^1$ | $R^2$ | $R^3$ |
|---|---|---|---|
| 1-373 | 3,4-diF-Ph | 4-Pyr | 2-Pym-$CH_2$ |
| 1-374 | 3-Cl-Ph | 4-Pyr | $H_2N$-$CH_2$ |
| 1-375 | 3-Cl-Ph | 4-Pyr | $H_2N$-$(CH_2)_2$ |
| 1-376 | 3-Cl-Ph | 4-Pyr | $H_2N$-$(CH_2)_3$ |
| 1-377 | 3-Cl-Ph | 4-Pyr | $H_2N$-$(CH_2)_4$ |
| 1-378 | 3-Cl-Ph | 4-Pyr | MeNH-$CH_2$ |
| 1-379 | 3-Cl-Ph | 4-Pyr | MeNH-$(CH_2)_2$ |
| 1-380 | 3-Cl-Ph | 4-Pyr | MeNH-$(CH_2)_3$ |
| 1-381 | 3-Cl-Ph | 4-Pyr | MeNH-$(CH_2)_4$ |
| 1-382 | 3-Cl-Ph | 4-Pyr | EtNH-$CH_2$ |
| 1-383 | 3-Cl-Ph | 4-Pyr | EtNH-$(CH_2)_2$ |
| 1-384 | 3-Cl-Ph | 4-Pyr | EtNH-$(CH_2)_3$ |
| 1-385 | 3-Cl-Ph | 4-Pyr | EtNH-$(CH_2)_4$ |
| 1-386 | 3-Cl-Ph | 4-Pyr | $Me_2N$-$CH_2$ |
| 1-387 | 3-Cl-Ph | 4-Pyr | $Me_2N$-$(CH_2)_2$ |
| 1-388 | 3-Cl-Ph | 4-Pyr | $Me_2N$-$(CH_2)_3$ |
| 1-389 | 3-Cl-Ph | 4-Pyr | $Me_2N$-$(CH_2)_4$ |
| 1-390 | 3-Cl-Ph | 4-Pyr | 1-Azt-$CH_2$ |
| 1-391 | 3-Cl-Ph | 4-Pyr | 1-Azt-$(CH_2)_2$ |
| 1-392 | 3-Cl-Ph | 4-Pyr | 1-Azt-$(CH_2)_3$ |
| 1-393 | 3-Cl-Ph | 4-Pyr | 1-Azt-$(CH_2)_4$ |
| 1-394 | 3-Cl-Ph | 4-Pyr | 1-Pyrd-$CH_2$ |
| 1-395 | 3-Cl-Ph | 4-Pyr | 1-Pyrd-$(CH_2)_2$ |
| 1-396 | 3-Cl-Ph | 4-Pyr | 1-Pyrd-$(CH_2)_3$ |
| 1-397 | 3-Cl-Ph | 4-Pyr | 1-Pyrd-$(CH_2)_4$ |
| 1-398 | 3-Cl-Ph | 4-Pyr | 1-Pip-$CH_2$ |
| 1-399 | 3-Cl-Ph | 4-Pyr | 1-Pip-$(CH_2)_2$ |
| 1-400 | 3-Cl-Ph | 4-Pyr | 1-Pip-$(CH_2)_3$ |
| 1-401 | 3-Cl-Ph | 4-Pyr | 1-Pip-$(CH_2)_4$ |
| 1-402 | 3-Cl-Ph | 4-Pyr | 1-Mor-$CH_2$ |
| 1-403 | 3-Cl-Ph | 4-Pyr | 1-Mor-$(CH_2)_2$ |
| 1-404 | 3-Cl-Ph | 4-Pyr | 1-Mor-$(CH_2)_3$ |
| 1-405 | 3-Cl-Ph | 4-Pyr | 1-Mor-$(CH_2)_4$ |
| 1-406 | 3-Cl-Ph | 4-Pyr | 1-Tmor-$CH_2$ |
| 1-407 | 3-Cl-Ph | 4-Pyr | 1-Tmor-$(CH_2)_2$ |
| 1-408 | 3-Cl-Ph | 4-Pyr | 1-Tmor-$(CH_2)_3$ |
| 1-409 | 3-Cl-Ph | 4-Pyr | 1-Tmor-$(CH_2)_4$ |
| 1-410 | 3-Cl-Ph | 4-Pyr | 1-Piz-$CH_2$ |

Table 1 (continued)

| Compound No. | $R^1$ | $R^2$ | $R^3$ |
|---|---|---|---|
| 1-411 | 3-Cl-Ph | 4-Pyr | 1-Piz-$(CH_2)_2$ |
| 1-412 | 3-Cl-Ph | 4-Pyr | 1-Piz-$(CH_2)_3$ |
| 1-413 | 3-Cl-Ph | 4-Pyr | 4-Me-1-Piz-$(CH_2)_3$ |
| 1-414 | 3-Cl-Ph | 4-Pyr | 1-Piz-$(CH_2)_4$ |
| 1-415 | 3-Cl-Ph | 4-Pyr | 3-Azt |
| 1-416 | 3-Cl-Ph | 4-Pyr | 1-Me-3-Azt |
| 1-417 | 3-Cl-Ph | 4-Pyr | 1-Bn-3-Azt |
| 1-418 | 3-Cl-Ph | 4-Pyr | 3-Pyrd |
| 1-419 | 3-Cl-Ph | 4-Pyr | 1-Me-3-Pyrd |
| 1-420 | 3-Cl-Ph | 4-Pyr | 3-Pip |
| 1-421 | 3-Cl-Ph | 4-Pyr | 4-Pip |
| 1-422 | 3-Cl-Ph | 4-Pyr | 4-(3,4-deH-Pip) |
| 1-423 | 3-Cl-Ph | 4-Pyr | 1-Me-4-Pip |
| 1-424 | 3-Cl-Ph | 4-Pyr | 1-Me-4-(3,4-deH-Pip) |
| 1-425 | 3-Cl-Ph | 4-Pyr | 1-Et-4-Pip |
| 1-426 | 3-Cl-Ph | 4-Pyr | 1-Bn-4-Pip |
| 1-427 | 3-Cl-Ph | 4-Pyr | 3-Hpip |
| 1-428 | 3-Cl-Ph | 4-Pyr | 1-Me-3-Hpip |
| 1-429 | 3-Cl-Ph | 4-Pyr | 4-Hpip |
| 1-430 | 3-Cl-Ph | 4-Pyr | 1-Me-4-Hpip |
| 1-431 | 3-Cl-Ph | 4-Pyr | 2-Mor |
| 1-432 | 3-Cl-Ph | 4-Pyr | 1-Me-2-Mor |
| 1-433 | 3-Cl-Ph | 4-Pyr | 2-Tmor |
| 1-434 | 3-Cl-Ph | 4-Pyr | 1-Me-2-Tmor |
| 1-435 | 3-Cl-Ph | 4-Pyr | 1-Piz |
| 1-436 | 3-Cl-Ph | 4-Pyr | 4-Me-1-Piz |
| 1-437 | 3-Cl-Ph | 4-Pyr | 2-Piz |
| 1-438 | 3-Cl-Ph | 4-Pyr | 4-Pyr |
| 1-439 | 3-Cl-Ph | 4-Pyr | 3-Pyr |
| 1-440 | 3-Cl-Ph | 4-Pyr | 2-Pyr |
| 1-441 | 3-Cl-Ph | 4-Pyr | 4-Pym |
| 1-442 | 3-Cl-Ph | 4-Pyr | 5-Pym |
| 1-443 | 3-Cl-Ph | 4-Pyr | 2-Pym |
| 1-444 | 3-Cl-Ph | 4-Pyr | 3-Azt-$CH_2$ |
| 1-445 | 3-Cl-Ph | 4-Pyr | 1-Me-3-Azt-$CH_2$ |
| 1-446 | 3-Cl-Ph | 4-Pyr | 3-Pyrd-$CH_2$ |
| 1-447 | 3-Cl-Ph | 4-Pyr | 1-Me-3-Pyrd-$CH_2$ |
| 1-448 | 3-Cl-Ph | 4-Pyr | 4-Pip-$CH_2$ |

Table 1 (continued)

| Compound No. | $R^1$ | $R^2$ | $R^3$ |
|---|---|---|---|
| 1-449 | 3-Cl-Ph | 4-Pyr | 1-Me-4-Pip-$CH_2$ |
| 1-450 | 3-Cl-Ph | 4-Pyr | 3-Hpip-$CH_2$ |
| 1-451 | 3-Cl-Ph | 4-Pyr | 1-Me-3-Hpip-$CH_2$ |
| 1-452 | 3-Cl-Ph | 4-Pyr | 4-Hpip-$CH_2$ |
| 1-453 | 3-Cl-Ph | 4-Pyr | 1-Me-4-Hpip-$CH_2$ |
| 1-454 | 3-Cl-Ph | 4-Pyr | 2-Mor-$CH_2$ |
| 1-455 | 3-Cl-Ph | 4-Pyr | 1-Me-2-Mor-$CH_2$ |
| 1-456 | 3-Cl-Ph | 4-Pyr | 2-Tmor-$CH_2$ |
| 1-457 | 3-Cl-Ph | 4-Pyr | 1-Me-2-Tmor-$CH_2$ |
| 1-458 | 3-Cl-Ph | 4-Pyr | 1-Piz-$CH_2$ |
| 1-459 | 3-Cl-Ph | 4-Pyr | 4-Me-1-Piz-$CH_2$ |
| 1-460 | 3-Cl-Ph | 4-Pyr | 2-Piz-$CH_2$ |
| 1-461 | 3-Cl-Ph | 4-Pyr | 4-Pyr-$CH_2$ |
| 1-462 | 3-Cl-Ph | 4-Pyr | 3-Pyr-$CH_2$ |
| 1-463 | 3-Cl-Ph | 4-Pyr | 2-Pyr-$CH_2$ |
| 1-464 | 3-Cl-Ph | 4-Pyr | 4-Pym-$CH_2$ |
| 1-465 | 3-Cl-Ph | 4-Pyr | 5-Pym-$CH_2$ |
| 1-466 | 3-Cl-Ph | 4-Pyr | 2-Pym-$CH_2$ |
| 1-467 | Ph | 2-$NH_2$-4-Pym | $H_2N$-$CH_2$ |
| 1-468 | Ph | 2-$NH_2$-4-Pym | $H_2N$-$(CH_2)_2$ |
| 1-469 | Ph | 2-$NH_2$-4-Pym | $H_2N$-$(CH_2)_3$ |
| 1-470 | Ph | 2-$NH_2$-4-Pym | $H_2N$-$(CH_2)_4$ |
| 1-471 | Ph | 2-$NH_2$-4-Pym | MeNH-$CH_2$ |
| 1-472 | Ph | 2-$NH_2$-4-Pym | MeNH-$(CH_2)_2$ |
| 1-473 | Ph | 2-$NH_2$-4-Pym | MeNH-$(CH_2)_3$ |
| 1-474 | Ph | 2-$NH_2$-4-Pym | MeNH-$(CH_2)_4$ |
| 1-475 | Ph | 2-$NH_2$-4-Pym | EtNH-$CH_2$ |
| 1-476 | Ph | 2-$NH_2$-4-Pym | EtNH-$(CH_2)_2$ |
| 1-477 | Ph | 2-$NH_2$-4-Pym | EtNH-$(CH_2)_3$ |
| 1-478 | Ph | 2-$NH_2$-4-Pym | EtNH-$(CH_2)_4$ |
| 1-479 | Ph | 2-$NH_2$-4-Pym | $Me_2N$-$CH_2$ |
| 1-480 | Ph | 2-$NH_2$-4-Pym | $Me_2N$-$(CH_2)_2$ |
| 1-481 | Ph | 2-$NH_2$-4-Pym | $Me_2N$-$(CH_2)_3$ |
| 1-482 | Ph | 2-$NH_2$-4-Pym | $Me_2N$-$(CH_2)_4$ |
| 1-483 | Ph | 2-$NH_2$-4-Pym | 1-Azt-$CH_2$ |
| 1-484 | Ph | 2-$NH_2$-4-Pym | 1-Azt-$(CH_2)_2$ |
| 1-485 | Ph | 2-$NH_2$-4-Pym | 1-Azt-$(CH_2)_3$ |
| 1-486 | Ph | 2-$NH_2$-4-Pym | 1-Azt-$(CH_2)_4$ |

Table 1 (continued)

| Compound No. | $R^1$ | $R^2$ | $R^3$ |
|---|---|---|---|
| 1-487 | Ph | $2-NH_2-4-Pym$ | $1-Pyrd-CH_2$ |
| 1-488 | Ph | $2-NH_2-4-Pym$ | $1-Pyrd-(CH_2)_2$ |
| 1-489 | Ph | $2-NH_2-4-Pym$ | $1-Pyrd-(CH_2)_3$ |
| 1-490 | Ph | $2-NH_2-4-Pym$ | $1-Pyrd-(CH_2)_4$ |
| 1-491 | Ph | $2-NH_2-4-Pym$ | $1-Pip-CH_2$ |
| 1-492 | Ph | $2-NH_2-4-Pym$ | $1-Pip-(CH_2)_2$ |
| 1-493 | Ph | $2-NH_2-4-Pym$ | $1-Pip-(CH_2)_3$ |
| 1-494 | Ph | $2-NH_2-4-Pym$ | $1-Pip-(CH_2)_4$ |
| 1-495 | Ph | $2-NH_2-4-Pym$ | $1-Mor-CH_2$ |
| 1-496 | Ph | $2-NH_2-4-Pym$ | $1-Mor-(CH_2)_2$ |
| 1-497 | Ph | $2-NH_2-4-Pym$ | $1-Mor-(CH_2)_3$ |
| 1-498 | Ph | $2-NH_2-4-Pym$ | $1-Mor-(CH_2)_4$ |
| 1-499 | Ph | $2-NH_2-4-Pym$ | $1-Tmor-CH_2$ |
| 1-500 | Ph | $2-NH_2-4-Pym$ | $1-Tmor-(CH_2)_2$ |
| 1-501 | Ph | $2-NH_2-4-Pym$ | $1-Tmor-(CH_2)_3$ |
| 1-502 | Ph | $2-NH_2-4-Pym$ | $1-Tmor-(CH_2)_4$ |
| 1-503 | Ph | $2-NH_2-4-Pym$ | $1-Piz-CH_2$ |
| 1-504 | Ph | $2-NH_2-4-Pym$ | $1-Piz-(CH_2)_2$ |
| 1-505 | Ph | $2-NH_2-4-Pym$ | $1-Piz-(CH_2)_3$ |
| 1-506 | Ph | $2-NH_2-4-Pym$ | $4-Me-1-Piz-(CH_2)_3$ |
| 1-507 | Ph | $2-NH_2-4-Pym$ | $1-Piz-(CH_2)_4$ |
| 1-508 | Ph | $2-NH_2-4-Pym$ | 3-Azt |
| 1-509 | Ph | $2-NH_2-4-Pym$ | 1-Me-3-Azt |
| 1-510 | Ph | $2-NH_2-4-Pym$ | 1-Bn-3-Azt |
| 1-511 | Ph | $2-NH_2-4-Pym$ | 3-Pyrd |
| 1-512 | Ph | $2-NH_2-4-Pym$ | 1-Me-3-Pyrd |
| 1-513 | Ph | $2-NH_2-4-Pym$ | 3-Pip |
| 1-514 | Ph | $2-NH_2-4-Pym$ | 4-Pip |
| 1-515 | Ph | $2-NH_2-4-Pym$ | 4-(3,4-deH-Pip) |
| 1-516 | Ph | $2-NH_2-4-Pym$ | 1-Me-4-Pip |
| 1-517 | Ph | $2-NH_2-4-Pym$ | 1-Me-4-(3,4-deH-Pip) |
| 1-518 | Ph | $2-NH_2-4-Pym$ | 1-Et-4-Pip |
| 1-519 | Ph | $2-NH_2-4-Pym$ | 1-Bn-4-Pip |
| 1-520 | Ph | $2-NH_2-4-Pym$ | 3-Hpip |
| 1-521 | Ph | $2-NH_2-4-Pym$ | 1-Me-3-Hpip |
| 1-522 | Ph | $2-NH_2-4-Pym$ | 4-Hpip |
| 1-523 | Ph | $2-NH_2-4-Pym$ | 1-Me-4-Hpip |
| 1-524 | Ph | $2-NH_2-4-Pym$ | 2-Mor |

Table 1 (continued)

| Compound No. | $R^1$ | $R^2$ | $R^3$ |
|---|---|---|---|
| 1-525 | Ph | 2-$NH_2$-4-Pym | 1-Me-2-Mor |
| 1-526 | Ph | 2-$NH_2$-4-Pym | 2-Tmor |
| 1-527 | Ph | 2-$NH_2$-4-Pym | 1-Me-2-Tmor |
| 1-528 | Ph | 2-$NH_2$-4-Pym | 1-Piz |
| 1-529 | Ph | 2-$NH_2$-4-Pym | 4-Me-1-Piz |
| 1-530 | Ph | 2-$NH_2$-4-Pym | 2-Piz |
| 1-531 | Ph | 2-$NH_2$-4-Pym | 4-Pyr |
| 1-532 | Ph | 2-$NH_2$-4-Pym | 3-Pyr |
| 1-533 | Ph | 2-$NH_2$-4-Pym | 2-Pyr |
| 1-534 | Ph | 2-$NH_z$-4-Pym | 4-Pym |
| 1-535 | Ph | 2-$NH_2$-4-Pym | 5-Pym |
| 1-536 | Ph | 2-$NH_2$-4-Pym | 2-Pym |
| 1-537 | Ph | 2-$NH_2$-4-Pym | 3-Azt-$CH_2$ |
| 1-538 | Ph | 2-$NH_2$-4-Pym | 1-Me-3-Azt-$CH_2$ |
| 1-539 | Ph | 2-$NH_2$-4-Pym | 3-Pyrd-$CH_2$ |
| 1-540 | Ph | 2-$NH_2$-4-Pym | 1-Me-3-Pyrd-$CH_2$ |
| 1-541 | Ph | 2-$NH_2$-4-Pym | 4-Pip-$CH_2$ |
| 1-542 | Ph | 2-$NH_2$-4-Pym | 1-Me-4-Pip-$CH_2$ |
| 1-543 | Ph | 2-$NH_2$-4-Pym | 3-Hpip-$CH_2$ |
| 1-544 | Ph | 2-$NH_2$-4-Pym | 1-Me-3-Hpip-$CH_2$ |
| 1-545 | Ph | 2-$NH_2$-4-Pym | 4-Hpip-$CH_2$ |
| 1-546 | Ph | 2-$NH_2$-4-Pym | 1-Me-4-Hpip-$CH_2$ |
| 1-547 | Ph | 2-$NH_2$-4-Pym | 2-Mor-$CH_2$ |
| 1-548 | Ph | 2-$NH_2$-4-Pym | 1-Me-2-Mor-$CH_2$ |
| 1-549 | Ph | 2-$NH_2$-4-Pym | 2-Tmor-$CH_2$ |
| 1-550 | Ph | 2-$NH_2$-4-Pym | 1-Me-2-Tmor-$CH_2$ |
| 1-551 | Ph | 2-$NH_2$-4-Pym | 1-Piz-$CH_2$ |
| 1-552 | Ph | 2-$NH_2$-4-Pym | 4-Me-1-Piz-$CH_2$ |
| 1-553 | Ph | 2-$NH_2$-4-Pym | 2-Piz-$CH_2$ |
| 1-554 | Ph | 2-$NH_2$-4-Pym | 4-Pyr-$CH_2$ |
| 1-555 | Ph | 2-$NH_2$-4-Pym | 3-Pyr-$CH_2$ |
| 1-556 | Ph | 2-$NH_2$-4-Pym | 2-Pyr-$CH_2$ |
| 1-557 | Ph | 2-$NH_2$-4-Pym | 4-Pym-$CH_2$ |
| 1-558 | Ph | 2-$NH_2$-4-Pym | 5-Pym-$CH_2$ |
| 1-559 | Ph | 2-$NH_2$-4-Pym | 2-Pym-$CH_2$ |
| 1-560 | 4-F-Ph | 2-$NH_2$-4-Pym | $H_2N$-$CH_2$ |
| 1-561 | 4-F-Ph | 2-$NH_2$-4-Pym | $H_2N$-$(CH_2)_2$ |
| 1-562 | 4-F-Ph | 2-$NH_2$-4-Pym | $H_2N$-$(CH_2)_3$ |

Table 1 (continued)

| Compound No. | $R^1$ | $R^2$ | $R^3$ |
|---|---|---|---|
| 1-563 | 4-F-Ph | $2-NH_2$-4-Pym | $H_2N-(CH_2)_4$ |
| 1-564 | 4-F-Ph | $2-NH_2$-4-Pym | $MeNH-CH_2$ |
| 1-565 | 4-F-Ph | $2-NH_2$-4-Pym | $MeNH-(CH_2)_2$ |
| 1-566 | 4-F-Ph | $2-NH_2$-4-Pym | $MeNH-(CH_2)_3$ |
| 1-567 | 4-F-Ph | $2-NH_2$-4-Pym | $MeNH-(CH_2)_4$ |
| 1-568 | 4-F-Ph | $2-NH_2$-4-Pym | $EtNH-CH_2$ |
| 1-569 | 4-F-Ph | $2-NH_2$-4-Pym | $EtNH-(CH_2)_2$ |
| 1-570 | 4-F-Ph | $2-NH_2$-4-Pym | $EtNH-(CH_2)_3$ |
| 1-571 | 4-F-Ph | $2-NH_2$-4-Pym | $EtNH-(CH_2)_4$ |
| 1-572 | 4-F-Ph | $2-NH_2$-4-Pym | $Me_2N-CH_2$ |
| 1-573 | 4-F-Ph | $2-NH_2$-4-Pym | $Me_2N-(CH_2)_2$ |
| 1-574 | 4-F-Ph | $2-NH_2$-4-Pym | $Me_2N-(CH_2)_3$ |
| 1-575 | 4-F-Ph | $2-NH_2$-4-Pym | $Me_2N-(CH_2)_4$ |
| 1-576 | 4-F-Ph | $2-NH_2$-4-Pym | 1-Azt-$CH_2$ |
| 1-577 | 4-F-Ph | $2-NH_2$-4-Pym | 1-Azt-$(CH_2)_2$ |
| 1-578 | 4-F-Ph | $2-NH_2$-4-Pym | 1-Azt-$(CH_2)_3$ |
| 1-579 | 4-F-Ph | $2-NH_2$-4-Pym | 1-Azt-$(CH_2)_4$ |
| 1-580 | 4-F-Ph | $2-NH_2$-4-Pym | 1-Pyrd-$CH_2$ |
| 1-581 | 4-F-Ph | $2-NH_2$-4-Pym | 1-Pyrd-$(CH_2)_2$ |
| 1-582 | 4-F-Ph | $2-NH_2$-4-Pym | 1-Pyrd-$(CH_2)_3$ |
| 1-583 | 4-F-Ph | $2-NH_2$-4-Pym | 1-Pyrd-$(CH_2)_4$ |
| 1-584 | 4-F-Ph | $2-NH_2$-4-Pym | 1-Pip-$CH_2$ |
| 1-585 | 4-F-Ph | $2-NH_2$-4-Pym | 1-Pip-$(CH_2)_2$ |
| 1-586 | 4-F-Ph | $2-NH_2$-4-Pym | 1-Pip-$(CH_2)_3$ |
| 1-587 | 4-F-Ph | $2-NH_2$-4-Pym | 1-Pip-$(CH_2)_4$ |
| 1-588 | 4-F-Ph | $2-NH_2$-4-Pym | 1-Mor-$CH_2$ |
| 1-589 | 4-F-Ph | $2-NH_2$-4-Pym | 1-Mor-$(CH_2)_2$ |
| 1-590 | 4-F-Ph | $2-NH_2$-4-Pym | 1-Mor-$(CH_2)_3$ |
| 1-591 | 4-F-Ph | $2-NH_2$-4-Pym | 1-Mor-$(CH_2)_4$ |
| 1-592 | 4-F-Ph | $2-NH_2$-4-Pym | 1-Tmor-$CH_2$ |
| 1-593 | 4-F-Ph | $2-NH_2$-4-Pym | 1-Tmor-$(CH_2)_2$ |
| 1-594 | 4-F-Ph | $2-NH_2$-4-Pym | 1-Tmor-$(CH_2)_3$ |
| 1-595 | 4-F-Ph | $2-NH_2$-4-Pym | 1-Tmor-$(CH_2)_4$ |
| 1-596 | 4-F-Ph | $2-NH_2$-4-Pym | 1-Piz-$CH_2$ |
| 1-597 | 4-F-Ph | $2-NH_2$-4-Pym | 1-Piz-$(CH_2)_2$ |
| 1-598 | 4-F-Ph | $2-NH_2$-4-Pym | 1-Piz-$(CH_2)_3$ |
| 1-599 | 4-F-Ph | $2-NH_2$-4-Pym | 4-Me-1-Piz-$(CH_2)_3$ |
| 1-600 | 4-F-Ph | $2-NH_2$-4-Pym | 1-Piz-$(CH_2)_4$ |

Table 1 (continued)

| Compound No. | $R^1$ | $R^2$ | $R^3$ |
|---|---|---|---|
| 1-601 | 4-F-Ph | $2-NH_2-4-Pym$ | 3-Azt |
| 1-602 | 4-F-Ph | $2-NH_2-4-Pym$ | 1-Me-3-Azt |
| 1-603 | 4-F-Ph | $2-NH_2-4-Pym$ | 1-Bn-3-Azt |
| 1-604 | 4-F-Ph | $2-NH_2-4-Pym$ | 3-Pyrd |
| 1-605 | 4-F-Ph | $2-NH_2-4-Pym$ | 1-Me-3-Pyrd |
| 1-606 | 4-F-Ph | $2-NH_2-4-Pym$ | 3-Pip |
| 1-607 | 4-F-Ph. | $2-NH_2-4-Pym$ | 4-Pip |
| 1-608 | 4-F-Ph | $2-NH_2-4-Pym$ | 4-(3,4-deH-Pip) |
| 1-609 | 4-F-Ph | $2-NH_2-4-Pym$ | 1-Me-4-Pip |
| 1-610 | 4-F-Ph | $2-NH_2-4-Pym$ | 1-Me-4-(3,4-deH-Pip) |
| 1-611 | 4-F-Ph | $2-NH_2-4-Pym$ | 1-Et-4-Pip |
| 1-612 | 4-F-Ph | $2-NH_2-4-Pym$ | 1-Bn-4-Pip |
| 1-613 | 4-F-Ph | $2-NH_2-4-Pym$ | 3-Hpip |
| 1-614 | 4-F-Ph | $2-NH_2-4-Pym$ | 1-Me-3-Hpip |
| 1-615 | 4-F-Ph | $2-NH_2-4-Pym$ | 4-Hpip |
| 1-616 | 4-F-Ph | $2-NH_2-4-Pym$ | 1-Me-4-Hpip |
| 1-617 | 4-F-Ph | $2-NH_2-4-Pym$ | 2-Mor |
| 1-618 | 4-F-Ph | $2-NH_2-4-Pym$ | 1-Me-2-Mor |
| 1-619 | 4-F-Ph | $2-NH_2-4-Pym$ | 2-Tmor |
| 1-620 | 4-F-Ph | $2-NH_2-4-Pym$ | 1-Me-2-Tmor |
| 1-621 | 4-F-Ph | $2-NH_2-4-Pym$ | 1-Piz |
| 1-622 | 4-F-Ph | $2-NH_2-4-Pym$ | 4-Me-1-Piz |
| 1-623 | 4-F-Ph | $2-NH_2-4-Pym$ | 2-Piz |
| 1-624 | 4-F-Ph | $2-NH_2-4-Pym$ | 4-Pyr |
| 1-625 | 4-F-Ph | $2-NH_2-4-Pym$ | 3-Pyr |
| 1-626 | 4-F-Ph | $2-NH_2-4-Pym$ | 2-Pyr |
| 1-627 | 4-F-Ph | $2-NH_2-4-Pym$ | 4-Pym |
| 1-628 | 4-F-Ph | $2-NH_2-4-Pym$ | 5-Pym |
| 1-629 | 4-F-Ph | $2-NH_2-4-Pym$ | 2-Pym |
| 1-630 | 4-F-Ph | $2-NH_2-4-Pym$ | $3-Azt-CH_2$ |
| 1-631 | 4-F-Ph | $2-NH_2-4-Pym$ | $1-Me-3-Azt-CH_2$ |
| 1-632 | 4-F-Ph | $2-NH_2-4-Pym$ | $3-Pyrd-CH_2$ |
| 1-633 | 4-F-Ph | $2-NH_2-4-Pym$ | $1-Me-3-Pyrd-CH_2$ |
| 1-634 | 4-F-Ph | $2-NH_2-4-Pym$ | $4-Pip-CH_2$ |
| 1-635 | 4-F-Ph | $2-NH_2-4-Pym$ | $1-Me-4-Pip-CH_2$ |
| 1-636 | 4-F-Ph | $2-NH_2-4-Pym$ | $3-Hpip-CH_2$ |
| 1-637 | 4-F-Ph | $2-NH_2-4-Pym$ | $1-Me-3-Hpip-CH_2$ |
| 1-638 | 4-F-Ph | $2-NH_2-4-Pym$ | $4-Hpip-CH_2$ |

Table 1 (continued)

| Compound No. | $R^1$ | $R^2$ | $R^3$ |
|---|---|---|---|
| 1-639 | 4-F-Ph | 2-$NH_2$-4-Pym | 1-Me-4-Hpip-$CH_2$ |
| 1-640 | 4-F-Ph | 2-$NH_2$-4-Pym | 2-Mor-$CH_2$ |
| 1-641 | 4-F-Ph | 2-$NH_2$-4-Pym | 1-Me-2-Mor-$CH_2$ |
| 1-642 | 4-F-Ph | 2-$NH_2$-4-Pym | 2-Tmor-$CH_2$ |
| 1-643 | 4-F-Ph | 2-$NH_2$-4-Pym | 1-Me-2-Tmor-$CH_2$ |
| 1-644 | 4-F-Ph | 2-$NH_2$-4-Pym | 1-Piz-$CH_2$ |
| 1-645 | 4-F-Ph | 2-$NH_2$-4-Pym | 4-Me-1-Piz-$CH_2$ |
| 1-646 | 4-F-Ph | 2-$NH_2$-4-Pym | 2-Piz-$CH_2$ |
| 1-647 | 4-F-Ph | 2-$NH_2$-4-Pym | 4-Pyr-$CH_2$ |
| 1-648 | 4-F-Ph | 2-$NH_2$-4-Pym | 3-Pyr-$CH_2$ |
| 1-649 | 4-F-Ph | 2-$NH_2$-4-Pym | 2-Pyr-$CH_2$ |
| 1-650 | 4-F-Ph | 2-$NH_2$-4-Pym | 4-Pym-$CH_2$ |
| 1-651 | 4-F-Ph | 2-$NH_2$-4-Pym | 5-Pym-$CH_2$ |
| 1-652 | 4-F-Ph | 2-$NH_2$-4-Pym | 2-Pym-$CH_2$ |
| 1-653 | 3-F-Ph | 2-$NH_2$-4-Pym | $H_2N$-$CH_2$ |
| 1-654 | 3-F-Ph | 2-$NH_2$-4-Pym | $H_2N$-$(CH_2)_2$ |
| 1-655 | 3-F-Ph | 2-$NH_2$-4-Pym | $H_2N$-$(CH_2)_3$ |
| 1-656 | 3-F-Ph | 2-$NH_2$-4-Pym | $H_2N$-$(CH_2)_4$ |
| 1-657 | 3-F-Ph | 2-$NH_2$-4-Pym | MeNH-$CH_2$ |
| 1-658 | 3-F-Ph | 2-$NH_2$-4-Pym | MeNH-$(CH_2)_2$ |
| 1-659 | 3-F-Ph | 2-$NH_2$-4-Pym | MeNH-$(CH_2)_3$ |
| 1-660 | 3-F-Ph | 2-$NH_2$-4-Pym | MeNH-$(CH_2)_4$ |
| 1-661 | 3-F-Ph | 2-$NH_2$-4-Pym | EtNH-$CH_2$ |
| 1-662 | 3-F-Ph | 2-$NH_2$-4-Pym | EtNH-$(CH_2)_2$ |
| 1-663 | 3-F-Ph | 2-$NH_2$-4-Pym | EtNH-$(CH_2)_3$ |
| 1-664 | 3-F-Ph | 2-$NH_2$-4-Pym | EtNH-$(CH_2)_4$ |
| 1-665 | 3-F-Ph | 2-$NH_2$-4-Pym | $Me_2N$-$CH_2$ |
| 1-666 | 3-F-Ph | 2-$NH_2$-4-Pym | $Me_2N$-$(CH_2)_2$ |
| 1-667 | 3-F-Ph | 2-$NH_2$-4-Pym | $Me_2N$-$(CH_2)_3$ |
| 1-668 | 3-F-Ph | 2-$NH_2$-4-Pym | $Me_2N$-$(CH_2)_4$ |
| 1-669 | 3-F-Ph | 2-$NH_2$-4-Pym | 1-Azt-$CH_2$ |
| 1-670 | 3-F-Ph | 2-$NH_2$-4-Pym | 1-Azt-$(CH_2)_2$ |
| 1-671 | 3-F-Ph | 2-$NH_2$-4-Pym | 1-Azt-$(CH_2)_3$ |
| 1-672 | 3-F-Ph | 2-$NH_2$-4-Pym | 1-Azt-$(CH_2)_4$ |
| 1-673 | 3-F-Ph | 2-$NH_2$-4-Pym | 1-Pyrd-$CH_2$ |
| 1-674 | 3-F-Ph | 2-$NH_2$-4-Pym | 1-Pyrd-$(CH_2)_2$ |
| 1-675 | 3-F-Ph | 2-NH2-4-Pym | 1-Pyrd-$(CH_2)_3$ |
| 1-676 | 3-F-Ph | 2-$NH_2$-4-Pym | 1-Pyrd-$(CH_2)_4$ |

Table 1 (continued)

| Compound No. | $R^1$ | $R^2$ | $R^3$ |
|---|---|---|---|
| 1-677 | 3-F-Ph | $2-NH_2-4-Pym$ | $1-Pip-CH_2$ |
| 1-678 | 3-F-Ph | $2-NH_2-4-Pym$ | $1-Pip-(CH_2)_2$ |
| 1-679 | 3-F-Ph | $2-NH_2-4-Pym$ | $1-Pip-(CH_2)_3$ |
| 1-680 | 3-F-Ph | $2-NH_2-4-Pym$ | $1-Pip-(CH_2)_4$ |
| 1-681 | 3-F-Ph | $2-NH_2-4-Pym$ | $1-Mor-CH_2$ |
| 1-682 | 3-F-Ph | $2-NH_2-4-Pym$ | $1-Mor-(CH_2)_2$ |
| 1-683 | 3-F-Ph | $2-NH_2-4-Pym$ | $1-Mor-(CH_2)_3$ |
| 1-684 | 3-F-Ph | $2-NH_2-4-Pym$ | $1-Mor-(CH_2)_4$ |
| 1-685 | 3-F-Ph | $2-NH_2-4-Pym$ | $1-Tmor-CH_2$ |
| 1-686 | 3-F-Ph | $2-NH_2-4-Pym$ | $1-Tmor-(CH_2)_2$ |
| 1-687 | 3-F-Ph | $2-NH_2-4-Pym$ | $1-Tmor-(CH_2)_3$ |
| 1-688 | 3-F-Ph | $2-NH_2-4-Pym$ | $1-Tmor-(CH_2)_4$ |
| 1-689 | 3-F-Ph | $2-NH_2-4-Pym$ | $1-Piz-CH_2$ |
| 1-690 | 3-F-Ph | $2-NH_2-4-Pym$ | $1-Piz-(CH_2)_2$ |
| 1-691 | 3-F-Ph | $2-NH_2-4-Pym$ | $1-Piz-(CH_2)_3$ |
| 1-692 | 3-F-Ph | $2-NH_2-4-Pym$ | $4-Me-1-Piz-(CH_2)_3$ |
| 1-693 | 3-F-Ph | $2-NH_2-4-Pym$ | $1-Piz-(CH_2)_4$ |
| 1-694 | 3-F-Ph | $2-NH_2-4-Pym$ | 3-Azt |
| 1-695 | 3-F-Ph | $2-NH_2-4-Pym$ | 1-Me-3-Azt |
| 1-696 | 3-F-Ph | $2-NH_2-4-Pym$ | 1-Bn-3-Azt |
| 1-697 | 3-F-Ph | $2-NH_2-4-Pym$ | 3-Pyrd |
| 1-698 | 3-F-Ph | $2-NH_2-4-Pym$ | 1-Me-3-Pyrd |
| 1-699 | 3-F-Ph | $2-NH_2-4-Pym$ | 3-Pip |
| 1-700 | 3-F-Ph | $2-NH_2-4-Pym$ | 4-Pip |
| 1-701 | 3-F-Ph | $2-NH_2-4-Pym$ | 4-(3,4-deH-Pip) |
| 1-702 | 3-F-Ph | $2-NH_2-4-Pym$ | 1-Me-4-Pip |
| 1-703 | 3-F-Ph | $2-NH_2-4-Pym$ | 1-Me-4-(3,4-deH-Pip) |
| 1-704 | 3-F-Ph | $2-NH_2-4-Pym$ | 1-Et-4-Pip |
| 1-705 | 3-F-Ph | $2-NH_2-4-Pym$ | 1-Bn-4-Pip |
| 1-706 | 3-F-Ph | $2-NH_2-4-Pym$ | 3-Hpip |
| 1-707 | 3-F-Ph | $2-NH_2-4-Pym$ | 1-Me-3-Hpip |
| 1-708 | 3-F-Ph | $2-NH_2-4-Pym$ | 4-Hpip |
| 1-709 | 3-F-Ph | $2-NH_2-4-Pym$ | 1-Me-4-Hpip |
| 1-710 | 3-F-Ph | $2-NH_2-4-Pym$ | 2-Mor |
| 1-711 | 3-F-Ph | $2-NH_2-4-Pym$ | 1-Me-2-Mor |
| 1-712 | 3-F-Ph | $2-NH_2-4-Pym$ | 2-Tmor |
| 1-713 | 3-F-Ph | $2-NH_2-4-Pym$ | 1-Me-2-Tmor |
| 1-714 | 3-F-Ph | $2-NH_2-4-Pym$ | 1-Piz |

Table 1 (continued)

| Compound No. | $R^1$ | $R^2$ | $R^3$ |
|---|---|---|---|
| 1-715 | 3-F-Ph | 2-$NH_2$-4-Pym | 4-Me-1-Piz |
| 1-716 | 3-F-Ph | 2-$NH_2$-4-Pym | 2-Piz |
| 1-717 | 3-F-Ph | 2-$NH_2$-4-Pym | 4-Pyr |
| 1-718 | 3-F-Ph | 2-$NH_2$-4-Pym | 3-Pyr |
| 1-719 | 3-F-Ph | 2-$NH_2$-4-Pym | 2-Pyr |
| 1-720 | 3-F-Ph | 2-$NH_2$-4-Pym | 4-Pym |
| 1-721 | 3-F-Ph | 2-$NH_2$-4-Pym | 5-Pym |
| 1-722 | 3-F-Ph | 2-$NH_2$-4-Pym | 2-Pym |
| 1-723 | 3-F-Ph | 2-$NH_2$-4-Pym | 3-Azt-$CH_2$ |
| 1-724 | 3-F-Ph | 2-$NH_2$-4-Pym | 1-Me-3-Azt-$CH_2$ |
| 1-725 | 3-F-Ph | 2-$NH_2$-4-Pym | 3-Pyrd-$CH_2$ |
| 1-726 | 3-F-Ph | 2-$NH_2$-4-Pym | 1-Me-3-Pyrd-$CH_2$ |
| 1-727 | 3-F-Ph | 2-$NH_2$-4-Pym | 4-Pip-$CH_2$ |
| 1-728 | 3-F-Ph | 2-$NH_2$-4-Pym | 1-Me-4-Pip-$CH_2$ |
| 1-729 | 3-F-Ph | 2-$NH_2$-4-Pym | 3-Hpip-$CH_2$ |
| 1-730 | 3-F-Ph | 2-$NH_2$-4-Pym | 1-Me-3-Hpip-$CH_2$ |
| 1-731 | 3-F-Ph | 2-$NH_2$-4-Pym | 4-Hpip-$CH_2$ |
| 1-732 | 3-F-Ph | 2-$NH_2$-4-Pym | 1-Me-4-Hpip-$CH_2$ |
| 1-733 | 3-F-Ph | 2-$NH_2$-4-Pym | 2-Mor-$CH_2$ |
| 1-734 | 3-F-Ph | 2-$NH_2$-4-Pym | 1-Me-2-Mor-$CH_2$ |
| 1-735 | 3-F-Ph | 2-$NH_2$-4-Pym | 2-Tmor-$CH_2$ |
| 1-736 | 3-F-Ph | 2-$NH_2$-4-Pym | 1-Me-2-Tmor-$CH_2$ |
| 1-737 | 3-F-Ph | 2-$NH_2$-4-Pym | 1-Piz-$CH_2$ |
| 1-738 | 3-F-Ph | 2-$NH_2$-4-Pym | 4-Me-1-Piz-$CH_2$ |
| 1-739 | 3-F-Ph | 2-$NH_2$-4-Pym | 2-Piz-$CH_2$ |
| 1-740 | 3-F-Ph | 2-$NH_2$-4-Pym | 4-Pyr-$CH_2$ |
| 1-741 | 3-F-Ph | 2-$NH_2$-4-Pym | 3-Pyr-$CH_2$ |
| 1-742 | 3-F-Ph | 2-$NH_2$-4-Pym | 2-Pyr-$CH_2$ |
| 1-743 | 3-F-Ph | 2-$NH_2$-4-Pym | 4-Pym-$CH_2$ |
| 1-744 | 3-F-Ph | 2-$NH_2$-4-Pym | 5-Pym-$CH_2$ |
| 1-745 | 3-F-Ph | 2-$NH_2$-4-Pym | 2-Pym-$CH_2$ |
| 1-746 | 3,4-diF-Ph | 2-$NH_2$-4-Pym | $H_2N$-$CH_2$ |
| 1-747 | 3,4-diF-Ph | 2-$NH_2$-4-Pym | $H_2N$-$(CH_2)_2$ |
| 1-748 | 3,4-diF-Ph | 2-$NH_2$-4-Pym | $H_2N$-$(CH_2)_3$ |
| 1-749 | 3,4-diF-Ph | 2-$NH_2$-4-Pym | $H_2N$-$(CH_2)_4$ |
| 1-750 | 3,4-diF-Ph | 2-$NH_2$-4-Pym | MeNH-$CH_2$ |
| 1-751 | 3,4-diF-Ph | 2-$NH_2$-4-Pym | MeNH-$(CH_2)_2$ |
| 1-752 | 3,4-diF-Ph | 2-$NH_2$-4-Pym | MeNH-$(CH_2)_3$ |

Table 1 (continued)

| Compound No. | $R^1$ | $R^2$ | $R^3$ |
|---|---|---|---|
| 1-753 | 3,4-diF-Ph | 2-$NH_2$-4-Pym | MeNH-$(CH_2)_4$ |
| 1-754 | 3,4-diF-Ph | 2-$NH_2$-4-Pym | EtNH-$CH_2$ |
| 1-755 | 3,4-diF-Ph | 2-$NH_2$-4-Pym | EtNH-$(CH_2)_2$ |
| 1-756 | 3,4-diF-Ph | 2-$NH_2$-4-Pym | EtNH-$(CH_2)_3$ |
| 1-757 | 3,4-diF-Ph | 2-$NH_2$-4-Pym | EtNH-$(CH_2)_4$ |
| 1-758 | 3,4-diF-Ph | 2-$NH_2$-4-Pym | $Me_2N$-$CH_2$ |
| 1-759 | 3,4-diF-Ph | 2-$NH_2$-4-Pym | $Me_2N$-$(CH_2)_2$ |
| 1-760 | 3,4-diF-Ph | 2-$NH_2$-4-Pym | $Me_2N$-$(CH_2)_3$ |
| 1-761 | 3,4-diF-Ph | 2-$NH_2$-4-Pym | $Me_2N$-$(CH_2)_4$ |
| 1-762 | 3,4-diF-Ph | 2-$NH_2$-4-Pym | 1-Azt-$CH_2$ |
| 1-763 | 3,4-diF-Ph | 2-$NH_2$-4-Pym | 1-Azt-$(CH_2)_2$ |
| 1-769 | 3,4-diF-Ph | 2-$NH_2$-4-Pym | 1-Azt-$(CH_2)_3$ |
| 1-765 | 3,4-diF-Ph | 2-$NH_2$-4-Pym | 1-Azt-$(CH_2)_4$ |
| 1-766 | 3,4-diF-Ph | 2-$NH_2$-4-Pym | 1-Pyrd-$CH_2$ |
| 1-767 | 3,4-diF-Ph | 2-$NH_2$-4-Pym | 1-Pyrd-$(CH_2)_2$ |
| 1-768 | 3,4-diF-Ph | 2-$NH_2$-4-Pym | 1-Pyrd-$(CH_2)_3$ |
| 1-769 | 3,4-diF-Ph | 2-$NH_2$-4-Pym | 1-Pyrd-$(CH_2)_4$ |
| 1-770 | 3,4-diF-Ph | 2-$NH_2$-4-Pym | 1-Pip-$CH_2$ |
| 1-771 | 3,4-diF-Ph | 2-$NH_2$-4-Pym | 1-Pip-$(CH_2)_2$ |
| 1-772 | 3,4-diF-Ph | 2-$NH_2$-4-Pym | 1-Pip-$(CH_2)_3$ |
| 1-773 | 3,4-diF-Ph | 2-$NH_2$-4-Pym | 1-Pip-$(CH_2)_4$ |
| 1-774 | 3,4-diF-Ph | 2-$NH_2$-4-Pym | 1-Mor-$CH_2$ |
| 1-775 | 3,4-diF-Ph | 2-$NH_2$-4-Pym | 1-Mor-$(CH_2)_2$ |
| 1-776 | 3,4-diF-Ph | 2-$NH_2$-4-Pym | 1-Mor-$(CH_2)_3$ |
| 1-777 | 3,4-diF-Ph | 2-$NH_2$-4-Pym | 1-Mor-$(CH_2)_4$ |
| 1-778 | 3,4-diF-Ph | 2-$NH_2$-4-Pym | 1-Tmor-$CH_2$ |
| 1-779 | 3,4-diF-Ph | 2-$NH_2$-4-Pym | 1-Tmor-$(CH_2)_2$ |
| 1-780 | 3,4-diF-Ph | 2-$NH_2$-4-Pym | 1-Tmor-$(CH_2)_3$ |
| 1-781 | 3,4-diF-Ph | 2-$NH_2$-4-Pym | 1-Tmor-$(CH_2)_4$ |
| 1-782 | 3,4-diF-Ph | 2-$NH_2$-4-Pym | 1-Piz-$CH_2$ |
| 1-783 | 3,4-diF-Ph | 2-$NH_2$-4-Pym | 1-Piz-$(CH_2)_2$ |
| 1-784 | 3,4-diF-Ph | 2-$NH_2$-4-Pym | 1-Piz-$(CH_2)_3$ |
| 1-785 | 3, 4-diF-Ph | 2-$NH_2$-4-Pym | 4-Me-1-Piz-$(CH_2)_3$ |
| 1-786 | 3,4-diF-Ph | 2-$NH_2$-4-Pym | 1-Piz-$(CH_2)_4$ |
| 1-787 | 3,4-diF-Ph | 2-$NH_2$-4-Pym | 3-Azt |
| 1-788 | 3,4-diF-Ph | 2-$NH_2$-4-Pym | 1-Me-3-Azt |
| 1-789 | 3,4-diF-Ph | 2-$NH_2$-4-Pym | 1-Bn-3-Azt |
| 1-790 | 3,4-diF-Ph | 2-$NH_2$-4-Pym | 3-Pyrd |

Table 1 (continued)

| Compound No. | $R^1$ | $R^2$ | $R^3$ |
|---|---|---|---|
| 1-791 | 3,4-diF-Ph | 2-$NH_2$-4-Pym | 1-Me-3-Pyrd |
| 1-792 | 3,4-diF-Ph | 2-$NH_2$-4-Pym | 3-Pip |
| 1-793 | 3,4-diF-Ph | 2-$NH_2$-4-Pym | 4-Pip |
| 1-794 | 3,4-diF-Ph | 2-$NH_2$-4-Pym | 4-(3,4-deH-Pip) |
| 1-795 | 3,4-diF-Ph | 2-$NH_2$-4-Pym | 1-Me-4-Pip |
| 1-796 | 3,4-diF-Ph | 2-$NH_2$-4-Pym | 1-Me-4-(3,4-deH-Pip) |
| 1-797 | 3,4-diF-Ph | 2-$NH_2$-4-Pym | 1-Et-4-Pip |
| 1-798 | 3,4-diF-Ph | 2-$NH_2$-4-Pym | 1-Bn-4-Pip |
| 1-799 | 3,4-diF-Ph | 2-$NH_2$-4-Pym | 3-Hpip |
| 1-800 | 3,4-diF-Ph | 2-$NH_2$-4-Pym | 1-Me-3-Hpip |
| 1-801 | 3,4-diF-Ph | 2-$NH_2$-4-Pym | 4-Hpip |
| 1-802 | 3,4-diF-Ph | 2-$NH_2$-4-Pym | 1-Me-4-Hpip |
| 1-803 | 3,4-diF-Ph | 2-$NH_2$-4-Pym | 2-Mor |
| 1-804 | 3,4-diF-Ph | 2-$NH_2$-4-Pym | 1-Me-2-Mor |
| 1-805 | 3,4-diF-Ph | 2-$NH_2$-4-Pym | 2-Tmor |
| 1-806 | 3,4-diF-Ph | 2-$NH_2$-4-Pym | 1-Me-2-Tmor |
| 1-807 | 3,4-diF-Ph | 2-$NH_2$-4-Pym | 1-Piz |
| 1-808 | 3,4-diF-Ph | 2-$NH_2$-4-Pym | 4-Me-1-Piz |
| 1-809 | 3,4-diF-Ph | 2-$NH_2$-4-Pym | 2-Piz |
| 1-810 | 3,4-diF-Ph | 2-$NH_2$-4-Pym | 4-Pyr |
| 1-811 | 3,4-diF-Ph | 2-$NH_2$-4-Pym | 3-Pyr |
| 1-812 | 3,4-diF-Ph | 2-$NH_2$-4-Pym | 2-Pyr |
| 1-813 | 3,4-diF-Ph | 2-$NH_2$-4-Pym | 4-Pym |
| 1-814 | 3,4-diF-Ph | 2-$NH_2$-4-Pym | 5-Pym |
| 1-815 | 3,4-diF-Ph | 2-$NH_2$-4-Pym | 2-Pym |
| 1-816 | 3,4-diF-Ph | 2-$NH_2$-4-Pym | 3-Azt-$CH_2$ |
| 1-817 | 3,4-diF-Ph | 2-$NH_2$-4-Pym | 1-Me-3-Azt-$CH_2$ |
| 1-818 | 3,4-diF-Ph | 2-$NH_2$-4-Pym | 3-Pyrd-$CH_2$ |
| 1-819 | 3,4-diF-Ph | 2-$NH_2$-4-Pym | 1-Me-3-Pyrd-$CH_2$ |
| 1-820 | 3,4-diF-Ph | 2-$NH_2$-4-Pym | 4-Pip-$CH_2$ |
| 1-821 | 3,4-diF-Ph | 2-$NH_2$-4-Pym | 1-Me-4-Pip-$CH_2$ |
| 1-822 | 3,4-diF-Ph | 2-$NH_2$-4-Pym | 3-Hpip-$CH_2$ |
| 1-823 | 3,4-diF-Ph | 2-$NH_2$-4-Pym | 1-Me-3-Hpip-$CH_2$ |
| 1-824 | 3,4-diF-Ph | 2-$NH_2$-4-Pym | 4-Hpip-$CH_2$ |
| 1-825 | 3,4-diF-Ph | 2-$NH_2$-4-Pym | 1-Me-4-Hpip-$CH_2$ |
| 1-826 | 3,4-diF-Ph | 2-$NH_2$-4-Pym | 2-Mor-$CH_2$ |
| 1-827 | 3,4-diF-Ph | 2-$NH_2$-4-Pym | 1-Me-2-Mor-$CH_2$ |
| 1-828 | 3,4-diF-Ph | 2-$NH_2$-4-Pym | 2-Tmor-$CH_2$ |

Table 1 (continued)

| Compound No. | $R^1$ | $R^2$ | $R^3$ |
|---|---|---|---|
| 1-829 | 3,4-diF-Ph | 2-$NH_2$-4-Pym | 1-Me-2-Tmor-$CH_2$ |
| 1-830 | 3,4-diF-Ph | 2-$NH_2$-4-Pym | 1-Piz-$CH_2$ |
| 1-831 | 3,4-diF-Ph | 2-$NH_2$-4-Pym | 4-Me-1-Piz-$CH_2$ |
| 1-832 | 3,4-diF-Ph | 2-$NH_2$-4-Pym | 2-Piz-$CH_2$ |
| 1-833 | 3,4-diF-Ph | 2-$NH_2$-4-Pym | 4-Pyr-$CH_2$ |
| 1-834 | 3,4-diF-Ph | 2-$NH_2$-4-Pym | 3-Pyr-$CH_2$ |
| 1-835 | 3,4-diF-Ph | 2-$NH_2$-4-Pym | 2-Pyr-$CH_2$ |
| 1-836 | 3,4-diF-Ph | 2-$NH_2$-4-Pym | 4-Pym-$CH_2$ |
| 1-837 | 3,4-diF-Ph | 2-$NH_2$-4-Pym | 5-Pym-CH2 |
| 1-838 | 3,4-diF-Ph | 2-$NH_2$-4-Pym | 2-Pym-$CH_2$ |
| 1-839 | 3-Cl-Ph | 2-$NH_2$-4-Pym | $H_2N$-$CH_2$ |
| 1-840 | 3-Cl-Ph | 2-$NH_2$-4-Pym | $H_2N$-$(CH_2)_2$ |
| 1-841 | 3-Cl-Ph | 2-$NH_2$-4-Pym | $H_2N$-$(CH_2)_3$ |
| 1-842 | 3-Cl-Ph | 2-$NH_2$-4-Pym | $H_2N$-$(CH_2)_4$ |
| 1-843 | 3-Cl-Ph | 2-$NH_2$-4-Pym | MeNH-$CH_2$ |
| 1-844 | 3-Cl-Ph | 2-$NH_2$-4-Pym | MeNH-$(CH_2)_2$ |
| 1-845 | 3-Cl-Ph | 2-$NH_2$-4-Pym | MeNH-$(CH_2)_3$ |
| 1-846 | 3-Cl-Ph | 2-$NH_2$-4-Pym | MeNH-$(CH_2)_4$ |
| 1-847 | 3-Cl-Ph | 2-$NH_2$-4-Pym | EtNH-$CH_2$ |
| 1-848 | 3-Cl-Ph | 2-NH2-4-Pym | EtNH-$(CH_2)_2$ |
| 1-849 | 3-Cl-Ph | 2-$NH_2$-4-Pym | EtNH-$(CH_2)_3$ |
| 1-850 | 3-Cl-Ph | 2-$NH_2$-4-Pym | EtNH-$(CH_2)_4$ |
| 1-851 | 3-Cl-Ph | 2-$NH_2$-4-Pym | $Me_2N$-$CH_2$ |
| 1-852 | 3-Cl-Ph | 2-$NH_2$-4-Pym | $Me_2N$-$(CH_2)_2$ |
| 1-853 | 3-Cl-Ph | 2-$NH_2$-4-Pym | $Me_2N$-$(CH_2)_3$ |
| 1-854 | 3-Cl-Ph | 2-$NH_2$-4-Pym | $Me_2N$-$(CH_2)_4$ |
| 1-855 | 3-Cl-Ph | 2-NH2-4-Pym | 1-Azt-$CH_2$ |
| 1-856 | 3-Cl-Ph | 2-$NH_2$-4-Pym | 1-Azt-$(CH_2)_2$ |
| 1-857 | 3-Cl-Ph | 2-$NH_2$-4-Pym | 1-Azt-$(CH_2)_3$ |
| 1-858 | 3-Cl-Ph | 2-$NH_2$-4-Pym | 1-Azt-$(CH_2)_4$ |
| 1-859 | 3-Cl-Ph | 2-$NH_2$-4-Pym | 1-Pyrd-$CH_2$ |
| 1-860 | 3-Cl-Ph | 2-$NH_2$-4-Pym | 1-Pyrd-$(CH_2)_2$ |
| 1-861 | 3-Cl-Ph | 2-$NH_2$-4-Pym | 1-Pyrd-$(CH_2)_3$ |
| 1-862 | 3-Cl-Ph | 2-$NH_2$-4-Pym | 1-Pyrd-$(CH_2)_4$ |
| 1-863 | 3-Cl-Ph | 2-$NH_2$-4-Pym | 1-Pip-$CH_2$ |
| 1-864 | 3-Cl-Ph | 2-$NH_2$-4-Pym | 1-Pip-$(CH_2)_2$ |
| 1-865 | 3-Cl-Ph | 2-$NH_2$-4-Pym | 1-Pip-$(CH_2)_3$ |
| 1-866 | 3-Cl-Ph | 2-$NH_2$-4-Pym | 1-Pip-$(CH_2)_4$ |

Table 1 (continued)

| Compound No. | $R^1$ | $R^2$ | $R^3$ |
|---|---|---|---|
| 1-867 | 3-Cl-Ph | 2-$NH_2$-4-Pym | 1-Mor-$CH_2$ |
| 1-868 | 3-Cl-Ph | 2-$NH_2$-4-Pym | 1-Mor-$(CH_2)_2$ |
| 1-869 | 3-Cl-Ph | 2-$NH_2$-4-Pym | 1-Mor-$(CH_2)_3$ |
| 1-870 | 3-Cl-Ph | 2-$NH_2$-4-Pym | 1-Mor-$(CH_2)_4$ |
| 1-871 | 3-Cl-Ph | 2-$NH_2$-4-Pym | 1-Tmor-$CH_2$ |
| 1-872 | 3-Cl-Ph | 2-$NH_2$-4-Pym | 1-Tmor-$(CH_2)_2$ |
| 1-873 | 3-Cl-Ph | 2-$NH_2$-4-Pym | 1-Tmor-$(CH_2)_3$ |
| 1-874 | 3-Cl-Ph | 2-$NH_2$-4-Pym | 1-Tmor-$(CH_2)_4$ |
| 1-875 | 3-Cl-Ph | 2-$NH_2$-4-Pym | 1-Piz-$CH_2$ |
| 1-876 | 3-Cl-Ph | 2-$NH_2$-4-Pym | 1-Piz-$(CH_2)_2$ |
| 1-877 | 3-Cl-Ph | 2-$NH_2$-4-Pym | 1-Piz-$(CH_2)_3$ |
| 1-878 | 3-Cl-Ph | 2-$NH_2$-4-Pym | 4-Me-1-Piz-$(CH_2)_3$ |
| 1-879 | 3-Cl-Ph | 2-$NH_2$-4-Pym | 1-Piz-$(CH_2)_4$ |
| 1-880 | 3-Cl-Ph | 2-$NH_2$-4-Pym | 3-Azt |
| 1-881 | 3-Cl-Ph | 2-$NH_2$-4-Pym | 1-Me-3-Azt |
| 1-882 | 3-Cl-Ph | 2-$NH_2$-4-Pym | 1-Bn-3-Azt |
| 1-883 | 3-Cl-Ph | 2-$NH_2$-4-Pym | 3-Pyrd |
| 1-884 | 3-Cl-Ph | 2-$NH_2$-4-Pym | 1-Me-3-Pyrd |
| 1-885 | 3-Cl-Ph | 2-$NH_2$-4-Pym | 3-Pip |
| 1-886 | 3-Cl-Ph | 2-$NH_2$-4-Pym | 4-Pip |
| 1-887 | 3-Cl-Ph | 2-$NH_2$-4-Pym | 4-(3,4-deH-Pip) |
| 1-888 | 3-Cl-Ph | 2-$NH_2$-4-Pym | 1-Me-4-Pip |
| 1-889 | 3-Cl-Ph | 2-$NH_2$-4-Pym | 1-Me-4-(3,4-deH-Pip) |
| 1-890 | 3-Cl-Ph | 2-$NH_2$-4-Pym | 1-Et-4-Pip |
| 1-891 | 3-Cl-Ph | 2-$NH_2$-4-Pym | 1-Bn-4-Pip |
| 1-892 | 3-Cl-Ph | 2-$NH_2$-4-Pym | 3-Hpip |
| 1-893 | 3-Cl-Ph | 2-$NH_2$-4-Pym | 1-Me-3-Hpip |
| 1-894 | 3-Cl-Ph | 2-$NH_2$-4-Pym | 4-Hpip |
| 1-895 | 3-Cl-Ph | 2-$NH_2$-4-Pym | 1-Me-4-Hpip |
| 1-896 | 3-Cl-Ph | 2-$NH_2$-4-Pym | 2-Mor |
| 1-897 | 3-Cl-Ph | 2-$NH_2$-4-Pym | 1-Me-2-Mor |
| 1-898 | 3-Cl-Ph | 2-$NH_2$-4-Pym | 2-Tmor |
| 1-899 | 3-Cl-Ph | 2-$NH_2$-4-Pym | 1-Me-2-Tmor |
| 1-900 | 3-Cl-Ph | 2-$NH_2$-4-Pym | 1-Piz |
| 1-901 | 3-Cl-Ph | 2-$NH_2$-4-Pym | 4-Me-1-Piz |
| 1-902 | 3-Cl-Ph | 2-$NH_2$-4-Pym | 2-Piz |
| 1-903 | 3-Cl-Ph | 2-$NH_2$-4-Pym | 4-Pyr |
| 1-904 | 3-Cl-Ph | 2-$NH_2$-4-Pym | 3-Pyr |

Table 1 (continued)

| Compound No. | $R^1$ | $R^2$ | $R^3$ |
|---|---|---|---|
| 1-905 | 3-Cl-Ph | $2-NH_2-4-Pym$ | 2-Pyr |
| 1-906 | 3-Cl-Ph | $2-NH_2-4-Pym$ | 4-Pym |
| 1-907 | 3-Cl-Ph | $2-NH_2-4-Pym$ | 5-Pym |
| 1-908 | 3-Cl-Ph | $2-NH_2-4-Pym$ | 2-Pym |
| 1-909 | 3-Cl-Ph | $2-NH_2-4-Pym$ | $3-Azt-CH_2$ |
| 1-910 | 3-Cl-Ph | $2-NH_2-4-Pym$ | $1-Me-3-Azt-CH_2$ |
| 1-911 | 3-Cl-Ph | $2-NH_2-4-Pym$ | $3-Pyrd-CH_2$ |
| 1-912 | 3-Cl-Ph | $2-NH_2-4-Pym$ | $1-Me-3-Pyrd-CH_2$ |
| 1-913 | 3-Cl-Ph | $2-NH_2-4-Pym$ | $4-Pip-CH_2$ |
| 1-914 | 3-Cl-Ph | $2-NH_2-4-Pym$ | $1-Me-4-Pip-CH_2$ |
| 1-915 | 3-Cl-Ph | $2-NH_2-4-Pym$ | $3-Hpip-CH_2$ |
| 1-916 | 3-Cl-Ph | $2-NH_2-4-Pym$ | $1-Me-3-Hpip-CH_2$ |
| 1-917 | 3-Cl-Ph | $2-NH_2-4-Pym$ | $4-Hpip-CH_2$ |
| 1-918 | 3-Cl-Ph | $2-NH_2-4-Pym$ | $1-Me-4-Hpip-CH_2$ |
| 1-919 | 3-Cl-Ph | $2-NH_2-4-Pym$ | $2-Mor-CH_2$ |
| 1-920 | 3-Cl-Ph | $2-NH_2-4-Pym$ | $1-Me-2-Mor-CH_2$ |
| 1-921 | 3-Cl-Ph | $2-NH_2-4-Pym$ | $2-Tmor-CH_2$ |
| 1-922 | 3-Cl-Ph | $2-NH_2-4-Pym$ | $1-Me-2-Tmor-CH_2$ |
| 1-923 | 3-Cl-Ph | $2-NH_2-4-Pym$ | $1-Piz-CH_2$ |
| 1-924 | 3-Cl-Ph | $2-NH_2-4-Pym$ | $4-Me-1-Piz-CH_2$ |
| 1-925 | 3-Cl-Ph | $2-NH_2-4-Pym$ | $2-Piz-CH_2$ |
| 1-926 | 3-Cl-Ph | $2-NH_2-4-Pym$ | $4-Pyr-CH_2$ |
| 1-927 | 3-Cl-Ph | $2-NH_2-4-Pym$ | $3-Pyr-CH_2$ |
| 1-928 | 3-Cl-Ph | $2-NH_2-4-Pym$ | $2-Pyr-CH_2$ |
| 1-929 | 3-Cl-Ph | $2-NH_2-4-Pym$ | $4-Pym-CH_2$ |
| 1-930 | 3-Cl-Ph | $2-NH_2-4-Pym$ | $5-Pym-CH_2$ |
| 1-931 | 3-Cl-Ph | $2-NH_2-4-Pym$ | $2-Pym-CH_2$ |
| 1-932 | Ph | 2-MeNH-4-Pym | $H_2N-CH_2$ |
| 1-933 | Ph | 2-MeNH-4-Pym | $H_2N-(CH_2)_2$ |
| 1-934 | Ph | 2-MeNH-4-Pym | $H_2N-(CH_2)_3$ |
| 1-935 | Ph | 2-MeNH-4-Pym | $H_2N-(CH_2)_4$ |
| 1-936 | Ph | 2-MeNH-4-Pym | $MeNH-CH_2$ |
| 1-937 | Ph | 2-MeNH-4-Pym | $MeNH-(CH_2)_2$ |
| 1-938 | Ph | 2-MeNH-4-Pym | $MeNH-(CH_2)_3$ |
| 1-939 | Ph | 2-MeNH-4-Pym | $MeNH-(CH_2)_4$ |
| 1-940 | Ph | 2-MeNH-4-Pym | $EtNH-CH_2$ |
| 1-941 | Ph | 2-MeNH-4-Pym | $EtNH-(CH_2)_2$ |
| 1-942 | Ph | 2-MeNH-4-Pym | $EtNH-(CH_2)_3$ |

Table 1 (continued)

| Compound No. | $R^1$ | $R^2$ | $R^3$ |
|---|---|---|---|
| 1-943 | Ph | 2-MeNH-4-Pym | EtNH-$(CH_2)_4$ |
| 1-944 | Ph | 2-MeNH-4-Pym | $Me_2N$-$CH_2$ |
| 1-945 | Ph | 2-MeNH-4-Pym | $Me_2N$-$(CH_2)_2$ |
| 1-946 | Ph | 2-MeNH-4-Pym | $Me_2N$-$(CH_2)_3$ |
| 1-947 | Ph | 2-MeNH-4-Pym | $Me_2N$-$(CH_2)_4$ |
| 1-948 | Ph | 2-MeNH-4-Pym | 1-Azt-$CH_2$ |
| 1-999 | Ph | 2-MeNH-4-Pym | 1-Azt-$(CH_2)_2$ |
| 1-950 | Ph | 2-MeNH-4-Pym | 1-Azt-$(CH_2)_3$ |
| 1-951 | Ph | 2-MeNH-4-Pym | 1-Azt-$(CH_2)_4$ |
| 1-952 | Ph | 2-MeNH-4-Pym | 1-Pyrd-$CH_2$ |
| 1-953 | Ph | 2-MeNH-4-Pym | 1-Pyrd-$(CH_2)_2$ |
| 1-954 | Ph | 2-MeNH-4-Pym | 1-Pyrd-$(CH_2)_3$ |
| 1-955 | Ph | 2-MeNH-4-Pym | 1-Pyrd-$(CH_2)_4$ |
| 1-956 | Ph | 2-MeNH-4-Pym | 1-Pip-$CH_2$ |
| 1-957 | Ph | 2-MeNH-4-Pym | 1-Pip-$(CH_2)_2$ |
| 1-958 | Ph | 2-MeNH-4-Pym | 1-Pip-$(CH_2)_3$ |
| 1-959 | Ph | 2-MeNH-4-Pym | 1-Pip-$(CH_2)_4$ |
| 1-960 | Ph | 2-MeNH-4-Pym | 1-Mor-$CH_2$ |
| 1-961 | Ph | 2-MeNH-4-Pym | 1-Mor-$(CH_2)_2$ |
| 1-962 | Ph | 2-MeNH-4-Pym | 1-Mor-$(CH_2)_3$ |
| 1-963 | Ph | 2-MeNH-4-Pym | 1-Mor-$(CH_2)_4$ |
| 1-964 | Ph | 2-MeNH-4-Pym | 1-Tmor-$CH_2$ |
| 1-965 | Ph | 2-MeNH-4-Pym | 1-Tmor-$(CH_2)_2$ |
| 1-966 | Ph | 2-MeNH-4-Pym | 1-Tmor-$(CH_2)_3$ |
| 1-967 | Ph | 2-MeNH-4-Pym | 1-Tmor-$(CH_2)_4$ |
| 1-968 | Ph | 2-MeNH-4-Pym | 1-Piz-$CH_2$ |
| 1-969 | Ph | 2-MeNH-4-Pym | 1-Piz-$(CH_2)_2$ |
| 1-970 | Ph | 2-MeNH-4-Pym | 1-Piz-$(CH_2)_3$ |
| 1-971 | Ph | 2-MeNH-4-Pym | 4-Me-1-Piz-$(CH_2)_3$ |
| 1-972 | Ph | 2-MeNH-4-Pym | 1-Piz-$(CH_2)_4$ |
| 1-973 | Ph | 2-MeNH-4-Pym | 3-Azt |
| 1-974 | Ph | 2-MeNH-4-Pym | 1-Me-3-Azt |
| 1-975 | Ph | 2-MeNH-4-Pym | 1-Bn-3-Azt |
| 1-976 | Ph | 2-MeNH-4-Pym | 3-Pyrd |
| 1-977 | Ph | 2-MeNH-4-Pym | 1-Me-3-Pyrd |
| 1-978 | Ph | 2-MeNH-4-Pym | 3-Pip |
| 1-979 | Ph | 2-MeNH-4-Pym | 4-Pip |
| 1-980 | Ph | 2-MeNH-4-Pym | 4-(3,4-deH-Pip) |

Table 1 (continued)

| Compound No. | $R^1$ | $R^2$ | $R^3$ |
|---|---|---|---|
| 1-981 | Ph | 2-MeNH-4-Pym | 1-Me-4-Pip |
| 1-982 | Ph | 2-MeNH-4-Pym | 1-Me-4-(3,4-deH-Pip) |
| 1-983 | Ph | 2-MeNH-4-Pym | 1-Et-4-Pip |
| 1-984 | Ph | 2-MeNH-4-Pym | 1-Bn-4-Pip |
| 1-985 | Ph | 2-MeNH-4-Pym | 3-Hpip |
| 1-986 | Ph | 2-MeNH-4-Pym | 1-Me-3-Hpip |
| 1-987 | Ph | 2-MeNH-4-Pym | 4-Hpip |
| 1-988 | Ph | 2-MeNH-4-Pym | 1-Me-4-Hpip |
| 1-989 | Ph | 2-MeNH-4-Pym | 2-Mor |
| 1-990 | Ph | 2-MeNH-4-Pym | 1-Me-2-Mor |
| 1-991 | Ph | 2-MeNH-4-Pym | 2-Tmor |
| 1-992 | Ph | 2-MeNH-4-Pym | 1-Me-2-Tmor |
| 1-993 | Ph | 2-MeNH-4-Pym | 1-Piz |
| 1-994 | Ph | 2-MeNH-4-Pym | 4-Me-1-Piz |
| 1-995 | Ph | 2-MeNH-4-Pym | 2-Piz |
| 1-996 | Ph | 2-MeNH-4-Pym | 4-Pyr |
| 1-997 | Ph | 2-MeNH-4-Pym | 3-Pyr |
| 1-998 | Ph | 2-MeNH-4-Pym | 2-Pyr |
| 1-999 | Ph | 2-MeNH-4-Pym | 4-Pym |
| 1-1000 | Ph | 2-MeNH-4-Pym | 5-Pym |
| 1-1001 | Ph | 2-MeNH-4-Pym | 2-Pym |
| 1-1002 | Ph | 2-MeNH-4-Pym | 3-Azt-$CH_2$ |
| 1-1003 | Ph | 2-MeNH-4-Pym | 1-Me-3-Azt-$CH_2$ |
| 1-1004 | Ph | 2-MeNH-4-Pym | 3-Pyrd-$CH_2$ |
| 1-1005 | Ph | 2-MeNH-4-Pym | 1-Me-3-Pyrd-$CH_2$ |
| 1-1006 | Ph | 2-MeNH-4-Pym | 4-Pip-$CH_2$ |
| 1-1007 | Ph | 2-MeNH-4-Pym | 1-Me-4-Pip-$CH_2$ |
| 1-1008 | Ph | 2-MeNH-4-Pym | 3-Hpip-$CH_2$ |
| 1-1009 | Ph | 2-MeNH-4-Pym | 1-Me-3-Hpip-$CH_2$ |
| 1-1010 | Ph | 2-MeNH-4-Pym | 4-Hpip-$CH_2$ |
| 1-1011 | Ph | 2-MeNH-4-Pym | 1-Me-4-Hpip-$CH_2$ |
| 1-1012 | Ph | 2-MeNH-4-Pym | 2-Mor-$CH_2$ |
| 1-1013 | Ph | 2-MeNH-4-Pym | 1-Me-2-Mor-$CH_2$ |
| 1-1014 | Ph | 2-MeNH-4-Pym | 2-Tmor-$CH_2$ |
| 1-1015 | Ph | 2-MeNH-4-Pym | 1-Me-2-Tmor-$CH_2$ |
| 1-1016 | Ph | 2-MeNH-4-Pym | 1-Piz-$CH_2$ |
| 1-1017 | Ph | 2-MeNH-4-Pym | 4-Me-1-Piz-$CH_2$ |
| 1-1018 | Ph | 2-MeNH-4-Pym | 2-Piz-$CH_2$ |

Table 1 (continued)

| Compound No. | $R^1$ | $R^2$ | $R^3$ |
|---|---|---|---|
| 1-1019 | Ph | 2-MeNH-4-Pym | 4-Pyr-$CH_2$ |
| 1-1020 | Ph | 2-MeNH-4-Pym | 3-Pyr-$CH_2$ |
| 1-1021 | Ph | 2-MeNH-4-Pym | 2-Pyr-$CH_2$ |
| 1-1022 | Ph | 2-MeNH-4-Pym | 4-Pym-$CH_2$ |
| 1-1023 | Ph | 2-MeNH-4-Pym | 5-Pym-$CH_2$ |
| 1-1024 | Ph | 2-MeNH-4-Pym | 2-Pym-$CH_2$ |
| 1-1025 | 4-F-Ph | 2-MeNH-4-Pym | $H_2N$-$CH_2$ |
| 1-1026 | 4-F-Ph | 2-MeNH-4-Pym | $H_2N$-$(CH_2)_2$ |
| 1-1027 | 4-F-Ph | 2-MeNH-4-Pym | $H_2N$-$(CH_2)_3$ |
| 1-1028 | 4-F-Ph | 2-MeNH-4-Pym | $H_2N$-$(CH_2)_4$ |
| 1-1029 | 4-F-Ph | 2-MeNH-4-Pym | MeNH-$CH_2$ |
| 1-1030 | 4-F-Ph | 2-MeNH-4-Pym | MeNH-$(CH_2)_2$ |
| 1-1031 | 4-F-Ph | 2-MeNH-4-Pym | MeNH-$(CH_2)_3$ |
| 1-1032 | 4-F-Ph | 2-MeNH-4-Pym | MeNH-$(CH_2)_4$ |
| 1-1033 | 4-F-Ph | 2-MeNH-4-Pym | EtNH-$CH_2$ |
| 1-1034 | 4-F-Ph | 2-MeNH-4-Pym | EtNH-$(CH_2)_2$ |
| 1-1035 | 4-F-Ph | 2-MeNH-4-Pym | EtNH-$(CH_2)_3$ |
| 1-1036 | 4-F-Ph | 2-MeNH-4-Pym | EtNH-$(CH_2)_4$ |
| 1-1037 | 4-F-Ph | 2-MeNH-4-Pym | $Me_2N$-$CH_2$ |
| 1-1038 | 4-F-Ph | 2-MeNH-4-Pym | $Me_2N$-$(CH_2)_2$ |
| 1-1039 | 4-F-Ph | 2-MeNH-4-Pym | $Me_2N$-$(CH_2)_3$ |
| 1-1040 | 4-F-Ph | 2-MeNH-4-Pym | $Me_2N$-$(CH_2)_4$ |
| 1-1041 | 4-F-Ph | 2-MeNH-4-Pym | 1-Azt-$CH_2$ |
| 1-1042 | 4-F-Ph | 2-MeNH-4-Pym | 1-Azt-$(CH_2)_2$ |
| 1-1043 | 4-F-Ph | 2-MeNH-4-Pym | 1-Azt-$(CH_2)_3$ |
| 1-1044 | 4-F-Ph | 2-MeNH-4-Pym | 1-Azt-$(CH_2)_4$ |
| 1-1045 | 4-F-Ph | 2-MeNH-4-Pym | 1-Pyrd-$CH_2$ |
| 1-1046 | 4-F-Ph | 2-MeNH-4-Pym | 1-Pyrd-$(CH_2)_2$ |
| 1-1047 | 4-F-Ph | 2-MeNH-4-Pym | 1-Pyrd-$(CH_2)_3$ |
| 1-1048 | 4-F-Ph | 2-MeNH-4-Pym | 1-Pyrd-$(CH_2)_4$ |
| 1-1049 | 4-F-Ph | 2-MeNH-4-Pym | 1-Pip-$CH_2$ |
| 1-1050 | 4-F-Ph | 2-MeNH-4-Pym | 1-Pip-$(CH_2)_2$ |
| 1-1051 | 4-F-Ph | 2-MeNH-4-Pym | 1-Pip-$(CH_2)_3$ |
| 1-1052 | 4-F-Ph | 2-MeNH-4-Pym | 1-Pip-$(CH_2)_4$ |
| 1-1053 | 4-F-Ph | 2-MeNH-4-Pym | 1-Mor-$CH_2$ |
| 1-1054 | 4-F-Ph | 2-MeNH-4-Pym | 1-Mor-$(CH_2)_2$ |
| 1-1055 | 4-F-Ph | 2-MeNH-4-Pym | 1-Mor-$(CH_2)_3$ |
| 1-1056 | 4-F-Ph | 2-MeNH-4-Pym | 1-Mor-$(CH_2)_4$ |

Table 1 (continued)

| Compound No. | $R^1$ | $R^2$ | $R^3$ |
|---|---|---|---|
| 1-1057 | 4-F-Ph | 2-MeNH-4-Pym | 1-Tmor-$CH_2$ |
| 1-1058 | 4-F-Ph | 2-MeNH-4-Pym | 1-Tmor-$(CH_2)_2$ |
| 1-1059 | 4-F-Ph | 2-MeNH-4-Pym | 1-Tmor-$(CH_2)_3$ |
| 1-1060 | 4-F-Ph | 2-MeNH-4-Pym | 1-Tmor-$(CH_2)_4$ |
| 1-1061 | 4-F-Ph | 2-MeN$_{H}$-4-Pym | 1-Piz-$CH_2$ |
| 1-1062 | 4-F-Ph | 2-MeNH-4-Pym | 1-Piz-$(CH_2)_2$ |
| 1-1063 | 4-F-Ph | 2-MeNH-4-Pym | 1-Piz-$(CH_2)_3$ |
| 1-1064 | 4-F-Ph | 2-MeNH-4-Pym | 4-Me-1-Piz-$(CH_2)_3$ |
| 1-1065 | 4-F-Ph | 2-MeNH-4-Pym | 1-Piz-$(CH_2)_4$ |
| 1-1066 | 4-F-Ph | 2-MeNH-4-Pym | 3-Azt |
| 1-1067 | 4-F-Ph | 2-MeNH-4-Pym | 1-Me-3-Azt |
| 1-1068 | 4-F-Ph | 2-MeNH-4-Pym | 1-Bn-3-Azt |
| 1-1069 | 4-F-Ph | 2-MeNH-4-Pym | 3-Pyrd |
| 1-1070 | 4-F-Ph | 2-MeNH-4-Pym | 1-Me-3-Pyrd |
| 1-1071 | 4-F-Ph | 2-MeNH-4-Pym | 3-Pip |
| 1-1072 | 4-F-Ph | 2-MeNH-4-Pym | 4-Pip |
| 1-1073 | 4-F-Ph | 2-MeNH-4-Pym | 4-(3,4-deH-Pip) |
| 1-1074 | 4-F-Ph | 2-MeNH-4-Pym | 1-Me-4-Pip |
| 1-1075 | 4-F-Ph | 2-MeNH-4-Pym | 1-Me-4-(3,4-deH-Pip) |
| 1-1076 | 4-F-Ph | 2-MeNH-4-Pym | 1-Et-4-Pip |
| 1-1077 | 4-F-Ph | 2-MeNH-4-Pym | 1-Bn-4-Pip |
| 1-1078 | 4-F-Ph | 2-MeNH-4-Pym | 3-Hpip |
| 1-1079 | 4-F-Ph | 2-MeNH-4-Pym | 1-Me-3-Hpip |
| 1-1080 | 4-F-Ph | 2-MeNH-4-Pym | 4-Hpip |
| 1-1081 | 4-F-Ph | 2-MeNH-4-Pym | 1-Me-4-Hpip |
| 1-1082 | 4-F-Ph | 2-MeNH-4-Pym | 2-Mor |
| 1-1083 | 4-F-Ph | 2-MeNH-4-Pym | 1-Me-2-Mor |
| 1-1084 | 4-F-Ph | 2-MeNH-4-Pym | 2-Tmor |
| 1-1085 | 4-F-Ph | 2-MeNH-4-Pym | 1-Me-2-Tmor |
| 1-1086 | 4-F-Ph | 2-MeNH-4-Pym | 1-Piz |
| 1-1087 | 4-F-Ph | 2-MeNH-4-Pym | 4-Me-1-Piz |
| 1-1088 | 4-F-Ph | 2-MeNH-4-Pym | 2-Piz |
| 1-1089 | 4-F-Ph | 2-MeNH-4-Pym | 4-Pyr |
| 1-1090 | 4-F-Ph | 2-MeNH-4-Pym | 3-Pyr |
| 1-1091 | 4-F-Ph | 2-MeNH-4-Pym | 2-Pyr |
| 1-1092 | 4-F-Ph | 2-MeNH-4-Pym | 4-Pym |
| 1-1093 | 4-F-Ph | 2-MeNH-4-Pym | 5-Pym |
| 1-1094 | 4-F-Ph | 2-MeNH-4-Pym | 2-Pym |

Table 1 (continued)

| Compound No. | $R^1$ | $R^2$ | $R^3$ |
|---|---|---|---|
| 1-1095 | 4-F-Ph | 2-MeNH-4-Pym | 3-Azt-$CH_2$ |
| 1-1096 | 4-F-Ph | 2-MeNH-4-Pym | 1-Me-3-Azt-$CH_2$ |
| 1-1097 | 4-F-Ph | 2-MeNH-4-Pym | 3-Pyrd-$CH_2$ |
| 1-1098 | 4-F-Ph | 2-MeNH-4-Pym | 1-Me-3-Pyrd-$CH_2$ |
| 1-1099 | 4-F-Ph | 2-MeNH-4-Pym | 4-Pip-$CH_2$ |
| 1-1100 | 4-F-Ph | 2-MeNH-4-Pym | 1-Me-4-Pip-$CH_2$ |
| 1-1101 | 4-F-Ph | 2-MeNH-4-Pym | 3-Hpip-$CH_2$ |
| 1-1102 | 4-F-Ph | 2-MeNH-4-Pym | 1-Me-3-Hpip-$CH_2$ |
| 1-1103 | 4-F-Ph | 2-MeNH-4-Pym | 4-Hpip-$CH_2$ |
| 1-1104 | 4-F-Ph | 2-MeNH-4-Pym | 1-Me-4-Hpip-$CH_2$ |
| 1-1105 | 4-F-Ph. | 2-MeNH-4-Pym | 2-Mor-$CH_2$ |
| 1-1106 | 4-F-Ph | 2-MeNH-4-Pym | 1-Me-2-Mor-$CH_2$ |
| 1-1107 | 4-F-Ph | 2-MeNH-4-Pym | 2-Tmor-$CH_2$ |
| 1-1108 | 4-F-Ph | 2-MeNH-4-Pym | 1-Me-2-Tmor-$CH_2$ |
| 1-1109 | 4-F-Ph | 2-MeNH-4-Pym | 1-Piz-$CH_2$ |
| 1-1110 | 4-F-Ph | 2-MeNH-4-Pym | 4-Me-1-Piz-$CH_2$ |
| 1-1111 | 4-F-Ph | 2-MeNH-4-Pym | 2-Piz-$CH_2$ |
| 1-1112 | 4-F-Ph | 2-MeNH-4-Pym | 4-Pyr-$CH_2$ |
| 1-1113 | 4-F-Ph | 2-MeNH-4-Pym | 3-Pyr-$CH_2$ |
| 1-1114 | 4-F-Ph | 2-MeNH-4-Pym | 2-Pyr-$CH_2$ |
| 1-1115 | 4-F-Ph | 2-MeNH-4-Pym | 4-Pym-$CH_2$ |
| 1-1116 | 4-F-Ph | 2-MeNH-4-Pym | 5-Pym-$CH_2$ |
| 1-1117 | 4-F-Ph | 2-MeNH-4-Pym | 2-Pym-$CH_2$ |
| 1-1118 | 3-F-Ph | 2-MeNH-4-Pym | $H_2N$-$CH_2$ |
| 1-1119 | 3-F-Ph | 2-MeNH-4-Pym | $H_2N$-$(CH_2)_2$ |
| 1-1120 | 3-F-Ph | 2-MeNH-4-Pym | $H_2N$-$(CH_2)_3$ |
| 1-1121 | 3-F-Ph | 2-MeNH-4-Pym | $H_2N$-$(CH_2)_4$ |
| 1-1122 | 3-F-Ph | 2-MeNH-4-Pym | MeNH-$CH_2$ |
| 1-1123 | 3-F-Ph | 2-MeNH-4-Pym | MeNH-$(CH_2)_2$ |
| 1-1124 | 3-F-Ph | 2-MeNH-4-Pym | MeNH-$(CH_2)_3$ |
| 1-1125 | 3-F-Ph | 2-MeNH-4-Pym | MeNH-$(CH_2)_4$ |
| 1-1126 | 3-F-Ph | 2-MeNH-4-Pym | EtNH-$CH_2$ |
| 1-1127 | 3-F-Ph | 2-MeNH-4-Pym | EtNH-$(CH_2)_2$ |
| 1-1128 | 3-F-Ph | 2-MeNH-4-Pym | EtNH-$(CH_2)_3$ |
| 1-1129 | 3-F-Ph | 2-MeNH-4-Pym | EtNH-$(CH_2)_4$ |
| 1-1130 | 3-F-Ph | 2-MeNH-4-Pym | $Me_2N$-$CH_2$ |
| 1-1131 | 3-F-Ph | 2-MeNH-4-Pym | $Me_2N$-$(CH_2)_2$ |
| 1-1132 | 3-F-Ph | 2-MeNH-4-Pym | $Me_2N$-$(CH_2)_3$ |

Table 1 (continued)

| Compound No. | $R^1$ | $R^2$ | $R^3$ |
|---|---|---|---|
| 1-1133 | 3-F-Ph | 2-MeNH-4-Pym | $Me_2N-(CH_2)_4$ |
| 1-1134 | 3-F-Ph | 2-MeNH-4-Pym | 1-Azt-$CH_2$ |
| 1-1135 | 3-F-Ph | 2-MeNH-4-Pym | 1-Azt-$(CH_2)_2$ |
| 1-1136 | 3-F-Ph | 2-MeNH-4-Pym | 1-Azt-$(CH_2)_3$ |
| 1-1137 | 3-F-Ph | 2-MeNH-4-Pym | 1-Azt-$(CH_2)_4$ |
| 1-1138 | 3-F-Ph | 2-MeNH-4-Pym | 1-Pyrd-$CH_2$ |
| 1-1139 | 3-F-Ph | 2-MeNH-4-Pym | 1-Pyrd-$(CH_2)_2$ |
| 1-1140 | 3-F-Ph | 2-MeNH-4-Pym | 1-Pyrd-$(CH_2)_3$ |
| 1-1141 | 3-F-Ph | 2-MeNH-4-Pym | 1-Pyrd-$(CH_2)_4$ |
| 1-1142 | 3-F-Ph | 2-MeNH-4-Pym | 1-Pip-$CH_2$ |
| 1-1143 | 3-F-Ph | 2-MeNH-4-Pym | 1-Pip-$(CH_2)_2$ |
| 1-1144 | 3-F-Ph | 2-MeNH-4-Pym | 1-Pip-$(CH_2)_3$ |
| 1-1145 | 3-F-Ph | 2-MeNH-4-Pym | 1-Pip-$(CH_2)_4$ |
| 1-1146 | 3-F-Ph | 2-MeNH-4-Pym | 1-Mor-$CH_2$ |
| 1-1147 | 3-F-Ph | 2-MeNH-4-Pym | 1-Mor-$(CH_2)_2$ |
| 1-1148 | 3-F-Ph | 2-MeNH-4-Pym | 1-Mor-$(CH_2)_3$ |
| 1-1149 | 3-F-Ph | 2-MeNH-4-Pym | 1-Mor-$(CH_2)_4$ |
| 1-1150 | 3-F-Ph | 2-MeNH-4-Pym | 1-Tmor-$CH_2$ |
| 1-1151 | 3-F-Ph | 2-MeNH-4-Pym | 1-Tmor-$(CH_2)_2$ |
| 1-1152 | 3-F-Ph | 2-MeNH-4-Pym | 1-Tmor-$(CH_2)_3$ |
| 1-1153 | 3-F-Ph | 2-MeNH-4-Pym | 1-Tmor-$(CH_2)_4$ |
| 1-1154 | 3-F-Ph | 2-MeNH-4-Pym | 1-Piz-$CH_2$ |
| 1-1155 | 3-F-Ph | 2-MeNH-4-Pym | 1-Piz-$(CH_2)_2$ |
| 1-1156 | 3-F-Ph | 2-MeNH-4-Pym | 1-Piz-$(CH_2)_3$ |
| 1-1157 | 3-F-Ph | 2-MeNH-4-Pym | 4-Me-1-Piz-$(CH_2)_3$ |
| 1-1158 | 3-F-Ph | 2-MeNH-4-Pym | 1-Piz-$(CH_2)_4$ |
| 1-1159 | 3-F-Ph | 2-MeNH-4-Pym | 3-Azt |
| 1-1160 | 3-F-Ph | 2-MeNH-4-Pym | 1-Me-3-Azt |
| 1-1161 | 3-F-Ph | 2-MeNH-4-Pym | 1-Bn-3-Azt |
| 1-1162 | 3-F-Ph | 2-MeNH-4-Pym | 3-Pyrd |
| 1-1163 | 3-F-Ph | 2-MeNH-4-Pym | 1-Me-3-Pyrd |
| 1-1164 | 3-F-Ph | 2-MeNH-4-Pym | 3-Pip |
| 1-1165 | 3-F-Ph | 2-MeNH-4-Pym | 4-Pip |
| 1-1166 | 3-F-Ph | 2-MeNH-4-Pym | 4-(3,4-deH-Pip) |
| 1-1167 | 3-F-Ph | 2-MeNH-4-Pym | 1-Me-4-Pip |
| 1-1168 | 3-F-Ph | 2-MeNH-4-Pym | 1-Me-4-(3,4-deH-Pip) |
| 1-1169 | 3-F-Ph | 2-MeNH-4-Pym | 1-Et-4-Pip |
| 1-1170 | 3-F-Ph | 2-MeNH-4-Pym | 1-Bn-4-Pip |

Table 1 (continued)

| Compound No. | $R^1$ | $R^2$ | $R^3$ |
|---|---|---|---|
| 1-1171 | 3-F-Ph | 2-MeNH-4-Pym | 3-Hpip |
| 1-1172 | 3-F-Ph | 2-MeNH-4-Pym | 1-Me-3-Hpip |
| 1-1173 | 3-F-Ph | 2-MeNH-4-Pym | 4-Hpip |
| 1-1174 | 3-F-Ph | 2-MeNH-4-Pym | 1-Me-4-Hpip |
| 1-1175 | 3-F-Ph | 2-MeNH-4-Pym | 2-Mor |
| 1-1176 | 3-F-Ph | 2-MeNH-4-Pym | 1-Me-2-Mor |
| 1-1177 | 3-F-Ph | 2-MeNH-4-Pym | 2-Tmor |
| 1-1178 | 3-F-Ph | 2-MeNH-4-Pym | 1-Me-2-Tmor |
| 1-1179 | 3-F-Ph | 2-MeNH-4-Pym | 1-Piz |
| 1-1180 | 3-F-Ph | 2-MeNH-4-Pym | 4-Me-1-Piz |
| 1-1181 | 3-F-Ph | 2-MeNH-4-Pym | 2-Piz |
| 1-1182 | 3-F-Ph | 2-MeNH-4-Pym | 4-Pyr |
| 1-1183 | 3-F-Ph | 2-MeNH-4-Pym | 3-Pyr |
| 1-1184 | 3-F-Ph | 2-MeNH-4-Pym | 2-Pyr |
| 1-1185 | 3-F-Ph | 2-MeNH-4-Pym | 4-Pym |
| 1-1186 | 3-F-Ph | 2-MeNH-4-Pym | 5-Pym |
| 1-1187 | 3-F-Ph | 2-MeNH-4-Pym | 2-Pym |
| 1-1188 | 3-F-Ph | 2-MeNH-4-Pym | 3-Azt-$CH_2$ |
| 1-1189 | 3-F-Ph | 2-MeNH-4-Pym | 1-Me-3-Azt-$CH_2$ |
| 1-1190 | 3-F-Ph | 2-MeNH-4-Pym | 3-Pyrd-$CH_2$ |
| 1-1191 | 3-F-Ph | 2-MeNH-4-Pym | 1-Me-3-Pyrd-$CH_2$ |
| 1-1192 | 3-F-Ph | 2-MeNH-4-Pym | 4-Pip-$CH_2$ |
| 1-1193 | 3-F-Ph | 2-MeNH-4-Pym | 1-Me-4-Pip-$CH_2$ |
| 1-1194 | 3-F-Ph | 2-MeNH-4-Pym | 3-Hpip-$CH_2$ |
| 1-1195 | 3-F-Ph | 2-MeNH-4-Pym | 1-Me-3-Hpip-$CH_2$ |
| 1-1196 | 3-F-Ph | 2-MeNH-4-Pym | 4-Hpip-$CH_2$ |
| 1-1197 | 3-F-Ph | 2-MeNH-4-Pym | 1-Me-4-Hpip-$CH_2$ |
| 1-1198 | 3-F-Ph | 2-MeNH-4-Pym | 2-Mor-$CH_2$ |
| 1-1199 | 3-F-Ph | 2-MeNH-4-Pym | 1-Me-2-Mor-$CH_2$ |
| 1-1200 | 3-F-Ph | 2-MeNH-4-Pym | 2-Tmor-$CH_2$ |
| 1-1201 | 3-F-Ph | 2-MeNH-4-Pym | 1-Me-2-Tmor-$CH_2$ |
| 1-1202 | 3-F-Ph | 2-MeNH-4-Pym | 1-Piz-$CH_2$ |
| 1-1203 | 3-F-Ph | 2-MeNH-4-Pym | 4-Me-1-Piz-$CH_2$ |
| 1-1204 | 3-F-Ph | 2-MeNH-4-Pym | 2-Piz-$CH_2$ |
| 1-1205 | 3-F-Ph | 2-MeNH-4-Pym | 4-Pyr-$CH_2$ |
| 1-1206 | 3-F-Ph | 2-MeNH-4-Pym | 3-Pyr-$CH_2$ |
| 1-1207 | 3-F-Ph | 2-MeNH-4-Pym | 2-Pyr-$CH_2$ |
| 1-1208 | 3-F-Ph | 2-MeNH-4-Pym | 4-Pym-$CH_2$ |

Table 1 (continued)

| Compound No. | $R^1$ | $R^2$ | $R^3$ |
|---|---|---|---|
| 1-1209 | 3-F-Ph | 2-MeNH-4-Pym | 5-Pym-$CH_2$ |
| 1-1210 | 3-F-Ph | 2-MeNH-4-Pym | 2-Pym-$CH_2$ |
| 1-1211 | 3,4-diF-Ph | 2-MeNH-4-Pym | $H_2N$-$CH_2$ |
| 1-1212 | 3,4-diF-Ph | 2-MeNH-4-Pym | $H_2N$-$(CH_2)_2$ |
| 1-1213 | 3,4-diF-Ph | 2-MeNH-4-Pym | $H_2N$-$(CH_2)_3$ |
| 1-1214 | 3,4-diF-Ph | 2-MeNH-4-Pym | $H_2N$-$(CH_2)_4$ |
| 1-1215 | 3,4-diF-Ph | 2-MeNH-4-Pym | MeNH-$CH_2$ |
| 1-1216 | 3,4-diF-Ph | 2-MeNH-4-Pym | MeNH-$(CH_2)_2$ |
| 1-1217 | 3,4-diF-Ph | 2-MeNH-4-Pym | MeNH-$(CH_2)_3$ |
| 1-1218 | 3,4-diF-Ph | 2-MeNH-4-Pym | MeNH-$(CH_2)_4$ |
| 1-1219 | 3,4-diF-Ph | 2-MeNH-4-Pym | EtNH-$CH_2$ |
| 1-1220 | 3,4-diF-Ph | 2-MeNH-4-Pym | EtNH-$(CH_2)_2$ |
| 1-1221 | 3,4-diF-Ph | 2-MeNH-4-Pym | EtNH-$(CH_2)_3$ |
| 1-1222 | 3,4-diF-Ph | 2-MeNH-4-Pym | EtNH-$(CH_2)_4$ |
| 1-1223 | 3,4-diF-Ph | 2-MeNH-4-Pym | $Me_2N$-$CH_2$ |
| 1-1224 | 3,4-diF-Ph | 2-MeNH-4-Pym | $Me_2N$-$(CH_2)_2$ |
| 1-1225 | 3,4-diF-Ph | 2-MeNH-4-Pym | $Me_2N$-$(CH_2)_3$ |
| 1-1226 | 3,4-diF-Ph | 2-MeNH-4-Pym | $Me_2N$-$(CH_2)_4$ |
| 1-1227 | 3,4-diF-Ph | 2-MeNH-4-Pym | 1-Azt-$CH_2$ |
| 1-1228 | 3,4-diF-Ph | 2-MeNH-4-Pym | 1-Azt-$(CH_2)_2$ |
| 1-1229 | 3,4-diF-Ph | 2-MeNH-4-Pym | 1-Azt-$(CH_2)_3$ |
| 1-1230 | 3,4-diF-Ph | 2-MeNH-4-Pym | 1-Azt-$(CH_2)_4$ |
| 1-1231 | 3,4-diF-Ph | 2-MeNH-4-Pym | 1-Pyrd-$CH_2$ |
| 1-1232 | 3,4-diF-Ph | 2-MeNH-4-Pym | 1-Pyrd-$(CH_2)_2$ |
| 1-1233 | 3,4-diF-Ph | 2-MeNH-4-Pym | 1-Pyrd-$(CH_2)_3$ |
| 1-1234 | 3,4-diF-Ph | 2-MeNH-4-Pym | 1-Pyrd-$(CH_2)_4$ |
| 1-1235 | 3,4-diF-Ph | 2-MeNH-4-Pym | 1-Pip-$CH_2$ |
| 1-1236 | 3,4-diF-Ph | 2-MeNH-4-Pym | 1-Pip-$(CH_2)_2$ |
| 1-1237 | 3,4-diF-Ph | 2-MeNH-4-Pym | 1-Pip-$(CH_2)_3$ |
| 1-1238 | 3,4-diF-Ph | 2-MeNH-4-Pym | 1-Pip-$(CH_2)_4$ |
| 1-1239 | 3,4-diF-Ph | 2-MeNH-4-Pym | 1-Mor-$CH_2$ |
| 1-1240 | 3,4-diF-Ph | 2-MeNH-4-Pym | 1-Mor-$(CH_2)_2$ |
| 1-1241 | 3,4-diF-Ph | 2-MeNH-4-Pym | 1-Mor-$(CH_2)_3$ |
| 1-1242 | 3,4-diF-Ph | 2-MeNH-4-Pym | 1-Mor-$(CH_2)_4$ |
| 1-1243 | 3,4-diF-Ph | 2-MeNH-4-Pym | 1-Tmor-$CH_2$ |
| 1-1244 | 3,4-diF-Ph | 2-MeNH-4-Pym | 1-Tmor-$(CH_2)_2$ |
| 1-1245 | 3,4-diF-Ph | 2-MeNH-4-Pym | 1-Tmor-$(CH_2)_3$ |
| 1-1246 | 3,4-diF-Ph | 2-MeNH-4-Pym | 1-Tmor-$(CH_2)_4$ |

Table 1 (continued)

| Compound No. | $R^1$ | $R^2$ | $R^3$ |
|---|---|---|---|
| 1-1247 | 3,4-diF-Ph | 2-MeNH-4-Pym | 1-Piz-$CH_2$ |
| 1-1248 | 3,4-diF-Ph | 2-MeNH-4-Pym | 1-Piz-$(CH_2)_2$ |
| 1-1249 | 3,4-diF-Ph | 2-MeNH-4-Pym | 1-Piz-$(CH_2)_3$ |
| 1-1250 | 3,4-diF-Ph | 2-MeNH-4-Pym | 4-Me-1-Piz-$(CH_2)_3$ |
| 1-1251 | 3,4-diF-Ph | 2-MeNH-4-Pym | 1-Piz-$(CH_2)_4$ |
| 1-1252 | 3,4-diF-Ph | 2-MeNH-4-Pym | 3-Azt |
| 1-1253 | 3,4-diF-Ph | 2-MeNH-4-Pym | 1-Me-3-Azt |
| 1-1254 | 3,4-diF-Ph | 2-MeNH-4-Pym | 1-Bn-3-Azt |
| 1-1255 | 3,4-diF-Ph | 2-MeNH-4-Pym | 3-Pyrd |
| 1-1256 | 3,4-diF-Ph | 2-MeNH-4-Pym | 1-Me-3-Pyrd |
| 1-1257 | 3,4-diF-Ph | 2-MeNH-4-Pym | 3-Pip |
| 1-1258 | 3,4-diF-Ph | 2-MeNH-4-Pym | 4-Pip |
| 1-1259 | 3,4-diF-Ph | 2-MeNH-4-Pym | 4-(3,4-deH-Pip) |
| 1-1260 | 3,4-diF-Ph | 2-MeNH-4-Pym | 1-Me-4-Pip |
| 1-1261 | 3,4-diF-Ph | 2-MeNH-4-Pym | 1-Me-4-(3,4-deH-Pip) |
| 1-1262 | 3,4-diF-Ph | 2-MeNH-4-Pym | 1-Et-4-Pip |
| 1-1263 | 3,4-diF-Ph | 2-MeNH-4-Pym | 1-Bn-4-Pip |
| 1-1264 | 3,4-diF-Ph | 2-MeNH-4-Pym | 3-Hpip |
| 1-1265 | 3,4-diF-Ph | 2-MeNH-4-Pym | 1-Me-3-Hpip |
| 1-1266 | 3,4-diF-Ph | 2-MeNH-4-Pym | 4-Hpip |
| 1-1267 | 3,4-diF-Ph | 2-MeNH-4-Pym | 1-Me-4-Hpip |
| 1-1268 | 3,4-diF-Ph | 2-MeNH-4-Pym | 2-Mor |
| 1-1269 | 3,4-diF-Ph | 2-MeNH-4-Pym | 1-Me-2-Mor |
| 1-1270 | 3,4-diF-Ph | 2-MeNH-4-Pym | 2-Tmor |
| 1-1271 | 3,4-diF-Ph | 2-MeNH-4-Pym | 1-Me-2-Tmor |
| 1-1272 | 3,4-diF-Ph | 2-MeNH-4-Pym | 1-Piz |
| 1-1273 | 3,4-diF-Ph | 2-MeNH-4-Pym | 4-Me-1-Piz |
| 1-1274 | 3,4-diF-Ph | 2-MeNH-4-Pym | 2-Piz |
| 1-1275 | 3,4-diF-Ph | 2-MeNH-4-Pym | 4-Pyr |
| 1-1276 | 3,4-diF-Ph | 2-MeNH-4-Pym | 3-Pyr |
| 1-1277 | 3,4-diF-Ph | 2-MeNH-4-Pym | 2-Pyr |
| 1-1278 | 3,4-diF-Ph | 2-MeNH-4-Pym | 4-Pym |
| 1-1279 | 3,4-diF-Ph | 2-MeNH-4-Pym | 5-Pym |
| 1-1280 | 3,4-diF-Ph | 2-MeNH-4-Pym | 2-Pym |
| 1-1281 | 3,4-diF-Ph | 2-MeNH-4-Pym | 3-Azt-$CH_2$ |
| 1-1282 | 3,4-diF-Ph | 2-MeNH-4-Pym | 1-Me-3-Azt-$CH_2$ |
| 1-1283 | 3,4-diF-Ph | 2-MeNH-4-Pym | 3-Pyrd-$CH_2$ |
| 1-1284 | 3,4-diF-Ph | 2-MeNH-4-Pym | 1-Me-3-Pyrd-$CH_2$ |

Table 1 (continued)

| Compound No. | $R^1$ | $R^2$ | $R^3$ |
|---|---|---|---|
| 1-1285 | 3,4-diF-Ph | 2-MeNH-4-Pym | 4-Pip-$CH_2$ |
| 1-1286 | 3,4-diF-Ph | 2-MeNH-4-Pym | 1-Me-4-Pip-$CH_2$ |
| 1-1287 | 3,4-diF-Ph | 2-MeNH-4-Pym | 3-Hpip-$CH_2$ |
| 1-1288 | 3,4-diF-Ph | 2-MeNH-4-Pym | 1-Me-3-Hpip-$CH_2$ |
| 1-1289 | 3,4-diF-Ph | 2-MeNH-4-Pym | 4-Hpip-$CH_2$ |
| 1-1290 | 3,4-diF-Ph | 2-MeNH-4-Pym | 1-Me-4-Hpip-$CH_2$ |
| 1-1291 | 3,4-diF-Ph | 2-MeNH-4-Pym | 2-Mor-$CH_2$ |
| 1-1292 | 3,4-diF-Ph | 2-MeNH-4-Pym | 1-Me-2-Mor-$CH_2$ |
| 1-1293 | 3,4-diF-Ph | 2-MeNH-4-Pym | 2-Tmor-$CH_2$ |
| 1-1294 | 3,4-diF-Ph | 2-MeNH-4-Pym | 1-Me-2-Tmor-$CH_2$ |
| 1-1295 | 3,4-diF-Ph | 2-MeNH-4-Pym | 1-Piz-$CH_2$ |
| 1-1296 | 3,4-diF-Ph | 2-MeNH-4-Pym | 4-Me-1-Piz-$CH_2$ |
| 1-1297 | 3,4-diF-Ph | 2-MeNH-4-Pym | 2-Piz-$CH_2$ |
| 1-1298 | 3,4-diF-Ph | 2-MeNH-4-Pym | 4-Pyr-$CH_2$ |
| 1-1299 | 3,4-diF-Ph | 2-MeNH-4-Pym | 3-Pyr-$CH_2$ |
| 1-1300 | 3,4-diF-Ph | 2-MeNH-4-Pym | 2-Pyr-$CH_2$ |
| 1-1301 | 3,4-diF-Ph | 2-MeNH-4-Pym | 4-Pym-$CH_2$ |
| 1-1302 | 3,4-diF-Ph | 2-MeNH-4-Pym | 5-Pym-$CH_2$ |
| 1-1303 | 3,4-diF-Ph | 2-MeNH-4-Pym | 2-Pym-$CH_2$ |
| 1-1304 | 3-Cl-Ph | 2-MeNH-4-Pym | $H_2N$-$CH_2$ |
| 1-1305 | 3-Cl-Ph | 2-MeNH-4-Pym | $H_2N$-$(CH_2)_2$ |
| 1-1306 | 3-Cl-Ph | 2-MeNH-4-Pym | $H_2N$-$(CH_2)_3$ |
| 1-1307 | 3-Cl-Ph | 2-MeNH-4-Pym | $H_2N$-$(CH_2)_4$ |
| 1-1308 | 3-Cl-Ph | 2-MeNH-4-Pym | MeNH-$CH_2$ |
| 1-1309 | 3-Cl-Ph | 2-MeNH-4-Pym | MeNH-$(CH_2)_2$ |
| 1-1310 | 3-Cl-Ph | 2-MeNH-4-Pym | MeNH-$(CH_2)_3$ |
| 1-1311 | 3-Cl-Ph | 2-MeNH-4-Pym | MeNH-$(CH_2)_4$ |
| 1-1312 | 3-Cl-Ph | 2-MeNH-4-Pym | EtNH-$CH_2$ |
| 1-1313 | 3-Cl-Ph | 2-MeNH-4-Pym | EtNH-$(CH_2)_2$ |
| 1-1314 | 3-Cl-Ph | 2-MeNH-4-Pym | EtNH-$(CH_2)_3$ |
| 1-1315 | 3-Cl-Ph | 2-MeNH-4-Pym | EtNH-$(CH_2)_4$ |
| 1-1316 | 3-Cl-Ph | 2-MeNH-4-Pym | $Me_2N$-$CH_2$ |
| 1-1317 | 3-Cl-Ph | 2-MeNH-4-Pym | $Me_2N$-$(CH_2)_2$ |
| 1-1318 | 3-Cl-Ph | 2-MeNH-4-Pym | $Me_2N$-$(CH_2)_3$ |
| 1-1319 | 3-Cl-Ph | 2-MeNH-4-Pym | $Me_2N$-$(CH_2)_4$ |
| 1-1320 | 3-Cl-Ph | 2-MeNH-4-Pym | 1-Azt-$CH_2$ |
| 1-1321 | 3-Cl-Ph | 2-MeNH-4-Pym | 1-Azt-$(CH_2)_2$ |
| 1-1322 | 3-Cl-Ph | 2-MeNH-4-Pym | 1-Azt-$(CH_2)_3$ |

Table 1 (continued)

| Compound No. | $R^1$ | $R^2$ | $R^3$ |
|---|---|---|---|
| 1-1323 | 3-Cl-Ph | 2-MeNH-4-Pym | 1-Azt-$(CH_2)_4$ |
| 1-1324 | 3-Cl-Ph | 2-MeNH-4-Pym | 1-Pyrd-$CH_2$ |
| 1-1325 | 3-Cl-Ph | 2-MeNH-4-Pym | 1-Pyrd-$(CH_2)_2$ |
| 1-1326 | 3-Cl-Ph | 2-MeNH-4-Pym | 1-Pyrd-$(CH_2)_3$ |
| 1-1327 | 3-Cl-Ph | 2-MeNH-4-Pym | 1-Pyrd-$(CH_2)_4$ |
| 1-1328 | 3-Cl-Ph | 2-MeNH-4-Pym | 1-Pip-$CH_2$ |
| 1-1329 | 3-Cl-Ph | 2-MeNH-4-Pym | 1-Pip-$(CH_2)_2$ |
| 1-1330 | 3-Cl-Ph | 2-MeNH-4-Pym | 1-Pip-$(CH_2)_3$ |
| 1-1331 | 3-Cl-Ph | 2-MeNH-4-Pym | 1-Pip-$(CH_2)_4$ |
| 1-1332 | 3-Cl-Ph | 2-MeNH-4-Pym | 1-Mor-$CH_2$ |
| 1-1333 | 3-Cl-Ph | 2-MeNH-4-Pym | 1-Mor-$(CH_2)_2$ |
| 1-1334 | 3-Cl-Ph | 2-MeNH-4-Pym | 1-Mor-$(CH_2)_3$ |
| 1-1335 | 3-Cl-Ph | 2-MeNH-4-Pym | 1-Mor-$(CH_2)_4$ |
| 1-1336 | 3-Cl-Ph | 2-MeNH-4-Pym | 1-Tmor-$CH_2$ |
| 1-1337 | 3-Cl-Ph | 2-MeNH-4-Pym | 1-Tmor-$(CH_2)_2$ |
| 1-1338 | 3-Cl-Ph | 2-MeNH-4-Pym | 1-Tmor-$(CH_2)_3$ |
| 1-1339 | 3-Cl-Ph | 2-MeNH-4-Pym | 1-Tmor-$(CH_2)_4$ |
| 1-1340 | 3-Cl-Ph | 2-MeNH-4-Pym | 1-Piz-$CH_2$ |
| 1-1341 | 3-Cl-Ph | 2-MeNH-4-Pym | 1-Piz-$(CH_2)_2$ |
| 1-1342 | 3-Cl-Ph | 2-MeNH-4-Pym | 1-Piz-$(CH_2)_3$ |
| 1-1343 | 3-Cl-Ph | 2-MeNH-4-Pym | 4-Me-1-Piz-$(CH_2)_3$ |
| 1-1344 | 3-Cl-Ph | 2-MeNH-4-Pym | 1-Piz-$(CH_2)_4$ |
| 1-1345 | 3-Cl-Ph | 2-MeNH-4-Pym | 3-Azt |
| 1-1346 | 3-Cl-Ph | 2-MeNH-4-Pym | 1-Me-3-Azt |
| 1-1347 | 3-Cl-Ph | 2-MeNH-4-Pym | 1-Bn-3-Azt |
| 1-1348 | 3-Cl-Ph | 2-MeNH-4-Pym | 3-Pyrd |
| 1-1349 | 3-Cl-Ph | 2-MeNH-4-Pym | 1-Me-3-Pyrd |
| 1-1350 | 3-Cl-Ph | 2-MeNH-4-Pym | 3-Pip |
| 1-1351 | 3-Cl-Ph | 2-MeNH-4-Pym | 4-Pip |
| 1-1352 | 3-Cl-Ph | 2-MeNH-4-Pym | 4-(3,4-deH-Pip) |
| 1-1353 | 3-Cl-Ph | 2-MeNH-4-Pym | 1-Me-4-Pip |
| 1-1354 | 3-Cl-Ph | 2-MeNH-4-Pym | 1-Me-4-(3,4-deH-Pip) |
| 1-1355 | 3-Cl-Ph | 2-MeNH-4-Pym | 1-Et-4-Pip |
| 1-1356 | 3-Cl-Ph | 2-MeNH-4-Pym | 1-Bn-4-Pip |
| 1-1357 | 3-Cl-Ph | 2-MeNH-4-Pym | 3-Hpip |
| 1-1358 | 3-Cl-Ph | 2-MeNH-4-Pym | 1-Me-3-Hpip |
| 1-1359 | 3-Cl-Ph | 2-MeNH-4-Pym | 4-Hpip |
| 1-1360 | 3-Cl-Ph | 2-MeNH-4-Pym | 1-Me-4-Hpip |

Table 1 (continued)

| Compound No. | $R^1$ | $R^2$ | $R^3$ |
|---|---|---|---|
| 1-1361 | 3-Cl-Ph | 2-MeNH-4-Pym | 2-Mor |
| 1-1362 | 3-Cl-Ph | 2-MeNH-4-Pym | 1-Me-2-Mor |
| 1-1363 | 3-Cl-Ph | 2-MeNH-4-Pym | 2-Tmor |
| 1-1364 | 3-Cl-Ph | 2-MeNH-4-Pym | 1-Me-2-Tmor |
| 1-1365 | 3-Cl-Ph | 2-MeNH-4-Pym | 1-Piz |
| 1-1366 | 3-Cl-Ph | 2-MeNH-4-Pym | 4-Me-1-Piz |
| 1-1367 | 3-Cl-Ph | 2-MeNH-4-Pym | 2-Piz |
| 1-1368 | 3-Cl-Ph | 2-MeNH-4-Pym | 4-Pyr |
| 1-1369 | 3-Cl-Ph | 2-MeNH-4-Pym | 3-Pyr |
| 1-1370 | 3-Cl-Ph | 2-MeNH-4-Pym | 2-Pyr |
| 1-1371 | 3-Cl-Ph | 2-MeNH-4-Pym | 4-Pym |
| 1-1372 | 3-Cl-Ph | 2-MeNH-4-Pym | 5-Pym |
| 1-1373 | 3-Cl-Ph | 2-MeNH-4-Pym | 2-Pym |
| 1-1374 | 3-Cl-Ph | 2-MeNH-4-Pym | 3-Azt-$CH_2$ |
| 1-1375 | 3-Cl-Ph | 2-MeNH-4-Pym | 1-Me-3-Azt-$CH_2$ |
| 1-1376 | 3-Cl-Ph | 2-MeNH-4-Pym | 3-Pyrd-$CH_2$ |
| 1-1377 | 3-Cl-Ph | 2-MeNH-4-Pym | 1-Me-3-Pyrd-$CH_2$ |
| 1-1378 | 3-Cl-Ph | 2-MeNH-4-Pym | 4-Pip-$CH_2$ |
| 1-1379 | 3-Cl-Ph | 2-MeNH-4-Pym | 1-Me-4-Pip-$CH_2$ |
| 1-1380 | 3-Cl-Ph | 2-MeNH-4-Pym | 3-Hpip-$CH_2$ |
| 1-1381 | 3-Cl-Ph | 2-MeNH-4-Pym | 1-Me-3-Hpip-$CH_2$ |
| 1-1382 | 3-Cl-Ph | 2-MeNH-4-Pym | 4-Hpip-$CH_2$ |
| 1-1383 | 3-Cl-Ph | 2-MeNH-4-Pym | 1-Me-4-Hpip-$CH_2$ |
| 1-1384 | 3-Cl-Ph | 2-MeNH-4-Pym | 2-Mor-$CH_2$ |
| 1-1385 | 3-Cl-Ph | 2-MeNH-4-Pym | 1-Me-2-Mor-$CH_2$ |
| 1-1386 | 3-Cl-Ph | 2-MeNH-4-Pym | 2-Tmor-$CH_2$ |
| 1-1387 | 3-Cl-Ph | 2-MeNH-4-Pym | 1-Me-2-Tmor-$CH_2$ |
| 1-1388 | 3-Cl-Ph | 2-MeNH-4-Pym | 1-Piz-$CH_2$ |
| 1-1389 | 3-Cl-Ph | 2-MeNH-4-Pym | 4-Me-1-Piz-$CH_2$ |
| 1-1390 | 3-Cl-Ph | 2-MeNH-4-Pym | 2-Piz-$CH_2$ |
| 1-1391 | 3-Cl-Ph | 2-MeNH-4-Pym | 4-Pyr-$CH_2$ |
| 1-1392 | 3-Cl-Ph | 2-NeNH-4-Pym | 3-Pyr-$CH_2$ |
| 1-1393 | 3-Cl-Ph | 2-MeNH-4-Pym | 2-Pyr-$CH_2$ |
| 1-1394 | 3-Cl-Ph | 2-MeNH-4-Pym | 4-Pym-$CH_2$ |
| 1-1395 | 3-Cl-Ph | 2-MeNH-4-Pym | 5-Pym-$CH_2$ |
| 1-1396 | 3-Cl-Ph | 2-MeNH-4-Pym | 2-Pym-$CH_2$ |
| 1-1397 | 3-Cl-4-F-Ph | 4-Pyr | $H_2N-(CH_2)_3$ |
| 1-1398 | 3-Cl-4-F-Ph | 4-Pyr | $MeNH-(CH_2)_3$ |

Table 1 (continued)

| Compound No. | $R^1$ | $R^2$ | $R^3$ |
|---|---|---|---|
| 1-1399 | 3-Cl-4-F-Ph | 4-Pyr | EtNH-$(CH_2)_3$ |
| 1-1400 | 3-Cl-4-F-Ph | 4-Pyr | $Me_2N$-$(CH_2)_3$ |
| 1-1401 | 3-Cl-4-F-Ph | 4-Pyr | 1-Azt-$(CH_2)_3$ |
| 1-1402 | 3-Cl-4-F-Ph | 4-Pyr | 1-Pyrd-$(CH_2)_3$ |
| 1-1403 | 3-Cl-4-F-Ph | 4-Pyr | 1-Pip-$(CH_2)_3$ |
| 1-1404 | 3-Cl-4-F-Ph | 4-Pyr | 1-Mor-$(CH_2)_3$ |
| 1-1405 | 3-Cl-4-F-Ph | 4-Pyr | 1-Trnor-$(CH_2)_3$ |
| 1-1406 | 3-Cl-4-F-Ph | 4-Pyr | 1-Piz-$(CH_2)_3$ |
| 1-1407 | 3-Cl-4-F-Ph | 4-Pyr | 4-Me-1-Piz-$(CH_2)_3$ |
| 1-1408 | 3-Cl-4-F-Ph | 4-Pyr | 3-Azt |
| 1-1409 | 3-Cl-4-F-Ph | 4-Pyr | 1-Me-3-Azt |
| 1-1410 | 3-Cl-4-F-Ph | 4-Pyr | 3-Pyrd |
| 1-1411 | 3-Cl-4-F-Ph | 4-Pyr | 1-Me-3-Pyrd |
| 1-1412 | 3-Cl-4-F-Ph | 4-Pyr | 4-Pip |
| 1-1413 | 3-Cl-4-F-Ph | 4-Pyr | 4-(3,4-deH-Pip) |
| 1-1414 | 3-Cl-4-F-Ph | 4-Pyr | 1-Me-4-Pip |
| 1-1415 | 3-Cl-4-F-Ph | 4-Pyr | 1-Me-4-(3,4-deH-Pip) |
| 1-1416 | 3-Cl-4-F-Ph | 4-Pyr | 1-Piz |
| 1-1417 | 3-Cl-4-F-Ph | 4-Pyr | 4-Me-1-Piz |
| 1-1418 | 3-Cl-4-F-Ph | 4-Pyr | 4-Pyr |
| 1-1419 | 3-Cl-4-F-Ph | 4-Pyr | 3-Pyr |
| 1-1420 | 3-Cl-4-F-Ph | 4-Pyr | 4-Pym |
| 1-1421 | 3-Cl-4-F-Ph | 4-Pyr | 5-Pym |
| 1-1422 | 3-Cl-4-F-Ph | 4-Pyr | 3-pyrd-$CH_2$ |
| 1-1423 | 3-Cl-4-F-Ph | 4-Pyr | 1-Me-3-Pyrd-$CH_2$ |
| 1-1424 | 3-Cl-4-F-Ph | 4-Pyr | 4-Pip-$CH_2$ |
| 1-1425 | 3-Cl-4-F-Ph | 4-Pyr | 1-Me-4-Pip-$CH_2$ |
| 1-1426 | 3-Cl-4-F-Ph | 4-Pyr | 2-Piz-$CH_2$ |
| 1-1427 | 3-Cl-4-F-Ph | 4-Pyr | 4-Pyr-$CH_2$ |
| 1-1428 | 3-Cl-4-F-Ph | 4-Pyr | 3-Pyr-$CH_2$ |
| 1-1429 | 3-Cl-4-F-Ph | 4-Pyr | 4-Pym-$CH_2$ |
| 1-1430 | 3-Cl-4-F-Ph | 4-Pyr | 5-Pym-$CH_2$ |
| 1-1431 | 3-Cl-4-F-Ph | 4-Pyr | 2-Pym-$CH_2$ |
| 1-1432 | 3,4,5-triF-Ph | 4-Pyr | $H_2N$-$(CH_2)_3$ |
| 1-1433 | 3,4,5-triF-Ph | 4-Pyr | MeNH-$(CH_2)_3$ |
| 1-1434 | 3,4,5-triF-Ph | 4-Pyr | EtNH-$(CH_2)_3$ |
| 1-1435 | 3,4,5-triF-Ph | 4-Pyr | $Me_2N$-$(CH_2)_3$ |
| 1-1436 | 3,4,5-triF-Ph | 4-Pyr | 1-Azt-$(CH_2)_3$ |

Table 1 (continued)

| Compound No. | $R^1$ | $R^2$ | $R^3$ |
|---|---|---|---|
| 1-1437 | 3,4,5-triF-Ph | 4-Pyr | 1-Pyrd-$(CH_2)_3$ |
| 1-1438 | 3,4,5-triF-Ph | 4-Pyr | 1-Pip-$(CH_2)_3$ |
| 1-1439 | 3,4,5-triF-Ph | 4-Pyr | 1-Mor-$(CH_2)_3$ |
| 1-1440 | 3,4,5-triF-Ph | 4-Pyr | 1-Tmor-$(CH_2)_3$ |
| 1-1441 | 3,4,5-triF-Ph | 4-Pyr | 1-Piz-$(CH_2)_3$ |
| 1-1442 | 3,4,5-triF-Ph | 4-Pyr | 4-Me-1-Piz-$(CH_2)_3$ |
| 1-1443 | 3,4,5-triF-Ph | 4-Pyr | 3-Azt |
| 1-1444 | 3,4,5-triF-Ph | 4-Pyr | 1-Me-3-Azt |
| 1-1445 | 3,4,5-triF-Ph | 4-Pyr | 3-Pyrd |
| 1-1446 | 3,4,5-triF-Ph | 4-Pyr | 1-Me-3-Pyrd |
| 1-1447 | 3,4,5-triF-Ph | 4-Pyr | 4-Pip |
| 1-1448 | 3,4,5-triF-Ph | 4-Pyr | 4-(3,4-deH-Pip) |
| 1-1449 | 3,4,5-triF-Ph | 4-Pyr | 1-Me-4-Pip |
| 1-1450 | 3,4,5-triF-Ph | 4-Pyr | 1-Me-4-(3,4-deH-Pip) |
| 1-1451 | 3,4,5-triF-Ph | 4-Pyr | 1-Piz |
| 1-1452 | 3,4,5-triF-Ph | 4-Pyr | 4-Me-1-Piz |
| 1-1453 | 3,4,5-triF-Ph | 4-Pyr | 4-Pyr |
| 1-1454 | 3,4,5-triF-Ph | 4-Pyr | 3-Pyr |
| 1-1455 | 3,4,5-triF-Ph | 4-Pyr | 4-Pym |
| 1-1456 | 3,4,5-triF-Ph | 4-Pyr | 5-Pym |
| 1-1457 | 3,4,5-triF-Ph | 4-Pyr | 3-Pyrd-$CH_2$ |
| 1-1458 | 3,4,5-triF-Ph | 4-Pyr | 1-Me-3-Pyrd-$CH_2$ |
| 1-1459 | 3,4,5-triF-Ph | 4-Pyr | 4-Pip-$CH_2$ |
| 1-1460 | 3,4,5-triF-Ph | 4-Pyr | 1-Me-4-Pip-$CH_2$ |
| 1-1461 | 3,4,5-triF-Ph | 4-Pyr | 2-Piz-$CH_2$ |
| 1-1462 | 3,4,5-triF-Ph | 4-Pyr | 4-Pyr-$CH_2$ |
| 1-1463 | 3,4,5-triF-Ph | 4-Pyr | 3-Pyr-$CH_2$ |
| 1-1464 | 3,4,5-triF-Ph | 4-Pyr | 4-Pym-$CH_2$ |
| 1-1465 | 3,4,5-triF-Ph | 4-Pyr | 5-Pym-$CH_2$ |
| 1-1466 | 3,4,5-triF-Ph | 4-Pyr | 2-Pym-$CH_2$ |
| 1-1467 | 3-$CF_3$-Ph | 4-Pyr | $H_2N$-$(CH_2)_3$ |
| 1-1468 | 3-$CF_3$-Ph | 4-Pyr | MeNH-$(CH_2)_3$ |
| 1-1469 | 3-$CF_3$-Ph | 4-Pyr | EtNH-$(CH_2)_3$ |
| 1-1470 | 3-$CF_3$-Ph | 4-Pyr | $Me_2N$-$(CH_2)_3$ |
| 1-1471 | 3-$CF_3$-Ph | 4-Pyr | 1-Azt-$(CH_2)_3$ |
| 1-1472 | 3-$CF_3$-Ph | 4-Pyr | 1-Pyrd-$(CH_2)_3$ |
| 1-1473 | 3-$CF_3$-Ph | 4-Pyr | 1-Pip-$(CH_2)_3$ |
| 1-1474 | 3-$CF_3$-Ph | 4-Pyr | 1-Mor-$(CH_2)_3$ |

Table 1 (continued)

| Compound No. | $R^1$ | $R^2$ | $R^3$ |
|---|---|---|---|
| 1-1475 | 3-$CF_3$-Ph | 4-Pyr | 1-Tmor-$(CH_2)_3$ |
| 1-1476 | 3-$CF_3$-Ph | 4-Pyr | 1-Piz-$(CH_2)_3$ |
| 1-1477 | 3-$CF_3$-Ph | 4-Pyr | 4-Me-1-Piz-$(CH_2)_3$ |
| 1-1478 | 3-$CF_3$-Ph | 4-Pyr | 3-Azt |
| 1-1479 | 3-$CF_3$-Ph | 4-Pyr | 1-Me-3-Azt |
| 1-1480 | 3-$CF_3$-Ph | 4-Pyr | 3-Pyrd |
| 1-1481 | 3-$CF_3$-Ph | 4-Pyr | 1-Me-3-Pyrd |
| 1-1482 | 3-$CF_3$-Ph | 4-Pyr | 4-Pip |
| 1-1483 | 3-$CF_3$-Ph | 4-Pyr | 4-(3,4-deH-Pip) |
| 1-1484 | 3-$CF_3$-Ph | 4-Pyr | 1-Me-4-Pip |
| 1-1485 | 3-$CF_3$-Ph | 4-Pyr | 1-Me-4-(3,4-deH-Pip) |
| 1-1486 | 3-$CF_3$-Ph | 4-Pyr | 1-Piz |
| 1-1487 | 3-$CF_3$-Ph | 4-Pyr | 4-Me-1-Piz |
| 1-1488 | 3-$CF_3$-Ph | 4-Pyr | 4-Pyr |
| 1-1489 | 3-$CF_3$-Ph | 4-Pyr | 3-Pyr |
| 1-1490 | 3-$CF_3$-Ph | 4-Pyr | 4-Pym |
| 1-1491 | 3-$CF_3$-Ph | 4-Pyr | 5-Pym |
| 1-1492 | 3-$CF_3$-Ph | 4-Pyr | 3-Pyrd-$CH_2$ |
| 1-1493 | 3-$CF_3$-Ph | 4-Pyr | 1-Me-3-Pyrd-$CH_2$ |
| 1-1494 | 3-$CF_3$-Ph | 4-Pyr | 4-Pip-$CH_2$ |
| 1-1495 | 3-$CF_3$-Ph | 4-Pyr | 1-Me-4-Pip-$CH_2$ |
| 1-1496 | 3-$CF_3$-Ph | 4-Pyr | 2-Piz-$CH_2$ |
| 1-1497 | 3-$CF_3$-Ph | 4-Pyr | 4-Pyr-$CH_2$ |
| 1-1498 | 3-$CF_3$-Ph | 4-Pyr | 3-Pyr-$CH_2$ |
| 1-1499 | 3-$CF_3$-Ph | 4-Pyr | 4-Pym-$CH_2$ |
| 1-1500 | 3-$CF_3$-Ph | 4-Pyr | 5-Pym-$CH_2$ |
| 1-1501 | 3-$CF_3$-Ph | 4-Pyr | 2-Pym-$CH_2$ |
| 1-1502 | 3-$CHF_2O$-Ph | 4-Pyr | $H_2N$-$(CH_2)_3$ |
| 1-1503 | 3-$CHF_2O$-Ph | 4-Pyr | MeNH-$(CH_2)_3$ |
| 1-1504 | 3-$CHF_2O$-Ph | 4-Pyr | EtNH-$(CH_2)_3$ |
| 1-1505 | 3-$CHF_2O$-Ph | 4-Pyr | $Me_2N$-$(CH_2)_3$ |
| 1-1506 | 3-$CHF_2O$-Ph | 4-Pyr | 1-Azt-$(CH_2)_3$ |
| 1-1507 | 3-$CHF_2O$-Ph | 4-Pyr | 1-Pyrd-$(CH_2)_3$ |
| 1-1508 | 3-$CHF_2O$-Ph | 4-Pyr | 1-Pip-$(CH_2)_3$ |
| 1-1509 | 3-$CHF_2O$-Ph | 4-Pyr | 1-Mor-$(CH_2)_3$ |
| 1-1510 | 3-$CHF_2O$-Ph | 4-Pyr | 1-Tmor-$(CH_2)_3$ |
| 1-1511 | 3-$CHF_2O$-Ph | 4-Pyr | 1-Piz-$(CH_2)_3$ |
| 1-1512 | 3-$CHF_2O$-Ph | 4-Pyr | 4-Me-1-Piz-$(CH_2)_3$ |

Table 1 (continued)

| Compound No. | $R^1$ | $R^2$ | $R^3$ |
|---|---|---|---|
| 1-1513 | 3-$CHF_2$O-Ph | 4-Pyr | 3-Azt |
| 1-1514 | 3-$CHF_2$O-Ph | 4-Pyr | 1-Me-3-Azt |
| 1-1515 | 3-$CHF_2$O-Ph | 4-Pyr | 3-Pyrd |
| 1-1516 | 3-$CHF_2$O-Ph | 4-Pyr | 1-Me-3-Pyrd |
| 1-1517 | 3-$CHF_2$O-Ph | 4-Pyr | 4-Pip |
| 1-1518 | 3-$CHF_2$O-Ph | 4-Pyr | 4-(3,4-deH-Pip) |
| 1-1519 | 3-$CHF_2$O-Ph | 4-Pyr | 1-Me-4-Pip |
| 1-1520 | 3-$CHF_2$O-Ph | 4-Pyr | 1-Me-4-(3,4-deH-Pip) |
| 1-1521 | 3-$CHF_2$O-Ph | 4-Pyr | 1-Piz |
| 1-1522 | 3-$CHF_2$O-Ph | 4-Pyr | 4-Me-1-Piz |
| 1-1523 | 3-$CHF_2$O-Ph | 4-Pyr | 4-Pyr |
| 1-1524 | 3-$CHF_2$O-Ph | 4-Pyr | 3-Pyr |
| 1-1525 | 3-$CHF_2$O-Ph | 4-Pyr | 4-Pym |
| 1-1526 | 3-$CHF_2$O-Ph | 4-Pyr | 5-Pym |
| 1-1527 | 3-$CHF_2$O-Ph | 4-Pyr | 3-Pyrd-$CH_2$ |
| 1-1528 | 3-$CHF_2$O-Ph | 4-Pyr | 1-Me-3-Pyrd-$CH_2$ |
| 1-1529 | 3-$CHF_2$O-Ph | 4-Pyr | 4-Pip-$CH_2$ |
| 1-1530 | 3-$CHF_2$O-Ph | 4-Pyr | 1-Me-4-Pip-$CH_2$ |
| 1-1531 | 3-$CHF_2$O-Ph | 4-Pyr | 2-Piz-$CH_2$ |
| 1-1532 | 3-$CHF_2$O-Ph | 4-Pyr | 4-Pyr-$CH_2$ |
| 1-1533 | 3-$CHF_2$O-Ph | 4-Pyr | 3-Pyr-$CH_2$ |
| 1-1534 | 3-$CHF_2$O-Ph | 4-Pyr | 4-Pym-$CH_2$ |
| 1-1535 | 3-$CHF_2$O-Ph | 4-Pyr | 5-Pym-$CH_2$ |
| 1-1536 | 3-$CHF_2$O-Ph | 4-Pyr | 2-Pym-$CH_2$ |
| 1-1537 | 3-Cl-4-F-Ph | 2-$NH_2$-4-Pym | $H_2N-(CH_2)_3$ |
| 1-1538 | 3-Cl-4-F-Ph | 2-$NH_2$-4-Pym | MeNH-$(CH_2)_3$ |
| 1-1539 | 3-Cl-4-F-Ph | 2-$NH_2$-4-Pym | EtNH-$(CH_2)_3$ |
| 1-1540 | 3-Cl-4-F-Ph | 2-$NH_2$-4-Pym | $Me_2N-(CH_2)_3$ |
| 1-1541 | 3-Cl-4-F-Ph | 2-$NH_2$-4-Pym | 1-Azt-$(CH_2)_3$ |
| 1-1542 | 3-Cl-4-F-Ph | 2-$NH_2$-4-Pym | 1-Pyrd-$(CH_2)_3$ |
| 1-1543 | 3-Cl-4-F-Ph | 2-$NH_2$-4-Pym | 1-Pip-$(CH_2)_3$ |
| 1-1544 | 3-Cl-4-F-Ph | 2-$NH_2$-4-Pym | 1-Mor-$(CH_2)_3$ |
| 1-1545 | 3-Cl-4-F-Ph | 2-$NH_2$-4-Pym | 1-Tmor-$(CH_2)_3$ |
| 1-1546 | 3-Cl-4-F-Ph | 2-$NH_2$-4-Pym | 1-Piz-$(CH_2)_3$ |
| 1-1547 | 3-Cl-4-F-Ph | 2-$NH_2$-4-Pym | 4-Me-1-Piz-$(CH_2)_3$ |
| 1-1548 | 3-Cl-4-F-Ph | 2-$NH_2$-4-Pym | 3-Azt |
| 1-1549 | 3-Cl-4-F-Ph | 2-$NH_2$-4-Pym | 1-Me-3-Azt |
| 1-1550 | 3-Cl-4-F-Ph | 2-$NH_2$-4-Pym | 3-Pyrd |

Table 1 (continued)

| Compound No. | $R^1$ | $R^2$ | $R^3$ |
|---|---|---|---|
| 1-1551 | 3-Cl-4-F-Ph | 2-$NH_2$-4-Pym | 1-Me-3-Pyrd |
| 1-1552 | 3-Cl-4-F-Ph | 2-$NH_2$-4-Pym | 4-Pip |
| 1-1553 | 3-Cl-4-F-Ph | 2-$NH_2$-4-Pym | 4-(3,4-deH-Pip) |
| 1-1554 | 3-Cl-4-F-Ph | 2-$NH_2$-4-Pym | 1-Me-4-Pip |
| 1-1555 | 3-Cl-4-F-Ph | 2-$NH_2$-4-Pym | 1-Me-4-(3,4-deH-Pip) |
| 1-1556 | 3-Cl-4-F-Ph | 2-$NH_2$-4-Pym | 1-Piz |
| 1-1557 | 3-Cl-4-F-Ph | 2-$NH_2$-4-Pym | 4-Me-1-Piz |
| 1-1558 | 3-Cl-4-F-Ph | 2-$NH_2$-4-Pym | 4-Pyr |
| 1-1559 | 3-Cl-4-F-Ph | 2-$NH_2$-4-Pym | 3-Pyr |
| 1-1560 | 3-Cl-4-F-Ph | 2-$NH_2$-4-Pym | 4-Pym |
| 1-1561 | 3-Cl-4-F-Ph | 2-$NH_2$-4-Pym | 5-Pym |
| 1-1562 | 3-Cl-4-F-Ph | 2-$NH_2$-4-Pym | 3-Pyrd-$CH_2$ |
| 1-1563 | 3-Cl-4-F-Ph | 2-$NH_2$-4-Pym | 1-Me-3-Pyrd-$CH_2$ |
| 1-1564 | 3-Cl-4-F-Ph | 2-$NH_2$-4-Pym | 4-Pip-$CH_2$ |
| 1-1565 | 3-Cl-4-F-Ph | 2-$NH_2$-4-Pym | 1-Me-4-Pip-$CH_2$ |
| 1-1566 | 3-Cl-4-F-Ph | 2-$NH_2$-4-Pym | 2-Piz-$CH_2$ |
| 1-1567 | 3-Cl-4-F-Ph | 2-$NH_2$-4-Pym | 4-Pyr-$CH_2$ |
| 1-1568 | 3-Cl-4-F-Ph | 2-$NH_2$-4-Pym | 3-Pyr-$CH_2$ |
| 1-1569 | 3-Cl-4-F-Ph | 2-$NH_2$-4-Pym | 4-Pym-$CH_2$ |
| 1-1570 | 3-Cl-4-F-Ph | 2-$NH_2$-4-Pym | 5-Pym-$CH_2$ |
| 1-1571 | 3-Cl-4-F-Ph | 2-$NH_2$-4-Pym | 2-Pym-$CH_2$ |
| 1-1572 | 3,4,5-triF-Ph | 2-$NH_2$-4-Pym | $H_2N-(CH_2)_3$ |
| 1-1573 | 3,4,5-triF-Ph | 2-$NH_2$-4-Pym | $MeNH-(CH_2)_3$ |
| 1-1574 | 3,4,5-triF-Ph | 2-$NH_2$-4-Pym | $EtNH-(CH_2)_3$ |
| 1-1575 | 3,4,5-triF-Ph | 2-$NH_2$-4-Pym | $Me_2N-(CH_2)_3$ |
| 1-1576 | 3,4,5-triF-Ph | 2-$NH_2$-4-Pym | 1-Azt-$(CH_2)_3$ |
| 1-1577 | 3,4,5-triF-Ph | 2-$NH_2$-4-Pym | 1-Pyrd-$(CH_2)_3$ |
| 1-1578 | 3,4,5-triF-Ph | 2-$NH_2$-4-Pym | 1-Pip-$(CH_2)_3$ |
| 1-1579 | 3,4,5-triF-Ph | 2-$NH_2$-4-Pym | 1-Mor-$(CH_2)_3$ |
| 1-1580 | 3,4,5-triF-Ph | 2-$NH_2$-4-Pym | 1-Tmor-$(CH_2)_3$ |
| 1-1581 | 3,4,5-triF-Ph | 2-$NH_2$-4-Pym | 1-Piz-$(CH_2)_3$ |
| 1-1582 | 3,4,5-triF-Ph | 2-$NH_2$-4-Pym | 4-Me-1-Piz-$(CH_2)_3$ |
| 1-1583 | 3,4,5-triF-Ph | 2-$NH_2$-4-Pym | 3-Azt |
| 1-1584 | 3,4,5-triF-Ph | 2-$NH_2$-4-Pym | 1-Me-3-Azt |
| 1-1585 | 3,4,5-triF-Ph | 2-$NH_2$-4-Pym | 3-Pyrd |
| 1-1586 | 3,4,5-triF-Ph | 2-$NH_2$-4-Pym | 1-Me-3-Pyrd |
| 1-1587 | 3,4,5-triF-Ph | 2-$NH_2$-4-Pym | 4-Pip |
| 1-1588 | 3,4,5-triF-Ph | 2-$NH_2$-4-Pym | 4-(3,4-deH-Pip) |

Table 1 (continued)

| Compound No. | $R^1$ | $R^2$ | $R^3$ |
|---|---|---|---|
| 1-1589 | 3,4,5-triF-Ph | 2-$NH_2$-4-Pym | 1-Me-4-Pip |
| 1-1590 | 3,4,5-triF-Ph | 2-$NH_2$-4-Pym | 1-Me-4-(3,4-deH-Pip) |
| 1-1591 | 3,4,5-triF-Ph | 2-$NH_2$-4-Pym | 1-Piz |
| 1-1592 | 3,4,5-triF-Ph | 2-$NH_2$-4-Pym | 4-Me-1-Piz |
| 1-1593 | 3,4,5-triF-Ph | 2-$NH_2$-4-Pym | 4-Pyr |
| 1-1594 | 3,4,5-triF-Ph | 2-$NH_2$-4-Pym | 3-Pyr |
| 1-1595 | 3,4,5-triF-Ph | 2-$NH_2$-4-Pym | 4-Pym |
| 1-1596 | 3,4,5-triF-Ph | 2-$NH_2$-4-Pym | 5-Pym |
| 1-1597 | 3,4,5-triF-Ph | 2-$NH_2$-4-Pym | 3-Pyrd-$CH_2$ |
| 1-1598 | 3,4,5-triF-Ph | 2-$NH_2$-4-Pym | 1-Me-3-Pyrd-$CH_2$ |
| 1-1599 | 3,4,5-triF-Ph | 2-$NH_2$-4-Pym | 4-Pip-$CH_2$ |
| 1-1600 | 3,4,5-triF-Ph | 2-$NH_2$-4-Pym | 1-Me-4-Pip-$CH_2$ |
| 1-1601 | 3,4,5-triF-Ph | 2-$NH_2$-4-Pym | 2-Piz-$CH_2$ |
| 1-1602 | 3,4,5-triF-Ph | 2-$NH_2$-4-Pym | 4-Pyr-$CH_2$ |
| 1-1603 | 3,4,5-triF-Ph | 2-$NH_2$-4-Pym | 3-Pyr-$CH_2$ |
| 1-1604 | 3,4,5-triF-Ph | 2-$NH_2$-4-Pym | 4-Pym-$CH_2$ |
| 1-1605 | 3,4,5-triF-Ph | 2-$NH_2$-4-Pym | 5-Pym-$CH_2$ |
| 1-1606 | 3,4,5-triF-Ph | 2-$NH_2$-4-Pym | 2-Pym-$CH_2$ |
| 1-1607 | 3-$CF_3$-Ph | 2-$NH_2$-4-Pym | $H_2N$-$(CH_2)_3$ |
| 1-1608 | 3-$CF_3$-Ph | 2-$NH_2$-4-Pym | MeNH-$(CH_2)_3$ |
| 1-1609 | 3-$CF_3$-Ph | 2-$NH_2$-4-Pym | EtNH-$(CH_2)_3$ |
| 1-1610 | 3-$CF_3$-Ph | 2-$NH_2$-4-Pym | $Me_2N$-$(CH_2)_3$ |
| 1-1611 | 3-$CF_3$-Ph | 2-$NH_2$-4-Pym | 1-Azt-$(CH_2)_3$ |
| 1-1612 | 3-$CF_3$-Ph | 2-$NH_2$-4-Pym | 1-Pyrd-$(CH_2)_3$ |
| 1-1613 | 3-$CF_3$-Ph | 2-$NH_2$-4-Pym | 1-Pip-$(CH_2)_3$ |
| 1-1614 | 3-$CF_3$-Ph | 2-$NH_2$-4-Pym | 1-Mor-$(CH_2)_3$ |
| 1-1615 | 3-$CF_3$-Ph | 2-$NH_2$-4-Pym | 1-Tmor-$(CH_2)_3$ |
| 1-1616 | 3-$CF_3$-Ph | 2-$NH_2$-4-Pym | 1-Piz-$(CH_2)_3$ |
| 1-1617 | 3-$CF_3$-Ph | 2-$NH_2$-4-Pym | 4-Me-1-Piz-$(CH_2)_3$ |
| 1-1618 | 3-$CF_3$-Ph | 2-$NH_2$-4-Pym | 3-Azt |
| 1-1619 | 3-$CF_3$-Ph | 2-$NH_2$-4-Pym | 1-Me-3-Azt |
| 1-1620 | 3-$CF_3$-Ph | 2-$NH_2$-4-Pym | 3-Pyrd |
| 1-1621 | 3-$CF_3$-Ph | 2-$NH_2$-4-Pym | 1-Me-3-Pyrd |
| 1-1622 | 3-$CF_3$-Ph | 2-$NH_2$-4-Pym | 4-Pip |
| 1-1623 | 3-$CF_3$-Ph | 2-$NH_2$-4-Pym | 4-(3,4-deH-Pip) |
| 1-1624 | 3-$CF_3$-Ph | 2-$NH_2$-4-Pym | 1-Me-4-Pip |
| 1-1625 | 3-$CF_3$-Ph | 2-$NH_2$-4-Pym | 1-Me-4- ( 3, 4-deH-Pip) |
| 1-1626 | 3-$CF_3$-Ph | 2-$NH_2$-4-Pym | 1-Piz |

Table 1 (continued)

| Compound No. | $R^1$ | $R^2$ | $R^3$ |
|---|---|---|---|
| 1-1627 | 3-$CF_3$-Ph | 2-$NH_2$-4-Pym | 4-Me-1-Piz |
| 1-1628 | 3-$CF_3$-Ph | 2-$NH_2$-4-Pym | 4-Pyr |
| 1-1629 | 3-$CF_3$-Ph | 2-$NH_2$-4-Pym | 3-Pyr |
| 1-1630 | 3-$CF_3$-Ph | 2-$NH_2$-4-Pym | 4-Pym |
| 1-1631 | 3-$CF_3$-Ph | 2-$NH_2$-4-Pym | 5-Pym |
| 1-1632 | 3-$CF_3$-Ph | 2-$NH_2$-4-Pym | 3-Pyrd-$CH_2$ |
| 1-1633 | 3-$CF_3$-Ph | 2-$NH_2$-4-Pym | 1-Me-3-Pyrd-$CH_2$ |
| 1-1634 | 3-$CF_3$-Ph | 2-$NH_2$-4-Pym | 4-Pip-$CH_2$ |
| 1-1635 | 3-$CF_3$-Ph | 2-$NH_2$-4-Pym | 1-Me-4-Pip-$CH_2$ |
| 1-1636 | 3-$CF_3$-Ph | 2-$NH_2$-4-Pym | 2-Piz-$CH_2$ |
| 1-1637 | 3-$CF_3$-Ph | 2-$NH_2$-4-Pym | 4-Pyr-$CH_2$ |
| 1-1638 | 3-$CF_3$-Ph | 2-$NH_2$-4-Pym | 3-Pyr-$CH_2$ |
| 1-1639 | 3-$CF_3$-Ph | 2-$NH_2$-4-Pym | 4-Pym-$CH_2$ |
| 1-1640 | 3-$CF_3$-Ph | 2-$NH_2$-4-Pym | 5-Pym-$CH_2$ |
| 1-1641 | 3-$CF_3$-Ph | 2-$NH_2$-4-Pym | 2-Pym-$CH_2$ |
| 1-1642 | 3-$CHF_2$O-Ph | 2-$NH_2$-4-Pym | $H_2$N-$(CH_2)_3$ |
| 1-1643 | 3-$CHF_2$O-Ph | 2-$NH_2$-4-Pym | MeNH-$(CH_2)_3$ |
| 1-1644 | 3-$CHF_2$O-Ph | 2-$NH_2$-4-Pym | EtNH-$(CH_2)_3$ |
| 1-1645 | 3-$CHF_2$O-Ph | 2-$NH_2$-4-Pym | $Me_2$N-$(CH_2)_3$ |
| 1-1646 | 3-$CHF_2$O-Ph | 2-$NH_2$-4-Pym | 1-Azt-$(CH_2)_3$ |
| 1-1647 | 3-$CHF_2$O-Ph | 2-$NH_2$-4-Pym | 1-Pyrd-$(CH_2)$3 |
| 1-1648 | 3-$CHF_2$O-Ph | 2-$NH_2$-4-Pym | 1-Pip-$(CH_2)_3$ |
| 1-1649 | 3-$CHF_2$O-Ph | 2-$NH_2$-4-Pym | 1-Mor-$(CH_2)_3$ |
| 1-1650 | 3-$CHF_z$O-Ph | 2-$NH_2$-4-Pym | 1-Tmor-$(CH_2)_3$ |
| 1-1651 | 3-$CHF_2$O-Ph | 2-$NH_2$-4-Pym | 1-Piz-$(CH_2)_3$ |
| 1-1652 | 3-$CHF_2$O-Ph | 2-$NH_2$-4-Pym | 4-Me-1-Piz-$(CH_2)_3$ |
| 1-1653 | 3-$CHF_2$O-Ph | 2-$NH_2$-4-Pym | 3-Azt |
| 1-1654 | 3-$CHF_2$O-Ph | 2-$NH_2$-4-Pym | 1-Me-3-Azt |
| 1-1655 | 3-$CHF_2$O-Ph | 2-$NH_2$-4-Pym | 3-Pyrd |
| 1-1656 | 3-$CHF_2$O-Ph | 2-$NH_2$-4-Pym | 1-Me-3-Pyrd |
| 1-1657 | 3-$CHF_2$O-Ph | 2-$NH_2$-4-Pym | 4-Pip |
| 1-1658 | 3-$CHF_2$O-Ph | 2-$NH_2$-4-Pym | 4-(3,4-deH-Pip) |
| 1-1659 | 3-$CHF_2$O-Ph | 2-$NH_2$-4-Pym | 1-Ne-4-Pip |
| 1-1660 | 3-$CHF_2$O-Ph | 2-$NH_2$-4-Pym | 1-Me-4-(3,4-deH-Pip) |
| 1-1661 | 3-$CHF_2$O-Ph | 2-$NH_2$-4-Pym | 1-Piz |
| 1-1662 | 3-$CHF_2$O-Ph | 2-$NH_2$-4-Pym | 4-Me-1-Piz |
| 1-1663 | 3-$CHF_2$O-Ph | 2-$NH_2$-4-Pym | 4-Pyr |
| 1-1664 | 3-$CHF_2$O-Ph | 2-$NH_2$-4-Pym | 3-Pyr |

Table 1 (continued)

| Compound No. | $R^1$ | $R^2$ | $R^3$ |
|---|---|---|---|
| 1-1665 | 3-$CHF_2$O-Ph | 2-$NH_2$-4-Pym | 4-Pym |
| 1-1666 | 3-$CHF_2$O-Ph | 2-$NH_2$-4-Pym | 5-Pym |
| 1-1667 | 3-$CHF_2$O-Ph | 2-$NH_2$-4-Pym | 3-Pyrd-$CH_2$ |
| 1-1668 | 3-$CHF_2$O-Ph | 2-$NH_2$-4-Pym | 1-Me-3-Pyrd-$CH_2$ |
| 1-1669 | 3-$CHF_2$O-Ph | 2-$NH_2$-4-Pym | 4-Pip-$CH_2$ |
| 1-1670 | 3-$CHF_2$O-Ph | 2-$NH_2$-4-Pym | 1-Me-4-Pip-$CH_2$ |
| 1-1671 | 3-$CHF_2$O-Ph | 2-$NH_2$-4-Pym | 2-Piz-$CH_2$ |
| 1-1672 | 3-$CHF_2$O-Ph | 2-$NH_2$-4-Pym | 4-Pyr-$CH_2$ |
| 1-1673 | 3-$CHF_2$O-Ph | 2-$NH_2$-4-Pym | 3-Pyr-$CH_2$ |
| 1-1674 | 3-$CHF_2$O-Ph | 2-$NH_2$-4-Pym | 4-Pym-$CH_2$ |
| 1-1675 | 3-$CHF_2$O-Ph | 2-$NH_2$-4-Pym | 5-Pym-$CH_2$ |
| 1-1676 | 3-$CHF_2$O-Ph | 2-$NH_2$-4-Pym | 2-Pym-$CH_2$ |
| 1-1677 | 3-Cl-4-F-Ph | 2-MeNH-4-Pym | $H_2N-(CH_2)_3$ |
| 1-1678 | 3-Cl-4-F-Ph | 2-MeNH-4-Pym | MeNH-$(CH_2)_3$ |
| 1-1679 | 3-Cl-4-F-Ph | 2-MeNH-4-Pym | EtNH-$(CH_2)_3$ |
| 1-1680 | 3-Cl-4-F-Ph | 2-MeNH-4-Pym | $Me_2N-(CH_2)_3$ |
| 1-1681 | 3-Cl-4-F-Ph | 2-MeNH-4-Pym | 1-Azt-$(CH_2)_3$ |
| 1-1682 | 3-Cl-4-F-Ph | 2-MeNH-4-Pym | 1-Pyrd-$(CH_2)_3$ |
| 1-1683 | 3-Cl-4-F-Ph | 2-MeNH-4-Pym | 1-Pip-$(CH_2)_3$ |
| 1-1684 | 3-Cl-4-F-Ph | 2-MeNH-4-Pym | 1-Mor-$(CH_2)_3$ |
| 1-1685 | 3-Cl-4-F-Ph | 2-MeNH-4-Pym | 1-Tmor-$(CH_2)_3$ |
| 1-1686 | 3-Cl-4-F-Ph | 2-MeNH-4-Pym | 1-Piz-$(CH_2)_3$ |
| 1-1687 | 3-Cl-4-F-Ph | 2-MeNH-4-Pym | 4-Me-1-Piz-$(CH_2)_3$ |
| 1-1688 | 3-Cl-4-F-Ph | 2-MeNH-4-Pym | 3-Azt |
| 1-1689 | 3-Cl-4-F-Ph | 2-MeNH-4-Pym | 1-Me-3-Azt |
| 1-1690 | 3-Cl-4-F-Ph | 2-MeNH-4-Pym | 3-Pyrd |
| 1-1691 | 3-Cl-4-F-Ph | 2-MeNH-4-Pym | 1-Me-3-Pyrd |
| 1-1692 | 3-Cl-4-F-Ph | 2-MeNH-4-Pym | 4-Pip |
| 1-1693 | 3-Cl-4-F-Ph | 2-MeNH-4-Pym | 4-(3,4-deH-Pip) |
| 1-1694 | 3-Cl-4-F-Ph | 2-MeNH-4-Pym | 1-Me-4-Pip |
| 1-1695 | 3-Cl-4-F-Ph | 2-MeNH-4-Pym | 1-Me-4-(3,4-deH-Pip) |
| 1-1696 | 3-Cl-4-F-Ph | 2-MeNH-4-Pym | 1-Piz |
| 1-1697 | 3-Cl-4-F-Ph | 2-MeNH-4-Pym | 4-Me-1-Piz |
| 1-1698 | 3-Cl-4-F-Ph | 2-MeNH-4-Pym | 4-Pyr |
| 1-1699 | 3-Cl-4-F-Ph | 2-MeNH-4-Pym | 3-Pyr |
| 1-1700 | 3-Cl-4-F-Ph | 2-MeNH-4-Pym | 4-Pym |
| 1-1701 | 3-Cl-4-F-Ph | 2-MeNH-4-Pym | 5-Pym |
| 1-1702 | 3-Cl-4-F-Ph | 2-MeNH-4-Pym | 3-Pyrd-$CH_2$ |

Table 1 (continued)

| Compound No. | $R^1$ | $R^2$ | $R^3$ |
|---|---|---|---|
| 1-1703 | 3-Cl-4-F-Ph | 2-MeNH-4-Pym | 1-Me-3-Pyrd-$CH_2$ |
| 1-1704 | 3-Cl-4-F-Ph | 2-MeNH-4-Pym | 4-Pip-$CH_2$ |
| 1-1705 | 3-Cl-4-F-Ph | 2-MeNH-4-Pym | 1-Me-4-Pip-$CH_2$ |
| 1-1706 | 3-Cl-4-F-Ph | 2-MeNH-4-Pym | 2-Piz-$CH_2$ |
| 1-1707 | 3-Cl-4-F-Ph | 2-MeNH-4-Pym | 4-Pyr-$CH_2$ |
| 1-1708 | 3-Cl-4-F-Ph | 2-MeNH-4-Pym | 3-Pyr-$CH_2$ |
| 1-1709 | 3-Cl-4-F-Ph | 2-MeNH-4-Pym | 4-Pym-$CH_2$ |
| 1-1710 | 3-Cl-4-F-Ph | 2-MeNH-4-Pym | 5-Pym-$CH_2$ |
| 1-1711 | 3-Cl-4-F-Ph | 2-MeNH-4-Pym | 2-Pym-$CH_2$ |
| 1-1712 | 3,4,5-triF-Ph | 2-MeNH-4-Pym | $H_2N-(CH_2)_3$ |
| 1-1713 | 3,4,5-triF-Ph | 2-MeNH-4-Pym | MeNH-$(CH_2)_3$ |
| 1-1714 | 3,4,5-triF-Ph | 2-MeNH-4-Pym | EtNH-$(CH_2)_3$ |
| 1-1715 | 3,4,5-triF-Ph | 2-MeNH-4-Pym | $Me_2N-(CH_2)_3$ |
| 1-1716 | 3,4,5-triF-Ph | 2-MeNH-4-Pym | 1-Azt-$(CH_2)_3$ |
| 1-1717 | 3,4,5-triF-Ph | 2-MeNH-4-Pym | 1-Pyrd-$(CH_2)_3$ |
| 1-1718 | 3,4,5-triF-Ph | 2-MeNH-4-Pym | 1-Pip-$(CH_2)_3$ |
| 1-1719 | 3,4,5-triF-Ph | 2-MeNH-4-Pym | 1-Mor-$(CH_2)_3$ |
| 1-1720 | 3,4,5-triF-Ph | 2-MeNH-4-Pym | 1-Tmor-$(CH_2)_3$ |
| 1-1721 | 3,4,5-triF-Ph | 2-MeNH-4-Pym | 1-Piz-$(CH_2)_3$ |
| 1-1722 | 3,4,5-triF-Ph | 2-MeNH-4-Pym | 4-Me-1-Piz-$(CH_2)_3$ |
| 1-1723 | 3,4,5-triF-Ph | 2-MeNH-4-Pym | 3-Azt |
| 1-1724 | 3,4,5-triF-Ph | 2-MeNH-4-Pym | 1-Me-3-Azt |
| 1-1725 | 3,4,5-triF-Ph | 2-MeNH-4-Pym | 3-Pyrd |
| 1-1726 | 3,4,5-triF-Ph | 2-MeNH-4-Pym | 1-Me-3-Pyrd |
| 1-1727 | 3,4,5-triF-Ph | 2-MeNH-4-Pym | 4-Pip |
| 1-1728 | 3,4,5-triF-Ph | 2-MeNH-4-Pym | 4-(3,4-deH-Pip) |
| 1-1729 | 3,4,5-triF-Ph | 2-MeNH-4-Pym | 1-Me-4-Pip |
| 1-1730 | 3,4,5-triF-Ph | 2-MeNH-4-Pym | 1-Me-4-(3,4-deH-Pip) |
| 1-1731 | 3,4,5-triF-Ph | 2-MeNH-4-Pym | 1-Piz |
| 1-1732 | 3,4,5-triF-Ph | 2-MeNH-4-Pym | 4-Me-1-Piz |
| 1-1733 | 3,4,5-triF-Ph | 2-MeNH-4-Pym | 4-Pyr |
| 1-1734 | 3,4,5-triF-Ph | 2-MeNH-4-Pym | 3-Pyr |
| 1-1735 | 3,4,5-triF-Ph | 2-MeNH-4-Pym | 4-Pym |
| 1-1736 | 3,4,5-triF-Ph | 2-MeNH-4-Pym | 5-Pym |
| 1-1737 | 3,4,5-triF-Ph | 2-MeNH-4-Pym | 3-Pyrd-$CH_2$ |
| 1-1738 | 3,4,5-triF-Ph | 2-MeNH-4-Pym | 1-Me-3-Pyrd-$CH_2$ |
| 1-1739 | 3,4,5-triF-Ph | 2-MeNH-4-Pym | 4-Pip-$CH_2$ |
| 1-1740 | 3,4,5-triF-Ph | 2-MeNH-4-Pym | 1-Me-4-Pip-$CH_2$ |

Table 1 (continued)

| Compound No. | $R^1$ | $R^2$ | $R^3$ |
|---|---|---|---|
| 1-1741 | 3,4,5-triF-Ph | 2-MeNH-4-Pym | 2-Piz-$CH_2$ |
| 1-1742 | 3,4,5-triF-Ph | 2-MeNH-4-Pym | 4-Pyr-$CH_2$ |
| 1-1743 | 3,4,5-triF-Ph | 2-MeNH-4-Pym | 3-Pyr-$CH_2$ |
| 1-1744 | 3,4,5-triF-Ph | 2-MeNH-4-Pym | 4-Pym-$CH_2$ |
| 1-1745 | 3,4,5-triF-Ph | 2-MeNH-4-Pym | 5-Pym-$CH_2$ |
| 1-1746 | 3,4,5-triF-Ph | 2-MeNH-4-Pym | 2-Pym-$CH_2$ |
| 1-1747 | 3-$CF_3$-Ph | 2-MeNH-4-Pym | $H_2N$-$(CH_2)_3$ |
| 1-1748 | 3-$CF_3$-Ph | 2-MeNH-4-Pym | MeNH-$(CH_2)_3$ |
| 1-1749 | 3-$CF_3$-Ph | 2-MeNH-4-Pym | EtNH-$(CH_2)_3$ |
| 1-1750 | 3-$CF_3$-Ph | 2-MeNH-4-Pym | $Me_2N$-$(CH_2)_3$ |
| 1-1751 | 3-$CF_3$-Ph | 2-MeNH-4-Pym | 1-Azt-$(CH_2)_3$ |
| 1-1752 | 3-$CF_3$-Ph | 2-MeNH-4-Pym | 1-Pyrd-$(CH_2)_3$ |
| 1-1753 | 3-$CF_3$-Ph | 2-MeNH-4-Pym | 1-Pip-$(CH_2)_3$ |
| 1-1754 | 3-$CF_3$-Ph | 2-MeNH-4-Pym | 1-Mor-$(CH_2)_3$ |
| 1-1755 | 3-$CF_3$-Ph | 2-MeNH-4-Pym | 1-Tmor-$(CH_2)_3$ |
| 1-1756 | 3-$CF_3$-Ph | 2-MeNH-4-Pym | 1-Piz-$(CH_2)_3$ |
| 1-1757 | 3-$CF_3$-Ph | 2-MeNH-4-Pym | 4-Me-1-Piz-$(CH_2)_3$ |
| 1-1758 | 3-$CF_3$-Ph | 2-MeNH-4-Pym | 3-Azt |
| 1-1759 | 3-$CF_3$-Ph | 2-MeNH-4-Pym | 1-Me-3-Azt |
| 1-1760 | 3-$CF_3$-Ph | 2-MeNH-4-Pym | 3-Pyrd |
| 1-1761 | 3-$CF_3$-Ph | 2-MeNH-4-Pym | 1-Me-3-Pyrd |
| 1-1762 | 3-$CF_3$-Ph | 2-MeNH-4-Pym | 4-Pip |
| 1-1763 | 3-$CF_3$-Ph | 2-MeNH-4-Pym | 4-(3,4-deH-Pip) |
| 1-1764 | 3-$CF_3$-Ph | 2-MeNH-4-Pym | 1-Me-4-Pip |
| 1-1765 | 3-$CF_3$-Ph | 2-MeNH-4-Pym | 1-Me-4-(3,4-deH-Pip) |
| 1-1766 | 3-$CF_3$-Ph | 2-MeNH-4-Pym | 1-Piz |
| 1-1767 | 3-$CF_3$-Ph | 2-MeNH-4-Pym | 4-Me-1-Piz |
| 1-1768 | 3-$CF_3$-Ph | 2-MeNH-4-Pym | 4-Pyr |
| 1-1769 | 3-$CF_3$-Ph | 2-MeNH-4-Pym | 3-Pyr |
| 1-1770 | 3-$CF_3$-Ph | 2-MeNH-4-Pym | 4-Pym |
| 1-1771 | 3-$CF_3$-Ph | 2-MeNH-4-Pym | 5-Pym |
| 1-1772 | 3-$CF_3$-Ph | 2-MeNH-4-Pym | 3-Pyrd-$CH_2$ |
| 1-1773 | 3-$CF_3$-Ph | 2-MeNH-4-Pym | 1-Me-3-Pyrd-$CH_2$ |
| 1-1774 | 3-$CF_3$-Ph | 2-MeNH-4-Pym | 4-Pip-$CH_2$ |
| 1-1775 | 3-$CF_3$-Ph | 2-MeNH-4-Pym | 1-Me-4-Pip-$CH_2$ |
| 1-1776 | 3-$CF_3$-Ph | 2-MeNH-4-Pym | 2-Piz-$CH_2$ |
| 1-1777 | 3-$CF_3$-Ph | 2-MeNH-4-Pym | 4-Pyr-$CH_2$ |
| 1-1778 | 3-$CF_3$-Ph | 2-MeNH-4-Pym | 3-Pyr-$CH_2$ |

Table 1 (continued)

| Compound No. | $R^1$ | $R^2$ | $R^3$ |
|---|---|---|---|
| 1-1779 | 3-$CF_3$-Ph | 2-MeNH-4-Pym | 4-Pym-$CH_2$ |
| 1-1780 | 3-$CF_3$-Ph | 2-MeNH-4-Pym | 5-Pym-$CH_2$ |
| 1-1781 | 3-$CF_3$-Ph | 2-MeNH-4-Pym | 2-Pym-$CH_2$ |
| 1-1782 | 3-$CHF_2O$-Ph | 2-MeNH-4-Pym | $H_2N$-$(CH_2)_3$ |
| 1-1783 | 3-$CHF_2O$-Ph | 2-MeNH-4-Pym | MeNH-$(CH_2)_3$ |
| 1-1784 | 3-$CHF_2O$-Ph | 2-MeNH-4-Pym | EtNH-$(CH_2)_3$ |
| 1-1785 | 3-$CHF_2O$-Ph | 2-MeNH-4-Pym | $Me_2N$-$(CH_2)_3$ |
| 1-1786 | 3-$CHF_2O$-Ph | 2-MeNH-4-Pym | 1-Azt-$(CH_2)_3$ |
| 1-1787 | 3-$CHF_2O$-Ph | 2-MeNH-4-Pym | 1-Pyrd-$(CH_2)_3$ |
| 1-1788 | 3-$CHF_2O$-Ph | 2-MeNH-4-Pym | 1-Pip-$(CH_2)_3$ |
| 1-1789 | 3-$CHF_2O$-Ph | 2-MeNH-4-Pym | 1-Mor-$(CH_2)_3$ |
| 1-1790 | 3-$CHF_2O$-Ph | 2-MeNH-4-Pym | 1-Tmor-$(CH_2)_3$ |
| 1-1791 | 3-$CHF_2O$-Ph | 2-MeNH-4-Pym | 1-Piz-$(CH_2)_3$ |
| 1-1792 | 3-$CHF_2O$-Ph | 2-MeNH-4-Pym | 4-Me-1-Piz-$(CH_2)_3$ |
| 1-1793 | 3-$CHP_2O$-Ph | 2-MeNH-4-Pym | 3-Azt |
| 1-1794 | 3-$CHF_2O$-Ph | 2-MeNH-4-Pym | 1-Me-3-Azt |
| 1-1795 | 3-$CHF_2O$-Ph | 2-MeNH-4-Pym | 3-Pyrd |
| 1-1796 | 3-$CHF_2O$-Ph | 2-MeNH-4-Pym | 1-Me-3-Pyrd |
| 1-1797 | 3-$CHF_2O$-Ph | 2-MeNH-4-Pym | 4-Pip |
| 1-1798 | 3-$CHF_2O$-Ph | 2-MeNH-4-Pym | 4-(3,4-deH-Pip) |
| 1-1799 | 3-$CHF_2O$-Ph | 2-MeNH-4-Pym | 1-Me-4-Pip |
| 1-1800 | 3-$CHF_2O$-Ph | 2-MeNH-4-Pym | 1-Me-4-(3,4-deH-Pip) |
| 1-1801 | 3-$CHF_2O$-Ph | 2-MeNH-4-Pym | 1-Piz |
| 1-1802 | 3-$CHF_2O$-Ph | 2-MeNH-4-Pym | 4-Me-1-Piz |
| 1-1803 | 3-$CHF_2O$-Ph | 2-MeNH-4-Pym | 4-Pyr |
| 1-1804 | 3-$CHF_2O$-Ph | 2-MeNH-4-Pym | 3-Pyr |
| 1-1805 | 3-$CHF_2O$-Ph | 2-MeNH-4-Pym | 4-Pym |
| 1-1806 | 3-$CHF_2O$-Ph | 2-MeNH-4-Pym | 5-Pym |
| 1-1807 | 3-$CHF_2O$-Ph | 2-MeNH-4-Pym | 3-Pyrd-$CH_2$ |
| 1-1808 | 3-$CHF_2O$-Ph | 2-MeNH-4-Pym | 1-Me-3-Pyrd-$CH_2$ |
| 1-1809 | 3-$CHF_2O$-Ph | 2-MeNH-4-Pym | 4-Pip-$CH_2$ |
| 1-1810 | 3-$CHF_2O$-Ph | 2-MeNH-4-Pym | 1-Me-4-Pip-$CH_2$ |
| 1-1811 | 3-$CHF_2O$-Ph | 2-MeNH-4-Pym | 2-Piz-$CH_2$ |
| 1-1812 | 3-$CHF_2O$-Ph | 2-MeNH-4-Pym | 4-Pyr-$CH_2$ |
| 1-1813 | 3-$CHF_2O$-Ph | 2-MeNH-4-Pym | 3-Pyr-$CH_2$ |
| 1-1814 | 3-$CHF_2O$-Ph | 2-MeNH-4-Pym | 4-Pym-$CH_2$ |
| 1-1815 | 3-$CHF_2O$-Ph | 2-MeNH-4-Pym | 5-Pym-$CH_2$ |
| 1-1816 | 3-$CHF_2O$-Ph | 2-MeNH-4-Pym | 2-Pym-$CH_2$ |

Table 1 (continued)

| Compound No. | $R^1$ | $R^2$ | $R^3$ |
|---|---|---|---|
| 1-1817 | Ph | 2-$NH_2$-4-Pyr | $H_2N-(CH_2)_3$ |
| 1-1818 | Ph | 2-$NH_2$-4-Pyr | MeNH-$(CH_2)_3$ |
| 1-1819 | Ph | 2-$NH_2$-4-Pyr | EtNH-$(CH_2)_3$ |
| 1-1820 | Ph | 2-$NH_2$-4-Pyr | $Me_2N-(CH_2)_3$ |
| 1-1821 | Ph | 2-$NH_2$-4-Pyr | 1-Azt-$(CH_2)_3$ |
| 1-1822 | Ph | 2-$NH_2$-4-Pyr | 1-Pyrd-$(CH_2)_3$ |
| 1-1823 | Ph | 2-$NH_2$-4-Pyr | 1-Pip-$(CH_2)_3$ |
| 1-1824 | Ph | 2-$NH_2$-4-Pyr | 1-Mor-$(CH_2)_3$ |
| 1-1825 | Ph | 2-$NH_2$-4-Pyr | 1-Tmor-$(CH_2)_3$ |
| 1-1826 | Ph | 2-$NH_2$-4-Pyr | 1-Piz-$(CH_2)_3$ |
| 1-1827 | Ph | 2-$NH_2$-4-Pyr | 4-Me-1-Piz-$(CH_2)_3$ |
| 1-1828 | Ph | 2-$NH_2$-4-Pyr | 3-Azt |
| 1-1829 | Ph | 2-$NH_2$-4-Pyr | 1-Me-3-Azt |
| 1-1830 | Ph | 2-$NH_2$-4-Pyr | 3-Pyrd |
| 1-1831 | Ph | 2-$NH_2$-4-Pyr | 1-Me-3-Pyrd |
| 1-1832 | Ph | 2-$NH_2$-4-Pyr | 4-Pip |
| 1-1833 | Ph | 2-$NH_2$-4-Pyr | 4-(3, 4-deH-Pip) |
| 1-1834 | Ph | 2-$NH_2$-4-Pyr | 1-Me-4-Pip |
| 1-1835 | Ph | 2-$NH_2$-4-Pyr | 1-Me-4-(3,4-deH-Pip) |
| 1-1836 | Ph | 2-$NH_2$-4-Pyr | 1-Piz |
| 1-1837 | Ph | 2-$NH_2$-4-Pyr | 4-Me-1-Piz |
| 1-1838 | Ph | 2-$NH_2$-4-Pyr | 4-Pyr |
| 1-1839 | Ph | 2-$NH_2$-4-Pyr | 3-Pyr |
| 1-1840 | Ph | 2-$NH_2$-4-Pyr | 4-Pym |
| 1-1841 | Ph | 2-$NH_2$-4-Pyr | 5-Pym |
| 1-1842 | Ph | 2-$NH_2$-4-Pyr | 3-Pyrd-$CH_2$ |
| 1-1843 | Ph | 2-$NH_2$-4-Pyr | 1-Me-3-Pyrd-$CH_2$ |
| 1-1844 | Ph | 2-$NH_2$-4-Pyr | 4-Pip-$CH_2$ |
| 1-1845 | Ph | 2-$NH_2$-4-Pyr | 1-Me-4-Pip-$CH_2$ |
| 1-1846 | Ph | 2-$NH_2$-4-Pyr | 2-Piz-$CH_2$ |
| 1-1847 | Ph | 2-$NH_2$-4-Pyr | 4-Pyr-$CH_2$ |
| 1-1848 | Ph | 2-$NH_2$-4-Pyr | 3-Pyr-$CH_2$ |
| 1-1849 | Ph | 2-$NH_2$-4-Pyr | 4-Pym-$CH_2$ |
| 1-1850 | Ph | 2-$NH_2$-4-Pyr | 5-Pym-$CH_2$ |
| 1-1851 | Ph | 2-$NH_2$-4-Pyr | 2-Pym-$CH_2$ |
| 1-1852 | 4-F-Ph | 2-$NH_2$-4-Pyr | $H_2N-(CH_2)_3$ |
| 1-1853 | 4-F-Ph | 2-$NH_2$-4-Pyr | MeNH-$(CH_2)_3$ |
| 1-1854 | 4-F-Ph | 2-$NH_2$-4-Pyr | EtNH-$(CH_2)_3$ |

Table 1 (continued)

| Compound No. | $R^1$ | $R^2$ | $R^3$ |
|---|---|---|---|
| 1-1855 | 4-F-Ph | $2-NH_2-4-Pyr$ | $Me_2N-(CH_2)_3$ |
| 1-1856 | 4-F-Ph | $2-NH_2-4-Pyr$ | $1-Azt-(CH_2)_3$ |
| 1-1857 | 4-F-Ph | $2-NH_2-4-Pyr$ | $1-Pyrd-(CH_2)_3$ |
| 1-1858 | 4-F-Ph | $2-NH_2-4-Pyr$ | $1-Pip-(CH_2)_3$ |
| 1-1859 | 4-F-Ph | $2-NH_2-4-Pyr$ | $1-Mor-(CH_2)_3$ |
| 1-1860 | 4-F-Ph | $2-NH_2-4-Pyr$ | $1-Tmor-(CH_2)_3$ |
| 1-1861 | 4-F-Ph | $2-NH_2-4-Pyr$ | $1-Piz-(CH_2)_3$ |
| 1-1862 | 4-F-Ph | $2-NH_2-4-Pyr$ | $4-Me-1-Piz-(CH_2)_3$ |
| 1-1863 | 4-F-Ph | $2-NH_2-4-Pyr$ | 3-Azt |
| 1-1864 | 4-F-Ph | $2-NH_2-4-Pyr$ | 1-Me-3-Azt |
| 1-1865 | 4-F-Ph | $2-NH_2-4-Pyr$ | 3-Pyrd |
| 1-1866 | 4-F-Ph | $2-NH_2-4-Pyr$ | 1-Me-3-Pyrd |
| 1-1867 | 4-F-Ph | $2-NH_2-4-Pyr$ | 4-Pip |
| 1-1868 | 4-F-Ph | $2-NH_2-4-Pyr$ | 4-(3,4-deH-Pip) |
| 1-1869 | 4-F-Ph | $2-NH_2-4-Pyr$ | 1-Me-4-Pip |
| 1-1870 | 4-F-Ph | $2-NH_2-4-Pyr$ | 1-Me-4-(3,4-deH-Pip) |
| 1-1871 | 4-F-Ph | $2-NH_2-4-Pyr$ | 1-Piz |
| 1-1872 | 4-F-Ph | $2-NH_2-4-Pyr$ | 4-Me-1-Piz |
| 1-1873 | 4-F-Ph | $2-NH_2-4-Pyr$ | 4-Pyr |
| 1-1874 | 4-F-Ph | $2-NH_2-4-Pyr$ | 3-Pyr |
| 1-1875 | 4-P-Ph | $2-NH_2-4-Pyr$ | 4-Pym |
| 1-1876 | 4-F-Ph | $2-NH_2-4-Pyr$ | 5-Pym |
| 1-1877 | 4-F-Ph | $2-NH_2-4-Pyr$ | $3-Pyrd-CH_2$ |
| 1-1878 | 4-F-Ph | $2-NH_2-4-Pyr$ | $1-Me-3-Pyrd-CH_2$ |
| 1-1879 | 4-F-Ph | $2-NH_2-4-Pyr$ | $4-Pip-CH_2$ |
| 1-1880 | 4-F-Ph | $2-NH_2-4-Pyr$ | $1-Me-4-Pip-CH_2$ |
| 1-1881 | 4-F-Ph | $2-NH_2-4-Pyr$ | $2-Piz-CH_2$ |
| 1-1882 | 4-F-Ph | $2-NH_2-4-Pyr$ | $4-Pyr-CH_2$ |
| 1-1883 | 4-F-Ph | $2-NH_2-4-Pyr$ | $3-Pyr-CH_2$ |
| 1-1884 | 4-F-Ph | $2-NH_2-4-Pyr$ | $4-Pym-CH_2$ |
| 1-1885 | 4-F-Ph | $2-NH_2-4-Pyr$ | $5-Pym-CH_2$ |
| 1-1886 | 4-F-Ph | $2-NH_2-4-Pyr$ | $2-Pym-CH_2$ |
| 1-1887 | 3-F-Ph | $2-NH_2-4-Pyr$ | $H_2N-(CH_2)_3$ |
| 1-1888 | 3-F-Ph | $2-NH_2-4-Pyr$ | $MeNH-(CH_2)_3$ |
| 1-1889 | 3-F-Ph | $2-NH_2-4-Pyr$ | $EtNH-(CH_2)_3$ |
| 1-1890 | 3-F-Ph | $2-NH_2-4-Pyr$ | $Me_2N-(CH_2)_3$ |
| 1-1891 | 3-F-Ph | $2-NH_2-4-Pyr$ | $1-Azt-(CH_2)_3$ |
| 1-1892 | 3-F-Ph | $2-NH_2-4-Pyr$ | $1-Pyrd-(CH_2)_3$ |

Table 1 (continued)

| Compound No. | $R^1$ | $R^2$ | $R^3$ |
|---|---|---|---|
| 1-1893 | 3-F-Ph | $2-NH_2-4-Pyr$ | $1-Pip-(CH_2)_3$ |
| 1-1894 | 3-F-Ph | $2-NH_2-4-Pyr$ | $1-Mor-(CH_2)_3$ |
| 1-1895 | 3-F-Ph | $2-NH_2-4-Pyr$ | $1-Tmor-(CH_2)_3$ |
| 1-1896 | 3-F-Ph | $2-NH_2-4-Pyr$ | $1-Piz-(CH_2)_3$ |
| 1-1897 | 3-F-Ph | $2-NH_2-4-Pyr$ | $4-Me-1-Piz-(CH_2)_3$ |
| 1-1898 | 3-F-Ph | $2-NH_2-4-Pyr$ | 3-Azt |
| 1-1899 | 3-F-Ph | $2-NH_2-4-Pyr$ | 1-Me-3-Azt |
| 1-1900 | 3-F-Ph | $2-NH_2-4-Pyr$ | 3-Pyrd |
| 1-1901 | 3-F-Ph | $2-NH_2-4-Pyr$ | 1-Me-3-Pyrd |
| 1-1902 | 3-F-Ph | $2-NH_2-4-Pyr$ | 4-Pip |
| 1-1903 | 3-F-Ph | $2-NH_2-4-Pyr$ | 4-(3,4-deH-Pip) |
| 1-1904 | 3-F-Ph | $2-NH_2-4-Pyr$ | 1-Me-4-Pip |
| 1-1905 | 3-F-Ph | $2-NH_2-4-Pyr$ | 1-Me-4-(3,4-deH-Pip) |
| 1-1906 | 3-F-Ph | $2-NH_2-4-Pyr$ | 1-Piz |
| 1-1907 | 3-F-Ph | $2-NH_2-4-Pyr$ | 4-Me-1-Piz |
| 1-1908 | 3-F-Ph | $2-NH_2-4-Pyr$ | 4-Pyr |
| 1-1909 | 3-F-Ph | $2-NH_2-4-Pyr$ | 3-Pyr |
| 1-1910 | 3-F-Ph | $2-NH_2-4-Pyr$ | 4-Pym |
| 1-1911 | 3-F-Ph | $2-NH_2-4-Pyr$ | 5-Pym |
| 1-1912 | 3-F-Ph | $2-NH_2-4-Pyr$ | $3-Pyrd-CH_2$ |
| 1-1913 | 3-F-Ph | $2-NH_2-4-Pyr$ | $1-Me-3-Pyrd-CH_2$ |
| 1-1914 | 3-F-Ph | $2-NH_2-4-Pyr$ | $4-Pip-CH_2$ |
| 1-1915 | 3-F-Ph | $2-NH_2-4-Pyr$ | $1-Me-4-Pip-CH_2$ |
| 1-1916 | 3-F-Ph | $2-NH_2-4-Pyr$ | $2-Piz-CH_2$ |
| 1-1917 | 3-F-Ph | $2-NH_2-4-Pyr$ | $4-Pyr-CH_2$ |
| 1-1918 | 3-F-Ph | $2-NH_2-4-Pyr$ | $3-Pyr-CH_2$ |
| 1-1919 | 3-F-Ph | $2-NH_2-4-Pyr$ | $4-Pym-CH_2$ |
| 1-1920 | 3-F-Ph | $2-NH_2-4-Pyr$ | $5-Pym-CH_2$ |
| 1-1921 | 3-F-Ph | $2-NH_2-4-Pyr$ | $2-Pym-CH_2$ |
| 1-1922 | 3,4-diF-Ph | $2-NH_2-4-Pyr$ | $H_2N-(CH_2)_3$ |
| 1-1923 | 3,4-diF-Ph | $2-NH_2-4-Pyr$ | $MeNH-(CH_2)_3$ |
| 1-1924 | 3,4-diF-Ph | $2-NH_2-4-Pyr$ | $EtNH-(CH_2)_3$ |
| 1-1925 | 3,4-diF-Ph | $2-NH_2-4-Pyr$ | $Me_2N-(CH_2)_3$ |
| 1-1926 | 3,4-diF-Ph | $2-NH_2-4-Pyr$ | $1-Azt-(CH_2)_3$ |
| 1-1927 | 3,4-diF-Ph | $2-NH_2-4-Pyr$ | $1-Pyrd-(CH_2)_3$ |
| 1-1928 | 3,4-diF-Ph | $2-NH_2-4-Pyr$ | $1-Pip-(CH_2)_3$ |
| 1-1929 | 3,4-diF-Ph | $2-NH_2-4-Pyr$ | $1-Mor-(CH_2)_3$ |
| 1-1930 | 3,4-diF-Ph | $2-NH_2-4-Pyr$ | $1-Tmor-(CH_2)_3$ |

Table 1 (continued)

| Compound No. | $R^1$ | $R^2$ | $R^3$ |
|---|---|---|---|
| 1-1931 | 3,4-diF-Ph | $2-NH_2-4-Pyr$ | $1-Piz-(CH_2)_3$ |
| 1-1932 | 3,4-diF-Ph | $2-NH_2-4-Pyr$ | $4-Me-1-Piz-(CH_2)_3$ |
| 1-1933 | 3,4-diF-Ph | $2-NH_2-4-Pyr$ | 3-Azt |
| 1-1934. | 3,4-diF-Ph | $2-NH_2-4-Pyr$ | 1-Me-3-Azt |
| 1-1935 | 3,4-diF-Ph | $2-NH_2-4-Pyr$ | 3-Pyrd |
| 1-1936 | 3,4-diF-Ph | $2-NH_2-4-Pyr$ | 1-Me-3-Pyrd |
| 1-1937 | 3,4-diF-Ph | $2-NH_2-4-Pyr$ | 4-Pip |
| 1-1938 | 3,4-diF-Ph | $2-NH_2-4-Pyr$ | 4-(3,4-deH-Pip) |
| 1-1939 | 3,4-diF-Ph | $2-NH_2-4-Pyr$ | 1-Me-4-Pip |
| 1-1940 | 3,4-diF-Ph | $2-NH_2-4-Pyr$ | 1-Me-4-(3,4-deH-Pip) |
| 1-1941 | 3,4-diF-Ph | $2-NH_2-4-Pyr$ | 1-Piz |
| 1-1942 | 3,4-diF-Ph | $2-NH_2-4-Pyr$ | 4-Me-1-Piz |
| 1-1943 | 3,4-diF-Ph | $2-NH_2-4-Pyr$ | 4-Pyr |
| 1-1944 | 3,4-diF-Ph | $2-NH_2-4-Pyr$ | 3-Pyr |
| 1-1945 | 3,4-diF-Ph | $2-NH_2-4-Pyr$ | 4-Pym |
| 1-1946 | 3,4-diF-Ph | $2-NH_2-4-Pyr$ | 5-Pym |
| 1-1947 | 3,4-diF-Ph | $2-NH_2-4-Pyr$ | $3-Pyrd-CH_2$ |
| 1-1948 | 3,4-diF-Ph | $2-NH_2-4-Pyr$ | $1-Me-3-Pyrd-CH_2$ |
| 1-1949 | 3,4-diF-Ph | $2-NH_2-4-Pyr$ | $4-Pip-CH_2$ |
| 1-1950 | 3,4-diF-Ph | $2-NH_2-4-Pyr$ | $1-Me-4-Pip-CH_2$ |
| 1-1951 | 3,4-diF-Ph | $2-NH_2-4-Pyr$ | $2-Piz-CH_2$ |
| 1-1952 | 3,4-diF-Ph | $2-NH_2-4-Pyr$ | $4-Pyr-CH_2$ |
| 1-1953 | 3,4-diF-Ph | $2-NH_2-4-Pyr$ | $3-Pyr-CH_2$ |
| 1-1954 | 3,4-diF-Ph | $2-NH_2-4-Pyr$ | $4-Pym-CH_2$ |
| 1-1955 | 3,4-diF-Ph | $2-NH_2-4-Pyr$ | $5-Pym-CH_2$ |
| 1-1956 | 3,4-diF-Ph | $2-NH_2-4-Pyr$ | $2-Pym-CH_2$ |
| 1-1957 | 3-Cl-Ph | $2-NH_2-4-Pyr$ | $H_2N-(CH_2)_3$ |
| 1-1958 | 3-Cl-Ph | $2-NH_2-4-Pyr$ | $MeNH-(CH_2)_3$ |
| 1-1959 | 3-Cl-Ph | $2-NH_2-4-Pyr$ | $EtNH-(CH_2)_3$ |
| 1-1960 | 3-Cl-Ph | $2-NH_2-4-Pyr$ | $Me_2N-(CH_2)_3$ |
| 1-1961 | 3-Cl-Ph | $2-NH_2-4-Pyr$ | $1-Azt-(CH_2)_3$ |
| 1-1962 | 3-Cl-Ph | $2-NH_2-4-Pyr$ | $1-Pyrd-(CH_2)_3$ |
| 1-1963 | 3-Cl-Ph | $2-NH_2-4-Pyr$ | $1-Pip-(CH_2)_3$ |
| 1-1964 | 3-Cl-Ph | $2-NH_2-4-Pyr$ | $1-Mor-(CH_2)_3$ |
| 1-1965 | 3-Cl-Ph | $2-NH_2-4-Pyr$ | $1-Tmor-(CH_2)_3$ |
| 1-1966 | 3-Cl-Ph | $2-NH_2-4-Pyr$ | $1-Piz-(CH_2)_3$ |
| 1-1967 | 3-Cl-Ph | $2-NH_2-4-Pyr$ | $4-Me-1-Piz-(CH_2)_3$ |
| 1-1968 | 3-Cl-Ph | $2-NH_2-4-Pyr$ | 3-Azt |

Table 1 (continued)

| Compound No. | $R^1$ | $R^2$ | $R^3$ |
|---|---|---|---|
| 1-1969 | 3-Cl-Ph | $2-NH_2-4-Pyr$ | 1-Me-3-Azt |
| 1-1970 | 3-Cl-Ph | $2-NH_2-4-Pyr$ | 3-Pyrd |
| 1-1971 | 3-Cl-Ph | $2-NH_2-4-Pyr$ | 1-Me-3-Pyrd |
| 1-1972 | 3-Cl-Ph | $2-NH_2-4-Pyr$ | 4-Pip |
| 1-1973 | 3-Cl-Ph | $2-NH_2-4-Pyr$ | 4-(3,4-deH-Pip) |
| 1-1974 | 3-Cl-Ph | $2-NH_2-4-Pyr$ | 1-Me-4-Pip |
| 1-1975 | 3-Cl-Ph | $2-NH_2-4-Pyr$ | 1-Me-4-(3,4-deH-Pip) |
| 1-1976 | 3-Cl-Ph | $2-NH_2-4-Pyr$ | 1-Piz |
| 1-1977 | 3-Cl-Ph | $2-NH_2-4-Pyr$ | 4-Me-1-Piz |
| 1-1978 | 3-Cl-Ph | $2-NH_2-4-Pyr$ | 4-Pyr |
| 1-1979 | 3-Cl-Ph | $2-NH_2-4-Pyr$ | 3-Pyr |
| 1-1980 | 3-Cl-Ph | $2-NH_2-4-Pyr$ | 4-Pym |
| 1-1981 | 3-Cl-Ph | $2-NH_2-4-Pyr$ | 5-Pym |
| 1-1982 | 3-Cl-Ph | $2-NH_2-4-Pyr$ | $3-Pyrd-CH_2$ |
| 1-1983 | 3-Cl-Ph | $2-NH_2-4-Pyr$ | $1-Me-3-Pyrd-CH_2$ |
| 1-1984 | 3-Cl-Ph | $2-NH_2-4-Pyr$ | $4-Pip-CH_2$ |
| 1-1985 | 3-Cl-Ph | $2-NH_2-4-Pyr$ | $1-Me-4-Pip-CH_2$ |
| 1-1986 | 3-Cl-Ph | $2-NH_2-4-Pyr$ | $2-Piz-CH_2$ |
| 1-1987 | 3-Cl-Ph | $2-NH_2-4-Pyr$ | $4-Pyr-CH_2$ |
| 1-1988 | 3-Cl-Ph | $2-NH_2-4-Pyr$ | $3-Pyr-CH_2$ |
| 1-1989 | 3-Cl-Ph | $2-NH_2-4-Pyr$ | $4-Pym-CH_2$ |
| 1-1990 | 3-Cl-Ph | $2-NH_2-4-Pyr$ | $5-Pym-CH_2$ |
| 1-1991 | 3-Cl-Ph | $2-NH_2-4-Pyr$ | $2-Pym-CH_2$ |
| 1-1992 | Ph | 2-MeNH-4-Pyr | $H_2N-(CH_2)_3$ |
| 1-1993 | Ph | 2-MeNH-4-Pyr | $MeNH-(CH_2)_3$ |
| 1-1994 | Ph | 2-MeNH-4-Pyr | $EtNH-(CH_2)_3$ |
| 1-1995 | Ph | 2-MeNH-4-Pyr | $Me_2N-(CH_2)_3$ |
| 1-1996 | Ph | 2-MeNH-4-Pyr | $1-Azt-(CH_2)_3$ |
| 1-1997 | Ph | 2-MeNH-4-Pyr | $1-Pyrd-(CH_2)_3$ |
| 1-1998 | Ph | 2-MeNH-4-Pyr | $1-Pip-(CH_2)_3$ |
| 1-1999 | Ph | 2-MeNH-4-Pyr | $1-Mor-(CH_2)_3$ |
| 1-2000 | Ph | 2-MeNH-4-Pyr | $1-Tmor-(CH_2)_3$ |
| 1-2001 | Ph | 2-MeNH-4-Pyr | $1-Piz-(CH_2)_3$ |
| 1-2002 | Ph | 2-MeNH-4-Pyr | $4-Me-1-Piz-(CH_2)_3$ |
| 1-2003 | Ph | 2-MeNH-4-Pyr | 3-Azt |
| 1-2004 | Ph | 2-MeNH-4-Pyr | 1-Me-3-Azt |
| 1-2005 | Ph | 2-MeNH-4-Pyr | 3-Pyrd |
| 1-2006 | Ph | 2-MeNH-4-Pyr | 1-Me-3-Pyrd |

Table 1 (continued)

| Compound No. | $R^1$ | $R^2$ | $R^3$ |
|---|---|---|---|
| 1-2007 | Ph | 2-MeNH-4-Pyr | 4-Pip |
| 1-2008 | Ph | 2-MeNH-4-Pyr | 4-(3,4-deH-Pip) |
| 1-2009 | Ph | 2-MeNH-4-Pyr | 1-Me-4-Pip |
| 1-2010 | Ph | 2-MeNH-4-Pyr | 1-Me-4-(3,4-deH-Pip) |
| 1-2011 | Ph | 2-MeNH-4-Pyr | 1-Piz |
| 1-2012 | Ph | 2-MeNH-4-Pyr | 4-Me-1-Piz |
| 1-2013 | Ph | 2-MeNH-4-Pyr | 4-Pyr |
| 1-2014 | Ph | 2-MeNH-4-Pyr | 3-Pyr |
| 1-2015 | Ph | 2-MeNH-4-Pyr | 4-Pym |
| 1-2016 | Ph | 2-MeNH-4-Pyr | 5-Pym |
| 1-2017 | Ph | 2-MeNH-4-Pyr | 3-Pyrd-$CH_2$ |
| 1-2018 | Ph | 2-MeNH-4-Pyr | 1-Me-3-Pyrd-$CH_2$ |
| 1-2019 | Ph | 2-MeNH-4-Pyr | 4-Pip-$CH_2$ |
| 1-2020 | Ph | 2-MeNH-4-Pyr | 1-Me-4-Pip-$CH_2$ |
| 1-2021 | Ph | 2-MeNH-4-Pyr | 2-Piz-$CH_2$ |
| 1-2022 | Ph | 2-MeNH-4-Pyr | 4-Pyr-$CH_2$ |
| 1-2023 | Ph | 2-MeNH-4-Pyr | 3-Pyr-$CH_2$ |
| 1-2024 | Ph | 2-MeNH-4-Pyr | 4-Pym-$CH_2$ |
| 1-2025 | Ph | 2-MeNH-4-Pyr | 5-Pym-$CH_2$ |
| 1-2026 | Ph | 2-MeNH-4-Pyr | 2-Pym-$CH_2$ |
| 1-2027 | 4-F-Ph | 2-MeNH-4-Pyr | $H_2N-(CH_2)_3$ |
| 1-2028 | 4-F-Ph | 2-MeNH-4-Pyr | MeNH-$(CH_2)_3$ |
| 1-2029 | 4-F-Ph | 2-MeNH-4-Pyr | EtNH-$(CH_2)_3$ |
| 1-2030 | 4-F-Ph | 2-MeNH-4-Pyr | $Me_2N-(CH_2)_3$ |
| 1-2031 | 4-F-Ph | 2-MeNH-4-Pyr | 1-Azt-$(CH_2)_3$ |
| 1-2032 | 4-F-Ph | 2-MeNH-4-Pyr | 1-Pyrd-$(CH_2)_3$ |
| 1-2033 | 4-F-Ph | 2-MeNH-4-Pyr | 1-Pip-$(CH_2)_3$ |
| 1-2034 | 4-F-Ph | 2-MeNH-4-Pyr | 1-Mor-$(CH_2)_3$ |
| 1-2035 | 4-F-Ph | 2-MeNH-4-Pyr | 1-Tmor-$(CH_2)_3$ |
| 1-2036 | 4-F-Ph | 2-MeNH-4-Pyr | 1-Piz-$(CH_2)_3$ |
| 1-2037 | 4-F-Ph | 2-MeNH-4-Pyr | 4-Me-1-Piz-$(CH_2)_3$ |
| 1-2038 | 4-F-Ph | 2-MeNH-4-Pyr | 3-Azt |
| 1-2039 | 4-F-Ph | 2-MeNH-4-Pyr | 1-Me-3-Azt |
| 1-2040 | 4-F-Ph | 2-MeNH-4-Pyr | 3-Pyrd |
| 1-2041 | 4-F-Ph | 2-MeNH-4-Pyr | 1-Me-3-Pyrd |
| 1-2042 | 4-F-Ph | 2-MeNH-4-Pyr | 4-Pip |
| 1-2043 | 4-F-Ph | 2-MeNH-4-Pyr | 4-(3,4-deH-Pip) |
| 1-2044 | 4-F-Ph | 2-MeNH-4-Pyr | 1-Me-4-Pip |

Table 1 (continued)

| Compound No. | $R^1$ | $R^2$ | $R^3$ |
|---|---|---|---|
| 1-2045 | 4-F-Ph | 2-MeNH-4-Pyr | 1-Me-4-(3,4-deH-Pip) |
| 1-2046 | 4-F-Ph | 2-MeNH-4-Pyr | 1-Piz |
| 1-2047 | 4-F-Ph | 2-MeNH-4-Pyr | 4-Me-1-Piz |
| 1-2048 | 4-F-Ph | 2-MeNH-4-Pyr | 4-Pyr |
| 1-2049 | 4-F-Ph | 2-MeNH-4-Pyr | 3-Pyr |
| 1-2050 | 4-F-Ph | 2-MeNH-4-Pyr | 4-Pym |
| 1-2051 | 4-F-Ph | 2-MeNH-4-Pyr | 5-Pym |
| 1-2052 | 4-F-Ph | 2-MeNH-4-Pyr | 3-Pyrd-$CH_2$ |
| 1-2053 | 4-F-Ph | 2-MeNH-4-Pyr | 1-Me-3-Pyrd-$CH_2$ |
| 1-2054 | 4-F-Ph | 2-MeNH-4-Pyr | 4-Pip-$CH_2$ |
| 1-2055 | 4-F-Ph | 2-MeNH-4-Pyr | 1-Me-4-Pip-$CH_2$ |
| 1-2056 | 4-F-Ph | 2-MeNH-4-Pyr | 2-Piz-$CH_2$ |
| 1-2057 | 4-F-Ph | 2-MeNH-4-Pyr | 4-Pyr-$CH_2$ |
| 1-2058 | 4-F-Ph | 2-MeNH-4-Pyr | 3-Pyr-$CH_2$ |
| 1-2059 | 4-F-Ph | 2-MeNH-4-Pyr | 4-Pym-$CH_2$ |
| 1-2060 | 4-F-Ph | 2-MeNH-4-Pyr | 5-Pym-$CH_2$ |
| 1-2061 | 4-F-Ph | 2-MeNH-4-Pyr | 2-Pym-$CH_2$ |
| 1-2062 | 3-F-Ph | 2-MeNH-4-Pyr | $H_2N$-$(CH_2)_3$ |
| 1-2063 | 3-F-Ph | 2-MeNH-4-Pyr | MeNH-$(CH_2)_3$ |
| 1-2064 | 3-F-Ph | 2-MeNH-4-Pyr | EtNH-$(CH_2)_3$ |
| 1-2065 | 3-F-Ph | 2-MeNH-4-Pyr | $Me_2N$-$(CH_2)_3$ |
| 1-2066 | 3-F-Ph | 2-MeNH-4-Pyr | 1-Azt-$(CH_2)_3$ |
| 1-2067 | 3-F-Ph | 2-MeNH-4-Pyr | 1-Pyrd-$(CH_2)_3$ |
| 1-2068 | 3-F-Ph | 2-MeNH-4-Pyr | 1-Pip-$(CH_2)_3$ |
| 1-2069 | 3-F-Ph | 2-MeNH-4-Pyr | 1-Mor-$(CH_2)_3$ |
| 1-2070 | 3-F-Ph | 2-MeNH-4-Pyr | 1-Tmor-$(CH_2)_3$ |
| 1-2071 | 3-F-Ph | 2-MeNH-4-Pyr | 1-Piz-$(CH_2)_3$ |
| 1-2072 | 3-F-Ph | 2-MeNH-4-Pyr | 4-Me-1-Piz-$(CH_2)_3$ |
| 1-2073 | 3-F-Ph | 2-MeNH-4-Pyr | 3-Azt |
| 1-2074 | 3-F-Ph | 2-MeNH-4-Pyr | 1-Me-3-Azt |
| 1-2075 | 3-F-Ph | 2-MeNH-4-Pyr | 3-Pyrd |
| 1-2076 | 3-F-Ph | 2-MeNH-4-Pyr | 1-Me-3-Pyrd |
| 1-2077 | 3-F-Ph | 2-MeNH-4-Pyr | 4-Pip |
| 1-2078 | 3-F-Ph | 2-MeNH-4-Pyr | 4-(3,4-deH-Pip) |
| 1-2079 | 3-F-Ph | 2-MeNH-4-Pyr | 1-Me-4-Pip |
| 1-2080 | 3-F-Ph | 2-MeNH-4-Pyr | 1-Me-4-(3,4-deH-Pip) |
| 1-2081 | 3-F-Ph | 2-MeNH-4-Pyr | 1-Piz |
| 1-2082 | 3-F-Ph | 2-MeNH-4-Pyr | 4-Me-1-Piz |

Table 1 (continued)

| Compound No. | $R^1$ | $R^2$ | $R^3$ |
|---|---|---|---|
| 1-2083 | 3-F-Ph | 2-MeNH-4-Pyr | 4-Pyr |
| 1-2084 | 3-F-Ph | 2-MeNH-4-Pyr | 3-Pyr |
| 1-2085 | 3-F-Ph | 2-MeNH-4-Pyr | 4-Pym |
| 1-2086 | 3-F-Ph | 2-MeNH-4-Pyr | 5-Pym |
| 1-2087 | 3-F-Ph | 2-MeNH-4-Pyr | 3-pyrd-$CH_2$ |
| 1-2088 | 3-F-Ph | 2-MeNH-4-Pyr | 1-Me-3-Pyrd-$CH_2$ |
| 1-2089 | 3-F-Ph | 2-MeNH-4-Pyr | 4-Pip-$CH_2$ |
| 1-2090 | 3-P-Ph | 2-MeNH-4-Pyr | 1-Me-4-Pip-$CH_2$ |
| 1-2091 | 3-F-Ph | 2-MeNH-4-Pyr | 2-Piz-$CH_2$ |
| 1-2092 | 3-F-Ph | 2-MeNH-4-Pyr | 4-Pyr-$CH_2$ |
| 1-2093 | 3-F-Ph | 2-MeNH-4-Pyr | 3-Pyr-$CH_2$ |
| 1-2094 | 3-F-Ph | 2-MeNH-4-Pyr | 4-Pym-$CH_2$ |
| 1-2095 | 3-F-Ph | 2-MeNH-4-Pyr | 5-Pym-$CH_2$ |
| 1-2096 | 3-F-Ph | 2-MeNH-4-Pyr | 2-Pym-$CH_2$ |
| 1-2097 | 3,4-diF-Ph | 2-MeNH-4-Pyr | $H_2N-(CH_2)_3$ |
| 1-2098 | 3,4-diF-Ph | 2-MeNH-4-Pyr | $MeNH-(CH_2)_3$ |
| 1-2099 | 3,4-diF-Ph | 2-MeNH-4-Pyr | $EtNH-(CH_2)_3$ |
| 1-2100 | 3,4-diF-Ph | 2-MeNH-4-Pyr | $Me_2N-(CH_2)_3$ |
| 1-2101 | 3,4-diF-Ph | 2-MeNH-4-Pyr | 1-Azt-$(CH_2)_3$ |
| 1-2102 | 3,4-diF-Ph | 2-MeNH-4-Pyr | 1-Pyrd-$(CH_2)_3$ |
| 1-2103 | 3/4-diF-Ph | 2-MeNH-4-Pyr | 1-Pip-$(CH_2)_3$ |
| 1-2104 | 3,4-diF-Ph | 2-MeNH-4-Pyr | 1-Mor-$(CH_2)_3$ |
| 1-2105 | 3,4-diF-Ph | 2-MeNH-4-Pyr | 1-Tmor-$(CH_2)_3$ |
| 1-2106 | 3,4-diF-Ph | 2-MeNH-4-Pyr | 1-Piz-$(CH_2)_3$ |
| 1-2107 | 3,4-diF-Ph | 2-MeNH-4-Pyr | 4-Me-1-Piz-$(CH_2)_3$ |
| 1-2108 | 3,4-diF-Ph | 2-MeNH-4-Pyr | 3-Azt |
| 1-2109 | 3,4-diF-Ph | 2-MeNH-4-Pyr | 1-Me-3-Azt |
| 1-2110 | 3,4-diF-Ph | 2-MeNH-4-Pyr | 3-Pyrd |
| 1-2111 | 3,4-diF-Ph | 2-MeNH-4-Pyr | 1-Me-3-Pyrd |
| 1-2112 | 3,4-diF-Ph | 2-MeNH-4-Pyr | 4-Pip |
| 1-2113 | 3,4-diF-Ph | 2-MeNH-4-Pyr | 4-(3,4-deH-Pip) |
| 1-2114 | 3,4-diF-Ph | 2-MeNH-4-Pyr | 1-Me-4-Pip |
| 1-2115 | 3,4-diF-Ph | 2-MeNH-4-Pyr | 1-Me-4-(3,4-deH-Pip) |
| 1-2116 | 3,4-diF-Ph | 2-MeNH-4-Pyr | 1-Piz |
| 1-2117 | 3,4-diF-Ph | 2-MeNH-4-Pyr | 4-Me-1-Piz |
| 1-2118 | 3,4-diF-Ph | 2-MeNH-4-Pyr | 4-Pyr |
| 1-2119 | 3,4-diF-Ph | 2-MeNH-4-Pyr | 3-Pyr |
| 1-2120 | 3,4-diF-Ph | 2-MeNH-4-Pyr | 4-Pym |

Table 1 (continued)

| Compound No. | $R^1$ | $R^2$ | $R^3$ |
|---|---|---|---|
| 1-2121 | 3,4-diF-Ph | 2-MeNH-4-Pyr | 5-Pym |
| 1-2122 | 3,4-diF-Ph | 2-MeNH-4-Pyr | 3-Pyrd-$CH_2$ |
| 1-2123 | 3,4-diF-Ph | 2-MeNH-4-Pyr | 1-Me-3-Pyrd-$CH_2$ |
| 1-2124 | 3,4-diF-Ph | 2-MeNH-4-Pyr | 4-Pip-$CH_2$ |
| 1-2125 | 3,4-diF-Ph | 2-MeNH-4-Pyr | 1-Me-4-Pip-$CH_2$ |
| 1-2126 | 3,4-diF-Ph | 2-MeNH-4-Pyr | 2-Piz-$CH_2$ |
| 1-2127 | 3,4-diF-Ph | 2-MeNH-4-Pyr | 4-Pyr-$CH_2$ |
| 1-2128 | 3,4-diF-Ph | 2-MeNH-4-Pyr | 3-Pyr-$CH_2$ |
| 1-2129 | 3,4-diF-Ph | 2-MeNH-4-Pyr | 4-Pym-$CH_2$ |
| 1-2130 | 3,4-diF-Ph | 2-MeNH-4-Pyr | 5-Pym-$CH_2$ |
| 1-2131 | 3,4-diF-Ph | 2-MeNH-4-Pyr | 2-Pym-$CH_2$ |
| 1-2132 | 3-Cl-Ph | 2-MeNH-4-Pyr | $H_2N-(CH_2)_3$ |
| 1-2133 | 3-Cl-Ph | 2-MeNH-4-Pyr | MeNH-$(CH_2)_3$ |
| 1-2134 | 3-Cl-Ph | 2-MeNH-4-Pyr | EtNH-$(CH_2)_3$ |
| 1-2135 | 3-Cl-Ph | 2-MeNH-4-Pyr | $Me_2N-(CH_2)_3$ |
| 1-2136 | 3-Cl-Ph | 2-MeNH-4-Pyr | 1-Azt-$(CH_2)_3$ |
| 1-2137 | 3-Cl-Ph | 2-MeNH-4-Pyr | 1-Pyrd-$(CH_2)_3$ |
| 1-2138 | 3-Cl-Ph | 2-MeNH-4-Pyr | 1-Pip-$(CH_2)_3$ |
| 1-2139 | 3-Cl-Ph | 2-MeNH-4-Pyr | 1-Mor-$(CH_2)_3$ |
| 1-2140 | 3-Cl-Ph | 2-MeNH-4-Pyr | 1-Tmor-$(CH_2)_3$ |
| 1-2141 | 3-Cl-Ph | 2-MeNH-4-Pyr | 1-Piz-$(CH_2)_3$ |
| 1-2142 | 3-Cl-Ph | 2-MeNH-4-Pyr | 4-Me-1-Piz-$(CH_2)_3$ |
| 1-2143 | 3-Cl-Ph | 2-MeNH-4-Pyr | 3-Azt |
| 1-2144 | 3-Cl-Ph | 2-MeNH-4-Pyr | 1-Me-3-Azt |
| 1-2145 | 3-Cl-Ph | 2-MeNH-4-Pyr | 3-Pyrd |
| 1-2146 | 3-Cl-Ph | 2-MeNH-4-Pyr | 1-Me-3-Pyrd |
| 1-2147 | 3-Cl-Ph | 2-MeNH-4-Pyr | 4-Pip |
| 1-2148 | 3-Cl-Ph | 2-MeNH-4-Pyr | 4-(3,4-deH-Pip) |
| 1-2149 | 3-Cl-Ph | 2-MeNH-4-Pyr | 1-Me-4-Pip |
| 1-2150 | 3-Cl-Ph | 2-MeNH-4-Pyr | 1-Me-4-(3,4-deH-Pip) |
| 1-2151 | 3-Cl-Ph | 2-MeNH-4-Pyr | 1-Piz |
| 1-2152 | 3-Cl-Ph | 2-MeNH-4-Pyr | 4-Me-1-Piz |
| 1-2153 | 3-Cl-Ph | 2-MeNH-4-Pyr | 4-Pyr |
| 1-2154 | 3-Cl-Ph | 2-MeNH-4-Pyr | 3-Pyr |
| 1-2155 | 3-Cl-Ph | 2-MeNH-4-Pyr | 4-Pym |
| 1-2156 | 3-Cl-Ph | 2-MeNH-4-Pyr | 5-Pym |
| 1-2157 | 3-Cl-Ph | 2-MeNH-4-Pyr | 3-Pyrd-$CH_2$ |
| 1-2158 | 3-Cl-Ph | 2-MeNH-4-Pyr | 1-Me-3-Pyrd-$CH_2$ |

Table 1 (continued)

| Compound No. | $R^1$ | $R^2$ | $R^3$ |
|---|---|---|---|
| 1-2159 | 3-Cl-Ph | 2-MeNH-4-Pyr | 4-Pip-$CH_2$ |
| 1-2160 | 3-Cl-Ph | 2-MeNH-4-Pyr | 1-Me-4-Pip-$CH_2$ |
| 1-2161 | 3-Cl-Ph | 2-MeNH-4-Pyr | 2-Piz-$CH_2$ |
| 1-2162 | 3-Cl-Ph | 2-MeNH-4-Pyr | 4-Pyr-$CH_2$ |
| 1-2163 | 3-Cl-Ph | 2-MeNH-4-Pyr | 3-Pyr-$CH_2$ |
| 1-2164 | 3-Cl-Ph | 2-MeNH-4-Pyr | 4-Pym-$CH_2$ |
| 1-2165 | 3-Cl-Ph | 2-MeNH-4-Pyr | 5-Pym-$CH_2$ |
| 1-2166 | 3-Cl-Ph | 2-MeNH-4-Pyr | 2-Pym-$CH_2$ |
| 1-2167 | Ph | 4-Pym | $H_2N-(CH_2)_3$ |
| 1-2168 | Ph | 4-Pym | MeNH-$(CH_2)_3$ |
| 1-2169 | Ph | 4-Pym | EtNH-$(CH_2)_3$ |
| 1-2170 | Ph | 4-Pym | $Me_2N-(CH_2)_3$ |
| 1-2171 | Ph | 4-Pym | 1-Azt-$(CH_2)_3$ |
| 1-2172 | Ph | 4-Pym | 1-Pyrd-$(CH_2)_3$ |
| 1-2173 | Ph | 4-Pym | 1-Pip-$(CH_2)_3$ |
| 1-2174 | Ph | 4-Pym | 1-Mor-$(CH_2)_3$ |
| 1-2175 | Ph | 4-Pym | 1-Tmor-$(CH_2)_3$ |
| 1-2176 | Ph | 4-Pym | 1-Piz-$(CH_2)_3$ |
| 1-2177 | Ph | 4-Pym | 4-Me-1-Piz-$(CH_2)_3$ |
| 1-2178 | Ph | 4-Pym | 3-Azt |
| 1-2179 | Ph | 4-Pym | 1-Me-3-Azt |
| 1-2180 | Ph | 4-Pym | 3-Pyrd |
| 1-2181 | Ph | 4-Pym | 1-Me-3-Pyrd |
| 1-2182 | Ph | 4-Pym | 4-Pip |
| 1-2183 | Ph | 4-Pym | 4-(3,4-deH-Pip) |
| 1-2184 | Ph | 4-Pym | 1-Me-4-Pip |
| 1-2185 | Ph | 4-Pym | 1-Me-4-(3,4-deH-Pip) |
| 1-2186 | Ph | 4-Pym | 1-Piz |
| 1-2187 | Ph | 4-Pym | 4-Me-1-Piz |
| 1-2188 | Ph | 4-Pym | 4-Pyr |
| 1-2189 | Ph | 4-Pym | 3-Pyr |
| 1-2190 | Ph | 4-Pym | 4-Pym |
| 1-2191 | Ph | 4-Pym | 5-Pym |
| 1-2192 | Ph | 4-Pym | 3-Pyrd-$CH_2$ |
| 1-2193 | Ph | 4-Pym | 1-Me-3-Pyrd-$CH_2$ |
| 1-2194 | Ph | 4-Pym | 4-Pip-$CH_2$ |
| 1-2195 | Ph | 4-Pym | 1-Me-4-Pip-$CH_2$ |
| 1-2196 | Ph | 4-Pym | 2-Piz-$CH_2$ |

Table 1 (continued)

| Compound No. | $R^1$ | $R^2$ | $R^3$ |
|---|---|---|---|
| 1-2197 | Ph | 4-Pym | 4-Pyr-$CH_2$ |
| 1-2198 | Ph | 4-Pym | 3-Pyr-$CH_2$ |
| 1-2199 | Ph | 4-Pym | 4-Pym-$CH_2$ |
| 1-2200 | Ph | 4-Pym | 5-Pym-$CH_2$ |
| 1-2201 | Ph | 4-Pym | 2-Pym-$CH_2$ |
| 1-2202 | 4-F-Ph | 4-Pym | $H_2N$-$(CH_2)_3$ |
| 1-2203 | 4-F-Ph | 4-Pym | MeNH-$(CH_2)_3$ |
| 1-2204 | 4-F-Ph | 4-Pym | EtNH-$(CH_2)_3$ |
| 1-2205 | 4-F-Ph | 4-Pym | $Me_2N$-$(CH_2)_3$ |
| 1-2206 | 4-F-Ph | 4-Pym | 1-Azt-$(CH_2)_3$ |
| 1-2207 | 4-F-Ph | 4-Pym | 1-Pyrd-$(CH_2)_3$ |
| 1-2208 | 4-F-Ph | 4-Pym | 1-Pip-$(CH_2)_3$ |
| 1-2209 | 4-F-Ph | 4-Pym | 1-Mor-$(CH_2)_3$ |
| 1-2210 | 4-F-Ph | 4-Pym | 1-Tmor-$(CH_2)_3$ |
| 1-2211 | 4-F-Ph | 4-Pym | 1-Piz-$(CH_2)_3$ |
| 1-2212 | 4-F-Ph | 4-Pym | 4-Me-1-Piz-$(CH_2)_3$ |
| 1-2213 | 4-F-Ph | 4-Pym | 3-Azt |
| 1-2214 | 4-F-Ph | 4-Pym | 1-Me-3-Azt |
| 1-2215 | 4-F-Ph | 4-Pym | 3-Pyrd |
| 1-2216 | 4-F-Ph | 4-Pym | 1-Me-3-Pyrd |
| 1-2217 | 4-F-Ph | 4-Pym | 4-Pip |
| 1-2218 | 4-F-Ph | 4-Pym | 4-(3,4-deH-Pip) |
| 1-2219 | 4-F-Ph | 4-Pym | 1-Me-4-Pip |
| 1-2220 | 4-F-Ph | 4-Pym | 1-Me-4-(3,4-deH-Pip) |
| 1-2221 | 4-F-Ph | 4-Pym | 1-Piz |
| 1-2222 | 4-F-Ph | 4-Pym | 4-Me-1-Piz |
| 1-2223 | 4-F-Ph | 4-Pym | 4-Pyr |
| 1-2224 | 4-F-Ph | 4-Pym | 3-Pyr |
| 1-2225 | 4-F-Ph | 4-Pym | 4-Pym |
| 1-2226 | 4-F-Ph | 4-Pym | 5-Pym |
| 1-2227 | 4-F-Ph | 4-Pym | 3-Pyrd-$CH_2$ |
| 1-2228 | 4-F-Ph | 4-Pym | 1-Me-3-Pyrd-$CH_2$ |
| 1-2229 | 4-F-Ph | 4-Pym | 4-Pip-$CH_2$ |
| 1-2230 | 4-F-Ph | 4-Pym | 1-Me-4-Pip-$CH_2$ |
| 1-2231 | 4-F-Ph | 4-Pym | 2-Piz-$CH_2$ |
| 1-2232 | 4-F-Ph | 4-Pym | 4-Pyr-$CH_2$ |
| 1-2233 | 4-F-Ph | 4-Pym | 3-Pyr-$CH_2$ |
| 1-2234 | 4-F-Ph | 4-Pym | 4-Pym-$CH_2$ |

Table 1 (continued)

| Compound No. | $R^1$ | $R^2$ | $R^3$ |
|---|---|---|---|
| 1-2235 | 4-F-Ph | 4-Pym | 5-Pym-$CH_2$ |
| 1-2236 | 4-F-Ph | 4-Pym | 2-Pym-$CH_2$ |
| 1-2237 | 3-F-Ph | 4-Pym | $H_2N-(CH_2)_3$ |
| 1-2238 | 3-F-Ph | 4-Pym | MeNH-$(CH_2)_3$ |
| 1-2239 | 3-F-Ph | 4-Pym | EtNH-$(CH_2)_3$ |
| 1-2240 | 3-F-Ph | 4-Pym | $Me_2N-(CH_2)_3$ |
| 1-2241 | 3-F-Ph | 4-Pym | 1-Azt-$(CH_2)_3$ |
| 1-2242 | 3-F-Ph | 4-Pym | 1-Pyrd-$(CH_2)_3$ |
| 1-2243 | 3-F-Ph | 4-Pym | 1-Pip-$(CH_2)_3$ |
| 1-2244 | 3-F-Ph | 4-Pym | 1-Mor-$(CH_2)_3$ |
| 1-2245 | 3-F-Ph | 4-Pym | 1-Tmor-$(CH_2)_3$ |
| 1-2246 | 3-F-Ph | 4-Pym | 1-Piz-$(CH_2)_3$ |
| 1-2247 | 3-F-Ph | 4-Pym | 4-Me-1-Piz-$(CH_2)_3$ |
| 1-2248 | 3-F-Ph | 4-Pym | 3-Azt |
| 1-2249 | 3-F-Ph | 4-Pym | 1-Me-3-Azt |
| 1-2250 | 3-F-Ph | 4-Pym | 3-Pyrd |
| 1-2251 | 3-F-Ph | 4-Pym | 1-Me-3-Pyrd |
| 1-2252 | 3-F-Ph | 4-Pym | 4-Pip |
| 1-2253 | 3-F-Ph | 4-Pym | 4-(3,4-deH-Pip) |
| 1-2254 | 3-F-Ph | 4-Pym | 1-Me-4-Pip |
| 1-2255 | 3-F-Ph | 4-Pym | 1-Me-4-(3,4-deH-Pip) |
| 1-2256 | 3-F-Ph | 4-Pym | 1-Piz |
| 1-2257 | 3-F-Ph | 4-Pym | 4-Me-1-Piz |
| 1-2258 | 3-F-Ph | 4-Pym | 4-Pyr |
| 1-2259 | 3-F-Ph | 4-Pym | 3-Pyr |
| 1-2260 | 3-F-Ph | 4-Pym | 4-Pym |
| 1-2261 | 3-F-Ph | 4-Pym | 5-Pym |
| 1-2262 | 3-F-Ph | 4-Pym | 3-Pyrd-$CH_2$ |
| 1-2263 | 3-F-Ph | 4-Pym | 1-Me-3-Pyrd-$CH_2$ |
| 1-2264 | 3-F-Ph | 4-Pym | 4-Pip-$CH_2$ |
| 1-2265 | 3-F-Ph | 4-Pym | 1-Me-4-Pip-$CH_2$ |
| 1-2266 | 3-F-Ph | 4-Pym | 2-Piz-$CH_2$ |
| 1-2267 | 3-F-Ph | 4-Pym | 4-Pyr-$CH_2$ |
| 1-2268 | 3-F-Ph | 4-Pym | 3-Pyr-$CH_2$ |
| 1-2269 | 3-F-Ph | 4-Pym | 4-Pym-$CH_2$ |
| 1-2270 | 3-F-Ph | 4-Pym | 5-Pym-$CH_2$ |
| 1-2271 | 3-F-Ph | 4-Pym | 2-Pym-$CH_2$ |
| 1-2272 | 3,4-diF-Ph | 4-Pym | $H_2N-(CH_2)_3$ |

Table 1 (continued)

| Compound No. | $R^1$ | $R^2$ | $R^3$ |
|---|---|---|---|
| 1-2273 | 3,4-diF-Ph | 4-Pym | MeNH-$(CH_2)_3$ |
| 1-2274 | 3,4-diF-Ph | 4-Pym | EtNH-$(CH_2)_3$ |
| 1-2275 | 3,4-diF-Ph | 4-Pym | $Me_2N$-$(CH_2)_3$ |
| 1-2276 | 3,4-diF-Ph | 4-Pym | 1-Azt-$(CH_2)_3$ |
| 1-2277 | 3,4-diF-Ph | 4-Pym | 1-Pyrd-$(CH_2)_3$ |
| 1-2278 | 3,4-diF-Ph | 4-Pym | 1-Pip-$(CH_2)_3$ |
| 1-2279 | 3,4-diF-Ph | 4-Pym | 1-Mor-$(CH_2)_3$ |
| 1-2280 | 3,4-diF-Ph | 4-Pym | 1-Tmor-$(CH_2)_3$ |
| 1-2281 | 3,4-diF-Ph | 4-Pym | 1-Piz-$(CH_2)_3$ |
| 1-2282 | 3,4-diF-Ph | 4-Pym | 4-Me-1-Piz-$(CH_2)_3$ |
| 1-2283 | 3,4-diF-Ph | 4-Pym | 3-Azt |
| 1-2284 | 3,4-diF-Ph | 4-Pym | 1-Me-3-Azt |
| 1-2285 | 3,4-diF-Ph | 4-Pym | 3-Pyrd |
| 1-2286 | 3,4-diF-Ph | 4-Pym | 1-Me-3-Pyrd |
| 1-2287 | 3,4-diF-Ph | 4-Pym | 4-Pip |
| 1-2288 | 3,4-diF-Ph | 4-Pym | 4-(3,4-deH-Pip) |
| 1-2289 | 3,4-diF-Ph | 4-Pym | 1-Me-4-Pip |
| 1-2290 | 3,4-diF-Ph | 4-Pym | 1-Me-4-(3,4-deH-Pip) |
| 1-2291 | 3,4-diF-Ph | 4-Pym | 1-Piz |
| 1-2292 | 3,4-diF-Ph | 4-Pym | 4-Me-1-Piz |
| 1-2293 | 3,4-diF-Ph | 4-Pym | 4-Pyr |
| 1-2294 | 3/4-diF-Ph | 4-Pym | 3-Pyr |
| 1-2295 | 3,4-diF-Ph | 4-Pym | 4-Pym |
| 1-2296 | 3,4-diF-Ph | 4-Pym | 5-Pym |
| 1-2297 | 3,4-diF-Ph | 4-Pym | 3-Pyrd-$CH_2$ |
| 1-2298 | 3,4-diF-Ph | 4-Pym | 1-Me-3-Pyrd-$CH_2$ |
| 1-2299 | 3,4-diF-Ph | 4-Pym | 4-Pip-$CH_2$ |
| 1-2300 | 3,4-diF-Ph | 4-Pym | 1-Me-4-Pip-$CH_2$ |
| 1-2301 | 3,4-diF-Ph | 4-Pym | 2-Piz-$CH_2$ |
| 1-2302 | 3,4-diF-Ph | 4-Pym | 4-Pyr-$CH_2$ |
| 1-2303 | 3,4-diF-Ph | 4-Pym | 3-Pyr-$CH_2$ |
| 1-2304 | 3,4-diF-Ph | 4-Pym | 4-Pym-$CH_2$ |
| 1-2305 | 3,4-diF-Ph | 4-Pym | 5-Pym-$CH_2$ |
| 1-2306 | 3,4-diF-Ph | 4-Pym | 2-Pym-$CH_2$ |
| 1-2307 | 3-Cl-Ph | 4-Pym | $H_2N$-$(CH_2)_3$ |
| 1-2308 | 3-Cl-Ph | 4-Pym | MeNH-$(CH_2)_3$ |
| 1-2309 | 3-Cl-Ph | 4-Pym | EtNH-$(CH_2)_3$ |
| 1-2310 | 3-Cl-Ph | 4-Pym | $Me_2N$-$(CH_2)_3$ |

Table 1 (continued)

| Compound No. | $R^1$ | $R^2$ | $R^3$ |
|---|---|---|---|
| 1-2311 | 3-Cl-Ph | 4-Pym | 1-Azt-$(CH_2)_3$ |
| 1-2312 | 3-Cl-Ph | 4-Pym | 1-Pyrd-$(CH_2)_3$ |
| 1-2313 | 3-Cl-Ph | 4-Pym | 1-Pip-$(CH_2)_3$ |
| 1-2314 | 3-Cl-Ph | 4-Pym | 1-Mor-$(CH_2)_3$ |
| 1-2315 | 3-Cl-Ph | 4-Pym | 1-Tmor-$(CH_2)_3$ |
| 1-2316 | 3-Cl-Ph | 4-Pym | 1-Piz-$(CH_2)_3$ |
| 1-2317 | 3-Cl-Ph | 4-Pym | 4-Me-1-Piz-$(CH_2)_3$ |
| 1-2318 | 3-Cl-Ph | 4-Pym | 3-Azt |
| 1-2319 | 3-Cl-Ph | 4-Pym | 1-Me-3-Azt |
| 1-2320 | 3-Cl-Ph | 4-Pym | 3-Pyrd |
| 1-2321 | 3-Cl-Ph | 4-Pym | 1-Me-3-Pyrd |
| 1-2322 | 3-Cl-Ph | 4-Pym | 4-Pip |
| 1-2323 | 3-Cl-Ph | 4-Pym | 4-(3,4-deH-Pip) |
| 1-2324 | 3-Cl-Ph | 4-Pym | 1-Me-4-Pip |
| 1-2325 | 3-Cl-Ph | 4-Pym | 1-Me-4-(3,4-deH-Pip) |
| 1-2326 | 3-Cl-Ph | 4-Pym | 1-Piz |
| 1-2327 | 3-Cl-Ph | 4-Pym | 4-Me-1-Piz |
| 1-2328 | 3-Cl-Ph | 4-Pym | 4-Pyr |
| 1-2329 | 3-Cl-Ph | 4-Pym | 3-Pyr |
| 1-2330 | 3-Cl-Ph | 4-Pym | 4-Pym |
| 1-2331 | 3-Cl-Ph | 4-Pym | 5-Pym |
| 1-2332 | 3-Cl-Ph | 4-Pym | 3-Pyrd-$CH_2$ |
| 1-2333 | 3-Cl-Ph | 4-Pym | 1-Me-3-Pyrd-$CH_2$ |
| 1-2334 | 3-Cl-Ph | 4-Pym | 4-Pip-$CH_2$ |
| 1-2335 | 3-Cl-Ph | 4-Pym | 1-Me-4-Pip-$CH_2$ |
| 1-2336 | 3-Cl-Ph | 4-Pym | 2-Piz-$CH_2$ |
| 1-2337 | 3-Cl-Ph | 4-Pym | 4-Pyr-$CH_2$ |
| 1-2338 | 3-Cl-Ph | 4-Pym | 3-Pyr-$CH_2$ |
| 1-2339 | 3-Cl-Ph | 4-Pym | 4-Pym-$CH_2$ |
| 1-2340 | 3-Cl-Ph | 4-Pym | 5-Pym-$CH_2$ |
| 1-2341 | 3-Cl-Ph | 4-Pym | 2-Pym-$CH_2$ |
| 1-2342 | Ph | 2-MeO-4-Pym | $H_2N$-$(CH_2)_3$ |
| 1-2343 | Ph | 2-MeO-4-Pym | MeNH-$(CH_2)_3$ |
| 1-2344 | Ph | 2-MeO-4-Pym | EtNH-$(CH_2)_3$ |
| 1-2345 | Ph | 2-MeO-4-Pym | $Me_2N$-$(CH_2)_3$ |
| 1-2346 | Ph | 2-MeO-4-Pym | 1-Azt-$(CH_2)_3$ |
| 1-2347 | Ph | 2-MeO-4-Pym | 1-Pyrd-$(CH_2)_3$ |
| 1-2348 | Ph | 2-MeO-4-Pym | 1-Pip-$(CH_2)_3$ |

Table 1 (continued)

| Compound No. | $R^1$ | $R^2$ | $R^3$ |
|---|---|---|---|
| 1-2349 | Ph | 2-MeO-4-Pym | 1-Mor-$(CH_2)_3$ |
| 1-2350 | Ph | 2-MeO-4-Pym | 1-Tmor-$(CH_2)_3$ |
| 1-2351 | Ph | 2-MeO-4-Pym | 1-Piz-$(CH_2)_3$ |
| 1-2352 | Ph | 2-MeO-4-Pym | 4-Me-1-Piz-$(CH_2)_3$ |
| 1-2353 | Ph | 2-MeO-4-Pym | 3-Azt |
| 1-2354 | Ph | 2-MeO-4-Pym | 1-Me-3-Azt |
| 1-2355 | Ph | 2-MeO-4-Pym | 3-Pyrd |
| 1-2356 | Ph | 2-MeO-4-Pym | 1-Me-3-Pyrd |
| 1-2357 | Ph | 2-MeO-4-Pym | 4-Pip |
| 1-2358 | Ph | 2-MeO-4-Pym | 4-(3,4-deH-Pip) |
| 1-2359 | Ph | 2-MeO-4-Pym | 1-Me-4-Pip |
| 1-2360 | Ph | 2-MeO-4-Pym | 1-Me-4-(3,4-deH-Pip) |
| 1-2361 | Ph | 2-MeO-4-Pym | 1-Piz |
| 1-2362 | Ph | 2-MeO-4-Pym | 4-Me-1-Piz |
| 1-2363 | Ph | 2-MeO-4-Pym | 4-Pyr |
| 1-2364 | Ph | 2-MeO-4-Pym | 3-Pyr |
| 1-2365 | Ph | 2-MeO-4-Pym | 4-Pym |
| 1-2366 | Ph | 2-MeO-4-Pym | 5-Pym |
| 1-2367 | Ph | 2-MeO-4-Pym | 3-Pyrd-$CH_2$ |
| 1-2368 | Ph | 2-MeO-4-Pym | 1-Me-3-Pyrd-$CH_2$ |
| 1-2369 | Ph | 2-MeO-4-Pym | 4-Pip-$CH_2$ |
| 1-2370 | Ph | 2-MeO-4-Pym | 1-Me-4-Pip-$CH_2$ |
| 1-2371 | Ph | 2-MeO-4-Pym | 2-Piz-$CH_2$ |
| 1-2372 | Ph | 2-MeO-4-Pym | 4-Pyr-$CH_2$ |
| 1-2373 | Ph | 2-MeO-4-Pym | 3-Pyr-$CH_2$ |
| 1-2374 | Ph | 2-MeO-4-Pym | 4-Pym-$CH_2$ |
| 1-2375 | Ph | 2-MeO-4-Pym | 5-Pym-$CH_2$ |
| 1-2376 | Ph | 2-MeO-4-Pym | 2-Pym-$CH_2$ |
| 1-2377 | 4-F-Ph | 2-MeO-4-Pym | $H_2N$-$(CH_2)_3$ |
| 1-2378 | 4-F-Ph | 2-MeO-4-Pym | MeNH-$(CH_2)_3$ |
| 1-2379 | 4-F-Ph | 2-MeO-4-Pym | EtNH-$(CH_2)_3$ |
| 1-2380 | 4-F-Ph | 2-MeO-4-Pym | $Me_2N$-$(CH_2)_3$ |
| 1-2381 | 4-F-Ph | 2-MeO-4-Pym | 1-Azt-$(CH_2)_3$ |
| 1-2382 | 4-F-Ph | 2-MeO-4-Pym | 1-Pyrd-$(CH_2)_3$ |
| 1-2383 | 4-F-Ph | 2-MeO-4-Pym | 1-Pip-$(CH_2)_3$ |
| 1-2384 | 4-F-Ph | 2-MeO-4-Pym | 1-Mor-$(CH_2)_3$ |
| 1-2385 | 4-F-Ph | 2-MeO-4-Pym | 1-Tmor-$(CH_2)_3$ |
| 1-2386 | 4-F-Ph | 2-MeO-4-Pym | 1-Piz-$(CH_2)_3$ |

Table 1 (continued)

| Compound No. | $R^1$ | $R^2$ | $R^3$ |
|---|---|---|---|
| 1-2387 | 4-F-Ph | 2-MeO-4-Pym | 4-Me-1-Piz-$(CH_2)_3$ |
| 1-2388 | 4-F-Ph | 2-MeO-4-Pym | 3-Azt |
| 1-2389 | 4-F-Ph | 2-MeO-4-Pym | 1-Me-3-Azt |
| 1-2390 | 4-F-Ph | 2-MeO-4-Pym | 3-Pyrd |
| 1-2391 | 4-F-Ph | 2-MeO-4-Pym | 1-Me-3-Pyrd |
| 1-2392 | 4-F-Ph | 2-MeO-4-Pym | 4-Pip |
| 1-2393 | 4-F-Ph | 2-MeO-4-Pym | 4-(3,4-deH-Pip) |
| 1-2394 | 4-F-Ph | 2-MeO-4-Pym | 1-Me-4-Pip |
| 1-2395 | 4-F-Ph | 2-MeO-4-Pym | 1-Me-4-(3,4-deH Pip) |
| 1-2396 | 4-F-Ph | 2-MeO-4-Pym | 1-Piz |
| 1-2397 | 4-F-Ph | 2-MeO-4-Pym | 4-Me-1-Piz |
| 1-2398 | 4-F-Ph | 2-MeO-4-Pym | 4-Pyr |
| 1-2399 | 4-F-Ph | 2-MeO-4-Pym | 3-Pyr |
| 1-2400 | 4-F-Ph | 2-MeO-4-Pym | 4-Pym |
| 1-2401 | 4-F-Ph | 2-MeO-4-Pym | 5-Pym |
| 1-2402 | 4-F-Ph | 2-MeO-4-Pym | 3-Pyrd-$CH_2$ |
| 1-2403 | 4-F-Ph | 2-MeO-4-Pym | 1-Me-3-Pyrd-$CH_2$ |
| 1-2404 | 4-F-Ph | 2-MeO-4-Pym | 4-Pip-$CH_2$ |
| 1-2405 | 4-F-Ph | 2-MeO-4-Pym | 1-Me-4-Pip-$CH_2$ |
| 1-2406 | 4-F-Ph | 2-MeO-4-Pym | 2-Piz-$CH_2$ |
| 1-2407 | 4-F-Ph | 2-MeO-4-Pym | 4-Pyr-$CH_2$ |
| 1-2408 | 4-F-Ph | 2-MeO-4-Pym | 3-Pyr-$CH_2$ |
| 1-2409 | 4-F-Ph | 2-MeO-4-Pym | 4-Pym-$CH_2$ |
| 1-2410 | 4-F-Ph | 2-MeO-4-Pym | 5-Pym-$CH_2$ |
| 1-2411 | 4-F-Ph | 2-MeO-4-Pym | 2-Pym-$CH_2$ |
| 1-2412 | 3-F-Ph | 2-MeO-4-Pym | $H_2N$-$(CH_2)_3$ |
| 1-2413 | 3-F-Ph | 2-MeO-4-Pym | MeNH-$(CH_2)_3$ |
| 1-2414 | 3-F-Ph | 2-MeO-4-Pym | EtNH-$(CH_2)_3$ |
| 1-2415 | 3-F-Ph | 2-MeO-4-Pym | $Me_2N$-$(CH_2)_3$ |
| 1-2416 | 3-F-Ph | 2-MeO-4-Pym | 1-Azt-$(CH_2)_3$ |
| 1-2417 | 3-F-Ph | 2-MeO-4-Pym | 1-Pyrd-$(CH_2)_3$ |
| 1-2418 | 3-F-Ph | 2-MeO-4-Pym | 1-Pip-$(CH_2)_3$ |
| 1-2419 | 3-F-Ph | 2-MeO-4-Pym | 1-Mor-$(CH_2)_3$ |
| 1-2420 | 3-F-Ph | 2-MeO-4-Pym | 1-Tmor-$(CH_2)_3$ |
| 1-2421 | 3-F-Ph | 2-MeO-4-Pym | 1-Piz-$(CH_2)_3$ |
| 1-2422 | 3-F-Ph | 2-MeO-4-Pym | 4-Me-1-Piz-$(CH_2)_3$ |
| 1-2423 | 3-F-Ph | 2-MeO-4-Pym | 3-Azt |
| 1-2424 | 3-F-Ph | 2-MeO-4-Pym | 1-Me-3-Azt |

Table 1 (continued)

| Compound No. | $R^1$ | $R^2$ | $R^3$ |
|---|---|---|---|
| 1-2425 | 3-F-Ph | 2-MeO-4-Pym | 3-Pyrd |
| 1-2426 | 3-F-Ph | 2-MeO-4-Pym | 1-Me-3-Pyrd |
| 1-2427 | 3-F-Ph | 2-MeO-4-Pym | 4-Pip |
| 1-2428 | 3-F-Ph | 2-MeO-4-Pym | 4-(3,4-deH-Pip) |
| 1-2429 | 3-F-Ph | 2-MeO-4-Pym | 1-Me-4-Pip |
| 1-2430 | 3-F-Ph | 2-MeO-4-Pym | 1-Me-4-(3,4-deH-Pip) |
| 1-2431 | 3-F-Ph | 2-MeO-4-Pym | 1-Piz |
| 1-2432 | 3-F-Ph | 2-MeO-4-Pym | 4-Me-1-Piz |
| 1-2433 | 3-F-Ph | 2-MeO-4-Pym | 4-Pyr |
| 1-2434 | 3-F-Ph | 2-MeO-4-Pym | 3-Pyr |
| 1-2435 | 3-F-Ph | 2-MeO-4-Pym | 4-Pym |
| 1-2436 | 3-F-Ph | 2-MeO-4-Pym | 5-Pym |
| 1-2437 | 3-F-Ph | 2-MeO-4-Pym | 3-Pyrd-$CH_2$ |
| 1-2438 | 3-F-Ph | 2-MeO-4-Pym | 1-Me-3-Pyrd-$CH_2$ |
| 1-2439 | 3-F-Ph | 2-MeO-4-Pym | 4-Pip-$CH_2$ |
| 1-2440 | 3-F-Ph | 2-MeO-4-Pym | 1-Me-4-Pip-$CH_2$ |
| 1-2441 | 3-F-Ph | 2-MeO-4-Pym | 2-Piz-$CH_2$ |
| 1-2442 | 3-F-Ph | 2-MeO-4-Pym | 4-Pyr-$CH_2$ |
| 1-2443 | 3-F-Ph | 2-MeO-4-Pym | 3-Pyr-$CH_2$ |
| 1-2444 | 3-F-Ph | 2-MeO-4-Pym | 4-Pym-$CH_2$ |
| 1-2445 | 3-F-Ph | 2-MeO-4-Pym | 5-Pym-$CH_2$ |
| 1-2446 | 3-F-Ph | 2-MeO-4-Pym | 2-Pym-$CH_2$ |
| 1-2447 | 3,4-diF-Ph | 2-MeO-4-Pym | $H_2N-(CH_2)_3$ |
| 1-2448 | 3,4-diF-Ph | 2-MeO-4-Pym | $MeNH-(CH_2)_3$ |
| 1-2449 | 3,4-diF-Ph | 2-MeO-4-Pym | $EtNH-(CH_2)_3$ |
| 1-2450 | 3,4-diF-Ph | 2-MeO-4-Pym | $Me_2N-(CH_2)_3$ |
| 1-2451 | 3,4-diF-Ph | 2-MeO-4-Pym | 1-Azt-$(CH_2)_3$ |
| 1-2452 | 3,4-diF-Ph | 2-MeO-4-Pym | 1-Pyrd-$(CH_2)_3$ |
| 1-2453 | 3,4-diF-Ph | 2-MeO-4-Pym | 1-Pip-$(CH_2)_3$ |
| 1-2454 | 3,4-diF-Ph | 2-MeO-4-Pym | 1-Mor-$(CH_2)_3$ |
| 1-2455 | 3,4-diF-Ph | 2-MeO-4-Pym | 1-Tmor-$(CH_2)_3$ |
| 1-2456 | 3,4-diF-Ph | 2-MeO-4-Pym | 1-Piz-$(CH_2)_3$ |
| 1-2457 | 3,4-diF-Ph | 2-MeO-4-Pym | 4-Me-1-Piz-$(CH_2)_3$ |
| 1-2458 | 3,4-diF-Ph | 2-MeO-4-Pym | 3-Azt |
| 1-2459 | 3,4-diF-Ph | 2-MeO-4-Pym | 1-Me-3-Azt |
| 1-2460 | 3,4-diF-Ph | 2-MeO-4-Pym | 3-Pyrd |
| 1-2461 | 3,4-diF-Ph | 2-MeO-4-Pym | 1-Me-3-Pyrd |
| 1-2462 | 3,4-diF-Ph | 2-MeO-4-Pym | 4-Pip |

Table 1 (continued)

| Compound No. | $R^1$ | $R^2$ | $R^3$ |
|---|---|---|---|
| 1-2463 | 3,4-diF-Ph | 2-MeO-4-Pym | 4-(3,4-deH-Pip) |
| 1-2464 | 3,4-diF-Ph | 2-MeO-4-Pym | 1-Me-4-Pip |
| 1-2465 | 3,4-diF-Ph | 2-MeO-4-Pym | 1-Me-4-(3,4-deH-Pip) |
| 1-2466 | 3,4-diF-Ph | 2-MeO-4-Pym | 1-Piz |
| 1-2467 | 3,4-diF-Ph | 2-MeO-4-Pym | 4-Me-1-Piz |
| 1-2468 | 3,4-diF-Ph | 2-MeO-4-Pym | 4-Pyr |
| 1-2469 | 3,4-diF-Ph | 2-MeO-4-Pym | 3-Pyr |
| 1-2470 | 3,4-diF-Ph | 2-MeO-4-Pym | 4-Pym |
| 1-2471 | 3,4-diF-Ph | 2-MeO-4-Pym | 5-Pym |
| 1-2472 | 3,4-diF-Ph | 2-MeO-4-Pym | 3-Pyrd-$CH_2$ |
| 1-2473 | 3,4-diF-Ph | 2-MeO-4-Pym | 1-Me-3-Pyrd-$CH_2$ |
| 1-2474 | 3,4-diF-Ph | 2-MeO-4-Pym | 4-Pip-$CH_2$ |
| 1-2475 | 3,4-diF-Ph | 2-MeO-4-Pym | 1-Me-4-Pip-$CH_2$ |
| 1-2476 | 3,4-diF-Ph | 2-MeO-4-Pym | 2-Piz-$CH_2$ |
| 1-2477 | 3,4-diF-Ph | 2-MeO-4-Pym | 4-Pyr-$CH_2$ |
| 1-2478 | 3,4-diF-Ph | 2-MeO-4-Pym | 3-Pyr-$CH_2$ |
| 1-2479 | 3,4-diF-Ph | 2-MeO-4-Pym | 4-Pym-$CH_2$ |
| 1-2480 | 3,4-diF-Ph | 2-MeO-4-Pym | 5-Pym-$CH_2$ |
| 1-2481 | 3,4-diF-Ph | 2-MeO-4-Pym | 2-Pym-$CH_2$ |
| 1-2482 | 3-Cl-Ph | 2-MeO-4-Pym | $H_2N$-$(CH_2)_3$ |
| 1-2483 | 3-Cl-Ph | 2-MeO-4-Pym | MeNH-$(CH_2)_3$ |
| 1-2484 | 3-Cl-Ph | 2-MeO-4-Pym | EtNH-$(CH_2)_3$ |
| 1-2485 | 3-Cl-Ph | 2-MeO-4-Pym | $Me_2N$-$(CH_2)_3$ |
| 1-2486 | 3-Cl-Ph | 2-MeO-4-Pym | 1-Azt-$(CH_2)_3$ |
| 1-2487 | 3-Cl-Ph | 2-MeO-4-Pym | 1-Pyrd-$(CH_2)_3$ |
| 1-2488 | 3-Cl-Ph | 2-MeO-4-Pym | 1-Pip-$(CH_2)_3$ |
| 1-2489 | 3-Cl-Ph | 2-MeO-4-Pym | 1-Mor-$(CH_2)_3$ |
| 1-2490 | 3-Cl-Ph | 2-MeO-4-Pym | 1-Tmor-$(CH_2)_3$ |
| 1-2491 | 3-Cl-Ph | 2-MeO-4-Pym | 1-Piz-$(CH_2)_3$ |
| 1-2492 | 3-Cl-Ph | 2-MeO-4-Pym | 4-Me-1-Piz-$(CH_2)_3$ |
| 1-2493 | 3-Cl-Ph | 2-MeO-4-Pym | 3-Azt |
| 1-2494 | 3-Cl-Ph | 2-MeO-4-Pym | 1-Me-3-Azt |
| 1-2495 | 3-Cl-Ph | 2-MeO-4-Pym | 3-Pyrd |
| 1-2496 | 3-Cl-Ph | 2-MeO-4-Pym | 1-Me-3-Pyrd |
| 1-2497 | 3-Cl-Ph | 2-MeO-4-Pym | 4-Pip |
| 1-2498 | 3-Cl-Ph | 2-MeO-4-Pym | 4-(3,4-deH-Pip) |
| 1-2499 | 3-Cl-Ph | 2-MeO-4-Pym | 1-Me-4-Pip |
| 1-2500 | 3-Cl-Ph | 2-MeO-4-Pym | 1-Me-4-(3,4-deH-Pip) |

Table 1 (continued)

| Compound No. | $R^1$ | $R^2$ | $R^3$ |
|---|---|---|---|
| 1-2501 | 3-Cl-Ph | 2-MeO-4-Pym | 1-Piz |
| 1-2502 | 3-Cl-Ph | 2-MeO-4-Pym | 4-Me-1-Piz |
| 1-2503 | 3-Cl-Ph | 2-MeO-4-Pym | 4-Pyr |
| 1-2504 | 3-Cl-Ph | 2-MeO-4-Pym | 3-Pyr |
| 1-2505 | 3-Cl-Ph | 2-MeO-4-Pym | 4-Pym |
| 1-2506 | 3-Cl-Ph | 2-MeO-4-Pym | 5-Pym |
| 1-2507 | 3-Cl-Ph | 2-MeO-4-Pym | 3-Pyrd-$CH_2$ |
| 1-2508 | 3-Cl-Ph | 2-MeO-4-Pym | 1-Me-3-Pyrd-$CH_2$ |
| 1-2509 | 3-Cl-Ph | 2-MeO-4-Pym | 4-Pip-$CH_2$ |
| 1-2510 | 3-Cl-Ph | 2-MeO-4-Pym | 1-Me-4-Pip-$CH_2$ |
| 1-2511 | 3-Cl-Ph | 2-MeO-4-Pym | 2-Piz-$CH_2$ |
| 1-2512 | 3-Cl-Ph | 2-MeO-4-Pym | 4-Pyr-$CH_2$ |
| 1-2513 | 3-Cl-Ph | 2-MeO-4-Pym | 3-Pyr-$CH_2$ |
| 1-2514 | 3-Cl-Ph | 2-MeO-4-Pym | 4-Pym-$CH_2$ |
| 1-2515 | 3-Cl-Ph | 2-MeO-4-Pym | 5-Pym-$CH_2$ |
| 1-2516 | 3-Cl-Ph | 2-MeO-4-Pym | 2-Pym-$CH_2$ |
| 1-2517 | 4-F-Ph | 4-Pyr | $H_2N$-$CH_2CH$=CH |
| 1-2518 | 4-F-Ph | 4-Pyr | MeNH-$CH_2CH$=CH |
| 1-2519 | 4-F-Ph | 4-Pyr | $Me_2N$-$CH_2CH$=CH |
| 1-2520 | 4-F-Ph | 4-Pyr | 3-Pip-$CH_2$ |
| 1-2521 | 4-F-Ph | 4-Pyr | 1-Me-3-Pip-$CH_2$ |
| 1-2522 | 4-F-Ph | 4-Pyr | 2-Me-4-Pip |
| 1-2523 | 4-F-Ph | 4-Pyr | 2,2,6,6-tetraMe-4-Pip |
| 1-2524 | 4-F-Ph | 4-Pyr | 1-Ac-4-Pip |
| 1-2525 | 4-F-Ph | 4-Pyr | 1-Ac-4-(3,4-deH-Pip) |
| 1-2526 | 4-F-Ph | 4-Pyr | 4-OH-4-Pip |
| 1-2527 | 4-F-Ph | 4-Pyr | 4-OH-1-Me-4-Pip |
| 1-2528 | 4-F-Ph | 4-Pyr | AcNH-$(CH_2)_3$ |
| 1-2529 | 4-F-Ph | 4-Pyr | 4-$NH_2$-cHx |
| 1-2530 | 4-F-Ph | 4-Pyr | 4-Pyr-CH(OH) |
| 1-2531 | 4-F-Ph | 4-Pyr | 3-Pyr-CH(OH) |
| 1-2532 | 4-F-Ph | 4-Pyr | 2-Pyr-CH(OH) |
| 1-2533 | 4-F-Ph | 4-Pyr | $CF_3$CONH-$(CH_2)_3$ |
| 1-2534 | 4-F-Ph | 4-Pyr | BzNH-$(CH_2)_3$ |
| 1-2535 | 4-F-Ph | 4-Pyr | 2,4,6-triF-BzNH-$CH_2$ |
| 1-2536 | 4-F-Ph | 4-Pyr | MeSO2NH-$(CH_2)_3$ |
| 1-2537 | 4-F-Ph | 4-Pyr | 1-NO2 $(CH_2)_2$-4-Pip |
| 1-2538 | 4-F-Ph | 4-Pyr | 2,3,5,6-tetraF-4-Pyr |

Table 1 (continued)

| Compound No. | $R^1$ | $R^2$ | $R^3$ |
|---|---|---|---|
| 1-2539 | 4-F-Ph | 4-Pyr | 3-Qun |
| 1-2540 | 4-F-Ph | 4-Pyr | 3-(2,3-deH-Qun) |
| 1-2541 | 4-F-Ph | 4-Pyr | 3-ABO |
| 1-2542 | 4-F-Ph | 4-Pyr | 8-Me-3-ABO |
| 1-2543 | 4-F-Ph | 4-Pyr | 3-(2,3-deH-ABO) |
| 1-2544 | 4-F-Ph | 4-Pyr | 8-Me-3-(2,3-deH-ABO) |
| 1-2545 | 4-F-Ph | 4-Pyr | 3-ABN |
| 1-2546 | 4-F-Ph | 4-Pyr | 9-Me-3-ABN |
| 1-2547 | 4-F-Ph | 4-Pyr | 3-(2,3-deH-ABN) |
| 1-2548 | 4-F-Ph | 4-Pyr | 9-Me-3-(2,3-deH-ABN) |
| 1-2549 | 4-F-Ph | $2-NH_2-4-Pym$ | $H_2N-CH_2CH=CH$ |
| 1-2550 | 4-F-Ph | $2-NH_2-4-Pym$ | $MeNH-CH_2CH=CH$ |
| 1-2551 | 4-F-Ph | $2-NH_2-4-Pym$ | $Me_2N-CH_2CH=CH$ |
| 1-2552 | 4-F-Ph | $2-NH_2-4-Pym$ | $3-Pip-CH_2$ |
| 1-2553 | 4-F-Ph | $2-NH_2-4-Pym$ | $1-Me-3-Pip-CH_2$ |
| 1-2554 | 4-F-Ph | $2-NH_2-4-Pym$ | 2-Me-4-Pip |
| 1-2555 | 4-F-Ph | $2-NH_2-4-Pym$ | 2,2,6,6-tetraMe-4-Pip |
| 1-2556 | 4-F-Ph | $2-NH_2-4-Pym$ | 1-Ac-4-Pip |
| 1-2557 | 4-F-Ph | $2-NH_2-4-Pym$ | 1-Ac-4-(3,4-deH-Pip) |
| 1-2558 | 4-F-Ph | $2-NH_2-4-Pym$ | 4-OH-4-Pip |
| 1-2559 | 4-F-Ph | $2-NH_2-4-Pym$ | 4-OH-1-Me-4-Pip |
| 1-2560 | 4-F-Ph | $2-NH_2-4-Pym$ | $AcNH-(CH_2)_3$ |
| 1-2561 | 4-F-Ph | $2-NH_2-4-Pym$ | $4-NH_2-cHx$ |
| 1-2562 | 4-F-Ph | $2-NH_2-4-Pym$ | 3-Qun |
| 1-2563 | 4-F-Ph | $2-NH_2-4-Pym$ | 3-(2,3-deH-Qun) |
| 1-2564 | 4-F-Ph | $2-NH_2-4-Pym$ | 3-ABO |
| 1-2565 | 4-F-Ph | $2-NH_2-4-Pym$ | 8-Me-3-ABO |
| 1-2566 | 4-F-Ph | $2-NH_2-4-Pym$ | 3-(2,3-deH-ABO) |
| 1-2567 | 4-F-Ph | $2-NH_2-4-Pym$ | 8-Me-3-(2,3-deH-ABO) |
| 1-2568 | 4-F-Ph | $2-NH_2-4-Pym$ | 3-ABN |
| 1-2569 | 4-F-Ph | $2-NH_2-4-Pym$ | 9-Me-3-ABN |
| 1-2570 | 4-F-Ph | $2-NH_2-4-Pym$ | 3-(2,3-deH-ABN) |
| 1-2571 | 4-F-Ph | $2-NH_2-4-Pym$ | 9-Me-3-(2,3-deH-ABN) |
| 1-2572 | 4-F-Ph | 2-MeNH-4-Pym | $H_2N-CH_2CH=CH$ |
| 1-2573 | 4-F-Ph | 2-MeNH-4-Pym | $MeNH-CH_2CH=CH$ |
| 1-2574 | 4-F-Ph | 2-MeNH-4-Pym | $Me_2N-CH_2CH=CH$ |
| 1-2575 | 4-F-Ph | 2-MeNH-4-Pym | $3-Pip-CH_2$ |
| 1-2576 | 4-F-Ph | 2-MeNH-4-Pym | $1-Me-3-Pip-CH_2$ |

Table 1 (continued)

| Compound No. | $R^1$ | $R^2$ | $R^3$ |
|---|---|---|---|
| 1-2577 | 4-F-Ph | 2-MeNH-4-Pym | 2-Me-4-Pip |
| 1-2578 | 4-F-Ph | 2-MeNH-4-Pym | 2,2,6,6-tetraMe-4-Pip |
| 1-2579 | 4-F-Ph | 2-MeNH-4-Pym | 1-Ac-4-Pip |
| 1-2580 | 4-F-Ph | 2-MeNH-4-Pym | 1-Ac-4-(3,4-deH-Pip) |
| 1-2581 | 4-F-Ph | 2-MeNH-4-Pym | 4-OH-4-Pip |
| 1-2582 | 4-F-Ph | 2-MeNH-4-Pym | 4-OH-1-Me-4-Pip |
| 1-2583 | 4-F-Ph | 2-MeNH-4-Pym | AcNH-$(CH_2)_3$ |
| 1-2584 | 4-F-Ph | 2-MeNH-4-Pym | 4-$NH_2$-cHx |
| 1-2585 | 4-F-Ph | 2-MeNH-4-Pym | 3-Qun |
| 1-2586 | 4-F-Ph | 2-MeNH-4-Pym | 3-(2,3-deH-Qun) |
| 1-2587 | 4-F-Ph | 2-MeNH-4-Pym | 3-ABO |
| 1-2588 | 4-F-Ph | 2-MeNH-4-Pym | 8-Me-3-ABO |
| 1-2589 | 4-F-Ph | 2-MeNH-4-Pym | 3-(2,3-deH-ABO) |
| 1-2590 | 4-F-Ph | 2-MeNH-4-Pym | 8-Me-3-(2,3-deH-ABO) |
| 1-2591 | 4-F-Ph | 2-MeNH-4-Pym | 3-ABN |
| 1-2592 | 4-F-Ph | 2-MeNH-4-Pym | 9-Me-3-ABN |
| 1-2593 | 4-F-Ph | 2-MeNH-4-Pym | 3-(2,3-deH-ABN) |
| 1-2594 | 4-F-Ph | 2-MeNH-4-Pym | 9-Me-3-(2,3-deH-ABN) |
| 1-2595 | 4-F-Ph | 2-BnNH-4-Pyr | 4-Pip |
| 1-2596 | 4-F-Ph | 2-BnNH-4-Pyr | 4-(3,4-deH-Pip) |
| 1-2597 | 4-F-Ph | 2-BnNH-4-Pym | 4-Pip |
| 1-2598 | 4-F-Ph | 2-BnNH-4-Pym | 4-(3,4-deH-Pip) |
| 1-2599 | 4-F-Ph | 2-(α-Me-BnNH)-4-Pyr | 4-Pip |
| 1-2600 | 4-F-Ph | 2-(α-Me-BnNH)-4-Pyr | 4-(3,4-deH-Pip) |
| 1-2601 | 4-F-Ph | 2-(α-Me-BnNH)-4-Pym | 4-Pip |
| 1-2602 | 4-F-Ph | 2-(α-Me-BnNH)-4-Pym | 4-(3,4-deH-Pip) |
| 1-2603 | 3-Cl-Ph | 2-BnNH-4-Pyr | 4-Pip |
| 1-2604 | 3-Cl-Ph | 2-BnNH-4-Pyr | 4-(3,4-deH-Pip) |
| 1-2605 | 3-Cl-Ph | 2-BnNH-4-Pym | 4-Pip |
| 1-2606 | 3-Cl-Ph | 2-BnNH-4-Pym | 4-(3,4-deH-Pip) |
| 1-2607 | 3-Cl-Ph | 2-(α-Me-BnNH)-4-Pyr | 4-Pip |
| 1-2608 | 3-Cl-Ph | 2-(α-Me-BnNH)-4-Pyr | 4-(3,4-deH-Pip) |
| 1-2609 | 3-Cl-Ph | 2-(α-Me-BnNH)-4-Pym | 4-Pip |
| 1-2610 | 3-Cl-Ph | 2-(α-Me-BnNH)-4-Pym | 4-(3,4-deH-Pip) |
| 1-2611 | 3-$CF_3$-Ph | 2-BnNH-4-Pyr | 4-Pip |

Table 1 (continued)

| Compound No. | $R^1$ | $R^2$ | $R^3$ |
|---|---|---|---|
| 1-2612 | $3-CF_3-Ph$ | 2-BnNH-4-Pyr | 4-(3,4-deH-Pip) |
| 1-2613 | $3-CF_3-Ph$ | 2-BnNH-4-Pym | 4-Pip |
| 1-2614 | $3-CF_3-Ph$ | 2-BnNH-4-Pym | 4-(3,4-deH-Pip) |
| 1-2615 | $3-CF_3-Ph$ | 2-(α-Me-BnNH)-4-Pyr | 4-Pip |
| 1-2616 | $3-CF_3-Ph$ | 2-(α-Me-BnNH)-4-Pyr | 4-(3,4-deH-Pip) |
| 1-2617 | $3-CF_3-Ph$ | 2-(α-Me-BnNH)-4-Pym | 4-Pip |
| 1-2618 | $3-CF_3-Ph$ | 2-(α-Me-BnNH)-4-Pym | 4-(3,4-deH-Pip) |
| 1-2619 | 4-F-Ph | 4-Pyr | $2-NH_2-4-Pym$ |
| 1-2620 | 4-F-Ph | 4-Pyr | 2-MeNH-4-pym |
| 1-2621 | 4-F-Ph | 4-Pyr | $2-NH_2-4-Pyr$ |
| 1-2622 | 4-F-Ph | 4-Pyr | 2-MeNH-4-pyr |
| 1-2623 | 4-F-Ph | 4-Pyr | $H_2N-CH_2C(Me)_2CH_2$ |
| 1-2624 | 4-F-Ph | 4-Pyr | $MeNH-CH_2C(Me)_2CH_2$ |
| 1-2625 | 4-F-Ph | 4-Pyr | $EtNH-CH_2C(Me)_2CH_2$ |
| 1-2626 | 4-F-Ph | 4-Pyr | $Me_2N-CH_2C(Me)_2CH_2$ |
| 1-2627 | 4-F-Ph | 4-Pyr | 3-(3,4-deH-Pip) |
| 1-2628 | 4-F-Ph | 4-Pyr | 1-Me-3-(3,4-deH-Pip) |
| 1-2629 | 4-F-Ph | 4-Pyr | 1-Et-4-(3,4-deH-Pip) |
| 1-2630 | 4-F-Ph | 4-Pyr | 1-Pr-4-(3,4-deH-Pip) |
| 1-2631 | 4-F-Ph | 4-Pyr | 1-Pr-4-Pip |
| 1-2632 | 4-F-Ph | 4-Pyr | 1-iPr-4-(3/4-deH-Pip) |
| 1-2633 | 4-F-Ph | 4-Pyr | 1-iPr-4-Pip |
| 1-2634 | 4-F-Ph | 4-Pyr | 1-Bu-4-(3,4-deH-Pip) |
| 1-2635 | 4-F-Ph | 4-Pyr | 1-tBu-4-(3,4-deH-Pip) |
| 1-2636 | 4-F-Ph | 4-Pyr | 1-Pn-4-(3,4-deH-Pip) |
| 1-2637 | 4-F-Ph | 4-Pyr | 1-Hx-4-(3,4-deH-Pip) |
| 1-2638 | 4-F-Ph | 4-Pyr | 1-Hp-4-(3,4-deH-Pip) |
| 1-2639 | 4-F-Ph | 4-Pyr | 1-Oc-4-(3,4-deH-Pip) |
| 1-2640 | 4-F-Ph | 4-Pyr | 1-Nn-4-(3,4-deH-Pip) |
| 1-2641 | 4-F-Ph | 4-Pyr | 1-cPr-4-(3,4-deH-Pip) |
| 1-2642 | 4-F-Ph | 4-Pyr | 1-cPn-4-(3,4-deH-Pip) |
| 1-2643 | 4-F-Ph | 4-Pyr | 1-cHx-4-(3,4-deH-Pip) |
| 1-2644 | 4-F-Ph | 4-Pyr | 1-Bn-4-(3,4-deH-Pip) |
| 1-2645 | 4-F-Ph | 4-Pyr | 1-Phet-4-(3,4-deH-Pip) |
| 1-2646 | 4-F-Ph | 4-Pyr | 1-(3-Ph-Pr)-4-(3,4-deH-Pip) |
| 1-2647 | 4-F-Ph | 4-Pyr | 1-(4-Ph-Bu)-4-(3,4-deH-Pip) |

Table 1 (continued)

| Compound No. | R1 | R2 | R3 |
|---|---|---|---|
| 1-2648 | 4-F-Ph | 4-Pyr | 1-Allyl-4-(3,4-deH-Pip) |
| 1-2649 | 4-F-Ph | 4-Pyr | 1-Propargyl-4-(3,4-deH-Pip) |
| 1-2650 | 4-F-Ph | 4-Pyr | 2,2,6,6-tetraMe-4-(3,4-deH-Pip) |
| 1-2651 | 4-F-Ph | 4-Pyr | 1,2,2,6,6-pentaMe-4-(3,4-deH-Pip) |
| 1-2652 | 4-F-Ph | 4-Pyr | 1,2,2,6,6-pentaMe-4-Pip) |
| 1-2653 | 4-F-Ph | 4-Pyr | 7-(1,2,3,5,6,8a-hexaH-Ind) |
| 1-2654 | 4-F-Ph | 4-Pyr | 7-(1,2,3,5,8,8a-hexaH-Ind) |
| 1-2655 | 4-F-Ph | 4-Pyr | 7-octaH-Ind |
| 1-2656 | 4-F-Ph | 4-Pyr | 8-(1,3,4,6,7,9a-hexaH-2H-Qui) |
| 1-2657 | 4-F-Ph | 4-Pyr | 8-(1,3,4,6,9,9a-hexaH-2H-Qui) |
| 1-2658 | 4-F-Ph | 4-Pyr | 8-octaH-Qui |
| 1-2659 | 4-F-Ph | 4-Pyr | 2,2-diMe-4-(3,4-deH-PiP) |
| 1-2660 | 4-F-Ph | 4-Pyr | 1,2,2-triMe-4-(3,4-deH-PiP) |
| 1-2661 | 4-F-Ph | 4-Pyr | 2,2-diMe-4-(4,5-deH-PiP) |
| 1-2662 | 4-F-Ph | 4-Pyr | 1,2,2-triMe-4-(4,5-deH-PiP) |
| 1-2663 | 4-F-Ph | 4-Pyr | 2,6-diMe-4-(3,4-deH-PiP) |
| 1-2664 | 4-F-Ph | 4-Pyr | 1,2,6-triMe-4-(3,4-deH-PiP) |
| 1-2665 | 4-F-Ph | 4-Pyr | 2-Me-4-(3,4-deH-Pip) |
| 1-2666 | 4-F-Ph | 4-Pyr | 1,2-diMe-4-(3,4-deH-Pip) |
| 1-2667 | 4-F-Ph | 4-Pyr | 1-Et-2-Me-4-(3,4-deH-Pip) |
| 1-2668 | 4-F-Ph | 4-Pyr | 1-Pr-2-Me-4- (3,4-deH-Pip) |
| 1-2669 | 4-F-Ph | 4-Pyr | 1-Bu-2-Me-4-(3,4-deH-Pip) |
| 1-2670 | 4-F-Ph | 4-Pyr | 1-Phet-2-Me-4-(3,4-deH-Pip) |
| 1-2671 | 4-F-Ph | 4-Pyr | 2-Et-4-(3,4-deH-Pip) |
| 1-2672 | 4-F-Ph | 4-Pyr | 1-Me-2-Et-4-(3,4-deH-Pip) |
| 1-2673 | 4-F-Ph | 4-Pyr | 1,2-diEt-4-(3,4-deH-Pip) |
| 1-2674 | 4-F-Ph | 4-Pyr | 1-Pr-2-Et-4-(3,4-deH-Pip) |
| 1-2675 | 4-F-Ph | 4-Pyr | 1-Bu-2-Et-4-(3,4-deH-Pip) |
| 1-2676 | 4-F-Ph | 4-Pyr | 1-Phet-2-Et-4-(3,4-deH-Pip) |
| 1-2677 | 4-F-Ph | 4-Pyr | 2-Pr-4-(3,4-deH-Pip) |
| 1-2678 | 4-F-Ph | 4-Pyr | 1-Me-2-Pr-4-(3,4-deH-Pip) |
| 1-2679 | 4-F-Ph | 4-Pyr | 1-Et-2-Pr-4-(3,4-deH-Pip) |
| 1-2680 | 4-F-Ph | 4-Pyr | 1,2-diPr-4-(3,4-deH-Pip) |
| 1-2681 | 4-F-Ph | 4-Pyr | 1-Bu-2-Pr-4-(3,4(-deH-Pip) |
| 1-2682 | 4-F-Ph | 4-Pyr | 1-Phet-2-Pr-4-(3,4-deH-Pip) |
| 1-2683 | 4-F-Ph | 4-Pyr | 2-Bu-4-(3,4-deH-Pip) |
| 1-2684 | 4-F-Ph | 4-Pyr | 1-Me-2-Bu-4-(3,4-deH-Pip) |
| 1-2685 | 4-F-Ph | 4-Pyr | 1-Et-2-Bu-4-(3,4-deH-Pip) |

Table 1 (continued)

| Compound No. | $R^1$ | $R^2$ | $R^3$ |
|---|---|---|---|
| 1-2686 | 4-F-Ph | 4-Pyr | 1-Pr-2-Bu-4-(3,4-deH-Pip) |
| 1-2687 | 4-F-Ph | 4-Pyr | 1,2-diBu-4-(3,4-deH-Pip) |
| 1-2688 | 4-F-Ph | 4-Pyr | 1-Phet-2-Bu-4(3,4-deH-Pip) |
| 1-2689 | 4-F-Ph | 4-Pyr | 2-Allyl-4-(3,4-deH-Pip) |
| 1-2690 | 4-F-Ph | 4-Pyr | 1-Me-2-Allyl-4-(3,4-deH-Pip) |
| 1-2691 | 4-F-Ph | 4-Pyr | 1-Et-2-Allyl-4-(3,4-deH-Pip) |
| 1-2692 | 4-F-Ph | 4-Pyr | 1-Pr-2-Allyl-4-(3,4-deH-Pip) |
| 1-2693 | 4-F-Ph | 4-Pyr | 1-Bu-2-Allyl-4-(3,4-deH-Pip) |
| 1-2694 | 4-F-Ph | 4-Pyr | 1-Phet-2-Allyl-4-(3,4-deH-Pip) |
| 1-2695 | 4-F-Ph | 4-Pyr | 2-Bn-4-(3,4-deH-Pip) |
| 1-2696 | 4-F-Ph | 4-Pyr | 1-Me-2-Bn-4-(3,4-deH-Pip) |
| 1-2697 | 4-F-Ph | 4-Pyr | 1-Et-2-Bn-4-(3,4-deH-Pip) |
| 1-2698 | 4-F-Ph | 4-Pyr | 1-Pr-2-Bn-4-(3,4-deH-Pip) |
| 1-2699 | 4-F-Ph | 4-Pyr | 1-Bu-2-Bn-4-(3,4-deH-Pip) |
| 1-2700 | 4-F-Ph | 4-Pyr | 1-Phet-2-Bn-4-(3,4-deH-Pip) |
| 1-2701 | 4-F-Ph | 4-Pyr | 2-Phet-4-(3,4-deH-Pip) |
| 1-2702 | 4-F-Ph | 4-Pyr | 1-Me-2-Phet-4-(3,4-deH-Pip) |
| 1-2703 | 4-F-Ph | 4-Pyr | 1-Et-2-Phet-4-(3,4-deH-Pip) |
| 1-2704 | 4-F-Ph | 4-Pyr | 1-Pr-2-Phet-4-(3,4-deH-Pip) |
| 1-2705 | 4-F-Ph | 4-Pyr | 1-Bu-2-Phet-4-(3,4-deH-Pip) |
| 1-2706 | 4-F-Ph | 4-Pyr | 1,2-diPhet-4-(3,4-deH-Pip) |
| 1-2707 | 4-F-Ph | 4-Pyr | 2-Me-4-(4,5-deH-Pip) |
| 1-2708 | 4-F-Ph | 4-Pyr | 1,2-diMe-4-(4,5-deH-Pip) |
| 1-2709 | 4-F-Ph | 4-Pyr | 1-Et-2-Me-4-(4,5-deH-Pip) |
| 1-2710 | 4-F-Ph | 4-Pyr | 1-Pr-2-Me-4-(4,5-deH-Pip) |
| 1-2711 | 4-F-Ph | 4-Pyr | 1-Bu-2-Me-4-(4,5-deH-Pip) |
| 1-2712 | 4-F-Ph | 4-Pyr | 1-Phet-2-Me-4-(4,5-deH-Pip) |
| 1-2713 | 4-F-Ph | 4-Pyr | 2-Et-4-(4,5-deH-Pip) |
| 1-2714 | 4-F-Ph | 4-Pyr | 1-Me-2-Et-4-(4,5-deH-Pip) |
| 1-2715 | 4-F-Ph | 4-Pyr | 1,2-diEt-4-(4,5-deH-Pip) |
| 1-2716 | 4-F-Ph | 4-Pyr | 1-Pr-2-Et-4-(4,5-deH-Pip) |
| 1-2717 | 4-F-Ph | 4-Pyr | 1-Bu-2-Et-4-(4,5-deH-Pip) |
| 1-2718 | 4-F-Ph | 4-Pyr | 1-Phet-2-Et-4-(4,5-deH-Pip) |
| 1-2719 | 4-F-Ph | 4-Pyr | 2-Pr-4-(4,5-deH-Pip) |
| 1-2720 | 4-F-Ph | 4-Pyr | 1-Me-2-Pr-4-(4,5-deH-Pip) |
| 1-2721 | 4-F-Ph | 4-Pyr | 1-Et-2-Pr-4-(4,5-deH-Pip) |
| 1-2722 | 4-F-Ph | 4-Pyr | 1,2-diPr-4-(4,5-deH-Pip) |
| 1-2723 | 4-F-Ph | 4-Pyr | 1-Bu-2-Pr-4-(4,5-deH-Pip) |

Table 1 (continued)

| Compound No. | $R^1$ | $R^2$ | $R^3$ |
|---|---|---|---|
| 1-2724 | 4-F-Ph | 4-Pyr | 1-Phet-2-Pr-4-(4,5-deH-Pip) |
| 1-2725 | 4-F-Ph | 4-Pyr | 2-Bu-4-(4,5-deH-Pip) |
| 1-2726 | 4-F-Ph | 4-Pyr | 1-Me-2-Bu-4-(4,5-deH-Pip) |
| 1-2727 | 4-F-Ph | 4-Pyr | 1-Et-2-Bu-4-(4,5-deH-Pip) |
| 1-2728 | 4-F-Ph | 4-Pyr | 1-Pr-2-Bu-4-(4,5-deH-Pip) |
| 1-2729 | 4-F-Ph | 4-Pyr | 1,2-diBu-4-(4,5-deH-Pip) |
| 1-2730 | 4-F-Ph | 4-Pyr | 1-Phet-2-Bu-4-(4,5-deH-Pip) |
| 1-2731 | 4-F-Ph | 4-Pyr | 2-Allyl-4-(4,5-deH-Pip) |
| 1-2732 | 4-F-Ph | 4-Pyr | 1-Me-2-Allyl-4-(4,5-deH-Pip) |
| 1-2733 | 4-F-Ph | 4-Pyr | 1-Et-2-Allyl-4-(4,5-deH-Pip) |
| 1-2734 | 4-F-Ph | 4-Pyr | 1-Pr-2-Allyl-4-(4,5-deH-Pip) |
| 1-2735 | 4-F-Ph | 4-Pyr | 1-Bu-2-Allyl-4-(4,5-deH-Pip) |
| 1-2736 | 4-F-Ph | 4-Pyr | 1-Phet-2-Allyl-4-(4,5-deH-Pip) |
| 1-2737 | 4-F-Ph | 4-Pyr | 2-Bn-4-(4,5-deH-Pip) |
| 1-2738 | 4-F-Ph | 4-Pyr | 1-Me-2-Bn-4-(4,5-deH-Pip) |
| 1-2739 | 4-F-Ph | 4-Pyr | 1-Et-2-Bn-4-(4,5-deH-Pip) |
| 1-2740 | 4-F-Ph | 4-Pyr | 1-Pr-2-Bn-4-(4,5-deH-Pip) |
| 1-2741 | 4-F-Ph | 4-Pyr | 1-Bu-2-Bn-4-(4,5-deH-Pip) |
| 1-2742 | 4-F-Ph | 4-Pyr | 1-Phet-2-Bn-4-(4,5-deH-Pip) |
| 1-2743 | 4-F-Ph | 4-Pyr | 2-Phet-4-(4,5-deH-Pip) |
| 1-2744 | 4-F-Ph | 4-Pyr | 1-Me-2-Phet-4-(4,5-deH-Pip) |
| 1-2745 | 4-F-Ph | 4-Pyr | 1-Et-2-Phet-4-(4,5-deH-Pip) |
| 1-2746 | 4-F-Ph | 4-Pyr | 1-Pr-2-Phet-4-(4,5-deH-Pip) |
| 1-2747 | 4-F-Ph | 4-Pyr | 1-Bu-2-Phet-4-(4,5-deH-Pip) |
| 1-2748 | 4-F-Ph | 4-Pyr | 1,2-diPhet-4-(4,5-deH-Pip) |
| 1-2749 | 4-F-Ph | 2-$NH_2$-4-Pym | $H_2N$-$CH_2C$ $(Me)_2CH_2$ |
| 1-2750 | 4-F-Ph | 2-$NH_2$-4-Pym | MeNH-$CH_2C(Me)_2CH_2$ |
| 1-2751 | 4-F-Ph | 2-$NH_2$-4-Pym | EtNH-$CH_2C(Me)_2CH_2$ |
| 1-2752 | 4-F-Ph | 2-$NH_2$-4-Pym | $Me_2N$-$CH_2C$ $(Me)_2CH_2$ |
| 1-2753 | 4-F-Ph | 2-$NH_2$-4-Pym | 3-(3,4-deH-Pip) |
| 1-2754 | 4-F-Ph | 2-$NH_2$-4-Pym | 1-Me-3-(3,4-deH-Pip) |
| 1-2755 | 4-F-Ph | 2-$NH_2$-4-Pym | 1-Et-4-(3,4-deH-Pip) |
| 1-2756 | 4-F-Ph | 2-$NH_2$-4-Pym | 1-Pr-4-(3,4-deH-Pip) |
| 1-2757 | 4-F-Ph | 2-$NH_2$-4-Pym | 1-Pr-4-Pip |
| 1-2758 | 4-F-Ph | 2-$NH_2$-4-Pym | 1-iPr-4-(3,4-deH-Pip) |
| 1-2759 | 4-F-Ph | 2-$NH_2$-4-Pym | 1-iPr-4-Pip |
| 1-2760 | 4-F-Ph | 2-$NH_2$-4-Pym | 1-Bu-4-(3,4-deH-Pip) |
| 1-2761 | 4-F-Ph | 2-$NH_2$-4-Pym | 1-tBu-4-(3,4-deH-Pip) |

Table 1 (continued)

| Compound No. | $R^1$ | $R^2$ | $R^3$ |
|---|---|---|---|
| 1-2762 | 4-F-Ph | $2-NH_2-4-Pym$ | 1-Pn-4-(3,4-deH-Pip) |
| 1-2763 | 4-F-Ph | $2-NH_2-4-Pym$ | 1-Hx-4-(3,4-deH-Pip) |
| 1-2764 | 4-F-Ph | $2-NH_2-4-Pym$ | 1-Hp-4-(3,4-deH-Pip) |
| 1-2765 | 4-F-Ph | $2-NH_2-4-Pym$ | 1-Oc-4-(3,4-deH-Pip) |
| 1-2766 | 4-F-Ph | $2-NH_2-4-Pym$ | 1-Nn-4-(3,4-deH-Pip) |
| 1-2767 | 4-F-Ph | $2-NH_2-4-Pym$ | 1-cPr-4-(3,4-deH-Pip) |
| 1-2768 | 4-F-Ph | $2-NH_2-4-Pym$ | 1-cPn-4-(3,4-deH-Pip) |
| 1-2769 | 4-F-Ph | $2-NH_2-4-Pym$ | 1-cHx-4-(3,4-deH-Pip) |
| 1-2770 | 4-F-Ph | $2-NH_2-4-Pym$ | 1-Bn-4-(3,4-deH-Pip) |
| 1-2771 | 4-F-Ph | $2-NH_2-4-Pym$ | 1-Phet-4-(3,4-deH-Pip) |
| 1-2772 | 4-F-Ph | $2-NH_2-4-Pym$ | 1-(3-Ph-Pr)-4-(3,4-deH-Pip) |
| 1-2773 | 4-F-Ph | $2-NH_2-4-Pym$ | 1-(4-Ph-Bu)-4-(3,4-deH-Pip) |
| 1-2774 | 4-F-Ph | $2-NH_2-4-Pym$ | 1-Allyl-4-(3,4-deH-Pip) |
| 1-2775 | 4-F-Ph | $2-NH_2-4-Pym$ | 1-Propargyl-4-(3,4-deH-Pip) |
| 1-2776 | 4-F-Ph | $2-NH_2-4-Pym$ | 2,2,6,6-tetraMe-4-(3,4-deH-Pip) |
| 1-2777 | 4-F-Ph | $2-NH_2-4-Pym$ | 1,2,2,6,6-pentaMe-4-(3,4-deH-Pip) |
| 1-2778 | 4-F-Ph | $2-NH_2-4-Pym$ | 1,2,2,6,6-pentaMe-4-Pip) |
| 1-2779 | 4-F-Ph | $2-NH_2-4-Pym$ | 7-(1,2,3,5,6,8a-hexaH-Ind) |
| 1-2780 | 4-F-Ph | $2-NH_2-4-Pym$ | 7-(1,2,3,5,8,8a-hexaH-Ind) |
| 1-2781 | 4-F-Ph | $2-NH_2-4-Pym$ | 7-octaH-Ind |
| 1-2782 | 4-F-Ph | $2-NH_2-4-Pym$ | 8-(1,3,4,6,7,9a-hexaH-2H-Qui) |
| 1-2783 | 4-F-Ph | $2-NH_2-4-Pym$ | 8-(1,3,4,6,9,9a-hexaH-2H-Qui) |
| 1-2784 | 4-F-Ph | $2-NH_2-4-Pym$ | 8-octaH-Qui |
| 1-2785 | 4-F-Ph | $2-NH_2-4-Pym$ | 2,2-diMe-4-(3,4-deH-PiP) |
| 1-2786 | 4-F-Ph | $2-NH_2-4-Pym$ | 1,2,2-triMe-4-(3,4-deH-PiP) |
| 1-2787 | 4-F-Ph | $2-NH_2-4-Pym$ | 2,2-diMe-4-(4,5-deH-PiP) |
| 1-2788 | 4-F-Ph | $2-NH_2-4-Pym$ | 1,2,2-triMe-4-(4,5-deH-PiP) |
| 1-2789 | 4-F-Ph | $2-NH_2-4-Pym$ | 2,6-diMe-4-(3,4-deH-PiP) |
| 1-2790 | 4-F-Ph | $2-NH_2-4-Pym$ | 1,2,6-triMe-4-(3,4-deH-PiP) |
| 1-2791 | 4-F-Ph | $2-NH_2-4-Pym$ | 2-Me-4-(3,4-deH-Pip) |
| 1-2792 | 4-F-Ph | $2-NH_2-4-Pym$ | 1,2-dime-4-(3,4-deH-Pip) |
| 1-2793 | 4-F-Ph | $2-NH_2-4-Pym$ | 1-Et-2-Me-4-(3,4-deH-Pip) |
| 1-2794 | 4-F-Ph | $2-NH_2-4-Pym$ | 1-Pr-2-Me-4-(3,4-deH-Pip) |
| 1-2795 | 4-F-Ph | $2-NH_2-4-Pym$ | 1-Bu-2-Me-4-(3,4-deH-Pip) |
| 1-2796 | 4-F-Ph | $2-NH_2-4-Pym$ | 1-Phet-2-Me-4-(3,4-deH-Pip) |
| 1-2797 | 4-F-Ph | $2-NH_2-4-Pym$ | 2-Et-4-(3,4-deH-Pip) |
| 1-2798 | 4-F-Ph | $2-NH_2-4-Pym$ | 1-Me-2-Et-4-(3,4-deH-Pip) |
| 1-2799 | 4-F-Ph | $2-NH_2-4-Pym$ | 1,2-diEt-4-(3,4-deH-Pip) |

Table 1 (continued)

| Compound No. | $R^1$ | $R^2$ | $R^3$ |
|---|---|---|---|
| 1-2800 | 4-F-Ph | $2-NH_2-4-Pym$ | 1-Pr-2-Et-4-(3,4-deH-Pip) |
| 1-2801 | 4-F-Ph | $2-NH_2-4-Pym$ | 1-Bu-2-Et-4-(3,4-deH-Pip) |
| 1-2802 | 4-F-Ph | $2-NH_2-4-Pym$ | 1-Phet-2-Et-4-(3,4-deH-Pip) |
| 1-2803 | 4-F-Ph | $2-NH_2-4-Pym$ | 2-Pr-4-(3,4-deH-Pip) |
| 1-2804 | 4-F-Ph | $2-NH_2-4-Pym$ | 1-Me-2-Pr-4-(3,4-deH-Pip) |
| 1-2805 | 4-F-Ph | $2-NH_2-4-Pym$ | 1-Et-2-Pr-4-(3,4-deH-Pip) |
| 1-2806 | 4-F-Ph | $2-NH_2-4-Pym$ | 1,2-diPr-4-(3,4-deH-Pip) |
| 1-2807 | 4-F-Ph | $2-NH_2-4-Pym$ | 1-Bu-2-Pr-4-(3,4-deH-Pip) |
| 1-2808 | 4-F-Ph | $2-NH_2-4-Pym$ | 1-Phet-2-Pr-4-(3,4-deH-Pip) |
| 1-2809 | 4-F-Ph | $2-NH_2-4-Pym$ | 2-Bu-4-(3,4-deH-Pip) |
| 1-2810 | 4-F-Ph | $2-NH_2-4-Pym$ | 1-Me-2-Bu-4-(3,4-deH-Pip) |
| 1-2811 | 4-F-Ph | $2-NH_2-4-Pym$ | 1-Et-2-Bu-4-(3,4-deH-Pip) |
| 1-2812 | 4-F-Ph | $2-NH_2-4-Pym$ | 1-Pr-2-Bu-4-(3,4-deH-Pip) |
| 1-2813 | 4-F-Ph | $2-NH_2-4-Pym$ | 1,2-diBu-4-(3,4-deH-Pip) |
| 1-2814 | 4-F-Ph | $2-NH_2-4-Pym$ | 1-Phet-2-Bu-4-(3,4-deH-Pip) |
| 1-2815 | 4-F-Ph | $2-NH_2-4-Pym$ | 2-Allyl-4-(3,4-deH-Pip) |
| 1-2816 | 4-F-Ph | $2-NH_2-4-Pym$ | 1-Me-2-Allyl-4-(3,4-deH-Pip) |
| 1-2817 | 4-F-Ph | $2-NH_2-4-Pym$ | 1-Et-2-Allyl-9-(3,4-deH-Pip) |
| 1-2818 | 4-F-Ph | $2-NH_2-4-Pym$ | 1-Pr-2-Allyl-4-(3,4-deH-Pip) |
| 1-2819 | 4-F-Ph | $2-NH_2-4-Pym$ | 1-Bu-2-Allyl-4-(3,4-deH-Pip) |
| 1-2820 | 4-F-Ph | $2-NH_2-4-Pym$ | 1-Phet-2-Allyl-4-(3,4-deH-Pip) |
| 1-2821 | 4-F-Ph | $2-NH_2-4-Pym$ | 2-Bn-4-(3,4-deH-Pip) |
| 1-2822 | 4-F-Ph | $2-NH_2-4-Pym$ | 1-Me-2-Bn-4-(3,4-deH-Pip) |
| 1-2823 | 4-F-Ph | $2-NH_2-4-Pym$ | 1-Et-2-Bn-4-(3,4-deH-Pip) |
| 1-2824 | 4-F-Ph | $2-NH_2-4-Pym$ | 1-Pr-2-Bn-4-(3,4-deH-Pip) |
| 1-2825 | 4-F-Ph | $2-NH_2-4-Pym$ | 1-Bu-2-Bn-4-(3,4-deH-Pip) |
| 1-2826 | 4-F-Ph | $2-NH_2-4-Pym$ | 1-Phet-2-Bn-4-(3,4-deH-Pip) |
| 1-2827 | 4-F-Ph | $2-NH_2-4-Pym$ | 2-Phet-4-(3,4-deH-Pip) |
| 1-2828 | 4-F-Ph | $2-NH_2-4-Pym$ | 1-Me-2-Phet-4-(3,4-deH-Pip) |
| 1-2829 | 4-F-Ph | $2-NH_2-4-Pym$ | 1-Et-2-Phet-4-(3,4-deH-Pip) |
| 1-2830 | 4-F-Ph | $2-NH_2-4-Pym$ | 1-Pr-2-Phet-4-(3,4-deH-Pip) |
| 1-2831 | 4-F-Ph | $2-NH_2-4-Pym$ | 1-Bu-2-Phet-4-(3,4-deH-Pip) |
| 1-2832 | 4-F-Ph | $2-NH_2-4-Pym$ | 1,2-diPhet-4-(3,4-deH-Pip) |
| 1-2833 | 4-F-Ph | $2-NH_2-4-Pym$ | 2-Me-4-(4,5-deH-Pip) |
| 1-2834 | 4-F-Ph | $2-NH_2-4-Pym$ | 1,2-diMe-4-(4,5-deH-Pip) |
| 1-2835 | 4-F-Ph | $2-NH_2-4-Pym$ | 1-Et-2-Me-4-(4,5-deH-Pip) |
| 1-2836 | 4-F-Ph | $2-NH_2-4-Pym$ | 1-Pr-2-Me-4-(4,5-deH-Pip) |
| 1-2837 | 4-F-Ph | $2-NH_2-4-Pym$ | 1-Bu-2-Me-4-(4,5-deH-Pip) |

Table 1 (continued)

| Compound No. | $R^1$ | $R^2$ | $R^3$ |
|---|---|---|---|
| 1-2838 | 4-F-Ph | $2-NH_2-4-Pym$ | 1-Phet-2-Me-4-(4,5-deH-Pip) |
| 1-2839 | 4-F-Ph | $2-NH_2-4-Pym$ | 2-Et-4-(4,5-deH-Pip) |
| 1-2840 | 4-F-Ph | $2-NH_2-4-Pym$ | 1-Me-2-Et-4-(4,5-deH-Pip) |
| 1-2841 | 4-F-Ph | $2-NH_2-4-Pym$ | 1,2-diEt-4-(4,5-deH-Pip) |
| 1-2842 | 4-F-Ph | $2-NH_2-4-Pym$ | 1-Pr-2-Et-4-(4,5-deH-Pip) |
| 1-2843 | 4-F-Ph | $2-NH_2-4-Pym$ | 1-Bu-2-Et-4-(4,5-deH-Pip) |
| 1-2844 | 4-F-Ph | $2-NH_2-4-Pym$ | 1-Phet-2-Et-4-(4,5-deH-Pip) |
| 1-2845 | 4-F-Ph | $2-NH_2-4-Pym$ | 2-Pr-4-(4,5-deH-Pip) |
| 1-2846 | 4-F-Ph | $2-NH_2-4-Pym$ | 1-Me-2-Pr-4-(4,5-deH-Pip) |
| 1-2847 | 4-F-Ph | $2-NH_2-4-Pym$ | 1-Et-2-Pr-4-(4,5-deH-Pip) |
| 1-2848 | 4-F-Ph | $2-NH_2-4-Pym$ | 1,2-diPr-4-(4,5-deH-Pip) |
| 1-2849 | 4-F-Ph | $2-NH_2-4-Pym$ | 1-Bu-2-Pr-4-(4,5-deH-Pip) |
| 1-2850 | 4-F-Ph | $2-NH_2-4-Pym$ | 1-Phet-2-Pr-4-(4,5-deH-Pip) |
| 1-2851 | 4-F-Ph | $2-NH_2-4-Pym$ | 2-Bu-4-(4,5-deH-Pip) |
| 1-2852 | 4-F-Ph | $2-NH_2-4-Pym$ | 1-Me-2-Bu-4-(4,5-deH-Pip) |
| 1-2853 | 4-F-Ph | $2-NH_2-4-Pym$ | 1-Et-2-Bu-4-(4,5-deH-Pip) |
| 1-2854 | 4-F-Ph | $2-NH_2-4-Pym$ | 1-Pr-2-Bu-4-(4,5-deH-Pip) |
| 1-2855 | 4-F-Ph | $2-NH_2-4-Pym$ | 1,2-diBu-4-(4,5-deH-Pip) |
| 1-2856 | 4-F-Ph | $2-NH_2-4-Pym$ | 1-Phet-2-Bu-4-(4,5-deH-Pip) |
| 1-2857 | 4-F-Ph | $2-NH_2-4-Pym$ | 2-Allyl-4-(4,5-deH-Pip) |
| 1-2858 | 4-F-Ph | $2-NH_2-4-Pym$ | 1-Me-2-Allyl-4-(4,5-deH-Pip) |
| 1-2859 | 4-F-Ph | $2-NH_2-4-Pym$ | 1-Et-2-Allyl-4-(4,5-deH-Pip) |
| 1-2860 | 4-F-Ph | $2-NH_2-4-Pym$ | 1-Pr-2-Allyl-4-(4,5-deH-Pip) |
| 1-2861 | 4-F-Ph | $2-NH_2-4-Pym$ | 1-Bu-2-Allyl-4-(4,5-deH-Pip) |
| 1-2862 | 4-F-Ph | $2-NH_2-4-Pym$ | 1-Phet-2-Allyl-4-(4,5-deH-Pip) |
| 1-2863 | 4-F-Ph | $2-NH_2-4-Pym$ | 2-Bn-4-(4,5-deH-Pip) |
| 1-2864 | 4-F-Ph | $2-NH_2-4-Pym$ | 1-Me-2-Bn-4-(4,5-deH-Pip) |
| 1-2865 | 4-F-Ph | $2-NH_2-4-Pym$ | 1-Et-2-Bn-4-(4,5-deH-Pip) |
| 1-2866 | 4-F-Ph | $2-NH_2-4-Pym$ | 1-Pr-2-Bn-4-(4,5-deH-Pip) |
| 1-2867 | 4-F-Ph | $2-NH_2-4-Pym$ | 1-Bu-2-Bn-4-(4,5-deH-Pip) |
| 1-2868 | 4-F-Ph | $2-NH_2-4-Pym$ | 1-Phet-2-Bn-4-(4,5-deH-Pip) |
| 1-2869 | 4-F-Ph | $2-NH_2-4-Pym$ | 2-Phet-4-(4,5-deH-Pip) |
| 1-2870 | 4-F-Ph | $2-NH_2-4-Pym$ | 1-Me-2-Phet-4-(4,5-deH-Pip) |
| 1-2871 | 4-F-Ph | $2-NH_2-4-Pym$ | 1-Et-2-Phet-4-(4,5-deH-Pip) |
| 1-2872 | 4-F-Ph | $2-NH_2-4-Pym$ | 1-Pr-2-Phet-4-(4, 5-deH-Pip) |
| 1-2873 | 4-F-Ph | $2-NH_2-4-Pym$ | 1-Bu-2-Phet-4-(4,5-deH-Pip) |
| 1-2874 | 4-F-Ph | $2-NH_2-4-Pym$ | 1,2-diPhet-4-(4,5-deH-Pip) |
| 1-2875 | 4-F-Ph | 2-MeNH-4-Pym | $H_2N-CH_2C(Me)_2CH_2$ |

Table 1 (continued)

| Compound No. | $R^1$ | $R^2$ | $R^3$ |
|---|---|---|---|
| 1-2876 | 4-F-Ph | 2-MeNH-4-Pym | MeNH-$CH_2C(Me)_2CH_2$ |
| 1-2877 | 4-F-Ph | 2-MeNH-4-Pym | EtNH-$CH_2C(Me)_2CH_2$ |
| 1-2878 | 4-F-Ph | 2-MeNH-4-Pym | $Me_2N$-$CH_2C(Me)_2CH_2$ |
| 1-2879 | 4-F-Ph | 2-MeNH-4-Pym | 3-(3,4-deH-Pip) |
| 1-2880 | 4-F-Ph | 2-MeNH-4-Pym | 1-Me-3-(3,4-deli-Pip) |
| 1-2881 | 4-F-Ph | 2-MeNH-4-Pym | 1-Et-4-(3,4-deH-Pip) |
| 1-2882 | 4-F-Ph | 2-MeNH-4-Pym | 1-Pr-4-(3,4-deH-Pip) |
| 1-2883 | 4-F-Ph | 2-MeNH-4-Pym | 1-Pr-4-Pip |
| 1-2884 | 4-F-Ph | 2-MeNH-4-Pym | 1-iPr-4-(3,4-deH-Pip) |
| 1-2885 | 4-F-Ph | 2-MeNH-4-Pym | 1-iPr-4-Pip |
| 1-2886 | 4-F-Ph | 2-MeNH-4-Pym | 1-Bu-4-(3,4-deH-Pip) |
| 1-2887 | 4-F-Ph | 2-MeNH-4-Pym | 1-tBu-4-(3,4-deH-Pip) |
| 1-2888 | 4-F-Ph | 2-MeNH-4-Pym | 1-Pn-4-(3,4-deH-Pip) |
| 1-2889 | 4-F-Ph | 2-MeNH-4-Pym | 1-Hx-4-(3,4-deH-Pip) |
| 1-2890 | 4-F-Ph | 2-MeNH-4-Pym | 1-Hp-4-(3,4-deH-Pip) |
| 1-2891 | 4-F-Ph | 2-MeNH-4-Pym | 1-Oc-4-(3,4-deH-Pip) |
| 1-2892 | 4-F-Ph | 2-MeNH-4-Pym | 1-Nn-4-(3,4-deH-Pip) |
| 1-2893 | 4-F-Ph | 2-MeNH-4-Pym | 1-cPr-4-(3,4-deH-Pip) |
| 1-2894 | 4-F-Ph | 2-MeNH-4-Pym | 1-cPn-4-(3,4-deH-Pip) |
| 1-2895 | 4-F-Ph | 2-MeNH-4-Pym | 1-cHx-4-(3,4-deH-Pip) |
| 1-2896 | 4-F-Ph | 2-MeNH-4-Pym | 1-Bn-4-(3,4-deH-Pip) |
| 1-2897 | 4-F-Ph | 2-MeNH-4-Pym | 1-Phet-4-(3,4-deH-Pip) |
| 1-2898 | 4-F-Ph | 2-MeNH-4-Pym | 1-(3-Ph-Pr)-4-(3,4-deH-Pip) |
| 1-2899 | 4-F-Ph | 2-MeNH-4-Pym | 1-(4-Ph-Bu)-4-(3,4-deH-Pip) |
| 1-2900 | 4-F-Ph | 2-MeNH-4-Pym | 1-Allyl-4-(3,4-deH-Pip) |
| 1-2901 | 4-F-Ph | 2-MeNH-4-Pym | 1-Propargyl-4-(3,4-deH-Pip) |
| 1-2902 | 4-F-Ph | 2-MeNH-4-Pym | 2,2,6,6-tetraMe-4-(3,4-deH-Pip) |
| 1-2903 | 4-F-Ph | 2-MeNH-4-Pym | 1,2,2,6,6-pentaMe-4-(3,4-deH-Pip) |
| 1-2904 | 4-F-Ph | 2-MeNH-4-Pym | 1,2,2,6,6-pentaMe-4-Pip) |
| 1-2905 | 4-F-Ph | 2-MeNH-4-Pym | 7-(1,2,3,5,6,8a-hexaH-Ind) |
| 1-2906 | 4-F-Ph | 2-MeNH-4-Pym | 7-(1,2,3,5,8,8a-hexaH-Ind) |
| 1-2907 | 4-F-Ph | 2-MeNH-4-Pym | 7-octaH-Ind |
| 1-2908 | 4-F-Ph | 2-MeNH-4-Pym | 8-(1,3,4,6,7,9a-hexaH-2H-Qui) |
| 1-2909 | 4-F-Ph | 2-MeNH-4-Pym | 8-(1,3,4,6,9,9a-hexaH-2H-Qui) |
| 1-2910 | 4-F-Ph | 2-MeNH-4-Pym | 8-octaH-Qui |
| 1-2911 | 4-F-Ph | 2-MeNH-4-Pym | 2,2-dime-4-(3,4-deH-PiP) |
| 1-2912 | 4-F-Ph | 2-MeNH-4-Pym | 1,2,2-triMe-4-(3,4-deH-PiP) |
| 1-2913 | 4-F-Ph | 2-MeNH-4-Pym | 2,2-diMe-4-(4,5-deH-PiP) |

Table 1 (continued)

| Compound No. | $R^1$ | $R^2$ | $R^3$ |
|---|---|---|---|
| 1-2914 | 4-F-Ph | 2-MeNH-4-Pym | 1,2,2-triMe-4-(4,5-deH-PiP) |
| 1-2915 | 4-F-Ph | 2-MeNH-4-Pym | 2,6-diMe-4-(3,4-deH-PiP) |
| 1-2916 | 4-F-Ph | 2-MeNH-4-Pym | 1,2,6-triMe-4-(3,4-deH-PiP) |
| 1-2917 | 4-F-Ph | 2-MeNH-4-Pym | 2-Me-4-(3,4-deH-Pip) |
| 1-2918 | 4-F-Ph | 2-MeNH-4-Pym | 1,2-dime-4-(3,4-deH-Pip) |
| 1-2919 | 4-F-Ph | 2-MeNH-4-Pym | 1-Et-2-Me-4-(3,4-deH-Pip) |
| 1-2920 | 4-F-Ph | 2-MeNH-4-Pym | 1-Pr-2-Me-4-(3,4-deH-Pip) |
| 1-2921 | 4-F-Ph | 2-MeNH-4-Pym | 1-Bu-2-Me-4-(3,4-deH-Pip) |
| 1-2922 | 4-F-Ph | 2-MeNH-4-Pym | 1-Phet-2-Me-4-(3,4-deH-Pip) |
| 1-2923 | 4-F-Ph | 2-MeNH-4-Pym | 2-Et-4-(3,4-deH-Pip) |
| 1-2924 | 4-F-Ph | 2-MeNH-4-Pym | 1-Me-2-Et-4-(3,4-deH-Pip) |
| 1-2925 | 4-F-Ph | 2-MeNH-4-Pym | 1,2-diEt-4-(3,4-deH-Pip) |
| 1-2926 | 4-F-Ph | 2-MeNH-4-Pym | 1-Pr-2-Et-4-(3,4-deH-Pip) |
| 1-2927 | 4-F-Ph | 2-MeNH-4-Pym | 1-Bu-2-Et-4-(3,4-deH-Pip) |
| 1-2928 | 4-F-Ph | 2-MeNH-4-Pym | 1-Phet-2-Et-4-(3,4-deH-Pip) |
| 1-2929 | 4-F-Ph | 2-MeNH-4-Pym | 2-Pr-4-(3,4-deH-Pip) |
| 1-2930 | 4-F-Ph | 2-MeNH-4-Pym | 1-Me-2-Pr-4-(3,4-deH-Pip) |
| 1-2931 | 4-F-Ph | 2-MeNH-4-Pym | 1-Et-2-Pr-4-(3,4-deH-Pip) |
| 1-2932 | 4-F-Ph | 2-MeNH-4-Pym | 1,2-diPr-4-(3,4-deH-Pip) |
| 1-2933 | 4-F-Ph | 2-MeNH-4-Pym | 1-Bu-2-Pr-4-(3,4-deH-Pip) |
| 1-2934 | 4-F-Ph | 2-MeNH-4-Pym | 1-Phet-2-Pr-4-(3,4-deH-Pip) |
| 1-2935 | 4-F-Ph | 2-MeNH-4-Pym | 2-Bu-4-(3,4-deH-Pip) |
| 1-2936 | 4-F-Ph | 2-MeNH-4-Pym | 1-Me-2-Bu-4-(3,4-deH-Pip) |
| 1-2937 | 4-F-Ph | 2-MeNH-4-Pym | 1-Et-2-Bu-4-(3,4-deH-Pip) |
| 1-2938 | 4-F-Ph | 2-MeNH-4-Pym | 1-Pr-2-Bu-4-(3,4-deH-Pip) |
| 1-2939 | 4-F-Ph | 2-MeNH-4-Pym | 1,2-diBu-4-(3,4-deH-Pip) |
| 1-2940 | 4-F-Ph | 2-MeNH-4-Pym | 1-Phet-2-Bu-4-(3,4-deH-Pip) |
| 1-2941 | 4-F-Ph | 2-MeNH-4-Pym | 2-Allyl-4-(3,4-deH-Pip) |
| 1-2942 | 4-F-Ph | 2-MeNH-4-Pym | 1-Me-2-Allyl-4-(3,4-deH-Pip) |
| 1-2943 | 4-F-Ph | 2-MeNH-4-Pym | 1-Et-2-Allyl-4-(3,4-deH-Pip) |
| 1-2944 | 4-F-Ph | 2-MeNH-4-Pym | 1-Pr-2-Allyl-4-(3,4-deH-Pip) |
| 1-2945 | 4-F-Ph | 2-MeNH-4-Pym | 1-Bu-2-Allyl-4-(3,4-deH-Pip) |
| 1-2946 | 4-F-Ph | 2-MeNH-4-Pym | 1-Phet-2-Allyl-9-(3,4-deH-Pip) |
| 1-2947 | 4-F-Ph | 2-MeNH-4-Pym | 2-Bn-4-(3,4-deH-Pip) |
| 1-2948 | 4-F-Ph | 2-MeNH-4-Pym | 1-Me-2-Bn-4-(3,4-deH-Pip) |
| 1-2949 | 4-F-Ph | 2-MeNH-4-Pym | 1-Et-2-Bn-4-(3,4-deH-Pip) |
| 1-2950 | 4-F-Ph | 2-MeNH-4-Pym | 1-Pr-2-Bn-4-(3,4-deH-Pip) |
| 1-2951 | 4-F-Ph | 2-MeNH-4-Pym | 1-Bu-2-Bn-4-(3,4-deH-Pip) |

Table 1 (continued)

| Compound No. | $R^1$ | $R^2$ | $R^3$ |
|---|---|---|---|
| 1-2952 | 4-F-Ph | 2-MeNH-4-Pym | 1-Phet-2-Bn-4-(3,4-deH-Pip) |
| 1-2953 | 4-F-Ph | 2-MeNH-4-Pym | 2-Phet-4-(3,4-deH-Pip) |
| 1-2954 | 4-F-Ph | 2-MeNH-4-Pym | 1-Me-2-Phet-4-(3,4-deH-Pip) |
| 1-2955 | 4-F-Ph | 2-MeNH-4-Pym | 1-Et-2-Phet-4-(3,4-deH-Pip) |
| 1-2956 | 4-F-Ph | 2-MeNH-4-Pym | 1-Pr-2-Phet-4-(3,4-deH-Pip) |
| 1-2957 | 4-F-Ph | 2-MeNH-4-Pym | 1-Bu-2-Phet-4-(3,4-deH-Pip) |
| 1-2958 | 4-F-Ph | 2-MeNH-4-Pym | 1,2-diPhet-4-(3,4-deH-Pip) |
| 1-2959 | 4-F-Ph | 2-MeNH-4-Pym | 2-Me-4-(4,5-deH-Pip) |
| 1-2960 | 4-F-Ph | 2-MeNH-4-Pym | 1,2-diMe-4-(4,5-deH-Pip) |
| 1-2961 | 4-F-Ph | 2-MeNH-4-Pym | 1-Et-2-Me-4-(4,5-deH-Pip) |
| 1-2962 | 4-F-Ph | 2-MeNH-4-Pym | 1-Pr-2-Me-4-(4,5-deH-Pip) |
| 1-2963 | 4-F-Ph | 2-MeNH-4-Pym | 1-Bu-2-Me-4-(4,5-deH-Pip) |
| 1-2964 | 4-F-Ph | 2-MeNH-4-Pym | 1-Phet-2-Me-4-(4,5-deH-Pip) |
| 1-2965 | 4-F-Ph | 2-MeNH-4-Pym | 2-Et-4-(4,5-deH-Pip) |
| 1-2966 | 4-F-Ph | 2-MeNH-4-Pym | 1-Me-2-Et-4-(4,5-deH-Pip) |
| 1-2967 | 4-F-Ph | 2-MeNH-4-Pym | 1,2-diEt-4-(4,5-deH-Pip) |
| 1-2968 | 4-F-Ph | 2-MeNH-4-Pym | 1-Pr-2-Et-4-(4,5-deH-Pip) |
| 1-2969 | 4-F-Ph | 2-MeNH-4-Pym | 1-Bu-2-Et-4-(4,5-deH-Pip) |
| 1-2970 | 4-F-Ph | 2-MeNH-4-Pym | 1-Phet-2-Et-4-(4,5-deH-Pip) |
| 1-2971 | 4-F-Ph | 2-MeNH-4-Pym | 2-Pr-4-(4,5-deH-Pip) |
| 1-2972 | 4-F-Ph | 2-MeNH-4-Pym | 1-Me-2-Pr-4-(4,5-deH-Pip) |
| 1-2973 | 4-F-Ph | 2-MeNH-4-Pym | 1-Et-2-Pr-4-(4,5-deH-Pip) |
| 1-2974 | 4-F-Ph | 2-MeNH-4-Pym | 1,2-diPr-4-(4,5-deH-Pip) |
| 1-2975 | 4-F-Ph | 2-MeNH-4-Pym | 1-Bu-2-Pr-4- (4,5-deH-Pip) |
| 1-2976 | 4-F-Ph | 2-MeNH-4-Pym | 1-Phet-2-Pr-4-(4,5-deH-Pip) |
| 1-2977 | 4-F-Ph | 2-MeNH-4-Pym | 2-Bu-4-(4,5-deH-Pip) |
| 1-2978 | 4-F-Ph | 2-MeNH-4-Pym | 1-Me-2-Bu-4-(4,5-deH-Pip) |
| 1-2979 | 4-F-Ph | 2-MeNH-4-Pym | 1-Et-2-Bu-4-(4,5-deH-Pip) |
| 1-2980 | 4-F-Ph | 2-MeNH-4-Pym | 1-Pr-2-Bu-4-(4,5-deH-Pip) |
| 1-2981 | 4-F-Ph | 2-MeNH-4-Pym | 1,2-diBu-4-(4,5-deH-Pip) |
| 1-2982 | 4-F-Ph | 2-MeNH-4-Pym | 1-Phet-2-Bu-4 (4,5-deH-Pip) |
| 1-2983 | 4-F-Ph | 2-MeNH-4-Pym | 2-Allyl-4-(4,5-deH-Pip) |
| 1-2984 | 4-F-Ph | 2-MeNH-4-Pym | 1-Me-2-Allyl-4-(4,5-deH-Pip) |
| 1-2985 | 4-F-Ph | 2-MeNH-4-Pym | 1-Et-2-Allyl-4-(4,5-deH-Pip) |
| 1-2986 | 4-F-Ph | 2-MeNH-4-Pym | 1-Pr-2-Allyl-4-(4,5-deH-Pip) |
| 1-2987 | 4-F-Ph | 2-MeNH-4-Pym | 1-Bu-2-Allyl-4-(4,5-deH-Pip) |
| 1-2988 | 4-F-Ph | 2-MeNH-4-Pym | 1-Phet-2-Allyl-4-(4,5-deH-Pip) |
| 1-2989 | 4-F-Ph | 2-MeNH-4-Pym | 2-Bn-4-(4,5-deH-Pip) |

Table 1 (continued)

| Compound No. | $R^1$ | $R^2$ | $R^3$ |
|---|---|---|---|
| 1-2990 | 4-F-Ph | 2-MeNH-4-Pym | 1-Me-2-Bn-4-(4,5-deH-Pip) |
| 1-2991 | 4-F-Ph | 2-MeNH-4-Pym | 1-Et-2-Bn-4-(4,5-deH-Pip) |
| 1-2992 | 4-F-Ph | 2-MeNH-4-Pym | 1-Pr-2-Bn-4-(4,5-deH-Pip) |
| 1-2993 | 4-F-Ph | 2-MeNH-4-Pym | 1-Bu-2-Bn-4-(4,5-deH-Pip) |
| 1-2994 | 4-F-Ph | 2-MeNH-4-Pym | 1-Phet-2-Bn-4-(4,5-deH-Pip) |
| 1-2995 | 4-F-Ph | 2-MeNH-4-Pym | 2-Phet-4-(4,5-deH-Pip) |
| 1-2996 | 4-F-Ph | 2-MeNH-4-Pym | 1-Me-2-Phet-4-(4,5-deH-Pip) |
| 1-2997 | 4-F-Ph | 2-MeNH-4-Pym | 1-Et-2-Phet-4-(4,5-deH-Pip) |
| 1-2998 | 4-F-Ph | 2-MeNH-4-Pym | 1-Pr-2-Phet-4-(4,5-deH-Pip) |
| 1-2999 | 4-F-Ph | 2-MeNH-4-Pym | 1-Bu-2-Phet-4-(4,5-deH-Pip) |
| 1-3000 | 4-F-Ph | 2-MeNH-4-Pym | 1,2-diPhet-4-(4,5-deH-Pip) |
| 1-3001 | 4-F-Ph | $2-NH_2-4-Pyr$ | $H_2N-CH_2C(Me)_2CH_2$ |
| 1-3002 | 4-F-Ph | $2-NH_2-4-Pyr$ | $MeNH-CH_2C(Me)_2CH_2$ |
| 1-3003 | 4-F-Ph | $2-NH_2-4-Pyr$ | $EtNH-CH_2C(Me)_2CH_2$ |
| 1-3004 | 4-F-Ph | $2-NH_2-4-Pyr$ | $Me_2N-CH_2C(Me)_2CH_2$ |
| 1-3005 | 4-F-Ph | $2-NH_2-4-Pyr$ | 3-(3, 4-deH-Pip) |
| 1-3006 | 4-F-Ph | $2-NH_2-4-Pyr$ | 1-Me-3-(3,4-deH-Pip) |
| 1-3007 | 4-F-Ph | $2-NH_2-4-Pyr$ | 1-Et-4-(3,4-deH-Pip) |
| 1-3008 | 4-F-Ph | $2-NH_2-4-Pyr$ | 1-Pr-4-(3,4-deH-Pip) |
| 1-3009 | 4-F-Ph | $2-NH_2-4-Pyr$ | 1-Pr-4-Pip |
| 1-3010 | 4-F-Ph | $2-NH_2-4-Pyr$ | 1-iPr-4-(3,4-deH-Pip) |
| 1-3011 | 4-F-Ph | $2-NH_2-4-Pyr$ | 1-iPr-4-Pip |
| 1-3012 | 4-F-Ph | $2-NH_2-4-Pyr$ | 1-Bu-4-(3,4-deH-Pip) |
| 1-3013 | 4-F-Ph | $2-NH_2-4-Pyr$ | 1-tBu-4-(3,4-deli-Pip) |
| 1-3014 | 4-F-Ph | $2-NH_2-4-Pyr$ | 1-Pn-4-(3,4-deli-Pip) |
| 1-3015 | 4-F-Ph | $2-NH_2-4-Pyr$ | 1-Hx-4-(3,4-deH-Pip) |
| 1-3016 | 4-F-Ph | $2-NH_2-4-Pyr$ | 1-Hp-4-(3,4-deH-Pip) |
| 1-3017 | 4-F-Ph | $2-NH_2-4-Pyr$ | 1-Oc-4-(3,4-deH-Pip) |
| 1-3018 | 4-F-Ph | $2-NH_2-4-Pyr$ | 1-Nn-4-(3,4-deH-Pip) |
| 1-3019 | 4-F-Ph | $2-NH_2-4-Pyr$ | 1-cPr-4-(3,4-deH-Pip) |
| 1-3020 | 4-F-Ph | $2-NH_2-4-Pyr$ | 1-cPn-4-(3,4-deH-Pip) |
| 1-3021 | 4-F-Ph | $2-NH_2-4-Pyr$ | 1-CHx-4-(3,4-deH-Pip) |
| 1-3022 | 4-F-Ph | $2-NH_2-4-Pyr$ | 1-Bn-4-(3,4-deH-Pip) |
| 1-3023 | 4-F-Ph | $2-NH_2-4-Pyr$ | 1-Phet-4-(3,4-deH-Pip) |
| 1-3024 | 4-F-Ph | $2-NH_2-4-Pyr$ | 1-(3-Ph-Pr)-4-(3,4-deH-Pip) |
| 1-3025 | 4-F-Ph | $2-NH_2-4-Pyr$ | 1-(4-Ph-Bu)-4-(3,4-deH-Pip) |
| 1-3026 | 4-F-Ph | $2-NH_2-4-Pyr$ | 1-Allyl-4-(3,4-deH-Pip) |
| 1-3027 | 4-F-Ph | $2-NH_2-4-Pyr$ | 1-Propargyl-4-(3,4-deH-Pip) |

Table 1 (continued)

| Compound No. | $R^1$ | $R^2$ | $R^3$ |
|---|---|---|---|
| 1-3028 | 4-F-Ph | $2-NH_2-4-Pyr$ | 2,2,6,6-tetraMe-4-(3,4-deH-Pip) |
| 1-3029 | 4-F-Ph | $2-NH_2-4-Pyr$ | 1,2,2,6,6-pentaMe-4-(3,4-deH-Pip) |
| 1-3030 | 4-F-Ph | $2-NH_2-4-Pyr$ | 1,2,2,6,6-pentaMe-4-Pip) |
| 1-3031 | 4-F-Ph | $2-NH_2-4-Pyr$ | 7-(1,2,3,5,6,8a-hexaH-Ind) |
| 1-3032 | 4-F-Ph | $2-NH_2-4-Pyr$ | 7-(1,2,3,5,8,8a-hexaH-Ind) |
| 1-3033 | 4-F-Ph | $2-NH_2-4-Pyr$ | 7-octaH-Ind |
| 1-3034 | 4-F-Ph | $2-NH_2-4-Pyr$ | 8-(1,3,4,6,7,9a-hexaH-2H-Qui) |
| 1-3035 | 4-F-Ph | $2-NH_2-4-Pyr$ | 8-(1,3,4,6,9,9a-hexaH-2H-Qui) |
| 1-3036 | 4-F-Ph | $2-NH_2-4-Pyr$ | 8-octaH-Qui |
| 1-3037 | 4-F-Ph | $2-NH_2-4-Pyr$ | 2,2-dime-4- (3, 4-deH-PiP) |
| 1-3038 | 4-F-Ph | $2-NH_2-4-Pyr$ | 1,2,2-triMe-4-(3,4-deH-PiP) |
| 1-3039 | 4-F-Ph | $2-NH_2-4-Pyr$ | 2,2-diMe-4-(4,5-deH-PiP) |
| 1-3040 | 4-F-Ph | $2-NH_2-4-Pyr$ | 1,2,2-triMe-4-(4,5-deH-PiP) |
| 1-3041 | 4-F-Ph | $2-NH_2-4-Pyr$ | 2,6-diMe-4-(3,4-deH-PiP) |
| 1-3042 | 4-F-Ph | $2-NH_2-4-Pyr$ | 1,2,6-triMe-4-(3,4-deH-PiP) |
| 1-3043 | 4-F-Ph | $2-NH_2-4-Pyr$ | 2-Me-4-(3,4-deH-Pip) |
| 1-3044 | 4-F-Ph | $2-NH_2-4-Pyr$ | 1,2-diMe-4-(3,4-deH-Pip) |
| 1-3045 | 4-F-Ph | $2-NH_2-4-Pyr$ | 1-Et-2-Me-4-(3,4-deH-Pip) |
| 1-3046 | 4-F-Ph | $2-NH_2-4-Pyr$ | 1-Pr-2-Me-4-(3,4-deH-Pip) |
| 1-3047 | 4-F-Ph | $2-NH_2-4-Pyr$ | 1-Bu-2-Me-4-(3,4-deH-Pip) |
| 1-3048 | 4-F-Ph | $2-MH_2-4-Pyr$ | 1-Phet-2-Me-4-(3,4-deH-Pip) |
| 1-3049 | 4-F-Ph | $2-NH_2-4-Pyr$ | 2-Et-4-(3,4-deH-Pip) |
| 1-3050 | 4-F-Ph | $2-NH_2-4-Pyr$ | 1-Me-2-Et-4-(3,4-deH-Pip) |
| 1-3051 | 4-F-Ph | $2-NH_2-4-Pyr$ | 1,2-diEt-4-(3,4-deH-Pip) |
| 1-3052 | 4-F-Ph | $2-NH_2-4-Pyr$ | 1-Pr-2-Et-4-(3,4-deH-Pip) |
| 1-3053 | 4-F-Ph | $2-NH_2-4-Pyr$ | 1-Bu-2-Et-4-(3,4-deH-Pip) |
| 1-3054 | 4-F-Ph | $2-NH_2-4-Pyr$ | 1-Phet-2-Et-4-(3,4-deH-Pip) |
| 1-3055 | 4-F-Ph | $2-NH_2-4-Pyr$ | 2-Pr-4-(3,4-deH-Pip) |
| 1-3056 | 4-F-Ph | $2-NH_2-4-Pyr$ | 1-Me-2-Pr-4-(3,4-deH-Pip) |
| 1-3057 | 4-F-Ph | $2-NH_2-4-Pyr$ | 1-Et-2-Pr-4-(314-deH-Pip) |
| 1-3058 | 4-F-Ph | $2-NH_2-4-Pyr$ | 1,2-diPr-4-(3,4-deH-Pip) |
| 1-3059 | 4-F-Ph | $2-NH_2-4-Pyr$ | 1-Bu-2-Pr-4-(3,4-deH-Pip) |
| 1-3060 | 4-F-Ph | $2-NH_2-4-Pyr$ | 1-Phet-2-Pr-4-(3,4-deH-Pip) |
| 1-3061 | 4-F-Ph | $2-NH_2-4-Pyr$ | 2-Bu-4-(3,4-deH-Pip) |
| 1-3062 | 4-F-Ph | $2-NH_2-4-Pyr$ | 1-Me-2-Bu-4-(3,4-deH-Pip) |
| 1-3063 | 4-F-Ph | $2-NH_2-4-Pyr$ | 1-Et-2-Bu-4-(3,4-deH-Pip) |
| 1-3064 | 4-F-Ph | $2-NH_2-4-Pyr$ | 1-Pr-2-Bu-4-(3,4-deH-Pip) |
| 1-3065 | 4-F-Ph | $2-NH_2-4-Pyr$ | 1,2-diBu-4-(3,4-deH-Pip) |

Table 1 (continued)

| Compound No. | $R^1$ | $R^2$ | $R^3$ |
|---|---|---|---|
| 1-3066 | 4-F-Ph | 2-$NH_2$-4-Pyr | 1-Phet-2-Bu-4-(3,4-deH-Pip) |
| 1-3067 | 4-F-Ph | 2-$NH_2$-4-Pyr | 2-Allyl-4-(3,4-deH-Pip) |
| 1-3068 | 4-F-Ph | 2-$NH_2$-4-Pyr | 1-Me-2-Allyl-4-(3,4-deH-Pip) |
| 1-3069 | 4-F-Ph | 2-$NH_2$-4-Pyr | 1-Et-2-Allyl-4-(3,4-deH-Pip) |
| 1-3070 | 4-F-Ph | 2-$NH_2$-4-Pyr | 1-Pr-2-Allyl-4-(3,4-deH-Pip) |
| 1-3071 | 4-F-Ph | 2-$NH_2$-4-Pyr | 1-Bu-2-Allyl-4-(3,4-deH-Pip) |
| 1-3072 | 4-F-Ph | 2-$NH_2$-4-Pyr | 1-Phet-2-Allyl-4-(3,4-deH-Pip) |
| 1-3073 | 4-F-Ph | 2-$NH_2$-4-Pyr | 2-Bn-4-(3,4-deH-Pip) |
| 1-3074 | 4-F-Ph | 2-$NH_2$-4-Pyr | 1-Me-2-Bn-4-(3,4-deH-Pip) |
| 1-3075 | 4-F-Ph | 2-$NH_2$-4-Pyr | 1-Et-2-Bn-4-(3,4-deH-Pip) |
| 1-3076 | 4-F-Ph | 2-$NH_2$-4-Pyr | 1-Pr-2-Bn-4-(3,4-deH-Pip) |
| 1-3077 | 4-F-Ph | 2-$NH_2$-4-Pyr | 1-Bu-2-Bn-4-(3,4-deH-Pip) |
| 1-3078 | 4-F-Ph | 2-$NH_2$-4-Pyr | 1-Phet-2-Bn-4-(3,4-deH-Pip) |
| 1-3079 | 4-F-Ph | 2-$NH_2$-4-Pyr | 2-Phet-4-(3,4-deH-Pip) |
| 1-3080 | 4-F-Ph | 2-$NH_2$-4-Pyr | 1-Me-2-Phet-4-(3,4-deH-Pip) |
| 1-3081 | 4-F-Ph | 2-$NH_2$-4-Pyr | 1-Et-2-Phet-4-(3,4-deH-Pip) |
| 1-3082 | 4-F-Ph | 2-$NH_2$-4-Pyr | 1-Pr-2-Phet-4-(3,4-deH-Pip) |
| 1-3083 | 4-F-Ph | 2-$NH_2$-4-Pyr | 1-Bu-2-Phet-4-(3,4-deH-Pip) |
| 1-3084 | 4-F-Ph | 2-$NH_2$-4-Pyr | 1,2-diPhet-4-(3,4-deH-Pip) |
| 1-3085 | 4-F-Ph | 2-$NH_2$-4-Pyr | 2-Me-4-(4,5-deH-Pip) |
| 1-3086 | 4-F-Ph | 2-$NH_2$-4-Pyr | 1,2-diMe-4-(4,5-deH-Pip) |
| 1-3087 | 4-F-Ph | 2-$NH_2$-4-Pyr | 1-Et-2-Me-4-(4,5-deH-Pip) |
| 1-3088 | 4-F-Ph | 2-$NH_2$-4-Pyr | 1-Pr-2-Me-4-(4,5-deH-Pip) |
| 1-3089 | 4-F-Ph | 2-$NH_2$-4-Pyr | 1-Bu-2-Me-4-(4,5-deH-Pip) |
| 1-3090 | 4-F-Ph | 2-$NH_2$-4-Pyr | 1-Phet-2-Me-4-(4,5-deH-Pip) |
| 1-3091 | 4-F-Ph | 2-$NH_2$-4-Pyr | 2-Et-4-(4,5-deH-Pip) |
| 1-3092 | 4-F-Ph | 2-NH2-4-Pyr | 1-Me-2-Et-4-(4,5-deH-Pip) |
| 1-3093 | 4-F-Ph | 2-$NH_2$-4-Pyr | 1, 2-diEt-9-(4,5-deH-Pip) |
| 1-3094 | 4-F-Ph | 2-$NH_2$-4-Pyr | 1-Pr-2-Et-4-(4,5-deH-Pip) |
| 1-3095 | 4-F-Ph | 2-$NH_2$-4-Pyr | 1-Bu-2-Et-4-(4,5-deH-Pip) |
| 1-3096 | 4-F-Ph | 2-$NH_2$-4-Pyr | 1-Phet-2-Et-4-(4,5-deH-Pip) |
| 1-3097 | 4-F-Ph | 2-$NH_2$-4-Pyr | 2-Pr-4-(4,5-deH-Pip) |
| 1-3098 | 4-F-Ph | 2-$NH_2$-4-Pyr | 1-Me-2-Pr-4-(4,5-deH-Pip) |
| 1-3099 | 4-F-Ph | 2-$NH_2$-4-Pyr | 1-Et-2-Pr-4-(4,5-deH-Pip) |
| 1-3100 | 4-F-Ph | 2-$NH_2$-4-Pyr | 1,2-diPr-4-(4,5-deH-Pip) |
| 1-3101 | 4-F-Ph | 2-$NH_2$-4-Pyr | 1-Bu-2-Pr-4-(4,5-deH-Pip) |
| 1-3102 | 4-F-Ph | 2-$NH_2$-4-Pyr | 1-Phet-2-Pr-4-(4,5-deH-Pip) |
| 1-3103 | 4-F-Ph | 2-$NH_2$-4-Pyr | 2-Bu-4-(4,5-deH-Pip) |

Table 1 (continued)

| Compound No. | $R^1$ | $R^2$ | $R^3$ |
|---|---|---|---|
| 1-3104 | 4-F-Ph | 2-$NH_2$-4-Pyr | 1-Me-2-Bu-4-(4,5-deH-Pip) |
| 1-3105 | 4-F-Ph | 2-$NH_2$-4-Pyr | 1-Et-2-Bu-4-(4,5-deH-Pip) |
| 1-3106 | 4-F-Ph | 2-$NH_2$-4-Pyr | 1-Pr-2-Bu-4-(4,5-deH-Pip) |
| 1-3107 | 4-F-Ph | 2-$NH_2$-4-Pyr | 1,2-diBu-4-(4,5-deH-Pip) |
| 1-3108 | 4-F-Ph | 2-$NH_2$-4-Pyr | 1-Phet-2-Bu-4-(4,5-deH-Pip) |
| 1-3109 | 4-F-Ph | 2-$NH_2$-4-Pyr | 2-Allyl-4-(4,5-deH-Pip) |
| 1-3110 | 4-F-Ph | 2-$NH_2$-4-Pyr | 1-Me-2-Allyl-4-(4,5-deH-Pip) |
| 1-3111 | 4-F-Ph | 2-$NH_2$-4-Pyr | 1-Et-2-Allyl-4-(4,5-deH-Pip) |
| 1-3112 | 4-F-Ph | 2-$NH_2$-4-Pyr | 1-Pr-2-Allyl-4-(4,5-deH-Pip) |
| 1-3113 | 4-F-Ph | 2-$NH_2$-4-Pyr | 1-Bu-2-Allyl-4-(4,5-deH-Pip) |
| 1-3114 | 4-F-Ph | 2-$NH_2$-4-Pyr | 1-Phet-2-Allyl-4-(4,5-deH-Pip) |
| 1-3115 | 4-F-Ph | 2-$NH_2$-4-Pyr | 2-Bn-4-(4,5-deH-Pip) |
| 1-3116 | 4-F-Ph | 2-$NH_2$-4-Pyr | 1-Me-2-Bn-4-(4,5-deH-Pip) |
| 1-3117 | 4-F-Ph | 2-$NH_2$-4-Pyr | 1-Et-2-Bn-4-(4,5-deH-Pip) |
| 1-3118 | 4-F-Ph | 2-$NH_2$-4-Pyr | 1-Pr-2-Bn-4-(4,5-deH-Pip) |
| 1-3119 | 4-F-Ph | 2-$NH_2$-4-Pyr | 1-Bu-2-Bn-4-(4,5-deH-Pip) |
| 1-3120 | 4-F-Ph | 2-$NH_2$-4-Pyr | 1-Phet-2-Bn-4-(4,5-deH-Pip) |
| 1-3121 | 4-F-Ph | 2-$NH_2$-4-Pyr | 2-Phet-4-(4,5-deH-Pip) |
| 1-3122 | 4-F-Ph | 2-$NH_2$-4-Pyr | 1-Me-2-Phet-4-(4,5-deH-Pip) |
| 1-3123 | 4-F-Ph | 2-$NH_2$-4-Pyr | 1-Et-2-Phet-4-(4,5-deH-Pip) |
| 1-3124 | 4-F-Ph | 2-$NH_2$-4-Pyr | 1-Pr-2-Phet-4-(4,5-deH-Pip) |
| 1-3125 | 4-F-Ph | 2-$NH_2$-4-Pyr | 1-Bu-2-Phet-4-(4,5-deH-Pip) |
| 1-3126 | 4-F-Ph | 2-$NH_2$-4-Pyr | 1,2-diPhet-4-(4,5-deH-Pip) |
| 1-3127 | 4-F-Ph | 2-MeNH-4-Pyr | $H_2N-CH_2C(Me)_2CH_2$ |
| 1-3128 | 4-F-Ph | 2-MeNH-4-Pyr | $MeNH-CH_2C(Me)_2CH_2$ |
| 1-3129 | 4-F-Ph | 2-MeNH-4-Pyr | $EtNH-CH_2C(Me)_2CH_2$ |
| 1-3130 | 4-F-Ph | 2-MeNH-4-Pyr | $Me_2N-CH_2C(Me)_2CH_2$ |
| 1-3131 | 4-F-Ph | 2-MeNH-4-Pyr | 3-(3,4-deH-Pip) |
| 1-3132 | 4-F-Ph | 2-MeNH-4-Pyr | 1-Me-3-(3,4-deH-Pip) |
| 1-3133 | 4-F-Ph | 2-MeNH-4-Pyr | 1-Et-4-(3,4-deH-Pip) |
| 1-3134 | 4-F-Ph | 2-MeNH-4-Pyr | 1-Pr-4-(3,4-deH-Pip) |
| 1-3135 | 4-F-Ph | 2-MeNH-4-Pyr | 1-Pr-4-Pip |
| 1-3136 | 4-F-Ph | 2-MeNH-4-Pyr | 1-iPr-4-(3,4-deH-Pip) |
| 1-3137 | 4-F-Ph | 2-MeNH-4-Pyr | 1-iPr-4-Pip |
| 1-3138 | 4-F-Ph | 2-MeNH-4-Pyr | 1-Bu-4-(3,4-deH-Pip) |
| 1-3139 | 4-F-Ph | 2-MeNH-4-Pyr | 1-tBu-4-(3,4-deH-Pip) |
| 1-3140 | 4-F-Ph | 2-MeNH-4-Pyr | 1-Pn-4-(3,4-deH-Pip) |
| 1-3141 | 4-F-Ph | 2-MeNH-4-Pyr | 1-Hx-4-(3,4-deH-Pip) |

Table 1 (continued)

| Compound No. | $R^1$ | $R^2$ | $R^3$ |
|---|---|---|---|
| 1-3142 | 4-F-Ph | 2-MeNH-4-Pyr | 1-Hp-4-(3,4-deH-Pip) |
| 1-3143 | 4-F-Ph | 2-MeNH-4-Pyr | 1-Oc-4-(3,4-deH-Pip) |
| 1-3144 | 4-F-Ph | 2-MeNH-4-Pyr | 1-Nn-4-(3,4-deH-Pip) |
| 1-3145 | 4-F-Ph | 2-MeNH-4-Pyr | 1-cPr-4-(3,4-deH-Pip) |
| 1-3146 | 4-F-Ph | 2-MeNH-4-Pyr | 1-cPn-4-(3,4-deH-Pip) |
| 1-3147 | 4-F-Ph | 2-MeNH-4-Pyr | 1-cHx-4-(3,4-deH-Pip) |
| 1-3148 | 4-F-Ph | 2-MeNH-4-Pyr | 1-Bn-4-(3,4-deH-Pip) |
| 1-3149 | 4-F-Ph | 2-MeNH-4-Pyr | 1-Phet-4-(3,4-deH-Pip) |
| 1-3150 | 4-F-Ph | 2-MeNH-4-Pyr | 1-(3-Ph-Pr)-4-(3,4-deH-Pip) |
| 1-3151 | 4-F-Ph | 2-MeNH-4-Pyr | 1-(4-Ph-Bu)-4-(3,4-deH-Pip) |
| 1-3152 | 4-F-Ph | 2-MeNH-4-Pyr | 1-Allyl-4-(3,4-deH-Pip) |
| 1-3153 | 4-F-Ph | 2-MeNH-4-Pyr | 1-Propargyl-4-(3,4-deH-Pip) |
| 1-3154 | 4-F-Ph | 2-MeNH-4-Pyr | 2,2,6,6-tetraMe-4-(3,4-deH-Pip) |
| 1-3155 | 4-F-Ph | 2-MeNH-4-Pyr | 1,2,2,6,6-pentaMe-4-(3,4-deH-Pip) |
| 1-3156 | 4-F-Ph | 2-MeNH-4-Pyr | 1,2,2,6/6-pentaMe-4-Pip) |
| 1-3157 | 4-F-Ph | 2-MeNH-4-Pyr | 7-(1,2,3,5,6,8a-hexaH-Ind) |
| 1-3158 | 4-F-Ph | 2-MeNH-4-Pyr | 7-(1,2,3,5,8,8a-hexaH-Ind) |
| 1-3159 | 4-F-Ph | 2-MeNH-4-Pyr | 7-octaH-Ind |
| 1-3160 | 4-F-Ph | 2-MeNH-4-Pyr | 8-(1,3,4,6,7,9a-hexaH-2H-Qui) |
| 1-3161 | 4-F-Ph | 2-MeNH-4-Pyr | 8-(1,3,4,6,9,9a-hexaH-2H-Qui) |
| 1-3162 | 4-F-Ph | 2-MeNH-4-Pyr | 8-octaH-Qui |
| 1-3163 | 4-F-Ph | 2-MeNH-4-Pyr | 2,2-diMe-4-(3,4-deH-PiP) |
| 1-3164 | 4-F-Ph | 2-MeNH-4-Pyr | 1,2,2-triMe-4-(3,4-deH-PiP) |
| 1-3165 | 4-F-Ph | 2-MeNH-4-Pyr | 2,2-diMe-4-(4,5-deH-PiP) |
| 1-3166 | 4-F-Ph | 2-MeNH-4-Pyr | 1,2,2-triMe-4-(4,5-deH-PiP) |
| 1-3167 | 4-F-Ph | 2-MeNH-4-Pyr | 2,6-diMe-4-(3,4-deH-PiP) |
| 1-3168 | 4-F-Ph | 2-MeNH-4-Pyr | 1,2,6-triMe-4-(3,4-deH-PiP) |
| 1-3169 | 4-F-Ph | 2-MeNH-4-Pyr | 2-Me-4-(3,4-deH-Pip) |
| 1-3170 | 4-F-Ph | 2-MeNH-4-Pyr | 1,2-diMe-4-(3,4-deH-Pip) |
| 1-3171 | 4-F-Ph | 2-MeNH-4-Pyr | 1-Et-2-Me-4-(3,4-deH-Pip) |
| 1-3172 | 4-F-Ph | 2-MeNH-4-Pyr | 1-Pr-2-Me-4-(3,4-deH-Pip) |
| 1-3173 | 4-F-Ph | 2-MeNH-4-Pyr | 1-Bu-2-Me-4-(3,4-deH-Pip) |
| 1-3174 | 4-F-Ph | 2-MeNH-4-Pyr | 1-Phet-2-Me-4-(3,4-deH-Pip) |
| 1-3175 | 4-F-Ph | 2-MeNH-4-Pyr | 2-Et-4-(3,4-deH-Pip) |
| 1-3176 | 4-F-Ph | 2-MeNH-4-Pyr | 1-Me-2-Et-4-(3,4-deH-Pip) |
| 1-3177 | 4-F-Ph | 2-MeNH-4-Pyr | 1,2-diEt-4-(3,4-deH-Pip) |
| 1-3178 | 4-F-Ph | 2-MeNH-4-Pyr | 1-Pr-2-Et-4-(3,4-deH-Pip) |
| 1-3179 | 4-F-Ph | 2-MeNH-4-Pyr | 1-Bu-2-Et-4-(3,4-deH-Pip) |

Table 1 (continued)

| Compound No. | $R^1$ | $R^2$ | $R^3$ |
|---|---|---|---|
| 1-3180 | 4-F-Ph | 2-MeNH-4-Pyr | 1-Phet-2-Et-4-(3,4-deH-Pip) |
| 1-3181 | 4-F-Ph | 2-MeNH-4-Pyr | 2-Pr-4-(3,4-deH-Pip) |
| 1-3182 | 4-F-Ph | 2-MeNH-4-Pyr | 1-Me-2-Pr-4-(3,4-deH-Pip) |
| 1-3183 | 4-F-Ph | 2-MeNH-4-Pyr | 1-Et-2-Pr-4-(3,4-deH-Pip) |
| 1-3184 | 4-F-Ph | 2-MeNH-4-Pyr | 1,2-diPr-4-(3,4-deH-Pip) |
| 1-3185 | 4-F-Ph | 2-MeNH-4-Pyr | 1-Bu-2-Pr-4-(3,4-deH-Pip) |
| 1-3186 | 4-F-Ph | 2-MeNH-4-Pyr | 1-Phet-2-Pr-4-(3,4-deH-Pip) |
| 1-3187 | 4-F-Ph | 2-MeNH-4-Pyr | 2-Bu-4-(3,4-deH-Pip) |
| 1-3188 | 4-F-Ph | 2-MeNH-4-Pyr | 1-Me-2-Bu-4-(3,4-deH-Pip) |
| 1-3189 | 4-F-Ph | 2-MeNH-4-Pyr | 1-Et-2-Bu-4-(3,4-deH-Pip) |
| 1-3190 | 4-F-Ph | 2-MeNH-4-Pyr | 1-Pr-2-Bu-4-(3,4-deH-Pip) |
| 1-3191 | 4-F-Ph | 2-MeNH-4-Pyr | 1,2-diBu-4-(3,4-deH-Pip) |
| 1-3192 | 4-F-Ph | 2-MeNH-4-Pyr | 1-Phet-2-Bu-4-(3,4-deH-Pip) |
| 1-3193 | 4-F-Ph | 2-MeNH-4-Pyr | 2-Allyl-4-(3,4-deH-Pip) |
| 1-3194 | 4-F-Ph | 2-MeNH-4-Pyr | 1-Me-2-Allyl-4-(3,4-deH-Pip) |
| 1-3195 | 4-F-Ph | 2-MeNH-4-Pyr | 1-Et-2-Allyl-4-(3,4-deH-Pip) |
| 1-3196 | 4-F-Ph | 2-MeNH-4-Pyr | 1-Pr-2-Allyl-4-(3,4-deH-Pip) |
| 1-3197 | 4-F-Ph | 2-MeNH-4-Pyr | 1-Bu-2-Allyl-4-(3,4-deH-Pip) |
| 1-3198 | 4-F-Ph | 2-MeNH-4-Pyr | 1-Phet-2-Allyl-4-(3,4-deH-Pip) |
| 1-3199 | 4-F-Ph | 2-MeNH-4-Pyr | 2-Bn-4-(3,4-deH-Pip) |
| 1-3200 | 4-F-Ph | 2-MeNH-4-Pyr | 1-Me-2-Bn-4-(3,4-deH-Pip) |
| 1-3201 | 4-F-Ph | 2-MeNH-4-Pyr | 1-Et-2-Bn-4-(3,4-deH-Pip) |
| 1-3202 | 4-F-Ph | 2-MeNH-4-Pyr | 1-Pr-2-Bn-4-(3,4-deH-Pip) |
| 1-3203 | 4-F-Ph | 2-MeNH-4-Pyr | 1-Bu-2-Bn-4-(3,4-deH-Pip) |
| 1-3204 | 4-F-Ph | 2-MeNH-4-Pyr | 1-Phet-2-Bn-4-(3,4-deH-Pip) |
| 1-3205 | 4-F-Ph | 2-MeNH-4-Pyr | 2-Phet-4-(3,4-deH-Pip) |
| 1-3206 | 4-F-Ph | 2-MeNH-4-Pyr | 1-Me-2-Phet-4-(3,4-deH-Pip) |
| 1-3207 | 4-F-Ph | 2-MeNH-4-Pyr | 1-Et-2-Phet-4-(3,4-deH-Pip) |
| 1-3208 | 4-F-Ph | 2-MeNH-4-Pyr | 1-Pr-2-Phet-4-(3,4-deH-Pip) |
| 1-3209 | 4-F-Ph | 2-MeNH-4-Pyr | 1-Bu-2-Phet-4-(3,4-deH-Pip) |
| 1-3210 | 4-F-Ph | 2-MeNH-4-Pyr | 1,2-diPhet-4-(3,4-deH-Pip) |
| 1-3211 | 4-F-Ph | 2-MeNH-4-Pyr | 2-Me-4-(9,5-deH-Pip) |
| 1-3212 | 4-F-Ph | 2-MeNH-4-Pyr | 1,2-diMe-4-(4,5-deH-Pip) |
| 1-3213 | 4-F-Ph | 2-MeNH-4-Pyr | 1-Et-2-Me-4-(4,5-deH-Pip) |
| 1-3214 | 4-F-Ph | 2-MeNH-4-Pyr | 1-Pr-2-Me-4-(4,5-deH-Pip) |
| 1-3215 | 4-F-Ph | 2-MeNH-4-Pyr | 1-Bu-2-Me-4-(4,5-deH-Pip) |
| 1-3216 | 4-F-Ph | 2-MeNH-4-Pyr | 1-Phet-2-Me-4-(4,5-deH-Pip) |
| 1-3217 | 4-F-Ph | 2-MeNH-4-Pyr | 2-Et-4-(4,5-deH-Pip) |

Table 1 (continued)

| Compound No. | $R^1$ | $R^2$ | $R^3$ |
|---|---|---|---|
| 1-3218 | 4-F-Ph | 2-MeNH-4-Pyr | 1-Me-2-Et-4-(4,5-deH-Pip) |
| 1-3219 | 4-F-Ph | 2-MeNH-4-Pyr | 1,2-diEt-4-(4,5-deH-Pip) |
| 1-3220 | 4-F-Ph | 2-MeNH-4-Pyr | 1-Pr-2-Et-4-(4,5-deH-Pip) |
| 1-3221 | 4-F-Ph | 2-MeNH-4-Pyr | 1-Bu-2-Et-4-(4,5-deH-Pip) |
| 1-3222 | 4-F-Ph | 2-MeNH-4-Pyr | 1-Phet-2-Et-4-(4,5-deH-Pip) |
| 1-3223 | 4-F-Ph | 2-MeNH-4-Pyr | 2-Pr-4-(4,5-deH-Pip) |
| 1-3224 | 4-F-Ph | 2-MeNH-4-Pyr | 1-Me-2-Pr-4-(4,5-deH-Pip) |
| 1-3225 | 4-F-Ph | 2-MeNH-4-Pyr | 1-Et-2-Pr-4-(4,5-deH-Pip) |
| 1-3226 | 4-F-Ph | 2-MeNH-4-Pyr | 1,2-diPr-4-(4,5-deH-Pip) |
| 1-3227 | 4-F-Ph | 2-MeNH-4-Pyr | 1-Bu-2-Pr-4-(4,5-deH-Pip) |
| 1-3228 | 4-F-Ph | 2-MeNH-4-Pyr | 1-Phet-2-Pr-4-(4,5-deH-Pip) |
| 1-3229 | 4-F-Ph | 2-MeNH-4-Pyr | 2-Bu-4-(4,5-deH-Pip) |
| 1-3230 | 4-F-Ph | 2-MeNH-4-Pyr | 1-Me-2-Bu-4-(4,5-deH-Pip) |
| 1-3231 | 4-F-Ph | 2-MeNH-4-Pyr | 1-Et-2-Bu-4-(4,5-deH-Pip) |
| 1-3232 | 4-F-Ph | 2-MeNH-4-Pyr | 1-Pr-2-Bu-4-(4,5-deH-Pip) |
| 1-3233 | 4-F-Ph | 2-MeNH-4-Pyr | 1,2-diBu-4-(4,5-deH-Pip) |
| 1-3234 | 4-F-Ph | 2-MeNH-4-Pyr | 1-Phet-2-Bu-4-(4,5-deH-Pip) |
| 1-3235 | 4-F-Ph | 2-MeNH-4-Pyr | 2-Allyl-4-(4,5-deH-Pip) |
| 1-3236 | 4-F-Ph | 2-MeNH-4-Pyr | 1-Me-2-Allyl-4-(4,5-deH-Pip) |
| 1-3237 | 4-F-Ph | 2-MeNH-4-Pyr | 1-Et-2-Allyl-4-(4,5-deH-Pip) |
| 1-3238 | 4-F-Ph | 2-MeNH-4-Pyr | 1-Pr-2-Allyl-4-(4,5-deH-Pip) |
| 1-3239 | 4-F-Ph | 2-MeNH-4-Pyr | 1-Bu-2-Allyl-4-(4,5-deH-Pip) |
| 1-3240 | 4-F-Ph | 2-MeNH-4-Pyr | 1-Phet-2-Allyl-4-(4,5-deH-Pip) |
| 1-3241 | 4-F-Ph | 2-MeNH-4-Pyr | 2-Bn-4-(4,5-deH-Pip) |
| 1-3242 | 4-F-Ph | 2-MeNH-4-Pyr | 1-Me-2-Bn-4-(4,5-deH-Pip) |
| 1-3243 | 4-F-Ph | 2-MeNH-4-Pyr | 1-Et-2-Bn-4-(4,5-deH-Pip) |
| 1-3244 | 4-F-Ph | 2-MeNH-4-Pyr | 1-Pr-2-Bn-4-(4,5-deH-Pip) |
| 1-3245 | 4-F-Ph | 2-MeNH-4-Pyr | 1-Bu-2-Bn-4-(4,5-deH-Pip) |
| 1-3246 | 4-F-Ph | 2-MeNH-4-Pyr | 1-Phet-2-Bn-4-(4,5-deH-Pip) |
| 1-3247 | 4-F-Ph | 2-MeNH-4-Pyr | 2-Phet-4-(4,5-deH-Pip) |
| 1-3248 | 4-F-Ph | 2-MeNH-4-Pyr | 1-Me-2-Phet-4-(4,5-deH-Pip) |
| 1-3249 | 4-F-Ph | 2-MeNH-4-Pyr | 1-Et-2-Phet-4-(4,5-deH-Pip) |
| 1-3250 | 4-F-Ph | 2-MeNH-4-Pyr | 1-Pr-2-Phet-4-(4,5-deH-Pip) |
| 1-3251 | 4-F-Ph | 2-MeNH-4-Pyr | 1-Bu-2-Phet-4-(4,5-deH-Pip) |
| 1-3252 | 4-F-Ph | 2-MeNH-4-Pyr | 1,2-diPhet-4-(4,5-deH-Pip) |
| 1-3253 | Ph | 4-Pyr | 7-(1,2,3,5,6,8a-hexaH-Ind) |
| 1-3254 | Ph | 4-Pyr | 7-(1,2,3,5,8,8a-hexaH-Ind) |
| 1-3255 | 3-F-Ph | 4-Pyr | 7-(1,2,3,5,6,8a-hexaH-Ind) |

Table 1 (continued)

| Compound No. | $R^1$ | $R^2$ | $R^3$ |
|---|---|---|---|
| 1-3256 | 3-F-Ph | 4-Pyr | 7-(1,2,3,5,8,8a-hexaH-Ind) |
| 1-3257 | 3-Cl-Ph | 4-Pyr | 7-(1,2,3,5,6,8a-hexaH-Ind) |
| 1-3258 | 3-Cl-Ph | 4-Pyr | 7-(1,2,3,5,8,8a-hexaH-Ind) |
| 1-3259 | 3,4-diF-Ph | 4-Pyr | 7-(1,2,3,5,6,8a-hexaH-Ind) |
| 1-3260 | 3,4-diF-Ph | 4-Pyr | 7-(1,2,3,5,8,8a-hexaH-Ind) |
| 1-3261 | 3-$CF_3$-Ph | 4-Pyr | 7-(1,2,3,5,6,8a-hexaH-Ind) |
| 1-3262 | 3-$CF_3$-Ph | 4-Pyr | 7-(1,2,3,5,8,8a-hexaH-Ind) |
| 1-3263 | 4-F-Ph | 4-Pyr | 2-Pyrd-CH=CH- |
| 1-3264 | 4-F-Ph | 4-Pyr | 1-Me-2-Pyrd-CH=CH- |
| 1-3265 | 4-F-Ph | 4-Pyr | 1-Et-2-Pyrd-CH=CH- |
| 1-3266 | 4-F-Ph | 4-Pyr | 1-Pr-2-Pyrd-CH=CH- |
| 1-3267 | 4-F-Ph | 4-Pyr | 2-Pip-CH=CH- |
| 1-3268 | 4-F-Ph | 4-Pyr | 1-Me-2-Pip-CH=CH- |
| 1-3269 | 4-F-Ph | 4-Pyr | 1-Et-2-Pip-CH=CH- |
| 1-3270 | 4-F-Ph | 4-Pyr | 1-Pr-2-Pip-CH=CH- |
| 1-3271 | Ph | 4-Pyr | 2-Pyrd-CH=CH- |
| 1-3272 | Ph | 4-Pyr | 1-Me-2-Pyrd-CH=CH- |
| 1-3273 | Ph | 4-Pyr | 1-Et-2-Pyrd-CH=CH- |
| 1-3274 | Ph | 4-Pyr | 1-Pr-2-Pyrd-CH=CH- |
| 1-3275 | Ph | 4-Pyr | 2-Pip-CH=CH- |
| 1-3276 | Ph | 4-Pyr | 1-Me-2-Pip-CH=CH- |
| 1-3277 | Ph | 4-Pyr | 1-Et-2-Pip-CH=CH- |
| 1-3278 | Ph | 4-Pyr | 1-Pr-2-Pip-CH=CH- |
| 1-3279 | 3-F-Ph | 4-Pyr | 2-Pyrd-CH=CH- |
| 1-3280 | 3-F-Ph | 4-Pyr | 1-Me-2-Pyrd-CH=CH- |
| 1-3281 | 3-F-Ph | 4-Pyr | 1-Et-2-Pyrd-CH=CH- |
| 1-3282 | 3-F-Ph | 4-Pyr | 1-Pr-2-Pyrd-CH=CH- |
| 1-3283 | 3-F-Ph | 4-Pyr | 2-Pip-CH=CH- |
| 1-3284 | 3-F-Ph | 4-Pyr | 1-Me-2-Pip-CH=CH- |
| 1-3285 | 3-F-Ph | 4-Pyr | 1-Et-2-Pip-CH=CH- |
| 1-3286 | 3-F-Ph | 4-Pyr | 1-Pr-2-Pip-CH=CH- |
| 1-3287 | 3-Cl-Ph | 4-Pyr | 2-Pyrd-CH=CH- |
| 1-3288 | 3-Cl-Ph | 4-Pyr | 1-Me-2-Pyrd-CH=CH- |
| 1-3289 | 3-Cl-Ph | 4-Pyr | 1-Et-2-Pyrd-CH=CH- |
| 1-3290 | 3-Cl-Ph | 4-Pyr | 1-Pr-2-Pyrd-CH=CH- |
| 1-3291 | 3-Cl-Ph | 4-Pyr | 2-Pip-CH=CH- |
| 1-3292 | 3-Cl-Ph | 4-Pyr | 1-Me-2-Pip-CH=CH- |
| 1-3293 | 3-Cl-Ph | 4-Pyr | 1-Et-2-Pip-CH=CH- |

Table 1 (continued)

| Compound No. | $R^1$ | $R^2$ | $R^3$ |
|---|---|---|---|
| 1-3294 | 3-Cl-Ph | 4-Pyr | 1-Pr-2-Pip-CH=CH- |
| 1-3295 | 3,4-diF-Ph | 4-Pyr | 2-Pyrd-CH=CH- |
| 1-3296 | 3,4-diF-Ph | 4-Pyr | 1-Me-2-Pyrd-CH=CH- |
| 1-3297 | 3,4-diF-Ph | 4-Pyr | 1-Et-2-Pyrd-CH=CH- |
| 1-3298 | 3,4-diF-Ph | 4-Pyr | 1-Pr-2-Pyrd-CH=CH- |
| 1-3299 | 3,4-diF-Ph | 4-Pyr | 2-Pip-CH=CH- |
| 1-3300 | 3,4-diF-Ph | 4-Pyr | 1-Me-2-Pip-CH=CH- |
| 1-3301 | 3,4-diF-Ph | 4-Pyr | 1-Et-2-Pip-CH=CH- |
| 1-3302 | 3,4-diF-Ph | 4-Pyr | 1-Pr-2-Pip-CH=CH- |
| 1-3303 | 3-$CF_3$-Ph | 4-Pyr | 2-Pyrd-CH=CH- |
| 1-3304 | 3-$CF_3$-Ph | 4-Pyr | 1-Me-2-Pyrd-CH=CH- |
| 1-3305 | 3-$CF_3$-Ph | 4-Pyr | 1-Et-2-Pyrd-CH=CH- |
| 1-3306 | 3-$CF_3$-Ph | 4-Pyr | 1-Pr-2-Pyrd-CH=CH- |
| 1-3307 | 3-$CF_3$-Ph | 4-Pyr | 2-Pip-CH=CH- |
| 1-3308 | 3-$CF_3$-Ph | 4-Pyr | 1-Me-2-Pip-CH=CH- |
| 1-3309 | 3-$CF_3$-Ph | 4-Pyr | 1-Et-2-Pip-CH=CH- |
| 1-3310 | 3-$CF_3$-Ph | 4-Pyr | 1-Pr-2-Pip-CH=CH- |

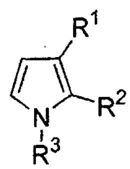

(I-3)

Table 2

| Compound No. | $R^1$ | $R^2$ | $R^3$ |
|---|---|---|---|
| 2-1 | Ph | 4-Pyr | $H_2N-CH_2$ |
| 2-2 | Ph | 4-Pyr | $H_2N-(CH_2)_2$ |
| 2-3 | Ph | 4-Pyr | $H_2N-(CH_2)_3$ |
| 2-4 | Ph | 4-Pyr | $H_2N-(CH_2)_4$ |
| 2-5 | Ph | 4-Pyr | $MeNH-CH_2$ |
| 2-6 | Ph | 4-Pyr | $MeNH-(CH_2)_2$ |
| 2-7 | Ph | 4-Pyr | $MeNH-(CH_2)_3$ |
| 2-8 | Ph | 4-Pyr | $MeNH-(CH_2)_4$ |
| 2-9 | Ph | 4-Pyr | $EtNH-CH_2$ |
| 2-10 | Ph | 4-Pyr | $EtNH-(CH_2)_2$ |

Table 2 (continued)

| Compound No. | $R^1$ | $R^2$ | $R^3$ |
|---|---|---|---|
| 2-11 | Ph | 4-Pyr | EtNH-$(CH_2)_3$ |
| 2-12 | Ph | 4-Pyr | EtNH-$(CH_2)_4$ |
| 2-13 | Ph | 4-Pyr | $Me_2N$-$CH_2$ |
| 2-14 | Ph | 4-Pyr | $Me_2N$-$(CH_2)_2$ |
| 2-15 | Ph | 4-Pyr | $Me_2N$-$(CH_2)_3$ |
| 2-16 | Ph | 4-Pyr | $Me_2N$-$(CH_2)_4$ |
| 2-17 | Ph | 4-Pyr | 1-Azt-$CH_2$ |
| 2-18 | Ph | 4-Pyr | 1-Azt-$(CH_2)_2$ |
| 2-19 | Ph | 4-Pyr | 1-Azt-$(CH_2)_3$ |
| 2-20 | Ph | 4-Pyr | 1-Azt-$(Ch_2)_4$ |
| 2-21 | Ph | 4-Pyr | 1-Pyrd-$CH_2$ |
| 2-22 | Ph | 4-Pyr | 1-Pyrd-$(CH_2)_2$ |
| 2-23 | Ph | 4-Pyr | 1-Pyrd- $(CH_2)_3$ |
| 2-24 | Ph | 4-Pyr | 1-Pyrd-$(CH_2)_4$ |
| 2-25 | Ph | 4-Pyr | 1-Pip-$CH_2$ |
| 2-26 | Ph | 4-Pyr | 1-Pip-$(CH_2)_2$ |
| 2-27 | Ph | 4-Pyr | 1-Pip-$(CH_2)_3$ |
| 2-28 | Ph | 4-Pyr | 1-Pip-$(CH_2)_4$ |
| 2-29 | Ph | 4-Pyr | 1-Mor-$CH_2$ |
| 2-30 | Ph | 4-Pyr | 1-Mor-$(CH_2)_2$ |
| 2-31 | Ph | 4-Pyr | 1-Mor-$(CH_2)_3$ |
| 2-32 | Ph | 4-Pyr | 1-Mor-$(CH_2)_4$ |
| 2-33 | Ph | 4-Pyr | 1-Tmor-$CH_2$ |
| 2-34 | Ph | 4-Pyr | 1-Tmor-$(CH_2)_2$ |
| 2-35 | Ph | 4-Pyr | 1-Tmor-$(CH_2)_3$ |
| 2-36 | Ph | 4-Pyr | 1-Tmor-$(CH_2)_4$ |
| 2-37 | Ph | 4-Pyr | 1-Piz-$CH_2$ |
| 2-38 | Ph | 4-Pyr | 1-Piz-$(CH_2)_2$ |
| 2-39 | Ph | 4-Pyr | 1-Piz-$(CH_2)_3$ |
| 2-40 | Ph | 4-Pyr | 4-Me-1-Piz-$(CH_2)_3$ |
| 2-41 | Ph | 4-Pyr | 1-Piz-$(CH_2)_4$ |
| 2-42 | Ph | 4-Pyr | 3-Azt |
| 2-43 | Ph | 4-Pyr | 1-Me-3-Azt |
| 2-44 | Ph | 4-Pyr | 1-Bn-3-Azt |
| 2-45 | Ph | 4-Pyr | 3-Pyrd |
| 2-46 | Ph | 4-Pyr | 1-Me-3-Pyrd |
| 2-47 | Ph | 4-Pyr | 3-Pip |
| 2-48 | Ph | 4-Pyr | 4-Pip |

Table 2 (continued)

| Compound No. | $R^1$ | $R^2$ | $R^3$ |
|---|---|---|---|
| 2-49 | Ph | 4-Pyr | 4-(3,4-deH-Pip) |
| 2-50 | Ph | 4-Pyr | 1-Me-4-Pip |
| 2-51 | Ph | 4-Pyr | 1-Me-4-(3,4-deH-Pip) |
| 2-52 | Ph | 4-Pyr | 1-Et-4-Pip |
| 2-53 | Ph | 4-Pyr | 1-Bn-4-Pip |
| 2-54 | Ph | 4-Pyr | 3-Hpip |
| 2-55 | Ph | 4-Pyr | 1-Me-3-Hpip |
| 2-56 | Ph | 4-Pyr | 4-Hpip |
| 2-57 | Ph | 4-Pyr | 1-Me-4-Hpip |
| 2-58 | Ph | 4-Pyr | 2-Mor |
| 2-59 | Ph | 4-Pyr | 1-Me-2-Mor |
| 2-60 | Ph | 4-Pyr | 2-Tmor |
| 2-61 | Ph | 4-Pyr | 1-Me-2-Tmor |
| 2-62 | Ph | 4-Pyr | 1-Piz |
| 2-63 | Ph | 4-Pyr | 4-Me-1-Piz |
| 2-64 | Ph | 4-Pyr | 2-Piz |
| 2-65 | Ph | 4-Pyr | 4-Pyr |
| 2-66 | Ph | 4-Pyr | 3-Pyr |
| 2-67 | Ph | 4-Pyr | 2-Pyr |
| 2-68 | Ph | 4-Pyr | 4-Pym |
| 2-69 | Ph | 4-Pyr | 5-Pym |
| 2-70 | Ph | 4-Pyr | 2-Pym |
| 2-71 | Ph | 4-Pyr | 3-Azt-$CH_2$ |
| 2-72 | Ph | 4-Pyr | 1-Me-3-Azt-$CH_2$ |
| 2-73 | Ph | 4-Pyr | 3-Pyrd-$CH_2$ |
| 2-74 | Ph | 4-Pyr | 1-Me-3-Pyrd-$CH_2$ |
| 2-75 | Ph | 4-Pyr | 4-Pip-$CH_2$ |
| 2-76 | Ph | 4-Pyr | 1-Me-4-Pip-$CH_2$ |
| 2-77 | Ph | 4-Pyr | 3-Hpip-$CH_2$ |
| 2-78 | Ph | 4-Pyr | 1-Me-3-Hpip-$CH_2$ |
| 2-79 | Ph | 4-Pyr | 4-Hpip-$CH_2$ |
| 2-80 | Ph | 4-Pyr | 1-Me-4-Hpip-$CH_2$ |
| 2-81 | Ph | 4-Pyr | 2-Mor-$CH_2$ |
| 2-82 | Ph | 4-Pyr | 1-Me-2-Mor-$CH_2$ |
| 2-83 | Ph | 4-Pyr | 2-Tmor-$CH_2$ |
| 2-84 | Ph | 4-Pyr | 1-Me-2-Tmor-$CH_2$ |
| 2-85 | Ph | 4-Pyr | 1-Piz-$CH_2$ |
| 2-86 | Ph | 4-Pyr | 4-Me-1-Piz-$CH_2$ |

Table 2 (continued)

| Compound No. | $R^1$ | $R^2$ | $R^3$ |
|---|---|---|---|
| 2-87 | Ph | 4-Pyr | 2-Piz-$CH_2$ |
| 2-88 | Ph | 4-Pyr | 4-Pyr-$CH_2$ |
| 2-89 | Ph | 4-Pyr | 3-Pyr-$CH_2$ |
| 2-90 | Ph | 4-Pyr | 2-Pyr-$CH_2$ |
| 2-91 | Ph | 4-Pyr | 4-Pym-$CH_2$ |
| 2-92 | Ph | 4-Pyr | 5-Pym-$CH_2$ |
| 2-93 | Ph | 4-Pyr | 2-Pym-$CH_2$ |
| 2-94 | Ph | 4-Pyr | $H_2N$-$CH_2$CH=CH |
| 2-95 | 4-F-Ph | 4-Pyr | $H_2N$-$CH_2$ |
| 2-96 | 4-F-Ph | 4-Pyr | $H_2N$-$(CH_2)_2$ |
| 2-97 | 4-F-Ph | 4-Pyr | $H_2N$-$(CH_2)_3$ |
| 2-98 | 4-F-Ph | 4-Pyr | $H_2N$-$(CH_2)_4$ |
| 2-99 | 4-F-Ph | 4-Pyr | MeNH-$CH_2$ |
| 2-100 | 4-F-Ph | 4-Pyr | MeNH-$(CH_2)_2$ |
| 2-101 | 4-F-Ph | 4-Pyr | MeNH-$(CH_2)_3$ |
| 2-102 | 4-F-Ph | 4-Pyr | MeNH-$(CH_2)_4$ |
| 2-103 | 4-F-Ph | 4-Pyr | EtNH-$CH_2$ |
| 2-104 | 4-F-Ph | 4-Pyr | EtNH-$(CH_2)_2$ |
| 2-105 | 4-F-Ph | 4-Pyr | EtNH-$(CH_2)_3$ |
| 2-106 | 4-F-Ph | 4-Pyr | EtNH-$(CH_2)_4$ |
| 2-107 | 4-F-Ph | 4-Pyr | $Me_2N$-$CH_2$ |
| 2-108 | 4-F-Ph | 4-Pyr | $Me_2N$-$(CH_2)_2$ |
| 2-109 | 4-F-Ph | 4-Pyr | $Me_2N$-$(CH_2)_3$ |
| 2-110 | 4-F-Ph | 4-Pyr | $Me_2N$-$(CH_2)_4$ |
| 2-111 | 4-F-Ph | 4-Pyr | 1-Azt-$CH_2$ |
| 2-112 | 4-F-Ph | 4-Pyr | 1-Azt-$(CH_2)_2$ |
| 2-113 | 4-F-Ph | 4-Pyr | 1-Azt-$(CH_2)_3$ |
| 2-114 | 4-F-Ph | 4-Pyr | 1-Azt-$(CH_2)_4$ |
| 2-115 | 4-F-Ph | 4-Pyr | 1-Pyrd-$CH_2$ |
| 2-116 | 4-F-Ph | 4-Pyr | 1-Pyrd-$(CH_2)_2$ |
| 2-117 | 4-F-Ph | 4-Pyr | 1-Pyrd-$(CH_2)_3$ |
| 2-118 | 4-F-Ph | 4-Pyr | 1-Pyrd-$(CH_2)_4$ |
| 2-119 | 4-F-Ph | 4-Pyr | 1-Pip-$CH_2$ |
| 2-120 | 4-F-Ph | 4-Pyr | 1-Pip-$(CH_2)_2$ |
| 2-121 | 4-F-Ph | 4-Pyr | 1-Pip-$(CH_2)_3$ |
| 2-122 | 4-F-Ph | 4-Pyr | 1-Pip-$(CH_2)_4$ |
| 2-123 | 4-F-Ph | 4-Pyr | 1-Mor-$CH_2$ |
| 2-124 | 4-F-Ph | 4-Pyr | 1-Mor-$(CH_2)_2$ |

Table 2 (continued)

| Compound No. | $R^1$ | $R^2$ | $R^3$ |
|---|---|---|---|
| 2-125 | 4-F-Ph | 4-Pyr | 1-Mor-$(CH_2)_3$ |
| 2-126 | 4-F-Ph | 4-Pyr | 1-Mor-$(CH_2)_4$ |
| 2-127 | 4-F-Ph | 4-Pyr | 1-Tmor-$CH_2$ |
| 2-128 | 4-F-Ph | 4-Pyr | 1-Tmor-$(CH_2)_2$ |
| 2-129 | 4-F-Ph | 4-Pyr | 1-Tmor-$(CH_2)_3$ |
| 2-130 | 4-F-Ph | 4-Pyr | 1-Tmor-$(CH_2)_4$ |
| 2-131 | 4-F-Ph | 4-Pyr | 1-Piz-$CH_2$ |
| 2-132 | 4-F-Ph | 4-Pyr | 1-Piz-$(CH_2)_2$ |
| 2-133 | 4-F-Ph | 4-Pyr | 1-Piz-$(CH_2)_3$ |
| 2-134 | 4-F-Ph | 4-Pyr | 4-Me-1-Piz-$(CH_2)_3$ |
| 2-135 | 4-F-Ph | 4-Pyr | 4-Bn-1-Piz-$(CH_2)_3$ |
| 2-136 | 4-F-Ph | 4-Pyr | 3-Azt |
| 2-137 | 4-F-Ph | 4-Pyr | 1-Me-3-Azt |
| 2-138 | 4-F-Ph | 4-Pyr | 1-Bn-3-Azt |
| 2-139 | 4-F-Ph | 4-Pyr | 3-Pyrd |
| 2-140 | 4-F-Ph | 4-Pyr | 1-Me-3-Pyrd |
| 2-141 | 4-F-Ph | 4-Pyr | 3-Pip |
| 2-142 | 4-F-Ph | 4-Pyr | 4-Pip |
| 2-143 | 4-F-Ph | 4-Pyr | 4-(3,4-deH-Pip) |
| 2-144 | 4-F-Ph | 4-Pyr | 1-Me-4-Pip |
| 2-145 | 4-F-Ph | 4-Pyr | 1-Me-4-(3,4-deH-Pip) |
| 2-146 | 4-F-Ph | 4-Pyr | 1-Et-4-Pip |
| 2-147 | 4-F-Ph | 4-Pyr | 1-Bn-4-Pip |
| 2-148 | 4-F-Ph | 4-Pyr | 3-Hpip |
| 2-149 | 4-F-Ph | 4-Pyr | 1-Me-3-Hpip |
| 2-150 | 4-F-Ph | 4-Pyr | 4-Hpip |
| 2-151 | 4-F-Ph | 4-Pyr | 1-Me-4-Hpip |
| 2-152 | 4-F-Ph | 4-Pyr | 2-Mor |
| 2-153 | 4-F-Ph | 4-Pyr | 1-Me-2-Mor |
| 2-154 | 4-F-Ph | 4-Pyr | 2-Tmor |
| 2-155 | 4-F-Ph | 4-Pyr | 1-Me-2-Tmor |
| 2-156 | 4-F-Ph | 4-Pyr | 1-Piz |
| 2-157 | 4-F-Ph | 4-Pyr | 4-Me-1-Piz |
| 2-158 | 4-F-Ph | 4-Pyr | 2-Piz |
| 2-159 | 4-F-Ph | 4-Pyr | 4-Pyr |
| 2-160 | 4-F-Ph | 4-Pyr | 3-Pyr |
| 2-161 | 4-F-Ph | 4-Pyr | 2-Pyr |
| 2-162 | 4-F-Ph | 4-Pyr | 4-Pym |

Table 2 (continued)

| Compound No. | $R^1$ | $R^2$ | $R^3$ |
|---|---|---|---|
| 2-163 | 4-F-Ph | 4-Pyr | 5-Pym |
| 2-164 | 4-F-Ph | 4-Pyr | 2-Pym |
| 2-165 | 4-F-Ph | 4-Pyr | 3-Azt-$CH_2$ |
| 2-166 | 4-F-Ph | 4-Pyr | 1-Me-3-Azt-$CH_2$ |
| 2-167 | 4-F-Ph | 4-Pyr | 3-Pyrd-$CH_2$ |
| 2-168 | 4-F-Ph | 4-Pyr | 1-Me-3-Pyrd-$CH_2$ |
| 2-169 | 4-F-Ph | 4-Pyr | 4-Pip-$CH_2$ |
| 2-170 | 4-F-Ph | 4-Pyr | 1-Me-4-Pip-$CH_2$ |
| 2-171 | 4-F-Ph | 4-Pyr | 3-Hpip-$CH_2$ |
| 2-172 | 4-F-Ph | 4-Pyr | 1-Me-3-Hpip-$CH_2$ |
| 2-173 | 4-F-Ph | 4-Pyr | 4-Hpip-$CH_2$ |
| 2-174 | 4-F-Ph | 4-Pyr | 1-Me-4-Hpip-$CH_2$ |
| 2-175 | 4-F-Ph | 4-Pyr | 2-Mor-$CH_2$ |
| 2-176 | 4-F-Ph | 4-Pyr | 1-Me-2-Mor-$CH_2$ |
| 2-177 | 4-F-Ph | 4-Pyr | 2-Tmor-$CH_2$ |
| 2-178 | 4-F-Ph | 4-Pyr | 1-Me-2-Tmor-$CH_2$ |
| 2-179 | 4-F-Ph | 4-Pyr | 1-Piz-$CH_2$ |
| 2-180 | 4-F-Ph | 4-Pyr | 4-Me-1-Piz-$CH_2$ |
| 2-181 | 4-F-Ph | 4-Pyr | 2-Piz-$CH_2$ |
| 2-182 | 4-F-Ph | 4-Pyr | 4-Pyr-$CH_2$ |
| 2-183 | 4-F-Ph | 4-Pyr | 3-Pyr-$CH_2$ |
| 2-184 | 4-F-Ph | 4-Pyr | 2-Pyr-$CH_2$ |
| 2-185 | 4-F-Ph | 4-Pyr | 4-Pym-$CH_2$ |
| 2-186 | 4-F-Ph | 4-Pyr | 5-Pym-$CH_2$ |
| 2-187 | 4-F-Ph | 4-Pyr | 2-Pym-$CH_2$ |
| 2-188 | 3-F-Ph | 4-Pyr | $H_2N$-$CH_2$ |
| 2-189 | 3-F-Ph | 4-Pyr | $H_2N$-$(CH_2)_2$ |
| 2-190 | 3-F-Ph | 4-Pyr | $H_2N$-$(CH_2)_3$ |
| 2-191 | 3-F-Ph | 4-Pyr | $H_2N$-$(CH_2)_4$ |
| 2-192 | 3-F-Ph | 4-Pyr | MeNH-$CH_2$ |
| 2-193 | 3-F-Ph | 4-Pyr | MeNH-$(CH_2)_2$ |
| 2-194 | 3-F-Ph | 4-Pyr | MeNH-$(CH_2)_3$ |
| 2-195 | 3-F-Ph | 4-Pyr | MeNH-$(CH_2)_4$ |
| 2-196 | 3-F-Ph | 4-Pyr | EtNH-$CH_2$ |
| 2-197 | 3-F-Ph | 4-Pyr | EtNH-$(CH_2)_2$ |
| 2-198 | 3-F-Ph | 4-Pyr | EtNH-$(CH_2)_3$ |
| 2-199 | 3-F-Ph | 4-Pyr | EtNH-$(CH_2)_4$ |
| 2-200 | 3-F-Ph | 4-Pyr | $Me_2N$-$CH_2$ |

Table 2 (continued)

| Compound No. | $R^1$ | $R^2$ | $R^3$ |
|---|---|---|---|
| 2-201 | 3-F-Ph | 4-Pyr | $Me_2N-(CH_2)_2$ |
| 2-202 | 3-F-Ph | 4-Pyr | $Me_2N-(CH_2)_3$ |
| 2-203 | 3-F-Ph | 4-Pyr | $Me_2N-(CH_2)_4$ |
| 2-204 | 3-F-Ph | 4-Pyr | $1-Azt-CH_2$ |
| 2-205 | 3-F-Ph | 4-Pyr | $1-Azt-(CH_2)_2$ |
| 2-206 | 3-F-Ph | 4-Pyr | $1-Azt-(CH_2)_3$ |
| 2-207 | 3-F-Ph | 4-Pyr | $1-Azt-(CH_2)_4$ |
| 2-208 | 3-F-Ph | 4-Pyr | $1-pyrd-CH_2$ |
| 2-209 | 3-F-Ph | 4-Pyr | $1-Pyrd-(CH_2)_2$ |
| 2-210 | 3-F-Ph | 4-Pyr | $1-Pyrd-(CH_2)_3$ |
| 2-211 | 3-F-Ph | 4-Pyr | $1-Pyrd-(CH_2)_4$ |
| 2-212 | 3-F-Ph | 4-Pyr | $1-Pip-CH_2$ |
| 2-213 | 3-F-Ph | 4-Pyr | $1-Pip-(CH_2)_2$ |
| 2-214 | 3-F-Ph | 4-Pyr | $1-Pip-(CH_2)_3$ |
| 2-215 | 3-F-Ph | 4-Pyr | $1-Pip-(CH_2)_4$ |
| 2-216 | 3-F-Ph | 4-Pyr | $1-Mor-CH_2$ |
| 2-217 | 3-F-Ph | 4-Pyr | $1-Mor-(CH_2)_2$ |
| 2-218 | 3-F-Ph | 4-Pyr | $1-Mor-(CH_2)_3$ |
| 2-219 | 3-F-Ph | 4-Pyr | $1-Mor-(CH_2)_4$ |
| 2-220 | 3-F-Ph | 4-Pyr | $1-Tmor-CH_2$ |
| 2-221 | 3-F-Ph | 4-Pyr | $1-Tmor-(CH_2)_2$ |
| 2-222 | 3-F-Ph | 4-Pyr | $1-Tmor-(CH_2)_3$ |
| 2-223 | 3-F-Ph | 4-Pyr | $1-Tmor-(CH_2)_4$ |
| 2-224 | 3-F-Ph | 4-Pyr | $1-Piz-CH_2$ |
| 2-225 | 3-F-Ph | 4-Pyr | $1-Piz-(CH_2)_2$ |
| 2-226 | 3-F-Ph | 4-Pyr | $1-Piz-(CH_2)_3$ |
| 2-227 | 3-F-Ph | 4-Pyr | $4-Me-1-Piz-(CH_2)_3$ |
| 2-228 | 3-F-Ph | 4-Pyr | $1-Piz-(CH_2)_4$ |
| 2-229 | 3-F-Ph | 4-Pyr | 3-Azt |
| 2-230 | 3-F-Ph | 4-Pyr | 1-Me-3-Azt |
| 2-231 | 3-F-Ph | 4-Pyr | 1-Bn-3-Azt |
| 2-232 | 3-F-Ph | 4-Pyr | 3-Pyrd |
| 2-233 | 3-F-Ph | 4-Pyr | 1-Me-3-Pyrd |
| 2-234 | 3-F-Ph | 4-Pyr | 3-Pip |
| 2-235 | 3-F-Ph | 4-Pyr | 4-Pip |
| 2-236 | 3-F-Ph | 4-Pyr | 4-(3,4-deH-Pip) |
| 2-237 | 3-F-Ph | 4-Pyr | 1-Me-4-Pip |
| 2-238 | 3-F-Ph | 4-Pyr | 1-Me-4-(3,4-deH-Pip) |

Table 2 (continued)

| Compound No. | $R^1$ | $R^2$ | $R^3$ |
|---|---|---|---|
| 2-239 | 3-F-Ph | 4-Pyr | 1-Et-4-Pip |
| 2-240 | 3-F-Ph | 4-Pyr | 1-Bn-4-Pip |
| 2-241 | 3-F-Ph | 4-Pyr | 3-Hpip |
| 2-242 | 3-F-Ph | 4-Pyr | 1-Me-3-Hpip |
| 2-243 | 3-F-Ph | 4-Pyr | 4-Hpip |
| 2-244 | 3-F-Ph | 4-Pyr | 1-Me-4-Hpip |
| 2-245 | 3-F-Ph | 4-Pyr | 2-Mor |
| 2-246 | 3-F-Ph | 4-Pyr | 1-Me-2-Mor |
| 2-247 | 3-F-Ph | 4-Pyr | 2-Tmor |
| 2-248 | 3-F-Ph | 4-Pyr | 1-Me-2-Tmor |
| 2-249 | 3-F-Ph | 4-Pyr | 1-Piz |
| 2-250 | 3-F-Ph | 4-Pyr | 4-Me-1-Piz |
| 2-251 | 3-F-Ph | 4-Pyr | 2-Piz |
| 2-252 | 3-F-Ph | 4-Pyr | 4-Pyr |
| 2-253 | 3-F-Ph | 4-Pyr | 3-Pyr |
| 2-254 | 3-F-Ph | 4-Pyr | 2-Pyr |
| 2-255 | 3-F-Ph | 4-Pyr | 4-Pym |
| 2-256 | 3-F-Ph | 4-Pyr | 5-Pym |
| 2-257 | 3-F-Ph | 4-Pyr | 2-Pym |
| 2-258 | 3-F-Ph | 4-Pyr | 3-Azt-$CH_2$ |
| 2-259 | 3-F-Ph | 4-Pyr | 1-Me-3-Azt-$CH_2$ |
| 2-260 | 3-F-Ph | 4-Pyr | 3-Pyrd-$CH_2$ |
| 2-261 | 3-F-Ph | 4-Pyr | 1-Me-3-Pyrd-$CH_2$ |
| 2-262 | 3-F-Ph | 4-Pyr | 4-Pip-$CH_2$ |
| 2-263 | 3-F-Ph | 4-Pyr | 1-Me-4-Pip-$CH_2$ |
| 2-264 | 3-F-Ph | 4-Pyr | 3-Hpip-$CH_2$ |
| 2-265 | 3-F-Ph | 4-Pyr | 1-Me-3-Hpip-$CH_2$ |
| 2-266 | 3-F-Ph | 4-Pyr | 4-Hpip-$CH_2$ |
| 2-267 | 3-F-Ph | 4-Pyr | 1-Me-4-Hpip-$CH_2$ |
| 2-268 | 3-F-Ph | 4-Pyr | 2-Mor-$CH_2$ |
| 2-269 | 3-F-Ph | 4-Pyr | 1-Me-2-Mor-$CH_2$ |
| 2-270 | 3-F-Ph | 4-Pyr | 2-Tmor-$CH_2$ |
| 2-271 | 3-F-Ph | 4-Pyr | 1-Me-2-Tmor-$CH_2$ |
| 2-272 | 3-F-Ph | 4-Pyr | 1-Piz-$CH_2$ |
| 2-273 | 3-F-Ph | 4-Pyr | 4-Me-1-Piz-$CH_2$ |
| 2-274 | 3-F-Ph | 4-Pyr | 2-Piz-$CH_2$ |
| 2-275 | 3-F-Ph | 4-Pyr | 4-Pyr-$CH_2$ |
| 2-276 | 3-F-Ph | 4-Pyr | 3-Pyr-$CH_2$ |

Table 2 (continued)

| Compound No. | $R^1$ | $R^2$ | $R^3$ |
|---|---|---|---|
| 2-277 | 3-F-Ph | 4-Pyr | 2-Pyr-$CH_2$ |
| 2-278 | 3-F-Ph | 4-Pyr | 4-Pym-$CH_2$ |
| 2-279 | 3-F-Ph | 4-Pyr | 5-Pym-$CH_2$ |
| 2-280 | 3-F-Ph | 4-Pyr | 2-Pym-$CH_2$ |
| 2-281 | 3,4-diF-Ph | 4-Pyr | $H_2N$-$CH_2$ |
| 2-282 | 3,4-diF-Ph | 4-Pyr | $H_2N$-$(CH_2)_2$ |
| 2-283 | 3,4-diF-Ph | 4-Pyr | $H_2N$-$(CH_2)_3$ |
| 2-284 | 3,4-diF-Ph | 4-Pyr | $H_2N$-$(CH_2)_4$ |
| 2-285 | 3,4-diF-Ph | 4-Pyr | MeNH-$CH_2$ |
| 2-286 | 3,4-diF-Ph | 4-Pyr | MeNH-$(CH_2)_2$ |
| 2-287 | 3,4-diF-Ph | 4-Pyr | MeNH-$(CH_2)_3$ |
| 2-288 | 3,4-diF-Ph | 4-Pyr | MeNH-$(CH_2)_4$ |
| 2-289 | 3,4-diF-Ph | 4-Pyr | EtNH-$CH_2$ |
| 2-290 | 3,4-diF-Ph | 4-Pyr | EtNH-$(CH_2)_2$ |
| 2-291 | 3,4-diF-Ph | 4-Pyr | EtNH-$(CH_2)_3$ |
| 2-292 | 3,4-diF-Ph | 4-Pyr | EtNH-$(CH_2)_4$ |
| 2-293 | 3,4-diF-Ph | 4-Pyr | $Me_2N$-$CH_2$ |
| 2-294 | 3,4-diF-Ph | 4-Pyr | $Me_2N$-$(CH_2)_2$ |
| 2-295 | 3,4-diF-Ph | 4-Pyr | $Me_2N$-$(CH_2)_3$ |
| 2-296 | 3,4-diF-Ph | 4-Pyr | $Me_2N$-$(CH_2)_4$ |
| 2-297 | 3,4-diF-Ph | 4-Pyr | 1-Azt-$CH_2$ |
| 2-298 | 3,4-diF-Ph | 4-Pyr | 1-Azt-$(CH_2)_2$ |
| 2-299 | 3,4-diF-Ph | 4-Pyr | 1-Azt-$(CH_2)_3$ |
| 2-300 | 3,4-diF-Ph | 4-Pyr | 1-Azt-$(CH_2)_4$ |
| 2-301 | 3,4-diF-Ph | 4-Pyr | 1-Pyrd-$CH_2$ |
| 2-302 | 3,4-diF-Ph | 4-Pyr | 1-Pyxd-$(CH_2)_2$ |
| 2-303 | 3,4-diF-Ph | 4-Pyr | 1-Pyrd-$(CH_2)_3$ |
| 2-304 | 3,4-diF-Ph | 4-Pyr | 1-Pyrd-$(CH_2)_4$ |
| 2-305 | 3,4-diF-Ph | 4-Pyr | 1-Pip-$CH_2$ |
| 2-306 | 3,4-diF-Ph | 4-Pyr | 1-Pip-$(CH_2)_2$ |
| 2-307 | 3,4-diF-Ph | 4-Pyr | 1-Pip-$(CH_2)_3$ |
| 2-308 | 3,4-diF-Ph | 4-Pyr | 1-Pip-$(CH_2)_4$ |
| 2-309 | 3,4-diF-Ph | 4-Pyr | 1-Mor-$CH_2$ |
| 2-310 | 3,4-diF-Ph | 4-Pyr | 1-Mor-$(CH_2)_2$ |
| 2-311 | 3,4-diF-Ph | 4-Pyr | 1-Mor-$(CH_2)_3$ |
| 2-312 | 3,4-diF-Ph | 4-Pyr | 1-Mor-$(CH_2)_4$ |
| 2-313 | 3,4-diF-Ph | 4-Pyr | 1-Tmor-$CH_2$ |
| 2-314 | 3,4-diF-Ph | 4-Pyr | 1-Tmor-$(CH_2)_2$ |

Table 2 (continued)

| Compound No. | $R^1$ | $R^2$ | $R^3$ |
|---|---|---|---|
| 2-315 | 3,4-diF-Ph | 4-Pyr | 1-Tmor-$(CH_2)_3$ |
| 2-316 | 3,4-diF-Ph | 4-Pyr | 1-Tmor-$(CH_2)_4$ |
| 2-317 | 3,4-diF-Ph | 4-Pyr | 1-Piz-$CH_2$ |
| 2-318 | 3,4-diF-Ph | 4-Pyr | 1-Piz-$(CH_2)_2$ |
| 2-319 | 3,4-diF-Ph | 4-Pyr | 1-Piz-$(CH_2)_3$ |
| 2-320 | 3,4-diF-Ph | 4-Pyr | 4-Me-1-Piz-$(CH_2)_3$ |
| 2-321 | 3,4-diF-Ph | 4-Pyr | 1-Piz-$(CH_2)_4$ |
| 2-322 | 3,4-diF-Ph | 4-Pyr | 3-Azt |
| 2-323 | 3,4-diF-Ph | 4-Pyr | 1-Me-3-Azt |
| 2-324 | 3,4-diF-Ph | 4-Pyr | 1-Bn-3-Azt |
| 2-325 | 3,4-diF-Ph | 4-Pyr | 3-Pyrd |
| 2-326 | 3,4-diF-Ph | 4-Pyr | 1-Me-3-Pyrd |
| 2-327 | 3,4-diF-Ph | 4-Pyr | 3-Pip |
| 2-328 | 3,4-diF-Ph | 4-Pyr | 4-Pip |
| 2-329 | 3,4-diF-Ph | 4-Pyr | 4-(3,4-deH-Pip) |
| 2-330 | 3,4-diF-Ph | 4-Pyr | 1-Me-4-Pip |
| 2-331 | 3,4-diF-Ph | 4-Pyr | 1-Me-4-(3,4-deH-Pip) |
| 2-332 | 3,4-diF-Ph | 4-Pyr | 1-Et-4-Pip |
| 2-333 | 3,4-diF-Ph | 4-Pyr | 1-Bn-4-Pip |
| 2-334 | 3,4-diF-Ph | 4-Pyr | 3-Hpip |
| 2-335 | 3,4-diF-Ph | 4-Pyr | 1-Me-3-Hpip |
| 2-336 | 3,4-diF-Ph | 4-Pyr | 4-Hpip |
| 2-337 | 3,4-diF-Ph | 4-Pyr | 1-Me-4-Hpip |
| 2-338 | 3,4-diF-Ph | 4-Pyr | 2-Mor |
| 2-339 | 3,4-diF-Ph | 4-Pyr | 1-Me-2-Mor |
| 2-340 | 3,4-diF-Ph | 4-Pyr | 2-Tmor |
| 2-341 | 3,4-diF-Ph | 4-Pyr | 1-Me-2-Tmor |
| 2-342 | 3,4-diF-Ph | 4-Pyr | 1-Piz |
| 2-343 | 3,4-diF-Ph | 4-Pyr | 4-Me-1-Piz |
| 2-344 | 3,4-diF-Ph | 4-Pyr | 2-Piz |
| 2-345 | 3,4-diF-Ph | 4-Pyr | 4-Pyr |
| 2-346 | 3,4-diF-Ph | 4-Pyr | 3-Pyr |
| 2-347 | 3,4-diF-Ph | 4-Pyr | 2-Pyr |
| 2-348 | 3,4-diF-Ph | 4-Pyr | 4-Pym |
| 2-349 | 3,4-diF-Ph | 4-Pyr | 5-Pym |
| 2-350 | 3,4-diF-Ph | 4-Pyr | 2-Pym |
| 2-351 | 3,4-diF-Ph | 4-Pyr | 3-Azt-$CH_2$ |
| 2-352 | 3,4-diF-Ph | 4-Pyr | 1-Me-3-Azt-$CH_2$ |

Table 2 (continued)

| Compound No. | $R^1$ | $R^2$ | $R^3$ |
|---|---|---|---|
| 2-353 | 3,4-diF-Ph | 4-Pyr | 3-Pyrd-$CH_2$ |
| 2-354 | 3,4-diF-Ph | 4-Pyr | 1-Me-3-Pyrd-$CH_2$ |
| 2-355 | 3,4-diF-Ph | 4-Pyr | 4-Pip-$CH_2$ |
| 2-356 | 3,4-diF-Ph | 4-Pyr | 1-Me-4-Pip-$CH_2$ |
| 2-357 | 3,4-diF-Ph | 4-Pyr | 3-Hpip-$CH_2$ |
| 2-358 | 3,4-diF-Ph | 4-Pyr | 1-Me-3-Hpip-$CH_2$ |
| 2-359 | 3,4-diF-Ph | 4-Pyr | 4-Hpip-$CH_2$ |
| 2-360 | 3,4-diF-Ph | 4-Pyr | 1-Me-4-Hpip-$CH_2$ |
| 2-361 | 3,4-diF-Ph | 4-Pyr | 2-Mor-$CH_2$ |
| 2-362 | 3,4-diF-Ph | 4-Pyr | 1-Me-2-Mor-$CH_2$ |
| 2-363 | 3,4-diF-Ph | 4-Pyr | 2-Tmor-$CH_2$ |
| 2-364 | 3,4-diF-Ph | 4-Pyr | 1-Me-2-Tmor-$CH_2$ |
| 2-365 | 3,4-diF-Ph | 4-Pyr | 1-Piz-$CH_2$ |
| 2-366 | 3,4-diF-Ph | 4-Pyr | 4-Me-1-Piz-$CH_2$ |
| 2-367 | 3,4-diF-Ph | 4-Pyr | 2-Piz-$CH_2$ |
| 2-368 | 3,4-diF-Ph | 4-Pyr | 4-Pyr-$CH_2$ |
| 2-369 | 3,4-diF-Ph | 4-Pyr | 3-Pyr-$CH_2$ |
| 2-370 | 3,4-diF-Ph | 4-Pyr | 2-Pyr-$CH_2$ |
| 2-371 | 3,4-diF-Ph | 4-Pyr | 4-Pym-$CH_2$ |
| 2-372 | 3,4-diF-Ph | 4-Pyr | 5-Pym-$CH_2$ |
| 2-373 | 3,4-diF-Ph | 4-Pyr | 2-Pym-$CH_2$ |
| 2-374 | 3-Cl-Ph | 4-Pyr | $H_2N$-$CH_2$ |
| 2-375 | 3-Cl-Ph | 4-Pyr | $H_2N$-$(CH_2)_2$ |
| 2-376 | 3-Cl-Ph | 4-Pyr | $H_2N$-$(CH_2)_3$ |
| 2-377 | 3-Cl-Ph | 4-Pyr | $H_2N$-$(CH_2)_4$ |
| 2-378 | 3-Cl-Ph | 4-Pyr | MeNH-$CH_2$ |
| 2-379 | 3-Cl-Ph | 4-Pyr | MeNH-$(CH_2)_2$ |
| 2-380 | 3-Cl-Ph | 4-Pyr | MeNH-$(CH_2)_3$ |
| 2-381 | 3-Cl-Ph | 4-Pyr | MeNH-$(CH_2)_4$ |
| 2-382 | 3-Cl-Ph | 4-Pyr | EtNH-$CH_2$ |
| 2-383 | 3-Cl-Ph | 4-Pyr | EtNH-$(CH_2)_2$ |
| 2-384 | 3-Cl-Ph | 4-Pyr | EtNH-$(CH_2)_3$ |
| 2-385 | 3-Cl-Ph | 4-Pyr | EtNH-$(CH_2)_4$ |
| 2-386 | 3-Cl-Ph | 4-Pyr | $Me_2N$-$CH_2$ |
| 2-387 | 3-Cl-Ph | 4-Pyr | $Me_2N$-$(CH_2)_2$ |
| 2-388 | 3-Cl-Ph | 4-Pyr | $Me_2N$-$(CH_2)_3$ |
| 2-389 | 3-Cl-Ph | 4-Pyr | $Me_2N$-$(CH_2)_4$ |
| 2-390 | 3-Cl-Ph | 4-Pyr | 1-Azt-$CH_2$ |

Table 2 (continued)

| Compound No. | $R^1$ | $R^2$ | $R^3$ |
|---|---|---|---|
| 2-391 | 3-Cl-Ph | 4-Pyr | 1-Azt-$(CH_2)_2$ |
| 2-392 | 3-Cl-Ph | 4-Pyr | 1-Azt-$(CH_2)_3$ |
| 2-393 | 3-Cl-Ph | 4-Pyr | 1-Azt-$(CH_2)_4$ |
| 2-394 | 3-Cl-Ph | 4-Pyr | 1-Pyrd-$CH_2$ |
| 2-395 | 3-Cl-Ph | 4-Pyr | 1-Pyrd-$(CH_2)_2$ |
| 2-396 | 3-Cl-Ph | 4-Pyr | 1-Pyrd-$(CH_2)_3$ |
| 2-397 | 3-Cl-Ph | 4-Pyr | 1-Pyrd-$(CH_2)_4$ |
| 2-398 | 3-Cl-Ph | 4-Pyr | 1-Pip-$CH_2$ |
| 2-399 | 3-Cl-Ph | 4-Pyr | 1-Pip-$(CH_2)_2$ |
| 2-400 | 3-Cl-Ph | 4-Pyr | 1-Pip-$(CH_2)_3$ |
| 2-401 | 3-Cl-Ph | 4-Pyr | 1-Pip-$(CH_2)_4$ |
| 2-402 | 3-Cl-Ph | 4-Pyr | 1-Mor-$CH_2$ |
| 2-403 | 3-Cl-Ph | 4-Pyr | 1-Mor-$(CH_2)_2$ |
| 2-404 | 3-Cl-Ph | 4-Pyr | 1-Mor-$(CH_2)_3$ |
| 2-405 | 3-Cl-Ph | 4-Pyr | 1-Mor-$(CH_2)_4$ |
| 2-406 | 3-Cl-Ph | 4-Pyr | 1-Tmor-$CH_2$ |
| 2-407 | 3-Cl-Ph | 4-Pyr | 1-Tmor-$(CH_2)_2$ |
| 2-408 | 3-Cl-Ph | 4-Pyr | 1-Tmor-$(CH_2)_3$ |
| 2-409 | 3-Cl-Ph | 4-Pyr | 1-Tmor-$(CH_2)_4$ |
| 2-410 | 3-Cl-Ph | 4-Pyr | 1-Piz-$CH_2$ |
| 2-411 | 3-Cl-Ph | 4-Pyr | 1-Piz-$(CH_2)_2$ |
| 2-412 | 3-Cl-Ph | 4-Pyr | 1-Piz-$(CH_2)_3$ |
| 2-413 | 3-Cl-Ph | 4-Pyr | 4-Me-1-Piz-$(CH_2)_3$ |
| 2-414 | 3-Cl-Ph | 4-Pyr | 1-Piz-$(CH_2)_4$ |
| 2-415 | 3-Cl-Ph | 4-Pyr | 3-Azt |
| 2-416 | 3-Cl-Ph | 4-Pyr | 1-Me-3-Azt |
| 2-417 | 3-Cl-Ph | 4-Pyr | 1-Bn-3-Azt |
| 2-418 | 3-Cl-Ph | 4-Pyr | 3-Pyrd |
| 2-419 | 3-Cl-Ph | 4-Pyr | 1-Me-3-Pyrd |
| 2-420 | 3-Cl-Ph | 4-Pyr | 3-Pip |
| 2-421 | 3-Cl-Ph | 4-Pyr | 4-Pip |
| 2-422 | 3-Cl-Ph | 4-Pyr | 4-(3,4-deH-Pip) |
| 2-423 | 3-Cl-Ph | 4-Pyr | 1-Me-4-Pip |
| 2-424 | 3-Cl-Ph | 4-Pyr | 1-Me-4-(3,4-deH-Pip) |
| 2-425 | 3-Cl-Ph | 4-Pyr | 1-Et-4-Pip |
| 2-426 | 3-Cl-Ph | 4-Pyr | 1-Bn-4-Pip |
| 2-427 | 3-Cl-Ph | 4-Pyr | 3-Hpip |
| 2-428 | 3-Cl-Ph | 4-Pyr | 1-Me-3-Hpip |

Table 2 (continued)

| Compound No. | $R^1$ | $R^2$ | $R^3$ |
|---|---|---|---|
| 2-429 | 3-Cl-Ph | 4-Pyr | 4-Hpip |
| 2-430 | 3-Cl-Ph | 4-Pyr | 1-Me-4-Hpip |
| 2-431 | 3-Cl-Ph | 4-Pyr | 2-Mor |
| 2-432 | 3-Cl-Ph | 4-Pyr | 1-Me-2-Mor |
| 2-433 | 3-Cl-Ph | 4-Pyr | 2-Tmor |
| 2-434 | 3-Cl-Ph | 4-Pyr | 1-Me-2-Tmor |
| 2-435 | 3-Cl-Ph | 4-Pyr | 1-Piz |
| 2-436 | 3-Cl-Ph | 4-Pyr | 4-Me-1-Piz |
| 2-437 | 3-Cl-Ph | 4-Pyr | 2-Piz |
| 2-438 | 3-Cl-Ph | 4-Pyr | 4-Pyr |
| 2-439 | 3-Cl-Ph | 4-Pyr | 3-Pyr |
| 2-440 | 3-Cl-Ph | 4-Pyr | 2-Pyr |
| 2-441 | 3-Cl-Ph | 4-Pyr | 4-Pym |
| 2-442 | 3-Cl-Ph | 4-Pyr | 5-Pym |
| 2-443 | 3-Cl-Ph | 4-Pyr | 2-Pym |
| 2-444 | 3-Cl-Ph | 4-Pyr | 3-Azt-$CH_2$ |
| 2-445 | 3-Cl-Ph | 4-Pyr | 1-Me-3-Azt-$CH_2$ |
| 2-446 | 3-Cl-Ph | 4-Pyr | 3-Pyrd-$CH_2$ |
| 2-447 | 3-Cl-Ph | 4-Pyr | 1-Me-3-Pyrd-$CH_2$ |
| 2-448 | 3-Cl-Ph | 4-Pyr | 4-Pip-$CH_2$ |
| 2-449 | 3-Cl-Ph | 4-Pyr | 1-Me-4-Pip-$CH_2$ |
| 2-450 | 3-Cl-Ph | 4-Pyr | 3-Hpip-$CH_2$ |
| 2-451 | 3-Cl-Ph | 4-Pyr | 1-Me-3-Hpip-$CH_2$ |
| 2-452 | 3-Cl-Ph | 4-Pyr | 4-Hpip-$CH_2$ |
| 2-453 | 3-Cl-Ph | 4-Pyr | 1-Me-4-Hpip-$CH_2$ |
| 2-454 | 3-Cl-Ph | 4-Pyr | 2-Mor-$CH_2$ |
| 2-455 | 3-Cl-Ph | 4-Pyr | 1-Me-2-Mor-$CH_2$ |
| 2-456 | 3-Cl-Ph | 4-Pyr | 2-Tmor-$CH_2$ |
| 2-457 | 3-Cl-Ph | 4-Pyr | 1-Me-2-Tmor-$CH_2$ |
| 2-458 | 3-Cl-Ph | 4-Pyr | 1-Piz-$CH_2$ |
| 2-459 | 3-Cl-Ph | 4-Pyr | 4-Me-1-Piz-$CH_2$ |
| 2-460 | 3-Cl-Ph | 4-Pyr | 2-Piz-$CH_2$ |
| 2-461 | 3-Cl-Ph | 4-Pyr | 4-Pyr-$CH_2$ |
| 2-462 | 3-Cl-Ph | 4-Pyr | 3-Pyr-$CH_2$ |
| 2-463 | 3-Cl-Ph | 4-Pyr | 2-Pyr-$CH_2$ |
| 2-464 | 3-Cl-Ph | 4-Pyr | 4-Pym-$CH_2$ |
| 2-465 | 3-Cl-Ph | 4-Pyr | 5-Pym-$CH_2$ |
| 2-466 | 3-Cl-Ph | 4-Pyr | 2-Pym-$CH_2$ |

Table 2 (continued)

| Compound No. | $R^1$ | $R^2$ | $R^3$ |
|---|---|---|---|
| 2-467 | Ph | $2-NH_2-4-Pym$ | $H_2N-CH_2$ |
| 2-468 | Ph | $2-NH_2-4-Pym$ | $H_2N-(CH_2)_2$ |
| 2-469 | Ph | $2-NH_2-4-Pym$ | $H_2N-(CH_2)_3$ |
| 2-470 | Ph | $2-NH_2-4-Pym$ | $H_2N-(CH_2)_4$ |
| 2-471 | Ph | $2-NH_2-4-Pym$ | $MeNH-CH_2$ |
| 2-472 | Ph | $2-NH_2-4-Pym$ | $MeNH-(CH_2)_2$ |
| 2-473 | Ph | $2-NH_2-4-Pym$ | $MeNH-(CH_2)_3$ |
| 2-474 | Ph | $2-NH_2-4-Pym$ | $MeNH-(CH_2)_4$ |
| 2-475 | Ph | $2-NH_2-4-Pym$ | $EtNH-CH_2$ |
| 2-476 | Ph | $2-NH_2-4-Pym$ | $EtNH-(CH_2)_2$ |
| 2-477 | Ph | $2-NH_2-4-Pym$ | $EtNH-(CH_2)_3$ |
| 2-478 | Ph | $2-NH_2-4-Pym$ | $EtNH-(CH_2)_4$ |
| 2-479 | Ph | $2-NH_2-4-Pym$ | $Me_2N-CH_2$ |
| 2-480 | Ph | $2-NH_2-4-Pym$ | $Me_2N-(CH_2)_2$ |
| 2-481 | Ph | $2-NH_2-4-Pym$ | $Me_2N-(CH_2)_3$ |
| 2-482 | Ph | $2-NH_2-4-Pym$ | $Me_2N-(CH_2)_4$ |
| 2-483 | Ph | $2-NH_2-4-Pym$ | $1-Azt-CH_2$ |
| 2-484 | Ph | $2-NH_2-4-Pym$ | $1-Azt-(CH_2)_2$ |
| 2-485 | Ph | $2-NH_2-4-Pym$ | $1-Azt-(CH_2)_3$ |
| 2-486 | Ph | $2-NH_2-4-Pym$ | $1-Azt-(CH_2)_4$ |
| 2-487 | Ph | $2-NH_2-4-Pym$ | $1-Pyrd-CH_2$ |
| 2-488 | Ph | $2-NH_2-4-Pym$ | $1-Pyrd-(CH_2)_2$ |
| 2-489 | Ph | $2-NH_2-4-Pym$ | $1-Pyrd-(CH_2)_3$ |
| 2-490 | Ph | $2-NH_2-4-Pym$ | $1-Pyrd-(CH_2)_4$ |
| 2-491 | Ph | $2-NH_2-4-Pym$ | $1-Pip-CH_2$ |
| 2-492 | Ph | $2-NH_2-4-Pym$ | $1-Pip-(CH_2)_2$ |
| 2-493 | Ph | $2-NH_2-4-Pym$ | $1-Pip-(CH_2)_3$ |
| 2-494 | Ph | $2-NH_2-4-Pym$ | $1-Pip-(CH_2)_4$ |
| 2-495 | Ph | $2-NH_2-4-Pym$ | $1-Mor-CH_2$ |
| 2-496 | Ph | $2-NH_2-4-Pym$ | $1-Mor-(CH_2)_2$ |
| 2-497 | Ph | $2-NH_2-4-Pym$ | $1-Mor-(CH_2)_3$ |
| 2-498 | Ph | $2-NH_2-4-Pym$ | $1-Mor-(CH_2)_4$ |
| 2-499 | Ph | $2-NH_2-4-Pym$ | $1-Tmor-CH_2$ |
| 2-500 | Ph | $2-NH_2-4-Pym$ | $1-Tmor-(CH_2)_2$ |
| 2-501 | Ph | $2-NH_2-4-Pym$ | $1-Tmor-(CH_2)_3$ |
| 2-502 | Ph | $2-NH_2-4-Pym$ | $1-Tmor-(CH_2)_4$ |
| 2-503 | Ph | $2-NH_2-4-Pym$ | $1-Piz-CH_2$ |
| 2-504 | Ph | $2-NH_2-4-Pym$ | $1-Piz-(CH_2)_2$ |

Table 2 (continued)

| Compound No. | $R^1$ | $R^2$ | $R^3$ |
|---|---|---|---|
| 2-505 | Ph | $2-NH_2-4-Pym$ | $1-Piz-(CH_2)_3$ |
| 2-506 | Ph | $2-NH_2-4-Pym$ | $4-Me-1-Piz-(CH_2)_3$ |
| 2-507 | Ph | $2-NH_2-4-Pym$ | $1-Piz-(CH_2)_4$ |
| 2-508 | Ph | $2-NH_2-4-Pym$ | 3-Azt |
| 2-509 | Ph | $2-NH_2-4-Pym$ | 1-Me-3-Azt |
| 2-510 | Ph | $2-NH_2-4-Pym$ | 1-Bn-3-Azt |
| 2-511 | Ph | $2-NH_2-4-Pym$ | 3-Pyrd |
| 2-512 | Ph | $2-NH_2-4-Pym$ | 1-Me-3-Pyrd |
| 2-513 | Ph | $2-NH_2-4-Pym$ | 3-Pip |
| 2-514 | Ph | $2-NH_2-4-Pym$ | 4-Pip |
| 2-515 | Ph | $2-NH_2-4-Pym$ | 4-(3,4-deH-Pip) |
| 2-516 | Ph | $2-NH_2-4-Pym$ | 1-Me-4-Pip |
| 2-517 | Ph | $2-NH_2-4-Pym$ | 1-Me-4-(3,4-deH-Pip) |
| 2-518 | Ph | $2-NH_2-4-Pym$ | 1-Et-4-Pip |
| 2-519 | Ph | $2-NH_2-4-Pym$ | 1-Bn-4-Pip |
| 2-520 | Ph | $2-NH_2-4-Pym$ | 3-Hpip |
| 2-521 | Ph | $2-NH_2-4-Pym$ | 1-Me-3-Hpip |
| 2-522 | Ph | $2-NH_2-4-Pym$ | 4-Hpip |
| 2-523 | Ph | $2-NH_2-4-Pym$ | 1-Me-4-Hpip |
| 2-524 | Ph | $2-NH_2-4-Pym$ | 2-Mor |
| 2-525 | Ph | $2-NH_2-4-Pym$ | 1-Me-2-Mor |
| 2-526 | Ph | $2-NH_2-4-Pym$ | 2-Tmor |
| 2-527 | Ph | $2-NH_2-4-Pym$ | 1-Me-2-Tmor |
| 2-528 | Ph | $2-NH_2-4-Pym$ | 1-Piz |
| 2-529 | Ph | $2-NH_2-4-Pym$ | 4-Me-1-Piz |
| 2-530 | Ph | $2-NH_2-4-Pym$ | 2-Piz |
| 2-531 | Ph | $2-NH_2-4-Pym$ | 4-Pyr |
| 2-532 | Ph | $2-NH_2-4-Pym$ | 3-Pyr |
| 2-533 | Ph | $2-NH_2-4-Pym$ | 2-Pyr |
| 2-534 | Ph | $2-NH_2-4-Pym$ | 4-Pym |
| 2-535 | Ph | $2-NH_2-4-Pym$ | 5-Pym |
| 2-536 | Ph | $2-NH_2-4-Pym$ | 2-Pym |
| 2-537 | Ph | $2-NH_2-4-Pym$ | $3-Azt-CH_2$ |
| 2-538 | Ph | $2-NH_2-4-Pym$ | $1-Me-3-Azt-CH_2$ |
| 2-539 | Ph | $2-NH_2-4-Pym$ | $3-Pyrd-CH_2$ |
| 2-540 | Ph | $2-NH_2-4-Pym$ | $1-Me-3-Pyrd-CH_2$ |
| 2-541 | Ph | $2-NH_2-4-Pym$ | $4-Pip-CH_2$ |
| 2-542 | Ph | $2-NH_2-4-Pym$ | $1-Me-4-Pip-CH_2$ |

Table 2 (continued)

| Compound No. | $R^1$ | $R^2$ | $R^3$ |
|---|---|---|---|
| 2-543 | Ph | $2-NH_2-4-Pym$ | $3-Hpip-CH_2$ |
| 2-544 | Ph | $2-NH_2-4-Pym$ | $1-Me-3-Hpip-CH_2$ |
| 2-545 | Ph | $2-NH_2-4-Pym$ | $4-Hpip-CH_2$ |
| 2-546 | Ph | $2-NH_2-4-Pym$ | $1-Me-4-Hpip-CH_2$ |
| 2-547 | Ph | $2-NH_2-4-Pym$ | $2-Mor-CH_2$ |
| 2-548 | Ph | $2-NH_2-4-Pym$ | $1-Me-2-Mor-CH_2$ |
| 2-549 | Ph | $2-NH_2-4-Pym$ | $2-Tmor-CH_2$ |
| 2-550 | Ph | $2-NH_2-4-Pym$ | $1-Me-2-Tmor-CH_2$ |
| 2-551 | Ph | $2-NH_2-4-Pym$ | $1-Piz-CH_2$ |
| 2-552 | Ph | $2-NH_2-4-Pym$ | $4-Me-1-Piz-CH_2$ |
| 2-553 | Ph | $2-NH_2-4-Pym$ | $2-Piz-CH_2$ |
| 2-554 | Ph | $2-NH_2-4-Pym$ | $4-Pyr-CH_2$ |
| 2-555 | Ph | $2-NH_2-4-Pym$ | $3-Pyr-CH_2$ |
| 2-556 | Ph | $2-NH_2-4-Pym$ | $2-Pyr-CH_2$ |
| 2-557 | Ph | $2-NH_2-4-Pym$ | $4-Pym-CH_2$ |
| 2-558 | Ph | $2-NH_2-4-Pym$ | $5-Pym-CH_2$ |
| 2-559 | Ph | $2-NH_2-4-Pym$ | $2-Pym-CH_2$ |
| 2-560 | 4-F-Ph | $2-NH_2-4-Pym$ | $H_2N-CH_2$ |
| 2-561 | 4-F-Ph | $2-NH_2-4-Pym$ | $H_2N-(CH_2)_2$ |
| 2-562 | 4-F-Ph | $2-NH_2-4-Pym$ | $H_2N-(CH_2)_3$ |
| 2-563 | 4-F-Ph | $2-NH_2-4-Pym$ | $H_2N-(CH_2)_4$ |
| 2-564 | 4-F-Ph | $2-NH_2-4-Pym$ | $MeNH-CH_2$ |
| 2-565 | 4-F-Ph | $2-NH_2-4-Pym$ | $MeNH-(CH_2)_2$ |
| 2-566 | 4-F-Ph | $2-NH_2-4-Pym$ | $MeNH-(CH_2)_3$ |
| 2-567 | 4-F-Ph | $2-NH_2-4-Pym$ | $MeNH-(CH_2)_4$ |
| 2-568 | 4-F-Ph | $2-NH_2-4-Pym$ | $EtNH-CH_2$ |
| 2-569 | 4-F-Ph | $2-NH_2-4-Pym$ | $EtNH-(CH_2)_2$ |
| 2-570 | 4-F-Ph | $2-NH_2-4-Pym$ | $EtNH-(CH_2)_3$ |
| 2-571 | 4-F-Ph | $2-NH_2-4-Pym$ | $EtNH-(CH_2)_4$ |
| 2-572 | 4-F-Ph | $2-NH_2-4-Pym$ | $Me_2N-CH_2$ |
| 2-573 | 4-F-Ph | $2-NH_2-4-Pym$ | $Me_2N-(CH_2)_2$ |
| 2-574 | 4-F-Ph | $2-NH_2-4-Pym$ | $Me_2N-(CH_2)_3$ |
| 2-575 | 4-F-Ph | $2-NH_2-4-Pym$ | $Me_2N-(CH_2)_4$ |
| 2-576 | 4-F-Ph | $2-NH_2-4-Pym$ | $1-Azt-CH_2$ |
| 2-577 | 4-F-Ph | $2-NH_2-4-Pym$ | $1-Azt-(CH_2)_2$ |
| 2-578 | 4-F-Ph | $2-NH_2-4-Pym$ | $1-Azt-(CH_2)_3$ |
| 2-579 | 4-F-Ph | $2-NH_2-4-Pym$ | $1-Azt-(CH_2)_4$ |
| 2-580 | 4-F-Ph | $2-NH_2-4-Pym$ | $1-Pyrd-CH_2$ |

Table 2 (continued)

| Compound No. | $R^1$ | $R^2$ | $R^3$ |
|---|---|---|---|
| 2-581 | 4-F-Ph | $2-NH_2-4-Pym$ | $1-Pyrd-(CH_2)_2$ |
| 2-582 | 4-F-Ph | $2-NH_2-4-Pym$ | $1-Pyrd-(CH_2)_3$ |
| 2-583 | 4-F-Ph | $2-NH_2-4-Pym$ | $1-Pyrd-(CH_2)_4$ |
| 2-584 | 4-F-Ph | $2-NH_2-4-Pym$ | $1-Pip-CH_2$ |
| 2-585 | 4-F-Ph | $2-NH_2-4-Pym$ | $1-Pip-(CH_2)_2$ |
| 2-586 | 4-F-Ph | $2-NH_2-4-Pym$ | $1-Pip-(CH_2)_3$ |
| 2-587 | 4-F-Ph | $2-NH_2-4-Pym$ | $1-Pip-(CH_2)_4$ |
| 2-588 | 4-F-Ph | $2-NH_2-4-Pym$ | $1-Mor-CH_2$ |
| 2-589 | 4-F-Ph | $2-NH_2-4-Pym$ | $1-Mor-(CH_2)_2$ |
| 2-590 | 4-F-Ph | $2-NH_2-4-Pym$ | $1-Mor-(CH_2)_3$ |
| 2-591 | 4-F-Ph | $2-NH_2-4-Pym$ | $1-Mor-(CH_2)_4$ |
| 2-592 | 4-F-Ph | $2-NH_2-4-Pym$ | $1-Tmor-CH_2$ |
| 2-593 | 4-F-Ph | $2-NH_2-4-Pym$ | $1-Tmor-(CH_2)_2$ |
| 2-594 | 4-F-Ph | $2-NH_2-4-Pym$ | $1-Tmor-(CH_2)_3$ |
| 2-595 | 4-F-Ph | $2-NH_2-4-Pym$ | $1-Tmor-(CH_2)_4$ |
| 2-596 | 4-F-Ph | $2-NH_2-4-Pym$ | $1-Piz-CH_2$ |
| 2-597 | 4-F-Ph | $2-NH_2-4-Pym$ | $1-Piz-(CH_2)_2$ |
| 2-598 | 4-F-Ph | $2-NH_2-4-Pym$ | $1-Piz-(CH_2)_3$ |
| 2-599 | 4-F-Ph | $2-NH_2-4-Pym$ | $4-Me-1-Piz-(CH_2)_3$ |
| 2-600 | 4-F-Ph | $2-NH_2-4-Pym$ | $1-Piz-(CH_2)_4$ |
| 2-601 | 4-F-Ph | $2-NH_2-4-Pym$ | 3-Azt |
| 2-602 | 4-F-Ph | $2-NH_2-4-Pym$ | 1-Me-3-Azt |
| 2-603 | 4-F-Ph | $2-NH_2-4-Pym$ | 1-Bn-3-Azt |
| 2-604 | 4-F-Ph | $2-NH_2-4-Pym$ | 3-Pyrd |
| 2-605 | 4-F-Ph | $2-NH_2-4-Pym$ | 1-Me-3-Pyrd |
| 2-606 | 4-F-Ph | $2-NH_2-4-Pym$ | 3-Pip |
| 2-607 | 4-F-Ph | $2-NH_2-4-Pym$ | 4-Pip |
| 2-608 | 4-F-Ph | $2-NH_2-4-Pym$ | 4-(3,4-deH-Pip) |
| 2-609 | 4-F-Ph | $2-NH_2-4-Pym$ | 1-Me-4-Pip |
| 2-610 | 4-F-Ph | $2-NH_2-4-Pym$ | 1-Me-4-(3,9-deH-Pip) |
| 2-611 | 4-F-Ph | $2-NH_2-4-Pym$ | 1-Et-4-Pip |
| 2-612 | 4-F-Ph | $2-NH_2-4-Pym$ | 1-Bn-4-Pip |
| 2-613 | 4-F-Ph | $2-NH_2-4-Pym$ | 3-Hpip |
| 2-614 | 4-F-Ph | $2-NH_2-4-Pym$ | 1-Me-3-Hpip |
| 2-615 | 4-F-Ph | $2-NH_2-4-Pym$ | 4-Hpip |
| 2-616 | 4-F-Ph | $2-NH_2-4-Pym$ | 1-Me-4-Hpip |
| 2-617 | 4-F-Ph | $2-NH_2-4-Pym$ | 2-Mor |
| 2-618 | 4-F-Ph | $2-NH_2-4-Pym$ | 1-Me-2-Mor |

Table 2 (continued)

| Compound No. | $R^1$ | $R^2$ | $R^3$ |
|---|---|---|---|
| 2-619 | 4-F-Ph | $2-NH_2-4-Pym$ | 2-Tmor |
| 2-620 | 4-F-Ph | $2-NH_2-4-Pym$ | 1-Me-2-Tmor |
| 2-621 | 4-F-Ph | $2-NH_2-4-Pym$ | 1-Piz |
| 2-622 | 4-F-Ph | $2-NH_2-4-Pym$ | 4-Me-1-Piz |
| 2-623 | 4-F-Ph | $2-NH_2-4-Pym$ | 2-Piz |
| 2-624 | 4-F-Ph | $2-NH_2-4-Pym$ | 4-Pyr |
| 2-625 | 4-F-Ph | $2-NH_2-4-Pym$ | 3-Pyr |
| 2-626 | 4-F-Ph | $2-NH_2-4-Pym$ | 2-Pyr |
| 2-627 | 4-F-Ph | $2-NH_2-4-Pym$ | 4-Pym |
| 2-628 | 4-F-Ph | $2-NH_2-4-Pym$ | 5-Pym |
| 2-629 | 4-F-Ph | $2-NH_2-4-Pym$ | 2-Pym |
| 2-630 | 4-F-Ph | $2-NH_2-4-Pym$ | $3-Azt-CH_2$ |
| 2-631 | 4-F-Ph | $2-NH_2-4-Pym$ | $1-Me-3-Azt-CH_2$ |
| 2-632 | 4-F-Ph | $2-NH_2-4-Pym$ | $3-Pyrd-CH_2$ |
| 2-633 | 4-F-Ph | $2-NH_2-4-Pym$ | $1-Me-3-Pyrd-CH_2$ |
| 2-634 | 4-F-Ph | $2-NH_2-4-Pym$ | $4-Pip-CH_2$ |
| 2-635 | 4-F-Ph | $2-NH_2-4-Pym$ | $1-Me-4-Pip-CH_2$ |
| 2-636 | 4-F-Ph | $2-NH_2-4-Pym$ | $3-Hpip-CH_2$ |
| 2-637 | 4-F-Ph | $2-NH_2-4-Pym$ | $1-Me-3-Hpip-CH_2$ |
| 2-638 | 4-F-Ph | $2-NH_2-4-Pym$ | $4-Hpip-CH_2$ |
| 2-639 | 4-F-Ph | $2-NH_2-4-Pym$ | $1-Me-4-Hpip-CH_2$ |
| 2-640 | 4-F-Ph | $2-NH_2-4-Pym$ | $2-Mor-CH_2$ |
| 2-641 | 4-F-Ph | $2-NH_2-4-Pym$ | $1-Me-2-Mor-CH_2$ |
| 2-642 | 4-F-Ph. | $2-NH_2-4-Pym$ | $2-Tmor-CH_2$ |
| 2-643 | 4-F-Ph | $2-NH_2-4-Pym$ | $1-Me-2-Tmor-CH_2$ |
| 2-644 | 4-F-Ph | $2-NH_2-4-Pym$ | $1-Piz-CH_2$ |
| 2-645 | 4-F-Ph | $2-NH_2-4-Pym$ | $4-Me-1-Piz-CH_2$ |
| 2-646 | 4-F-Ph | $2-NH_2-4-Pym$ | $2-Piz-CH_2$ |
| 2-647 | 4-F-Ph | $2-NH_2-4-Pym$ | $4-Pyr-CH_2$ |
| 2-648 | 4-F-Ph | $2-NH_2-4-Pym$ | $3-Pyr-CH_2$ |
| 2-649 | 4-F-Ph | $2-NH_2-4-Pym$ | $2-Pyr-CH_2$ |
| 2-650 | 4-F-Ph | $2-NH_2-4-Pym$ | $4-Pym-CH_2$ |
| 2-651 | 4-F-Ph | $2-NH_2-4-Pym$ | $5-Pym-CH_2$ |
| 2-652 | 4-F-Ph | $2-NH_2-4-Pym$ | $2-Pym-CH_2$ |
| 2-653 | 3-F-Ph | $2-NH_2-4-Pym$ | $H_2N-CH_2$ |
| 2-654 | 3-F-Ph | $2-NH_2-4-Pym$ | $H_2N-(CH_2)_2$ |
| 2-655 | 3-F-Ph | $2-NH_2-4-Pym$ | $H_2N-(CH_2)_3$ |
| 2-656 | 3-F-Ph | $2-NH_2-4-Pym$ | $H_2N-(CH_2)_4$ |

Table 2 (continued)

| Compound No. | $R^1$ | $R^2$ | $R^3$ |
|---|---|---|---|
| 2-657 | 3-F-Ph | $2-NH_2-4-Pym$ | $MeNH-CH_2$ |
| 2-658 | 3-F-Ph | $2-NH_2-4-Pym$ | $MeNH-(CH_2)_2$ |
| 2-659 | 3-F-Ph | $2-NH_2-4-Pym$ | $MeNH-(CH_2)_3$ |
| 2-660 | 3-F-Ph | $2-NH_2-4-Pym$ | $MeNH-(CH_2)_4$ |
| 2-661 | 3-F-Ph | $2-NH_2-4-Pym$ | $EtNH-CH_2$ |
| 2-662 | 3-F-Ph | $2-NH_2-4-Pym$ | $EtNH-(CH_2)_2$ |
| 2-663 | 3-F-Ph | $2-NH_2-4-Pym$ | $EtNH-(CH_2)_3$ |
| 2-664 | 3-F-Ph | $2-NH_2-4-Pym$ | $EtNH-(CH_2)_4$ |
| 2-665 | 3-F-Ph | $2-NH_2-4-Pym$ | $Me_2N-CH_2$ |
| 2-666 | 3-F-Ph | $2-NH_2-4-Pym$ | $Me_2N-(CH_2)_2$ |
| 2-667 | 3-F-Ph | $2-NH_2-4-Pym$ | $Me_2N-(CH_2)_3$ |
| 2-668 | 3-F-Ph | $2-NH_2-4-Pym$ | $Me_2N-(CH_2)_4$ |
| 2-669 | 3-F-Ph | $2-NH_2-4-Pym$ | $1-Azt-CH_2$ |
| 2-670 | 3-F-Ph | $2-NH_2-4-Pym$ | $1-Azt-(CH_2)_2$ |
| 2-671 | 3-F-Ph | $2-NH_2-4-Pym$ | $1-Azt-(CH_2)_3$ |
| 2-672 | 3-F-Ph | $2-NH_2-4-Pym$ | $1-Azt-(CH_2)_4$ |
| 2-673 | 3-F-Ph | $2-NH_2-4-Pym$ | $1-pyrd-CH_2$ |
| 2-674 | 3-F-Ph | $2-NH_2-4-Pym$ | $1-Pyrd-(CH_2)_2$ |
| 2-675 | 3-F-Ph | $2-NH_2-4-Pym$ | $1-Pyrd-(CH_2)_3$ |
| 2-676 | 3-F-Ph | $2-NH_2-4-Pym$ | $1-Pyrd-(CH_2)_4$ |
| 2-677 | 3-F-Ph | $2-NH_2-4-Pym$ | $1-Pip-CH_2$ |
| 2-678 | 3-F-Ph | $2-NH_2-4-Pym$ | $1-Pip-(CH_2)_2$ |
| 2-679 | 3-F-Ph | $2-NH_2-4-Pym$ | $1-Pip-(CH_2)_3$ |
| 2-680 | 3-F-Ph | $2-NH_2-4-Pym$ | $1-Pip-(CH_2)_4$ |
| 2-681 | 3-F-Ph | $2-NH_2-4-Pym$ | $1-Mor-CH_2$ |
| 2-682 | 3-F-Ph | $2-NH_2-4-Pym$ | $1-Mor-(CH_2)_2$ |
| 2-683 | 3-F-Ph | $2-NH_2-4-Pym$ | $1-Mor-(CH_2)_3$ |
| 2-684 | 3-F-Ph | $2-NH_2-4-Pym$ | $1-Mor-(CH_2)_4$ |
| 2-685 | 3-F-Ph | $2-NH_2-4-Pym$ | $1-Tmor-CH_2$ |
| 2-686 | 3-F-Ph | $2-NH_2-4-Pym$ | $1-Tmor-(CH_2)_2$ |
| 2-687 | 3-F-Ph | $2-NH_2-4-Pym$ | $1-Tmor-(CH_2)_3$ |
| 2-688 | 3-F-Ph | $2-NH_2-4-Pym$ | $1-Tmor-(CH_2)_4$ |
| 2-689 | 3-F-Ph | $2-NH_2-4-Pym$ | $1-Piz-CH_2$ |
| 2-690 | 3-F-Ph | $2-NH_2-4-Pym$ | $1-Piz-(CH_2)_2$ |
| 2-691 | 3-F-Ph | $2-NH_2-4-Pym$ | $1-Piz-(CH_2)_3$ |
| 2-692 | 3-F-Ph | $2-NH_2-4-Pym$ | $4-Me-1-Piz-(CH_2)_3$ |
| 2-693 | 3-F-Ph | $2-NH_2-4-Pym$ | $1-Piz-(CH_2)_4$ |
| 2-694 | 3-F-Ph | $2-NH_2-4-Pym$ | 3-Azt |

Table 2 (continued)

| Compound No. | $R^1$ | $R^2$ | $R^3$ |
|---|---|---|---|
| 2-695 | 3-F-Ph | $2-NH_2$-4-Pym | 1-Me-3-Azt |
| 2-696 | 3-F-Ph | $2-NH_2$-4-Pym | 1-Bn-3-Azt |
| 2-697 | 3-F-Ph | $2-NH_2$-4-Pym | 3-Pyrd |
| 2-698 | 3-F-Ph | $2-NH_2$-4-Pym | 1-Me-3-Pyrd |
| 2-699 | 3-F-Ph | $2-NH_2$-4-Pym | 3-Pip |
| 2-700 | 3-F-Ph | $2-NH_2$-4-Pym | 4-Pip |
| 2-701 | 3-F-Ph | $2-NH_2$-4-Pym | 4-(3,4-deH-Pip) |
| 2-702 | 3-F-Ph | $2-NH_2$-4-Pym | 1-Me-4-Pip |
| 2-703 | 3-F-Ph | $2-NH_2$-4-Pym | 1-Me-4-(3,4-deH-Pip) |
| 2-704 | 3-F-Ph | $2-NH_2$-4-Pym | 1-Et-4-Pip |
| 2-705 | 3-F-Ph | $2-NH_2$-4-Pym | 1-Bn-4-Pip |
| 2-706 | 3-F-Ph | $2-NH_2$-4-Pym | 3-Hpip |
| 2-707 | 3-F-Ph | $2-NH_2$-4-Pym | 1-Me-3-Hpip |
| 2-708 | 3-F-Ph | $2-NH_2$-4-Pym | 4-Hpip |
| 2-709 | 3-F-Ph | $2-NH_2$-4-Pym | 1-Me-4-Hpip |
| 2-710 | 3-F-Ph | $2-NH_2$-4-Pym | 2-Mor |
| 2-711 | 3-F-Ph | $2-NH_2$-4-Pym | 1-Me-2-Mor |
| 2-712 | 3-F-Ph | $2-NH_2$-4-Pym | 2-Tmor |
| 2-713 | 3-F-Ph | $2-NH_2$-4-Pym | 1-Me-2-Tmor |
| 2-714 | 3-F-Ph | $2-NH_2$-4-Pym | 1-Piz |
| 2-715 | 3-F-Ph | $2-NH_2$-4-Pym | 4-Me-1-Piz |
| 2-716 | 3-F-Ph | $2-NH_2$-4-Pym | 2-Piz |
| 2-717 | 3-F-Ph | $2-NH_2$-4-Pym | 4-Pyr |
| 2-718 | 3-F-Ph | $2-NH_2$-4-Pym | 3-Pyr |
| 2-719 | 3-F-Ph | $2-NH_2$-4-Pym | 2-Pyr |
| 2-720 | 3-F-Ph | $2-NH_2$-4-Pym | 4-Pym |
| 2-721 | 3-F-Ph | $2-NH_2$-4-Pym | 5-Pym |
| 2-722 | 3-F-Ph | $2-NH_2$-4-Pym | 2-Pym |
| 2-723 | 3-F-Ph | $2-NH_2$-4-Pym | 3-Azt-$CH_2$ |
| 2-724 | 3-F-Ph | $2-NH_2$-4-Pym | 1-Me-3-Azt-$CH_2$ |
| 2-725 | 3-F-Ph | $2-NH_2$-4-Pym | 3-Pyrd-$CH_2$ |
| 2-726 | 3-F-Ph | $2-NH_2$-4-Pym | 1-Me-3-Pyrd-$CH_2$ |
| 2-727 | 3-F-Ph | $2-NH_2$-4-Pym | 4-Pip-$CH_2$ |
| 2-728 | 3-F-Ph | $2-NH_2$-4-Pym | 1-Me-4-Pip-$CH_2$ |
| 2-729 | 3-F-Ph | $2-NH_2$-4-Pym | 3-Hpip-$CH_2$ |
| 2-730 | 3-F-Ph | $2-NH_2$-4-Pym | 1-Me-3-Hpip-$CH_2$ |
| 2-731 | 3-F-Ph | $2-NH_2$-4-Pym | 4-Hpip-$CH_2$ |
| 2-732 | 3-F-Ph | $2-NH_2$-4-Pym | 1-Me-4-Hpip-$CH_2$ |

Table 2 (continued)

| Compound No. | $R^1$ | $R^2$ | $R^3$ |
|---|---|---|---|
| 2-733 | 3-F-Ph | 2-$NH_2$-4-Pym | 2-Mor-$CH_2$ |
| 2-734 | 3-F-Ph | 2-$NH_2$-4-Pym | 1-Me-2-Mor-$CH_2$ |
| 2-735 | 3-F-Ph | 2-$NH_2$-4-Pym | 2-Tmor-$CH_2$ |
| 2-736 | 3-F-Ph | 2-$NH_2$-4-Pym | 1-Me-2-Tmor-$CH_2$ |
| 2-737 | 3-F-Ph | 2-$NH_2$-4-Pym | 1-Piz-$CH_2$ |
| 2-738 | 3-F-Ph | 2-$NH_2$-4-Pym | 4-Me-1-Piz-$CH_2$ |
| 2-739 | 3-F-Ph | 2-$NH_2$-4-Pym | 2-Piz-$CH_2$ |
| 2-740 | 3-F-Ph | 2-$NH_2$-4-Pym | 4-Pyr-$CH_2$ |
| 2-741 | 3-F-Ph | 2-$NH_2$-4-Pym | 3-Pyr-$CH_2$ |
| 2-742 | 3-F-Ph | 2-$NH_2$-4-Pym | 2-Pyr-$CH_2$ |
| 2-743 | 3-F-Ph | 2-$NH_2$-4-Pym | 4-Pym-$CH_2$ |
| 2-744 | 3-F-Ph | 2-$NH_2$-4-Pym | 5-Pym-$CH_2$ |
| 2-745 | 3-F-Ph | 2-$NH_2$-4-Pym | 2-Pym-$CH_2$ |
| 2-746 | 3,4-diF-Ph | 2-$NH_2$-4-Pym | $H_2N$-$CH_2$ |
| 2-747 | 3,4-diP-Ph | 2-$NH_z$-4-Pym | $H_2N$-$(CH_2)_2$ |
| 2-748 | 3,4-diP-Ph | 2-$NH_2$-4-Pym | $H_2N$-$(CH_2)_3$ |
| 2-749 | 3,4-diF-Ph | 2-$NH_2$-4-Pym | $H_2N$-$(CH_2)_4$ |
| 2-750 | 3,4-diF-Ph | 2-$NH_2$-4-Pym | MeNH-$CH_2$ |
| 2-751 | 3,4-diF-Ph | 2-$NH_2$-4-Pym | MeNH-$(CH_2)_2$ |
| 2-752 | 3,4-diP-Ph | 2-$NH_2$-4-Pym | MeNH-$(CH_2)_3$ |
| 2-753 | 3,4-diF-Ph | 2-$NH_2$-4-Pym | MeNH-$(CH_2)_4$ |
| 2-754 | 3,4-diF-Ph | 2-$NH_2$-4-Pym | EtNH-$CH_2$ |
| 2-755 | 3,4-diF-Ph | 2-$NH_2$-4-Pym | EtNH-$(CH_2)_2$ |
| 2-756 | 3,4-diF-Ph | 2-$NH_2$-4-Pym | EtNH-$(CH_2)_3$ |
| 2-757 | 3,4-diF-Ph | 2-$NH_2$-4-Pym | EtNH-$(CH_2)_4$ |
| 2-758 | 3,4-diF-Ph | 2-$MH_2$-4-Pym | $Me_2N$-$CH_2$ |
| 2-759 | 3,4-diF-Ph | 2-$NH_2$-4-Pym | $Me_2N$-$(CH_2)_2$ |
| 2-760 | 3,4-diF-Ph | 2-$NH_2$-4-Pym | $Me_2N$-$(CH_2)_3$ |
| 2-761 | 3,4-diF-Ph | 2-$NH_2$-4-Pym | $Me_2N$-$(CH_2)_4$ |
| 2-762 | 3,4-diF-Ph | 2-$NH_2$-4-Pym | 1-Azt-$CH_2$ |
| 2-763 | 3,4-diF-Ph | 2-$NH_2$-4-Pym | 1-Azt-$(CH_2)_2$ |
| 2-764 | 3,4-diF-Ph | 2-$NH_2$-4-Pym | 1-Azt-$(CH_2)_3$ |
| 2-765 | 3,4-diF-Ph | 2-$NH_2$-4-Pym | 1-Azt-$(CH_2)_4$ |
| 2-766 | 3,4-diF-Ph | 2-$NH_2$-4-Pym | 1-Pyrd-$CH_2$ |
| 2-767 | 3,4-diF-Ph | 2-$NH_2$-4-Pym | 1-Pyrd-$(CH_2)_2$ |
| 2-768 | 3,4-diF-Ph | 2-$NH_2$-4-Pym | 1-pyrd-$(CH_2)_3$ |
| 2-769 | 3,4-diF-Ph | 2-$NH_2$-4-Pym | 1-Pyrd-$(CH_2)_4$ |
| 2-770 | 3,4-diF-Ph | 2-$NH_2$-4-Pym | 1-Pip-$CH_2$ |

Table 2 (continued)

| Compound No. | $R^1$ | $R^2$ | $R^3$ |
|---|---|---|---|
| 2-771 | 3,4-diF-Ph | 2-$NH_2$-4-Pym | 1-Pip-$(CH_2)_2$ |
| 2-772 | 3,4-diF-Ph | 2-$NH_2$-4-Pym | 1-Pip-$(CH_2)_3$ |
| 2-773 | 3,4-diF-Ph | 2-$NH_2$-4-Pym | 1-Pip-$(CH_2)_4$ |
| 2-774 | 3,4-diF-Ph | 2-$NH_2$-4-Pym | 1-Mor-$CH_2$ |
| 2-775 | 3,4-diF-Ph | 2-$NH_2$-4-Pym | 1-Mor-$(CH_2)_2$ |
| 2-776 | 3,4-diF-Ph | 2-$NH_2$-4-Pym | 1-Mor-$(CH_2)_3$ |
| 2-777 | 3,4-diF-Ph | 2-$NH_2$-4-Pym | 1-Mor-$(CH_2)_4$ |
| 2-778 | 3,4-diF-Ph | 2-$NH_2$-4-Pym | 1-Tmor-$CH_2$ |
| 2-779 | 3,4-diF-Ph | 2-$NH_2$-4-Pym | 1-Tmor-$(CH_2)_2$ |
| 2-780 | 3,4-diF-Ph | 2-$NH_2$-4-Pym | 1-Tmor-$(CH_2)_3$ |
| 2-781 | 3,4-diF-Ph | 2-$NH_2$-4-Pym | 1-Tmor-$(CH_2)_4$ |
| 2-782 | 3,4-diF-Ph | 2-$NH_2$-4-Pym | 1-Piz-$CH_2$ |
| 2-783 | 3,4-diF-Ph | 2-$NH_2$-4-Pym | 1-Piz-$(CH_2)_2$ |
| 2-784 | 3,4-diF-Ph | 2-$NH_2$-4-Pym | 1-Piz-$(CH_2)_3$ |
| 2-785 | 3,4-diF-Ph | 2-$NH_2$-4-Pym | 4-Me-1-Piz-$(CH_2)_3$ |
| 2-786 | 3,4-diF-Ph | 2-$NH_2$-4-Pym | 1-Piz-$(CH_2)_4$ |
| 2-787 | 3,4-diF-Ph | 2-$NH_2$-4-Pym | 3-Azt |
| 2-788 | 3,4-diF-Ph | 2-$NH_2$-4-Pym | 1-Me-3-Azt |
| 2-789 | 3,4-diF-Ph | 2-$NH_2$-4-Pym | 1-Bn-3-Azt |
| 2-790 | 3,4-diF-Ph | 2-$NH_2$-4-Pym | 3-Pyrd |
| 2-791 | 3,4-diF-Ph | 2-$NH_2$-4-Pym | 1-Me-3-Pyrd |
| 2-792 | 3,4-diF-Ph | 2-$NH_2$-4-Pym | 3-Pip |
| 2-793 | 3,4-diF-Ph | 2-$NH_2$-4-Pym | 4-Pip |
| 2-794 | 3,4-diF-Ph | 2-$NH_2$-4-Pym | 4-(3,4-deH-Pip) |
| 2-795 | 3,4-diF-Ph | 2-$NH_2$-4-Pym | 1-Me-4-Pip |
| 2-796 | 3,4-diF-Ph | 2-$NH_2$-4-Pym | 1-Me-4-(3,4-deH-Pip) |
| 2-797 | 3,4-diF-Ph | 2-$NH_2$-4-Pym | 1-Et-4-Pip |
| 2-798 | 3,4-diF-Ph | 2-$NH_2$-4-Pym | 1-Bn-4-Pip |
| 2-799 | 3,4-diF-Ph | 2-$NH_2$-4-Pym | 3-Hpip |
| 2-800 | 3,4-diF-Ph | 2-$NH_2$-4-Pym | 1-Me-3-Hpip |
| 2-801 | 3,4-diF-Ph | 2-$NH_2$-4-Pym | 4-Hpip |
| 2-802 | 3,4-diF-Ph | 2-$NH_2$-4-Pym | 1-Me-4-Hpip |
| 2-803 | 3,4-diF-Ph | 2-$NH_2$-4-Pym | 2-Mor |
| 2-804 | 3,4-diF-Ph | 2-$NH_2$-4-Pym | 1-Me-2-Mor |
| 2-805 | 3,4-diF-Ph | 2-$NH_2$-4-Pym | 2-Tmor |
| 2-806 | 3,4-diF-Ph | 2-$NH_2$-4-Pym | 1-Me-2-Tmor |
| 2-807 | 3,4-diF-Ph | 2-$NH_2$-4-Pym | 1-Piz |
| 2-808 | 3,4-diF-Ph | 2-$NH_2$-4-Pym | 4-Me-1-Piz |

Table 2 (continued)

| Compound No. | $R^1$ | $R^2$ | $R^3$ |
|---|---|---|---|
| 2-809 | 3,4-diF-Ph | $2-NH_2-4-Pym$ | 2-Piz |
| 2-810 | 3,4-diF-Ph | $2-NH_2-4-Pym$ | 4-Pyr |
| 2-811 | 3,4-diF-Ph | $2-NH_2-4-Pym$ | 3-Pyr |
| 2-812 | 3,4-diF-Ph | $2-NH_2-4-Pym$ | 2-Pyr |
| 2-813 | 3,4-diF-Ph | $2-NH_2-4-Pym$ | 4-Pym |
| 2-814 | 3,4-diF-Ph | $2-NH_2-4-Pym$ | 5-Pym |
| 2-815 | 3,4-diF-Ph | $2-NH_2-4-Pym$ | 2-Pym |
| 2-816 | 3,4-diF-Ph | $2-NH_2-4-Pym$ | $3-Azt-CH_2$ |
| 2-817 | 3,4-diF-Ph | $2-NH_2-4-Pym$ | $1-Me-3-Azt-CH_2$ |
| 2-818 | 3,4-diF-Ph | $2-NH_2-4-Pym$ | $3-Pyrd-CH_2$ |
| 2-819 | 3,4-diF-Ph | $2-NH_2-4-Pym$ | $1-Me-3-Pyrd-CH_2$ |
| 2-820 | 3,4-diF-Ph | $2-NH_2-4-Pym$ | $4-Pip-CH_2$ |
| 2-821 | 3,4-diF-Ph | $2-NH_2-4-Pym$ | $1-Me-4-Pip-CH_2$ |
| 2-822 | 3,4-diF-Ph | $2-NH_2-4-Pym$ | $3-Hpip-CH_2$ |
| 2-823 | 3,4-diF-Ph | $2-NH_2-4-Pym$ | $1-Me-3-Hpip-CH_2$ |
| 2-824 | 3,4-diF-Ph | $2-NH_2-4-Pym$ | $4-Hpip-CH_2$ |
| 2-825 | 3,4-diF-Ph | $2-NH_2-4-Pym$ | $1-Me-4-Hpip-CH_2$ |
| 2-826 | 3,4-diF-Ph | $2-NH_2-4-Pym$ | $2-Mor-CH_2$ |
| 2-827 | 3,4-diF-Ph | $2-NH_2-4-Pym$ | $1-Me-2-Mor-CH_2$ |
| 2-828 | 3,4-diF-Ph | $2-NH_2-4-Pym$ | $2-Tmor-CH_2$ |
| 2-829 | 3,4-diF-Ph | $2-NH_2-4-Pym$ | 1-Me-2-Tmor-CH2 |
| 2-830 | 3,4-diF-Ph | $2-NH_2-4-Pym$ | $1-Piz-CH_2$ |
| 2-831 | 3,4-diF-Ph | $2-NH_2-4-Pym$ | $4-Me-1-Piz-CH_2$ |
| 2-832 | 3,4-diF-Ph | $2-NH_2-4-Pym$ | $2-Piz-CH_2$ |
| 2-833 | 3,4-diF-Ph | $2-NH_2-4-Pym$ | $4-Pyr-CH_2$ |
| 2-834 | 3,4-diF-Ph | $2-NH_2-4-Pym$ | $3-Pyr-CH_2$ |
| 2-835 | 3,4-diF-Ph | $2-NH_2-4-Pym$ | $2-Pyr-CH_2$ |
| 2-836 | 3,4-diF-Ph | $2-NH_2-4-Pym$ | $4-Pym-CH_2$ |
| 2-837 | 3,4-diF-Ph | $2-NH_2-4-Pym$ | $5-Pym-CH_2$ |
| 2-838 | 3,4-diF-Ph | $2-NH_2-4-Pym$ | $2-Pym-CH_2$ |
| 2-839 | 3-Cl-Ph | $2-NH_2-4-Pym$ | $H_2N-CH_2$ |
| 2-840 | 3-Cl-Ph | $2-NH_2-4-Pym$ | $H_2N-(CH_2)_2$ |
| 2-841 | 3-Cl-Ph | $2-NH_2-4-Pym$ | $H_2N-(CH_2)_3$ |
| 2-842 | 3-Cl-Ph | $2-NH_2-4-Pym$ | $H_2N-(CH_2)_4$ |
| 2-843 | 3-Cl-Ph | $2-NH_2-4-Pym$ | $MeNH-CH_2$ |
| 2-844 | 3-Cl-Ph | $2-NH_2-4-Pym$ | $MeNH-(CH_2)_2$ |
| 2-845 | 3-Cl-Ph | $2-NH_2-4-Pym$ | $MeNH-(CH_2)_3$ |
| 2-846 | 3-Cl-Ph | $2-NH_2-4-Pym$ | $MeNH-(CH_2)_4$ |

Table 2 (continued)

| Compound No. | $R^1$ | $R^2$ | $R^3$ |
|---|---|---|---|
| 2-847 | 3-Cl-Ph | 2-$NH_2$-4-Pym | EtNH-$CH_2$ |
| 2-848 | 3-Cl-Ph | 2-$NH_2$-4-Pym | EtNH-$(CH_2)_2$ |
| 2-849 | 3-Cl-Ph | 2-$NH_2$-4-Pym | EtNH-$(CH_2)_3$ |
| 2-850 | 3-Cl-Ph | 2-$NH_2$-4-Pym | EtNH-$(CH_2)_4$ |
| 2-851 | 3-Cl-Ph | 2-$NH_2$-4-Pym | $Me_2N$-$CH_2$ |
| 2-852 | 3-Cl-Ph | 2-$NH_2$-4-Pym | $Me_2N$-$(CH_2)_2$ |
| 2-853 | 3-Cl-Ph | 2-$NH_2$-4-Pym | $Me_2N$-$(CH_2)_3$ |
| 2-854 | 3-Cl-Ph | 2-$NH_2$-4-Pym | $Me_2N$-$(CH_2)_4$ |
| 2-855 | 3-Cl-Ph | 2-$NH_2$-4-Pym | 1-Azt-$CH_2$ |
| 2-856 | 3-Cl-Ph | 2-$NH_2$-4-Pym | 1-Azt-$(CH_2)_2$ |
| 2-857 | 3-Cl-Ph | 2-$NH_2$-4-Pym | 1-Azt-$(CH_2)_3$ |
| 2-858 | 3-Cl-Ph | 2-$NH_2$-4-Pym | 1-Azt-$(CH_2)_4$ |
| 2-859 | 3-Cl-Ph | 2-$NH_2$-4-Pym | 1-Pyrd-$CH_2$ |
| 2-860 | 3-Cl-Ph | 2-$NH_2$-4-Pym | 1-Pyrd-$(CH_2)_2$ |
| 2-861 | 3-Cl-Ph | 2-$NH_2$-4-Pym | 1-Pyrd-$(CH_2)_3$ |
| 2-862 | 3-Cl-Ph | 2-$NH_2$-4-Pym | 1-Pyrd-$(CH_2)_4$ |
| 2-863 | 3-Cl-Ph | 2-$NH_2$-4-Pym | 1-Pip-$CH_2$ |
| 2-864 | 3-Cl-Ph | 2-$NH_2$-4-Pym | 1-Pip-$(CH_2)_2$ |
| 2-865 | 3-Cl-Ph | 2-$NH_2$-4-Pym | 1-Pip-$(CH_2)_3$ |
| 2-866 | 3-Cl-Ph | 2-$NH_2$-4-Pym | 1-Pip-$(CH_2)_4$ |
| 2-867 | 3-Cl-Ph | 2-$NH_2$-4-Pym | 1-Mor-$CH_2$ |
| 2-868 | 3-Cl-Ph | 2-$NH_2$-4-Pym | 1-Mor-$(CH_2)_2$ |
| 2-869 | 3-Cl-Ph | 2-$NH_2$-4-Pym | 1-Mor-$(CH_2)_3$ |
| 2-870 | 3-Cl-Ph | 2-$NH_2$-4-Pym | 1-Mor-$(CH_2)_4$ |
| 2-871 | 3-Cl-Ph | 2-$NH_2$-4-Pym | 1-Tmor-$CH_2$ |
| 2-872 | 3-Cl-Ph | 2-$NH_2$-4-Pym | 1-Tmor-$(CH_2)_2$ |
| 2-873 | 3-Cl-Ph | 2-$NH_2$-4-Pym | 1-Tmor-$(CH_2)_3$ |
| 2-874 | 3-Cl-Ph | 2-$NH_2$-4-Pym | 1-Tmor-$(CH_2)_4$ |
| 2-875 | 3-Cl-Ph | 2-$NH_2$-4-Pym | 1-Piz-$CH_2$ |
| 2-876 | 3-Cl-Ph | 2-$NH_2$-4-Pym | 1-Piz-$(CH_2)_2$ |
| 2-877 | 3-Cl-Ph | 2-$NH_2$-4-Pym | 1-Piz-$(CH_2)_3$ |
| 2-878 | 3-Cl-Ph | 2-$NH_2$-4-Pym | 4-Me-1-Piz-$(CH_2)_3$ |
| 2-879 | 3-Cl-Ph | 2-$NH_2$-4-Pym | 1-Piz-$(CH_2)_4$ |
| 2-880 | 3-Cl-Ph | 2-$NH_2$-4-Pym | 3-Azt |
| 2-881 | 3-Cl-Ph | 2-$NH_2$-4-Pym | 1-Me-3-Azt |
| 2-882 | 3-Cl-Ph | 2-$NH_2$-4-Pym | 1-Bn-3-Azt |
| 2-883 | 3-Cl-Ph | 2-$NH_2$-4-Pym | 3-Pyrd |
| 2-884 | 3-Cl-Ph | 2-$NH_2$-4-Pym | 1-Me-3-Pyrd |

Table 2 (continued)

| Compound No. | $R^1$ | $R^2$ | $R^3$ |
|---|---|---|---|
| 2-885 | 3-Cl-Ph | $2-NH_2-4-Pym$ | 3-Pip |
| 2-886 | 3-Cl-Ph | $2-NH_2-4-Pym$ | 4-Pip |
| 2-887 | 3-Cl-Ph | $2-NH_2-4-Pym$ | 4-(3,4-deH-Pip) |
| 2-888 | 3-Cl-Ph | $2-NH_2-4-Pym$ | 1-Me-4-Pip |
| 2-889 | 3-Cl-Ph | $2-NH_2-4-Pym$ | 1-Me-4-(3,4-deH-Pip) |
| 2-890 | 3-Cl-Ph | $2-NH_2-4-Pym$ | 1-Et-4-Pip |
| 2-891 | 3-Cl-Ph | $2-NH_2-4-Pym$ | 1-Bn-4-Pip |
| 2-892 | 3-Cl-Ph | $2-NH_2-4-Pym$ | 3-Hpip |
| 2-893 | 3-Cl-Ph | $2-NH_2-4-Pym$ | 1-Me-3-Hpip |
| 2-894 | 3-Cl-Ph | $2-NH_2-4-Pym$ | 4-Hpip |
| 2-895 | 3-Cl-Ph | $2-NH_2-4-Pym$ | 1-Me-4-Hpip |
| 2-896 | 3-Cl-Ph | $2-NH_2-4-Pym$ | 2-Mor |
| 2-897 | 3-Cl-Ph | $2-NH_2-4-Pym$ | 1-Me-2-Mor |
| 2-898 | 3-Cl-Ph | $2-NH_2-4-Pym$ | 2-Tmor |
| 2-899 | 3-Cl-Ph | $2-NH_2-4-Pym$ | 1-Me-2-Tmor |
| 2-900 | 3-Cl-Ph | $2-NH_2-4-Pym$ | 1-Piz |
| 2-901 | 3-Cl-Ph | $2-NH_2-4-Pym$ | 4-Me-1-Piz |
| 2-902 | 3-Cl-Ph | $2-NH_2-4-Pym$ | 2-Piz |
| 2-903 | 3-Cl-Ph | $2-NH_2-4-Pym$ | 4-Pyr |
| 2-904 | 3-Cl-Ph | $2-NH_2-4-Pym$ | 3-Pyr |
| 2-905 | 3-Cl-Ph | $2-NH_2-4-Pym$ | 2-Pyr |
| 2-906 | 3-Cl-Ph | $2-NH_2-4-Pym$ | 4-Pym |
| 2-907 | 3-Cl-Ph | $2-NH_2-4-Pym$ | 5-Pym |
| 2-908 | 3-Cl-Ph | $2-NH_2-4-Pym$ | 2-Pym |
| 2-909 | 3-Cl-Ph | $2-NH_2-4-Pym$ | $3-Azt-CH_2$ |
| 2-910 | 3-Cl-Ph | $2-NH_2-4-Pym$ | $1-Me-3-Azt-CH_2$ |
| 2-911 | 3-Cl-Ph | $2-NH_2-4-Pym$ | $3-Pyrd-CH_2$ |
| 2-912 | 3-Cl-Ph | $2-NH_2-4-Pym$ | $1-Me-3-Pyrd-CH_2$ |
| 2-913 | 3-Cl-Ph | $2-NH_2-4-Pym$ | $4-Pip-CH_2$ |
| 2-914 | 3-Cl-Ph | $2-NH_2-4-Pym$ | $1-Me-4-Pip-CH_2$ |
| 2-915 | 3-Cl-Ph | $2-NH_2-4-Pym$ | $3-Hpip-CH_2$ |
| 2-916 | 3-Cl-Ph | $2-NH_2-4-Pym$ | $1-Me-3-Hpip-CH_2$ |
| 2-917 | 3-Cl-Ph | $2-NH_2-4-Pym$ | $4-Hpip-CH_2$ |
| 2-918 | 3-Cl-Ph | $2-NH_2-4-Pym$ | $1-Me-4-Hpip-CH_2$ |
| 2-919 | 3-Cl-Ph | $2-NH_2-4-Pym$ | $2-Mor-CH_2$ |
| 2-920 | 3-Cl-Ph | $2-NH_2-4-Pym$ | $1-Me-2-Mor-CH_2$ |
| 2-921 | 3-Cl-Ph | $2-NH_2-4-Pym$ | $2-Tmor-CH_2$ |
| 2-922 | 3-Cl-Ph | $2-NH_2-4-Pym$ | $1-Me-2-Tmor-CH_2$ |

Table 2 (continued)

| Compound No. | $R^1$ | $R^2$ | $R^3$ |
|---|---|---|---|
| 2-923 | 3-Cl-Ph | $2-NH_2$-4-Pym | 1-Piz-$CH_2$ |
| 2-924 | 3-Cl-Ph | $2-NH_2$-4-Pym | 4-Me-1-Piz-$CH_2$ |
| 2-925 | 3-Cl-Ph | $2-NH_2$-4-Pym | 2-Piz-$CH_2$ |
| 2-926 | 3-Cl-Ph | $2-NH_2$-4-Pym | 4-Pyr-$CH_2$ |
| 2-927 | 3-Cl-Ph | $2-NH_2$-4-Pym | 3-Pyr-$CH_2$ |
| 2-928 | 3-Cl-Ph | $2-NH_2$-4-Pym | 2-Pyr-$CH_2$ |
| 2-929 | 3-Cl-Ph | $2-NH_2$-4-Pym | 4-Pym-$CH_2$ |
| 2-930 | 3-Cl-Ph | $2-NH_2$-4-Pym | 5-Pym-$CH_2$ |
| 2-931 | 3-Cl-Ph | $2-NH_2$-4-Pym | 2-Pym-$CH_2$ |
| 2-932 | Ph | 2-MeNH-4-Pym | $H_2N-CH_2$ |
| 2-933 | Ph | 2-MeNH-4-Pym | $H_2N-(CH_2)_2$ |
| 2-934 | Ph | 2-MeNH-4-Pym | $H_2N-(CH_2)_3$ |
| 2-935 | Ph | 2-MeNH-4-Pym | $H_2N-(CH_2)_4$ |
| 2-936 | Ph | 2-MeNH-4-Pym | MeNH-$CH_2$ |
| 2-937 | Ph | 2-MeNH-4-Pym | MeNH-$(CH_2)_2$ |
| 2-938 | Ph | 2-MeNH-4-Pym | MeNH-$(CH_2)_3$ |
| 2-939 | Ph | 2-MeNH-4-Pym | MeNH-$(CH_2)_4$ |
| 2-940 | Ph | 2-MeNH-4-Pym | EtNH-$CH_2$ |
| 2-941 | Ph | 2-MeNH-4-Pym | EtNH-$(CH_2)_2$ |
| 2-942 | Ph | 2-MeNH-4-Pym | EtNH-$(CH_2)_3$ |
| 2-943 | Ph | 2-MeNH-4-Pym | EtNH-$(CH_2)_4$ |
| 2-944 | Ph | 2-MeNH-4-Pym | $Me_2N-CH_2$ |
| 2-945 | Ph | 2-MeNH-4-Pym | $Me_2N-(CH_2)_2$ |
| 2-946 | Ph | 2-MeNH-4-Pym | $Me_2N-(CH_2)_3$ |
| 2-947 | Ph | 2-MeNH-4-Pym | $Me_2N-(CH_2)_4$ |
| 2-948 | Ph | 2-MeNH-4-Pym | 1-Azt-$CH_2$ |
| 2-949 | Ph | 2-MeNH-4-Pym | 1-Azt-$(CH_2)_2$ |
| 2-950 | Ph | 2-MeNH-4-Pym | 1-Azt-$(CH_2)_3$ |
| 2-951 | Ph | 2-MeNH-4-Pym | 1-Azt-$(CH_2)_4$ |
| 2-952 | Ph | 2-MeNH-4-Pym | 1-Pyrd-$CH_2$ |
| 2-953 | Ph | 2-MeNH-4-Pym | 1-Pyrd-$(CH_2)_2$ |
| 2-954 | Ph | 2-MeNH-4-Pym | 1-Pyrd-$(CH_2)_3$ |
| 2-955 | Ph | 2-MeNH-4-Pym | 1-pyrd-$(CH_2)_4$ |
| 2-956 | Ph | 2-MeNH-4-Pym | 1-Pip-$CH_2$ |
| 2-957 | Ph | 2-MeNH-4-Pym | 1-Pip-$(CH_2)_2$ |
| 2-958 | Ph | 2-MeNH-4-Pym | 1-Pip-$(CH_2)_3$ |
| 2-959 | Ph | 2-MeNH-4-Pym | 1-Pip-$(CH_2)_4$ |
| 2-960 | Ph | 2-MeNH-4-Pym | 1-Mor-$CH_2$ |

Table 2 (continued)

| Compound No. | $R^1$ | $R^2$ | $R^3$ |
|---|---|---|---|
| 2-961 | Ph | 2-MeNH-4-Pym | 1-Mor-$(CH_2)_2$ |
| 2-962 | Ph | 2-MeNH-4-Pym | 1-Mor-$(CH_2)_3$ |
| 2-963 | Ph | 2-MeNH-4-Pym | 1-Mor-$(CH_2)_4$ |
| 2-964 | Ph | 2-MeNH-4-Pym | 1-Tmor-$CH_2$ |
| 2-965 | Ph | 2-MeNH-4-Pym | 1-Tmor-$(CH_2)_2$ |
| 2-966 | Ph | 2-MeNH-4-Pym | 1-Tmor-$(CH_2)_3$ |
| 2-967 | Ph | 2-MeNH-4-Pym | 1-Tmor-$(CH_2)_4$ |
| 2-968 | Ph | 2-MeNH-4-Pym | 1-Piz-$CH_2$ |
| 2-969 | Ph | 2-MeNH-4-Pym | 1-Piz-$(CH_2)_2$ |
| 2-970 | Ph | 2-MeNH-4-Pym | 1-Piz-$(CH_2)_3$ |
| 2-971 | Ph | 2-MeNH-4-Pym | 4-Me-1-Piz-$(CH_2)_3$ |
| 2-972 | Ph | 2-MeNH-4-Pym | 1-Piz-$(CH_2)_4$ |
| 2-973 | Ph | 2-MeNH-4-Pym | 3-Azt |
| 2-974 | Ph | 2-MeNH-4-Pym | 1-Me-3-Azt |
| 2-975 | Ph | 2-MeNH-4-Pym | 1-Bn-3-Azt |
| 2-976 | Ph | 2-MeNH-4-Pym | 3-Pyrd |
| 2-977 | Ph | 2-MeNH-4-Pym | 1-Me-3-Pyrd |
| 2-978 | Ph | 2-MeNH-4-Pym | 3-Pip |
| 2-979 | Ph | 2-MeNH-4-Pym | 4-Pip |
| 2-980 | Ph | 2-MeNH-4-Pym | 4-(3,4-deH-Pip) |
| 2-981 | Ph | 2-MeNH-4-Pym | 1-Me-4-Pip |
| 2-982 | Ph | 2-MeNH-4-Pym | 1-Me-4-(3,4-deH-Pip) |
| 2-983 | Ph | 2-MeNH-4-Pym | 1-Et-4-Pip |
| 2-984 | Ph | 2-MeNH-4-Pym | 1-Bn-4-Pip |
| 2-985 | Ph | 2-MeNH-4-Pym | 3-Hpip |
| 2-986 | Ph | 2-MeNH-4-Pym | 1-Me-3-Hpip |
| 2-987 | Ph | 2-MeNH-4-Pym | 4-Hpip |
| 2-988 | Ph | 2-MeNH-4-Pym | 1-Me-4-Hpip |
| 2-989 | Ph | 2-MeNH-4-Pym | 2-Mor |
| 2-990 | Ph | 2-MeNH-4-Pym | 1-Me-2-Mor |
| 2-991 | Ph | 2-MeNH-4-Pym | 2-Tmor |
| 2-992 | Ph | 2-MeNH-4-Pym | 1-Me-2-Tmor |
| 2-993 | Ph | 2-MeNH-4-Pym | 1-Piz |
| 2-994 | Ph | 2-MeNH-4-Pym | 4-Me-1-Piz |
| 2-995 | Ph | 2-MeNH-4-Pym | 2-Piz |
| 2-996 | Ph | 2-MeNH-4-Pym | 4-Pyr |
| 2-997 | Ph | 2-MeNH-4-Pym | 3-Pyr |
| 2-998 | Ph | 2-MeNH-4-Pym | 2-Pyr |

Table 2 (continued)

| Compound No. | $R^1$ | $R^2$ | $R^3$ |
|---|---|---|---|
| 2-999 | Ph | 2-MeNH-4-Pym | 4-Pym |
| 2-1000 | Ph | 2-MeNH-4-Pym | 5-Pym |
| 2-1001 | Ph | 2-MeNH-4-Pym | 2-Pym |
| 2-1002 | Ph | 2-MeNH-4-Pym | 3-Azt-$CH_2$ |
| 2-1003 | Ph | 2-MeNH-4-Pym | 1-Me-3-Azt-$CH_2$ |
| 2-1004 | Ph | 2-MeNH-4-Pym | 3-Pyrd-$CH_2$ |
| 2-1005 | Ph | 2-MeNH-4-Pym | 1-Me-3-Pyrd-$CH_2$ |
| 2-1006 | Ph | 2-MeNH-4-Pym | 4-Pip-$CH_2$ |
| 2-1007 | Ph | 2-MeNH-4-Pym | 1-Me-4-Pip-$CH_2$ |
| 2-1008 | Ph | 2-MeNH-4-Pym | 3-Hpip-$CH_2$ |
| 2-1009 | Ph | 2-MeNH-4-Pym | 1-Me-3-Hpip-$CH_2$ |
| 2-1010 | Ph | 2-MeNH-4-Pym | 4-Hpip-$CH_2$ |
| 2-1011 | Ph | 2-MeNH-4-Pym | 1-Me-4-Hpip-$CH_2$ |
| 2-1012 | Ph | 2-MeNH-4-Pym | 2-Mor-$CH_2$ |
| 2-1013 | Ph | 2-MeNH-4-Pym | 1-Me-2-Mor-$CH_2$ |
| 2-1014 | Ph | 2-MeNH-4-Pym | 2-Tmor-$CH_2$ |
| 2-1015 | Ph | 2-MeNH-4-Pym | 1-Me-2-Tmor-$CH_2$ |
| 2-1016 | Ph | 2-MeNH-4-Pym | 1-Piz-$CH_2$ |
| 2-1017 | Ph | 2-MeNH-4-Pym | 4-Me-1-Piz-$CH_2$ |
| 2-1018 | Ph | 2-MeNH-4-Pym | 2-Piz-$CH_2$ |
| 2-1019 | Ph | 2-MeNH-4-Pym | 4-Pyr-$CH_2$ |
| 2-1020 | Ph | 2-MeNH-4-Pym | 3-Pyr-$CH_2$ |
| 2-1021 | Ph | 2-MeNH-4-Pym | 2-Pyr-$CH_2$ |
| 2-1022 | Ph | 2-MeNH-4-Pym | 4-Pym-$CH_2$ |
| 2-1023 | Ph | 2-MeNH-4-Pym | 5-Pym-$CH_2$ |
| 2-1024 | Ph | 2-MeNH-4-Pym | 2-Pym-$CH_2$ |
| 2-1025 | 4-F-Ph | 2-MeNH-4-Pym | $H_2N$-$CH_2$ |
| 2-1026 | 4-F-Ph | 2-MeNH-4-Pym | $H_2N$-$(CH_2)_2$ |
| 2-1027 | 4-F-Ph | 2-MeNH-4-Pym | $H_2N$-$(CH_2)_3$ |
| 2-1028 | 4-F-Ph | 2-MeNH-4-Pym | $H_2N$-$(CH_2)_4$ |
| 2-1029 | 4-F-Ph | 2-MeNH-4-Pym | MeNH-$CH_2$ |
| 2-1030 | 4-F-Ph | 2-MeNH-4-Pym | MeNH-$(CH_2)_2$ |
| 2-1031 | 4-F-Ph | 2-MeNH-4-Pym | MeNH-$(CH_2)_3$ |
| 2-1032 | 4-F-Ph | 2-MeNH-4-Pym | MeNH-$(CH_2)_4$ |
| 2-1033 | 4-F-Ph | 2-MeNH-4-Pym | EtNH-$CH_2$ |
| 2-1034 | 4-F-Ph | 2-MeNH-4-Pym | EtNH-$(CH_2)_2$ |
| 2-1035 | 4-F-Ph | 2-MeNH-4-Pym | EtNH-$(CH_2)_3$ |
| 2-1036 | 4-F-Ph | 2-MeNH-4-Pym | EtNH-$(CH_2)_4$ |

Table 2 (continued)

| Compound No. | $R^1$ | $R^2$ | $R^3$ |
|---|---|---|---|
| 2-1037 | 4-F-Ph | 2-MeNH-4-Pym | $Me_2N$-$CR_2$ |
| 2-1038 | 4-F-Ph | 2-MeNH-4-Pym | $Me_2N$-$(CH_2)_2$ |
| 2-1039 | 4-F-Ph | 2-MeNH-4-Pym | $Me_2N$- $(CH_2)_3$ |
| 2-1040 | 4-F-Ph | 2-MeNH-4-Pym | $Me_2N$-$(CH_2)_4$ |
| 2-1041 | 4-F-Ph | 2-MeNH-4-Pym | 1-Azt-$CH_2$ |
| 2-1042 | 4-F-Ph | 2-MeNH-4-Pym | 1-Azt-$(CH_2)_2$ |
| 2-1043 | 4-F-Ph | 2-MeNH-4-Pym | 1-Azt-$(CH_2)_3$ |
| 2-1044 | 4-F-Ph | 2-MeNH-4-Pym | 1-Azt-$(CH_2)_4$ |
| 2-1045 | 4-F-Ph | 2-MeNH-4-Pym | 1-pyrd-$CH_2$ |
| 2-1046 | 4-F-Ph | 2-MeNH-4-Pym | 1-Pyrd-$(CH_2)_2$ |
| 2-1047 | 4-F-Ph | 2-MeNH-4-Pym | 1-Pyrd-$(CH_2)_3$ |
| 2-1048 | 4-F-Ph | 2-MeNH-4-Pym | 1-Pyrd-$(CH_2)_4$ |
| 2-1049 | 4-F-Ph | 2-MeNH-4-Pym | 1-Pip-$CH_2$ |
| 2-1050 | 4-F-Ph | 2-MeNH-4-Pym | 1-Pip-$(CH_2)_2$ |
| 2-1051 | 4-F-Ph | 2-MeNH-4-Pym | 1-Pip-$(CH_2)_3$ |
| 2-1052 | 4-F-Ph | 2-MeNH-4-Pym | 1-Pip-$(CH_2)_4$ |
| 2-1053 | 4-F-Ph | 2-MeNH-4-Pym | 1-Mor-$CH_2$ |
| 2-1054 | 4-F-Ph | 2-MeNH-4-Pym | 1-Mor-$(CH_2)_2$ |
| 2-1055 | 4-F-Ph | 2-MeNH-4-Pym | 1-Mor-$(CH_2)_3$ |
| 2-1056 | 4-F-Ph | 2-MeNH-4-Pym | 1-Mor-$(CH_2)_4$ |
| 2-1057 | 4-F-Ph | 2-MeNH-4-Pym | 1-Tmor-$CH_2$ |
| 2-1058 | 4-F-Ph | 2-MeNH-4-Pym | 1-Tmor-$(CH_2)_2$ |
| 2-1059 | 4-F-Ph | 2-MeNH-4-Pym | 1-Tmor-$(CH_2)_3$ |
| 2-1060 | 4-F-Ph | 2-MeNH-4-Pym | 1-Tmor-$(CH_2)_4$ |
| 2-1061 | 4-F-Ph | 2-MeNH-4-Pym | 1-Piz-$CH_2$ |
| 2-1062 | 4-F-Ph | 2-MeNH-4-Pym | 1-Piz-$(CH_2)_2$ |
| 2-1063 | 4-F-Ph | 2-MeNH-4-Pym | 1-Piz-$(CH_2)_3$ |
| 2-1064 | 4-F-Ph | 2-MeNH-4-Pym | 4-Me-1-Piz-$(CH_2)_3$ |
| 2-1065 | 4-F-Ph | 2-MeNH-4-Pym | 1-Piz-$(CH_2)_4$ |
| 2-1066 | 4-F-Ph | 2-MeNH-4-Pym | 3-Azt |
| 2-1067 | 4-F-Ph | 2-MeNH-4-Pym | 1-Me-3-Azt |
| 2-1068 | 4-F-Ph | 2-MeNH-4-Pym | 1-Bn-3-Azt |
| 2-1069 | 4-F-Ph | 2-MeNH-4-Pym | 3-Pyrd |
| 2-1070 | 4-F-Ph | 2-MeNH-4-Pym | 1-Me-3-Pyrd |
| 2-1071 | 4-F-Ph | 2-MeNH-4-Pym | 3-Pip |
| 2-1072 | 4-F-Ph | 2-MeNH-4-Pym | 4-Pip |
| 2-1073 | 4-F-Ph | 2-MeNH-4-Pym | 4-(3,4-deH-Pip) |
| 2-1074 | 4-F-Ph | 2-MeNH-4-Pym | 1-Me-4-Pip |

Table 2 (continued)

| Compound No. | $R^1$ | $R^2$ | $R^3$ |
|---|---|---|---|
| 2-1075 | 4-F-Ph | 2-MeNH-4-Pym | 1-Me-4-(3,4-deH-Pip) |
| 2-1076 | 4-F-Ph | 2-MeNH-4-Pym | 1-Et-4-Pip |
| 2-1077 | 4-F-Ph | 2-MeNH-4-Pym | 1-Bn-4-Pip |
| 2-1078 | 4-F-Ph | 2-MeNH-4-Pym | 3-Hpip |
| 2-1079 | 4-F-Ph | 2-MeNH-4-Pym | 1-Me-3-lipip |
| 2-1080 | 4-F-Ph | 2-MeNH-4-Pym | 4-Hpip |
| 2-1081 | 4-F-Ph | 2-MeNH-4-Pym | 1-Me-4-Hpip |
| 2-1082 | 4-F-Ph | 2-MeNH-4-Pym | 2-Mor |
| 2-1083 | 4-F-Ph | 2-MeNH-4-Pym | 1-Me-2-Mor |
| 2-1084 | 4-F-Ph | 2-MeNH-4-Pym | 2-Tmor |
| 2-1085 | 4-F-Ph | 2-MeNH-4-Pym | 1-Me-2-Tmor |
| 2-1086 | 4-F-Ph | 2-MeNH-4-Pym | 1-Piz |
| 2-1087 | 4-F-Ph | 2-MeNH-4-Pym | 4-Me-1-Piz |
| 2-1088 | 4-F-Ph | 2-MeNH-4-Pym | 2-Piz |
| 2-1089 | 4-F-Ph | 2-MeNH-4-Pym | 4-Pyr |
| 2-1090 | 4-F-Ph | 2-MeNH-4-Pym | 3-Pyr |
| 2-1091 | 4-F-Ph | 2-MeNH-4-Pym | 2-Pyr |
| 2-1092 | 4-F-Ph | 2-MeNH-4-Pym | 4-Pym |
| 2-1093 | 4-F-Ph | 2-MeNH-4-Pym | 5-Pym |
| 2-1094 | 4-F-Ph | 2-MeNH-4-Pym | 2-Pym |
| 2-1095 | 4-F-Ph | 2-MeNH-4-Pym | 3-Azt-$CH_2$ |
| 2-1096 | 4-F-Ph | 2-MeNH-4-Pym | 1-Me-3-Azt-$CH_2$ |
| 2-1097 | 4-F-Ph | 2-MeNH-4-Pym | 3-Pyrd-$CH_2$ |
| 2-1098 | 4-F-Ph | 2-MeNH-4-Pym | 1-Me-3-Pyrd-$CH_2$ |
| 2-1099 | 4-F-Ph | 2-MeNH-4-Pym | 4-Pip-$CH_2$ |
| 2-1100 | 4-F-Ph | 2-MeNH-4-Pym | 1-Me-4-Pip-$CH_2$ |
| 2-1101 | 4-F-Ph | 2-MeNH-4-Pym | 3-Hpip-$CH_2$ |
| 2-1102 | 4-F-Ph | 2-MeNH-4-Pym | 1-Me-3-Hpip-$CH_2$ |
| 2-1103 | 4-F-Ph | 2-MeNH-4-Pym | 4-Hpip-$CH_2$ |
| 2-1104 | 4-F-Ph | 2-MeNH-4-Pym | 1-Me-4-Hpip-$CH_2$ |
| 2-1105 | 4-F-Ph | 2-MeNH-4-Pym | 2-Mor-$CH_2$ |
| 2-1106 | 4-F-Ph | 2-MeNH-4-Pym | 1-Me-2-Mor-$CH_2$ |
| 2-1107 | 4-F-Ph | 2-MeNH-4-Pym | 2-Tmor-$CH_2$ |
| 2-1108 | 4-F-Ph | 2-MeNH-4-Pym | 1-Me-2-Tmor-$CH_2$ |
| 2-1109 | 4-F-Ph | 2-MeNH-4-Pym | 1-Piz-$CH_2$ |
| 2-1110 | 4-F-Ph | 2-MeNH-4-Pym | 4-Me-1-Piz-$CH_2$ |
| 2-1111 | 4-F-Ph | 2-MeNH-4-Pym | 2-Piz-$CH_2$ |
| 2-1112 | 4-F-Ph | 2-MeNH-4-Pym | 4-Pyr-$CH_2$ |

Table 2 (continued)

| Compound No. | $R^1$ | $R^2$ | $R^3$ |
|---|---|---|---|
| 2-1113 | 4-F-Ph | 2-MeNH-4-Pym | 3-Pyr-$CH_2$ |
| 2-1114 | 4-F-Ph | 2-MeNH-4-Pym | 2-Pyr-$CH_2$ |
| 2-1115 | 4-F-Ph | 2-MeNH-4-Pym | 4-Pym-$CH_2$ |
| 2-1116 | 4-F-Ph | 2-MeNH-4-Pym | 5-Pym-$CH_2$ |
| 2-1117 | 4-F-Ph | 2-MeNH-4-Pym | 2-Pym-$CH_2$ |
| 2-1118 | 3-F-Ph | 2-MeNH-4-Pym | $H_2N$-$CH_2$ |
| 2-1119 | 3-F-Ph | 2-MeNH-4-Pym | $H_2N$-$(CH_2)_2$ |
| 2-1120 | 3-F-Ph | 2-MeNH-4-Pym | $H_2N$-$(CH_2)_3$ |
| 2-1121 | 3-F-Ph | 2-MeNH-4-Pym | $H_2N$-$(CH_2)_4$ |
| 2-1122 | 3-F-Ph | 2-MeNH-4-Pym | MeNH-$CH_2$ |
| 2-1123 | 3-F-Ph | 2-MeNH-4-Pym | MeNH-$(CH_2)_2$ |
| 2-1124 | 3-F-Ph | 2-MeNH-4-Pym | MeNH-$(CH_2)_3$ |
| 2-1125 | 3-F-Ph | 2-MeNH-4-Pym | MeNH-$(CH_2)_4$ |
| 2-1126 | 3-F-Ph | 2-MeNH-4-Pym | EtNH-$CH_2$ |
| 2-1127 | 3-F-Ph | 2-MeNH-4-Pym | EtNH-$(CH_2)_2$ |
| 2-1128 | 3-F-Ph | 2-MeNH-4-Pym | EtNH-$(CH_2)_3$ |
| 2-1129 | 3-F-Ph | 2-MeNH-4-Pym | EtNH-$(CH_2)_4$ |
| 2-1130 | 3-F-Ph | 2-MeNH-4-Pym | $Me_2N$-$CH_2$ |
| 2-1131 | 3-F-Ph | 2-MeNH-4-Pym | $Me_2N$-$(CH_2)_2$ |
| 2-1132 | 3-F-Ph | 2-MeNH-4-Pym | $Me_2N$-$(CH_2)_3$ |
| 2-1133 | 3-F-Ph | 2-MeNH-4-Pym | $Me_2N$-$(CH_2)_4$ |
| 2-1134 | 3-F-Ph | 2-MeNH-4-Pym | 1-Azt-$CH_2$ |
| 2-1135 | 3-F-Ph | 2-MeNH-4-Pym | 1-Azt-$(CH_2)_2$ |
| 2-1136 | 3-F-Ph | 2-MeNH-4-Pym | 1-Azt-$(CH_2)_3$ |
| 2-1137 | 3-F-Ph | 2-MeNH-4-Pym | 1-Azt-$(CH_2)_4$ |
| 2-1138 | 3-F-Ph | 2-MeNH-4-Pym | 1-Pyrd-$CH_2$ |
| 2-1139 | 3-F-Ph | 2-MeNH-4-Pym | 1-Pyrd-$(CH_2)_2$ |
| 2-1140 | 3-F-Ph | 2-MeNH-4-Pym | 1-Pyrd-$(CH_2)_3$ |
| 2-1141 | 3-F-Ph | 2-MeNH-4-Pym | 1-Pyrd-$(CH_2)_4$ |
| 2-1142 | 3-F-Ph | 2-MeNH-4-Pym | 1-Pip-$CH_2$ |
| 2-1143 | 3-F-Ph | 2-MeNH-4-Pym | 1-Pip-$(CH_2)_2$ |
| 2-1144 | 3-F-Ph | 2-MeNH-4-Pym | 1-Pip-$(CH_2)_3$ |
| 2-1145 | 3-F-Ph | 2-MeNH-4-Pym | 1-Pip-$(CH_2)_4$ |
| 2-1146 | 3-F-Ph | 2-MeNH-4-Pym | 1-Mor-$CH_2$ |
| 2-1147 | 3-F-Ph | 2-MeNH-4-Pym | 1-Mor-$(CH_2)_2$ |
| 2-1148 | 3-F-Ph | 2-MeNH-4-Pym | 1-Mor-$(CH_2)_3$ |
| 2-1149 | 3-F-Ph | 2-MeNH-4-Pym | 1-Mor-$(CH_2)_4$ |
| 2-1150 | 3-F-Ph | 2-MeNH-4-Pym | 1-Tmor-$CH_2$ |

Table 2 (continued)

| Compound No. | $R^1$ | $R^2$ | $R^3$ |
|---|---|---|---|
| 2-1151 | 3-F-Ph | 2-MeNH-4-Pym | 1-Tmor-$(CH_2)_2$ |
| 2-1152 | 3-F-Ph | 2-MeNH-4-Pym | 1-Tmor-$(CH_2)_3$ |
| 2-1153 | 3-F-Ph | 2-MeNH-4-Pym | 1-Tmor-$(CH_2)_4$ |
| 2-1154 | 3-F-Ph | 2-MeNH-4-Pym | 1-Piz-$CH_2$ |
| 2-1155 | 3-F-Ph | 2-MeNH-4-Pym | 1-Piz-$(CH_2)_2$ |
| 2-1156 | 3-F-Ph | 2-MeNH-4-Pym | 1-Piz-$(CH_2)_3$ |
| 2-1157 | 3-F-Ph | 2-MeNH-4-Pym | 4-Me-1-Piz-$(CH_2)_3$ |
| 2-1158 | 3-F-Ph | 2-MeNH-4-Pym | 1-Piz-$(CH_2)_4$ |
| 2-1159 | 3-F-Ph | 2-MeNH-4-Pym | 3-Azt |
| 2-1160 | 3-F-Ph | 2-MeNH-4-Pym | 1-Me-3-A |
| 2-1161 | 3-F-Ph | 2-MeNH-4-Pym | 1-Bn-3-Azt |
| 2-1162 | 3-F-Ph | 2-MeNH-4-Pym | 3-Pyrd |
| 2-1163 | 3-F-Ph | 2-MeNH-4-Pym | 1-Me-3-Pyrd |
| 2-1164 | 3-F-Ph | 2-MeNH-4-Pym | 3-Pip |
| 2-1165 | 3-F-Ph | 2-MeNH-4-Pym | 4-Pip |
| 2-1166 | 3-F-Ph | 2-MeNH-4-Pym | 4-(3,4-deH-Pip) |
| 2-1167 | 3-F-Ph | 2-MeNH-4-Pym | 1-Me-4-Pip |
| 2-1168 | 3-F-Ph | 2-MeNH-4-Pym | 1-Me-4-(3,4-deH-Pip) |
| 2-1169 | 3-F-Ph | 2-MeNH-4-Pym | 1-Et-4-Pip |
| 2-1170 | 3-F-Ph | 2-MeNH-4-Pym | 1-Bn-4-Pip |
| 2-1171 | 3-F-Ph | 2-MeNH-4-Pym | 3-Hpip |
| 2-1172 | 3-F-Ph | 2-MeNH-4-Pym | 1-Me-3-Hpip |
| 2-1173 | 3-F-Ph | 2-MeNH-4-Pym | 4-Hpip |
| 2-1174 | 3-F-Ph | 2-MeNH-4-Pym | 1-Me-4-Hpip |
| 2-1175 | 3-F-Ph | 2-MeNH-4-Pym | 2-Mor |
| 2-1176 | 3-F-Ph | 2-MeNH-4-Pym | 1-Me-2-Mor |
| 2-1177 | 3-F-Ph | 2-MeNH-4-Pym | 2-Tmor |
| 2-1178 | 3-F-Ph | 2-MeNH-4-Pym | 1-Me-2-Tmor |
| 2-1179 | 3-F-Ph | 2-MeNH-4-Pym | 1-Piz |
| 2-1180 | 3-F-Ph | 2-MeNH-4-Pym | 4-Me-1-Piz |
| 2-1181 | 3-F-Ph | 2-MeNH-4-Pym | 2-Piz |
| 2-1182 | 3-F-Ph | 2-MeNH-4-Pym | 4-Pyr |
| 2-1183 | 3-F-Ph | 2-MeNH-4-Pym | 3-Pyr |
| 2-1184 | 3-F-Ph | 2-MeNH-4-Pym | 2-Pyr |
| 2-1185 | 3-F-Ph | 2-MeNH-4-Pym | 4-Pym |
| 2-1186 | 3-F-Ph | 2-MeNH-4-Pym | 5-Pym |
| 2-1187 | 3-F-Ph | 2-MeNH-4-Pym | 2-Pym |
| 2-1188 | 3-F-Ph | 2-MeNH-4-Pym | 3-Azt-$CH_2$ |

Table 2 (continued)

| Compound No. | $R^1$ | $R^2$ | $R^3$ |
|---|---|---|---|
| 2-1189 | 3-F-Ph | 2-MeNH-4-Pym | 1-Me-3-Azt-$CH_2$ |
| 2-1190 | 3-F-Ph | 2-MeNH-4-Pym | 3-Pyrd-$CH_2$ |
| 2-1191 | 3-F-Ph | 2-MeNH-4-Pym | 1-Me-3-Pyrd-$CH_2$ |
| 2-1192 | 3-F-Ph | 2-MeNH-4-Pym | 4-Pip-$CH_2$ |
| 2-1193 | 3-F-Ph | 2-MeNH-4-Pym | 1-Me-4-Pip-$CH_2$ |
| 2-1194 | 3-F-Ph | 2-MeNH-4-Pym | 3-Hpip-$CH_2$ |
| 2-1195 | 3-F-Ph | 2-MeNH-4-Pyrn | 1-Me-3-Hpip-$CH_2$ |
| 2-1196 | 3-F-Ph | 2-MeNH-4-Pym | 4-Hpip-$CH_2$ |
| 2-1197 | 3-F-Ph | 2-MeNH-4-Pym | 1-Me-4-Hpip-$CH_2$ |
| 2-1198 | 3-F-Ph | 2-MeNH-4-Pym | 2-Mor-$CH_2$ |
| 2-1199 | 3-F-Ph | 2-MeNH-4-Pym | 1-Me-2-Mor-$CH_2$ |
| 2-1200 | 3-F-Ph | 2-MeNH-4-Pym | 2-Tmor-$CH_2$ |
| 2-1201 | 3-F-Ph | 2-MeNH-4-Pym | 1-Me-2-Tmor-$CH_2$ |
| 2-1202 | 3-F-Ph | 2-MeNH-4-Pym | 1-Piz-$CH_2$ |
| 2-1203 | 3-F-Ph | 2-MeNH-4-Pym | 4-Me-1-Piz-$CH_2$ |
| 2-1204 | 3-F-Ph | 2-MeNH-4-Pym | 2-Piz-$CH_2$ |
| 2-1205 | 3-F-Ph | 2-MeNH-4-Pym | 4-Pyr-$CH_2$ |
| 2-1206 | 3-F-Ph | 2-MeNH-4-Pym | 3-Pyr-$CH_2$ |
| 2-1207 | 3-F-Ph | 2-MeNH-4-Pym | 2-Pyr-$CH_2$ |
| 2-1208 | 3-F-Ph | 2-MeNH-4-Pym | 4-Pym-$CH_2$ |
| 2-1209 | 3-F-Ph | 2-MeNH-4-Pym | 5-Pym-$CH_2$ |
| 2-1210 | 3-F-Ph | 2-MeNH-4-Pym | 2-Pym-$CH_2$ |
| 2-1211 | 3,4-diF-Ph | 2-MeNH-4-Pym | $H_2N$-$CH_2$ |
| 2-1212 | 3,4-diF-Ph | 2-MeNH-4-Pym | $H_2N$-$(CH_2)_2$ |
| 2-1213 | 3,4-diF-Ph | 2-MeNH-4-Pym | $H_2N$-$(CH_2)_3$ |
| 2-1214 | 3,4-diF-Ph | 2-MeNH-4-Pym | $H_2N$-$(CH_2)_4$ |
| 2-1215 | 3,4-diF-Ph | 2-MeNH-4-Pym | MeNH-$CH_2$ |
| 2-1216 | 3,4-diF-Ph | 2-MeNH-4-Pym | MeNH-$(CH_2)_2$ |
| 2-1217 | 3,4-diF-Ph | 2-MeNH-4-Pym | MeNH-$(CH_2)_3$ |
| 2-1218 | 3,4-diF-Ph | 2-MeNH-4-Pym | MeNH-$(CH_2)_4$ |
| 2-1219 | 3,4-diF-Ph | 2-MeNH-4-Pym | EtNH-$CH_2$ |
| 2-1220 | 3,4-diF-Ph | 2-MeNH-4-Pym | EtNH-$(CH_2)_2$ |
| 2-1221 | 3,4-diF-Ph | 2-MeNH-4-Pym | EtNH-$(CH_2)_3$ |
| 2-1222 | 3,4-diF-Ph | 2-MeNH-4-Pym | EtNH-$(CH_2)_4$ |
| 2-1223 | 3,4-diF-Ph | 2-MeNH-4-Pym | $Me_2N$-$CH_2$ |
| 2-1224 | 3,4-diF-Ph | 2-MeNH-4-Pym | $Me_2N$-$(CH_2)_2$ |
| 2-1225 | 3,4-diF-Ph | 2-MeNH-4-Pym | $Me_2N$-$(CH_2)_3$ |
| 2-1226 | 3,4-diF-Ph | 2-MeNH-4-Pym | $Me_2N$-$(CH_2)_4$ |

Table 2 (continued)

| Compound No. | $R^1$ | $R^2$ | $R^3$ |
|---|---|---|---|
| 2-1227 | 3,4-diF-Ph | 2-MeNH-4-Pym | 1-Azt-$CH_2$ |
| 2-1228 | 3,4-diF-Ph | 2-MeNH-4-Pym | 1-Azt-$(CH_2)_2$ |
| 2-1229 | 3,4-diF-Ph | 2-MeNH-4-Pym | 1-Azt-$(CH_2)_3$ |
| 2-1230 | 3,4-diF-Ph | 2-MeNH-4-Pym | 1-Azt-$(CH_2)_4$ |
| 2-1231 | 3,4-diF-Ph | 2-MeNH-4-Pym | 1-Pyrd-$CH_2$ |
| 2-1232 | 3,4-diF-Ph | 2-MeNH-4-Pym | 1-Pyrd-$(CH_2)_2$ |
| 2-1233 | 3,4-diF-Ph | 2-MeNH-4-Pym | 1-Pyrd-$(CH_2)_3$ |
| 2-1234 | 3,4-diF-Ph | 2-MeNH-4-Pym | 1-Pyrd-$(CH_2)_4$ |
| 2-1235 | 3,4-diF-Ph | 2-MeNH-4-Pym | 1-Pip-$CH_2$ |
| 2-1236 | 3,4-diF-Ph | 2-MeNH-4-Pym | 1-Pip-$(CH_2)_2$ |
| 2-1237 | 3,4-diF-Ph | 2-MeNH-4-Pym | 1-Pip-$(CH_2)_3$ |
| 2-1238 | 3,4-diF-Ph | 2-MeNH-4-Pym | 1-Pip-$(CH_2)_4$ |
| 2-1239 | 3,4-diF-Ph | 2-MeNH-4-Pym | 1-Mor-$CH_2$ |
| 2-1240 | 3,4-diF-Ph | 2-MeNH-4-Pym | 1-Mor-$(CH_2)_2$ |
| 2-1241 | 3,4-diF-Ph | 2-MeNH-4-Pym | 1-Mor-$(CH_2)_3$ |
| 2-1242 | 3,4-diF-Ph | 2-MeNH-4-Pym | 1-Mor-$(CH_2)_4$ |
| 2-1243 | 3,4-diF-Ph | 2-MeNH-4-Pym | 1-Tmor-$CH_2$ |
| 2-1244 | 3,4-diF-Ph | 2-MeNH-4-Pym | 1-Tmor-$(CH2)_2$ |
| 2-1245 | 3,4-diF-Ph | 2-MeNH-4-Pym | 1-Tmor-$(CH_2)_3$ |
| 2-1246 | 3,4-diF-Ph | 2-MeNH-4-Pym | 1-Tmor-$(CH_2)_4$ |
| 2-1247 | 3,4-diF-Ph | 2-MeNH-4-Pym | 1-Piz-$CH_2$ |
| 2-1248 | 3,4-diF-Ph | 2-MeNH-4-Pym | 1-Piz-$(CH_2)_2$ |
| 2-1249 | 3,4-diF-Ph | 2-MeNH-4-Pym | 1-Piz-$(CH_2)_3$ |
| 2-1250 | 3,4-diF-Ph | 2-MeNH-4-Pym | 4-Me-1-Piz-$(CH_2)_3$ |
| 2-1251 | 3,4-diF-Ph | 2-MeNH-4-Pym | 1-Piz-$(CH_2)_4$ |
| 2-1252 | 3,4-diF-Ph | 2-MeNH-4-Pym | 3-Azt |
| 2-1253 | 3,4-diF-Ph | 2-MeNH-4-Pym | 1-Me-3-Azt |
| 2-1254 | 3,4-diF-Ph | 2-MeNH-4-Pym | 1-Bn-3-Azt |
| 2-1255 | 3,4-diF-Ph | 2-MeNH-4-Pym | 3-Pyrd |
| 2-1256 | 3,4-diF-Ph | 2-MeNH-4-Pym | 1-Me-3-Pyrd |
| 2-1257 | 3,4-diF-Ph | 2-MeNH-4-Pym | 3-Pip |
| 2-1258 | 3,4-diF-Ph | 2-MeNH-4-Pym | 4-Pip |
| 2-1259 | 3,4-diF-Ph | 2-MeNH-4-Pym | 4-(3,4-deH-Pip) |
| 2-1260 | 3,4-diF-Ph | 2-MeNH-4-Pym | 1-Me-4-Pip |
| 2-1261 | 3,4-diF-Ph | 2-MeNH-4-Pym | 1-Me-4-(3,4-deH-Pip) |
| 2-1262 | 3,4-diF-Ph | 2-MeNH-4-Pym | 1-Et-4-Pip |
| 2-1263 | 3,4-diF-Ph | 2-MeNH-4-Pym | 1-Bn-4-Pip |
| 2-1264 | 3,4-diF-Ph | 2-MeNH-4-Pym | 3-Hpip |

Table 2 (continued)

| Compound No. | $R^1$ | $R^2$ | $R^3$ |
|---|---|---|---|
| 2-1265 | 3,4-diF-Ph | 2-MeNH-4-Pym | 1-Me-3-Hpip |
| 2-1266 | 3,4-diF-Ph | 2-MeNH-4-Pym | 4-Hpip |
| 2-1267 | 3,4-diF-Ph | 2-MeNH-4-Pym | 1-Me-4-Hpip |
| 2-1268 | 3,4-diF-Ph | 2-MeNH-4-Pym | 2-Mor |
| 2-1269 | 3,4-diF-Ph | 2-MeNH-4-Pym | 1-Me-2-Mor |
| 2-1270 | 3,4-diF-Ph | 2-MeNH-4-Pym | 2-Tmor |
| 2-1271 | 3,4-diF-Ph | 2-MeNH-4-Pym | 1-Me-2-Tmor |
| 2-1272 | 3,4-diF-Ph | 2-MeNH-4-Pym | 1-Piz |
| 2-1273 | 3,4-diF-Ph | 2-MeNH-4-Pym | 4-Me-1-Piz |
| 2-1274 | 3,4-diF-Ph | 2-MeNH-4-Pym | 2-Piz |
| 2-1275 | 3,4-diF-Ph | 2-MeNH-4-Pym | 4-Pyr |
| 2-1276 | 3,4-diF-Ph | 2-MeNH-4-Pym | 3-Pyr |
| 2-1277 | 3,4-diF-Ph | 2-MeNH-4-Pym | 2-Pyr |
| 2-1278 | 3,4-diF-Ph | 2-MeNH-4-Pym | 4-Pym |
| 2-1279 | 3,4-diF-Ph | 2-MeNH-4-Pym | 5-Pym |
| 2-1280 | 3,4-diF-Ph | 2-MeNH-4-Pym | 2-Pym |
| 2-1281 | 3,4-diF-Ph | 2-MeNH-4-Pym | 3-Azt-$CH_2$ |
| 2-1282 | 3,4-diF-Ph | 2-MeNH-4-Pym | 1-Me-3-Azt-$CH_2$ |
| 2-1283 | 3,4-diF-Ph | 2-MeNH-4-Pym | 3-Pyrd-$CH_2$ |
| 2-1281 | 3,4-diF-Ph | 2-MeNH-4-Pym | 1-Me-3-Pyrd-$CH_2$ |
| 2-1285 | 3,4-diF-Ph | 2-MeNH-4-Pym | 4-Pip-$CH_2$ |
| 2-1286 | 3,4-diF-Ph | 2-MeNH-4-Pym | 1-Me-4-Pip-$CH_2$ |
| 2-1287 | 3,4-diF-Ph | 2-MeNH-4-Pym | 3-Hpip-$CH_2$ |
| 2-1288 | 3,4-diF-Ph | 2-MeNH-4-Pym | 1-Me-3-Hpip-$CH_2$ |
| 2-1289 | 3,4-diF-Ph | 2-MeNH-4-Pym | 4-Hpip-$CH_2$ |
| 2-1290 | 3,4-diF-Ph | 2-MeNH-4-Pym | 1-Me-4-Hpip-$CH_2$ |
| 2-1291 | 3,4-diF-Ph | 2-MeNH-4-Pym | 2-Mor-$CH_2$ |
| 2-1292 | 3,4-diF-Ph | 2-MeNH-4-Pym | 1-Me-2-Mor-$CH_2$ |
| 2-1293 | 3,4-diF-Ph | 2-MeNH-4-Pym | 2-Tmor-$CH_2$ |
| 2-1294 | 3,4-diF-Ph | 2-MeNH-4-Pym | 1-Me-2-Tmor-$CH_2$ |
| 2-1295 | 3,4-diF-Ph | 2-MeNH-4-Pym | 1-Piz-$CH_2$ |
| 2-1296 | 3,4-diF-Ph | 2-MeNH-4-Pym | 4-Me-1-Piz-$CH_2$ |
| 2-1297 | 3,4-diF-Ph | 2-MeNH-4-Pym | 2-Piz-$CH_2$ |
| 2-1298 | 3,4-diF-Ph | 2-MeNH-4-Pym | 4-Pyr-$CH_2$ |
| 2-1299 | 3,4-diF-Ph | 2-MeNH-4-Pym | 3-Pyr-$CH_2$ |
| 2-1300 | 3,4-diF-Ph | 2-MeNH-4-Pym | 2-Pyr-$CH_2$ |
| 2-1301 | 3,4-diF-Ph | 2-MeNH-4-Pym | 4-Pym-$CH_2$ |
| 2-1302 | 3,4-diF-Ph | 2-MeNH-4-Pym | 5-Pym-$CH_2$ |

Table 2 (continued)

| Compound No. | $R^1$ | $R^2$ | $R^3$ |
|---|---|---|---|
| 2-1303 | 3,4-diF-Ph | 2-MeNH-4-Pym | 2-Pym-$CH_2$ |
| 2-1304 | 3-Cl-Ph | 2-MeNH-4-Pym | $H_2N$-$CH_2$ |
| 2-1305 | 3-Cl-Ph | 2-MeNH-4-Pym | $H_2N$-$(CH_2)_2$ |
| 2-1306 | 3-Cl-Ph | 2-MeNH-4-Pym | $H_2N$-$(CH_2)_3$ |
| 2-1307 | 3-Cl-Ph | 2-MeNH-4-Pym | $H_2N$-$(CH_2)_4$ |
| 2-1308 | 3-Cl-Ph | 2-MeNH-4-Pym | MeNH-$CH_2$ |
| 2-1309 | 3-Cl-Ph | 2-MeNH-4-Pym | MeNH-$(CH_2)_2$ |
| 2-1310 | 3-Cl-Ph | 2-MeNH-4-Pym | MeNH-$(CH_2)_3$ |
| 2-1311 | 3-Cl-Ph | 2-MeNH-4-Pym | MeNH-$(CH_2)_4$ |
| 2-1312 | 3-Cl-Ph | 2-MeNH-4-Pym | EtNH-$CH_2$ |
| 2-1313 | 3-Cl-Ph | 2-MeNH-4-Pym | EtNH-$(CH_2)_2$ |
| 2-1314 | 3-Cl-Ph | 2-MeNH-4-Pym | EtNH-$(CH_2)_3$ |
| 2-1315 | 3-Cl-Ph | 2-MeNH-4-Pym | EtNH-$(CH_2)_4$ |
| 2-1316 | 3-Cl-Ph | 2-MeNH-4-Pym | $Me_2N$-$CH_2$ |
| 2-1317 | 3-Cl-Ph | 2-MeNH-4-Pym | $Me_2N$-$(CH_2)_2$ |
| 2-1318 | 3-Cl-Ph | 2-MeNH-4-Pym | $Me_2N$-$(CH_2)$3 |
| 2-1319 | 3-Cl-Ph | 2-MeNH-4-Pym | $Me_2N$-$(CH_2)_4$ |
| 2-1320 | 3-Cl-Ph | 2-MeNH-4-Pym | 1-Azt-$CH_2$ |
| 2-1321 | 3-Cl-Ph | 2-MeNH-4-Pym | 1-Azt-$(CH_2)_2$ |
| 2-1322 | 3-Cl-Ph | 2-MeNH-4-Pym | 1-Azt-$(CH_2)_3$ |
| 2-1323 | 3-Cl-Ph | 2-MeNH-4-Pym | 1-Azt-$(CH_2)_4$ |
| 2-1324 | 3-Cl-Ph | 2-MeNH-4-Pym | 1-Pyrd-$CH_2$ |
| 2-1325 | 3-Cl-Ph | 2-MeNH-4-Pym | 2-Pyrd-$(CH_2)_2$ |
| 2-1326 | 3-Cl-Ph | 2-MeNH-4-Pym | 1-Pyrd-$(CH_2)_3$ |
| 2-1327 | 3-Cl-Ph | 2-MeNH-4-Pym | 1-pyrd-$(CH_2)_4$ |
| 2-1328 | 3-Cl-Ph | 2-MeNH-4-Pym | 1-Pip-$CH_2$ |
| 2-1329 | 3-Cl-Ph | 2-MeNH-4-Pym | 1-Pip-$(CH_2)_2$ |
| 2-1330 | 3-Cl-Ph | 2-MeNH-4-Pym | 1-Pip-$(CH_2)_3$ |
| 2-1331 | 3-Cl-Ph | 2-MeNH-4-Pym | 1-Pip-$(CH_2)_4$ |
| 2-1332 | 3-Cl-Ph | 2-MeNH-4-Pym | 1-Mor-$CH_2$ |
| 2-1333 | 3-Cl-Ph | 2-MeNH-4-Pym | 1-Mor-$(CH_2)_2$ |
| 2-1334 | 3-Cl-Ph | 2-MeNH-4-Pym | 1-Mor-$(CH_2)_3$ |
| 2-1335 | 3-Cl-Ph | 2-MeNH-4-Pym | 1-Mor-$(CH_2)_4$ |
| 2-1336 | 3-Cl-Ph | 2-MeNH-4-Pym | 1-Tmor-$CH_2$ |
| 2-1337 | 3-Cl-Ph | 2-MeNH-4-Pym | 1-Tmor-$(CH_2)_2$ |
| 2-1338 | 3-Cl-Ph | 2-MeNH-4-Pym | 1-Tmor-$(CH_2)_3$ |
| 2-1339 | 3-Cl-Ph | 2-MeNH-4-Pym | 1-Tmor-$(CH_2)_4$ |
| 2-1340 | 3-Cl-Ph | 2-MeNH-4-Pym | 1-Piz-$CH_2$ |

Table 2 (continued)

| Compound No. | $R^1$ | $R^2$ | $R^3$ |
|---|---|---|---|
| 2-1341 | 3-Cl-Ph | 2-MeNH-4-Pym | 1-Piz-$(CH_2)_2$ |
| 2-1342 | 3-Cl-Ph | 2-MeNH-4-Pym | 1-Piz-$(CH_2)_3$ |
| 2-1343 | 3-Cl-Ph | 2-MeNH-4-Pym | 4-Me-1-Piz-$(CH_2)_3$ |
| 2-1344 | 3-Cl-Ph | 2-MeNH-4-Pym | 1-Piz-$(CH_2)_4$ |
| 2-1345 | 3-Cl-Ph | 2-MeNH-4-Pym | 3-Azt |
| 2-1346 | 3-Cl-Ph | 2-MeNH-4-Pym | 1-Me-3-Azt |
| 2-1347 | 3-Cl-Ph | 2-MeNH-4-Pym | 1-Bn-3-Azt |
| 2-1348 | 3-Cl-Ph | 2-MeNH-4-Pym | 3-Pyrd |
| 2-1349 | 3-Cl-Ph | 2-MeNH-4-Pym | 1-Me-3-Pyrd |
| 2-1350 | 3-Cl-Ph | 2-MeNH-4-Pym | 3-Pip |
| 2-1351 | 3-Cl-Ph | 2-MeNH-4-Pym | 4-Pip |
| 2-1352 | 3-Cl-Ph | 2-MeNH-4-Pym | 4-(3,4-deH-Pip) |
| 2-1353 | 3-Cl-Ph | 2-MeNH-4-Pym | 1-Me-4-Pip |
| 2-1354 | 3-Cl-Ph | 2-MeNH-4-Pym | 1-Me-4-(3,4-deH-Pip) |
| 2-1355 | 3-Cl-Ph | 2-MeNH-4-Pym | 1-Et-4-Pip |
| 2-1356 | 3-Cl-Ph | 2-MeNH-4-Pym | 1-Bn-4-Pip |
| 2-1357 | 3-Cl-Ph | 2-MeNH-4-Pym | 3-Hpip |
| 2-1358 | 3-Cl-Ph | 2-MeNH-4-Pym | 1-Me-3-Hpip |
| 2-1359 | 3-Cl-Ph | 2-MeNH-4-Pym | 4-Hpip |
| 2-1360 | 3-Cl-Ph | 2-MeNH-4-Pym | 1-Me-4-Hpip |
| 2-1361 | 3-Cl-Ph | 2-MeNH-4-Pym | 2-Mor |
| 2-1362 | 3-Cl-Ph | 2-MeNH-4-Pym | 1-Me-2-Mor |
| 2-1363 | 3-Cl-Ph | 2-MeNH-4-Pym | 2-Tmor |
| 2-1364 | 3-Cl-Ph | 2-MeNH-4-Pym | 1-Me-2-Tmor |
| 2-1365 | 3-Cl-Ph | 2-MeNH-4-Pym | 1-Piz |
| 2-1366 | 3-Cl-Ph | 2-MeNH-4-Pym | 4-Me-1-Piz |
| 2-1367 | 3-Cl-Ph | 2-MeNH-4-Pym | 2-Piz |
| 2-1368 | 3-Cl-Ph | 2-MeNH-4-Pym | 4-Pyr |
| 2-1369 | 3-Cl-Ph | 2-MeNH-4-Pym | 3-Pyr |
| 2-1370 | 3-Cl-Ph | 2-MeNH-4-Pym | 2-Pyr |
| 2-1371 | 3-Cl-Ph | 2-MeNH-4-Pym | 4-Pym |
| 2-1372 | 3-Cl-Ph | 2-MeNH-4-Pym | 5-Pym |
| 2-1373 | 3-Cl-Ph | 2-MeNH-4-Pym | 2-Pym |
| 2-1374 | 3-Cl-Ph | 2-MeNH-4-Pym | 3-Azt-$CH_2$ |
| 2-1375 | 3-Cl-Ph | 2-MeNH-4-Pym | 1-Me-3-Azt-$CH_2$ |
| 2-1376 | 3-Cl-Ph | 2-MeNH-4-Pym | 3-Pyrd-$CH_2$ |
| 2-1377 | 3-Cl-Ph | 2-MeNH-4-Pym | 1-Me-3-Pyrd-$CH_2$ |
| 2-1378 | 3-Cl-Ph | 2-MeNH-4-Pym | 4-Pip-$CH_2$ |

Table 2 (continued)

| Compound No. | $R^1$ | $R^2$ | $R^3$ |
|---|---|---|---|
| 2-1379 | 3-Cl-Ph | 2-MeNH-4-Pym | 1-Me-4-Pip-$CH_2$ |
| 2-1380 | 3-Cl-Ph | 2-MeNH-4-Pym | 3-Hpip-$CH_2$ |
| 2-1381 | 3-Cl-Ph | 2-MeNH-4-Pym | 1-Me-3-Hpip-$CH_2$ |
| 2-1382 | 3-Cl-Ph | 2-MeNH-4-Pym | 4-Hpip-$CH_2$ |
| 2-1383 | 3-Cl-Ph | 2-MeNH-4-Pym | 1-Me-4-Hpip-$CH_2$ |
| 2-1384 | 3-Cl-Ph | 2-MeNH-4-Pym | 2-Mor-$CH_2$ |
| 2-1385 | 3-Cl-Ph | 2-MeNH-4-Pym | 1-Me-2-Mor-$CH_2$ |
| 2-1386 | 3-Cl-Ph | 2-MeNH-4-Pym | 2-Tmor-$CH_2$ |
| 2-1387 | 3-Cl-Ph | 2-MeNH-4-Pym | 1-Me-2-Tmor-$CH_2$ |
| 2-1388 | 3-Cl-Ph | 2-MeNH-4-Pym | 1-Piz-$CH_2$ |
| 2-1389 | 3-Cl-Ph | 2-MeNH-4-Pym | 4-Me-1-Piz-$CH_2$ |
| 2-1390 | 3-Cl-Ph | 2-MeNH-4-Pym | 2-Piz-$CH_2$ |
| 2-1391 | 3-Cl-Ph | 2-MeNH-4-Pym | 4-Pyr-$CH_2$ |
| 2-1392 | 3-Cl-Ph | 2-MeNH-4-Pym | 3-Pyr-$CH_2$ |
| 2-1393 | 3-Cl-Ph | 2-MeNH-4-Pym | 2-Pyr-$CH_2$ |
| 2-1394 | 3-Cl-Ph | 2-MeNH-4-Pym | 4-Pym-$CH_2$ |
| 2-1395 | 3-Cl-Ph | 2-MeNH-4-Pym | 5-Pym-$CH_2$ |
| 2-1396 | 3-Cl-Ph | 2-MeNH-4-Pym | 2-Pym-$CH_2$ |
| 2-1397 | 3-Cl-4-F-Ph | 4-Pyr | $H_2N$-$(CH_2)_3$ |
| 2-1398 | 3-Cl-4-F-Ph | 4-Pyr | MeNH-$(CH_2)_3$ |
| 2-1399 | 3-Cl-4-F-Ph | 4-Pyr | EtNH-$(CH_2)_3$ |
| 2-1400 | 3-Cl-4-F-Ph | 4-Pyr | $Me_2N$-$(CH_2)_3$ |
| 2-1401 | 3-Cl-4-F-Ph | 4-Pyr | 1-Azt-$(CH_2)_3$ |
| 2-1402 | 3-Cl-4-F-Ph | 4-Pyr | 1-Pyrd-$(CH_2)_3$ |
| 2-1403 | 3-Cl-4-F-Ph | 4-Pyr | 1-Pip-$(CH_2)_3$ |
| 2-1404 | 3-Cl-4-F-Ph | 4-Pyr | 1-Mor-$(CH_2)_3$ |
| 2-1405 | 3-Cl-4-F-Ph | 4-Pyr | 1-Tmor-$(CH_2)_3$ |
| 2-1406 | 3-Cl-4-F-Ph | 4-Pyr | 1-Piz-$(CH_2)_3$ |
| 2-1407 | 3-Cl-4-F-Ph | 4-Pyr | 4-Me-1-Piz-$(CH_2)_3$ |
| 2-1408 | 3-Cl-4-F-Ph | 4-Pyr | 3-Azt |
| 2-1409 | 3-Cl-4-F-Ph | 4-Pyr | 1-Me-3-Azt |
| 2-1410 | 3-Cl-4-F-Ph | 4-Pyr | 3-Pyrd |
| 2-1411 | 3-Cl-4-F-Ph | 4-Pyr | 1-Me-3-Pyrd |
| 2-1412 | 3-Cl-4-F-Ph | 4-Pyr | 4-Pip |
| 2-1413 | 3-Cl-4-F-Ph | 4-Pyr | 4-(3,4-deH-Pip) |
| 2-1414 | 3-Cl-4-F-Ph | 4-Pyr | 1-Me-4-Pip |
| 2-1415 | 3-Cl-4-F-Ph | 4-Pyr | 1-Me-4-(3,4-deH-Pip) |
| 2-1416 | 3-Cl-4-F-Ph | 4-Pyr | 1-Piz |

Table 2 (continued)

| Compound No. | $R^1$ | $R^2$ | $R^3$ |
|---|---|---|---|
| 2-1417 | 3-Cl-4-F-Ph | 4-Pyr | 4-Me-1-Piz |
| 2-1418 | 3-Cl-4-F-Ph | 4-Pyr | 4-Pyr |
| 2-1419 | 3-Cl-4-F-Ph | 4-Pyr | 3-Pyr |
| 2-1420 | 3-Cl-4-F-Ph | 4-Pyr | 4-Pym |
| 2-1421 | 3-Cl-4-F-Ph | 4-Pyr | 5-Pym |
| 2-1422 | 3-Cl-4-F-Ph | 4-Pyr | 3-Pyrd-$CH_2$ |
| 2-1423 | 3-Cl-4-F-Ph | 4-Pyr | 1-Me-3-Pyrd-$CH_2$ |
| 2-1424 | 3-Cl-4-F-Ph | 4-Pyr | 4-Pip-$CH_2$ |
| 2-1425 | 3-Cl-4-F-Ph | 4-Pyr | 1-Me-4-Pip-$CH_2$ |
| 2-1426 | 3-Cl-4-F-Ph | 4-Pyr | 2-Piz-$CH_2$ |
| 2-1427 | 3-Cl-4-F-Ph | 4-Pyr | 4-Pyr-$CH_2$ |
| 2-1428 | 3-Cl-4-F-Ph | 4-Pyr | 3-Pyr-$CH_2$ |
| 2-1429 | 3-Cl-4-F-Ph | 4-Pyr | 4-Pym-$CH_2$ |
| 2-1430 | 3-Cl-4-F-Ph | 4-Pyr | 5-Pym-$CH_2$ |
| 2-1431 | 3-Cl-4-F-Ph | 4-Pyr | 2-Pym-$CH_2$ |
| 2-1432 | 3,4,5-triF-Ph | 4-Pyr | $H_2N$-$(CH_2)_3$ |
| 2-1433 | 3,4,5-triF-Ph | 4-Pyr | MeNH-$(CH_2)_3$ |
| 2-1434 | 3,4,5-triF-Ph | 4-Pyr | EtNH-$(CH_2)_3$ |
| 2-1435 | 3,4,5-triF-Ph | 4-Pyr | $Me_2N$-$(CH_2)_3$ |
| 2-1436 | 3,4,5-triF-Ph | 4-Pyr | 1-Azt-$(CH_2)_3$ |
| 2-1437 | 3,4,5-triF-Ph | 4-Pyr | 1-Pyrd- $(CH_2)_3$ |
| 2-1438 | 3,4,5-triF-Ph | 4-Pyr | 1-Pip-$(CH_2)_3$ |
| 2-1439 | 3,4,5-triF-Ph | 4-Pyr | 1-Mor-$(CH_2)_3$ |
| 2-1440 | 3,4,5-triF-Ph | 4-Pyr | 1-Tmor-$(CH_2)_3$ |
| 2-1441 | 3,4,5-triF-Ph | 4-Pyr | 1-Piz-$(CH_2)_3$ |
| 2-1442 | 3,4,5-triF-Ph | 4-Pyr | 4-Me-1-Piz-$(CH_2)_3$ |
| 2-1443 | 3,4,5-triF-Ph | 4-Pyr | 3-Azt |
| 2-1444 | 3,4,5-triF-Ph | 4-Pyr | 1-Me-3-Azt |
| 2-1445 | 3,4,5-triF-Ph | 4-Pyr | 3-Pyrd |
| 2-1446 | 3,4,5-triF-Ph | 4-Pyr | 1-Me-3-Pyrd |
| 2-1447 | 3,4,5-triF-Ph | 4-Pyr | 4-Pip |
| 2-1448 | 3,4,5-triF-Ph | 4-Pyr | 4-(3,4-deH-Pip) |
| 2-1449 | 3,4,5-triF-Ph | 4-Pyr | 1-Me-4-Pip |
| 2-1450 | 3,4,5-triF-Ph | 4-Pyr | 1-Me-4-(3,4-deH-Pip) |
| 2-1451 | 3,4,5-triF-Ph | 4-Pyr | 1-Piz |
| 2-1452 | 3,4,5-triF-Ph | 4-Pyr | 4-Me-1-Piz |
| 2-1453 | 3,4,5-triF-Ph | 4-Pyr | 4-Pyr |
| 2-1454 | 3,4,5-triF-Ph | 4-Pyr | 3-Pyr |

Table 2 (continued)

| Compound No. | $R^1$ | $R^2$ | $R^3$ |
|---|---|---|---|
| 2-1455 | 3,4,5-triF-Ph | 4-Pyr | 4-Pym |
| 2-1456 | 3,4,5-triF-Ph | 4-Pyr | 5-Pym |
| 2-1457 | 3,4,5-triF-Ph | 4-Pyr | 3-Pyrd-$CH_2$ |
| 2-1458 | 3,4,5-triF-Ph | 4-Pyr | 1-Me-3-Pyrd-$CH_2$ |
| 2-1459 | 3,4,5-triF-Ph | 4-Pyr | 4-Pip-$CH_2$ |
| 2-1460 | 3,4,5-triF-Ph | 4-Pyr | 1-Me-4-Pip-$CH_2$ |
| 2-1461 | 3,4,5-triF-Ph | 4-Pyr | 2-Piz-$CH_2$ |
| 2-1462 | 3,4,5-triF-Ph | 4-Pyr | 4-Pyr-$CH_2$ |
| 2-1463 | 3,4,5-triF-Ph | 4-Pyr | 3-Pyr-$CH_2$ |
| 2-1464 | 3,4,5-triF-Ph | 4-Pyr | 4-Pym-$CH_2$ |
| 2-1465 | 3,4,5-triF-Ph | 4-Pyr | 5-Pym-$CH_2$ |
| 2-1466 | 3,4,5-triF-Ph | 4-Pyr | 2-Pym-$CH_2$ |
| 2-1467 | 3-$CF_3$-Ph | 4-Pyr | $H_2N$-$(CH_2)_3$ |
| 2-1468 | 3-$CF_3$-Ph | 4-Pyr | MeNH-$(CH_2)_3$ |
| 2-1469 | 3-$CF_3$-Ph | 4-Pyr | EtNH-$(CH_2)_3$ |
| 2-1470 | 3-$CF_3$-Ph | 4-Pyr | $Me_2N$-$(CH_2)_3$ |
| 2-1471 | 3-$CF_3$-Ph | 4-Pyr | 1-Azt-$(CH_2)_3$ |
| 2-1472 | 3-$CF_3$-Ph | 4-Pyr | 1-Pyrd-$(CH_2)_3$ |
| 2-1473 | 3-$CF_3$-Ph | 4-Pyr | 1-Pip-$(CH_2)_3$ |
| 2-1474 | 3-$CF_3$-Ph | 4-Pyr | 1-Mor-$(CH_2)_3$ |
| 2-1475 | 3-$CF_3$-Ph | 4-Pyr | 1-Tmor-$(CH_2)_3$ |
| 2-1476 | 3-$CF_3$-Ph | 4-Pyr | 1-Piz-$(CH_2)_3$ |
| 2-1477 | 3-$CF_3$-Ph | 4-Pyr | 4-Me-1-Piz-$(CH_2)_3$ |
| 2-1478 | 3-$CF_3$-Ph | 4-Pyr | 3-Azt |
| 2-1479 | 3-$CF_3$-Ph | 4-Pyr | 1-Me-3-Azt |
| 2-1480 | 3-$CF_3$-Ph | 4-Pyr | 3-Pyrd |
| 2-1481 | 3-$CF_3$-Ph | 4-Pyr | 1-Me-3-Pyrd |
| 2-1482 | 3-$CF_3$-Ph | 4-Pyr | 4-Pip |
| 2-1483 | 3-$CF_3$-Ph | 4-Pyr | 4-(3,4-deH-Pip) |
| 2-1484 | 3-$CF_3$-Ph | 4-Pyr | 1-Me-4-Pip |
| 2-1485 | 3-$CF_3$-Ph | 4-Pyr | 1-Me-4-(3,4-deH-Pip) |
| 2-1486 | 3-$CF_3$-Ph | 4-Pyr | 1-Piz |
| 2-1487 | 3-$CF_3$-Ph | 4-Pyr | 4-Me-1-Piz |
| 2-1488 | 3-$CF_3$-Ph | 4-Pyr | 4-Pyr |
| 2-1489 | 3-$CF_3$-Ph | 4-Pyr | 3-Pyr |
| 2-1490 | 3-$CF_3$-Ph | 4-Pyr | 4-Pym |
| 2-1491 | 3-$CF_3$-Ph | 4-Pyr | 5-Pym |
| 2-1492 | 3-$CF_3$-Ph | 4-Pyr | 3-Pyrd-$CH_2$ |

Table 2 (continued)

| Compound No. | $R^1$ | $R^2$ | $R^3$ |
|---|---|---|---|
| 2-1493 | 3-$CF_3$-Ph | 4-Pyr | 1-Me-3-Pyrd-$CH_2$ |
| 2-1494 | 3-$CF_3$-Ph | 4-Pyr | 4-Pip-$CH_2$ |
| 2-1495 | 3-$CF_3$-Ph | 4-Pyr | 1-Me-4-Pip-$CH_2$ |
| 2-1496 | 3-$CF_3$-Ph | 4-Pyr | 2-Piz-$CH_2$ |
| 2-1497 | 3-$CF_3$-Ph | 4-Pyr | 4-Pyr-$CH_2$ |
| 2-1498 | 3-$CF_3$-Ph | 4-Pyr | 3-Pyr-$CH_2$ |
| 2-1499 | 3-$CF_3$-Ph | 4-Pyr | 4-Pym-$CH_2$ |
| 2-1500 | 3-$CF_3$-Ph | 4-Pyr | 5-Pym-$CH_2$ |
| 2-1501 | 3-$CF_3$-Ph | 4-Pyr | 2-Pym-$CH_2$ |
| 2-1502 | 3-$CHF_2O$-Ph | 4-Pyr | $H_2N$-$(CH_2)_3$ |
| 2-1503 | 3-$CHF_2O$-Ph | 4-Pyr | MeNH-$(CH_2)_3$ |
| 2-1504 | 3-$CHF_2O$-Ph | 4-Pyr | EtNH-$(CH_2)_3$ |
| 2-1505 | 3-$CHF_2O$-Ph | 4-Pyr | $Me_2N$-$(CH_2)_3$ |
| 2-1506 | 3-$CHF_2O$-Ph | 4-Pyr | 1-Azt-$(CH_2)_3$ |
| 2-1507 | 3-$CHF_2O$-Ph | 4-Pyr | 1-Pyrd-$(CH_2)_3$ |
| 2-1508 | 3-$CHF_2O$-Ph | 4-Pyr | 1-Pip-$(CH_2)_3$ |
| 2-1509 | 3-$CHF_2O$-Ph | 4-Pyr | 1-Mor-$(CH_2)_3$ |
| 2-1510 | 3-$CHF_2O$-Ph | 4-Pyr | 1-Tmor-$(CH_2)_3$ |
| 2-1511 | 3-$CHF_2O$-Ph | 4-Pyr | 1-Piz-$(CH_2)_3$ |
| 2-1512 | 3-$CHF_2O$-Ph | 4-Pyr | 4-Me-1-Piz-$(CH_2)_3$ |
| 2-1513 | 3-$CHF_2O$-Ph | 4-Pyr | 3-Azt |
| 2-1514 | 3-$CHF_2O$-Ph | 4-Pyr | 1-Me-3-Azt |
| 2-1515 | 3-$CHF_2O$-Ph | 4-Pyr | 3-Pyrd |
| 2-1516 | 3-$CHF_2O$-Ph | 4-Pyr | 1-Me-3-Pyrd |
| 2-1517 | 3-$CHF_2O$-Ph | 4-Pyr | 4-Pip |
| 2-1518 | 3-$CHF_2O$-Ph | 4-Pyr | 4-(3,4-deH-Pip) |
| 2-1519 | 3-$CHF_2O$-Ph | 4-Pyr | 1-Me-4-Pip |
| 2-1520 | 3-$CHF_2O$-Ph | 4-Pyr | 1-Me-4-(3,4-deH-Pip) |
| 2-1521 | 3-$CHF_2O$-Ph | 4-Pyr | 1-Piz |
| 2-1522 | 3-$CHF_2O$-Ph | 4-Pyr | 4-Me-1-Piz |
| 2-1523 | 3-$CHF_2O$-Ph | 4-Pyr | 4-Pyr |
| 2-1524 | 3-$CHF_2O$-Ph | 4-Pyr | 3-Pyr |
| 2-1525 | 3-$CHF_2O$-Ph | 4-Pyr | 4-Pym |
| 2-1526 | 3-$CHF_2O$-Ph | 4-Pyr | 5-Pym |
| 2-1527 | 3-$CHF_2O$-Ph | 4-Pyr | 3-Pyrd-$CH_2$ |
| 2-1528 | S-$CHF_2O$-Ph | 4-Pyr | 1-Me-3-Pyrd-$CH_2$ |
| 2-1529 | 3-$CHF_2O$-Ph | 4-Pyr | 4-Pip-$CH_2$ |
| 2-1530 | 3-$CHF_2O$-Ph | 4-Pyr | 1-Me-4-Pip-$CH_2$ |

Table 2 (continued)

| Compound No. | $R^1$ | $R^2$ | $R^3$ |
|---|---|---|---|
| 2-1531 | 3-$CHF_2$O-Ph | 4-Pyr | 2-Piz-$CH_2$ |
| 2-1532 | 3-$CHF_2$O-Ph | 4-Pyr | 4-Pyr-$CH_2$ |
| 2-1533 | 3-$CHF_2$O-Ph | 4-Pyr | 3-Pyr-$CH_2$ |
| 2-1534 | 3-$CHF_2$O-Ph | 4-Pyr | 4-Pym-$CH_2$ |
| 2-1535 | 3-$CHF_2$O-Ph | 4-Pyr | 5-Pym-$CH_2$ |
| 2-1536 | 3-$CHF_2$O-Ph | 4-Pyr | 2-Pym-$CH_2$ |
| 2-1537 | 3-Cl-4-F-Ph | 2-$NH_2$-4-Pym | $H_2N-(CH_2)_3$ |
| 2-1538 | 3-Cl-4-F-Ph | 2-$NH_2$-4-Pym | MeNH-$(CH_2)_3$ |
| 2-1539 | 3-Cl-4-F-Ph | 2-$NH_2$-4-Pym | EtNH-$(CH_2)_3$ |
| 2-1540 | 3-Cl-4-F-Ph | 2-$NH_2$-4-Pym | $Me_2N-(CH_2)_3$ |
| 2-1541 | 3-Cl-4-F-Ph | 2-$NH_2$-4-Pym | 1-Azt-$(CH_2)_3$ |
| 2-1542 | 3-Cl-4-F-Ph | 2-$NH_2$-4-Pym | 1-Pyrd-$(CH_2)_3$ |
| 2-1543 | 3-Cl-4-F-Ph | 2-$NH_2$-4-Pym | 1-Pip-$(CH_2)_3$ |
| 2-1544 | 3-Cl-4-F-Ph | 2-$NH_2$-4-Pym | 1-Mor-$(CH_2)_3$ |
| 2-1545 | 3-Cl-4-F-Ph | 2-$NH_2$-4-Pym | 1-Tmor-$(CH_2)_3$ |
| 2-1546 | 3-Cl-4-F-Ph | 2-$NH_2$-4-Pym | 1-Piz-$(CH_2)_3$ |
| 2-1547 | 3-Cl-4-F-Ph | 2-$NH_2$-4-Pym | 4-Me-1-Piz-$(CH_2)_3$ |
| 2-1548 | 3-Cl-4-F-Ph | 2-$NH_2$-4-Pym | 3-Azt |
| 2-1549 | 3-Cl-4-F-Ph | 2-$NH_2$-4-Pym | 1-Me-3-Azt |
| 2-1550 | 3-Cl-4-F-Ph | 2-$NH_2$-4-Pym | 3-Pyrd |
| 2-1551 | 3-Cl-4-F-Ph | 2-$NH_2$-4-Pym | 1-Me-3-Pyrd |
| 2-1552 | 3-Cl-4-F-Ph | 2-$NH_2$-4-Pym | 4-Pip |
| 2-1553 | 3-Cl-4-F-Ph | 2-$NH_2$-4-Pym | 4-(3,4-deH-Pip) |
| 2-1554 | 3-Cl-4-F-Ph | 2-$NH_2$-4-Pym | 1-Me-4-Pip |
| 2-1555 | 3-Cl-4-F-Ph | 2-$NH_2$-4-Pym | 1-Me-4-(3,4-deH-Pip) |
| 2-1556 | 3-Cl-4-F-Ph | 2-$NH_2$-4-Pym | 1-Piz |
| 2-1557 | 3-Cl-4-F-Ph | 2-$NH_2$-4-Pym | 4-Me-1-Piz |
| 2-1558 | 3-Cl-4-F-Ph | 2-$NH_2$-4-Pym | 4-Pyr |
| 2-1559 | 3-Cl-4-F-Ph | 2-$NH_2$-4-Pym | 3-Pyr |
| 2-1560 | 3-Cl-4-F-Ph | 2-$NH_2$-4-Pym | 4-Pym |
| 2-1561 | 3-Cl-4-F-Ph | 2-$NH_2$-4-Pym | 5-Pym |
| 2-1562 | 3-Cl-4-F-Ph | 2-$NH_2$-4-Pym | 3-Pyrd-$CH_2$ |
| 2-1563 | 3-Cl-4-F-Ph | 2-$NH_2$-4-Pym | 1-Me-3-Pyrd-$CH_2$ |
| 2-1564 | 3-Cl-4-F-Ph | 2-$NH_2$-4-Pym | 4-Pip-$CH_2$ |
| 2-1565 | 3-Cl-4-F-Ph | 2-$NH_2$-4-Pym | 1-Me-4-Pip-$CH_2$ |
| 2-1566 | 3-Cl-4-F-Ph | 2-$NH_2$-4-Pym | 2-Piz-$CH_2$ |
| 2-1567 | 3-Cl-4-F-Ph | 2-$NH_2$-4-Pym | 4-Pyr-$CH_2$ |
| 2-1568 | 3-Cl-4-F-Ph | 2-$NH_2$-4-Pym | 3-Pyr-$CH_2$ |

Table 2 (continued)

| Compound No. | $R^1$ | $R^2$ | $R^3$ |
|---|---|---|---|
| 2-1569 | 3-Cl-4-F-Ph | $2-NH_2-4-Pym$ | $4-Pym-CH_2$ |
| 2-1570 | 3-Cl-4-F-Ph | $2-NH_2-4-Pym$ | $5-Pym-CH_2$ |
| 2-1571 | 3-Cl-4-F-Ph | $2-NH_2-4-Pym$ | $2-Pym-CH_2$ |
| 2-1572 | 3,4,5-triF-Ph | $2-NH_2-4-Pym$ | $H_2N-(CH_2)_3$ |
| 2-1573 | 3,4,5-triF-Ph | $2-NH_2-4-Pym$ | $MeNH-(CH_2)_3$ |
| 2-1574 | 3,4,5-triF-Ph | $2-NH_2-4-Pym$ | $EtNH-(CH_2)_3$ |
| 2-1575 | 3,4,5-triF-Ph | $2-NH_2-4-Pym$ | $Me_2N-(CH_2)_3$ |
| 2-1576 | 3,4,5-triF-Ph | $2-NH_2-4-Pym$ | $1-Azt-(CH_2)_3$ |
| 2-1577 | 3,4,5-triF-Ph | $2-NH_2-4-Pym$ | $1-Pyrd-(CH_2)_3$ |
| 2-1578 | 3,4,5-triF-Ph | $2-NH_2-4-Pym$ | $1-Pip-(CH_2)_3$ |
| 2-1579 | 3,4,5-triF-Ph | $2-NH_2-4-Pym$ | $1-Mor-(CH_2)_3$ |
| 2-1580 | 3,1,5-triF-Ph | $2-NH_2-4-Pym$ | $1-Tmor-(CH_2)_3$ |
| 2-1581 | 3,4,5-triF-Ph | $2-NH_2-4-Pym$ | $1-Piz-(CH_2)_3$ |
| 2-1582 | 3,4,5-triF-Ph | $2-NH_2-4-Pym$ | $4-Me-1-Piz-(CH_2)_3$ |
| 2-1583 | 3,4,5-triF-Ph | $2-NH_2-4-Pym$ | 3-Azt |
| 2-1584 | 3,4,5-triF-Ph | $2-NH_2-4-Pym$ | 1-Me-3-Azt |
| 2-1585 | 3,4,5-triF-Ph | $2-NH_2-4-Pym$ | 3-Pyrd |
| 2-1586 | 3,4,5-triF-Ph | $2-NH_2-4-Pym$ | 1-Me-3-Pyrd |
| 2-1587 | 3,4,5-triF-Ph | $2-NH_2-4-Pym$ | 4-Pip |
| 2-1588 | 3,4,5-triF-Ph | $2-NH_2-4-Pym$ | 4-(3,4-deH-Pip) |
| 2-1589 | 3,4,5-triF-Ph | $2-NH_2-4-Pym$ | 1-Me-4-Pip |
| 2-1590 | 3,4,5-triF-Ph | $2-NH_2-4-Pym$ | 1-Me-4-(3,4-deH-Pip) |
| 2-1591 | 3,4,5-triF-Ph | $2-NH_2-4-Pym$ | 1-Piz |
| 2-1592 | 3,4,5-triF-Ph | $2-NH_2-4-Pym$ | 4-Me-1-Piz |
| 2-1593 | 3,4,5-triF-Ph | $2-NH_2-4-Pym$ | 4-Pyr |
| 2-1594 | 3,4,5-triF-Ph | $2-NH_2-4-Pym$ | 3-Pyr |
| 2-1595 | 3,4,5-triF-Ph | $2-NH_2-4-Pym$ | 4-Pym |
| 2-1596 | 3,4,5-triF-Ph | $2-NH_2-4-Pym$ | 5-Pym |
| 2-1597 | 3,4,5-triF-Ph | $2-NH_2-4-Pym$ | $3-Pyrd-CH_2$ |
| 2-1598 | 3,4,5-triF-Ph | $2-NH_2-4-Pym$ | $1-Me-3-Pyrd-CH_2$ |
| 2-1599 | 3,4,5-triF-Ph | $2-NH_2-4-Pym$ | $4-Pip-CH_2$ |
| 2-1600 | 3,4,5-triF-Ph | $2-NH_2-4-Pym$ | $1-Me-4-Pip-CH_2$ |
| 2-1601 | 3,4,5-triF-Ph | $2-NH_2-4-Pym$ | $2-Piz-CH_2$ |
| 2-1602 | 3,4,5-triF-Ph | $2-NH_2-4-Pym$ | $4-Pyr-CH_2$ |
| 2-1603 | 3,4,5-triF-Ph | $2-NH_2-4-Pym$ | $3-Pyr-CH_2$ |
| 2-1604 | 3,4,5-triF-Ph | $2-NH_2-4-Pym$ | $4-Pym-CH_2$ |
| 2-1605 | 3,4,5-triF-Ph | $2-NH_2-4-Pym$ | $5-Pym-CH_2$ |
| 2-1606 | 3,4,5-triF-Ph | $2-NH_2-4-Pym$ | $2-Pym-CH_2$ |

Table 2 (continued)

| Compound No. | $R^1$ | $R^2$ | $R^3$ |
|---|---|---|---|
| 2-1607 | 3-$CF_3$-Ph | 2-$NH_2$-4-Pym | $H_2N$-$(CH_2)_3$ |
| 2-1608 | 3-$CF_3$-Ph | 2-$NH_2$-4-Pym | MeNH-$(CH_2)_3$ |
| 2-1609 | 3-$CF_3$-Ph | 2-$NH_2$-4-Pym | EtNH-$(CH_2)_3$ |
| 2-1610 | 3-$CF_3$-Ph | 2-$NH_2$-4-Pym | $Me_2N$-$(CH_2)_3$ |
| 2-1611 | 3-$CF_3$-Ph | 2-$NH_2$-4-Pym | 1-Azt-$(CH_2)_3$ |
| 2-1612 | 3-$CF_3$-Ph | 2-$NH_2$-4-Pym | 1-Pyrd-$(CH_2)_3$ |
| 2-1613 | 3-$CF_3$-Ph | 2-$NH_2$-4-Pym | 1-Pip-$(CH_2)_3$ |
| 2-1614 | 3-$CF_3$-Ph | 2-$NH_2$-4-Pym | 1-Mor-$(CH_2)_3$ |
| 2-1615 | 3-$CF_3$-Ph | 2-$NH_2$-4-Pym | 1-Tmor-$(CH_2)_3$ |
| 2-1616 | 3-$CF_3$-Ph | 2-$NH_2$-4-Pym | 1-Piz-$(CH_2)_3$ |
| 2-1617 | 3-$CF_3$-Ph | 2-$NH_2$-4-Pym | 4-Me-2-Piz-$(CH_2)_3$ |
| 2-1618 | 3-$CF_3$-Ph | 2-$NH_2$-4-Pym | 3-Azt |
| 2-1619 | 3-$CF_3$-Ph | 2-$NH_2$-4-Pym | 1-Me-3-Azt |
| 2-1620 | 3-$CF_3$-Ph | 2-$NH_2$-4-Pym | 3-Pyrd |
| 2-1621 | 3-$CF_3$-Ph | 2-$NH_2$-4-Pym | 1-Me-3-Pyrd |
| 2-1622 | 3-$CF_3$-Ph | 2-$NH_2$-4-Pym | 4-Pip |
| 2-1623 | 3-$CF_3$-Ph | 2-$NH_2$-4-Pym | 4-(3,4-deH-Pip) |
| 2-1624 | 3-$CF_3$-Ph | 2-$NH_2$-4-Pym | 1-Me-4-Pip |
| 2-1625 | 3-$CF_3$-Ph | 2-$NH_2$-4-Pym | 1-Me-4-(3,4-deH-Pip) |
| 2-1626 | 3-$CF_3$-Ph | 2-$NH_2$-4-Pym | 1-Piz |
| 2-1627 | 3-$CF_3$-Ph | 2-$NH_2$-4-Pym | 4-Me-1-Piz |
| 2-1628 | 3-$CF_3$-Ph | 2-$NH_2$-4-Pym | 4-Pyr |
| 2-1629 | 3-$CF_3$-Ph | 2-$NH_2$-4-Pym | 3-Pyr |
| 2-1630 | 3-$CF_3$-Ph | 2-$NH_2$-4-Pym | 4-Pym |
| 2-1631 | 3-$CF_3$-Ph | 2-$NH_2$-4-Pym | 5-Pym |
| 2-1632 | 3-$CF_3$-Ph | 2-$NH_2$-4-Pym | 3-Pyrd-$CH_2$ |
| 2-1633 | 3-$CF_3$-Ph | 2-$NH_2$-4-Pym | 1-Me-3-Pyrd-$CH_2$ |
| 2-1634 | 3-$CF_3$-Ph | 2-$NH_2$-4-Pym | 4-Pip-$CH_2$ |
| 2-1635 | 3-$CF_3$-ph | 2-$NH_2$-4-Pym | 1-Me-4-Pip-$CH_2$ |
| 2-1636 | 3-$CF_3$-Ph | 2-$NH_2$-4-Pym | 2-Piz-$CH_2$ |
| 2-1637 | 3-$CF_3$-Ph | 2-$NH_2$-4-Pym | 4-Pyr-$CH_2$ |
| 2-1638 | 3-$CF_3$-Ph | 2-$NH_2$-4-Pym | 3-Pyr-$CH_2$ |
| 2-1639 | 3-$CF_3$-Ph | 2-$NH_2$-4-Pym | 4-Pym-$CH_2$ |
| 2-1640 | 3-$CF_3$-Ph | 2-$NH_2$-4-Pym | 5-Pym-$CH_2$ |
| 2-1641 | 3-$CF_3$-Ph | 2-$NH_2$-4-Pym | 2-Pym-$CH_2$ |
| 2-1642 | 3-$CHF_2O$-Ph | 2-$NH_2$-4-Pym | $H_2N$-$(CH_2)_3$ |
| 2-1643 | 3-$CHF_2O$-Ph | 2-$NH_2$-4-Pym | MeNH-$(CH_2)_3$ |
| 2-1644 | 3-$CHF_2O$-Ph | 2-$NH_2$-4-Pym | EtNH-$(CH_2)_3$ |

Table 2 (continued)

| Compound No. | $R^1$ | $R^2$ | $R^3$ |
|---|---|---|---|
| 2-1645 | 3-$CHF_2$O-Ph | 2-$NH_2$-4-Pym | $Me_2N$-$(CH_2)_3$ |
| 2-1646 | 3-$CHF_2$O-Ph | 2-$NH_2$-4-Pym | 1-Azt-$(CH_2)_3$ |
| 2-1647 | 3-$CHP_2$O-Ph | 2-$NH_2$-4-Pym | 1-Pyrd-$(CH_2)_3$ |
| 2-1648 | 3-$CHF_2$O-Ph | 2-$NH_2$-4-Pym | 1-Pip-$(CH_2)_3$ |
| 2-1649 | 3-$CHF_2$O-Ph | 2-$NH_2$-4-Pym | 1-Mor-$(CH_2)_3$ |
| 2-1650 | 3-$CHF_2$O-Ph | 2-$NH_2$-4-Pym | 1-Tmor-$(CH_2)_3$ |
| 2-1651 | 3-$CHF_2$O-Ph | 2-$NH_2$-4-Pym | 1-Piz-$(CH_2)_3$ |
| 2-1652 | 3-$CHF_2$O-Ph | 2-$NH_2$-4-Pym | 4-Me-1-Piz-$(CH_2)_3$ |
| 2-1653 | 3-$CHF_2$O-Ph | 2-$NH_2$-4-Pym | 3-Azt |
| 2-1654 | 3-$CHF_2$O-Ph | 2-$NH_2$-4-Pym | 1-Me-3-Azt |
| 2-1655 | 3-$CHF_2$O-Ph | 2-$NH_2$-4-Pym | 3-Pyrd |
| 2-1656 | 3-$CHF_2$O-Ph | 2-$NH_2$-4-Pym | 1-Me-3-Pyrd |
| 2-1657 | 3-$CHF_2$O-Ph | 2-$NH_2$-4-Pym | 4-Pip |
| 2-1658 | 3-$CHF_2$O-Ph | 2-$NH_2$-4-Pym | 4-(3,4-deH-Pip) |
| 2-1659 | 3-$CHF_2$O-Ph | 2-$NH_2$-4-Pym | 1-Me-4-Pip |
| 2-1660 | 3-$CHF_2$O-Ph | 2-$NH_2$-4-Pym | 1-Me-4-(3,4-deH-Pip) |
| 2-1661 | 3-$CHF_2$O-Ph | 2-$NH_2$-4-Pym | 1-Piz |
| 2-1662 | 3-$CHF_2$O-Ph | 2-$NH_2$-4-Pym | 4-Me-1-Piz |
| 2-1663 | 3-$CHF_2$O-Ph | 2-$NH_2$-4-Pym | 4-Pyr |
| 2-1664 | 3-$CHF_2$O-Ph | 2-$NH_2$-4-Pym | 3-Pyr |
| 2-1665 | 3-$CHF_2$O-Ph | 2-$NH_2$-4-Pym | 4-Pym |
| 2-1666 | 3-$CHF_2$O-Ph | 2-$NH_2$-4-Pym | 5-Pym |
| 2-1667 | 3-$CHF_2$O-Ph | 2-$NH_2$-4-Pym | 3-Pyrd-$CH_2$ |
| 2-1668 | 3-$CHF_2$O-Ph | 2-$NH_2$-4-Pym | 1-Me-3-Pyrd-$CH_2$ |
| 2-1669 | 3-$CHF_2$O-Ph | 2-$NH_2$-4-Pym | 4-Pip-$CH_2$ |
| 2-1670 | 3-$CHF_2$O-Ph | 2-$NH_2$-4-Pym | 1-Me-4-Pip-$CH_2$ |
| 2-1671 | 3-$CHF_2$O-Ph | 2-$NH_2$-4-Pym | 2-Piz-$CH_2$ |
| 2-1672 | 3-$CHF_2$O-Ph | 2-$NH_2$-4-Pym | 4-Pyr-$CH_2$ |
| 2-1673 | 3-$CHF_2$O-Ph | 2-$NH_2$-4-Pym | 3-Pyr-$CH_2$ |
| 2-1674 | 3-$CHF_2$O-Ph | 2-$NH_2$-4-Pym | 4-Pym-$CH_2$ |
| 2-1675 | 3-$CHF_2$O-Ph | 2-$NH_2$-4-Pym | 5-Pym-$CH_2$ |
| 2-1676 | 3-$CHF_2$O-Ph | 2-$NH_2$-4-Pym | 2-Pym-$CH_2$ |
| 2-1677 | 3-Cl-4-F-Ph | 2-MeNH-4-Pym | $H_2N$-$(CH_2)_3$ |
| 2-1678 | 3-Cl-4-F-Ph | 2-MeNH-4-Pym | MeNH-$(CH_2)_3$ |
| 2-1679 | 3-Cl-4-F-Ph | 2-MeNH-4-Pym | EtNH-$(CH_2)_3$ |
| 2-1680 | 3-Cl-4-F-Ph | 2-MeNH-4-Pym | $Me_2N$-$(CH_2)_3$ |
| 2-1681 | 3-Cl-4-P-Ph | 2-MeNH-4-Pym | 1-Azt-$(CH_2)_3$ |
| 2-1682 | 3-Cl-4-F-Ph | 2-MeNH-4-Pym | 1-Pyrd-$(CH_2)_3$ |

Table 2 (continued)

| Compound No. | $R^1$ | $R^2$ | $R^3$ |
|---|---|---|---|
| 2-1683 | 3-Cl-4-F-Ph | 2-MeNH-4-Pym | 1-Pip-$(CH_2)_3$ |
| 2-1684 | 3-Cl-4-F-Ph | 2-MeNH-4-Pym | 1-Mor-$(CH_2)_3$ |
| 2-1685 | 3-Cl-4-F-Ph | 2-MeNH-4-Pym | 1-Tmor-$(CH_2)_3$ |
| 2-1686 | 3-Cl-4-F-Ph | 2-MeNH-4-Pym | 1-Piz-$(CH_2)_3$ |
| 2-1687 | 3-Cl-4-F-Ph | 2-MeNH-4-Pym | 4-Me-1-Piz-$(CH_2)_3$ |
| 2-1688 | 3-Cl-4-F-Ph | 2-MeNH-4-Pym | 3-Azt |
| 2-1689 | 3-Cl-4-F-Ph | 2-MeNH-4-Pym | 1-Me-3-Azt |
| 2-1690 | 3-Cl-4-F-Ph | 2-MeNH-4-Pym | 3-Pyrd |
| 2-1691 | 3-Cl-4-F-Ph | 2-MeNH-4-Pym | 1-Me-3-Pyrd |
| 2-1692 | 3-Cl-4-F-Ph | 2-MeNH-4-Pym | 4-Pip |
| 2-1693 | 3-Cl-4-F-Ph | 2-MeNH-4-Pym | 4-(3,4-deH-Pip) |
| 2-1694 | 3-Cl-4-F-Ph | 2-MeNH-4-Pym | 1-Me-4-Pip |
| 2-1695 | 3-Cl-4-F-Ph | 2-MeNH-4-Pym | 1-Me-4-(3,4-deH-Pip) |
| 2-1696 | 3-Cl-4-F-Ph | 2-MeNH-4-Pym | 1-Piz |
| 2-1697 | 3-Cl-4-F-Ph | 2-MeNH-4-Pym | 4-Me-1-Piz |
| 2-1698 | 3-Cl-I-P-Ph | 2-MeNH-4-Pym | 4-Pyr |
| 2-1699 | 3-Cl-4-F-Ph | 2-MeNH-4-Pym | 3-Pyr |
| 2-1700 | 3-Cl-4-F-Ph | 2-MeNH-4-Pym | 4-Pym |
| 2-1701 | 3-Cl-4-F-Ph | 2-MeNH-4-Pym | 5-Pym |
| 2-1702 | 3-Cl-4-F-Ph | 2-MeNH-4-Pym | 3-pyrd-$CH_2$ |
| 2-1703 | 3-Cl-4-F-Ph | 2-MeNH-4-Pym | 1-Me-3-Pyrd-$CH_2$ |
| 2-1704 | 3-Cl-4-F-Ph | 2-MeNH-4-Pym | 4-Pip-$CH_2$ |
| 2-1705 | 3-Cl-4-F-Ph | 2-MeNH-4-Pym | 1-Me-4-Pip-$CH_2$ |
| 2-1706 | 3-Cl-4-F-Ph | 2-MeNH-4-Pym | 2-Piz-$CH_2$ |
| 2-1707 | 3-Cl-4-F-Ph | 2-MeNH-4-Pym | 4-Pyr-$CH_2$ |
| 2-1708 | 3-Cl-4-F-Ph | 2-MeNH-4-Pym | 3-Pyr-$CH_2$ |
| 2-1709 | 3-Cl-4-F-Ph | 2-MeNH-4-Pym | 4-Pym-$CH_2$ |
| 2-1710 | 3-Cl-4-F-Ph | 2-MeNH-4-Pym | 5-Pym-$CH_2$ |
| 2-1711 | 3-Cl-4-F-Ph | 2-MeNH-4-Pym | 2-Pym-$CH_2$ |
| 2-1712 | 3,4,5-triF-Ph | 2-MeNH-4-Pym | $H_2N$-$(CH_2)_3$ |
| 2-1713 | 3,4,5-triF-Ph | 2-MeNH-4-Pym | MeNH-$(CH_2)_3$ |
| 2-1714 | 3,4,5-triF-Ph | 2-MeNH-4-Pym | EtNH-$(CH_2)_3$ |
| 2-1715 | 3,4,5-triF-Ph | 2-MeNH-4-Pym | $Me_2N$-$(CH_2)_3$ |
| 2-1716 | 3,4,5-triF-Ph | 2-MeNH-4-Pym | 1-Azt-$(CH_2)_3$ |
| 2-1717 | 3,4,5-triF-Ph | 2-MeNH-4-Pym | 1-Pyrd-$(CH_2)_3$ |
| 2-1718 | 3,4,5-triF-Ph | 2-MeNH-4-Pym | 1-Pip-$(CH_2)_3$ |
| 2-1719 | 3,4,5-triF-Ph | 2-MeNH-4-Pym | 1-Mor-$(CH_2)_3$ |
| 2-1720 | 3,4,5-triF-Ph | 2-MeNH-4-Pym | 1-Tmor-$(CH_2)_3$ |

Table 2 (continued)

| Compound No. | $R^1$ | $R^2$ | $R^3$ |
|---|---|---|---|
| 2-1721 | 3,4,5-triF-Ph | 2-MeNH-4-Pym | 1-Piz-$(CH_2)_3$ |
| 2-1722 | 3,4,5-triF-Ph | 2-MeNH-4-Pym | 4-Me-1-Piz-$(CH_2)_3$ |
| 2-1723 | 3,4,5-triF-Ph | 2-MeNH-4-Pym | 3-Azt |
| 2-1724 | 3,4,5-triF-Ph | 2-MeNH-4-Pym | 1-Me-3-Azt |
| 2-1725 | 3,4,5-triF-Ph | 2-MeNH-4-Pym | 3-Pyrd |
| 2-1726 | 3,4,5-triF-Ph | 2-MeNH-4-Pym | 1-Me-3-Pyrd |
| 2-1727 | 3,4,5-triF-Ph | 2-MeNH-4-Pym | 4-Pip |
| 2-1728 | 3,4,5-triF-Ph | 2-MeNH-4-Pym | 4-(3,4-deH-Pip) |
| 2-1729 | 3,4,5-triF-Ph | 2-MeNH-4-Pym | 1-Me-4-Pip |
| 2-1730 | 3,4,5-triF-Ph | 2-MeNH-4-Pym | 1-Me-4-(3,4-deH-Pip) |
| 2-1731 | 3,4,5-triF-Ph | 2-MeNH-4-Pym | 1-Piz |
| 2-1732 | 3,4,5-triF-Ph | 2-MeNH-4-Pym | 4-Me-1-Piz |
| 2-1733 | 3,4,5-triF-Ph | 2-MeNH-4-Pym | 4-Pyr |
| 2-1734 | 3,4,5-triF-Ph | 2-MeNH-4-Pym | 3-Pyr |
| 2-1735 | 3,4,5-triF-Ph | 2-MeNH-4-Pym | 4-Pym |
| 2-1736 | 3,4,5-triF-Ph | 2-MeNH-4-Pym | 5-Pym |
| 2-1737 | 3,4,5-triF-Ph | 2-MeNH-4-Pym | 3-Pyrd-$CH_2$ |
| 2-1738 | 3,4,5-triF-Ph | 2-MeNH-4-Pym | 1-Me-3-Pyrd-$CH_2$ |
| 2-1739 | 3,4,5-triF-Ph | 2-MeNH-4-Pym | 4-Pip-$CH_2$ |
| 2-1740 | 3,4,5-triF-Ph | 2-MeNH-4-Pym | 1-Me-4-Pip-$CH_2$ |
| 2-1741 | 3,4,5-triF-Ph | 2-MeNH-4-Pym | 2-Piz-$CH_2$ |
| 2-1742 | 3,4,5-triF-Ph | 2-MeNH-4-Pym | 4-Pyr-$CH_2$ |
| 2-1743 | 3,4,5-triF-Ph | 2-MeNH-4-Pym | 3-Pyr-$CH_2$ |
| 2-1744 | 3,4,5-triF-Ph | 2-MeNH-4-Pym | 4-Pym-$CH_2$ |
| 2-1745 | 3,4,5-triF-Ph | 2-MeNH-4-Pym | 5-Pym-$CH_2$ |
| 2-1746 | 3,4,5-triF-Ph | 2-MeNH-4-Pym | 2-Pym-$CH_2$ |
| 2-1747 | 3-$CF_3$-Ph | 2-MeNH-4-Pym | $H_2N$-$(CH_2)_3$ |
| 2-1748 | 3-$CF_3$-Ph | 2-MeNH-4-Pym | MeNH-$(CH_2)_3$ |
| 2-1749 | 3-$CF_3$-Ph | 2-MeNH-4-Pym | EtNH-$(CH_2)_3$ |
| 2-1750 | 3-$CF_3$-Ph | 2-MeNH-4-Pym | $Me_2N$-$(CH_2)_3$ |
| 2-1751 | 3-$CF_3$-Ph | 2-MeNH-4-Pym | 1-Azt-$(CH_2)_3$ |
| 2-1752 | 3-$CF_3$-Ph | 2-MeNH-4-Pym | 1-Pyrd-$(CH_2)_3$ |
| 2-1753 | 3-$CF_3$-Ph | 2-MeNH-4-Pym | 1-Pip-$(CH_2)_3$ |
| 2-1754 | 3-$CF_3$-Ph | 2-MeNH-4-Pym | 1-Mor-$(CH_2)_3$ |
| 2-1755 | 3-$CF_3$-Ph | 2-MeNH-4-Pym | 1-Tmor-$(CH_2)_3$ |
| 2-1756 | 3-$CF_3$-Ph | 2-MeNH-4-Pym | 1-Piz-$(CH_2)_3$ |
| 2-1757 | 3-$CF_3$-Ph | 2-MeNH-4-Pym | 4-Me-1-Piz-$(CH_2)_3$ |
| 2-1758 | 3-$CF_3$-Ph | 2-MeNH-4-Pym | 3-Azt |

Table 2 (continued)

| Compound No. | $R^1$ | $R^2$ | $R^3$ |
|---|---|---|---|
| 2-1759 | $3-CF_3-Ph$ | 2-MeNH-4-Pym | 1-Me-3-Azt |
| 2-1760 | $3-CF_3-Ph$ | 2-MeNH-4-Pym | 3-Pyrd |
| 2-1761 | $3-CF_3-Ph$ | 2-MeNH-4-Pym | 1-Me-3-Pyrd |
| 2-1762 | $3-CF_3-Ph$ | 2-MeNH-4-Pym | 4-Pip |
| 2-1763 | $3-CF_3-Ph$ | 2-MeNH-4-Pym | 4-(3,4-deH-Pip) |
| 2-1764 | $3-CF_3-Ph$ | 2-MeNH-4-Pym | 1-Me-4-Pip |
| 2-1765 | $3-CF_3-Ph$ | 2-MeNH-4-Pym | 1-Me-4-(3,4-deH-Pip) |
| 2-1766 | $3-CF_3-Ph$ | 2-MeNH-4-Pym | 1-Piz |
| 2-1767 | $3-CF_3-Ph$ | 2-MeNH-4-Pym | 4-Me-1-Piz |
| 2-1768 | $3-CF_3-Ph$ | 2-MeNH-4-Pym | 4-Pyr |
| 2-1769 | $3-CF_3-Ph$ | 2-MeNH-4-Pym | 3-Pyr |
| 2-1770 | $3-CF_3-Ph$ | 2-MeNH-4-Pym | 4-Pym |
| 2-1771 | $3-CF_3-Ph$ | 2-MeNH-4-Pym | 5-Pym |
| 2-1772 | $3-CF_3-Ph$ | 2-MeNH-4-Pym | $S-Pyrd-CH_2$ |
| 2-1773 | $3-CF_3-Ph$ | 2-MeNH-4-Pym | $1-Me-3-Pyrd-CH_2$ |
| 2-1774 | $3-CF_3-Ph$ | 2-MeNH-4-Pym | $4-Pip-CH_2$ |
| 2-1775 | $3-CF_3-Ph$ | 2-MeNH-4-Pym | $1-Me-4-Pip-CH_2$ |
| 2-1776 | $3-CF_3-Ph$ | 2-MeNH-4-Pym | $2-Piz-CH_2$ |
| 2-1777 | $3-CF_3-Ph$ | 2-MeNH-4-Pym | $4-Pyr-CH_2$ |
| 2-1778 | $3-CF_3-Ph$ | 2-MeNH-4-Pym | $3-Pyr-CH_2$ |
| 2-1779 | $3-CF_3-Ph$ | 2-MeNH-4-Pym | $4-Pym-CH_2$ |
| 2-1780 | $3-CF_3-Ph$ | 2-MeNH-4-Pym | $5-Pym-CH_2$ |
| 2-1781 | $3-CF_3-Ph$ | 2-MeNH-4-Pym | $2-Pym-CH_2$ |
| 2-1782 | $3-CHF_2O-Ph$ | 2-MeNH-4-Pym | $H_2N-(CH_2)_3$ |
| 2-1783 | $3-CHF_2O-Ph$ | 2-MeNH-4-Pym | $MeNH-(CH_2)_3$ |
| 2-1784 | $3-CHF_2O-Ph$ | 2-MeNH-4-Pym | $EtNH-(CH_2)_3$ |
| 2-1785 | $3-CHF_2O-Ph$ | 2-MeNH-4-Pym | $Me_2N-(CH_2)_3$ |
| 2-1786 | $3-CHF_2O-Ph$ | 2-MeNH-4-Pym | $1-Azt-(CH_2)_3$ |
| 2-1787 | $3-CHF_2O-Ph$ | 2-MeNH-4-Pym | $1-Pyrd-(CH_2)_3$ |
| 2-1788 | $3-CHF_2O-Ph$ | 2-MeNH-4-Pym | $1-Pip-(CH_2)_3$ |
| 2-1789 | $3-CHF_2O-Ph$ | 2-MeNH-4-Pym | $1-Mor-(CH_2)_3$ |
| 2-1790 | $3-CHF_2O-Ph$ | 2-MeNH-4-Pym | $1-Tmor-(CH_2)_3$ |
| 2-1791 | $3-CHF_2O-Ph$ | 2-MeNH-4-Pym | $1-Piz-(CH_2)_3$ |
| 2-1792 | $3-CHF_2O-Ph$ | 2-MeNH-4-Pym | $4-Me-1-Piz-(CH_2)_3$ |
| 2-1793 | $3-CHF_2O-Ph$ | 2-MeNH-4-Pym | 3-Azt |
| 2-1794 | $3-CHF_2O-Ph$ | 2-MeNH-4-Pym | 1-Me-3-Azt |
| 2-1795 | $3-CHF_2O-Ph$ | 2-MeNH-4-Pym | 3-Pyrd |
| 2-1796 | $3-CHF_2O-Ph$ | 2-MeNH-4-Pym | 1-Me-3-Pyrd |

Table 2 (continued)

| Compound No. | $R^1$ | $R^2$ | $R^3$ |
|---|---|---|---|
| 2-1797 | 3-$CHF_2$O-Ph | 2-MeNH-4-Pym | 4-Pip |
| 2-1798 | 3-$CHF_2$O-Ph | 2-MeNH-4-Pym | 4-(3,4-deH-Pip) |
| 2-1799 | 3-$CHF_2$O-Ph | 2-MeNH-4-Pym | 1-Me-4-Pip |
| 2-1800 | 3-$CHF_2$O-Ph | 2-MeNH-4-Pym | 1-Me-4-(3,4-deH-Pip) |
| 2-1801 | 3-$CHF_2$O-Ph | 2-MeNH-4-Pym | 1-Piz |
| 2-1802 | 3-$CHF_2$O-Ph | 2-MeNH-4-Pym | 4-Me-1-Piz |
| 2-1803 | 3-$CHF_2$O-Ph | 2-MeNH-4-Pym | 4-Pyr |
| 2-1804 | 3-$CHF_2$O-Ph | 2-MeNH-4-Pym | 3-Pyr |
| 2-1805 | 3-$CHF_2$O-Ph | 2-MeNH-4-Pym | 4-Pym |
| 2-1806 | 3-$CHF_2$O-Ph | 2-MeNH-4-Pym | 5-Pym |
| 2-1807 | 3-$CHF_2$O-Ph | 2-MeNH-4-Pym | 3-Pyrd-$CH_2$ |
| 2-1808 | 3-$CHF_2$O-Ph | 2-MeNH-4-Pym | 1-Me-3-Pyrd-$CH_2$ |
| 2-1809 | 3-$CHF_2$O-Ph | 2-MeNH-4-Pym | 4-Pip-$CH_2$ |
| 2-1810 | 3-$CHF_2$O-Ph | 2-MeNH-4-Pym | 1-Me-4-Pip-$CH_2$ |
| 2-1811 | 3-$CHF_2$O-Ph | 2-MeNH-4-Pym | 2-Piz-$CH_2$ |
| 2-1812 | 3-$CHF_2$O-Ph | 2-MeNH-4-Pym | 4-Pyr-$CH_2$ |
| 2-1813 | 3-$CHF_2$O-Ph | 2-MeNH-4-Pym | 3-Pyr-$CH_2$ |
| 2-1814 | 3-$CHF_2$O-Ph | 2-MeNH-4-Pym | 4-Pym-$CH_2$ |
| 2-1815 | 3-$CHF_2$O-Ph | 2-MeNH-4-Pym | 5-Pym-$CH_2$ |
| 2-1816 | 3-$CHF_2$O-Ph | 2-MeNH-4-Pym | 2-Pym-$CH_2$ |
| 2-1817 | Ph | 2-$NH_2$-4-Pyr | $H_2N$-$(CH_2)_3$ |
| 2-1818 | Ph | 2-$NH_2$-4-Pyr | MeNH-$(CH_2)_3$ |
| 2-1819 | Ph | 2-$NH_2$-4-Pyr | EtNH-$(CH_2)_3$ |
| 2-1820 | Ph | 2-$NH_2$-4-Pyr | $Me_2N$-$(CH_2)_3$ |
| 2-1821 | Ph | 2-$NH_2$-4-Pyr | 1-Azt-$(CH_2)_3$ |
| 2-1822 | Ph | 2-$NH_2$-4-Pyr | 1-Pyrd-$(CH_2)_3$ |
| 2-1823 | Ph | 2-$NH_2$-4-Pyr | 1-Pip-$(CH_2)_3$ |
| 2-1824 | Ph | 2-$NH_2$-4-Pyr | 1-Mor-$(CH_2)_3$ |
| 2-1825 | Ph | 2-$NH_2$-4-Pyr | 1-Tmor-$(CH_2)_3$ |
| 2-1826 | Ph | 2-$NH_2$-4-Pyr | 1-Piz-$(CH_2)_3$ |
| 2-1827 | Ph | 2-$NH_2$-4-Pyr | 4-Me-1-Piz-$(CH_2)_3$ |
| 2-1828 | Ph | 2-$NH_2$-4-Pyr | 3-Azt |
| 2-1829 | Ph | 2-$NH_2$-4-Pyr | 1-Me-3-Azt |
| 2-1830 | Ph | 2-$NH_2$-4-Pyr | 3-Pyrd |
| 2-1831 | Ph | 2-$NH_2$-4-Pyr | 1-Me-3-Pyrd |
| 2-1832 | Ph | 2-$NH_2$-4-Pyr | 4-Pip |
| 2-1833 | Ph | 2-$NH_2$-4-Pyr | 4-(3,4-deH-Pip) |
| 2-1834 | Ph | 2-$NH_2$-4-Pyr | 1-Me-4-Pip |

Table 2 (continued)

| Compound No. | $R^1$ | $R^2$ | $R^3$ |
|---|---|---|---|
| 2-1835 | Ph | $2-NH_2-4-Pyr$ | 1-Me-4-(3,4-deH-Pip) |
| 2-1836 | Ph | $2-NH_2-4-Pyr$ | 1-Piz |
| 2-1837 | Ph | $2-NH_2-4-Pyr$ | 4-Me-1-Piz |
| 2-1838 | Ph | $2-NH_2-4-Pyr$ | 4-Pyr |
| 2-1839 | Ph | $2-NH_2-4-Pyr$ | 3-Pyr |
| 2-1840 | Ph | $2-NH_2-4-Pyr$ | 4-Pym |
| 2-1841 | Ph | $2-NH_2-4-Pyr$ | 5-Pym |
| 2-1842 | Ph | $2-NH_2-4-Pyr$ | $3-Pyrd-CH_2$ |
| 2-1843 | Ph | $2-NH_2-4-Pyr$ | $1-Me-3-Pyrd-CH_2$ |
| 2-1844 | Ph | $2-NH_2-4-Pyr$ | $4-Pip-CH_2$ |
| 2-1845 | Ph | $2-NH_2-4-Pyr$ | $1-Me-4-Pip-CH_2$ |
| 2-1846 | Ph | $2-NH_2-4-Pyr$ | $2-Piz-CH_2$ |
| 2-1847 | Ph | $2-NH_2-4-Pyr$ | $4-Pyr-CH_2$ |
| 2-1848 | Ph | $2-NH_2-4-Pyr$ | $3-Pyr-CH_2$ |
| 2-1849 | Ph | $2-NH_2-4-Pyr$ | $4-Pym-CH_2$ |
| 2-1850 | Ph | $2-NH_2-4-Pyr$ | $5-Pym-CH_2$ |
| 2-1851 | Ph | $2-NH_2-4-Pyr$ | $2-Pym-CH_2$ |
| 2-1852 | 4-F-Ph | $2-NH_2-4-Pyr$ | $H_2N-(CH_2)_3$ |
| 2-1853 | 4-F-Ph | $2-NH_2-4-Pyr$ | $MeNH-(CH_2)_3$ |
| 2-1854 | 4-F-Ph | $2-NH_2-4-Pyr$ | $EtNH-(CH_2)_3$ |
| 2-1855 | 4-F-Ph | $2-NH_2-4-Pyr$ | $Me_2N-(CH_2)_3$ |
| 2-1856 | 4-F-Ph | $2-NH_2-4-Pyr$ | $1-Azt-(CH_2)_3$ |
| 2-1857 | 4-F-Ph | $2-NH_2-4-Pyr$ | $1-Pyrd-(CH_2)_3$ |
| 2-1858 | 4-F-Ph | $2-NH_2-4-Pyr$ | $1-Pip-(CH_2)_3$ |
| 2-1859 | 4-F-Ph | $2-NH_2-4-Pyr$ | $1-Mor-(CH_2)_3$ |
| 2-1860 | 4-F-Ph | $2-NH_2-4-Pyr$ | $1-Tmor-(CH_2)_3$ |
| 2-1861 | 4-F-Ph | $2-NH_2-4-Pyr$ | $1-Piz-(CH_2)_3$ |
| 2-1862 | 4-F-Ph | $2-NH_2-4-Pyr$ | $4-Me-1-Piz-(CH_2)_3$ |
| 2-1863 | 4-F-Ph | $2-NH_2-4-Pyr$ | 3-Azt |
| 2-1864 | 4-F-Ph | $2-NH_2-4-Pyr$ | 1-Me-3-Azt |
| 2-1865 | 4-F-Ph | $2-NH_2-4-Pyr$ | 3-Pyrd |
| 2-1866 | 4-F-Ph | $2-NH_2-4-Pyr$ | 1-Me-3-Pyrd |
| 2-1867 | 4-F-Ph | $2-NH_2-4-Pyr$ | 4-Pip |
| 2-1868 | 4-F-Ph | $2-NH_2-4-Pyr$ | 4-(3,4-deH-Pip) |
| 2-1869 | 4-F-Ph | $2-NH_2-4-Pyr$ | 1-Me-4-Pip |
| 2-1870 | 4-F-Ph | $2-NH_2-4-Pyr$ | 1-Me-4-(3,4-deH-Pip) |
| 2-1871 | 4-F-Ph | $2-NH_2-4-Pyr$ | 1-Piz |
| 2-1872 | 4-F-Ph | $2-NH_2-4-Pyr$ | 4-Me-1-Piz |

Table 2 (continued)

| Compound No. | $R^1$ | $R^2$ | $R^3$ |
|---|---|---|---|
| 2-1873 | 4-F-Ph | $2-NH_2-4-Pyr$ | 4-Pyr |
| 2-1874 | 4-F-Ph | $2-NH_2-4-Pyr$ | 3-Pyr |
| 2-1875 | 4-F-Ph | $2-NH_2-4-Pyr$ | 4-Pym |
| 2-1876 | 4-F-Ph | $2-NH_2-4-Pyr$ | 5-Pym |
| 2-1877 | 4-F-Ph | $2-NH_2-4-Pyr$ | $3-Pyrd-CH_2$ |
| 2-1878 | 4-F-Ph | $2-NH_2-4-Pyr$ | $1-Me-3-Pyrd-CH_2$ |
| 2-1879 | 4-F-Ph | $2-NH_2-4-Pyr$ | 4-Pip-CH2 |
| 2-1880 | 4-F-Ph | $2-NH_2-4-Pyr$ | $1-Me-4-Pip-CH_2$ |
| 2-1881 | 4-F-Ph | $2-NH_2-4-Pyr$ | $2-Piz-CH_2$ |
| 2-1882 | 4-F-Ph | $2-NH_2-4-Pyr$ | $4-Pyr-CH_2$ |
| 2-1883 | 4-F-Ph | $2-NH_2-4-Pyr$ | $3-Pyr-CH_2$ |
| 2-1884 | 4-F-Ph | $2-NH_2-4-Pyr$ | $4-Pym-CH_2$ |
| 2-1885 | 4-F-Ph | $2-NH_2-4-Pyr$ | $5-Pym-CH_2$ |
| 2-1886 | 4-F-Ph | $2-NH_2-4-Pyr$ | $2-Pym-CH_2$ |
| 2-1887 | 3-F-Ph | $2-NH_2-4-Pyr$ | $H_2N-(CH_2)_3$ |
| 2-1888 | 3-F-Ph | $2-NH_2-4-Pyr$ | $MeNH-(CH_2)_3$ |
| 2-1889 | 3-F-Ph | $2-NH_2-4-Pyr$ | $EtNH-(CH_2)_3$ |
| 2-1890 | 3-F-Ph | $2-NH_2-4-Pyr$ | $Me_2N-(CH_2)_3$ |
| 2-1891 | 3-F-Ph | $2-NH_2-4-Pyr$ | $1-Azt-(CH_2)_3$ |
| 2-1892 | 3-F-Ph | $2-NH_2-4-Pyr$ | $1-Pyrd-(CH_2)_3$ |
| 2-1893 | 3-F-Ph | $2-NH_2-4-Pyr$ | $1-Pip-(CH_2)_3$ |
| 2-1894 | 3-F-Ph | $2-NH_2-4-Pyr$ | $1-Mor-(CH_2)_3$ |
| 2-1895 | 3-F-Ph | $2-NH_2-4-Pyr$ | $1-Tmor-(CH_2)_3$ |
| 2-1896 | 3-F-Ph | $2-NH_2-4-Pyr$ | $1-Piz-(CH_2)_3$ |
| 2-1897 | 3-F-Ph | $2-NH_2-4-Pyr$ | $4-Me-1-Piz-(CH_2)_3$ |
| 2-1898 | 3-F-Ph | $2-NH_2-4-Pyr$ | 3-Azt |
| 2-1899 | 3-F-Ph | $2-NH_2-4-Pyr$ | 1-Me-3-Azt |
| 2-1900 | 3-F-Ph | $2-NH_2-4-Pyr$ | 3-Pyrd |
| 2-1901 | 3-F-Ph | $2-NH_2-4-Pyr$ | 1-Me-3-Pyrd |
| 2-1902 | 3-F-Ph | $2-NH_2-4-Pyr$ | 4-Pip |
| 2-1903 | 3-F-Ph | $2-NH_2-4-Pyr$ | 4-(3,4-deH-Pip) |
| 2-1904 | 3-F-Ph | $2-NH_2-4-Pyr$ | 1-Me-4-Pip |
| 2-1905 | 3-F-Ph | $2-NH_2-4-Pyr$ | 1-Me-4-(3,4-deH-Pip) |
| 2-1906 | 3-F-Ph | $2-NH_2-4-Pyr$ | 1-Piz |
| 2-1907 | 3-F-Ph | $2-NH_2-4-Pyr$ | 4-Me-1-Piz |
| 2-1908 | 3-F-Ph | $2-NH_2-4-Pyr$ | 4-Pyr |
| 2-1909 | 3-F-Ph | $2-NH_2-4-Pyr$ | 3-Pyr |
| 2-1910 | 3-F-Ph | $2-NH_2-4-Pyr$ | 4-Pym |

Table 2 (continued)

| Compound No. | $R^1$ | $R^2$ | $R^3$ |
|---|---|---|---|
| 2-1911 | 3-F-Ph | $2-NH_2-4-Pyr$ | 5-Pym |
| 2-1912 | 3-F-Ph | $2-NH_2-4-Pyr$ | $3-pyrd-CH_2$ |
| 2-1913 | 3-F-Ph | $2-NH_2-4-Pyr$ | $1-Me-3-Pyrd-CH_2$ |
| 2-1914 | 3-F-Ph | $2-NH_2-4-Pyr$ | $4-Pip-CH_2$ |
| 2-1915 | 3-F-Ph | $2-NH_2-4-Pyr$ | $1-Me-4-Pip-CH_2$ |
| 2-1916 | 3-F-Ph | $2-NH_2-4-Pyr$ | $2-Piz-CH_2$ |
| 2-1917 | 3-F-Ph | $2-NH_2-4-Pyr$ | $4-Pyr-CH_2$ |
| 2-1918 | 3-F-Ph | $2-NH_2-4-Pyr$ | $3-Pyr-CH_2$ |
| 2-1919 | 3-F-Ph | $2-NH_2-4-Pyr$ | $4-Pym-CH_2$ |
| 2-1920 | 3-F-Ph | $2-NH_2-4-Pyr$ | $5-Pym-CH_2$ |
| 2-1921 | 3-F-Ph | $2-NH_2-4-Pyr$ | $2-Pym-CH_2$ |
| 2-1922 | 3,4-diF-Ph | $2-NH_2-4-Pyr$ | $H_2N-(CH_2)_3$ |
| 2-1923 | 3,4-diF-Ph | $2-NH_2-4-Pyr$ | $MeNH-(CH_2)_3$ |
| 2-1924 | 3,4-diF-Ph | $2-NH_2-4-Pyr$ | $EtNH-(CH_2)_3$ |
| 2-1925 | 3,4-diF-Ph | $2-NH_2-4-Pyr$ | $Me_2N-(CH_2)_3$ |
| 2-1926 | 3,4-diF-Ph | $2-NH_2-4-Pyr$ | $1-Azt-(CH_2)_3$ |
| 2-1927 | 3,4-diF-Ph | $2-NH_2-4-Pyr$ | $1-Pyrd-(CH_2)_3$ |
| 2-1928 | 3,4-diF-Ph | $2-NH_2-4-Pyr$ | $1-Pip-(CH_2)_3$ |
| 2-1929 | 3,4-diF-Ph | $2-NH_2-4-Pyr$ | $1-Mor-(CH_2)_3$ |
| 2-1930 | 3,4-diF-Ph | $2-NH_2-4-Pyr$ | $1-Tmor-(CH_2)_3$ |
| 2-1931 | 3,4-diF-Ph | $2-NH_2-4-Pyr$ | $1-Piz-(CH_2)_3$ |
| 2-1932 | 3,4-diF-Ph | $2-NH_2-4-Pyr$ | $4-Me-1-Piz-(CH_2)_3$ |
| 2-1933 | 3,4-diF-Ph | $2-NH_2-4-Pyr$ | 3-Azt |
| 2-1934 | 3,4-diF-Ph | $2-NH_2-4-Pyr$ | 1-Me-3-Azt |
| 2-1935 | 3,4-diF-Ph | $2-NH_2-4-Pyr$ | 3-Pyrd |
| 2-1936 | 3,4-diF-Ph | $2-NH_2-4-Pyr$ | 1-Me-3-Pyrd |
| 2-1937 | 3,4-diF-Ph | $2-NH_2-4-Pyr$ | 4-Pip |
| 2-1938 | 3,4-diF-Ph | $2-NH_2-4-Pyr$ | 4-(3,4-deH-Pip) |
| 2-1939 | 3,4-diF-Ph | $2-NH_2-4-Pyr$ | 1-Me-4-Pip |
| 2-1940 | 3,4-diF-Ph | $2-NH_2-4-Pyr$ | 1-Me-4-(3,4-deH-Pip) |
| 2-1941 | 3,4-diF-Ph | $2-NH_2-4-Pyr$ | 1-Piz |
| 2-1942 | 3,4-diF-Ph | $2-NH_2-4-Pyr$ | 4-Me-1-Piz |
| 2-1943 | 3,4-diF-Ph | $2-NH_2-4-Pyr$ | 4-Pyr |
| 2-1944 | 3,4-diF-Ph | $2-NH_2-4-Pyr$ | 3-Pyr |
| 2-1945 | 3,4-diF-Ph | $2-NH_2-4-Pyr$ | 4-Pym |
| 2-1946 | 3,4-diF-Ph | $2-NH_2-4-Pyr$ | 5-Pym |
| 2-1947 | 3,4-diF-Ph | $2-NH_2-4-Pyr$ | $3-Pyrd-CH_2$ |
| 2-1948 | 3,4-diF-Ph | $2-NH_2-4-Pyr$ | $1-Me-3-Pyrd-CH_2$ |

Table 2 (continued)

| Compound No. | $R^1$ | $R^2$ | $R^3$ |
|---|---|---|---|
| 2-1949 | 3,4-diF-Ph | $2-NH_2-4-Pyr$ | $4-Pip-CH_2$ |
| 2-1950 | 3,4-diF-Ph | $2-NH_2-4-Pyr$ | $1-Me-4-Pip-CH_2$ |
| 2-1951 | 3,4-diF-Ph | $2-NH_2-4-Pyr$ | $2-Piz-CH_2$ |
| 2-1952 | 3,4-diF-Ph | $2-NH_2-4-Pyr$ | $4-Pyr-CH_2$ |
| 2-1953 | 3,4-diF-Ph | $2-NH_2-4-Pyr$ | $3-Pyr-CH_2$ |
| 2-1954 | 3,4-diF-Ph | $2-NH_2-4-Pyr$ | $4-Pym-CH_2$ |
| 2-1955 | 3,4-diF-Ph | $2-NH_2-4-Pyr$ | $5-Pym-CH_2$ |
| 2-1956 | 3,4-diF-Ph | $2-NH_2-4-Pyr$ | $2-Pym-CH_2$ |
| 2-1957 | 3-Cl-Ph | $2-NH_2-4-Pyr$ | $H_2N-(CH_2)_3$ |
| 2-1958 | 3-Cl-Ph | $2-NH_2-4-Pyr$ | $MeNH-(CH_2)_3$ |
| 2-1959 | 3-Cl-Ph | $2-NH_2-4-Pyr$ | $EtNH-(CH_2)_3$ |
| 2-1960 | 3-Cl-Ph | $2-NH_2-4-Pyr$ | $Me_2N-(CH_2)_3$ |
| 2-1961 | 3-Cl-Ph | $2-NH_2-4-Pyr$ | $1-Azt-(CH_2)_3$ |
| 2-1962 | 3-Cl-Ph | $2-NH_2-4-Pyr$ | $1-Pyrd-(CH_2)_3$ |
| 2-1963 | 3-Cl-Ph | $2-NH_2-4-Pyr$ | $1-Pip-(CH_2)_3$ |
| 2-1964 | 3-Cl-Ph | $2-NH_2-4-Pyr$ | $1-Mor-(CH_2)_3$ |
| 2-1965 | 3-Cl-Ph | $2-NH_2-4-Pyr$ | $1-Tmor-(CH_2)_3$ |
| 2-1966 | 3-Cl-Ph | $2-NH_2-4-Pyr$ | $1-Piz-(CH_2)_3$ |
| 2-1967 | 3-Cl-Ph | $2-NH_2-4-Pyr$ | $4-Me-1-Piz-(CH_2)_3$ |
| 2-1968 | 3-Cl-Ph | $2-NH_2-4-Pyr$ | 3-Azt |
| 2-1969 | 3-Cl-Ph | $2-NH_2-4-Pyr$ | 1-Me-3-Azt |
| 2-1970 | 3-Cl-Ph | $2-NH_2-4-Pyr$ | 3-Pyrd |
| 2-1971 | 3-Cl-Ph | $2-NH_2-4-Pyr$ | 1-Me-3-Pyrd |
| 2-1972 | 3-Cl-Ph | $2-NH_2-4-Pyr$ | 4-Pip |
| 2-1973 | 3-Cl-Ph | $2-NH_2-4-Pyr$ | 4-(3,4-deH-Pip) |
| 2-1974 | 3-Cl-Ph | $2-NH_2-4-Pyr$ | 1-Me-4-Pip |
| 2-1975 | 3-Cl-Ph | $2-NH_2-4-Pyr$ | 1-Me-4-(3,4-deH-Pip) |
| 2-1976 | 3-Cl-Ph | $2-NH_2-4-Pyr$ | 1-Piz |
| 2-1977 | 3-Cl-Ph | $2-NH_2-4-Pyr$ | 4-Me-1-Piz |
| 2-1978 | 3-Cl-Ph | $2-NH_2-4-Pyr$ | 4-Pyr |
| 2-1979 | 3-Cl-Ph | $2-NH_2-4-Pyr$ | 3-Pyr |
| 2-1980 | 3-Cl-Ph | $2-NH_2-4-Pyr$ | 4-Pym |
| 2-1981 | 3-Cl-Ph | $2-NH_2-4-Pyr$ | 5-Pym |
| 2-1982 | 3-Cl-Ph | $2-NH_2-4-Pyr$ | $3-Pyrd-CH_2$ |
| 2-1983 | 3-Cl-Ph | $2-NH_2-4-Pyr$ | $1-Me-3-Pyrd-CH_2$ |
| 2-1984 | 3-Cl-Ph | $2-NH_2-4-Pyr$ | $9-Pip-CH_2$ |
| 2-1985 | 3-Cl-Ph | $2-NH_2-4-Pyr$ | $1-Me-4-PiP-CH_2$ |
| 2-1986 | 3-Cl-Ph | $2-NH_2-4-Pyr$ | $2-Piz-CH_2$ |

Table 2 (continued)

| Compound No. | $R^1$ | $R^2$ | $R^3$ |
|---|---|---|---|
| 2-1987 | 3-Cl-Ph | $2-NH_2-4-Pyr$ | $4-Pyr-CH_2$ |
| 2-1988 | 3-Cl-Ph | $2-NH_2-4-Pyr$ | $3-Pyr-CH_2$ |
| 2-1989 | 3-Cl-Ph | $2-NH_2-4-Pyr$ | $4-Pym-CH_2$ |
| 2-1990 | 3-Cl-Ph | $2-NH_2-4-Pyr$ | $5-Pym-CH_2$ |
| 2-1991 | 3-Cl-Ph | $2-NH_2-4-Pyr$ | $2-Pym-CH_2$ |
| 2-1992 | Ph | 2-MeNH-4-Pyr | $H_2N-(CH_2)_3$ |
| 2-1993 | Ph | 2-MeNH-4-Pyr | $MeNH-(CH_2)_3$ |
| 2-1994 | Ph | 2-MeNH-4-Pyr | $EtNH-(CH_2)_3$ |
| 2-1995 | Ph | 2-MeNH-4-Pyr | $Me_2N-(CH_2)_3$ |
| 2-1996 | Ph | 2-MeNH-4-Pyr | $1-Azt-(CH_2)_3$ |
| 2-1997 | Ph | 2-MeNH-4-Pyr | $1-Pyrd-(CH_2)_3$ |
| 2-1998 | Ph | 2-MeNH-4-Pyr | $1-Pip-(CH_2)_3$ |
| 2-1999 | Ph | 2-MeNH-4-Pyr | $1-Mor-(CH_2)_3$ |
| 2-2000 | Ph | 2-MeNH-4-Pyr | $2-Tmor-(CH_2)_3$ |
| 2-2001 | Ph | 2-MeNH-4-Pyr | $1-Piz-(CH_2)_3$ |
| 2-2002 | Ph | 2-MeNH-4-Pyr | $4-Me-1-Piz-(CH_2)_3$ |
| 2-2003 | Ph | 2-MeNH-4-Pyr | 3-Azt |
| 2-2004 | Ph | 2-MeNH-4-Pyr | 1-Me-3-Azt |
| 2-2005 | Ph | 2-MeNH-4-Pyr | 3-Pyrd |
| 2-2006 | Ph | 2-MeNH-4-Pyr | 1-Me-3-Pyrd |
| 2-2007 | Ph | 2-MeNH-4-Pyr | 4-Pip |
| 2-2008 | Ph | 2-MeNH-4-Pyr | 4-(3,4-deH-Pip) |
| 2-2009 | Ph | 2-MeNH-4-Pyr | 1-Me-4-P |
| 2-2010 | Ph | 2-MeNH-4-Pyr | 1-Me-4-(3,4-deH-Pip) |
| 2-2011 | Ph | 2-MeNH-4-Pyr | 1-Piz |
| 2-2012 | Ph | 2-MeNH-4-Pyr | 4-Me-1-Piz |
| 2-2013 | Ph | 2-MeNH-4-Pyr | 4-Pyr |
| 2-2014 | Ph | 2-MeNH-4-Pyr | 3-Pyr |
| 2-2015 | Ph | 2-MeNH-4-Pyr | 4-Pym |
| 2-2016 | Ph | 2-MeNH-4-Pyr | 5-Pym |
| 2-2017 | Ph | 2-MeNH-4-Pyr | $3-Pyrd-CH_2$ |
| 2-2018 | Ph | 2-MeNH-4-Pyr | $1-Me-3-Pyrd-CH_2$ |
| 2-2019 | Ph | 2-MeNH-4-Pyr | $4-Pip-CH_2$ |
| 2-2020 | Ph | 2-MeNH-4-Pyr | $1-Me-4-Pip-CH_2$ |
| 2-2021 | Ph | 2-MeNH-4-Pyr | $2-Piz-CH_2$ |
| 2-2022 | Ph | 2-MeNH-4-Pyr | $4-Pyr-CH_2$ |
| 2-2023 | Ph | 2-MeNH-4-Pyr | $3-Pyr-CH_2$ |
| 2-2024 | Ph | 2-MeNH-4-Pyr | $4-Pym-CH_2$ |

Table 2 (continued)

| Compound No. | $R^1$ | $R^2$ | $R^3$ |
|---|---|---|---|
| 2-2025 | Ph | 2-MeNH-4-Pyr | 5-Pym-$CH_2$ |
| 2-2026 | Ph | 2-MeNH-4-Pyr | 2-Pym-$CH_2$ |
| 2-2027 | 4-F-Ph | 2-MeNH-4-Pyr | $H_2N-(CH_2)_3$ |
| 2-2028 | 4-F-Ph | 2-MeNH-4-Pyr | MeNH-$(CH_2)_3$ |
| 2-2029 | 4-F-Ph | 2-MeNH-4-Pyr | EtNH-$(CH_2)_3$ |
| 2-2030 | 4-F-Ph | 2-MeNH-4-Pyr | $Me_2N-(CH_2)_3$ |
| 2-2031 | 4-F-Ph | 2-MeNH-4-Pyr | 1-Azt-$(CH_2)_3$ |
| 2-2032 | 4-F-Ph | 2-MeNH-4-Pyr | 1-Pyrd-$(CH_2)_3$ |
| 2-2033 | 4-F-Ph | 2-MeNH-4-Pyr | 1-Pip-$(CH_2)_3$ |
| 2-2034 | 4-F-Ph | 2-MeNH-4-Pyr | 1-Mor-$(CH_2)_3$ |
| 2-2035 | 4-F-Ph | 2-MeNH-4-Pyr | 1-Tmor-$(CH_2)_3$ |
| 2-2036 | 4-F-Ph | 2-MeNH-4-Pyr | 1-Piz-$(CH_2)_3$ |
| 2-2037 | 4-F-Ph | 2-MeNH-4-Pyr | 4-Me-1-Piz-$(CH_2)_3$ |
| 2-2038 | 4-F-Ph | 2-MeNH-4-Pyr | 3-Azt |
| 2-2039 | 4-F-Ph | 2-MeNH-4-Pyr | 1-Me-3-Azt |
| 2-2040 | 4-F-Ph | 2-MeNH-4-Pyr | 3-Pyrd |
| 2-2041 | 4-F-Ph | 2-MeNH-4-Pyr | 1-Me-3-Pyrd |
| 2-2042 | 4-F-Ph | 2-MeNH-4-Pyr | 4-Pip |
| 2-2043 | 4-F-Ph | 2-MeNH-4-Pyr | 4-(3,4-deH-Pip) |
| 2-2044 | 4-F-Ph | 2-MeNH-4-Pyr | 1-Me-4-Pip |
| 2-2045 | 4-F-Ph | 2-MeNH-4-Pyr | 1-Me-4-(3,4-deH-Pip) |
| 2-2046 | 4-F-Ph | 2-MeNH-4-Pyr | 1-Piz |
| 2-2047 | 4-F-Ph | 2-MeNH-4-Pyr | 4-Me-1-Piz |
| 2-2048 | 4-F-Ph | 2-MeNH-4-Pyr | 4-Pyr |
| 2-2049 | 4-F-Ph | 2-MeNH-4-Pyr | 3-Pyr |
| 2-2050 | 4-F-Ph | 2-MeNH-4-Pyr | 4-Pym |
| 2-2051 | 4-F-Ph | 2-MeNH-4-Pyr | 5-Pym |
| 2-2052 | 4-F-Ph | 2-MeNH-4-Pyr | 3-Pyrd-$CH_2$ |
| 2-2053 | 4-F-Ph | 2-MeNH-4-Pyr | 1-Me-3-Pyrd-$CH_2$ |
| 2-2054 | 4-F-Ph | 2-MeNH-4-Pyr | 4-Pip-$CH_2$ |
| 2-2055 | 4-F-Ph | 2-MeNH-4-Pyr | 1-Me-4-Pip-$CH_2$ |
| 2-2056 | 4-F-Ph | 2-MeNH-4-Pyr | 2-Piz-$CH_2$ |
| 2-2057 | 4-F-Ph | 2-MeNH-4-Pyr | 4-Pyr-$CH_2$ |
| 2-2058 | 4-F-Ph | 2-MeNH-4-Pyr | 3-Pyr-$CH_2$ |
| 2-2059 | 4-F-Ph | 2-MeNH-4-Pyr | 4-Pym-$CH_2$ |
| 2-2060 | 4-F-Ph | 2-MeNH-4-Pyr | 5-Pym-$CH_2$ |
| 2-2061 | 4-F-Ph | 2-MeNH-4-Pyr | 2-Pym-$CH_2$ |
| 2-2062 | 3-F-Ph | 2-MeNH-4-Pyr | $H_2N-(CH_2)_3$ |

Table 2 (continued)

| Compound No. | $R^1$ | $R^2$ | $R^3$ |
|---|---|---|---|
| 2-2063 | 3-F-Ph | 2-MeNH-4-Pyr | MeNH-$(CH_2)_3$ |
| 2-2064 | 3-F-Ph | 2-MeNH-4-Pyr | EtNH-$(CH_2)_3$ |
| 2-2065 | 3-F-Ph | 2-MeNH-4-Pyr | $Me_2N$-$(CH_2)_3$ |
| 2-2066 | 3-F-Ph | 2-MeNH-4-Pyr | 1-Azt-$(CH_2)_3$ |
| 2-2067 | 3-F-Ph | 2-MeNH-4-Pyr | 1-Pyrd-$(CH_2)_3$ |
| 2-2068 | 3-F-Ph | 2-MeNH-4-Pyr | 1-Pip-$(CH_2)_3$ |
| 2-2069 | 3-F-Ph | 2-MeNH-4-Pyr | 1-Mor-$(CH_2)_3$ |
| 2-2070 | 3-F-Ph | 2-MeNH-4-Pyr | 1-Tmor-$(CH_2)_3$ |
| 2-2071 | 3-F-Ph | 2-MeNH-4-Pyr | 1-Piz-$(CH_2)_3$ |
| 2-2072 | 3-F-Ph | 2-MeNH-4-Pyr | 4-Me-1-Piz- $(CH_2)_3$ |
| 2-2073 | 3-F-Ph | 2-MeNH-4-Pyr | 3-Azt |
| 2-2074 | 3-F-Ph | 2-MeNH-4-Pyr | 1-Me-3-Azt |
| 2-2075 | 3-F-Ph | 2-MeNH-4-Pyr | 3-Pyrd |
| 2-2076 | 3-F-Ph | 2-MeNH-4-Pyr | 1-Me-3-Pyrd |
| 2-2077 | 3-F-Ph | 2-MeNH-4-Pyr | 4-Pip |
| 2-2078 | 3-F-Ph | 2-MeNH-4-Pyr | 4-(3,4-deH-Pip) |
| 2-2079 | 3-F-Ph | 2-MeNH-4-Pyr | 1-Me-4-Pip |
| 2-2080 | 3-F-Ph | 2-MeNH-4-Pyr | 1-Me-4-(3,4-deH-Pip) |
| 2-2081 | 3-F-Ph | 2-MeNH-4-Pyr | 1-Piz |
| 2-2082 | 3-F-Ph | 2-MeNH-4-Pyr | 4-Me-1-Piz |
| 2-2083 | 3-F-Ph | 2-MeNH-4-Pyr | 4-Pyr |
| 2-2084 | 3-F-Ph | 2-MeNH-4-Pyr | 3-Pyr |
| 2-2085 | 3-F-Ph | 2-MeNH-4-Pyr | 4-Pym |
| 2-2086 | 3-F-Ph | 2-MeNH-4-Pyr | 5-Pym |
| 2-2087 | 3-F-Ph | 2-MeNH-4-Pyr | 3-Pyrd-$CH_2$ |
| 2-2088 | 3-F-Ph | 2-MeNH-4-Pyr | 1-Me-3-Pyrd-$CH_2$ |
| 2-2089 | 3-F-Ph | 2-MeNH-4-Pyr | 4-Pip-$CH_2$ |
| 2-2090 | 3-F-Ph | 2-MeNH-4-Pyr | 1-Me-4-Pip-$CH_2$ |
| 2-2091 | 3-F-Ph | 2-MeNH-4-Pyr | 2-Piz-$CH_2$ |
| 2-2092 | 3-F-Ph | 2-MeNH-4-Pyr | 4-Pyr-$CH_2$ |
| 2-2093 | 3-F-Ph | 2-MeNH-4-Pyr | 3-Pyr-$CH_2$ |
| 2-2094 | 3-F-Ph | 2-MeNH-4-Pyr | 4-Pym-$CH_2$ |
| 2-2095 | 3-F-Ph | 2-MeNH-4-Pyr | 5-Pym-$CH_2$ |
| 2-2096 | 3-F-Ph | 2-MeNH-4-Pyr | 2-Pym-$CH_2$ |
| 2-2097 | 3,4-diF-Ph | 2-MeNH-4-Pyr | $H_2N$-$(CH_2)_3$ |
| 2-2098 | 3,4-diF-Ph | 2-MeNH-4-Pyr | MeNH-$(CH_2)_3$ |
| 2-2099 | 3,4-diF-Ph | 2-MeNH-4-Pyr | EtNH-$(CH_2)_3$ |
| 2-2100 | 3,4-diF-Ph | 2-MeNH-4-Pyr | $Me_2N$-$(CH_2)_3$ |

Table 2 (continued)

| Compound No. | $R^1$ | $R^2$ | $R^3$ |
|---|---|---|---|
| 2-2101 | 3,4-diF-Ph | 2-MeNH-4-Pyr | 1-Azt-$(CH_2)_3$ |
| 2-2102 | 3,4-diF-Ph | 2-MeNH-4-Pyr | 1-Pyrd-$(CH_2)_3$ |
| 2-2103 | 3,4-diF-Ph | 2-MeNH-4-Pyr | 1-Pip-$(CH_2)_3$ |
| 2-2104 | 3,4-diF-Ph | 2-MeNH-4-Pyr | 1-Mor-$(CH_2)_3$ |
| 2-2105 | 3,4-diF-Ph | 2-MeNH-4-Pyr | 1-Tmor-$(CH_2)_3$ |
| 2-2106 | 3,4-diF-Ph | 2-MeNH-4-Pyr | 1-Piz-$(CH_2)_3$ |
| 2-2107 | 3,4-diF-Ph | 2-MeNH-4-Pyr | 4-Me-1-Piz-$(CH_2)_3$ |
| 2-2108 | 3,4-diF-Ph | 2-MeNH-4-Pyr | 3-Azt |
| 2-2109 | 3,4-diF-Ph | 2-MeNH-4-Pyr | 1-Me-3-Azt |
| 2-2110 | 3,4-diF-Ph | 2-MeNH-4-Pyr | 3-Pyrd |
| 2-2111 | 3,4-diF-Ph | 2-MeNH-4-Pyr | 1-Me-3-Pyrd |
| 2-2112 | 3,4-diF-Ph | 2-MeNH-4-Pyr | 4-Pip |
| 2-2113 | 3,4-diF-Ph | 2-MeNH-4-Pyr | 4-(3,4-deH-Pip) |
| 2-2114 | 3,4-diF-Ph | 2-MeNH-4-Pyr | 1-Me-4-Pip |
| 2-2115 | 3,4-diF-Ph | 2-MeNH-4-Pyr | 1-Me-4-(3,4-deH-Pip) |
| 2-2116 | 3,4-diF-Ph | 2-MeNH-4-Pyr | 1-Piz |
| 2-2117 | 3,4-diF-Ph | 2-MeNH-4-Pyr | 4-Me-1-Piz |
| 2-2118 | 3,4-diF-Ph | 2-MeNH-4-Pyr | 4-Pyr |
| 2-2119 | 3,4-diF-Ph | 2-MeNH-4-Pyr | 3-Pyr |
| 2-2120 | 3,4-diF-Ph | 2-MeNH-4-Pyr | 4-Pym |
| 2-2121 | 3,4-diF-Ph | 2-MeNH-4-Pyr | 5-Pym |
| 2-2122 | 3,4-diF-Ph | 2-MeNH-4-Pyr | 3-pyrd-$CH_2$ |
| 2-2123 | 3,4-diF-Ph | 2-MeNH-4-Pyr | 1-Me-3-Pyrd-$CH_2$ |
| 2-2124 | 3,4-diF-Ph | 2-MeNH-4-Pyr | 4-Pip-$CH_2$ |
| 2-2125 | 3,4-diF-Ph | 2-MeNH-4-Pyr | 1-Me-4-Pip-$CH_2$ |
| 2-2126 | 3,4-diF-Ph | 2-MeNH-4-Pyr | 2-Piz-$CH_2$ |
| 2-2127 | 3,4-diF-Ph | 2-MeNH-4-Pyr | 4-Pyr-$CH_2$ |
| 2-2128 | 3,4-diF-Ph | 2-MeNH-4-Pyr | 3-Pyr-$CH_2$ |
| 2-2129 | 3,4-diF-Ph | 2-MeNH-4-Pyr | 4-Pym-$CH_2$ |
| 2-2130 | 3,4-diF-Ph | 2-MeNH-4-Pyr | 5-Pym-$CH_2$ |
| 2-2131 | 3,4-diF-Ph | 2-MeNH-4-Pyr | 2-Pym-$CH_2$ |
| 2-2132 | 3-Cl-Ph | 2-MeNH-4-Pyr | $H_2N$-$(CH_2)_3$ |
| 2-2133 | 3-Cl-Ph | 2-MeNH-4-Pyr | MeNH-$(CH_2)_3$ |
| 2-2134 | 3-Cl-Ph | 2-MeNH-4-Pyr | EtNH-$(CH_2)_3$ |
| 2-2135 | 3-Cl-Ph | 2-MeNH-4-Pyr | Me2N-$(CH_2)_3$ |
| 2-2136 | 3-Cl-Ph | 2-MeNH-4-Pyr | 1-Azt-$(CH_2)_3$ |
| 2-2137 | 3-Cl-Ph | 2-MeNH-4-Pyr | 1-Pyrd-$(CH_Z)_3$ |
| 2-2138 | 3-Cl-Ph | 2-MeNH-4-Pyr | 1-Pip-$(CH_2)_3$ |

Table 2 (continued)

| Compound No. | $R^1$ | $R^2$ | $R^3$ |
|---|---|---|---|
| 2-2139 | 3-Cl-Ph | 2-MeNH-4-Pyr | l-Mor-$(CH_2)_3$ |
| 2-2140 | 3-Cl-Ph | 2-MeNH-4-Pyr | 1-Tmor-$(CH_2)_3$ |
| 2-2141 | 3-Cl-Ph | 2-MeNH-4-Pyr | 1-Piz-$(CH_2)_3$ |
| 2-2142 | 3-Cl-Ph | 2-MeNH-4-Pyr | 4-Me-1-Piz-$(CH_2)_3$ |
| 2-2143 | 3-Cl-Ph | 2-MeNH-4-Pyr | 3-Azt |
| 2-2144 | 3-Cl-Ph | 2-MeNH-4-Pyr | 1-Me-3-Azt |
| 2-2145 | 3-Cl-Ph | 2-MeNH-4-Pyr | 3-Pyrd |
| 2-2146 | 3-Cl-Ph | 2-MeNH-4-Pyr | 1-Me-3-Pyrd |
| 2-2147 | 3-Cl-Ph | 2-MeNH-4-Pyr | 4-Pip |
| 2-2148 | 3-Cl-Ph | 2-MeNH-4-Pyr | 4-(3,4-deH-Pip) |
| 2-2149 | 3-Cl-Ph | 2-MeNH-4-Pyr | 1-Me-4-Pip |
| 2-2150 | 3-Cl-Ph | 2-MeNH-4-Pyr | 1-Me-4-(3,4-deH-Pip) |
| 2-2151 | 3-Cl-Ph | 2-MeNH-4-Pyr | 1-Piz |
| 2-2152 | 3-Cl-Ph | 2-MeNH-4-Pyr | 4-Me-1-Piz |
| 2-2153 | 3-Cl-Ph | 2-MeNH-4-Pyr | 4-Pyr |
| 2-2154 | 3-Cl-Ph | 2-MeNH-4-Pyr | 3-Pyr |
| 2-2155 | 3-Cl-Ph | 2-MeNH-4-Pyr | 4-Pym |
| 2-2156 | 3-Cl-Ph | 2-MeNH-4-Pyr | 5-Pym |
| 2-2157 | 3-Cl-Ph | 2-MeNH-4-Pyr | 3-Pyrd-$CH_2$ |
| 2-2158 | 3-Cl-Ph | 2-MeNH-4-Pyr | 1-Me-3-Pyrd-$CH_2$ |
| 2-2159 | 3-Cl-Ph | 2-MeNH-4-Pyr | 4-Pip-$CH_2$ |
| 2-2160 | 3-Cl-Ph | 2-MeNH-4-Pyr | 1-Me-4-Pip-$CH_2$ |
| 2-2161 | 3-Cl-Ph | 2-MeNH-4-Pyr | 2-Piz-$CH_2$ |
| 2-2162 | 3-Cl-Ph | 2-MeNH-4-Pyr | 4-Pyr-$CH_2$ |
| 2-2163 | 3-Cl-Ph | 2-MeNH-4-Pyr | 3-Pyr-$CH_2$ |
| 2-2164 | 3-Cl-Ph | 2-MeNH-4-Pyr | 4-Pym-$CH_2$ |
| 2-2165 | 3-Cl-Ph | 2-MeNH-4-Pyr | 5-Pym-$CH_2$ |
| 2-2166 | 3-Cl-Ph | 2-MeNH-4-Pyr | 2-Pym-$CH_2$ |
| 2-2167 | Ph | 4-Pym | $H_2N$-$(CH_2)_3$ |
| 2-2168 | Ph | 4-Pym | MeNH-$(CH_2)_3$ |
| 2-2169 | Ph | 4-Pym | EtNH-$(CH_2)_3$ |
| 2-2170 | Ph | 4-Pym | $Me_2N$-$(CH_2)_3$ |
| 2-2171 | Ph | 4-Pym | 1-Azt-$(CH_2)_3$ |
| 2-2172 | Ph | 4-Pym | 1-Pyrd-$(CH_2)_3$ |
| 2-2173 | Ph | 4-Pym | 1-Pip-$(CH_2)_3$ |
| 2-2174 | Ph | 4-Pym | 1-Mor-$(CH_2)_3$ |
| 2-2175 | Ph | 4-Pym | 1-Tmor-$(CH_2)_3$ |
| 2-2176 | Ph | 4-Pym | 1-Piz- $(CH_2)_3$ |

Table 2 (continued)

| Compound No. | $R^1$ | $R^2$ | $R^3$ |
|---|---|---|---|
| 2-2177 | Ph | 4-Pym | 4-Me-1-Piz-$(CH_2)_3$ |
| 2-2178 | Ph | 4-Pym | 3-Azt |
| 2-2179 | Ph | 4-Pym | 1-Me-3-Azt |
| 2-2180 | Ph | 4-Pym | 3-Pyrd |
| 2-2181 | Ph | 4-Pym | 1-Me-3-Pyrd |
| 2-2182 | Ph | 4-Pym | 4-Pip |
| 2-2183 | Ph | 4-Pym | 4-(3,4-deH-Pip) |
| 2-2184 | Ph | 4-Pym | 1-Me-4-Pip |
| 2-2185 | Ph | 4-Pym | 1-Me-4-(3,4-deH-Pip) |
| 2-2186 | Ph | 4-Pym | 1-Piz |
| 2-2187 | Ph | 4-Pym | 4-Me-1-Piz |
| 2-2188 | Ph | 4-Pym | 4-Pyr |
| 2-2189 | Ph | 4-Pym | 3-Pyr |
| 2-2190 | Ph | 4-Pym | 4-Pym |
| 2-2191 | Ph | 4-Pym | 5-Pym |
| 2-2192 | Ph | 4-Pym | 3-Pyrd-$CH_2$ |
| 2-2193 | Ph | 4-Pym | 1-Me-3-Pyrd-$CH_2$ |
| 2-2194 | Ph | 4-Pym | 4-Pip-$CH_2$ |
| 2-2195 | Ph | 4-Pym | 1-Me-4-Pip-$CH_2$ |
| 2-2196 | Ph | 4-Pym | 2-Piz-$CH_2$ |
| 2-2197 | Ph | 4-Pym | 4-Pyr-$CH_2$ |
| 2-2198 | Ph | 4-Pym | 3-Pyr-$CH_2$ |
| 2-2199 | Ph | 4-Pym | 4-Pym-$CH_2$ |
| 2-2200 | Ph | 4-Pym | 5-Pym-$CH_2$ |
| 2-2201 | Ph | 4-Pym | 2-Pym-$CH_2$ |
| 2-2202 | 4-F-Ph | 4-Pym | $H_2N$-$(CH_2)_3$ |
| 2-2203 | 4-F-Ph | 4-Pym | MeNH-$(CH_2)_3$ |
| 2-2204 | 4-F-Ph | 4-Pym | EtNH-$(CH_2)_3$ |
| 2-2205 | 4-F-Ph | 4-Pym | $Me_2N$-$(CH_2)_3$ |
| 2-2206 | 4-F-Ph | 4-Pym | 1-Azt-$(CH_2)_3$ |
| 2-2207 | 4-F-Ph | 4-Pym | 1-Pyrd-$(CH_2)_3$ |
| 2-2208 | 4-F-Ph | 4-Pym | 1-Pip-$(CH_2)_3$ |
| 2-2209 | 4-F-Ph | 4-Pym | 1-Mor-$(CH_2)_3$ |
| 2-2210 | 4-F-Ph | 4-Pym | 1-Tmor-$(CH_2)_3$ |
| 2-2211 | 4-F-Ph | 4-Pym | 1-Piz-$(CH_2)_3$ |
| 2-2212 | 4-F-Ph | 4-Pym | 4-Me-1-Piz-$(CH_2)_3$ |
| 2-2213 | 4-F-Ph | 4-Pym | 3-Azt |
| 2-2214 | 4-F-Ph | 4-Pym | 1-Me-3-Azt |

Table 2 (continued)

| Compound No. | $R^1$ | $R^2$ | $R^3$ |
|---|---|---|---|
| 2-2215 | 4-F-Ph | 4-Pym | 3-Pyrd |
| 2-2216 | 4-F-Ph | 4-Pym | 1-Me-3-Pyrd |
| 2-2217 | 4-F-Ph | 4-Pym | 4-Pip |
| 2-2218 | 4-F-Ph | 4-Pym | 4-(3,4-deH-Pip) |
| 2-2219 | 4-F-Ph | 4-Pym | 1-Me-4-Pip |
| 2-2220 | 4-F-Ph | 4-Pym | 1-Me-4-(3,4-deH-Pip) |
| 2-2221 | 4-F-Ph | 4-Pym | 1-Piz |
| 2-2222 | 4-F-Ph | 4-Pym | 4-Me-1-Piz |
| 2-2223 | 4-F-Ph | 4-Pym | 4-Pyr |
| 2-2224 | 4-F-Ph | 4-Pym | 3-Pyr |
| 2-2225 | 4-F-Ph | 4-Pym | 4-Pym |
| 2-2226 | 4-F-Ph | 4-Pym | 5-Pym |
| 2-2227 | 4-F-Ph | 4-Pym | 3-Pyrd-$CH_2$ |
| 2-2228 | 4-F-Ph | 4-Pym | 1-Me-3-Pyrd-$CH_2$ |
| 2-2229 | 4-F-Ph | 4-Pym | 4-Pip-$CH_2$ |
| 2-2230 | 4-F-Ph | 4-Pym | 1-Me-4-Pip-$CH_2$ |
| 2-2231 | 4-F-Ph | 4-Pym | 2-Piz-$CH_2$ |
| 2-2232 | 4-F-Ph | 4-Pym | 4-Pyr-$CH_2$ |
| 2-2233 | 4-F-Ph | 4-Pym | 3-Pyr-$CH_2$ |
| 2-2234 | 4-F-Ph | 4-Pym | 4-Pym-$CH_2$ |
| 2-2235 | 4-F-Ph | 4-Pym | 5-Pym-$CH_2$ |
| 2-2236 | 4-F-Ph | 4-Pym | 2-Pym-$CH_2$ |
| 2-2237 | 3-F-Ph | 4-Pym | $H_2N-(CH_2)_3$ |
| 2-2238 | 3-F-Ph | 4-Pym | $MeNH-(CH_2)_3$ |
| 2-2239 | 3-F-Ph | 4-Pym | $EtNH-(CH_2)_3$ |
| 2-2240 | 3-F-Ph | 4-Pym | $Me_2N-(CH_2)_3$ |
| 2-2241 | 3-F-Ph | 4-Pym | 1-Azt-$(CH_2)_3$ |
| 2-2242 | 3-F-Ph | 4-Pym | 1-Pyrd-$(CH_2)_3$ |
| 2-2243 | 3-F-Ph | 4-Pym | 1-Pip-$(CH_2)_3$ |
| 2-2244 | 3-F-Ph | 4-Pym | 1-Mor-$(CH_2)_3$ |
| 2-2245 | 3-F-Ph | 4-Pym | 1-Tmor-$(CH_2)_3$ |
| 2-2246 | 3-F-Ph | 4-Pym | 1-Piz-$(CH_2)_3$ |
| 2-2247 | 3-F-Ph | 4-Pym | 4-Me-1-Piz-$(CH_2)_3$ |
| 2-2248 | 3-F-Ph | 4-Pym | 3-Azt |
| 2-2249 | 3-F-Ph | 4-Pym | 1-Me-3-Azt |
| 2-2250 | 3-F-Ph | 4-Pym | 3-Pyrd |
| 2-2251 | 3-F-Ph | 4-Pym | 1-Me-3-Pyrd |
| 2-2252 | 3-F-Ph | 4-Pym | 4-Pip |

Table 2 (continued)

| Compound No. | $R^1$ | $R^2$ | $R^3$ |
|---|---|---|---|
| 2-2253 | 3-F-Ph | 4-Pym | 4-(3, 4-deH-Pip) |
| 2-2254 | 3-F-Ph | 4-Pym | 1-Me-4-Pip |
| 2-2255 | 3-F-Ph | 4-Pym | 1-Me-4-(3,4-deH-Pip) |
| 2-2256 | 3-F-Ph | 4-Pym | 1-Piz |
| 2-2257 | 3-F-Ph | 4-Pym | 4-Me-1-Piz |
| 2-2258 | 3-F-Ph | 4-Pym | 4-Pyr |
| 2-2259 | 3-F-Ph | 4-Pym | 3-Pyr |
| 2-2260 | 3-F-Ph | 4-Pym | 4-Pym |
| 2-2261 | 3-F-Ph | 4-Pym | 5-Pym |
| 2-2262 | 3-F-Ph | 4-Pym | 3-Pyrd-$CH_2$ |
| 2-2263 | 3-F-Ph | 4-Pym | 1-Me-3-Pyrd-$CH_2$ |
| 2-2264 | 3-F-Ph | 4-Pym | 4-Pip-$CH_2$ |
| 2-2265 | 3-F-Ph | 4-Pym | 1-Me-4-Pip-$CH_2$ |
| 2-2266 | 3-F-Ph | 4-Pym | 2-Piz-$CH_2$ |
| 2-2267 | 3-F-Ph | 4-Pym | 4-Pyr-$CH_2$ |
| 2-2268 | 3-F-Ph | 4-Pym | 3-Pyr-$CH_2$ |
| 2-2269 | 3-F-Ph | 4-Pym | 4-Pym-$CH_2$ |
| 2-2270 | 3-F-Ph | 4-Pym | 5-Pym-$CH_2$ |
| 2-2271 | 3-F-Ph | 4-Pym | 2-Pym-$CH_2$ |
| 2-2272 | 3,4-diF-Ph | 4-Pym | $H_2N$-$(CH_2)_3$ |
| 2-2273 | 3,4-diF-Ph | 4-Pym | MeNH-$(CH_2)_3$ |
| 2-2274 | 3,4-diF-Ph | 4-Pym | EtNH-$(CH_2)_3$ |
| 2-2275 | 3,4-diF-Ph | 4-Pym | $Me_2N$-$(CH_2)_3$ |
| 2-2276 | 3,4-diF-Ph | 4-Pym | 1-Azt-$(CH_2)_3$ |
| 2-2277 | 3,4-diF-Ph | 4-Pym | 1-Pyrd-$(CH_2)_3$ |
| 2-2278 | 3,4-diF-Ph | 4-Pym | 1-Pip-$(CH_2)_3$ |
| 2-2279 | 3,4-diF-Ph | 4-Pym | 1-Mor-$(CH_2)_3$ |
| 2-2280 | 3,4-diF-Ph | 4-Pym | 1-Tmor-$(CH_2)_3$ |
| 2-2281 | 3,4-diF-Ph | 4-Pym | 1-Piz-$(CH_2)_3$ |
| 2-2282 | 3,4-diF-Ph | 4-Pym | 4-Me-1-Piz-$(CH_2)_3$ |
| 2-2283 | 3,4-diF-Ph | 4-Pym | 3-Azt |
| 2-2284 | 3,4-diF-Ph | 4-Pym | 1-Me-3-Azt |
| 2-2285 | 3,4-diF-Ph | 4-Pym | 3-Pyrd |
| 2-2286 | 3,4-diF-Ph | 4-Pym | 1-Me-3-Pyrd |
| 2-2287 | 3,4-diF-Ph | 4-Pym | 4-Pip |
| 2-2288 | 3,4-diF-Ph | 4-Pym | 4-(3,4-deH-Pip) |
| 2-2289 | 3,4-diF-Ph | 4-Pym | 1-Me-4-Pip |
| 2-2290 | 3,4-diF-Ph | 4-Pym | 1-Me-4-(3,4-deH-Pip) |

Table 2 (continued)

| Compound No. | $R^1$ | $R^2$ | $R^3$ |
|---|---|---|---|
| 2-2291 | 3,4-diF-Ph | 4-Pym | 1-Piz |
| 2-2292 | 3,4-diF-Ph | 4-Pym | 4-Me-1-Piz |
| 2-2293 | 3,4-diF-Ph | 4-Pym | 4-Pyr |
| 2-2294 | 3,4-diF-Ph | 4-Pym | 3-Pyr |
| 2-2295 | 3,4-diF-Ph | 4-Pym | 4-Pym |
| 2-2296 | 3,4-diF-Ph | 4-Pym | 5-Pym |
| 2-2297 | 3,4-diF-Ph | 4-Pym | 3-Pyrd-$CH_2$ |
| 2-2298 | 3,4-diF-Ph | 4-Pym | 1-Me-3-Pyrd-$CH_2$ |
| 2-2299 | 3,4-diF-Ph | 4-Pym | 4-Pip-$CH_2$ |
| 2-2300 | 3,4-diF-Ph | 4-Pym | 1-Me-4-Pip-$CH_2$ |
| 2-2301 | 3,4-diF-Ph | 4-Pym | 2-Piz-$CH_2$ |
| 2-2302 | 3,4-diF-Ph | 4-Pym | 4-Pyr-$CH_2$ |
| 2-2303 | 3,4-diF-Ph | 4-Pym | 3-Pyr-$CH_2$ |
| 2-2304 | 3,4-diF-Ph | 4-Pym | 4-Pym-$CH_2$ |
| 2-2305 | 3,4-diF-Ph | 4-Pym | 5-Pym-$CH_2$ |
| 2-2306 | 3,4-diF-Ph | 4-Pym | 2-Pym-$CH_2$ |
| 2-2307 | 3-Cl-Ph | 4-Pym | $H_2N-(CH_2)_3$ |
| 2-2308 | 3-Cl-Ph | 4-Pym | $MeNH-(CH_2)_3$ |
| 2-2309 | 3-Cl-Ph | 4-Pym | $EtNH-(CH_2)_3$ |
| 2-2310 | 3-Cl-Ph | 4-Pym | $Me_2N-(CH_2)_3$ |
| 2-2311 | 3-Cl-Ph | 4-Pym | 1-Azt-$(CH_2)_3$ |
| 2-2312 | 3-Cl-Ph | 4-Pym | 1-Pyrd-$(CH_2)_3$ |
| 2-2313 | 3-Cl-Ph | 4-Pym | 1-Pip-$(CH_2)_3$ |
| 2-2314 | 3-Cl-Ph | 4-Pym | 1-Mor-$(CH_2)_3$ |
| 2-2315 | 3-Cl-Ph | 4-Pym | 1-Tmor-$(CH_2)_3$ |
| 2-2316 | 3-Cl-Ph | 4-Pym | 1-Piz-$(CH_2)_3$ |
| 2-2317 | 3-Cl-Ph | 4-Pym | 4-Me-1-Piz-$(CH_2)_3$ |
| 2-2318 | 3-Cl-Ph | 4-Pym | 3-Azt |
| 2-2319 | 3-Cl-Ph | 4-Pym | 1-Me-3-Azt |
| 2-2320 | 3-Cl-Ph | 4-Pym | 3-Pyrd |
| 2-2321 | 3-Cl-Ph | 4-Pym | 1-Me-3-Pyrd |
| 2-2322 | 3-Cl-Ph | 4-Pym | 4-Pip |
| 2-2323 | 3-Cl-Ph | 4-Pym | 4-(3,4-deH-Pip) |
| 2-2324 | 3-Cl-Ph | 4-Pym | 1-Me-4-Pip |
| 2-2325 | 3-Cl-Ph | 4-Pym | 1-Me-4-(3,4-deH-Pip) |
| 2-2326 | 3-Cl-Ph | 4-Pym | 1-Piz |
| 2-2327 | 3-Cl-Ph | 4-Pym | 4-Me-1-Piz |
| 2-2328 | 3-Cl-Ph | 4-Pym | 4-Pyr |

Table 2 (continued)

| Compound No. | $R^1$ | $R^2$ | $R^3$ |
|---|---|---|---|
| 2-2329 | 3-Cl-Ph | 4-Pym | 3-Pyr |
| 2-2330 | 3-Cl-Ph | 4-Pym | 4-Pym |
| 2-2331 | 3-Cl-Ph | 4-Pym | 5-Pym |
| 2-2332 | 3-Cl-Ph | 4-Pym | 3-Pyrd-$CH_2$ |
| 2-2333 | 3-Cl-Ph | 4-Pym | 1-Me-3-Pyrd-$CH_2$ |
| 2-2334 | 3-Cl-Ph | 4-Pym | 4-Pip-$CH_2$ |
| 2-2335 | 3-Cl-Ph | 4-Pym | 1-Me-4-Pip-$CH_2$ |
| 2-2336 | 3-Cl-Ph | 4-Pym | 2-Piz-$CH_2$ |
| 2-2337 | 3-Cl-Ph | 4-Pym | 4-Pyr-$CH_2$ |
| 2-2338 | 3-Cl-Ph | 4-Pym | 3-Pyr-$CH_2$ |
| 2-2339 | 3-Cl-Ph | 4-Pym | 4-Pym-$CH_2$ |
| 2-2340 | 3-Cl-Ph | 4-Pym | 5-Pym-$CH_2$ |
| 2-2341 | 3-Cl-Ph | 4-Pym | 2-Pym-$CH_2$ |
| 2-2342 | Ph | 2-MeO-4-Pym | $H_2N$-$(CH_2)_3$ |
| 2-2343 | Ph | 2-MeO-4-Pym | MeNH-$(CH_2)_3$ |
| 2-2344 | Ph | 2-MeO-4-Pym | EtNH-$(CH_2)_3$ |
| 2-2345 | Ph | 2-MeO-4-Pym | $Me_2N$-$(CH_2)_3$ |
| 2-2346 | Ph | 2-MeO-4-Pym | 1-Azt-$(CH_2)_3$ |
| 2-2347 | Ph | 2-MeO-4-Pym | 1-Pyrd-$(CH_2)_3$ |
| 2-2348 | Ph | 2-MeO-4-Pym | 1-Pip-$(CH_2)_3$ |
| 2-2349 | Ph | 2-MeO-4-Pym | 1-Mor-$(CH_2)_3$ |
| 2-2350 | Ph | 2-MeO-4-Pym | 1-Tmor-$(CH_2)_3$ |
| 2-2351 | Ph | 2-MeO-4-Pym | 1-Piz-$(CH_2)_3$ |
| 2-2352 | Ph | 2-MeO-4-Pym | 4-Me-1-Piz-$(CH_2)_3$ |
| 2-2353 | Ph | 2-MeO-4-Pym | 3-Azt |
| 2-2354 | Ph | 2-MeO-4-Pym | 1-Me-3-Azt |
| 2-2355 | Ph | 2-MeO-4-Pym | 3-Pyrd |
| 2-2356 | Ph | 2-MeO-4-Pym | 1-Me-3-Pyrd |
| 2-2357 | Ph | 2-MeO-4-Pym | 4-Pip |
| 2-2358 | Ph | 2-MeO-4-Pym | 4-(3,4-deH-Pip) |
| 2-2359 | Ph | 2-MeO-4-Pym | 1-Me-4-Pip |
| 2-2360 | Ph | 2-MeO-4-Pym | 1-Me-4-(3,4-deH-Pip) |
| 2-2361 | Ph | 2-MeO-4-Pym | 1-Piz |
| 2-2362 | Ph | 2-MeO-4-Pym | 4-Me-1-Piz |
| 2-2363 | Ph | 2-MeO-4-Pym | 4-Pyr |
| 2-2364 | Ph | 2-MeO-4-Pym | 3-Pyr |
| 2-2365 | Ph | 2-MeO-4-Pym | 4-Pym |
| 2-2366 | Ph | 2-MeO-4-Pym | 5-Pym |

Table 2 (continued)

| Compound No. | $R^1$ | $R^2$ | $R^3$ |
|---|---|---|---|
| 2-2367 | Ph | 2-MeO-4-Pym | 3-Pyrd-$CH_2$ |
| 2-2368 | Ph | 2-MeO-4-Pym | 1-Me-3-Pyrd-$CH_2$ |
| 2-2369 | Ph | 2-MeO-4-Pym | 4-Pip-$CH_2$ |
| 2-2370 | Ph | 2-MeO-4-Pym | 1-Me-4-Pip-$CH_2$ |
| 2-2371 | Ph | 2-MeO-4-Pym | 2-Piz-$CH_2$ |
| 2-2372 | Ph | 2-MeO-4-Pym | 4-Pyr-$CH_2$ |
| 2-2373 | Ph | 2-MeO-4-Pym | 3-Pyr-$CH_2$ |
| 2-2374 | Ph | 2-MeO-4-Pym | 4-Pym-$CH_2$ |
| 2-2375 | Ph | 2-MeO-4-Pym | 5-Pym-$CH_2$ |
| 2-2376 | Ph | 2-MeO-4-Pym | 2-Pym-$CH_2$ |
| 2-2377 | 4-F-Ph | 2-MeO-4-Pym | $H_2N-(CH_2)_3$ |
| 2-2378 | 4-F-Ph | 2-MeO-4-Pym | $MeNH-(CH_2)_3$ |
| 2-2379 | 4-F-Ph | 2-MeO-4-Pym | $EtNH-(CH_2)_3$ |
| 2-2380 | 4-F-Ph | 2-MeO-4-Pym | $Me_2N-(CH_2)_3$ |
| 2-2381 | 4-F-Ph | 2-MeO-4-Pym | 1-Azt-$(CH_2)_3$ |
| 2-2382 | 4-F-Ph | 2-MeO-4-Pym | 1-Pyrd-$(CH_2)_3$ |
| 2-2383 | 4-F-Ph | 2-MeO-4-Pym | 1-Pip-$(CH_2)_3$ |
| 2-2384 | 4-F-Ph | 2-MeO-4-Pym | 1-Mor-$(CH_2)_3$ |
| 2-2385 | 4-F-Ph | 2-MeO-4-Pym | 1-Tmor-$(CH_2)_3$ |
| 2-2386 | 4-F-Ph | 2-MeO-4-Pym | 1-Piz-$(CH_2)_3$ |
| 2-2387 | 4-F-Ph | 2-MeO-4-Pym | 4-Me-1-Piz-$(CH_2)_3$ |
| 2-2388 | 4-F-Ph | 2-MeO-4-Pym | 3-Azt |
| 2-2389 | 4-F-Ph | 2-MeO-4-Pym | 1-Me-3-Azt |
| 2-2390 | 4-F-Ph | 2-MeO-4-Pym | 3-Pyrd |
| 2-2391 | 4-F-Ph | 2-MeO-4-Pym | 1-Me-3-Pyrd |
| 2-2392 | 4-F-Ph | 2-MeO-4-Pym | 4-Pip |
| 2-2393 | 4-F-Ph | 2-MeO-4-Pym | 4-(3,4-deH-Pip) |
| 2-2394 | 4-F-Ph | 2-MeO-4-Pym | 1-Me-4-Pip |
| 2-2395 | 4-F-Ph | 2-MeO-4-Pym | 1-Me-4-(3,4-deH-Pip) |
| 2-2396 | 4-F-Ph | 2-MeO-4-Pym | 1-Piz |
| 2-2397 | 4-F-Ph | 2-MeO-4-Pym | 4-Me-1-Piz |
| 2-2398 | 4-F-Ph | 2-MeO-4-Pym | 4-Pyr |
| 2-2399 | 4-F-Ph | 2-MeO-4-Pym | 3-Pyr |
| 2-2400 | 4-F-Ph | 2-MeO-4-Pym | 4-Pym |
| 2-2401 | 4-F-Ph | 2-MeO-4-Pym | 5-Pym |
| 2-2402 | 4-F-Ph | 2-MeO-4-Pym | 3-Pyrd-$CH_2$ |
| 2-2403 | 4-F-Ph | 2-MeO-4-Pym | 1-Me-3-Pyrd-$CH_2$ |
| 2-2404 | 4-F-Ph | 2-MeO-4-Pym | 4-Pip-$CH_2$ |

Table 2 (continued)

| Compound No. | $R^1$ | $R^2$ | $R^3$ |
|---|---|---|---|
| 2-2405 | 4-F-Ph | 2-MeO-4-Pym | 1-Me-4-Pip-$CH_2$ |
| 2-2406 | 4-F-Ph | 2-MeO-4-Pym | 2-Piz-$CH_2$ |
| 2-2407 | 4-F-Ph | 2-MeO-4-Pym | 4-Pyr-$CH_2$ |
| 2-2408 | 4-F-Ph | 2-MeO-4-Pym | 3-Pyr-$CH_2$ |
| 2-2409 | 4-F-Ph | 2-MeO-4-Pym | 4-Pym-$CH_2$ |
| 2-2410 | 4-F-Ph | 2-MeO-4-Pym | 5-Pym-$CH_2$ |
| 2-2411 | 4-F-Ph | 2-MeO-4-Pym | 2-Pym-$CH_2$ |
| 2-2412 | 3-F-Ph | 2-MeO-4-Pym | $H_2N-(CH_2)_3$ |
| 2-2413 | 3-F-Ph | 2-MeO-4-Pym | $MeNH-(CH_2)_3$ |
| 2-2414 | 3-F-Ph | 2-MeO-4-Pym | $EtNH-(CH_2)_3$ |
| 2-2415 | 3-F-Ph | 2-MeO-4-Pym | $Me_2N-(CH_2)_3$ |
| 2-2416 | 3-F-Ph | 2-MeO-4-Pym | 1-Azt-$(CH_2)_3$ |
| 2-2417 | 3-F-Ph | 2-MeO-4-Pym | 1-Pyrd-$(CH_2)_3$ |
| 2-2418 | 3-F-Ph | 2-MeO-4-Pym | 1-Pip-$(CH_2)_3$ |
| 2-2419 | 3-F-Ph | 2-MeO-4-Pym | 1-Mor-$(CH_2)_3$ |
| 2-2420 | 3-F-Ph | 2-MeO-4-Pym | 1-Tmor-$(CH_2)_3$ |
| 2-2421 | 3-F-Ph | 2-MeO-4-Pym | 1-Piz-$(CH_2)_3$ |
| 2-2422 | 3-F-Ph | 2-MeO-4-Pym | 4-Me-1-Piz-$(CH_2)_3$ |
| 2-2423 | 3-F-Ph | 2-MeO-4-Pym | 3-Azt |
| 2-2424 | 3-F-Ph | 2-MeO-4-Pym | 1-Me-3-Azt |
| 2-2425 | 3-F-Ph | 2-MeO-4-Pym | 3-Pyrd |
| 2-2426 | 3-F-Ph | 2-MeO-4-Pym | 1-Me-3-Pyrd |
| 2-2427 | 3-F-Ph | 2-MeO-4-Pym | 4-Pip |
| 2-2428 | 3-F-Ph | 2-MeO-4-Pym | 4-(3,4-deH-Pip) |
| 2-2429 | 3-F-Ph | 2-MeO-4-Pym | 1-Me-4-Pip |
| 2-2430 | 3-F-Ph | 2-MeO-4-Pym | 1-Me-4-(3,4-deH-Pip) |
| 2-2431 | 3-F-Ph | 2-MeO-4-Pym | 1-Piz |
| 2-2432 | 3-F-Ph | 2-MeO-4-Pym | 4-Me-1-Piz |
| 2-2433 | 3-F-Ph | 2-MeO-4-Pym | 4-Pyr |
| 2-2434 | 3-F-Ph | 2-MeO-4-Pym | 3-Pyr |
| 2-2435 | 3-F-Ph | 2-MeO-4-Pym | 4-Pym |
| 2-2436 | 3-F-Ph | 2-MeO-4-Pym | 5-Pym |
| 2-2437 | 3-F-Ph | 2-MeO-4-Pym | 3-Pyrd-$CH_2$ |
| 2-2438 | 3-F-Ph | 2-MeO-4-Pym | 1-Me-3-Pyrd-$CH_2$ |
| 2-2439 | 3-F-Ph | 2-MeO-4-Pym | 4-Pip-$CH_2$ |
| 2-2440 | 3-F-Ph | 2-MeO-4-Pym | 1-Me-4-Pip-$CH_2$ |
| 2-2441 | 3-F-Ph | 2-MeO-4-Pym | 2-Piz-$CH_2$ |
| 2-2442 | 3-F-Ph | 2-MeO-4-Pym | 4-Pyr-$CH_2$ |

Table 2 (continued)

| Compound No. | $R^1$ | $R^2$ | $R^3$ |
|---|---|---|---|
| 2-2443 | 3-F-Ph | 2-MeO-4-Pym | 3-Pyr-$CH_2$ |
| 2-2444 | 3-F-Ph | 2-MeO-4-Pym | 4-Pym-$CH_2$ |
| 2-2445 | 3-F-Ph | 2-MeO-4-Pym | 5-Pym-$CH_2$ |
| 2-2446 | 3-F-Ph | 2-MeO-4-Pym | 2-Pym-$CH_2$ |
| 2-2447 | 3,4-diF-Ph | 2-MeO-4-Pym | $H_2N$-$(CH_2)_3$ |
| 2-2448 | 3,4-diF-Ph | 2-MeO-4-Pym | MeNH-$(CH_2)_3$ |
| 2-2449 | 3,4-diF-Ph | 2-MeO-4-Pym | EtNH-$(CH_2)_3$ |
| 2-2450 | 3, 4-diF-Ph | 2-MeO-4-Pym | $Me_2N$-$(CH_2)_3$ |
| 2-2451 | 3,4-diF-Ph | 2-MeO-4-Pym | 1-Azt-$(CH_2)_3$ |
| 2-2452 | 3,4-diF-Ph | 2-MeO-4-Pym | 1-Pyrd-$(CH_2)_3$ |
| 2-2453 | 3,4-diF-Ph | 2-MeO-4-Pym | 1-Pip-$(CH_2)_3$ |
| 2-2454 | 3,4-diF-Ph | 2-MeO-4-Pym | 1-Mor-$(CH_2)_3$ |
| 2-2455 | 3,4-diF-Ph | 2-MeO-4-Pym | 1-Tmor-$(CH_2)_3$ |
| 2-2456 | 3, 4-diF-Ph | 2-MeO-4-Pym | 1-Piz-$(CH_2)_3$ |
| 2-2457 | 3,4-diF-Ph | 2-MeO-1-Pym | 4-Me-1-Piz-$(CH_2)_3$ |
| 2-2458 | 3,4-diF-Ph | 2-MeO-4-Pym | 3-Azt |
| 2-2459 | 3,4-diF-Ph | 2-MeO-4-Pym | 1-Me-3-Azt |
| 2-2460 | 3,4-diF-Ph | 2-MeO-4-Pym | 3-Pyrd |
| 2-2461 | 3,4-diF-Ph | 2-MeO-4-Pym | 1-Me-3-Pyrd |
| 2-2462 | 3,4-diF-Ph | 2-MeO-4-Pym | 4-Pip |
| 2-2463 | 3,4-diF-Ph | 2-MeO-4-Pym | 4-(3,4-deH-Pip) |
| 2-2464 | 3,4-diF-Ph | 2-MeO-4-Pym | 1-Me-4-Pip |
| 2-2465 | 3,4-diF-Ph | 2-MeO-4-Pym | 1-Me-4-(3,4-deH-Pip) |
| 2-2466 | 3,4-diF-Ph | 2-MeO-4-Pym | 1-Piz |
| 2-2467 | 3,4-diF-Ph | 2-MeO-4-Pym | 4-Me-1-Piz |
| 2-2468 | 3,4-diF-Ph | 2-MeO-4-Pym | 4-Pyr |
| 2-2469 | 3,4-diF-Ph | 2-MeO-4-Pym | 3-Pyr |
| 2-2470 | 3,4-diF-Ph | 2-MeO-4-Pym | 4-Pym |
| 2-2471 | 3,4-diF-Ph | 2-MeO-4-Pym | 5-Pym |
| 2-2472 | 3,4-diF-Ph | 2-MeO-4-Pym | 3-Pyrd-$CH_2$ |
| 2-2473 | 3,4-diF-Ph | 2-MeO-4-Pym | 1-Me-3-Pyrd-$CH_2$ |
| 2-2474 | 3,4-diF-Ph | 2-MeO-4-Pym | 4-Pip-$CH_2$ |
| 2-2475 | 3,4-diF-Ph | 2-MeO-4-Pym | 1-Me-4-Pip-$CH_2$ |
| 2-2476 | 3,4-diF-Ph | 2-MeO-4-Pym | 2-Piz-$CH_2$ |
| 2-2477 | 3,4-diF-Ph | 2-MeO-4-Pym | 4-Pyr-$CH_2$ |
| 2-2478 | 3,4-diF-Ph | 2-MeO-4-Pym | 3-Pyr-$CH_2$ |
| 2-2479 | 3,4-diF-Ph | 2-MeO-4-Pym | 4-Pym-$CH_2$ |
| 2-2480 | 3,4-diF-Ph | 2-MeO-4-Pym | 5-Pym-$CH_2$ |

Table 2 (continued)

| Compound No. | $R^1$ | $R^2$ | $R^3$ |
|---|---|---|---|
| 2-2481 | 3,4-diF-Ph | 2-MeO-4-Pym | 2-Pym-$CH_2$ |
| 2-2482 | 3-Cl-Ph | 2-MeO-4-Pym | $H_2N$-$(CH_2)_3$ |
| 2-2483 | 3-Cl-Ph | 2-MeO-4-Pym | MeNH-$(CH_2)_3$ |
| 2-2484 | 3-Cl-Ph | 2-MeO-4-Pym | EtNH-$(CH_2)_3$ |
| 2-2485 | 3-Cl-Ph | 2-MeO-4-Pym | $Me_2N$-$(CH_2)_3$ |
| 2-2486 | 3-Cl-Ph | 2-MeO-4-Pym | 1-Azt-$(CH_2)_3$ |
| 2-2487 | 3-Cl-Ph | 2-MeO-4-Pym | 1-Pyrd-$(CH_2)_3$ |
| 2-2488 | 3-Cl-Ph | 2-MeO-4-Pym | 1-Pip-$(CH_2)_3$ |
| 2-2489 | 3-Cl-Ph | 2-MeO-4-Pym | 1-Mor-$(CH_2)_3$ |
| 2-2490 | 3-Cl-Ph | 2-MeO-4-Pym | 1-Tmor-$(CH_2)_3$ |
| 2-2491 | 3-Cl-Ph | 2-MeO-4-Pym | 1-Piz-$(CH_2)_3$ |
| 2-2492 | 3-Cl-Ph | 2-MeO-4-Pym | 4-Me-1-Piz-$(CH_2)_3$ |
| 2-2493 | 3-Cl-Ph | 2-MeO-4-Pym | 3-Azt |
| 2-2494 | 3-Cl-Ph | 2-MeO-4-Pym | 1-Me-3-Azt |
| 2-2495 | 3-Cl-Ph | 2-MeO-4-Pym | 3-Pyrd |
| 2-2496 | 3-Cl-Ph | 2-MeO-4-Pym | 1-Me-3-Pyrd |
| 2-2497 | 3-Cl-Ph | 2-MeO-4-Pym | 4-Pip |
| 2-2498 | 3-Cl-Ph | 2-MeO-4-Pym | 4-(3,4-deH-Pip) |
| 2-2499 | 3-Cl-Ph | 2-MeO-4-Pym | 1-Me-4-Pip |
| 2-2500 | 3-Cl-Ph | 2-MeO-4-Pym | 1-Me-4-(3,4-deH-Pip) |
| 2-2501 | 3-Cl-Ph | 2-MeO-4-Pym | 1-Piz |
| 2-2502 | 3-Cl-Ph | 2-MeO-4-Pym | 4-Me-1-Piz |
| 2-2503 | 3-Cl-Ph | 2-MeO-4-Pym | 4-Pyr |
| 2-2504 | 3-Cl-Ph | 2-MeO-4-Pym | 3-Pyr |
| 2-2505 | 3-Cl-Ph | 2-MeO-4-Pym | 4-Pym |
| 2-2506 | 3-Cl-Ph | 2-MeO-4-Pym | 5-Pym |
| 2-2507 | 3-Cl-Ph | 2-Meo-4-pym | 3-Pyrd-$CH_2$ |
| 2-2508 | 3-Cl-Ph | 2-MeO-4-Pym | 1-Me-3-Pyrd-$CH_2$ |
| 2-2509 | 3-Cl-Ph | 2-MeO-4-Pym | 4-Pip-$CH_2$ |
| 2-2510 | 3-Cl-Ph | 2-MeO-4-Pym | 1-Me-4-Pip-$CH_2$ |
| 2-2511 | 3-Cl-Ph | 2-MeO-4-Pym | 2-Piz-$CH_2$ |
| 2-2512 | 3-Cl-Ph | 2-MeO-4-Pym | 4-Pyr-$CH_2$ |
| 2-2513 | 3-Cl-Ph | 2-MeO-4-Pym | 3-Pyr-$CH_2$ |
| 2-2514 | 3-Cl-Ph | 2-MeO-4-Pym | 4-Pym-$CH_2$ |
| 2-2515 | 3-Cl-Ph | 2-MeO-4-Pym | 5-Pym-$CH_2$ |
| 2-2516 | 3-Cl-Ph | 2-MeO-4-Pym | 2-Pym-$CH_2$ |
| 2-2517 | 4-F-Ph | 4-Pyr | $H_2N$-$CH_2CH$=CH |
| 2-2518 | 4-F-Ph | 4-Pyr | MeNH-$CH_2CH$=CH |

Table 2 (continued)

| Compound No. | $R^1$ | $R^2$ | $R^3$ |
|---|---|---|---|
| 2-2519 | 4-F-Ph | 4-Pyr | $Me_2N$-$CH_2CH$=CH |
| 2-2520 | 4-F-Ph | 4-Pyr | 3-Pip-$CH_2$ |
| 2-2521 | 4-F-Ph | 4-Pyr | 1-Me-3-Pip-$CH_2$ |
| 2-2522 | 4-F-Ph | 4-Pyr | 2-Me-4-Pip |
| 2-2523 | 4-F-Ph | 4-Pyr | 2,2,6,6-tetraMe-4-Pip |
| 2-2524 | 4-F-Ph | 4-Pyr | 1-Ac-4-Pip |
| 2-2525 | 4-F-Ph | 4-Pyr | 1-Ac-4-(3,4-deH-Pip) |
| 2-2526 | 4-F-Ph | 4-Pyr | 4-OH-4-Pip |
| 2-2527 | 4-F-Ph | 4-Pyr | 4-OH-1-Me-4-Pip |
| 2-2528 | 4-F-Ph | 4-Pyr | AcNH-$(CH_2)_3$ |
| 2-2529 | 4-F-Ph | 4-Pyr | 4-$NH_2$-cHx |
| 2-2530 | 4-F-Ph | 4-Pyr | 4-Pyr-CH(OH) |
| 2-2531 | 4-F-Ph | 4-Pyr | 3-Pyr-CH(OH) |
| 2-2532 | 4-F-Ph | 4-Pyr | 2-Pyr-CH(OH) |
| 2-2533 | 4-F-Ph | 4-Pyr | $CF_3CONH$-$(CH_2)_3$ |
| 2-2534 | 4-F-Ph | 4-Pyr | BzNH-$(CH_2)_3$ |
| 2-2535 | 4-F-Ph | 4-Pyr | 2,4,6-triF-BzNH-$CH_2$ |
| 2-2536 | 4-F-Ph | 4-Pyr | MeSO2NH-$(CH_2)_3$ |
| 2-2537 | 4-F-Ph | 4-Pyr | 1-NO2$(CH_2)_2$-4-Pip |
| 2-2538 | 4-F-Ph | 4-Pyr | 2,3,5,6-tetraF-4-Pyr |
| 2-2539 | 4-F-Ph | 4-Pyr | 3-Qun |
| 2-2540 | 4-F-Ph | 4-Pyr | 3-(2,3-deH-Qun) |
| 2-2541 | 4-F-Ph | 4-Pyr | 3-ABO |
| 2-2542 | 4-F-Ph | 4-Pyr | 8-Me-3-ABO |
| 2-2543 | 4-F-Ph | 4-Pyr | 3-(2,3-deH-ABO) |
| 2-2544 | 4-F-Ph | 4-Pyr | 8-Me-3-(2,3-deH-ABO) |
| 2-2545 | 4-F-Ph | 4-Pyr | 3-ABN |
| 2-2546 | 4-F-Ph | 4-Pyr | 9-Me-3-ABN |
| 2-2547 | 4-F-Ph | 4-Pyr | 3-(2,3-deH-ABN) |
| 2-2548 | 4-F-Ph | 4-Pyr | 9-Me-3-(2,3-deH-ABN) |
| 2-2549 | 4-F-Ph | 2-$NH_2$-4-Pym | $H_2N$-$CH_2CH$=CH |
| 2-2550 | 4-F-Ph | 2-$NH_2$-4-Pym | MeNH-$CH_2CH$=CH |
| 2-2551 | 4-F-Ph | 2-$NH_2$-4-Pym | $Me_2N$-$CH_2CH$=CH |
| 2-2552 | 4-F-Ph | 2-$NH_2$-4-Pym | 3-Pip-$CH_2$ |
| 2-2553 | 4-F-Ph | 2-$NH_2$-4-Pym | 1-Me-3-Pip-$CH_2$ |
| 2-2554 | 4-F-Ph | 2-$NH_2$-4-Pym | 2-Me-4-Pip |
| 2-2555 | 4-F-Ph | 2-$NH_2$-4-Pym | 2,2,6,6-tetraMe-4-Pip |
| 2-2556 | 4-F-Ph | 2-$NH_2$-4-Pym | 1-Ac-4-Pip |

Table 2 (continued)

| Compound No. | $R^1$ | $R^2$ | $R^3$ |
|---|---|---|---|
| 2-2557 | 4-F-Ph | $2-NH_2$-4-Pym | 1-Ac-4-(3,4-deH-Pip) |
| 2-2558 | 4-F-Ph | $2-NH_2$-4-Pym | 4-OH-4-Pip |
| 2-2559 | 4-F-Ph | $2-NH_2$-4-Pym | 4-OH-1-Me-4-Pip |
| 2-2560 | 4-F-Ph | $2-NH_2$-4-Pym | $AcNH-(CH_2)_3$ |
| 2-2561 | 4-F-Ph | $2-NH_2$-4-Pym | $4-NH_2$-cHx |
| 2-2562 | 4-F-Ph | $2-NH_2$-4-Pym | 3-Qun |
| 2-2563 | 4-F-Ph | $2-NH_2$-4-Pym | 3-(2,3-deH-Qun) |
| 2-2564 | 4-F-Ph | $2-NH_2$-4-Pym | 3-ABO |
| 2-2565 | 4-F-Ph | $2-NH_2$-4-Pym | 8-Me-3-ABO |
| 2-2566 | 4-F-Ph | $2-NH_2$-4-Pym | 3-(2,3-deH-ABO) |
| 2-2567 | 4-F-Ph | $2-NH_2$-4-Pym | 8-Me-3-(2,3-deH-ABO) |
| 2-2568 | 4-F-Ph | $2-NH_2$-4-Pym | 3-ABN |
| 2-2569 | 4-F-Ph | $2-NH_2$-4-Pym | 9-Me-3-ABN |
| 2-2570 | 4-F-Ph | $2-NH_2$-4-Pym | 3-(2,3-deH-ABN) |
| 2-2571 | 4-F-Ph | $2-NH_2$-4-Pym | 9-Me-3-(2,3-deH-ABN) |
| 2-2572 | 4-F-Ph | 2-MeNH-4-Pym | $H_2N-CH_2CH=CH$ |
| 2-2573 | 4-F-Ph | 2-MeNH-4-Pym | $MeNH-CH_2CH=CH$ |
| 2-2574 | 4-F-Ph | 2-MeNH-4-Pym | $Me_2N-CH_2CH=CH$ |
| 2-2575 | 4-F-Ph | 2-MeNH-4-Pym | $3-Pip-CH_2$ |
| 2-2576 | 4-F-Ph | 2-MeNH-4-Pym | $1-Me-3-Pip-CH_2$ |
| 2-2577 | 4-F-Ph | 2-MeNH-4-Pym | 2-Me-4-Pip |
| 2-2578 | 4-F-Ph | 2-MeNH-4-Pym | 2,2,6,6-tetraMe-4-Pip |
| 2-2579 | 4-F-Ph | 2-MeNH-4-Pym | 1-Ac-4-Pip |
| 2-2580 | 4-F-Ph | 2-MeNH-4-Pym | 1-Ac-4-(3,4-deH-Pip) |
| 2-2581 | 4-F-Ph | 2-MeNH-4-Pym | 4-OH-4-Pip |
| 2-2582 | 4-F-Ph | 2-MeNH-4-Pym | 4-OH-1-Me-4-Pip |
| 2-2583 | 4-F-Ph | 2-MeNH-4-Pym | $AcNH-(CH_2)_3$ |
| 2-2584 | 4-F-Ph | 2-MeNH-4-Pym | $4-NH_2$-cHx |
| 2-2585 | 4-F-Ph | 2-MeNH-4-Pym | 3-Qun |
| 2-2586 | 4-F-Ph | 2-MeNH-4-Pym | 3-(2,3-deH-Qun) |
| 2-2587 | 4-F-Ph | 2-MeNH-4-Pym | 3-ABO |
| 2-2588 | 4-F-Ph | 2-MeNH-4-Pym | 8-Me-3-ABO |
| 2-2589 | 4-F-Ph | 2-MeNH-4-Pym | 3-(2,3-deH-ABO) |
| 2-2590 | 4-F-Ph | 2-MeNH-4-Pym | 8-Me-3-(2,3-deH-ABO) |
| 2-2591 | 4-F-Ph | 2-MeNH-4-Pym | 3-ABN |
| 2-2592 | 4-F-Ph | 2-MeNH-4-Pym | 9-Me-3-ABN |
| 2-2593 | 4-F-Ph | 2-MeNH-4-Pym | 3-(2,3-deH-ABN) |
| 2-2594 | 4-F-Ph | 2-MeNH-4-Pym | 9-Me-3-(2,3-deH-ABN) |

Table 2 (continued)

| Compound No. | $R^1$ | $R^2$ | $R^3$ |
|---|---|---|---|
| 2-2595 | 4-F-Ph | 2-BnNH-4-Pyr | 4-Pip |
| 2-2596 | 4-F-Ph | 2-BnNH-4-Pyr | 4-(3,4-deH-Pip) |
| 2-2597 | 4-F-Ph | 2-BnNH-4-Pym | 4-Pip |
| 2-2598 | 4-F-Ph | 2-BnNH-4-Pym | 4-(3,4-deH-Pip) |
| 2-2599 | 4-F-Ph | 2-(α-Me-BnNH)-4-Pyr | 4-Pip |
| 2-2600 | 4-F-Ph | 2-(α-Me-BnNH)-4-Pyr | 4-(3,4-deH-Pip) |
| 2-2601 | 4-F-Ph | 2-(α-Me-BnNH)-4-pym | 4-Pip |
| 2-2602 | 4-F-Ph | 2-(α-Me-BnNH)-4-Pym | 4-(3,4-deH-Pip) |
| 2-2603 | 3-Cl-Ph | 2-BnNH-4-Pyr | 4-Pip |
| 2-2604 | 3-Cl-Ph | 2-BnNH-4-Pyr | 4-(3,4-deH-Pip) |
| 2-2605 | 3-Cl-Ph | 2-BnNH-4-Pym | 4-Pip |
| 2-2606 | 3-Cl-Ph | 2-BnNH-4-Pym | 4-(3,4-deH-Pip) |
| 2-2607 | 3-Cl-Ph | 2-(α-Me-BnNH)-4-Pyr | 4-Pip |
| 2-2608 | 3-Cl-Ph | 2-(α-Me-BnNH)-4-Pyr | 4-(3,4-deH-Pip) |
| 2-2609 | 3-Cl-Ph | 2-(α-Me-BnNH)-4-Pym | 4-Pip |
| 2-2610 | 3-Cl-Ph | 2-(α-Me-BnNH)-4-Pym | 4-(3,4-deH-Pip) |
| 2-2611 | 3-$CF_3$-Ph | 2-BnNH-4-Pyr | 4-Pip |
| 2-2612 | 3-$CF_3$-Ph | 2-BnNH-4-Pyr | 4-(3,4-deH-Pip) |
| 2-2613 | 3-$CF_3$-Ph | 2-BnNH-4-Pym | 4-Pip |
| 2-2614 | 3-$CF_3$-Ph | 2-BnNH-4-Pym | 4-(3,4-deH-Pip) |
| 2-2615 | 3-$CF_3$-Ph | 2-(α-Me-BnNH)-4-Pyr | 4-Pip |
| 2-2616 | 3-$CF_3$-Ph | 2-(α-Me-BnNH)-4-Pyr | 4-(3,4-deH-Pip) |
| 2-2617 | 3-$CF_3$-Ph | 2-(α-Me-BnNH)-4-Pym | 4-Pip |
| 2-2618 | 3-$CF_3$-Ph | 2-(α-Me-BnNH)-4-Pym | 4-(3,4-deH-Pip) |
| 2-2619 | 4-F-Ph | 4-Pyr | 2-$NH_2$-4-Pym |
| 2-2620 | 4-F-Ph | 4-Pyr | 2-MeNH-4-pym |
| 2-2621 | 4-F-Ph | 4-Pyr | 2-$NH_2$-4-Pyr |
| 2-2622 | 4-F-Ph | 4-Pyr | 2-MeNH-4-pyr |
| 2-2623 | 4-F-Ph | 4-Pyr | $H_2N-CH_2C(Me)_2CH_2$ |
| 2-2624 | 4-F-Ph | 4-Pyr | $MeNH-CH_2C(Me)_2CH_2$ |
| 2-2625 | 4-F-Ph | 4-Pyr | $EtNH-CH_2C(Me)_2CH_2$ |
| 2-2626 | 4-F-Ph | 4-Pyr | $Me_2N-CH_2C (Me)_2CH_2$ |
| 2-2627 | 4-F-Ph | 4-Pyr | 3-(3,4-deH-Pip) |
| 2-2628 | 4-F-Ph | 4-Pyr | 1-Me-3-(3,4-deH-Pip) |

Table 2 (continued)

| Compound No. | $R^1$ | $R^2$ | $R^3$ |
|---|---|---|---|
| 2-2629 | 4-F-Ph | 4-Pyr | 1-Et-4-(3,4-deH-Pip) |
| 2-2630 | 4-F-Ph | 4-Pyr | 1-Pr-4-(3,4-deH-Pip) |
| 2-2631 | 4-F-Ph | 4-Pyr | 1-Pr-4-Pip |
| 2-2632 | 4-F-Ph | 4-Pyr | 1-iPr-4-(3,4-deH-Pip) |
| 2-2633 | 4-F-Ph | 4-Pyr | 1-iPr-4-Pip |
| 2-2634 | 4-F-Ph | 4-Pyr | 1-Bu-4-(3,4-deH-Pip) |
| 2-2635 | 4-F-Ph | 4-Pyr | 1-tBu-4-(3,4-deH-Pip) |
| 2-2636 | 4-F-Ph | 4-Pyr | 1-Pn-4-(3,4-deH-Pip) |
| 2-2637 | 4-F-Ph | 4-Pyr | 1-Hx-4-(3,4-deH-Pip) |
| 2-2638 | 4-F-Ph | 4-Pyr | 1-Hp-4-(3,4-deH-Pip) |
| 2-2639 | 4-F-Ph | 4-Pyr | 1-Oc-4-(3,4-deH-Pip) |
| 2-2640 | 4-F-Ph | 4-Pyr | 1-Nn-4-(3,4-deH-Pip) |
| 2-2641 | 4-F-Ph | 4-Pyr | 1-cPr-4-(3,4-deH-Pip) |
| 2-2642 | 4-F-Ph | 4-Pyr | 1-cPn-4-(3,4-deH-Pip) |
| 2-2643 | 4-F-Ph | 4-Pyr | 1-cHx-4-(3,4-deH-Pip) |
| 2-2644 | 4-F-Ph | 4-Pyr | 1-Bn-4-(3,4-deH-Pip) |
| 2-2645 | 4-F-Ph | 4-Pyr | 1-Phet-4-(3,4-deH-Pip) |
| 2-2646 | 4-F-Ph | 4-Pyr | 1-(3-Ph-Pr)-4-(3,4-deH-Pip) |
| 2-2647 | 4-F-Ph | 4-Pyr | 1-(4-Ph-Bu)-4-(3,4-deH-Pip) |
| 2-2648 | 4-F-Ph | 4-Pyr | 1-Allyl-4-(3,4-deH-Pip) |
| 2-2649 | 4-F-Ph | 4-Pyr | 1-Propargyl-4-(3,4-deH-Pip) |
| 2-2650 | 4-F-Ph | 4-Pyr | 2,2,6,6-tetraMe-4-(3,4-deH-Pip) |
| 2-2651 | 4-F-Ph | 4-Pyr | 1,2,2,6,6-pentaMe-4-(3,4-deH-Pip) |
| 2-2652 | 4-F-Ph | 4-Pyr | 1,2,2,6,6-pentaMe-4-Pip) |
| 2-2653 | 4-F-Ph | 4-Pyr | 7-(1,2,3,5,6,8a-hexaH-Ind) |
| 2-2654 | 4-F-Ph | 4-Pyr | 7-(1,2,3,5,8,8a-hexaH-Ind) |
| 2-2655 | 4-F-Ph | 4-Pyr | 7-octaH-Ind |
| 2-2656 | 4-F-Ph | 4-Pyr | 8-(1,3,4,6,7,9a-hexaH-2H-Qui) |
| 2-2657 | 4-F-Ph | 4-Pyr | 8-(1,3,4,6,9,9a-hexaH-2H-Qui) |
| 2-2658 | 4-F-Ph | 4-Pyr | 8-octaH-Qui |
| 2-2659 | 4-F-Ph | 4-Pyr | 2,2-diMe-4-(3,4-deH-PiP) |
| 2-2660 | 4-F-Ph | 4-Pyr | 1,2,2-triMe-4-(3,4-deH-PiP) |
| 2-2661 | 4-F-Ph | 4-Pyr | 2,2-diMe-4-(4,5-deH-PiP) |
| 2-2662 | 4-F-Ph | 4-Pyr | 1,2,2-triMe-4-(4,5-deH-PiP) |
| 2-2663 | 4-F-Ph | 4-Pyr | 2,6-diMe-4-(3,4-deH-PiP) |
| 2-2664 | 4-F-Ph | 4-Pyr | 1,2,6-triMe-4-(3, 4-deH-PiP) |
| 2-2665 | 4-F-Ph | 4-Pyr | 2-Me-4-(3,4-deH-Pip) |
| 2-2666 | 4-F-Ph | 4-Pyr | 1,2-diMe-4-(3,4-deH-Pip) |

Table 2 (continued)

| Compound No. | $R^1$ | $R^2$ | $R^3$ |
|---|---|---|---|
| 2-2667 | 4-F-Ph | 4-Pyr | 1-Et-2-Me-4-(3,4-deH-Pip) |
| 2-2668 | 4-F-Ph | 4-Pyr | 1-Pr-2-Me-4-(3,4-deH-Pip) |
| 2-2669 | 4-F-Ph | 4-Pyr | 1-Bu-2-Me-9-(3,4-deH-Pip) |
| 2-2670 | 4-F-Ph | 4-Pyr | 1-Phet-2-Me-4-(3,4-deH-Pip) |
| 2-2671 | 4-F-Ph | 4-Pyr | 2-Et-4-(3,4-deH-Pip) |
| 2-2672 | 4-F-Ph | 4-Pyr | 1-Me-2-Et-4-(3,4-deH-Pip) |
| 2-2673 | 4-F-Ph | 4-Pyr | 1,2-diEt-4-(3,4-deH-Pip) |
| 2-2674 | 4-F-Ph | 4-Pyr | 1-Pr-2-Et-4-(3,4-deH-Pip) |
| 2-2675 | 4-F-Ph | 4-Pyr | 1-Bu-2-Et-4-(3,4-deH-Pip) |
| 2-2676 | 4-F-Ph | 4-Pyr | 1-Phet-2-Et-4-(3,4-deH-Pip) |
| 2-2677 | 4-F-Ph | 4-Pyr | 2-Pr-4-(3,4-deH-Pip) |
| 2-2678 | 4-F-Ph | 4-Pyr | 1-Me-2-Pr-4-(3,4-deH-Pip) |
| 2-2679 | 4-F-Ph | 4-Pyr | 1-Et-2-Pr-4-(3,4-deH-Pip) |
| 2-2680 | 4-F-Ph | 4-Pyr | 1,2-diPr-4-(3,4-deH-Pip) |
| 2-2681 | 4-F-Ph | 4-Pyr | 1-Bu-2-Pr-4-(3,4-deH-Pip) |
| 2-2682 | 4-F-Ph | 4-Pyr | 1-Phet-2-Pr-4-(3,4-deH-Pip) |
| 2-2683 | 4-F-Ph | 4-Pyr | 2-Bu-4-(3,4-deH-Pip) |
| 2-2684 | 4-F-Ph | 4-Pyr | 1-Me-2-Bu-4-(3,4-deH-Pip) |
| 2-2685 | 4-F-Ph | 4-Pyr | 1-Et-2-Bu-4-(3,4-deH-Pip) |
| 2-2686 | 4-F-Ph | 4-Pyr | 1-Pr-2-Bu-4-(3,4-deH-Pip) |
| 2-2687 | 4-F-Ph | 4-Pyr | 1,2-diBu-4-(3,4-deH-Pip) |
| 2-2688 | 4-F-Ph | 4-Pyr | 1-Phet-2-Bu-4-(3,1-deH-Pip) |
| 2-2689 | 4-F-Ph | 4-Pyr | 2-Allyl-4-(3,4-deH-Pip) |
| 2-2690 | 4-F-Ph | 4-Pyr | 1-Me-2-Allyl-4-(3,4-deH-Pip) |
| 2-2691 | 4-F-Ph | 4-Pyr | 1-Et-2-Allyl-4-(3,4-deH-Pip) |
| 2-2692 | 4-F-Ph | 4-Pyr | 1-Pr-2-Allyl-4-(3,4-deH-Pip) |
| 2-2693 | 4-F-Ph | 4-Pyr | 1-Bu-2-Allyl-4-(3,4-deH-Pip) |
| 2-2694 | 4-F-Ph | 4-Pyr | 1-Phet-2-Allyl-4-(3,4-deH-Pip) |
| 2-2695 | 4-F-Ph | 4-Pyr | 2-Bn-4-(3,4-deH-Pip) |
| 2-2696 | 4-F-Ph | 4-Pyr | 1-Me-2-Bn-4-(3,4-deH-Pip) |
| 2-2697 | 4-F-Ph | 4-Pyr | 1-Et-2-Bn-4-(3,4-deH-Pip) |
| 2-2698 | 4-F-Ph | 4-Pyr | 1-Pr-2-Bn-4-(3,4-deH-Pip) |
| 2-2699 | 4-F-Ph | 4-Pyr | 1-Bu-2-Bn-4-(3,4-deH-Pip) |
| 2-2700 | 4-F-Ph | 4-Pyr | 1-Phet-2-Bn-4-(3,4-deH-Pip) |
| 2-2701 | 4-F-Ph | 4-Pyr | 2-Phet-4-(3,4-deH-Pip) |
| 2-2702 | 4-F-Ph | 4-Pyr | 1-Me-2-Phet-4-(3, 4-deH-Pip) |
| 2-2703 | 4-F-Ph | 4-Pyr | 1-Et-2-Phet-4-(3, 4-deH-Pip) |
| 2-2704 | 4-F-Ph | 4-Pyr | 1-Pr-2-Phet-4-(3,4-deH-Pip) |

Table 2 (continued)

| Compound No. | $R^1$ | $R^2$ | $R^3$ |
|---|---|---|---|
| 2-2705 | 4-F-Ph | 4-Pyr | 1-Bu-2-Phet-4-(3,4-deH-Pip) |
| 2-2706 | 4-F-Ph | 4-Pyr | 1,2-diPhet-4-(3,4-deH-Pip) |
| 2-2707 | 4-F-Ph | 4-Pyr | 2-Me-4-(4,5-deH-Pip) |
| 2-2708 | 4-F-Ph | 4-Pyr | 1,2-dime-4- (4,5-deH-Pip) |
| 2-2709 | 4-F-Ph | 4-Pyr | 1-Et-2-Me-4-(4,5-deH-Pip) |
| 2-2710 | 4-F-Ph | 4-Pyr | 1-Pr-2-Me-4-(4,5-deH-Pip) |
| 2-2711 | 4-F-Ph | 4-Pyr | 1-Bu-2-Me-4-(4,5-deH-Pip) |
| 2-2712 | 4-F-Ph | 4-Pyr | 1-Phet-2-Me-4-(4,5-deH-Pip) |
| 2-2713 | 4-F-Ph | 4-Pyr | 2-Et-4-(4,5-deH-Pip) |
| 2-2714 | 4-F-Ph | 4-Pyr | 1-Me-2-Et-4-(4,5-deH-Pip) |
| 2-2715 | 4-F-Ph | 4-Pyr | 1,2-diEt-4-(4,5-deH-Pip) |
| 2-2716 | 4-F-Ph | 4-Pyr | 1-Pr-2-Et-4-(4,5-deH-Pip) |
| 2-2717 | 4-F-Ph | 4-Pyr | 1-Bu-2-Et-4-(4,5-deH-Pip) |
| 2-2718 | 4-F-Ph | 4-Pyr | 1-Phet-2-Et-4-(4,5-deH-Pip) |
| 2-2719 | 4-F-Ph | 4-Pyr | 2-Pr-4-(4,5-deH-Pip) |
| 2-2720 | 4-F-Ph | 4-Pyr | 1-Me-2-Pr-4-(4,5-deH-Pip) |
| 2-2721 | 4-F-Ph | 4-Pyr | 1-Et-2-Pr-4-(4,5-deH-Pip) |
| 2-2722 | 4-F-Ph | 4-Pyr | 1,2-diPr-4-(4,5-deH-Pip) |
| 2-2723 | 4-F-Ph | 4-Pyr | 1-Bu-2-Pr-4-(4,5-deH-Pip) |
| 2-2724 | 4-F-Ph | 4-Pyr | 1-Phet-2-Pr-4-(4,5-deH-Pip) |
| 2-2725 | 4-F-Ph | 4-Pyr | 2-Bu-4-(4,5-deH-Pip) |
| 2-2726 | 4-F-Ph | 4-Pyr | 1-Me-2-Bu-4-(4,5-deH-Pip) |
| 2-2727 | 4-F-Ph | 4-Pyr | 1-Et-2-Bu-4-(4,5-deH-Pip) |
| 2-2728 | 4-F-Ph | 4-Pyr | 1-Pr-2-Bu-4-(4,5-deH-Pip) |
| 2-2729 | 4-F-Ph | 4-Pyr | 1,2-diBu-4-(4,5-deH-Pip) |
| 2-2730 | 4-F-Ph | 4-Pyr | 1-Phet-2-Bu-4-(4,5-deH-Pip) |
| 2-2731 | 4-F-Ph | 4-Pyr | 2-Allyl-4-(4,5-deH-Pip) |
| 2-2732 | 4-F-Ph | 4-Pyr | 1-Me-2-Allyl-4-(4,5-deH-Pip) |
| 2-2733 | 4-F-Ph | 4-Pyr | 1-Et-2-Allyl-4-(4,5-deH-Pip) |
| 2-2734 | 4-F-Ph | 4-Pyr | 1-Pr-2-Allyl-4-(4,5-deH-Pip) |
| 2-2735 | 4-F-Ph | 4-Pyr | 1-Bu-2-Allyl-4-(4,5-deH-Pip) |
| 2-2736 | 4-F-Ph | 4-Pyr | 1-Phet-2-Allyl-4-(4,5-deH-Pip) |
| 2-2737 | 4-F-Ph | 4-Pyr | 2-Bn-4-(4,5-deH-Pip) |
| 2-2738 | 4-F-Ph | 4-Pyr | 1-Me-2-Bn-4-(4,5-deH-Pip) |
| 2-2739 | 4-F-Ph | 4-Pyr | 1-Et-2-Bn-4-(4,5-deH-Pip) |
| 2-2740 | 4-F-Ph | 4-Pyr | 1-Pr-2-Bn-4-(4,5-deH-Pip) |
| 2-2741 | 4-F-Ph | 4-Pyr | 1-Bu-2-Bn-4-(4,5-deH-Pip) |
| 2-2742 | 4-F-Ph | 4-Pyr | 1-Phet-2-Bn-4-(4,5-deH-Pip) |

Table 2 (continued)

| Compound No. | $R^1$ | $R^2$ | $R^3$ |
|---|---|---|---|
| 2-2743 | 4-F-Ph | 4-Pyr | 2-Phet-4-(4,5-deH-Pip) |
| 2-2744 | 4-F-Ph | 4-Pyr | 1-Me-2-Phet-4-(4,5-deH-Pip) |
| 2-2745 | 4-F-Ph | 4-Pyr | 1-Et-2-Phet-4-(4,5-deH-Pip) |
| 2-2746 | 4-F-Ph | 4-Pyr | 1-Pr-2-Phet-4-(4,5-deH-Pip) |
| 2-2747 | 4-F-Ph | 4-Pyr | 1-Bu-2-Phet-4-(4,5-deH-Pip) |
| 2-2748 | 4-F-Ph | 4-Pyr | 1,2-diPhet-4-(4,5-deH-Pip) |
| 2-2749 | 4-F-Ph | 2-$NH_2$-4-Pym | $H_2N-CH_2C(Me)_2CH_2$ |
| 2-2750 | 4-F-Ph | 2-$NH_2$-4-Pym | $MeNH-CH_2C(Me)_2CH_2$ |
| 2-2751 | 4-F-Ph | 2-$NH_2$-4-Pym | $EtNH-CH_2C(Me)_2CH_2$ |
| 2-2752 | 4-F-Ph | 2-$NH_2$-4-Pym | $Me_2N-CH_2C(Me)_2CH_2$ |
| 2-2753 | 4-F-Ph | 2-$NH_2$-4-Pym | 3-(3,4-deH-Pip) |
| 2-2754 | 4-F-Ph | 2-$NH_2$-4-Pym | 1-Me-3-(3,4-deH-Pip) |
| 2-2755 | 4-F-Ph | 2-$NH_2$-4-Pym | 1-Et-4-(3,4-deH-Pip) |
| 2-2756 | 4-F-Ph | 2-$NH_2$-4-Pym | 1-Pr-4-(3,4-deH-Pip) |
| 2-2757 | 4-F-Ph | 2-$NH_2$-4-Pym | 1-Pr-4-Pip |
| 2-2758 | 4-F-Ph | 2-$NH_2$-4-Pym | 1-iPr-4-(3,4-deH-Pip) |
| 2-2759 | 4-F-Ph | 2-$NH_2$-4-Pym | 1-iPr-4-Pip |
| 2-2760 | 4-F-Ph | 2-$NH_2$-4-Pym | 1-Bu-4-(3,4-deH-Pip) |
| 2-2761 | 4-F-Ph | 2-$NH_2$-4-Pym | 1-tBu-4-(3,4-deH-Pip) |
| 2-2762 | 4-F-Ph | 2-$NH_2$-4-Pym | 1-Pn-4-(3,4-deH-Pip) |
| 2-2763 | 4-F-Ph | 2-$NH_2$-4-Pym | 1-Hx-4-(3,4-deH-Pip) |
| 2-2764 | 4-F-Ph | 2-$NH_2$-4-Pym | 1-Hp-4-(3,4-deH-Pip) |
| 2-2765 | 4-F-Ph | 2-$NH_2$-4-Pym | 1-Oc-4-(3,4-deH-Pip) |
| 2-2766 | 4-F-Ph | 2-$NH_2$-4-Pym | 1-Nn-4-(3,4-deH-Pip) |
| 2-2767 | 4-F-Ph | 2-$NH_2$-4-Pym | 1-cPr-4-(3,4-deH-Pip) |
| 2-2768 | 4-F-Ph | 2-$NH_2$-4-Pym | 1-cPn-4-(3,4-deH-Pip) |
| 2-2769 | 4-F-Ph | 2-$NH_2$-4-Pym | 1-cHx-4-(3,4-deH-Pip) |
| 2-2770 | 4-F-Ph | 2-$NH_2$-4-Pym | 1-Bn-4-(3,4-deH-Pip) |
| 2-2771 | 4-F-Ph | 2-$NH_2$-4-Pym | 1-Phet-4-(3,4-deH-Pip) |
| 2-2772 | 4-F-Ph | 2-$NH_2$-4-Pym | 1-(3-Ph-Pr)-4-(3,4-deH-Pip) |
| 2-2773 | 4-F-Ph | 2-$NH_2$-4-Pym | 1-(4-Ph-Bu)-4-(3,4-deH-Pip) |
| 2-2774 | 4-F-Ph | 2-$NH_2$-4-Pym | 1-Allyl-4-(3,4-deH-Pip) |
| 2-2775 | 4-F-Ph | 2-$NH_2$-4-Pym | 1-Propargyl-4-(3,4-deH-Pip) |
| 2-2776 | 4-F-Ph | 2-$NH_2$-4-Pym | 2,2,6,6-tetraMe-4-(3,4-deH-Pip) |
| 2-2777 | 4-F-Ph | 2-$NH_2$-4-Pym | 1,2,2,6,6-pentaMe-4-(3,4-deH-Pip) |
| 2-2778 | 4-F-Ph | 2-$NH_2$-4-Pym | 1,2,2,6,6-pentaMe-4-Pip) |
| 2-2779 | 4-F-Ph | 2-$NH_2$-4-Pym | 7-(1,2,3,5,6,8a-hexaH-Ind) |
| 2-2780 | 4-F-Ph | 2-$NH_2$-4-Pym | 7-(1,2,3,5,8,8a-hexaH-Ind) |

Table 2 (continued)

| Compound No. | $R^1$ | $R^2$ | $R^3$ |
|---|---|---|---|
| 2-2781 | 4-F-Ph | $2-NH_2$-4-Pym | 7-octaH-Ind |
| 2-2782 | 4-F-Ph | $2-NH_2$-4-Pym | 8-(1,3,4,6,7,9a-hexaH-2H-Qui) |
| 2-2783 | 4-F-Ph | $2-NH_2$-4-Pym | 8-(1,3,4,6,9,9a-hexaH-2H-Qui) |
| 2-2784 | 4-F-Ph | $2-NH_2$-4-Pym | 8-octaH-Qui |
| 2-2785 | 4-F-Ph | $2-NH_2$-4-Pym | 2,2-diMe-4-(3,4-deH-PiP) |
| 2-2786 | 4-F-Ph | $2-NH_2$-4-Pym | 1,2,2-triMe-4-(3,4-deH-PiP) |
| 2-2787 | 4-F-Ph | $2-NH_2$-4-Pym | 2,2-diMe-4-(4,5-deH-PiP) |
| 2-2788 | 4-F-Ph | $2-NH_2$-4-Pym | 1,2,2-triMe-4-(4,5-deH-PiP) |
| 2-2789 | 4-F-Ph | $2-NH_2$-4-Pym | 2,6-diMe-4-(3,4-deH-PiP) |
| 2-2790 | 4-F-Ph | $2-NH_2$-4-Pym | 1,2,6-triMe-4-(3,4-deH-PiP) |
| 2-2791 | 4-F-Ph | $2-NH_2$-4-Pym | 2-Me-4-(3,4-deH-Pip) |
| 2-2792 | 4-F-Ph | $2-NH_2$-4-Pym | 1,2-diMe-4-(3,4-deH-Pip) |
| 2-2793 | 4-F-Ph | $2-NH_2$-4-Pym | 1-Et-2-Me-4-(3,4-deH-Pip) |
| 2-2794 | 4-F-Ph | $2-NH_2$-4-Pym | 1-Pr-2-Me-4-(3,4-deH-Pip) |
| 2-2795 | 4-F-Ph | $2-NH_2$-4-Pym | 1-Bu-2-Me-4-(3,4-deH-Pip) |
| 2-2796 | 4-F-Ph | $2-NH_2$-4-Pym | 1-Phet-2-Me-4-(3,4-deH-Pip) |
| 2-2797 | 4-F-Ph | $2-NH_2$-4-Pym | 2-Et-4-(3,4-deH-Pip) |
| 2-2798 | 4-F-Ph | $2-NH_2$-4-Pym | 1-Me-2-Et-4-(3,4-deH-Pip) |
| 2-2799 | 4-F-Ph | $2-NH_2$-4-Pym | 1,2-diEt-4-(3,4-deH-Pip) |
| 2-2800 | 4-F-Ph | $2-NH_2$-4-Pym | 1-Pr-2-Et-4-(3,4-deH-Pip) |
| 2-2801 | 4-F-Ph | $2-NH_2$-4-Pym | 1-Bu-2-Et-4-(3,4-deH-Pip) |
| 2-2802 | 4-F-Ph | $2-NH_2$-4-Pym | 1-Phet-2-Et-4-(3,4-deH-Pip) |
| 2-2803 | 4-F-Ph | $2-NH_2$-4-Pym | 2-Pr-4-(3,4-deH-Pip) |
| 2-2804 | 4-F-Ph | $2-NH_2$-4-Pym | 1-Me-2-Pr-4-(3,4-deH-Pip) |
| 2-2805 | 4-F-Ph | $2-NH_2$-4-Pym | 1-Et-2-Pr-4-(3,4-deH-Pip) |
| 2-2806 | 4-F-Ph | $2-NH_2$-4-Pym | 1,2-diPr-4-(3,4-deH-Pip) |
| 2-2807 | 4-F-Ph | $2-NH_2$-4-Pym | 1-Bu-2-Pr-4-(3,4-deH-Pip) |
| 2-2808 | 4-F-Ph | $2-NH_2$-4-Pym | 1-Phet-2-Pr-4-(3,4-deH-Pip) |
| 2-2809 | 4-F-Ph | $2-NH_2$-4-Pym | 2-Bu-4-(3,4-deH-Pip) |
| 2-2810 | 4-F-Ph | $2-NH_2$-4-Pym | 1-Me-2-Bu-4-(3,4-deH-Pip) |
| 2-2811 | 4-F-Ph | $2-NH_2$-4-Pym | 1-Et-2-Bu-4-(3,4-deH-Pip) |
| 2-2812 | 4-F-Ph | $2-NH_2$-4-Pym | 1-Pr-2-Bu-4-(3,4-deH-Pip) |
| 2-2813 | 4-F-Ph | $2-NH_2$-4-Pym | 1,2-diBu-4-(3,4-deH-Pip) |
| 2-2814 | 4-F-Ph | $2-NH_2$-4-Pym | 1-Phet-2-Bu-4-(3,4-deH-Pip) |
| 2-2815 | 4-F-Ph | $2-NH_2$-4-Pym | 2-Allyl-4-(3,4-deH-Pip) |
| 2-2816 | 4-F-Ph | $2-NH_2$-4-Pym | 1-Me-2-Allyl-4-(3,4-deH-Pip) |
| 2-2817 | 4-F-Ph | $2-NH_2$-4-Pym | 1-Et-2-Allyl-4-(3,4-deH-Pip) |
| 2-2818 | 4-F-Ph | $2-NH_2$-4-Pym | 1-Pr-2-Allyl-4-(3,4-deH-Pip) |

Table 2 (continued)

| Compound No. | $R^1$ | $R^2$ | $R^3$ |
|---|---|---|---|
| 2-2819 | 4-F-Ph | 2-$NH_2$-4-Pym | 1-Bu-2-Allyl-4-(3,4-deH-Pip) |
| 2-2820 | 4-F-Ph | 2-$NH_2$-4-Pym | 1-Phet-2-Allyl-4-(3,4-deH-Pip) |
| 2-2821 | 4-F-Ph | 2-$NH_2$-4-Pym | 2-Bn-4-(3,4-deH-Pip) |
| 2-2822 | 4-F-Ph | 2-$NH_2$-4-Pym | 1-Me-2-Bn-4-(3,4-deH-Pip) |
| 2-2823 | 4-F-Ph | 2-$NH_2$-4-Pym | 1-Et-2-Bn-4-(3,4-deH-Pip) |
| 2-2824 | 4-F-Ph | 2-$NH_2$-4-Pym | 1-Pr-2-Bn-4-(3,4-deH-Pip) |
| 2-2825 | 4-F-Ph | 2-$NH_2$-4-Pym | 1-Bu-2-Bn-4-(3,4-deH-Pip) |
| 2-2826 | 4-F-Ph | 2-$NH_2$-4-Pym | 1-Phet-2-Bn-4-(3,4-deH-Pip) |
| 2-2827 | 4-F-Ph | 2-$NH_2$-4-Pym | 2-Phet-4-(3,4-deH-Pip) |
| 2-2828 | 4-F-Ph | 2-$NH_2$-4-Pym | 1-Me-2-Phet-4-(3,4-deH-Pip) |
| 2-2829 | 4-F-Ph | 2-$NH_2$-4-Pym | 1-Et-2-Phet-4-(3,4-deH-Pip) |
| 2-2830 | 4-F-Ph | 2-$NH_2$-4-Pym | 1-Pr-2-Phet-4-(3,4-deH-Pip) |
| 2-2831 | 4-F-Ph | 2-$NH_2$-4-Pym | 1-Bu-2-Phet-4-(3,4-deH-Pip) |
| 2-2832 | 4-F-Ph | 2-$NH_2$-4-Pym | 1,2-diPhet-4-(3,4-deH-Pip) |
| 2-2833 | 4-F-Ph | 2-$NH_2$-4-Pym | 2-Me-4-(4,5-deH-Pip) |
| 2-2834 | 4-F-Ph | 2-$NH_2$-4-Pym | 1,2-diMe-4-(4,5-deH-Pip) |
| 2-2835 | 4-F-Ph | 2-$NH_2$-4-Pym | 1-Et-2-Me-4-(4,5-deH-Pip) |
| 2-2836 | 4-F-Ph | 2-$NH_2$-4-Pym | 1-Pr-2-Me-4-(4,5-deH-Pip) |
| 2-2837 | 4-F-Ph | 2-$NH_2$-4-Pym | 1-Bu-2-Me-4-(4,5-deH-Pip) |
| 2-2838 | 4-F-Ph | 2-$NH_2$-4-Pym | 1-Phet-2-Me-4-(4,5-deH-Pip) |
| 2-2839 | 4-F-Ph | 2-$NH_2$-4-Pym | 2-Et-4-(4,5-deH-Pip) |
| 2-2840 | 4-F-Ph | 2-$NH_2$-4-Pym | 1-Me-2-Et-4-(4,5-deH-Pip) |
| 2-2841 | 4-F-Ph | 2-$NH_2$-4-Pym | 1,2-diEt-4-(4,5-deH-Pip) |
| 2-2842 | 4-F-Ph | 2-$NH_2$-4-Pym | 1-Pr-2-Et-4-(4,5-deH-Pip) |
| 2-2843 | 4-F-Ph | 2-$NH_2$-4-Pym | 1-Bu-2-Et-4-(4,5-deH-Pip) |
| 2-2844 | 4-F-Ph | 2-$NH_2$-4-Pym | 1-Phet-2-Et-4-(4,5-deH-Pip) |
| 2-2845 | 4-F-Ph | 2-$NH_2$-4-Pym | 2-Pr-4-(4,5-deH-Pip) |
| 2-2846 | 4-F-Ph | 2-$NH_2$-4-Pym | 1-Me-2-Pr-4-(4,5-deH-Pip) |
| 2-2847 | 4-F-Ph | 2-$NH_2$-4-Pym | 1-Et-2-Pr-4-(4,5-deH-Pip) |
| 2-2848 | 4-F-Ph | 2-$NH_2$-4-Pym | 1,2-diPr-4-(4,5-deH-Pip) |
| 2-2849 | 4-F-Ph | 2-$NH_2$-4-Pym | 1-Bu-2-Pr-4-(4,5-deH-Pip) |
| 2-2850 | 4-F-Ph | 2-$NH_2$-4-Pym | 1-Phet-2-Pr-4-(4,5-deH-Pip) |
| 2-2851 | 4-F-Ph | 2-$NH_2$-4-Pym | 2-Bu-4-(4,5-deH-Pip) |
| 2-2852 | 4-F-Ph | 2-$NH_2$-4-Pym | 1-Me-2-Bu-4-(4,5-deH-Pip) |
| 2-2853 | 4-F-Ph | 2-$NH_2$-4-Pym | 1-Et-2-Bu-4-(4,5-deH-Pip) |
| 2-2854 | 4-F-Ph | 2-$NH_2$-4-Pym | 1-Pr-2-Bu-4-(4,5-deH-Pip) |
| 2-2855 | 4-F-Ph | 2-$NH_2$-4-Pym | 1,2-diBu-4-(4,5-deH-Pip) |
| 2-2856 | 4-F-Ph | 2-$NH_2$-4-Pym | 1-Phet-2-Bu-4-(4,5-deH-Pip) |

Table 2 (continued)

| Compound No. | $R^1$ | $R^2$ | $R^3$ |
|---|---|---|---|
| 2-2857 | 4-F-Ph | $2-NH_2$-4-Pym | 2-Allyl-4-(4,5-deH-Pip) |
| 2-2858 | 4-F-Ph | $2-NH_2$-4-Pym | 1-Me-2-Allyl-4-(4,5-deH-Pip) |
| 2-2859 | 4-F-Ph | $2-NH_2$-4-Pym | 1-Et-2-Allyl-4-(4,5-deH-Pip) |
| 2-2860 | 4-F-Ph | $2-NH_2$-4-Pym | 1-Pr-2-Allyl-4-(4,5-deH-Pip) |
| 2-2861 | 4-F-Ph | $2-NH_2$-4-Pym | 1-Bu-2-Allyl-4-(4,5-deH-Pip) |
| 2-2862 | 4-F-Ph | $2-NH_2$-4-Pym | 1-Phet-2-Allyl-4-(4,5-deH-Pip) |
| 2-2863 | 4-F-Ph | $2-NH_2$-4-Pym | 2-Bn-4-(4,5-deH-Pip) |
| 2-2864 | 4-F-Ph | $2-NH_2$-4-Pym | 1-Me-2-Bn-4-(4,5-deH-Pip) |
| 2-2865 | 4-F-Ph | $2-NH_2$-4-Pym | 1-Et-2-Bn-4-(4,5-deH-Pip) |
| 2-2866 | 4-F-Ph | $2-NH_2$-4-Pym | 1-Pr-2-Bn-4-(4,5-deH-Pip) |
| 2-2867 | 4-F-Ph | $2-NH_2$-4-Pym | 1-Bu-2-Bn-4-(4,5-deH-Pip) |
| 2-2868 | 4-F-Ph | $2-NH_2$-4-Pym | 1-Phet-2-Bn-4-(4,5-deH-Pip) |
| 2-2869 | 4-F-Ph | $2-NH_2$-4-Pym | 2-Phet-4-(4,5-deH-Pip) |
| 2-2870 | 4-F-Ph | $2-NH_2$-4-Pym | 1-Me-2-Phet-4-(4,5-deH-Pip) |
| 2-2871 | 4-F-Ph | $2-NH_2$-4-Pym | 1-Et-2-Phet-4-(4,5-deH-Pip) |
| 2-2872 | 4-F-Ph | $2-NH_2$-4-Pym | 1-Pr-2-Phet-4-(4,5-deH-Pip) |
| 2-2873 | 4-F-Ph | $2-NH_2$-4-Pym | 1-Bu-2-Phet-4-(4,5-deH-Pip) |
| 2-2874 | 4-F-Ph | $2-NH_2$-4-Pym | 1,2-diPhet-4-(4,5-deH-Pip) |
| 2-2875 | 4-F-Ph | 2-MeNH-4-Pym | $H_2N-CH_2C(Me)_2CH_2$ |
| 2-2876 | 4-F-Ph | 2-MeNH-4-Pym | $MeNH-CH_2C(Me)_2CH_2$ |
| 2-2877 | 4-F-Ph | 2-MeNH-4-Pym | $EtNH-CH_2C(Me)_2CH_2$ |
| 2-2878 | 4-F-Ph | 2-MeNH-4-Pym | $Me_2N-CH_2C(Me)_2CH_2$ |
| 2-2879 | 4-F-Ph | 2-MeNH-4-Pym | 3-(3,4-deH-Pip) |
| 2-2880 | 4-F-Ph | 2-MeNH-4-Pym | 1-Me-3-(3,4-deH-Pip) |
| 2-2881 | 4-F-Ph | 2-MeNH-4-Pym | 1-Et-4-(3,4-deH-Pip) |
| 2-2882 | 4-F-Ph | 2-MeNH-4-Pym | 1-Pr-4-(3,4-deH-Pip) |
| 2-2883 | 4-F-Ph | 2-MeNH-4-Pym | 1-Pr-4-Pip |
| 2-2884 | 4-F-Ph | 2-MeNH-4-Pym | 1-iPr-4-(3,4-deH-Pip) |
| 2-2885 | 4-F-Ph | 2-MeNH-4-Pym | 1-iPr-4-Pip |
| 2-2886 | 4-F-Ph | 2-MeNH-4-Pym | 1-Bu-4-(3,4-deH-Pip) |
| 2-2887 | 4-F-Ph | 2-MeNH-4-Pym | 1-tBu-4-(3,4-deH-Pip) |
| 2-2888 | 4-F-Ph | 2-MeNH-4-Pym | 1-Pn-4-(3,4-deH-Pip) |
| 2-2889 | 4-F-Ph | 2-MeNH-4-Pym | 1-Hx-4-(3,4-deH-Pip) |
| 2-2890 | 4-F-Ph | 2-MeNH-4-Pym | 1-Hp-4-(3,4-deH-Pip) |
| 2-2891 | 4-F-Ph | 2-MeNH-4-Pym | 1-Oc-4-(3,4-deH-Pip) |
| 2-2892 | 4-F-Ph | 2-MeNH-4-Pym | 1-Nn-4-(3,4-deH-Pip) |
| 2-2893 | 4-F-Ph | 2-MeNH-4-Pym | 1-cPr-4-(3,4-deH-Pip) |
| 2-2894 | 4-F-Ph | 2-MeNH-4-Pym | 1-cPn-4-(3,4-deH-Pip) |

Table 2 (continued)

| Compound No. | $R^1$ | $R^2$ | $R^3$ |
|---|---|---|---|
| 2-2895 | 4-F-Ph | 2-MeNH-4-Pym | 1-cHx-4-(3,4-deH-Pip) |
| 2-2896 | 4-F-Ph | 2-MeNH-4-Pym | 1-Bn-4-(3,4-deH-Pip) |
| 2-2897 | 4-F-Ph | 2-MeNH-4-Pym | 1-Phet-4-(3,4-deH-Pip) |
| 2-2898 | 4-F-Ph | 2-MeNH-4-Pym | 1-(3-Ph-Pr)-4-(3,4-deH-Pip) |
| 2-2899 | 4-F-Ph | 2-MeNH-4-Pym | 1-(4-Ph-Bu)-4-(3,4-deH-Pip) |
| 2-2900 | 4-F-Ph | 2-MeNH-4-Pym | 1-Allyl-4-(3,4-deH-Pip) |
| 2-2901 | 4-F-Ph | 2-MeNH-4-Pym | 1-Propargyl-4-(3,4-deH-Pip) |
| 2-2902 | 4-F-Ph | 2-MeNH-4-Pym | 2,2,6,6-tetraMe-4-(3,4-deH-Pip) |
| 2-2903 | 4-F-Ph | 2-MeNH-4-Pym | 1,2,2,6,6-pentaMe-4-(3,4-deH-Pip) |
| 2-2904 | 4-F-Ph | 2-MeNH-4-Pym | 1,2,2,6,6-pentaMe-4-Pip) |
| 2-2905 | 4-F-Ph | 2-MeNH-4-Pym | 7-(1,2,3,5,6,8a-hexaH-Ind) |
| 2-2906 | 4-F-Ph | 2-MeNH-4-Pym | 7-(1,2,3,5,8,8a-hexaH-Ind) |
| 2-2907 | 4-F-Ph | 2-MeNH-4-Pym | 7-octaH-Ind |
| 2-2908 | 4-F-Ph | 2-MeNH-4-Pym | 8-(1,3,4,6,7,9a-hexaH-2H-Qui) |
| 2-2909 | 4-F-Ph | 2-MeNH-4-Pym | 8-(1,3,4,6,9,9a-hexaH-2H-Qui) |
| 2-2910 | 4-F-Ph | 2-MeNH-4-Pym | 8-octaH-Qui |
| 2-2911 | 4-F-Ph | 2-MeNH-4-Pym | 2,2-diMe-4-(3,4-deH-PiP) |
| 2-2912 | 4-F-Ph | 2-MeNH-4-Pym | 1,2,2-triMe-4-(3,4-deH-PiP) |
| 2-2913 | 4-F-Ph | 2-MeNH-4-Pym | 2,2-diMe-4-(4,5-deH-PiP) |
| 2-2914 | 4-F-Ph | 2-MeNH-4-Pym | 1,2,2-triMe-4-(4,5-deH-PiP) |
| 2-2915 | 4-F-Ph | 2-MeNH-4-Pym | 2,6-diMe-4-(3,4-deH-PiP) |
| 2-2916 | 4-F-Ph | 2-MeNH-4-Pym | 1,2,6-triMe-4-(3,4-deH-PiP) |
| 2-2917 | 4-F-Ph | 2-MeNH-4-Pym | 2-Me-4-(3,4-deH-Pip) |
| 2-2918 | 4-F-Ph | 2-MeNH-4-Pym | 1,2-dime-4-(3,4-deH-Pip) |
| 2-2919 | 4-F-Ph | 2-MeNH-4-Pym | 1-Et-2-Me-4-(3,4-deH-Pip) |
| 2-2920 | 4-F-Ph | 2-MeNH-4-Pym | 1-Pr-2-Me-4-(3,4-deH-Pip) |
| 2-2921 | 4-F-Ph | 2-MeNH-4-Pym | 1-Bu-2-Me-4-(3,4-deH-Pip) |
| 2-2922 | 4-F-Ph | 2-MeNH-4-Pym | 1-Phet-2-Me-4-(3,4-deH-Pip) |
| 2-2923 | 4-F-Ph | 2-MeNH-4-Pym | 2-Et-4-(3,4-deH-Pip) |
| 2-2924 | 4-F-Ph | 2-MeNH-4-Pym | 1-Me-2-Et-4-(3,4-deH-Pip) |
| 2-2925 | 4-F-Ph | 2-MeNH-4-Pym | 1,2-diEt-4-(3,4-deH-Pip) |
| 2-2926 | 4-F-Ph | 2-MeNH-4-Pym | 1-Pr-2-Et-4-(3,4-deH-Pip) |
| 2-2927 | 4-F-Ph | 2-MeNH-4-Pym | 1-Bu-2-Et-4-(3,4-deH-Pip) |
| 2-2928 | 4-F-Ph | 2-MeNH-4-Pym | 1-Phet-2-Et-4-(3,4-deH-Pip) |
| 2-2929 | 4-F-Ph | 2-MeNH-4-Pym | 2-Pr-4-(3,4-deH-Pip) |
| 2-2930 | 4-F-Ph | 2-MeNH-4-Pym | 1-Me-2-Pr-4-(3,4-deH-Pip) |
| 2-2931 | 4-F-Ph | 2-MeNH-4-Pym | 1-Et-2-Pr-4-(3,4-deH-Pip) |
| 2-2932 | 4-F-Ph | 2-MeNH-4-Pym | 1,2-diPr-4-(3,4-deH-Pip) |

Table 2 (continued)

| Compound No. | $R^1$ | $R^2$ | $R^3$ |
|---|---|---|---|
| 2-2933 | 4-F-Ph | 2-MeNH-4-Pym | 1-Bu-2-Pr-4-(3,4-deH-Pip) |
| 2-2934 | 4-F-Ph | 2-MeNH-4-Pym | 1-Phet-2-Pr-4-(3,4-deH-Pip) |
| 2-2935 | 4-F-Ph | 2-MeNH-4-Pym | 2-Bu-4-(3,4-deH-Pip) |
| 2-2936 | 4-F-Ph | 2-MeNH-4-Pym | 1-Me-2-Bu-4-(3,4-deH-Pip) |
| 2-2937 | 4-F-Ph | 2-MeNH-4-Pym | 1-Et-2-Bu-9-(3,4-deH-Pip) |
| 2-2938 | 4-F-Ph | 2-MeNH-4-Pym | 1-Pr-2-Bu-4-(3,4-deH-Pip) |
| 2-2939 | 4-F-Ph | 2-MeNH-4-Pym | 1,2-diBu-4-(3,4-deH-Pip) |
| 2-2940 | 4-F-Ph | 2-MeNH-4-Pym | 1-Phet-2-Bu-4-(3,4-deH-Pip) |
| 2-2941 | 4-F-Ph | 2-MeNH-4-Pym | 2-Allyl-4-(3,4-deH-Pip) |
| 2-2942 | 4-F-Ph | 2-MeNH-4-Pym | 1-Me-2-Allyl-4-(3,4-deH-Pip) |
| 2-2943 | 4-F-Ph | 2-MeNH-4-Pym | 1-Et-2-Allyl-4-(3,4-deH-Pip) |
| 2-2944 | 4-F-Ph | 2-MeNH-4-Pym | 1-Pr-2-Allyl-4-(3,4-deH-Pip) |
| 2-2945 | 4-F-Ph | 2-MeNH-4-Pym | 1-Bu-2-Allyl-4-(3,4-deH-Pip) |
| 2-2946 | 4-F-Ph | 2-MeNH-4-Pym | 1-Phet-2-Allyl-4-(3,4-deH-Pip) |
| 2-2947 | 4-F-Ph | 2-MeNH-4-Pym | 2-Bn-4-(3,4-deH-Pip) |
| 2-2948 | 4-F-Ph | 2-MeNH-4-Pym | 1-Me-2-Bn-4-(3,4-deH-Pip) |
| 2-2949 | 4-F-Ph | 2-MeNH-4-Pym | 1-Et-2-Bn-4-(3,4-deH-Pip) |
| 2-2950 | 4-F-Ph | 2-MeNH-4-Pym | 1-Pr-2-Bn-4-(3,4-deH-Pip) |
| 2-2951 | 4-F-Ph | 2-MeNH-4-Pym | 1-Bu-2-Bn-4-(3,4-deH-Pip) |
| 2-2952 | 4-F-Ph | 2-MeNH-4-Pym | 1-Phet-2-Bn-4-(3,4-deH-Pip) |
| 2-2953 | 4-F-Ph | 2-MeNH-4-Pym | 2-Phet-4-(3,4-deH-Pip) |
| 2-2954 | 4-F-Ph | 2-MeNH-4-Pym | 1-Me-2-Phet-4-(3,4-deH-Pip) |
| 2-2955 | 4-F-Ph | 2-MeNH-4-Pym | 1-Et-2-Phet-4-(3,4-deH-Pip) |
| 2-2956 | 4-F-Ph | 2-MeNH-4-Pym | 1-Pr-2-Phet-4-(3,4-deH-Pip) |
| 2-2957 | 4-F-Ph | 2-MeNH-4-Pym | 1-Bu-2-Phet-4-(3,4-deH-Pip) |
| 2-2958 | 4-F-Ph | 2-MeNH-4-Pym | 1,2-diPhet-4-(3,4-deH-Pip) |
| 2-2959 | 4-F-Ph | 2-MeNH-4-Pym | 2-Me-4-(4,5-deH-Pip) |
| 2-2960 | 4-F-Ph | 2-MeNH-4-Pym | 1,2-diMe-4-(4,5-deH-Pip) |
| 2-2961 | 4-F-Ph | 2-MeNH-4-Pym | 1-Et-2-Me-4-(4,5-deH-Pip) |
| 2-2962 | 4-F-Ph | 2-MeNH-4-Pym | 1-Pr-2-Me-4-(4,5-deH-Pip) |
| 2-2963 | 4-F-Ph | 2-MeNH-4-Pym | 1-Bu-2-Me-4-(4,5-deH-Pip) |
| 2-2964 | 4-F-Ph | 2-MeNH-4-Pym | 1-Phet-2-Me-4-(4,5-deH-Pip) |
| 2-2965 | 4-F-Ph | 2-MeNH-4-Pym | 2-Et-4-(4,5-deH-Pip) |
| 2-2966 | 4-F-Ph | 2-MeNH-4-Pym | 1-Me-2-Et-4-(4,5-deH-Pip) |
| 2-2967 | 4-F-Ph | 2-MeNH-4-Pym | 1,2-diEt-4-(4,5-deH-Pip) |
| 2-2968 | 4-F-Ph | 2-MeNH-4-Pym | 1-Pr-2-Et-4-(4,5-deH-Pip) |
| 2-2969 | 4-F-Ph | 2-MeNH-4-Pym | 1-Bu-2-Et-4-(4,5-deH-Pip) |
| 2-2970 | 4-F-Ph | 2-MeNH-4-Pym | 1-Phet-2-Et-4-(4,5-deH-Pip) |

Table 2 (continued)

| Compound No. | $R^1$ | $R^2$ | $R^3$ |
|---|---|---|---|
| 2-2971 | 4-F-Ph | 2-MeNH-4-Pym | 2-Pr-4-(4,5-deH-Pip) |
| 2-2972 | 4-F-Ph | 2-MeNH-4-Pym | 1-Me-2-Pr-4-(4,5-deH-Pip) |
| 2-2973 | 4-F-Ph | 2-MeNH-4-Pym | 1-Et-2-Pr-4-(4,5-deH-Pip) |
| 2-2974 | 4-F-Ph | 2-MeNH-4-Pym | 1,2-diPr-4-(4,5-deH-Pip) |
| 2-2975 | 4-F-Ph | 2-MeNH-4-Pym | 1-Bu-2-Pr-4-(4,5-deH-Pip) |
| 2-2976 | 4-F-Ph | 2-MeNH-4-Pym | 1-Phet-2-Pr-4-(4,5-deH-Pip) |
| 2-2977 | 4-F-Ph | 2-MeNH-4-Pym | 2-Bu-4-(4,5-deH-Pip) |
| 2-2978 | 4-F-Ph | 2-MeNH-4-Pym | 1-Me-2-Bu-4-(4,5-deH-Pip) |
| 2-2979 | 4-F-Ph | 2-MeNH-4-Pym | 1-Et-2-Bu-4-(4,5-deH-Pip) |
| 2-2980 | 4-F-Ph | 2-MeNH-4-Pym | 1-Pr-2-Bu-4-(4,5-deH-Pip) |
| 2-2981 | 4-F-Ph | 2-MeNH-4-Pym | 1,2-diBu-4-(4,5-deH-Pip) |
| 2-2982 | 4-F-Ph | 2-MeNH-4-Pym | 1-Phet-2-Bu-4-(4,5-deH-Pip) |
| 2-2983 | 4-F-Ph | 2-MeNH-4-Pym | 2-Allyl-4-(4,5-deH-Pip) |
| 2-2984 | 4-F-Ph | 2-MeNH-4-Pym | 1-Me-2-Allyl-4-(4,5-deH-Pip) |
| 2-2985 | 4-F-Ph | 2-MeNH-4-Pym | 1-Et-2-Allyl-4-(4,5-deH-Pip) |
| 2-2986 | 4-F-Ph | 2-MeNH-4-Pym | 1-Pr-2-Allyl-4-(4,5-deH-Pip) |
| 2-2987 | 4-F-Ph | 2-MeNH-4-Pym | 1-Bu-2-Allyl-4-(4,5-deH-Pip) |
| 2-2988 | 4-F-Ph | 2-MeNH-4-Pym | 1-Phet-2-Allyl-4-(4,5-deH-Pip) |
| 2-2989 | 4-F-Ph | 2-MeNH-4-Pym | 2-Bn-4-(4,5-deH-Pip) |
| 2-2990 | 4-F-Ph | 2-MeNH-4-Pym | 1-Me-2-Bn-4-(4,5-deH-Pip) |
| 2-2991 | 4-F-Ph | 2-MeNH-4-Pym | 1-Et-2-Bn-4-(4,5-deH-Pip) |
| 2-2992 | 4-F-Ph | 2-MeNH-4-Pym | 1-Pr-2-Bn-4-(4,5-deH-Pip) |
| 2-2993 | 4-F-Ph | 2-MeNH-4-Pym | 1-Bu-2-Bn-4-(4,5-deH-Pip) |
| 2-2994 | 4-F-Ph | 2-MeNH-4-Pym | 1-Phet-2-Bn-4-(4,5-deH-Pip) |
| 2-2995 | 4-F-Ph | 2-MeNH-4-Pym | 2-Phet-4-(4,5-deH-Pip) |
| 2-2996 | 4-F-Ph | 2-MeNH-4-Pym | 1-Me-2-Phet-4-(4,5-deH-Pip) |
| 2-2997 | 4-F-Ph | 2-MeNH-4-Pym | 1-Et-2-Phet-4-(4,5-deH-Pip) |
| 2-2998 | 4-F-Ph | 2-MeNH-4-Pym | 1-Pr-2-Phet-4-(4,5-deH-Pip) |
| 2-2999 | 4-F-Ph | 2-MeNH-4-Pym | 1-Bu-2-Phet-4-(4,5-deH-Pip) |
| 2-3000 | 4-F-Ph | 2-MeNH-4-Pym | 1,2-diPhet-4-(4,5-deH-Pip) |
| 2-3001 | 4-F-Ph | $2-NH_2-4-Pyr$ | $H_2N-CH_2C(Me)_2CH_2$ |
| 2-3002 | 4-F-Ph | $2-NH_2-4-Pyr$ | $MeNH-CH_2C(Me)_2CH_2$ |
| 2-3003 | 4-F-Ph | $2-NH_2-4-Pyr$ | $EtNH-CH_2C(Me)_2CH_2$ |
| 2-3004 | 4-F-Ph- | $2-NH_2-4-Pyr$ | $Me_2N-CH_2C(Me)_2CH_2$ |
| 2-3005 | 4-F-Ph | $2-NH_2-4-Pyr$ | 3-(3,4-deH-Pip) |
| 2-3006 | 4-F-Ph | $2-NH_2-4-Pyr$ | 1-Me-3-(3,4-deH-Pip) |
| 2-3007 | 4-F-Ph | $2-NH_2-4-Pyr$ | 1-Et-4-(3,4-deH-Pip) |
| 2-3008 | 4-F-Ph | $2-NH_2-4-Pyr$ | 1-Pr-4-(3,4-deH-Pip) |

Table 2 (continued)

| Compound No. | $R^1$ | $R^2$ | $R^3$ |
|---|---|---|---|
| 2-3009 | 4-F-Ph | $2-NH_2-4-Pyr$ | 1-Pr-4-Pip |
| 2-3010 | 4-F-Ph | $2-NH_2-4-Pyr$ | 1-iPr-4-(3,4-deH-Pip) |
| 2-3011 | 4-F-Ph | $2-NH_2-4-Pyr$ | 1-iPr-4-Pip |
| 2-3012 | 4-F-Ph | $2-NH_2-4-Pyr$ | 1-Bu-4-(3,4-deH-Pip) |
| 2-3013 | 4-F-Ph | $2-NH_2-4-Pyr$ | 1-tBu-4-(3,4-deH-Pip) |
| 2-3014 | 4-F-Ph | $2-NH_2-4-Pyr$ | 1-Pn-4-(3,4-deH-Pip) |
| 2-3015 | 4-F-Ph | $2-NH_2-4-Pyr$ | 1-Hx-4-(3,4-deH-Pip) |
| 2-3016 | 4-F-Ph | $2-NH_2-4-Pyr$ | 1-Hp-4-(3,4-deH-Pip) |
| 2-3017 | 4-F-Ph | $2-NH_2-4-Pyr$ | 1-Oc-4-(3,4-deH-Pip) |
| 2-3018 | 4-F-Ph | $2-NH_2-4-Pyr$ | 1-Nn-4-(3,4-deH-Pip) |
| 2-3019 | 4-F-Ph | $2-NH_2-4-Pyr$ | 1-cPr-4-(3,4-deH-Pip) |
| 2-3020 | 4-F-Ph | $2-NH_2-4-Pyr$ | 1-cPn-4-(3,4-deH-Pip) |
| 2-3021 | 4-F-Ph | $2-NH_2-4-Pyr$ | 1-cHx-4-(3,4-deH-Pip) |
| 2-3022 | 4-F-Ph | $2-NH_2-4-Pyr$ | 1-Bn-4-(3,4-deH-Pip) |
| 2-3023 | 4-F-Ph | $2-NH_2-4-Pyr$ | 1-Phet-4-(3,4-deH-Pip) |
| 2-3024 | 4-F-Ph | $2-NH_2-4-Pyr$ | 1-(3-Ph-Pr)-4-(3,4-deH-Pip) |
| 2-3025 | 4-F-Ph | $2-NH_2-4-Pyr$ | 1-(4-Ph-Bu)-4-(3,4-deH-Pip) |
| 2-3026 | 4-F-Ph | $2-NH_2-4-Pyr$ | 1-Allyl-4-(3,4-deH-Pip) |
| 2-3027 | 4-F-Ph | $2-NH_2-4-Pyr$ | 1-Propargyl-4-(3,4-deH-Pip) |
| 2-3028 | 4-F-Ph | $2-NH_2-4-Pyr$ | 2,2,6,6-tetraMe-4-(3,4-deH-Pip) |
| 2-3029 | 4-F-Ph | $2-NH_2-4-Pyr$ | 1,2,2,6,6-pentaMe-4-(3,4-deH-Pip) |
| 2-3030 | 4-F-Ph | $2-NH_2-4-Pyr$ | 1,2,2,6,6-pentaMe-4-Pip) |
| 2-3031 | 4-F-Ph | $2-NH_2-4-Pyr$ | 7-(1,2,3,5,6,8a-hexaH-Ind) |
| 2-3032 | 4-F-Ph | $2-NH_2-4-Pyr$ | 7-(1,2,3,5,8,8a-hexaH-Ind) |
| 2-3033 | 4-F-Ph | $2-NH_2-4-Pyr$ | 7-octaH-Ind |
| 2-3034 | 4-F-Ph | $2-NH_2-4-Pyr$ | 8-(1,3,4,6,7,9a-hexaH-2H-Qui) |
| 2-3035 | 4-F-Ph | $2-NH_2-4-Pyr$ | 8-(1,3,4,6,9,9a-hexaH-2H-Qui) |
| 2-3036 | 4-F-Ph | $2-NH_2-4-Pyr$ | 8-octaH-Qui |
| 2-3037 | 4-F-Ph | $2-NH_2-4-Pyr$ | 2,2-diMe-4-(3,4-deH-PiP) |
| 2-3038 | 4-F-Ph | $2-NH_2-4-Pyr$ | 1,2,2-triMe-4-(3,4-deH-PiP) |
| 2-3039 | 4-F-Ph | $2-NH_2-4-Pyr$ | 2,2-diMe-4-(4, 5-deH-PiP) |
| 2-3040 | 4-F-Ph | $2-NH_2-4-Pyr$ | 1,2,2-triMe-4-(4,5-deH-PiP) |
| 2-3041 | 4-P-Ph | $2-NH_2-4-Pyr$ | 2,6-diMe-4-(3,4-deH-PiP) |
| 2-3042 | 4-F-Ph | $2-NH_2-4-Pyr$ | 1,2,6-triMe-4-(3,4-deH-PiP) |
| 2-3043 | 4-F-Ph | $2-NH_2-4-Pyr$ | 2-Me-4-(3,4-deH-Pip) |
| 2-3044 | 4-F-Ph | $2-NH_2-4-Pyr$ | 1,2-diMe-4-(3,4-deH-Pip) |
| 2-3045 | 4-F-Ph | $2-NH_2-4-Pyr$ | 1-Et-2-Me-4-(3,4-deH-Pip) |
| 2-3046 | 4-F-Ph | $2-NH_2-4-Pyr$ | 1-Pr-2-Me-4-(3,4-deH-Pip) |

Table 2 (continued)

| Compound No. | $R^1$ | $R^2$ | $R^3$ |
|---|---|---|---|
| 2-3047 | 4-F-Ph | $2-NH_2$-4-Pyr | 1-Bu-2-Me-4-(3,4-deH-Pip) |
| 2-3048 | 4-F-Ph | $2-NH_2$-4-Pyr | 1-Phet-2-Me-4-(3,4-deH-Pip) |
| 2-3049 | 4-F-Ph | $2-NH_2$-4-Pyr | 2-Et-4-(3,4-deH-Pip) |
| 2-3050 | 4-F-Ph | $2-NH_2$-4-Pyr | 1-Me-2-Et-4-(3,4-deH-Pip) |
| 2-3051 | 4-F-Ph | $2-NH_2$-4-Pyr | 1,2-diEt-9-(3,4-deH-Pip) |
| 2-3052 | 4-F-Ph | $2-NH_2$-4-Pyr | 1-Pr-2-Et-4-(3,4-deH-Pip) |
| 2-3053 | 4-F-Ph | $2-NH_2$-4-Pyr | 1-Bu-2-Et-4-(3,4-deH-Pip) |
| 2-3054 | 4-F-Ph | $2-NH_2$-4-Pyr | 1-Phet-2-Et-4-(3, 4-deH-Pip) |
| 2-3055 | 4-F-Ph | $2-NH_2$-4-Pyr | 2-Pr-4-(3,4-deH-Pip) |
| 2-3056 | 4-F-Ph | $2-NH_2$-4-Pyr | 1-Me-2-Pr-4-(3,4-deH-Pip) |
| 2-3057 | 4-F-Ph | $2-NH_2$-4-Pyr | 1-Et-2-Pr-4-(3,4-deH-Pip) |
| 2-3058 | 4-F-Ph | $2-NH_2$-4-Pyr | 1,2-dipr-4-(3,4-deH-Pip) |
| 2-3059 | 4-F-Ph | $2-NH_2$-4-Pyr | 1-Bu-2-Pr-4-(3,4-deH-Pip) |
| 2-3060 | 4-F-Ph | $2-NH_2$-4-Pyr | 1-Phet-2-Pr-4-(3,4-deH-Pip) |
| 2-3061 | 4-F-Ph | $2-NH_2$-4-Pyr | 2-Bu-4-(3,4-deH-Pip) |
| 2-3062 | 4-F-Ph | $2-NH_2$-4-Pyr | 1-Me-2-Bu-4-(3,4-deH-Pip) |
| 2-3063 | 4-F-Ph | $2-NH_2$-4-Pyr | 1-Et-2-Bu-4-(3,4-deH-Pip) |
| 2-3064 | 4-F-Ph | $2-NH_2$-4-Pyr | 1-Pr-2-Bu-4-(3,4-deH-Pip) |
| 2-3065 | 4-F-Ph | $2-NH_2$-4-Pyr | 1,2-diBu-4-(3,4-deH-Pip) |
| 2-3066 | 4-F-Ph | $2-NH_2$-4-Pyr | 1-Phet-2-Bu-4-(3,4-deH-Pip) |
| 2-3067 | 4-F-Ph | $2-NH_2$-4-Pyr | 2-Allyl-4-(3,4-deH-Pip) |
| 2-3068 | 4-F-Ph | $2-NH_2$-4-Pyr | 1-Me-2-Allyl-4-(3,4-deH-Pip) |
| 2-3069 | 4-F-Ph | $2-NH_2$-4-Pyr | 1-Et-2-Allyl-4-(3,4-deH-Pip) |
| 2-3070 | 4-F-Ph | $2-NH_2$-4-Pyr | 1-Pr-2-Allyl-4-(3,4-deH-Pip) |
| 2-3071 | 4-F-Ph | $2-NH_2$-4-Pyr | 1-Bu-2-Allyl-4-(3,4-deH-Pip) |
| 2-3072 | 4-F-Ph | $2-NH_2$-4-Pyr | 1-Phet-2-Allyl-4-(3,4-deH-Pip) |
| 2-3073 | 4-F-Ph | $2-NH_2$-4-Pyr | 2-Bn-4-(3,4-deH-Pip) |
| 2-3074 | 4-F-Ph | $2-NH_2$-4-Pyr | 1-Me-2-Bn-4-(3,4-deH-Pip) |
| 2-3075 | 4-F-Ph | $2-NH_2$-4-Pyr | 1-Et-2-Bn-4-(3,4-deH-Pip) |
| 2-3076 | 4-F-Ph | $2-NH_2$-4-Pyr | 1-Pr-2-Bn-4-(3,4-deH-Pip) |
| 2-3077 | 4-F-Ph | $2-NH_2$-4-Pyr | 1-Bu-2-Bn-4-(3,4-deH-Pip) |
| 2-3078 | 4-F-Ph | $2-NH_2$-4-Pyr | 1-Phet-2-Bn-4-(3,4-deH-Pip) |
| 2-3079 | 4-F-Ph | $2-NH_2$-4-Pyr | 2-Phet-4-(3,4-deH-Pip) |
| 2-3080 | 4-F-Ph | $2-NH_2$-4-Pyr | 1-Me-2-Phet-4-(3,4-deH-Pip) |
| 2-3081 | 4-F-Ph | $2-NH_2$-4-Pyr | 1-Et-2-Phet-4-(3,4-deH-Pip) |
| 2-3082 | 4-F-Ph | $2-NH_2$-4-Pyr | 1-Pr-2-Phet-4-(3,4-deH-Pip) |
| 2-3083 | 4-F-Ph | $2-NH_2$-4-Pyr | 1-Bu-2-Phet-4-(3,4-deH-Pip) |
| 2-3084 | 4-F-Ph | $2-NH_2$-4-Pyr | 1,2-diPhet-4-(3,4-deH-Pip) |

Table 2 (continued)

| Compound No. | $R^1$ | $R^2$ | $R^3$ |
|---|---|---|---|
| 2-3085 | 4-F-Ph | 2-NH2-4-Pyr | 2-Me-4-(4,5-deH-Pip) |
| 2-3086 | 4-F-Ph | 2-$NH_2$-4-Pyr | 1,2-diMe-4-(4,5-deH-Pip) |
| 2-3087 | 4-F-Ph | 2-$NH_2$-4-Pyr | 1-Et-2-Me-4-(4,5-deH-Pip) |
| 2-3088 | 4-F-Ph | 2-$NH_2$-4-Pyr | 1-Pr-2-Me-4-(4,5-deH-Pip) |
| 2-3089 | 4-F-Ph | 2-$NH_2$-4-Pyr | 1-Bu-2-Me-4-(4,5-deH-Pip) |
| 2-3090 | 4-F-Ph | 2-$NH_2$-4-Pyr | 1-Phet-2-Me-4-(4,5-deH-Pip) |
| 2-3091 | 4-F-Ph | 2-$NH_2$-4-Pyr | 2-Et-4-(4,5-deH-Pip) |
| 2-3092 | 4-F-Ph | 2-$NH_2$-4-Pyr | 1-Me-2-Et-4-(4,5-deH-Pip) |
| 2-3093 | 4-F-Ph | 2-$NH_2$-4-Pyr | 1,2-diEt-4-(4,5-deH-Pip) |
| 2-3094 | 4-F-Ph | 2-$NH_2$-4-Pyr | 1-Pr-2-Et-4-(4,5-deH-Pip) |
| 2-3095 | 4-F-Ph | 2-$NH_2$-4-Pyr | 1-Bu-2-Et-4-(4,5-deH-Pip) |
| 2-3096 | 4-F-Ph | 2-$NH_2$-4-Pyr | 1-Phet-2-Et-4-(4,5-deH-Pip) |
| 2-3097 | 4-F-Ph | 2-$NH_2$-4-Pyr | 2-Pr-4-(4,5-deH-Pip) |
| 2-3098 | 4-F-Ph | 2-$NH_2$-4-Pyr | 1-Me-2-Pr-4-(4,5-deH-Pip) |
| 2-3099 | 4-F-Ph | 2-$NH_2$-4-Pyr | 1-Et-2-Pr-4-(4,5-deH-Pip) |
| 2-3100 | 4-F-Ph | 2-$NH_2$-4-Pyr | 1,2-diPr-4-(4,5-deH-Pip) |
| 2-3101 | 4-F-Ph | 2-$NH_2$-4-Pyr | 1-Bu-2-Pr-4-(4,5-deH-Pip) |
| 2-3102 | 4-F-Ph | 2-$NH_2$-4-Pyr | 1-Phet-2-Pr-4-(4,5-deH-Pip) |
| 2-3103 | 4-F-Ph | 2-$NH_2$-4-Pyr | 2-Bu-4-(4,5-deH-Pip) |
| 2-3104 | 4-F-Ph | 2-$NH_2$-4-Pyr | 1-Me-2-Bu-4-(4,5-deH-Pip) |
| 2-3105 | 4-F-Ph | 2-$NH_2$-4-Pyr | 1-Et-2-Bu-4-(4,5-deH-Pip) |
| 2-3106 | 4-F-Ph | 2-$NH_2$-4-Pyr | 1-Pr-2-Bu-4-(4,5-deH-Pip) |
| 2-3107 | 4-F-Ph | 2-$NH_2$-4-Pyr | 1,2-diBu-4-(4,5-deH-Pip) |
| 2-3108 | 4-F-Ph | 2-$NH_2$-4-Pyr | 1-Phet-2-Bu-4-(4,5-deH-Pip) |
| 2-3109 | 4-F-Ph | 2-$NH_2$-4-Pyr | 2-Allyl-4-(4,5-deH-Pip) |
| 2-3110 | 4-F-Ph | 2-$NH_2$-4-Pyr | 1-Me-2-Allyl-4-(4,5-deH-Pip) |
| 2-3111 | 4-F-Ph | 2-$NH_2$-4-Pyr | 1-Et-2-Allyl-4-(4,5-deH-Pip) |
| 2-3112 | 4-F-Ph | 2-$NH_2$-4-Pyr | 1-Pr-2-Allyl-4-(4,5-deH-Pip) |
| 2-3113 | 4-F-Ph | 2-$NH_2$-4-Pyr | 1-Bu-2-Allyl-4-(4,5-deH-Pip) |
| 2-3114 | 4-F-Ph | 2-$NH_2$-4-Pyr | 1-Phet-2-Allyl-4-(4,5-deH-Pip) |
| 2-3115 | 4-F-Ph | 2-$NH_2$-4-Pyr | 2-Bn-4-(4,5-deH-Pip) |
| 2-3116 | 4-F-Ph | 2-$NH_2$-4-Pyr | 1-Me-2-Bn-4-(4,5-deH-Pip) |
| 2-3117 | 4-F-Ph | 2-$NH_2$-4-Pyr | 1-Et-2-Bn-4-(4,5-deH-Pip) |
| 2-3118 | 4-F-Ph | 2-$NH_2$-4-Pyr | 1-Pr-2-Bn-4-(4,5-deH-Pip) |
| 2-3119 | 4-F-Ph | 2-$NH_2$-4-Pyr | 1-Bu-2-Bn-4-(4,5-deH-Pip) |
| 2-3120 | 4-F-Ph | 2-$NH_2$-4-Pyr | 1-Phet-2-Bn-4-(4,5-deH-Pip) |
| 2-3121 | 4-F-Ph | 2-$NH_2$-4-Pyr | 2-Phet-4-(4,5-deH-Pip) |
| 2-3122 | 4-F-Ph | 2-$NH_2$-4-Pyr | 1-Me-2-Phet-4-(4,5-deH-Pip) |

Table 2 (continued)

| Compound No. | $R^1$ | $R^2$ | $R^3$ |
|---|---|---|---|
| 2-3123 | 4-F-Ph | $2-NH_2-4-Pyr$ | 1-Et-2-Phet-4-(4,5-deH-Pip) |
| 2-3124 | 4-F-Ph | $2-NH_2-4-Pyr$ | 1-Pr-2-Phet-4-(4,5-deH-Pip) |
| 2-3125 | 4-F-Ph | $2-NH_2-4-Pyr$ | 1-Bu-2-Phet-4-(4,5-deH-Pip) |
| 2-3126 | 4-F-Ph | $2-NH_2-4-Pyr$ | 1,2-diPhet-4-(4,5-deH-Pip) |
| 2-3127 | 4-F-Ph | 2-MeNH-4-Pyr | $H_2N-CH_2C(Me)_2CH_2$ |
| 2-3128 | 4-F-Ph | 2-MeNH-4-Pyr | $MeNH-CH_2C(Me)_2CH_2$ |
| 2-3129 | 4-F-Ph | 2-MeNH-4-Pyr | $EtNH-CH_2C(Me)_2CH_2$ |
| 2-3130 | 4-F-Ph | 2-MeNH-4-Pyr | $Me_2N-CH_2C(Me)_2CH_2$ |
| 2-3131 | 4-F-Ph | 2-MeNH-4-Pyr | 3-(3,4-deH-Pip) |
| 2-3132 | 4-F-Ph | 2-MeNH-4-Pyr | 1-Me-3-(3,4-deH-Pip) |
| 2-3133 | 4-F-Ph | 2-MeNH-4-Pyr | 1-Et-9-(3,4-deH-Pip) |
| 2-3134 | 4-F-Ph | 2-MeNH-4-Pyr | 1-Pr-4-(3,4-deH-Pip) |
| 2-3135 | 4-F-Ph | 2-MeNH-4-Pyr | 1-Pr-4-Pip |
| 2-3136 | 4-F-Ph | 2-MeNH-4-Pyr | 1-iPr-4-(3,4-deH-Pip) |
| 2-3137 | 4-F-Ph | 2-MeNH-4-Pyr | 1-iPr-4-Pip |
| 2-3138 | 4-F-Ph | 2-MeNH-4-Pyr | 1-Bu-4-(3,4-deH-Pip) |
| 2-3139 | 4-F-Ph | 2-MeNH-4-Pyr | 1-tBu-4-(3,4-deH-Pip) |
| 2-3140 | 4-F-Ph | 2-MeNH-4-Pyr | 1-Pn-4-(3,4-deH-Pip) |
| 2-3141 | 4-F-Ph | 2-MeNH-4-Pyr | 1-Hx-4-(3,4-deH-Pip) |
| 2-3142 | 4-F-Ph | 2-MeNH-4-Pyr | 1-Hp-4-(3,4-deH-Pip) |
| 2-3143 | 4-F-Ph | 2-MeNH-4-Pyr | 1-Oc-4-(3,4-deH-Pip) |
| 2-3144 | 4-F-Ph | 2-MeNH-4-Pyr | 1-Nn-4-(3,4-deH-Pip) |
| 2-3145 | 4-F-Ph | 2-MeNH-4-Pyr | 1-cPr-4-(3,4-deH-Pip) |
| 2-3146 | 4-F-Ph | 2-MeNH-4-Pyr | 1-cPn-4-(3,4-deH-Pip) |
| 2-3147 | 4-F-Ph | 2-MeNH-4-Pyr | 1-cHx-4-(3,4-deH-Pip) |
| 2-3148 | 4-F-Ph | 2-MeNH-4-Pyr | 1-Bn-4-(3,4-deH-Pip) |
| 2-3149 | 4-F-Ph | 2-MeNH-4-Pyr | 1-Phet-4-(3,4-deH-Pip) |
| 2-3150 | 4-F-Ph | 2-MeNH-4-Pyr | 1-(3-Ph-Pr)-4-(3,4-deH-Pip) |
| 2-3151 | 4-F-Ph | 2-MeNH-4-Pyr | 1-(4-Ph-Bu)-4-(3,4-deH-Pip) |
| 2-3152 | 4-F-Ph | 2-MeNH-4-Pyr | 1-Allyl-4-(3,4-deH-Pip) |
| 2-3153 | 4-F-Ph | 2-MeNH-4-Pyr | 1-Propargyl-4-(3,4-deH-Pip) |
| 2-3154 | 4-F-Ph | 2-MeNH-4-Pyr | 2,2,6,6-tetraMe-4-(3,4-deH-Pip) |
| 2-3155 | 4-F-Ph | 2-MeNH-4-Pyr | 1,2,2,6,6-pentaMe-4-(3,4-deH-Pip) |
| 2-3156 | 4-F-Ph | 2-MeNH-4-Pyr | 1,2,2,6,6-pentaMe-4-Pip) |
| 2-3157 | 4-F-Ph | 2-MeNH-4-Pyr | 7-(1,2,3,5,6,8a-hexaH-Ind) |
| 2-3158 | 4-F-Ph | 2-MeNH-4-Pyr | 7-(1,2,3,5,8,8a-hexaH-Ind) |
| 2-3159 | 4-F-Ph | 2-MeNH-4-Pyr | 7-octaH-Ind |
| 2-3160 | 4-F-Ph | 2-MeNH-4-Pyr | 8-(1,3,4,6,7,9a-hexaH-2H-Qui) |

Table 2 (continued)

| Compound No. | $R^1$ | $R^2$ | $R^3$ |
|---|---|---|---|
| 2-3161 | 4-F-Ph | 2-MeNH-4-Pyr | 8-(1,3,4,6,9,9a-hexaH-2H-Qui) |
| 2-3162 | 4-F-Ph | 2-MeNH-4-Pyr | 8-octaH-Qui |
| 2-3163 | 4-F-Ph | 2-MeNH-4-Pyr | 2,2-diMe-4-(3,4-deH-PiP) |
| 2-3164 | 4-F-Ph | 2-MeNH-4-Pyr | 1,2,2-triMe-4-(3,4-deH-PiP) |
| 2-3165 | 4-F-Ph | 2-MeNH-4-Pyr | 2,2-diMe-4-(4,5-deH-PiP) |
| 2-3166 | 4-F-Ph | 2-MeNH-4-Pyr | 1,2,2-triMe-4-(4,5-deH-PiP) |
| 2-3167 | 4-F-Ph | 2-MeNH-4-Pyr | 2,6-diMe-4-(3,4-deH-PiP) |
| 2-3168 | 4-F-Ph | 2-MeNH-4-Pyr | 1,2,6-triMe-4-(3,4-deH-PiP) |
| 2-3169 | 4-F-Ph | 2-MeNH-4-Pyr | 2-Me-4-(3,4-deH-Pip) |
| 2-3170 | 4-F-Ph | 2-MeNH-4-Pyr | 1,2-diMe-4-(3,4-deH-Pip) |
| 2-3171 | 4-F-Ph | 2-MeNH-4-Pyr | 1-Et-2-Me-4-(3,4-deH-Pip) |
| 2-3172 | 4-F-Ph | 2-MeNH-4-Pyr | 1-Pr-2-Me-4-(3,4-deH-Pip) |
| 2-3173 | 4-F-Ph | 2-MeNH-4-Pyr | 1-Bu-2-Me-4-(3,4-deH-Pip) |
| 2-3174 | 4-F-Ph | 2-MeNH-4-Pyr | 1-Phet-2-Me-4-(3,4-deH-Pip) |
| 2-3175 | 4-F-Ph | 2-MeNH-4-Pyr | 2-Et-4-(3,4-deH-Pip) |
| 2-3176 | 4-F-Ph | 2-MeNH-4-Pyr | 1-Me-2-Et-4-(3,4-deH-Pip) |
| 2-3177 | 4-F-Ph | 2-MeNH-4-Pyr | 1,2-diEt-4-(3,4-deH-Pip) |
| 2-3178 | 4-F-Ph | 2-MeNH-4-Pyr | 1-Pr-2-Et-4-(3,4-deH-Pip) |
| 2-3179 | 4-F-Ph | 2-MeNH-4-Pyr | 1-Bu-2-Et-4-(3,4-deH-Pip) |
| 2-3180 | 4-F-Ph | 2-MeNH-4-Pyr | 1-Phet-2-Et-4-(3,4-deH-Pip) |
| 2-3181 | 4-F-Ph | 2-MeNH-4-Pyr | 2-Pr-4-(3,4-deH-Pip) |
| 2-3182 | 4-F-Ph | 2-MeNH-4-Pyr | 1-Me-2-Pr-4-(3,4-deH-Pip) |
| 2-3183 | 4-F-Ph | 2-MeNH-4-Pyr | 1-Et-2-Pr-4-(3,4-deH-Pip) |
| 2-3184 | 4-F-Ph | 2-MeNH-4-Pyr | 1,2-diPr-4-(3,4-deH-Pip) |
| 2-3185 | 4-F-Ph | 2-MeNH-4-Pyr | 1-Bu-2-Pr-4-(3,4-deH-Pip) |
| 2-3186 | 4-F-Ph | 2-MeNH-4-Pyr | 1-Phet-2-Pr-4-(3,4-deH-Pip) |
| 2-3187 | 4-F-Ph | 2-MeNH-4-Pyr | 2-Bu-4-(3,4-deH-Pip) |
| 2-3188 | 4-F-Ph | 2-MeNH-4-Pyr | 1-Me-2-Bu-4-(3,4-deH-Pip) |
| 2-3189 | 4-F-Ph | 2-MeNH-4-Pyr | 1-Et-2-Bu-4-(3,4-deH-Pip) |
| 2-3190 | 4-F-Ph | 2-MeNH-4-Pyr | 1-Pr-2-Bu-4-(3,4-deH-Pip) |
| 2-3191 | 4-F-Ph | 2-MeNH-4-Pyr | 1,2-diBu-4-(3,4-deH-Pip) |
| 2-3192 | 4-F-Ph | 2-MeNH-4-Pyr | 1-Phet-2-Bu-4-(3,4-deH-Pip) |
| 2-3193 | 4-F-Ph | 2-MeNH-4-Pyr | 2-Allyl-4-(3,4-deH-Pip) |
| 2-3194 | 4-F-Ph | 2-MeNH-4-Pyr | 1-Me-2-Allyl-4-(3,4-deH-Pip) |
| 2-3195 | 4-F-Ph | 2-MeNH-4-Pyr | 1-Et-2-Allyl-4-(3,4-deH-Pip) |
| 2-3196 | 4-F-Ph | 2-MeNH-4-Pyr | 1-Pr-2-Allyl-4-(3,4-deH-Pip) |
| 2-3197 | 4-F-Ph | 2-MeNH-4-Pyr | 1-Bu-2-Allyl-4-(3,4-deH-Pip) |
| 2-3198 | 4-F-Ph | 2-MeNH-4-Pyr | 1-Phet-2-Allyl-4-(3,4-deH-Pip) |

Table 2 (continued)

| Compound No. | $R^1$ | $R^2$ | $R^3$ |
|---|---|---|---|
| 2-3199 | 4-F-Ph | 2-MeNH-4-Pyr | 2-Bn-4-(3,4-deH-Pip) |
| 2-3200 | 4-F-Ph | 2-MeNH-4-Pyr | 1-Me-2-Bn-4-(3,4-deH-Pip) |
| 2-3201 | 4-F-Ph | 2-MeNH-4-Pyr | 1-Et-2-Bn-4-(3,4-deH-Pip) |
| 2-3202 | 4-F-Ph | 2-MeNH-4-Pyr | 1-Pr-2-Bn-4-(3,4-deH-Pip) |
| 2-3203 | 4-F-Ph | 2-MeNH-4-Pyr | 1-Bu-2-Bn-4-(3,4-deH-Pip) |
| 2-3204 | 4-F-Ph | 2-MeNH-4-Pyr | 1-Phet-2-Bn-4-(3,4-deH-Pip) |
| 2-3205 | 4-F-Ph | 2-MeNH-4-Pyr | 2-Phet-4-(3,4-deH-Pip) |
| 2-3206 | 4-F-Ph | 2-MeNH-4-Pyr | 1-Me-2-Phet-4-(3,4-deH-Pip) |
| 2-3207 | 4-F-Ph | 2-MeNH-4-Pyr | 1-Et-2-Phet-4-(3,4-deH-Pip) |
| 2-3208 | 4-F-Ph | 2-MeNH-4-Pyr | 1-Pr-2-Phet-4-(3,4-deH-Pip) |
| 2-3209 | 4-F-Ph | 2-MeNH-4-Pyr | 1-Bu-2-Phet-4-(3,4-deH-Pip) |
| 2-3210 | 4-F-Ph | 2-MeNH-4-Pyr | 1,2-diPhet-4-(3,4-deH-Pip) |
| 2-3211 | 4-F-Ph | 2-MeNH-4-Pyr | 2-Me-4-(4,5-deH-Pip) |
| 2-3212 | 4-F-Ph | 2-MeNH-4-Pyr | 1,2-diMe-4-(4,5-deH-Pip) |
| 2-3213 | 4-F-Ph | 2-MeNH-4-Pyr | 1-Et-2-Me-4-(4,5-deH-Pip) |
| 2-3214 | 4-F-Ph | 2-MeNH-4-Pyr | 1-Pr-2-Me-4-(4,5-deH-Pip) |
| 2-3215 | 4-F-Ph | 2-MeNH-4-Pyr | 1-Bu-2-Me-4-(4,5-deH-Pip) |
| 2-3216 | 4-F-Ph | 2-MeNH-4-Pyr | 1-Phet-2-Me-4-(4,5-deH-Pip) |
| 2-3217 | 4-F-Ph | 2-MeNH-4-Pyr | 2-Et-4-(4,5-deH-Pip) |
| 2-3218. | 4-F-Ph | 2-MeNH-4-Pyr | 1-Me-2-Et-4-(4,5-deH-Pip) |
| 2-3219 | 4-F-Ph | 2-MeNH-4-Pyr | 1,2-diEt-4-(4,5-deH-Pip) |
| 2-3220 | 4-F-Ph | 2-MeNH-4-Pyr | 1-Pr-2-Et-4-(I,5-deH-Pip) |
| 2-3221 | 4-F-Ph | 2-MeNH-4-Pyr | 1-Bu-2-Et-4-(4,5-deH-Pip) |
| 2-3222 | 4-F-Ph | 2-MeNH-4-Pyr | 1-Phet-2-Et-4-(4,5-deH-Pip) |
| 2-3223 | 4-F-Ph | 2-MeNH-4-Pyr | 2-Pr-4-(4,5-deH-Pip) |
| 2-3224 | 4-F-Ph | 2-MeNH-4-Pyr | 1-Me-2-Pr-4-(4,5-deH-Pip) |
| 2-3225 | 4-F-Ph | 2-MeNH-4-Pyr | 1-Et-2-Pr-4-(9,5-deH-Pip) |
| 2-3226 | 4-F-Ph | 2-MeNH-4-Pyr | 1,2-diPr-4-(4,5-deH-Pip) |
| 2-3227 | 4-F-Ph | 2-MeNH-4-Pyr | 1-Bu-2-Pr-4-(4,5-deH-Pip) |
| 2-3228 | 4-F-Ph | 2-MeNH-4-Pyr | 1-Phet-2-Pr-4-(4,5-deH-Pip) |
| 2-3229 | 4-F-Ph | 2-MeNH-4-Pyr | 2-Bu-4-(4,5-deH-Pip) |
| 2-3230 | 4-F-Ph | 2-MeNH-4-Pyr | 1-Me-2-Bu-4-(4,5-deH-Pip) |
| 2-3231 | 4-F-Ph | 2-MeNH-4-Pyr | 1-Et-2-Bu-4-(4,5-deH-Pip) |
| 2-3232 | 4-F-Ph | 2-MeNH-4-Pyr | 1-Pr-2-Bu-4-(4,5-deH-Pip) |
| 2-3233 | 4-F-Ph | 2-MeNH-4-Pyr | 1,2-diBu-4-(4,5-deH-Pip) |
| 2-3234 | 4-F-Ph | 2-MeNH-4-Pyr | 1-Phet-2-Bu-4-(4,5-deH-Pip) |
| 2-3235 | 4-F-Ph | 2-MeNH-4-Pyr | 2-Allyl-4-(4,5-deH-Pip) |
| 2-3236 | 4-F-Ph | 2-MeNH-4-Pyr | 1-Me-2-Allyl-4-(4,5-deH-Pip) |

Table 2 (continued)

| Compound No. | $R^1$ | $R^2$ | $R^3$ |
|---|---|---|---|
| 2-3237 | 4-F-Ph | 2-MeNH-4-Pyr | 1-Et-2-Allyl-4-(4,5-deH-Pip) |
| 2-3238 | 4-F-Ph | 2-MeNH-4-Pyr | 1-Pr-2-Allyl-4-(4,5-deH-Pip) |
| 2-3239 | 4-F-Ph | 2-MeNH-4-Pyr | 1-Bu-2-Allyl-4-(4,5-deH-Pip) |
| 2-3240 | 4-F-Ph | 2-MeNH-4-Pyr | 1-Phet-2-Allyl-4-(4,5-deH-Pip) |
| 2-3241 | 4-F-Ph | 2-MeNH-4-Pyr | 2-Bn-4-(4,5-deH-Pip) |
| 2-3242 | 4-F-Ph | 2-MeNH-4-Pyr | 1-Me-2-Bn-4-(4,5-deH-Pip) |
| 2-3243 | 4-F-Ph | 2-MeNH-4-Pyr | 1-Et-2-Bn-4-(4,5-deH-Pip) |
| 2-3244 | 4-F-Ph | 2-MeNH-4-Pyr | 1-Pr-2-Bn-4-(4,5-deH-Pip) |
| 2-3245 | 4-F-Ph | 2-MeNH-4-Pyr | 1-Bu-2-Bn-4-(4,5-deH-Pip) |
| 2-3246 | 4-F-Ph | 2-MeNH-4-Pyr | 1-Phet-2-Bn-4-(4,5-deH-Pip) |
| 2-3247 | 4-F-Ph | 2-MeNH-4-Pyr | 2-Phet-4-(4,5-deH-Pip) |
| 2-3248 | 4-F-Ph | 2-MeNH-4-Pyr | 1-Me-2-Phet-4-(4,5-deH-Pip) |
| 2-3249 | 4-F-Ph | 2-MeNH-4-Pyr | 1-Et-2-Phet-4-(4,5-deH-Pip) |
| 2-3250 | 4-F-Ph | 2-MeNH-4-Pyr | 1-Pr-2-Phet-4-(4,5-deH-Pip) |
| 2-3251 | 4-F-Ph | 2-MeNH-4-Pyr | 1-Bu-2-Phet-4-(4,5-deH-Pip) |
| 2-3252 | 4-F-Ph | 2-MeNH-4-Pyr | 1,2-diPhet-4-(4,5-deH-Pip) |
| 2-3253 | Ph | 4-Pyr | 7-(1,2,3,5,6,8a-hexaH-Ind) |
| 2-3254 | Ph | 4-Pyr | 7-(1,2,3,5,8,8a-hexaH-Ind) |
| 2-3255 | 3-F-Ph | 4-Pyr | 7-(1,2,3,5,6,8a-hexaH-Ind) |
| 2-3256 | 3-F-Ph | 4-Pyr | 7-(1,2,3,5,8,8a-hexaH-Ind) |
| 2-3257 | 3-Cl-Ph | 4-Pyr | 7-(1,2,3,5,6,8a-hexaH-Ind) |
| 2-3258 | 3-Cl-Ph | 4-Pyr | 7-(1,2,3,5,8,8a-hexaH-Ind) |
| 2-3259 | 3,4-diF-Ph | 4-Pyr | 7-(1,2,3,5,6,8a-hexaH-Ind) |
| 2-3260 | 3,4-diF-Ph | 4-Pyr | 7-(1,2,3,5,8,8a-hexaH-Ind) |
| 2-3261 | 3-$CF_3$-Ph | 4-Pyr | 7-(1,2,3,5,6,8a-hexaH-Ind) |
| 2-3262 | 3-$CF_3$-Ph | 4-Pyr | 7-(1,2,3,5,8,8a-hexaH-Ind) |
| 2-3263 | 4-F-Ph | 4-Pyr | 2-Pyrd-CH=CH- |
| 2-3264 | 4-F-Ph | 4-Pyr | 1-Me-2-Pyrd-CH=CH- |
| 2-3265 | 4-F-Ph | 4-Pyr | 1-Et-2-Pyrd-CH=CH- |
| 2-3266 | 4-F-Ph | 4-Pyr | 1-Pr-2-Pyrd-CH=CH- |
| 2-3267 | 4-F-Ph | 4-Pyr | 2-Pip-CH=CH- |
| 2-3268 | 4-F-Ph | 4-Pyr | 1-Me-2-Pip-CH=CH- |
| 2-3269 | 4-F-Ph | 4-Pyr | 1-Et-2-Pip-CH=CH- |
| 2-3270 | 4-F-Ph | 4-Pyr | 1-Pr-2-Pip-CH=CH- |
| 2-3271 | Ph | 4-Pyr | 2-Pyrd-CH=CH- |
| 2-3272 | Ph | 4-Pyr | 1-Me-2-Pyrd-CH=CH- |
| 2-3273 | Ph | 4-Pyr | 1-Et-2-Pyrd-CH=CH- |
| 2-3274 | Ph | 4-Pyr | 1-Pr-2-Pyrd-CH=CH- |

Table 2 (continued)

| Compound No. | $R^1$ | $R^2$ | $R^3$ |
|---|---|---|---|
| 2-3275 | Ph | 4-Pyr | 2-Pip-CH=CH- |
| 2-3276 | Ph | 4-Pyr | 1-Me-2-Pip-CH=CH- |
| 2-3277 | Ph | 4-Pyr | 1-Et-2-Pip-CH=CH- |
| 2-3278 | Ph | 4-Pyr | 1-Pr-2-Pip-CH=CH- |
| 2-3279 | 3-F-Ph | 4-Pyr | 2-Pyrd-CH=CH- |
| 2-3280 | 3-F-Ph | 4-Pyr | 1-Me-2-Pyrd-CH=CH- |
| 2-3281 | 3-F-Ph | 4-Pyr | 1-Et-2-Pyrd-CH=CH- |
| 2-3282 | 3-F-Ph | 4-Pyr | 1-Pr-2-Pyrd-CH=CH- |
| 2-3283 | 3-F-Ph | 4-Pyr | 2-Pip-CH=CH- |
| 2-3284 | 3-F-Ph | 4-Pyr | 1-Me-2-Pip-CH=CH- |
| 2-3285 | 3-F-Ph | 4-Pyr | 1-Et-2-Pip-CH=CH- |
| 2-3286 | 3-F-Ph | 4-Pyr | 1-Pr-2-Pip-CH=CH- |
| 2-3287 | 3-Cl-Ph | 4-Pyr | 2-Pyrd-CH=CH- |
| 2-3288 | 3-Cl-Ph | 4-Pyr | 1-Me-2-Pyrd-CH=CH- |
| 2-3289 | 3-Cl-Ph | 4-Pyr | 1-Et-2-Pyrd-CH=CH- |
| 2-3290 | 3-Cl-Ph | 4-Pyr | 1-Pr-2-Pyrd-CH=CH- |
| 2-3291 | 3-Cl-Ph | 4-Pyr | 2-Pip-CH=CH- |
| 2-3292 | 3-Cl-Ph | 4-Pyr | 1-Me-2-Pip-CH=CH- |
| 2-3293 | 3-Cl-Ph | 4-Pyr | 1-Et-2-Pip-CH=CH- |
| 2-3294 | 3-Cl-Ph | 4-Pyr | 1-Pr-2-Pip-CH=CH- |
| 2-3295 | 3,4-diF-Ph | 4-Pyr | 2-Pyrd-CH=CH- |
| 2-3296 | 3,4-diF-Ph | 4-Pyr | 1-Me-2-Pyrd-CH=CH- |
| 2-3297 | 3,4-diF-Ph | 4-Pyr | 1-Et-2-Pyrd-CH=CH- |
| 2-3298 | 3,4-diF-Ph | 4-Pyr | 1-Pr-2-Pyrd-CH=CH- |
| 2-3299 | 3,4-diF-Ph | 4-Pyr | 2-Pip-CH=CH- |
| 2-3300 | 3,4-diF-Ph | 4-Pyr | 1-Me-2-Pip-CH=CH- |
| 2-3301 | 3,4-diF-Ph | 4-Pyr | 1-Et-2-Pip-CH=CH- |
| 2-3302 | 3,4-diF-Ph | 4-Pyr | 1-Pr-2-Pip-CH=CH- |
| 2-3303 | 3-$CF_3$-Ph | 4-Pyr | 2-Pyrd-CH=CH- |
| 2-3304 | 3-$CF_3$-Ph | 4-Pyr | 1-Me-2-Pyrd-CH=CH- |
| 2-3305 | 3-$CF_3$-Ph | 4-Pyr | 1-Et-2-Pyrd-CH=CH- |
| 2-3306 | 3-$CF_3$-Ph | 4-Pyr | 1-Pr-2-Pyrd-CH=CH- |
| 2-3307 | 3-$CF_3$-Ph | 4-Pyr | 2-Pip-CH=CH- |
| 2-3308 | 3-$CF_3$-Ph | 4-Pyr | 1-Me-2-Pip-CH=CH- |
| 2-3309 | 3-$CF_3$-Ph | 4-Pyr | 1-Et-2-Pip-CH=CH- |
| 2-3310 | 3-$CF_3$-Ph | 4-Pyr | 1-Pr-2-Pip-CH=CH- |

In these Tables, the following abbreviations are used:

| | |
|---|---|
| ABN | 9-azabicyclo[3.3.1]nonanyl |
| deH-ABN | dehydro[9-azabicyclo[3.3.1]nonanyl] (i.e. 9-azabicyclo[3.3.1]nonenyl) |
| ABO | 8-azabicyclo[3.2.1]octanyl |
| deH-AOB | dehydro[8-azabicyclo[3.2.1]octanyl] (i.e. 8-azabicyclo[3.2.1]octenyl) |
| Ac | acetyl |
| Allyl | allyl |
| Azt | azetidinyl |
| Bn | benzyl |
| Bu | butyl |
| t-Bu | t-butyl |
| Bz | benzoyl |
| Et | ethyl |
| Hp | heptyl |
| Hpip | homopiperidinyl |
| Hx | hexyl |
| cHx | cyclohexyl |
| Ind | indolizinyl |
| Me | methyl |
| Mor | morpholinyl |
| Nn | nonyl |
| Oc | octyl |
| Ph | phenyl |
| Phet | phenethyl |
| Propargyl | propargyl |
| Pip | piperidyl |
| deH-Pip | dehydropiperidyl (i.e. tetrahydropyridyl) |
| Piz | piperazinyl |
| Pn | pentyl |
| cPn | cyclopentyl |
| Pr | propyl |
| iPr | isopropyl |
| cPr | cyclopropyl |
| Pym | pyrimidinyl |
| Pyr | pyridyl |
| Pyrd | pyrrolidinyl |
| Qui | quinolizinyl |
| Qun | quinuclidinyl |
| deH-Qun | dehydroquinuclidinyl (i.e. quinuclidienyl) |
| Tmor | thiomorpholinyl. |

Of the above compounds in Tables 1 and 2, the following are preferred, namely Compounds Nos.: 1-1 to 1-32, 1-35, 1-38, 1-40, 1-42, 1-43, 1-45 to 1-52, 1-65 to 1-67, 1-71 to 1-76, 1-88 to 1-90, 1-94 to 1-126, 1-129, 1-132, 1-134 to 1-137, 1-139 to 1-146, 1-159 to 1-161, 1-165 to 1-170, 1-182 to 1-184, 1-188 to 1-219, 1-222, 1-225, 1-227, 1-229, 1-230, 1-232 to 1-239, 1-252 to 1-254, 1-258 to 1-263, 1-275 to 1-277, 1-281 to 1-312, 1-315, 1-318, 1-320, 1-322, 1-323, 1-325 to 1-332, 1-345 to 1-347, 1-351 to 1-356, 1-368 to 1-370, 1-374 to 1-405, 1-408, 1-411, 1-413, 1-415, 1-416, 1-418 to 1-425, 1-438 to 1-440, 1-444 to 1-449, 1-461 to 1-463, 1-467 to 1-498, 1-501, 1-504, 1-506, 1-508, 1-509, 1-511 to 1-518, 1-531 to 1-533, 1-537 to 1-542, 1-554 to 1-556, 1-560 to 1-591, 1-594, 1-597, 1-599, 1-601, 1-602, 1-604 to 1-611, 1-624 to 1-626, 1-630 to 1-635, 1-647 to 1-649, 1-653 to 1-684, 1-687, 1-690, 1-692, 1-694, 1-695, 1-697 to 1-704, 1-717 to 1-719, 1-723 to 1-728, 1-740 to 1-742, 1-746 to 1-777, 1-780, 1-783, 1-785, 1-787, 1-788, 1-790 to 1-797, 1-810 to 1-812, 1-816 to 1-821, 1-833 to 1-835, 1-839 to 1-870, 1-873, 1-876, 1-878, 1-880, 1-881, 1-883 to 1-890, 1-903 to 1-905, 1-909 to 1-914, 1-926 to 1-928, 1-932 to 1-963, 1-966, 1-969, 1-971, 1-973, 1-974, 1-976 to 1-983, 1-996 to 1-998, 1-1002 to 1-1007, 1-1019 to 1-1021, 1-1025 to 1-1056, 1--1059, 1-1062, 1-1064, 1-1066, 1-1067, 1-1069 to 1-1076, 1-1089 to 1-1091, 1-1095 to 1-1100, 1-1112 to 1-1114, 1-1118 to 1-1149, 1-1152, 1-1155, 1-1157, 1-1159, 1-1160, 1-1162 to 1-1169, 1-1182 to 1-1184, 1-1188 to 1-1193, 1-1205 to 1-1207, 1-1211 to 1-1242, 1-1245, 1-1248, 1-1250, 1-1252, 1-1253, 1-1255 to 1-1262, 1-1275 to 1-1277, 1-1281 to 1-1286, 1-1298 to 1-1300, 1-1304 to 1-1335, 1-1338, 1-1341, 1-1343, 1-1345, 1-1346, 1-1348 to 1-1355, 1-1368 to 1-1370, 1-1374 to 1-1379, 1-1391 to 1-1393, 1-1397 to 1-1405, 1-1407 to 1-1415, 1-1418, 1-1419, 1-1422 to 1-1425, 1-1427, 1-1428, 1-1432 to 1-1440, 1-1442 to 1-1450, 1-1453, 1-1454, 1-1457 to 1-1460, 1-1462, 1-1463, 1-1467 to

1-1475, 1-1477 to 1-1485, 1-1488, 1-1489, 1-1492 to 1-1495, 1-1497, 1-1498, 1-1502 to 1-1510, 1-1512 to 1-1520, 1-1523, 1-1524, 1-1527 to 1-1530, 1-1532, 1-1533, 1-1537 to 1-1545, 1-1547 to 1-1555, 1-1558, 1-1559, 1-1562 to 1-1565, 1-1567, 1-1568, 1-1572 to 1-1580, 1-1582 to 1-1590, 1-1593, 1-1594, 1-1597 to 1-1600, 1-1602, 1-1603, 1-1607 to 1-1615, 1-1617 to 1-1625, 1-1628, 1-1629, 1-1632 to 1-1635, 1-1637, 1-1638, 1-1642 to 1-1650, 1-1652 to 1-1660, 1-1663, 1-1664, 1-1667 to 1-1670, 1-1672, 1-1673, 1-1677 to 1-1685, 1-1687 to 1-1695, 1-1698, 1-1699, 1-1702 to 1-1705, 1-1707, 1-1708, 1-1712 to 1-1720, 1-1722 to 1-1730, 1-1733, 1-1734, 1-1737 to 1-1740, 1-1742, 1-1743, 1-1747 to 1-1755, 1-1757 to 1-1765, 1-1768, 1-1769, 1-1772 to 1-1775, 1-1777, 1-1778, 1-1782 to 1-1790, 1-1792 to 1-1800, 1-1803, 1-1804, 1-1807 to 1-1810, 1-1812, 1-1813, 1-1817 to 1-1825, 1-1827 to 1-1835, 1-1838, 1-1839, 1-1842 to 1-1845, 1-1847, 1-1848, 1-1852 to 1-1860, 1-1862 to 1-1870, 1-1873, 1-1874, 1-1877 to 1-1880, 1-1882, 1-1883, 1-1887 to 1-1895, 1-1897 to 1-1905, 1-1908, 1-1909, 1-1912 to 1-1915, 1-1917, 1-1918, 1-1922 to 1-1930, 1-1932 to 1-1940, 1-1943, 1-1944, 1-1947 to 1-1950, 1-1952, 1-1953, 1-1957 to 1-1965, 1-1967 to 1-1975, 1-1978, 1-1979, 1-1982 to 1-1985, 1-1987, 1-1988, 1-1992 to 1-2000, 1-2002 to 1-2010, 1-2013, 1-2014, 1-2017 to 1-2020, 1-2022, 1-2023, 1-2027 to 1-2035, 1-2037 to 1-2045, 1-2048, 1-2049, 1-2052 to 1-2055, 1-2057, 1-2058, 1-2062 to 1-2070, 1-2072 to 1-2080, 1-2083, 1-2084, 1-2087 to 1-2090, 1-2092, 1-2093, 1-2097 to 1-2105, 1-2107 to 1-2115, 1-2118, 1-2119, 1-2122 to 1-2125, 1-2127, 1-2128, 1-2132 to 1-2140, 1-2142 to 1-2150, 1-2153, 1-2154, 1-2157 to 1-2160, 1-2162, 1-2163, 1-2167 to 1-2175, 1-2177 to 1-2185, 1-2188, 1-2189, 1-2192 to 1-2195, 1-2197, 1-2198, 1-2202 to 1-2210, 1-2212 to 1-2220, 1-2223, 1-2224, 1-2227 to 1-2230, 1-2232, 1-2233, 1-2237 to 1-2245, 1-2247 to 1-2255, 1-2258, 1-2259, 1-2262 to 1-2265, 1-2267, 1-2268, 1-2272 to 1-2280, 1-2282 to 1-2290, 1-2293, 1-2294, 1-2297 to 1-2300, 1-2302, 1-2303, 1-2307 to 1-2315, 1-2317 to 1-2325, 1-2328, 1-2329, 1-2332 to 1-2335, 1-2337, 1-2338, 1-2342 to 1-2350, 1-2352 to 1-2360, 1-2363, 1-2364, 1-2367 to 1-2370, 1-2372, 1-2373, 1-2377 to 1-2385, 1-2387 to 1-2395, 1-2398, 1-2399, 1-2402 to 1-2405, 1-2407, 1-2408, 1-2412 to 1-2420, 1-2422 to 1-2430, 1-2433, 1-2434, 1-2437 to 1-2440, 1-2442, 1-2443, 1-2447 to 1-2455, 1-2457 to 1-2465, 1-2468, 1-2469, 1-2472 to 1-2475, 1-2477, 1-2478, 1-2482 to 1-2490, 1-2492 to 1-2500, 1-2503, 1-2504, 1-2507 to 1-2510, 1-2512, 1-2513, 1-2517 to 1-2529, 1-2539 to 1-2594, 1-2623 to 1-3252,2-1 to 2-32, 2-35, 2-38, 2-40, 2-42, 2-43, 2-45 to 2-52, 2-65 to 2-67, 2-71 to 2-76, 2-88 to 2-90, 2-94 to 2-126, 2-129, 2-132, 2-134 to 2-137, 2-139 to 2-146, 2-159 to 2-161, 2-165 to 2-170, 2-182 to 2-184, 2-188 to 2-219, 2-222, 2-225, 2-227, 2-229, 2-230, 2-232 to 2-239, 2-252 to 2-254, 2-258 to 2-263, 2-275 to 2-277, 2-281 to 2-312, 2-315, 2-318, 2-320, 2-322, 2-323, 2-325 to 2-332, 2-345 to 2-347, 2-351 to 2-356, 2-368 to 2-370, 2-374 to 2-405, 2-408, 2-411, 2-413, 2-415, 2-416, 2-418 to 2-425, 2-438 to 2-440, 2-444 to 2-449, 2-461 to 2-463, 2-467 to 2-498, 2-501, 2-504, 2-506/ 2-508, 2-509, 2-511 to 2-518, 2-531 to 2-533, 2-537 to 2-542, 2-554 to 2-556, 2-560 to 2-591, 2-594, 2-597, 2-599, 2-601, 2-602, 2-604 to 2-611, 2-624 to 2-626, 2-630 to 2-635, 2-647 to 2-649, 2-653 to 2-684, 2-687, 2-690, 2-692, 2-694, 2-695, 2-697 to 2-704, 2-717 to 2-719, 2-723 to 2-728, 2-740 to 2-742, 2-746 to 2-777, 2-780, 2-783, 2-785, 2-787, 2-788, 2-790 to 2-797, 2-810 to 2-812, 2-816 to 2-821, 2-833 to 2-835, 2-839 to 2-870, 2-873, 2-876, 2-878, 2-880, 2-881, 2-883 to 2-890, 2-903 to 2-905, 2-909 to 2-914, 2-926 to 2-928, 2-932 to 2-963, 2-966, 2-969, 2-971, 2-973, 2-974, 2-976 to 2-983, 2-996 to 2-998, 2-1002 to 2-1007, 2-1019 to 2-1021, 2-1025 to 2-1056, 2-1059, 2-1062, 2-1064, 2-1066, 2-1067, 2-1069 to 2-1076, 2-1089 to 2-1091, 2-1095 to 2-1100, 2-1112 to 2-1114, 2-1118 to 2-1149, 2-1152, 2-1155, 2-1157, 2-1159, 2-1160, 2-1162 to 2-1169, 2-1182 to 2-1184, 2-1188 to 2-1193, 2-1205 to 2-1207, 2-1211 to 2-1242, 2-1245, 2-1248, 2-1250, 2-1252, 2-1253, 2-1255 to 2-1262, 2-1275 to 2-1277, 2-1281 to 2-1286, 2-1298 to 2-1300, 2-1304 to 2-1335, 2-1338, 2-1341, 2-1343, 2-1345, 2-1346, 2-1348 to 2-1355, 2-1368 to 2-1370, 2-1374 to 2-1379, 2-1391 to 2-1393, 2-1397 to 2-1405, 2-1407 to 2-1415, 2-1418, 2-1419, 2-1422 to 2-1425, 2-1427, 2-1428, 2-1432 to 2-1440, 2-1442 to 2-1450, 2-1453, 2-1454, 2-1457 to 2-1460, 2-1462, 2-1463, 2-1467 to 2-1475, 2-1477 to 2-1485, 2-1488, 2-1489, 2-1492 to 2-1495, 2-1497, 2-1498, 2-1502 to 2-1510, 2-1512 to 2-1520, 2-1523, 2-1524, 2-1527 to 2-1530, 2-1532, 2-1533, 2-1537 to 2-1545, 2-1547 to 2-1555, 2-1558, 2-1559, 2-1562 to 2-1565, 2-1567, 2-1568, 2-1572 to 2-1580, 2-1582 to 2-1590, 2-1593, 2-1594, 2-1597 to 2-1600, 2-1602, 2-1603, 2-1607 to 2-1615, 2-1617 to 2-1625, 2-1628, 2-1629, 2-1632 to 2-1635, 2-1637, 2-1638, 2-1642 to 2-1650, 2-1652 to 2-1660, 2-1663, 2-1664, 2-1667 to 2-1670, 2-1672, 2-1673, 2-1677 to 2-1685, 2-1687 to 2-1695, 2-1698, 2-1699, 2-1702 to 2-1705, 2-1707, 2-1708, 2-1712 to 2-1720, 2-1722 to 2-1730, 2-1733, 2-1734, 2-1737 to 2-1740, 2-1742, 2-1743, 2-1747 to 2-1755, 2-1757 to 2-1765, 2-1768, 2-1769, 2-1772 to 2-1775, 2-1777, 2-1778, 2-1782 to 2-1790, 2-1792 to 2-1800, 2-1803, 2-1804, 2-1807 to 2-1810, 2-1812, 2-1813, 2-1817 to 2-1825, 2-1827 to 2-1835, 2-1838, 2-1839, 2-1842 to 2-1845, 2-1847, 2-1848, 2-1852 to 2-1860, 2-1862 to 2-1870, 2-1873, 2-1874, 2-1877 to 2-1880, 2-1882, 2-1883, 2-1887 to 2-1895, 2-1897 to 2-1905, 2-1908, 2-1909, 2-1912 to 2-1915, 2-1917, 2-1918, 2-1922 to 2-1930, 2-1932 to 2-1940, 2-1943, 2-1944, 2-1947 to 2-1950, 2-1952, 2-1953, 2-1957 to 2-1965, 2-1967 to 2-1975, 2-1978, 2-1979, 2-1982 to 2-1985, 2-1987, 2-1988, 2-1992 to 2-2000, 2-2002 to 2-2010, 2-2013, 2-2014, 2-2017 to 2-2020, 2-2022, 2-2023, 2-2027 to 2-2035, 2-2037 to 2-2045, 2-2048, 2-2049, 2-2052 to 2-2055, 2-2057, 2-2058, 2-2062 to 2-2070, 2-2072 to 2-2080, 2-2083, 2-2084, 2-2087 to 2-2090, 2-2092, 2-2093, 2-2097 to 2-2105, 2-2107 to 2-2115, 2-2118, 2-2119, 2-2122 to 2-2125, 2-2127, 2-2128, 2-2132 to 2-2140, 2-2142 to 2-2150, 2-2153, 2-2154, 2-2157 to 2-2160, 2-2162, 2-2163, 2-2167 to 2-2175, 2-2177 to 2-2185, 2-2188, 2-2189, 2-2192 to 2-2195, 2-2197, 2-2198, 2-2202 to 2-2210, 2-2212 to 2-2220, 2-2223, 2-2224, 2-2227 to 2-2230, 2-2232, 2-2233, 2-2237 to 2-2245, 2-2247 to 2-2255, 2-2258, 2-2259, 2-2262 to 2-2265, 2-2267, 2-2268, 2-2272 to

2-2280, 2-2282 to 2-2290, 2-2293, 2-2294, 2-2297 to 2-2300, 2-2302, 2-2303, 2-2307 to 2-2315, 2-2317 to 2-2325, 2-2328, 2-2329, 2-2332 to 2-2335, 2-2337, 2-2338, 2-2342 to 2-2350, 2-2352 to 2-2360, 2-2363, 2-2364, 2-2367 to 2-2370, 2-2372, 2-2373, 2-2377 to 2-2385, 2-2387 to 2-2395, 2-2398, 2-2399, 2-2402 to 2-2405, 2-2407, 2-2408, 2-2412 to 2-2420, 2-2422 to 2-2430, 2-2433, 2-2434, 2-2437 to 2-2440, 2-2442, 2-2443, 2-2447 to 2-2455, 2-2457 to 2-2465, 2-2468, 2-2469, 2-2472 to 2-2475, 2-2477, 2-2478, 2-2482 to 2-2490, 2-2492 to 2-2500, 2-2503, 2-2504, 2-2507 to 2-2510, 2-2512, 2-2513, 2-2517 to 2-2529, 2-2539 to 2-2594 and 1-2623 to 1-3252;

more preferred are Compounds Nos.:

1-2 to 1-4, 1-6 to 1-8, 1-11, 1-14 to 1-16, 1-18 to 1-20, 1-22 to 1-24, 1-26 to 1-28, 1-30 to 1-32, 1-35, 1-42, 1-43, 1-45 to 1-52, 1-65, 1-66, 1-71 to 1-75, 1-89, 1-94, 1-96 to 1-98, 1-100 to 1-102, 1-105, 1-108 to 1-110, 1-112 to 1-114, 1-116 to 1-118, 1-120 to 1-122, 1-124 to 1-126, 1-129, 1-136, 1-137, 1-139 to 1-146, 1-159, 1-160, 1-165 to 1-169, 1-183, 1-189 to 1-191, 1-193 to 1-195, 1-198, 1-201 to 1-203, 1-205 to 1-207, 1-209 to 1-211, 1-213 to 1-215, 1-217 to 1-219, 1-222, 1-229, 1-230, 1-232 to 1-239, 1-252, 1-253, 1-258 to 1-262, 1-276, 1-282 to 1-284, 1-286 to 1-288, 1-291, 1-294 to 1-296, 1-298 to 1-300, 1-302 to 1-304, 1-306 to 1-308, 1-310 to 1-312, 1-315, 1-322, 1-323, 1-325 to 1-332, 1-345, 1-346, 1-351 to 1-355, 1-369, 1-375 to 1-377, 1-379 to 1-381, 1-384, 1-387 to 1-389, 1-391 to 1-393, 1-395 to 1-397, 1-399 to 1-401, 1-403 to 1-405, 1-408, 1-415, 1-416, 1-418 to 1-425, 1-438, 1-439, 1-444 to 1-448, 1-462, 1-468 to 1-470, 1-472 to 1-474, 1-477, 1-480 to 1-482, 1-484 to 1-486, 1-488 to 1-490, 1-492 to 1-494, 1-496 to 1-498, 1-501, 1-508, 1-509, 1-511 to 1-518, 1-531, 1-532, 1-537 to 1-541, 1-555, 1-561 to 1-563, 1-565 to 1-567, 1-570, 1-573 to 1-575, 1-577 to 1-579, 1-581 to 1-583, 1-585 to 1-587, 1-589 to 1-591, 1-594, 1-601, 1-602, 1-604 to 1-611, 1-624, 1-625, 1-630 to 1-634, 1-648, 1-654 to 1-656, 1-658 to 1-660, 1-663, 1-666 to 1-668, 1-670 to 1-672, 1-674 to 1-676, 1-678 to 1-680, 1-682 to 1-684, 1-687, 1-694, 1-695, 1-697 to 1-704, 1-717, 1-718, 1-723 to 1-727, 1-741, 1-747 to 1-749, 1-751 to 1-753, 1-756, 1-759 to 1-761, 1-763 to 1-765, 1-767 to 1-769, 1-771 to 1-773, 1-775 to 1-777, 1-780, 1-787, 1-788, 1-790 to 1-797, 1-810, 1-811, 1-816 to 1-820, 1-834, 1-840 to 1-842, 1-844 to 1-846, 1-849, 1-852 to 1-854, 1-856 to 1-858, 1-860 to 1-862, 1-864 to 1-866, 1-868 to 1-870, 1-873, 1-880, 1-881, 1-883 to 1-890, 1-903, 1-904, 1-909 to 1-913, 1-927, 1-933 to 1-935, 1-937 to 1-939, 1-942, 1-945 to 1-947, 1-949 to 1-951, 1-953 to 1-955, 1-957 to 1-959, 1-961 to 1-963, 1-966, 1-973, 1-974, 1-976 to 1-983, 1-996, 1-997, 1-1002 to 1-1006, 1-1020, 1-1026 to 1-1028, 1-1030 to 1-1032, 1-1035, 1-1038 to 1-1040, 1-1042 to 1-1044, 1-1046 to 1-1048, 1-1050 to 1-1052, 1-1054 to 1-1056, 1-1059, 1-1066, 1-1067, 1-1069 to 1-1076, 1-1089, 1-1090, 1-1095 to 1-1099, 1-1113, 1-1119 to 1-1121, 1-1123 to 1-1125, 1-1128, 1-1131 to 1-1133, 1-1135 to 1-1137, 1-1139 to 1-1141, 1-1143 to 1-1145, 1-1147 to 1-1149, 1-1152, 1-1159, 1-1160, 1-1162 to 1-1169, 1-1182, 1-1183, 1-1188 to 1-1192, 1-1206, 1-1212 to 1-1214, 1-1216 to 1-1218, 1-1221, 1-1224 to 1-1226, 1-1228 to 1-1230, 1-1232 to 1-1234, 1-1236 to 1-1238, 1-1240 to 1-1242, 1-1245, 1-1252, 1-1253, 1-1255 to 1-1262, 1-1275, 1-1276, 1-1281 to 1-1285, 1-1299, 1-1305 to 1-1307, 1-1309 to 1-1311, 1-1314, 1-1317 to 1-1319, 1-1321 to 1-1323, 1-1325 to 1-1327, 1-1329 to 1-1331, 1-1333 to 1-1335, 1-1338, 1-1345, 1-1346, 1-1348 to 1-1355, 1-1368, 1-1369, 1-1374 to 1-1378, 1-1392, 1-1397 to 1-1405, 1-1108 to 1-1415, 1-1418, 1-1419, 1-1422 to 1-1424, 1-1428, 1-1432 to 1-1440, 1-1443 to 1-1450, 1-1453, 1-1454, 1-1457 to 1-1459, 1-1463, 1-1467 to 1-1475, 1-1478 to 1-1485, 1-1488, 1-1489, 1-1492 to 1-1494, 1-1498, 1-1502 to 1-1510, 1-1513 to 1-1520, 1-1523, 1-1524, 1-1527 to 1-1529, 1-1533, 1-1537 to 1-1545, 1-1548 to 1-1555, 1-1558, 1-1559, 1-1562 to 1-1564, 1-1568, 1-1572 to 1-1580, 1-1583 to 1-1590, 1-1593, 1-1594, 1-1597 to 1-1599, 1-1603, 1-1607 to 1-1615, 1-1618 to 1-1625, 1-1628, 1-1629, 1-1632 to 1-1634, 1-1638, 1-1642 to 1-1650, 1-1653 to 1-1660, 1-1663, 1-1664, 1-1667 to 1-1669, 1-1673, 1-1677 to 1-1685, 1-1688 to 1-1695, 1-1698, 1-1699, 1-1702 to 1-1704, 1-1708; 1-1712 to 1-1720, 1-1723 to 1-1730, 1-1733, 1-1734, 1-1737 to 1-1739, 1-1743, 1-1747 to 1-1755, 1-1758 to 1-1765, 1-1768, 1-1769, 1-1772 to 1-1774, 1-1778, 1-1782 to 1-1790, 1-1793 to 1-1800, 1-1803, 1-1804, 1-1807 to 1-1809, 1-1813, 1-1817 to 1-1825, 1-1828 to 1-1835, 1-1838, 1-1839, 1-1842 to 1-1844, 1-1848, 1-1852 to 1-1860, 1-1863 to 1-1870, 1-1873, 1-1874, 1-1877 to 1-1879, 1-1883, 1-1887 to 1-1895, 1-1898 to 1-1905, 1-1908, 1-1909, 1-1912 to 1-1914, 1-1918, 1-1922 to 1-1930, 1-1933 to 1-1940, 1-1943, 1-1944, 1-1947 to 1-1949, 1-1953, 1-1957 to 1-1965, 1-1968 to 1-1975, 1-1978, 1-1979, 1-1982 to 1-1984, 1-1988, 1-1992 to 1-2000, 1-2003 to 1-2010, 1-2013, 1-2014, 1-2017 to 1-2019, 1-2023, 1-2027 to 1-2035, 1-2038 to 1-2045, 1-2048, 1-2049, 1-2052 to 1-2054, 1-2058, 1-2062 to 1-2070, 1-2073 to 1-2080, 1-2083, 1-2084, 1-2087 to 1-2089, 1-2093, 1-2097 to 1-2105, 1-2108 to 1-2115, 1-2118, 1-2119, 1-2122 to 1-2124, 1-2128, 1-2132 to 1-2140, 1-2143 to 1-2150, 1-2153, 1-2154, 1-2157 to 1-2159, 1-2163, 1-2167 to 1-2175, 1-2178 to 1-2185, 1-2188, 1-2189, 1-2192 to 1-2194, 1-2198, 1-2202 to 1-2210, 1-2213 to 1-2220, 1-2223, 1-2224, 1-2227 to 1-2229, 1-2233, 1-2237 to 1-2245, 1-2248 to 1-2255, 1-2258, 1-2259, 1-2262 to 1-2264, 1-2268, 1-2272 to 1-2280, 1-2283 to 1-2290, 1-2293, 1-2294, 1-2297 to 1-2299, 1-2303, 1-2307 to 1-2315, 1-2318 to 1-2325, 1-2328, 1-2329, 1-2332 to 1-2334, 1-2338, 1-2342 to 1-2350, 1-2353 to 1-2360, 1-2363, 1-2364, 1-2367 to 1-2369, 1-2373, 1-2377 to 1-2385, 1-2388 to 1-2395, 1-2398, 1-2399, 1-2402 to 1-2404, 1-2408, 1-2412 to 1-2420, 1-2423 to 1-2430, 1-2433, 1-2434, 1-2437 to 1-2439, 1-2443, 1-2447 to 1-2455, 1-2458 to 1-2465, 1-2468, 1-2469, 1-2472 to 1-2474, 1-2478, 1-2482 to 1-2490, 1-2493 to 1-2500, 1-2503, 1-2504, 1-2507 to 1-2509, 1-2513, 1-2517 to 1-2526, 1-2528, 1-2529, 1-2539 to 1-2554, 1-2556 to 1-2558, 1-2560 to 1-2577, 1-2579 to 1-2581, 1-2583 to 1-2594, 1-2626, 1-2629 to 1-2643, 1-2645, 1-2648 to 1-2651, 1-2653 to 1-2748, 1-2779, 1-2780, 1-2782, 1-2783, 1-2905, 1-2906, 1-2908, 1-2909, 1-3031, 1-3032, 1-3157, 1-3158, 2-2 to 2-4, 2-6 to 2-8, 2-11, 2-14 to 2-16, 2-18 to 2-20, 2-22 to 2-24, 2-26 to

2-28, 2-30 to 2-32, 2-35, 2-42, 2-43, 2-45 to 2-52, 2-65, 2-66, 2-71 to 2-75, 2-89, 2-94, 2-96 to 2-98, 2-100 to 2-102, 2-105, 2-108 to 2-110, 2-112 to 2-114, 2-116 to 2-118, 2-120 to 2-122, 2-124 to 2-126, 2-129, 2-136, 2-137, 2-139 to 2-146, 2-159, 2-160, 2-165 to 2-169, 2-183, 2-189 to 2-191, 2-193 to 2-195, 2-198, 2-201 to 2-203, 2-205 to 2-207, 2-209 to 2-211, 2-213 to 2-215, 2-217 to 2-219, 2-222, 2-229, 2-230, 2-232 to 2-239, 2-252, 2-253, 2-258 to 2-262, 2-276, 2-282 to 2-284, 2-286 to 2-288, 2-291, 2-294 to 2-296, 2-298 to 2-300, 2-302 to 2-304, 2-306 to 2-308, 2-310 to 2-312, 2-315, 2-322, 2-323, 2-325 to 2-332, 2-345, 2-346, 2-351 to 2-355, 2-369, 2-375 to 2-377, 2-379 to 2-381, 2-384, 2-387 to 2-389, 2-391 to 2-393, 2-395 to 2-397, 2-399 to 2-401, 2-403 to 2-405, 2-408, 2-415, 2-416, 2-418 to 2-425, 2-438, 2-439, 2-444 to 2-448, 2-462, 2-468 to 2-470, 2-472 to 2-474, 2-477, 2-480 to 2-482, 2-484 to 2-486, 2-488 to 2-490, 2-492 to 2-494, 2-496 to 2-498, 2-501, 2-508, 2-509, 2-511 to 2-518, 2-531, 2-532, 2-537 to 2-541, 2-555, 2-561 to 2-563, 2-565 to 2-567, 2-570, 2-573 to 2-575, 2-577 to 2-579, 2-581 to 2-583, 2-585 to 2-587, 2-589 to 2-591, 2-594, 2-601, 2-602, 2-604 to 2-611, 2-624, 2-625, 2-630 to 2-634, 2-648, 2-654 to 2-656, 2-658 to 2-660, 2-663, 2-666 to 2-668, 2-670 to 2-672, 2-674 to 2-676, 2-678 to 2-680, 2-682 to 2-684, 2-687, 2-694, 2-695, 2-697 to 2-704, 2-717, 2-718, 2-723 to 2-727, 2-741, 2-747 to 2-749, 2-751 to 2-753, 2-756, 2-759 to 2-761, 2-763 to 2-765, 2-767 to 2-769, 2-771 to 2-773, 2-775 to 2-777, 2-780, 2-787, 2-788, 2-790 to 2-797, 2-810, 2-811, 2-816 to 2-820, 2-834, 2-840 to 2-842, 2-844 to 2-846, 2-849, 2-852 to 2-854, 2-856 to 2-858, 2-860 to 2-862, 2-864 to 2-866, 2-868 to 2-870, 2-873, 2-880, 2-881, 2-883 to 2-890, 2-903, 2-904, 2-909 to 2-913, 2-927, 2-933 to 2-935, 2-937 to 2-939, 2-942, 2-945 to 2-947, 2-949 to 2-951, 2-953 to 2-955, 2-957 to 2-959, 2-961 to 2-963, 2-966, 2-973, 2-974, 2-976 to 2-983, 2-996, 2-997, 2-1002 to 2-1006, 2-1020, 2-1026 to 2-1028, 2-1030 to 2-1032, 2-1035, 2-1038 to 2-1040, 2-1042 to 2-1044, 2-1046 to 2-1048, 2-1050 to 2-1052, 2-1054 to 2-1056, 2-1059, 2-1066, 2-1067, 2-1069 to 2-1076, 2-1089, 2-1090, 2-1095 to 2-1099, 2-1113, 2-1119 to 2-1121, 2-1123 to 2-1125, 2-1128, 2-1131 to 2-1133, 2-1135 to 2-1137, 2-1139 to 2-1141, 2-1143 to 2-1145, 2-1147 to 2-1149, 2-1152, 2-1159, 2-1160, 2-1162 to 2-1169, 2-1182, 2-1183, 2-1188 to 2-1192, 2-1206, 2-1212 to 2-1214, 2-1216 to 2-1218, 2-1221, 2-1224 to 2-1226, 2-1228 to 2-1230, 2-1232 to 2-1234, 2-1236 to 2-1238, 2-1240 to 2-1242, 2-1245, 2-1252, 2-1253, 2-1255 to 2-1262, 2-1275, 2-1276, 2-1281 to 2-1285, 2-1299, 2-1305 to 2-1307, 2-1309 to 2-1311, 2-1314, 2-1317 to 2-1319, 2-1321 to 2-1323, 2-1325 to 2-1327, 2-1329 to 2-1331, 2-1333 to 2-1335, 2-1338, 2-1345, 2-1346, 2-1348 to 2-1355, 2-1368, 2-1369, 2-1374 to 2-1378, 2-1392, 2-1397 to 2-1405, 2-1408 to 2-1415, 2-1418, 2-1419, 2-1422 to 2-1424, 2-1428, 2-1432 to 2-1440, 2-1443 to 2-1450, 2-1453, 2-1454, 2-1457 to 2-1459, 2-1463, 2-1467 to 2-1475, 2-1478 to 2-1485, 2-1488, 2-1489, 2-1492 to 2-1494, 2-1498, 2-1502 to 2-1510, 2-1513 to 2-1520, 2-1523, 2-1524, 2-1527 to 2-1529, 2-1533, 2-1537 to 2-1545, 2-1548 to 2-1555, 2-1558, 2-1559, 2-1562 to 2-1564, 2-1568, 2-1572 to 2-1580, 2-1583 to 2-1590, 2-1593, 2-1594, 2-1597 to 2-1599, 2-1603, 2-1607 to 2-1615, 2-1618 to 2-1625, 2-1628, 2-1629, 2-1632 to 2-1634, 2-1638, 2-1642 to 2-1650, 2-1653 to 2-1660, 2-1663, 2-1664, 2-1667 to 2-1669, 2-1673, 2-1677 to 2-1685, 2-1688 to 2-1695, 2-1698, 2-1699, 2-1702 to 2-1704, 2-1708, 2-1712 to 2-1720, 2-1723 to 2-1730, 2-1733, 2-1734, 2-1737 to 2-1739, 2-1743, 2-1747 to 2-1755, 2-1758 to 2-1765, 2-1768, 2-1769, 2-1772 to 2-1774, 2-1778, 2-1782 to 2-1790, 2-1793 to 2-1800, 2-1803, 2-1804, 2-1807 to 2-1809, 2-1813, 2-1817 to 2-1825, 2-1828 to 2-1835, 2-1838, 2-1839, 2-1842 to 2-1844, 2-1848, 2-1852 to 2-1860, 2-1863 to 2-1870, 2-1873, 2-1874, 2-1877 to 2-1879, 2-1883, 2-1887 to 2-1895, 2-1898 to 2-1905, 2-1908, 2-1909, 2-1912 to 2-1914, 2-1918, 2-1922 to 2-1930, 2-1933 to 2-1940, 2-1943, 2-1944, 2-1947 to 2-1949, 2-1953, 2-1957 to 2-1965, 2-1968 to 2-1975, 2-1978, 2-1979, 2-1982 to 2-1984, 2-1988, 2-1992 to 2-2000, 2-2003 to 2-2010, 2-2013, 2-2014, 2-2017 to 2-2019, 2-2023, 2-2027 to 2-2035, 2-2038 to 2-2045, 2-2048, 2-2049, 2-2052 to 2-2054, 2-2058, 2-2062 to 2-2070, 2-2073 to 2-2080, 2-2083, 2-2084, 2-2087 to 2-2089, 2-2093, 2-2097 to 2-2105, 2-2108 to 2-2115, 2-2118, 2-2119, 2-2122 to 2-2124, 2-2128, 2-2132 to 2-2140, 2-2143 to 2-2150, 2-2153, 2-2154, 2-2157 to 2-2159, 2-2163, 2-2167 to 2-2175, 2-2178 to 2-2185, 2-2188, 2-2189, 2-2192 to 2-2194, 2-2198, 2-2202 to 2-2210, 2-2213 to 2-2220, 2-2223, 2-2224, 2-2227 to 2-2229, 2-2233, 2-2237 to 2-2245, 2-2248 to 2-2255, 2-2258, 2-2259, 2-2262 to 2-2264, 2-2268, 2-2272 to 2-2280, 2-2283 to 2-2290, 2-2293, 2-2294, 2-2297 to 2-2299, 2-2303, 2-2307 to 2-2315, 2-2318 to 2-2325, 2-2328, 2-2329, 2-2332 to 2-2334, 2-2338, 2-2342 to 2-2350, 2-2353 to 2-2360, 2-2363, 2-2364, 2-2367 to 2-2369, 2-2373, 2-2377 to 2-2385, 2-2388 to 2-2395, 2-2398, 2-2399, 2-2402 to 2-2404, 2-2408, 2-2412 to 2-2420, 2-2423 to 2-2430, 2-2433, 2-2434, 2-2437 to 2-2439, 2-2443, 2-2447 to 2-2455, 2-2458 to 2-2465, 2-2468, 2-2469, 2-2472 to 2-2474, 2-2478, 2-2482 to 2-2490, 2-2493 to 2-2500, 2-2503, 2-2504, 2-2507 to 2-2509, 2-2513, 2-2517 to 2-2522, 2-2524 to 2-2526, 2-2528, 2-2529, 2-2539 to 2-2551, 2-2556 to 2-2558, 2-2560 to 2-2577, 2-2579 to 2-2581 and 2-2583 to 2-2594;

still more preferred are Compounds Nos.:

1-3, 1-7, 1-11, 1-15, 1-19, 1-23, 1-27, 1-31, 1-35, 1-42, 1-45, 1-47 to 1-51, 1-65, 1-71, 1-73, 1-89, 1-94, 1-97, 1-98, 1-101, 1-105, 1-109, 1-113, 1-117, 1-121, 1-125, 1-129, 1-136, 1-139, 1-141 to 1-146, 1-159, 1-165, 1-167, 1-183, 1-190, 1-194, 1-198, 1-202, 1-206, 1-210, 1-214, 1-218, 1-222, 1-229, 1-232, 1-234 to 1-238, 1-252, 1-258, 1-260, 1-276, 1-283, 1-287, 1-291, 1-295, 1-299, 1-303, 1-307, 1-311, 1-315, 1-322, 1-325, 1-327 to 1-331, 1-345, 1-351, 1-353, 1-369, 1-376, 1-380, 1-384, 1-388, 1-392, 1-396, 1-400, 1-404, 1-408, 1-415, 1-418, 1-420 to 1-424, 1-438, 1-444, 1-446, 1-462, 1-469, 1-473, 1-477, 1-481, 1-485, 1-489, 1-493, 1-497, 1-501, 1-508, 1-511, 1-513 to 1-517, 1-531, 1-537, 1-539, 1-555, 1-562, 1-566, 1-570, 1-574, 1-578, 1-582, 1-586, 1-590, 1-594, 1-601, 1-604, 1-606 to 1-610, 1-624, 1-630, 1-632, 1-648, 1-655, 1-659, 1-663, 1-667, 1-671, 1-675, 1-679, 1-683, 1-687, 1-694, 1-697, 1-699

to 1-703, 1-717, 1-723, 1-725, 1-741, 1-748, 1-752, 1-756, 1-760, 1-764, 1-768, 1-772, 1-776, 1-780, 1-787, 1-790, 1-792 to 1-796, 1-810, 1-816, 1-818, 1-834, 1-841, 1-845, 1-849, 1-853, 1-857, 1-861, 1-865, 1-869, 1-873, 1-880, 1-883, 1-885 to 1-889, 1-903, 1-909, 1-911, 1-927, 1-934, 1-938, 1-942, 1-946, 1-950, 1-954, 1-958, 1-962, 1-966, 1-973, 1-976, 1-978 to 1-982, 1-996, 1-1002, 1-1004, 1-1020, 1-1027, 1-1031, 1-1035, 1-1039, 1-1043, 1-1047, 1-1051, 1-1055, 1-1059, 1-1066, 1-1069, 1-1071 to 1-1075, 1-1089, 1-1095, 1-1097, 1-1113, 1-1120, 1-1124, 1-1128, 1-1132, 1-1136, 1-1140, 1-1144, 1-1148, 1-1152, 1-1159, 1-1162, 1-1164 to 1-1168, 1-1182, 1-1188, 1-1190, 1-1206, 1-1213, 1-1217, 1-1221, 1-1225, 1-1229, 1-1233, 1-1237, 1-1241, 1-1245, 1-1252, 1-1255, 1-1257 to 1-1261, 1-1275, 1-1281, 1-1283, 1-1299, 1-1306, 1-1310, 1-1314, 1-1318, 1-1322, 1-1326, 1-1330, 1-1334, 1-1338, 1-1345, 1-1348, 1-1350 to 1-1354, 1-1368, 1-1374, 1-1376, 1-1392, 1-1397 to 1-1405, 1-1408, 1-1410, 1-1412 to 1-1415, 1-1418, 1-1422, 1-1428, 1-1432 to 1-1440, 1-1443, 1-1445, 1-1447 to 1-1450, 1-1453, 1-1457, 1-1463, 1-1467 to 1-1475, 1-1478, 1-1480, 1-1482 to 1-1485, 1-1488, 1-1492, 1-1498, 1-1502 to 1-1510, 1-1513, 1-1515, 1-1517 to 1-1520, 1-1523, 1-1527, 1-1533, 1-1537 to 1-1545, 1-1548, 1-1550, 1-1552 to 1-1555, 1-1558, 1-1562, 1-1568, 1-1572 to 1-1580, 1-1583, 1-1585, 1-1587 to 1-1590, 1-1593, 1-1597, 1-1603, 1-1607 to 1-1615, 1-1618, 1-1620, 1-1622 to 1-1625, 1-1628, 1-1632, 1-1638, 1-1642 to 1-1650, 1-1653, 1-1655, 1-1657 to 1-1660, 1-1663, 1-1667, 1-1673, 1-1677 to 1-1685, 1-1688, 1-1690, 1-1692 to 1-1695, 1-1698, 1-1702, 1-1708, 1-1712 to 1-1720, 1-1723, 1-1725, 1-1727 to 1-1730, 1-1733, 1-1737, 1-1743, 1-1747 to 1-1755, 1-1758, 1-1760, 1-1762 to 1-1765, 1-1768, 1-1772, 1-1778, 1-1782 to 1-1790, 1-1793, 1-1795, 1-1797 to 1-1800, 1-1803, 1-1807, 1-1813, 1-1817 to 1-1825, 1-1828, 1-1830, 1-1832 to 1-1835, 1-1838, 1-1842, 1-1848, 1-1852 to 1-1860, 1-1863, 1-1865, 1-1867 to 1-1870, 1-1873, 1-1877, 1-1883, 1-1887 to 1-1895, 1-1898, 1-1900, 1-1902 to 1-1905, 1-1908, 1-1912, 1-1918, 1-1922 to 1-1930, 1-1933, 1-1935, 1-1937 to 1-1940, 1-1943, 1-1947, 1-1953, 1-1957 to 1-1965, 1-1968, 1-1970, 1-1972 to 1-1975, 1-1978, 1-1982, 1-1988, 1-1992 to 1-2000, 1-2003, 1-2005, 1-2007 to 1-2010, 1-2013, 1-2017, 1-2023, 1-2027 to 1-2035, 1-2038, 1-2040, 1-2042 to 1-2045, 1-2048, 1-2052, 1-2058, 1-2062 to 1-2070, 1-2073, 1-2075, 1-2077 to 1-2080, 1-2083, 1-2087, 1-2093, 1-2097 to 1-2105, 1-2108, 1-2110, 1-2112 to 1-2115, 1-2118, 1-2122, 1-2128, 1-2132 to 1-2140., 1-2143, 1-2145, 1-2147 to 1-2150, 1-2153, 1-2157, 1-2163, 1-2167 to 1-2175, 1-2178, 1-2180, 1-2182 to 1-2185, 1-2188, 1-2192, 1-2198, 1-2202 to 1-2210, 1-2213, 1-2215, 1-2217 to 1-2220, 1-2223, 1-2227, 1-2233, 1-2237 to 1-2245, 1-2248, 1-2250, 1-2252 to 1-2255, 1-2258, 1-2262, 1-2268, 1-2272 to 1-2280, 1-2283, 1-2285, 1-2287 to 1-2290, 1-2293, 1-2297, 1-2303, 1-2307 to 1-2315, 1-2318, 1-2320, 1-2322 to 1-2325, 1-2328, 1-2332, 1-2338, 1-2342 to 1-2350, 1-2353, 1-2355, 1-2357 to 1-2360, 1-2363, 1-2367, 1-2373, 1-2377 to 1-2385, 1-2388, 1-2390, 1-2392 to 1-2395, 1-2398, 1-2402, 1-2408, 1-2412 to 1-2420, 1-2423, 1-2425, 1-2427 to 1-2430, 1-2433, 1-2437, 1-2443, 1-2447 to 1-2455, 1-2458, 1-2460, 1-2462 to 1-2465, 1-2468, 1-2472, 1-2478, 1-2482 to 1-2490, 1-2493, 1-2495, 1-2497 to 1-2500, 1-2503, 1-2507, 1-2513, 1-2517 to 1-2520, 1-2522 to 1-2524, 1-2525, 1-2528, 1-2529, 1-2539 to 1-2541, 1-2543, 1-2545, 1-2547, 1-2549 to 1-2552, 1-2554, 1-2556, 1-2557, 1-2560 to 1-2564, 1-2566, 1-2568, 1-2570, 1-2572 to 1-2575, 1-2577, 1-2579, 1-2580, 1-2583 to 1-2587, 1-2589, 1-2591, 1-2593, 1-2629, 1-2630, 1-2632, 1-2645, 1-2650, 1-2651, 1-2653, 1-2654, 1-2656, 1-2657, 1-2665 to 1-2667, 1-2707 to 1-2709, 1-2779, 1-2780, 1-2782, 1-2783, 1-2905, 1-2906, 1-2908, 1-2909, 1-3031, 1-3032, 1-3157, 1-3158, 2-3, 2-7, 2-11, 2-15, 2-19, 2-23, 2-27, 2-31, 2-35, 2-42, 2-45, 2-47 to 2-51, 2-65, 2-71, 2-73, 2-89, 2-94, 2-97, 2-101, 2-105, 2-109, 2-113, 2-117, 2-121, 2-125, 2-129, 2-136, 2-139, 2-141 to 2-145, 2-159, 2-165, 2-167, 2-183, 2-190, 2-194, 2-198, 2-202, 2-206, 2-210, 2-214, 2-218, 2-222, 2-229, 2-232, 2-234 to 2-238, 2-252, 2-258, 2-260, 2-276, 2-283, 2-287, 2-291, 2-295, 2-299, 2-303, 2-307, 2-311, 2-315, 2-322, 2-325, 2-327 to 2-331, 2-345, 2-351, 2-353, 2-369, 2-376, 2-380, 2-384, 2-388, 2-392, 2-396, 2-400, 2-404, 2-408, 2-415, 2-418, 2-420 to 2-424, 2-438, 2-444, 2-446, 2-462, 2-469, 2-473, 2-477, 2-481, 2-485, 2-489, 2-493, 2-497, 2-501, 2-508, 2-511, 2-513 to 2-517, 2-531, 2-537, 2-539, 2-555, 2-562, 2-566, 2-570, 2-574, 2-578, 2-582, 2-586, 2-590, 2-594, 2-601, 2-604, 2-606 to 2-610, 2-624, 2-630, 2-632, 2-648, 2-655, 2-659, 2-663, 2-667, 2-671, 2-675, 2-679, 2-683, 2-687, 2-694, 2-697, 2-699 to 2-703, 2-717, 2-723, 2-725, 2-741, 2-748, 2-752, 2-756, 2-760, 2-764, 2-768, 2-772, 2-776, 2-780, 2-787, 2-790, 2-792 to 2-796, 2-810, 2-816, 2-818, 2-834, 2-841, 2-845, 2-849, 2-853, 2-857, 2-861, 2-865, 2-869, 2-873, 2-880, 2-883, 2-885 to 2-889, 2-903, 2-909, 2-911, 2-927, 2-934, 2-938, 2-942, 2-946, 2-950, 2-954, 2-958, 2-962, 2-966, 2-973, 2-976, 2-978 to 2-982, 2-996, 2-1002, 2-1004, 2-1020, 2-1027, 2-1031, 2-1035, 2-1039, 2-1043, 2-1097, 2-1051, 2-1055, 2-1059, 2-1066, 2-1069, 2-1071 to 2-1075, 2-1089, 2-1095, 2-1097, 2-1113, 2-1120, 2-1124, 2-1128, 2-1132, 2-1136, 2-1140, 2-1144, 2-1148, 2-1152, 2-1159, 2-1162, 2-1164 to 2-1168, 2-1182, 2-1188, 2-1190, 2-1206, 2-1213, 2-1217, 2-1221, 2-1225, 2-1229, 2-1233, 2-1237, 2-1241, 2-1245, 2-1252, 2-1255, 2-1257 to 2-1261, 2-1275, 2-1281, 2-1283, 2-1299, 2-1306, 2-1310, 2-1314, 2-1318, 2-1322, 2-1326, 2-1330, 2-1334, 2-1338, 2-1345, 2-1348, 2-1350 to 2-1354, 2-1368, 2-1374, 2-1376, 2-1392, 2-1397 to 2-1405, 2-1408, 2-1410, 2-1412 to 2-1415, 2-1418, 2-1422, 2-1428, 2-1432 to 2-1440, 2-1443, 2-1445, 2-1447 to 2-1450, 2-1453, 2-1457, 2-1463, 2-1467 to 2-1475, 2-1478, 2-1480, 2-1482 to 2-1485, 2-1488, 2-1492, 2-1498, 2-1502 to 2-1510, 2-1513, 2-1515, 2-1517 to 2-1520, 2-1523, 2-1527, 2-1533, 2-1537 to 2-1545, 2-1548, 2-1550, 2-1552 to 2-1555, 2-1558, 2-1562, 2-1568, 2-1572 to 2-1580, 2-1583, 2-1585, 2-1587 to 2-1590, 2-1593, 2-1597, 2-1603, 2-1607 to 2-1615, 2-1618, 2-1620, 2-1622 to 2-1625, 2-1628, 2-1632, 2-1638, 2-1642 to 2-1650, 2-1653, 2-1655, 2-1657 to 2-1660, 2-1663, 2-1667, 2-1673, 2-1677 to 2-1685, 2-1688, 2-1690, 2-1692 to 2-1695, 2-1698, 2-1702, 2-1708, 2-1712 to 2-1720, 2-1723, 2-1725, 2-1727 to 2-1730, 2-1733, 2-1737, 2-1743, 2-1747 to 2-1755, 2-1758, 2-1760, 2-1762 to 2-1765, 2-1768, 2-1772, 2-1778, 2-1782

to 2-1790, 2-1793, 2-1795, 2-1797 to 2-1800, 2-1803, 2-1807, 2-1813, 2-1817 to 2-1825, 2-1828, 2-1830, 2-1832 to 2-1835, 2-1838, 2-1842, 2-1848, 2-1852 to 2-1860, 2-1863, 2-1865, 2-1867 to 2-1870, 2-1873, 2-1877, 2-1883, 2-1887 to 2-1895, 2-1898, 2-1900, 2-1902 to 2-1905, 2-1908, 2-1912, 2-1918, 2-1922 to 2-1930, 2-1933, 2-1935, 2-1937 to 2-1940, 2-1943, 2-1947, 2-1953, 2-1957 to 2-1965, 2-1968, 2-1970, 2-1972 to 2-1975, 2-1978, 2-1982, 2-1988, 2-1992 to 2-2000, 2-2003, 2-2005, 2-2007 to 2-2010, 2-2013, 2-2017, 2-2023, 2-2027 to 2-2035, 2-2038, 2-2040, 2-2042 to 2-2045, 2-2048, 2-2052, 2-2058, 2-2062 to 2-2070, 2-2073, 2-2075, 2-2077 to 2-2080, 2-2083, 2-2087, 2-2093, 2-2097 to 2-2105, 2-2108, 2-2110, 2-2112 to 2-2115, 2-2118, 2-2122, 2-2128, 2-2132 to 2-2140, 2-2143, 2-2145, 2-2147 to 2-2150, 2-2153, 2-2157, 2-2163, 2-2167 to 2-2175, 2-2178, 2-2180, 2-2182 to 2-2185, 2-2188, 2-2192, 2-2198, 2-2202 to 2-2210, 2-2213, 2-2215, 2-2217 to 2-2220, 2-2223, 2-2227, 2-2233, 2-2237 to 2-2245, 2-2248, 2-2250, 2-2252 to 2-2255, 2-2258, 2-2262, 2-2268, 2-2272 to 2-2280, 2-2283, 2-2285, 2-2287 to 2-2290, 2-2293, 2-2297, 2-2303, 2-2307 to 2-2315, 2-2318, 2-2320, 2-2322 to 2-2325, 2-2328, 2-2332, 2-2338, 2-2342 to 2-2350, 2-2353, 2-2355, 2-2357 to 2-2360, 2-2363, 2-2367, 2-2373, 2-2377 to 2-2385, 2-2388, 2-2390, 2-2392 to 2-2395, 2-2398, 2-2402, 2-2408, 2-2412 to 2-2420, 2-2423, 2-2425, 2-2427 to 2-2430, 2-2433, 2-2437, 2-2443, 2-2447 to 2-2455, 2-2458, 2-2460, 2-2462 to 2-2465, 2-2468, 2-2472, 2-2478, 2-2482 to 2-2490, 2-2493, 2-2495, 2-2497 to 2-2500, 2-2503, 2-2507, 2-2513, 2-2517 to 2-2520, 2-2522, 2-2524, 2-2525, 2-2528, 2-2529, 2-2539 to 2-2541, 2-2543, 2-2545, 2-2547, 2-2549 to 2-2552, 2-2554, 2-2556, 2-2557, 2-2560 to 2-2564, 2-2566, 2-2568, 2-2570, 2-2572 to 2-2575, 2-2577, 2-2579, 2-2580, 2-2583 to 2-2587, 2-2589, 2-2591 and 2-2593.

The most preferred compounds are:

4-(3-aminopropyl)-2-(4-fluorophenyl)-3-(pyridin-4-yl)-1*H*-pyrrole,
4-(3-acetylaminopropyl)-2-(4-fluorophenyl)-3-(pyridin-4-yl)-1*H*-pyrrole,
2-(4-fluorophenyl)-4-(3-methylaminopropyl)-3-(pyridin-4-yl)-1*H*-pyrrole,
[5-(4-fluorophenyl)-4-(pyridin-4-yl)-1*H*-pyrrol-3-yl]-(pyridin-4-yl)methanol,
4-(1-acetyl-1,2,3,6-tetrahydr*opyridin-4-yl)-2-(4-fluorophenyl)-3-(pyridin-4-yl)-1H*-pyrrole,
2-(4-fluorophenyl)-3-(pyridin-4-yl)-4-(1,2,3,6-tetrahydropyridin-4-yl)-1*H*-pyrrole,
2-(4-fluorophenyl)-4-(piperidin-4-yl)-3-(pyridin-4-yl)-1*H*-pyrrole,
3-(4-fluorophenyl)-1-(piperidin-4-yl)-2-(pyridin-4-yl)-1*H*-pyrrole,
1-(1-acetylpiperidin-4-yl)-3-(4-fluorophenyl)-2-(pyridin-4-yl)-1*H*-pyrrole,
3-(4-fluorophenyl)-1-[1-(2-nitroethyl)piperidin-4-yl]-2-(pyridin-4-yl)-1*H*-pyrrole,
3-(4-fluorophenyl)-1-[3-(morpholin-1-yl)propyl]-2-(pyridin-4-yl)-1H-pyrrole,
3-(4-fluorophenyl)-1-(piperidin-3-yl)-2-(pyridin-4-yl)-1*H*-pyrrole,
1-(azetidin-3-yl)-3-(4-fluorophenyl)-2-(pyridin-4-yl)-1*H*-pyrrole,
4-(3-dimethylaminopropyl)-2-(4-fluorophenyl)-3-(pyridin-4-yl)-1*H*-pyrrole,
2-(4-fluorophenyl)-4-[3-(piperidin-1-yl)propyl]-3-(pyridin-4-yl)-1*H*-pyrrole,
2-(4-fluorophenyl)-4-[3-(1-methylpiperazin-4-yl)propyl]-3-(pyridin-4-yl)-1*H*-pyrrole,
2-(4-fluorophenyl)-4-(1-methyl-1,2,3,6-tetrahydropyridin-4-yl)-3-(pyridin-4-yl)-1*H*-pyrrole,
2-(4-fluorophenyl)-4-(1-methylpiperidin-4-yl)-3-(pyridin-4-yl)-1*H*-pyrrole,
3-(4-fluorophenyl)-2-(pyridin-4-yl)-1-(2,2,6,6-tetramethylpiperidin-4-yl)-1*H*-pyrrole,
2-(4-fluorophenyl)-3-(pyridin-4-yl)-4- (quinuclidin-2-en-3-yl)-1*H*-pyrrole,
2-(4-fluorophenyl)-3-(pyridin-4-yl)-4-(quinuclidin-3-yl)-1*H*-pyrrole,
2-(4-fluorophenyl)-4-(4-hydroxypipe:ridin-4-yl)-3-(pyridin-4-yl)-1*H*-pyrrole,
3-(4-fluorophenyl)-2-(pyridin-4-yl)-1-(quinuclidin-3-yl)-1*H*-pyrrole,
1-(4-aminocyclohexyl)-3-(4-fluorophenyl)-2-(pyridin-4-yl)-1*H*-pyrrole,
3-(4-fluorophenyl)-2-(2-methylaminopyrimidin-4-yl)-1-(piperidin-4-yl)-1*H*-pyrrole,
2-(4-fluorophenyl)-4-(8-methyl-8-azabicyclo[3.2.1]oct-2-en-3-yl)-3-(pyridin-4-yl)-1*H*-pyrrole,
2-(4-fluorophenyl)-4-(8-methyl-8-azabicyclo[3.2.1]octan-3-yl)-3-(pyridin-4-yl)-1*H*-pyrrole,
4-(8-azabicyclo[3.2.1]oct-2-en-3-yl)-2-(4-fluorophenyl)-3-(pyridin-4-yl)-1*H*-pyrrole,
2-(4-fluorophenyl)-3-(pyridin-4-yl)-4(1,2,3,6-tetrahydro-2,2,6,6-tetramethylpyridin-4-yl)-1*H*-pyrrole,
2-(4-fluorophenyl)-3-(pyridin-4-yl)-4(2,2,6,6-tetramethylpiperidin-4-yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
4-(1-ethylpiperidin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole;
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,

4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(3-dimethylamino-1-propen-1-yl)-2-(4-fluorophenyl)-3-(pyridin-4-yl)-1*H*-pyrrole,
4-(4-aminobutyl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole, and
1-(3-dimethylaminopropyl)-3-(4-fluorophenyl)-2-(pyridin-4-yl)-1*H*-pyrrole;

and pharmacologically acceptable salts, esters or other derivatives thereof.

It should be noted that the specific combinations of substituents $R^1$, $R^2$ and $R^3$ shown in the above Tables 1 and 2 can also be applied to the substituents $R^1$, $R^2$ and $R^3$ in formulae (I-2), (I-4) and (I-5) above.

[Mode for carrying out the invention]

The compounds of formula (I) of the present invention can be prepared according to processes described below.

Method A

Method A is a method to prepare compounds of formula (I-1) of the present invention.

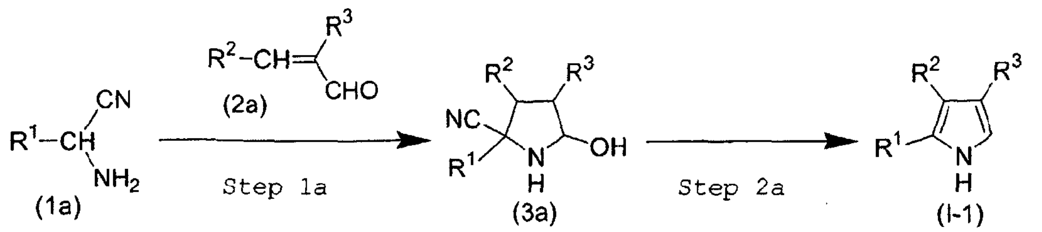


In the above formulae, $R^1$, $R^2$ and $R^3$ are as defined above.

Step 1a

In this Step, a pyrrolidine compound of formula (3a) is prepared by reacting an aminonitrile compound of formula (1a) with an α,β-unsaturated aldehyde compound of formula (2a). This reaction can be carried out using the method described in detail in EP 0799823.

Step 2a

In this Step, the desired pyrrole derivative of formula (I-1) of the present invention is prepared by the elimination of hydrogen cyanide and water from the compound of formula (3a). This reaction can be carried out using the method described in detail in EP 0799823.

Method B, Method C, Method D and Method E

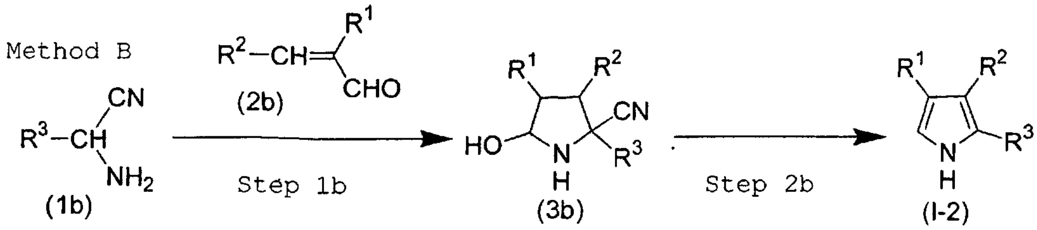


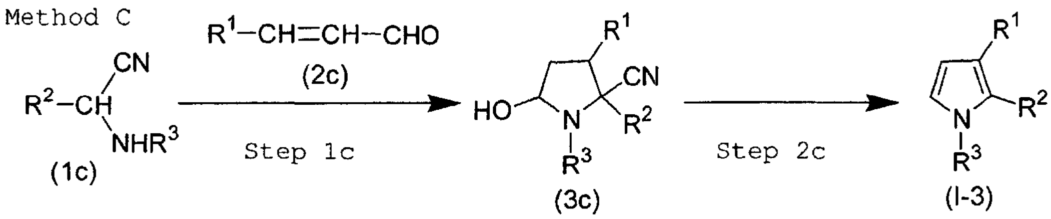


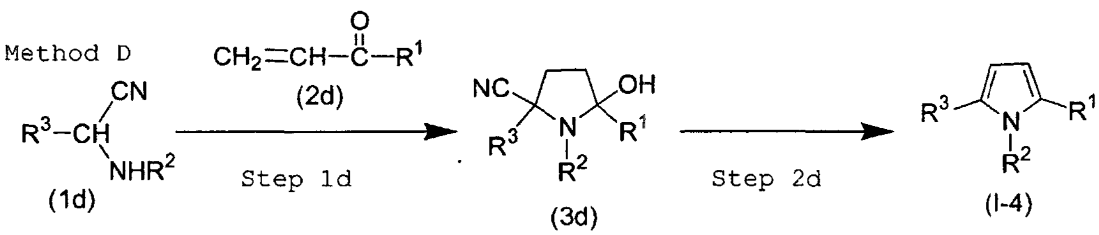


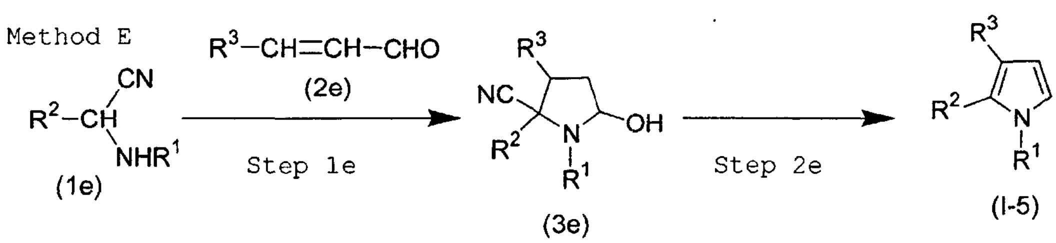


In the above formulae, $R^1$, $R^2$ and $R^3$ are as defined above.

Method B, Method C, Method D and Method E are used to prepare compounds of formulae (I-2), (I-3), (I-4) and (I-5) respectively.

In the above Reaction Schemes, Step 1b, Step 1c, Step 1d and Step 1e are carried out in a similar manner to Step 1a above, and Step 2b, Step 2c, Step 2d and Step 2e are carried out similarly to Step 2a above.

Method F

In this method, compounds of formula (I-1) of the present invention wherein $R^3$ is an aminomethyl, aminoethyl, aminopropyl or aminopropenyl group respectively are prepared as shown in Method F below.

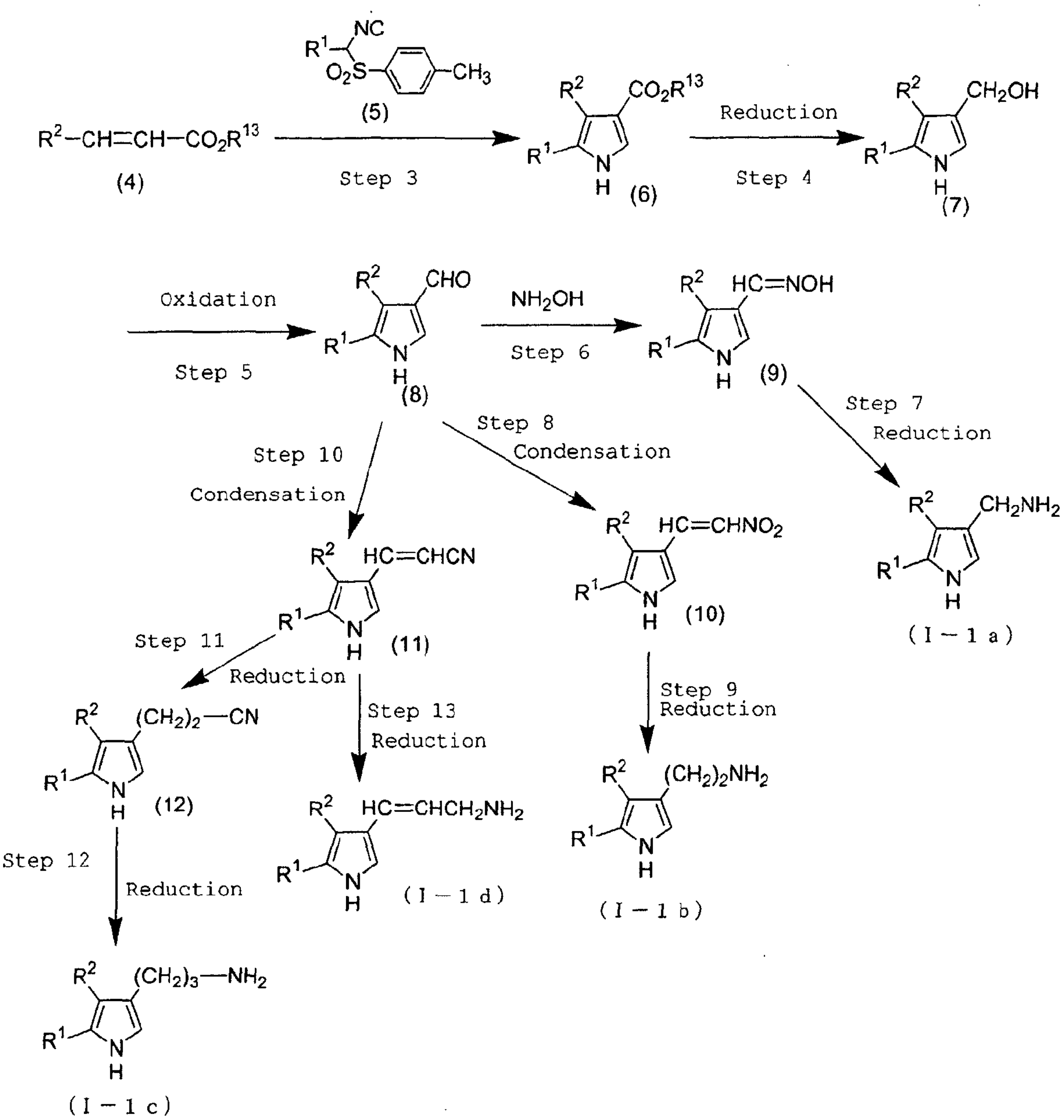


In the above formulae, $R^1$ and $R^2$ are as defined above and $R^{13}$ represents a hydrogen atom, a lower alkyl group as defined above or an aralkyl group as defined above.

Step 3

In this Step, a pyrrole carboxylic acid derivative of formula (6) is prepared by reacting an $\alpha,\beta$-unsaturated compound of formula (4) with an isonitrile compound of formula (5). This reaction can be carried out using the method described in detail in R. Di Santo et al., Synthetic Communications, 25(6), pp. 795-802 (1995).

Step 4

In this Step, a hydroxymethyl compound of formula (7) is prepared by reducing the pyrrole carboxylic acid derivative of formula (6). Reduction reactions can be carried out using the method described in detail in R. F. Nystrom et al., J. Am. Chem. Soc., 71, 3245 (1945), using a reducing agent such as lithium aluminum hydride, lithium borohydride or diisobutyl aluminum hydride.

Step 5

In this Step, an aldehyde compound of formula (8) is prepared by oxidizing the hydroxymethyl compound of formula (7). Oxidation reactions can be carried out using the method described in detail in S. Bartel & F. Bohlmann, Tetrahedron Lett., 685 (1985), using an oxidizing agent such as chromic acid, manganese dioxide or dimethylsulfoxide.

Step 6

In this Step, an oxime compound of formula (9) is prepared by reacting the aldehyde compound of formula (8) with hydroxyamine. Dehydration condensation reactions can be carried out using well known techniques.

Step 7

In this Step, the desired compound of formula (I-1a) of the present invention is prepared by reducing the oxime compound of formula (9). Reduction reactions can be carried out using catalytic reduction or reduction with diborane, examples of which are disclosed in detail in P. N. Rylander & D. R. Steele, Engelhald Ind. Tech. Bull., 3, 19 (1962), and J. A. Secrist, III & M. W. Logue, J. Org. Chem., 37, 335 (1972).

Step 8

In this Step, a nitroolefin compound of formula (10) is prepared by a dehydration condensation (aldol condensation) of the aldehyde compound of formula (8) and nitromethane. Dehydration condensation reactions can be carried out using the method described in detail in D. E. Worall, Org. Synth., I, 413 (1941).

Step 9

In this Step, the desired compound of formula (I-1b of the present invention is prepared by reducing the nitroolefin compound of formula (10). Reduction reactions can be carried out using the method described in detail in S. I. Murahashi et al., Bull. Chem. Soc. Jpn., 63, 1252 (1990).

Step 10

In this Step, a cyanoolefin compound of formula (11) is prepared from the aldehyde compound of formula (8). This transformation can be performed, for example, via an aldol condensation reaction similar to that employed in Step 8 above, a Wittig reaction [for example according to the method described in detail in The Organic Chemistry of Phosphorous, Elsevier (1967)], or a Wittig-Horner reaction [for example according to the method described in detail in L. Horner et al., Chem. Ber., 95, 581 (1962)].

Step 11

In this Step, a cyanoethyl compound of formula (12) is prepared by reduction of the double bond of the cyanoolefin compound of formula (11). Reduction reactions can be carried out using the method described in detail in S. M. Kerwin et al., J. Org. Chem., 52, 1686 (1987) and T. Hudlicky et al., J. org. Chem., 52, 4641 (1987).

Step 12

In this Step, the desired compound of formula (I-1c) of the present invention is prepared by reducing the cyano group of the cyanoethyl compound of formula (12). Reduction reactions can be carried out using the methods described in detail in N. M. Yoon & H. C. Brown, J. Am. Chem. Soc., 90, 2927 (1968) and J. Meinweld et al., J. Org. Chem., 29, 2914. (1964), using lithium aluminum hydride or diisobutyl aluminum hydride.

Step 13

In this Step, the desired compound of formula (I-1d) of the present invention is prepared by reducing the cyano group of the cyanoolefin compound of formula (11). This reduction is carried out in a similar manner to that described in Step 12 above.

Derivatives of the compounds of formula (I-1a), (I-1b) (I-1c) and (I-1d) in which a nitrogen atom of an amino group present therein is substituted can be synthesized by alkylating, acylating and/or sulfonylating said amino group

according to techniques well known in the field of synthetic organic chemistry.

Method G

In this method, compounds of formula (I-1) of the present invention wherein $R^3$ is a heterocyclyl group which may be substituted with group(s) selected from Substituent group α and Substituent group δ and which is substituted with group(s) selected from Substituent group β and Substituent group γ; or a heterocyclyl group having at least one nitrogen atom which may be substituted with group(s) selected from Substituent group a and Substituent group δ; or a group of the general formula - $CH(OH)-(CH_2)_k-R^4$ (wherein $R^4$ is as defined above, and the moiety $-CH(OH)-(CH_2)_k-$ is a lower alkylene group as defined above for the group of formula X which is substituted with a hydroxy group, and k is 0 or an integer of from 1 to 5) can be prepared, as shown in Method G below.

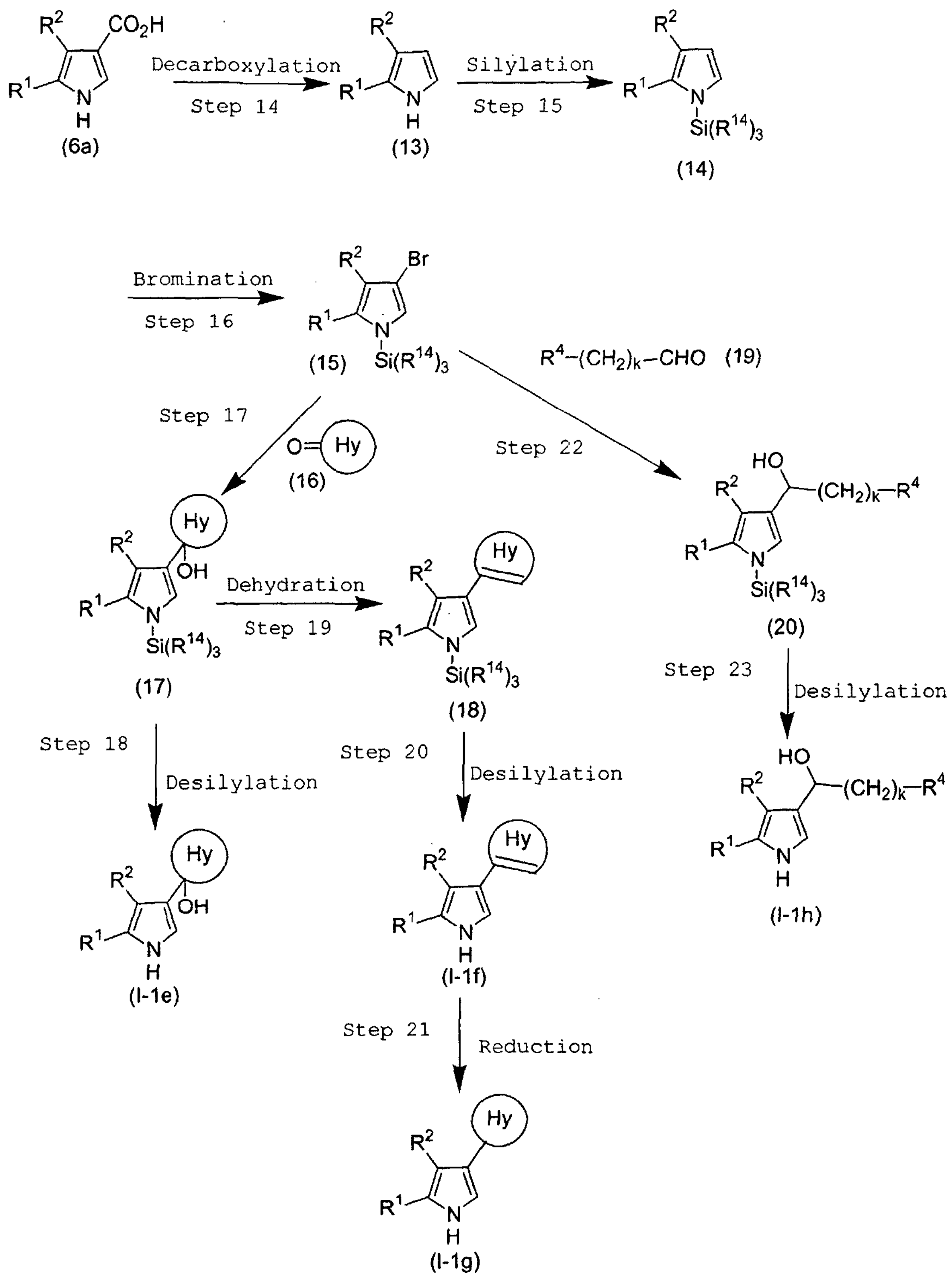


In the above formulae,

$R^1$, $R^2$, $R^4$ and k are as defined above,

the substituents $R^{14}$ may be the same or different from each other and each is selected from a hydrogen atom, a lower alkyl group as defined above, an aryl group as defined above and an aralkyl group as defined above, and

cyclic group Hy is a heterocyclyl group corresponding to the heterocyclyl group which may be substituted with group(s) selected from Substituent group $\alpha$ and Substituent group $\delta$ and which is substituted with group(s) selected from Substituent group $\beta$ and Substituent group $\gamma$ or a heterocyclyl group having at least one nitrogen atom, which may

be substituted with group(s) selected from Substituent group α and Substituent group δ in the definition of $R^4$.

Step 14

In this Step, a di-substituted pyrrole compound of formula (13) is prepared by decarboxylating a carboxylic acid compound of formula (6a). Decarboxylation of the compound of formula (6a) can be performed by heating under acidic, alkaline or neutral conditions according to techniques conventionally used in synthetic organic chemistry, for example according to the methods described in detail in N. Yoshida et al., Yakugaku Zasshi, 93(5), 584-598 (1973).

Steps 15

In this Step, a silyl compound of formula (14) is prepared by silylating the nitrogen atom at the 1-position of the di-substituted pyrrole compound of formula (13).

Step 16

In this Step, a bromopyrrole compound of formula (15) is prepared by brominating the silyl compound of formula (14) with a brominating agent (for example, N-bromosuccinimide, etc.).

Step 17

In this Step, a hydroxyheterocyclyl compound of formula (17) is prepared by lithiating the compound of formula (15) and then reacting it with a heterocyclyl ketone of formula (16).

Step 18

In this Step, the desired compound of formula (I-1e) is prepared by deprotecting (desilylating) the compound of formula (17) with, for example, tetrabutylammonium fluoride (TBAF).
Each of the reactions in Steps 15 to 18 can be carried out according to the method described in detail in Brian L. Bray et al., J. Org. Chem., 55, 6317-6318 (1990).

Step 19

In this Step, an unsaturated heterocyclyl compound of formula (18) is prepared by subjecting the hydroxyheterocyclyl compound of formula (17) to a dehydration reaction. Dehydration reactions can be carried out using well known techniques. For example, the dehydration reaction can typically be carried out in the presence of an acid catalyst such as sulfuric acid, a solid catalyst such as alumina or a halogenating agent such as thionyl chloride [e.g. in accordance with the methods described in detail in G. H. Coleman & H. F. Johnstone, Org. Synth., I, 183 (1941), R. L. Sawyer & D. W. Andrus, Org. Synth., III, 276 (1955) and J. S. Lomas et al., Tetrahedron Lett., 599 (1971)]. Alternatively, the dehydration reaction of this step can be accomplished by a reaction using a trialkylsilane, such as triethylsilane, tripropylsilane or tributylsilane, and trifluoroacetic acid, for example in accordance with the method described in Francis A. Carey & Henry S. Tremper, J. Am. Chem. Soc., 91, 2967-2972 (1969).

Step 20

In this Step, the desired compound of formula (I-1f) of the present invention is prepared by removing the protecting group (the silyl group) from the unsaturated heterocyclyl compound of formula (18) in a similar manner to that performed in Step 18.

Step 21

In this Step, the desired compound of formula (I-1g) of the present invention is prepared by reducing the double bond in the cyclic group Hy of the compound of formula (I-1f). This is carried out in a similar manner to the procedure described in Step 11 above.

Step 22

In this Step, a hydroxy compound of formula (20) is prepared by reaction between the bromopyrrole compound

of formula (15) and an aldehyde compound of formula (19). This is carried out in a procedure similar to that described in Step 17 above.

Step 23

In this Step, the desired compound of formula (I-1h) of the present invention is prepared by removing the protecting group (silyl group) from the hydroxy compound of formula (20). This is carried out in a similar manner to that described in Step 18 above.

Where k in the compound of formula (I-1h) above is an integer of from 1 to 5, the hydroxyalkane moiety can be converted into the corresponding alkenylene moiety by subjecting such a compound to a dehydration reaction in a similar manner to that described in Step 19 above.

Method H

Generally, the compounds of formula (I) of the present invention can be prepared by introducing the $R^3$ group into a pyrrole compound already substituted on the pyrrole ring with the $R^1$ group and $R^2$ group. Compounds of formula (I-1) can be prepared, for example, according to the Method H, as shown below.

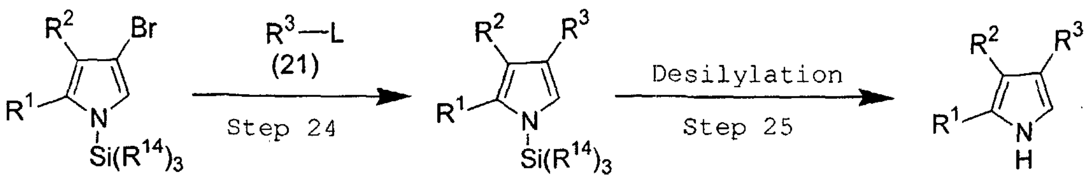


(15) (22) (I-1)

In the above formulae, $R^1$, $R^2$, $R^3$ and $R^{14}$ are as defined above, and
L represents a leaving group.

The leaving group L is a group which is capable of leaving as a nucleophilic residue. Examples include halogen atoms such as fluorine, chlorine, bromine and iodine, trihalogenomethyloxy groups such as trichloromethoxy, lower alkanesulfonyloxy groups such as methanesulfonyloxy and ethanesulfonyloxy groups, lower halogeno alkane sulfonyloxy groups such as trifluoromethanesulfonyloxy and pentafluoroethanesulfonyloxy groups, and arylsulfonyloxy groups such as benzenesulfonyloxy, p-toluenesulfonyloxy and p-nitrobenzenesulfonyloxy groups. Of these, halogen atoms are preferred, and bromine atom is particularly preferred.

Step 24

In this Step, a compound of formula (22) is prepared by lithiating the bromopyrrole compound of formula (15) using a procedure similar to that described in Step 17 above and then reacting the lithiated intermediate with a compound of formula (21). Substitution reactions can be carried out using, for example, the methods described in detail in WO 99/01449.

Step 25

In this Step, the desired compound of formula (I-1) is prepared by removing the protecting group (silyl group) from the compound of formula (22) according to a procedure similar to that used in Step 18 above.

Method I

In this method, compounds of formula (I) of the present invention wherein substituent $R^3$ is a group of formula $-X-R^4a$, wherein $R^4a$ is a heterocyclyl group which may be substituted with group(s) selected from Substituent group $\alpha$ and Substituent group $\delta$ and which is substituted with group(s) selected from Substituent group $\beta$ and Substituent group $\gamma$, or a heterocyclyl group having at least one nitrogen atom, which may be substituted with group(s) selected from Substituent group $\alpha$ and Substituent group $\delta$, or a group of formula $-NR^aR^b$, wherein $R^3$ and $R^b$ are as defined above, in which said group is bonded to the group X through the nitrogen atom in said group $R^4a$, can be prepared as shown below.

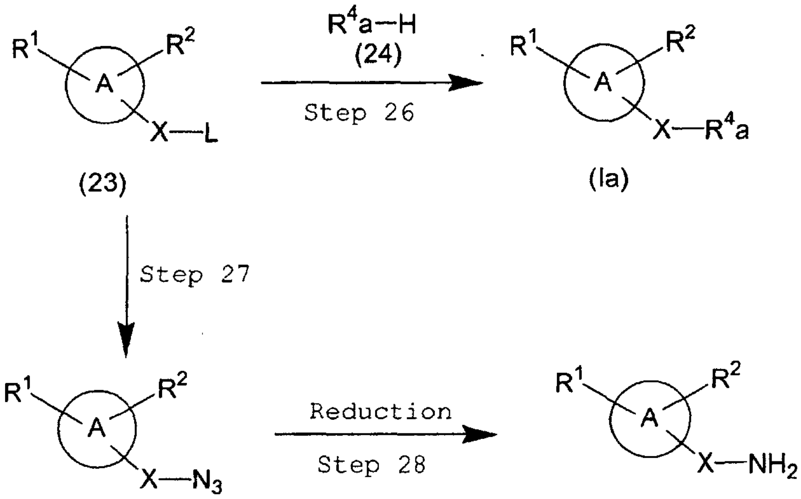

(23) (Ia) (25) (Ia')

In the above formulae, the cyclic group A, $R^1$, $R^2$, $R^4a$, L and X are as defined above.

Step 26

In this Step, the desired compound of formula (Ia) of the present invention is prepared by reacting a compound of formula (23) with an amine compound of formula (24) so as to replace the group L with the group $R^4a$. This reaction is usually carried out in a solvent in the presence or absence of a base.

There is no particular restriction on the nature of the solvent to be employed, provided that it has no adverse effect on the reaction. Examples of suitable solvents which can be used include: alcohols such as methanol, ethanol, propanol and isopropanol; ethers such as diethyl ether, diisopropyl ether, tetrahydrofuran and dioxane; aprotic polar solvents such as dimethylformamide, dimethylacetamide and dimethyl sulfoxide; nitriles such as acetonitrile; esters such as methyl acetate and ethyl acetate; aromatic hydrocarbons such as benzene, toluene and xylene; and aliphatic hydrocarbons such as pentane, hexane and heptane.

Examples of the base which can be used include: alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo [5.4.0]-7-undecene.

A compound of formula (Ia') of the present invention, which is a compound of formula (Ia) wherein $R^4a$ is an amino group ($-NH_2$), can also be prepared via an azide compound (25).

Step 27

In this Step, an azide compound of formula (25) is prepared by reacting a compound of formula (23) with sodium azide in the presence of a solvent.

Examples of suitable solvents which can be used include: halogenated hydrocarbons such as dichloromethane, chloroform, carbon tetrachloride and dichloroethane; ethers such as diethyl ether, diisopropyl ether, tetrahydrofuran and dioxane; aprotic polar solvents such as dimethylformamide, dimethylacetamide and dimethylsulfoxide; nitriles such as acetonitrile; esters such as methyl acetate and ethyl acetate; aromatic hydrocarbons such as benzene, toluene and xylene; aliphatic hydrocarbons such as pentane, hexane and heptane; and mixtures thereof.

Step 28

In this Step, the desired compound of formula (Ia') of the present invention is prepared by reducing the azide compound of formula (25). Reduction reactions can be carried out using the catalytic reduction method described in detail in E. J. Corey et al., Synthesis, 590 (1975).

Method J

In this method, compounds of formula (Ib) of the present invention wherein $R^2$ is a heteroaryl group having at least one nitrogen atom substituted with a group of formula $-NR^cR^d$, can be prepared, as shown in Method J below.

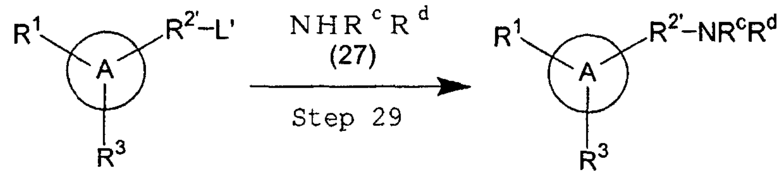


(26) (Ib)

In the above formulae, the cyclic group A, $R^1$, $R^3$, $R^c$ and $R^d$ are as defined above,

L' is a leaving group, and

the group $-R^{2'}-L'$ is a group consisting of a heteroaryl group wherein said heteroaryl group has at least one nitrogen atom as defined above in the definition of substituent $R^2$ and a leaving group (group L').

Thus, the group $-R^{2'}-L'$ is, for example, 2-methanesulfonylpyrimidin-4-yl, 2-methanesulfonylpyridin-4-yl, etc. "Heteroaryl group having at least one nitrogen atom" has the same meaning as "heteroaryl group having at least one nitrogen atom" in the definition of $R^2$.

The leaving group L' is a similar group to the leaving groups defined and exemplified above in the definition of L, or it is a lower alkylsulfonyl group such as a methanesulfonyl, ethanesulfonyl, propanesulfonyl or butanesulfonyl group. The group L' is preferably a lower alkylsulfonyl group, and more preferably a methanesulfonyl group.

Step 29

In this Step, the desired compound of formula (Ib) of the present invention is prepared by reacting a compound of formula (26) with an amine compound of formula (27) to replace the leaving group with a group of formula $-NR^cR^d$. This step is carried out using a procedure similar to that used in Step 26 above.

In this method, compounds of formula (I) of the present invention wherein $R^3$ is a group containing a pyridine ring can be prepared by subjecting a compound of formula (I) in which $R^3$ is a tetrahydropyridine group to a dehydrogenation reaction to convert the tetrahydropyridine group to a pyridine group, this dehydrogenation being as shown below.

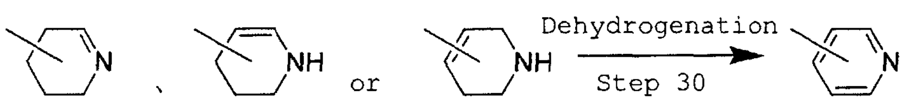


Dehydrogenation reactions of the type depicted in Step 30 can be carried out using the method described in detail in G. Wieslaw et al., J. Med. Chem., 28, 311-317 (1985), using a palladium-carbon catalyst.

The compounds which can be used as starting materials in Method A to Method J above, that is the compounds of formulae (1a), (1b), (1c) , (1d) , (1e), (2a), (2b) , (2c) , (2d) , (2e), (4), (5), (6a), (16), (19), (21), (24) and (27), are either known compounds themselves or are compounds which are easily obtainable by treating known compounds according to known methods (for example, according to the methods described in WO 97/5877). The compounds of formulae (23) and (26) respectively can be easily synthesized from known compounds by carrying out similar reactions to those described in Method A to Method E above.

The compound of formula (16) also can be prepared according to Method K to Method O.

Method K

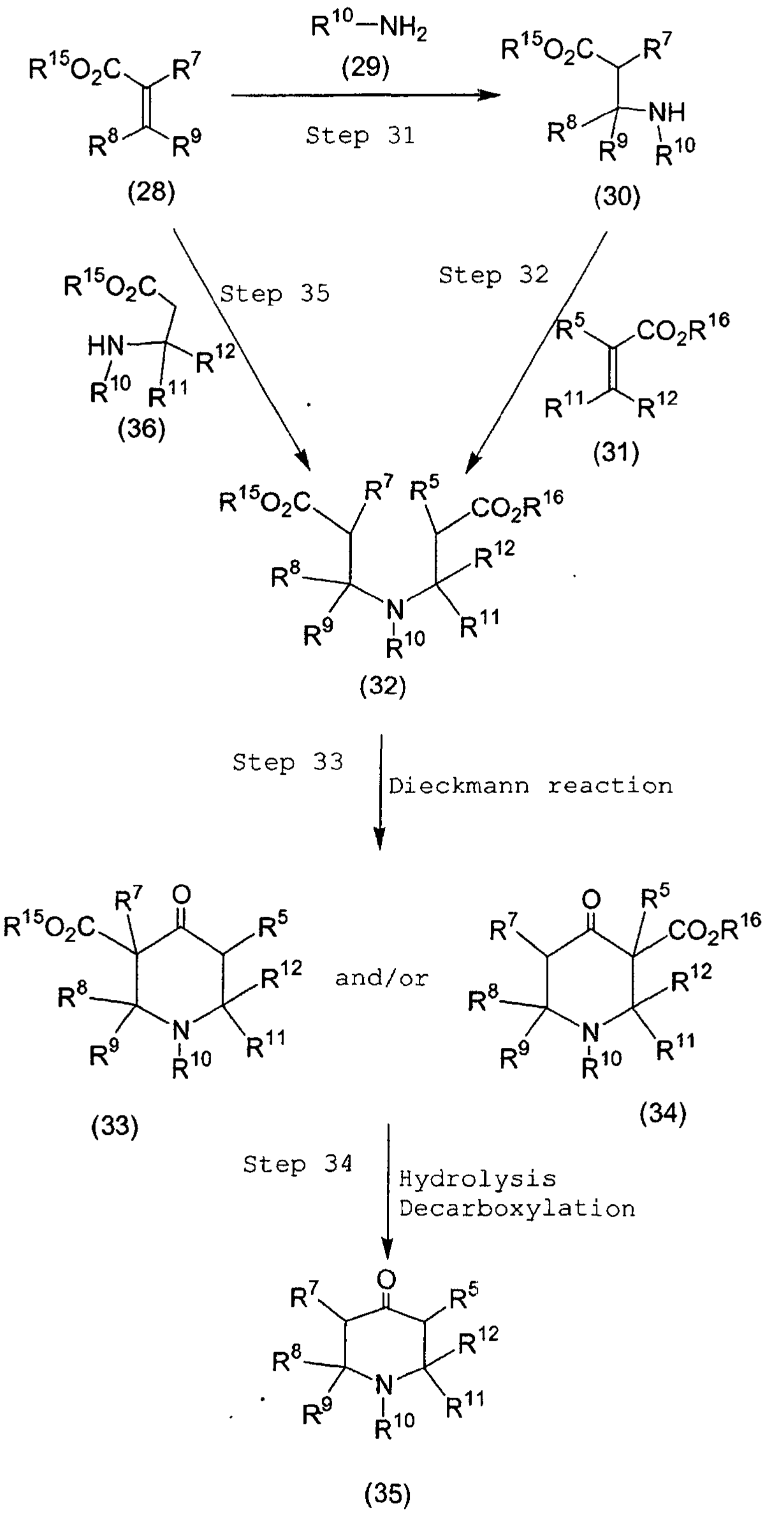


(28) (30) (36) (31) (32)

(33) and/or (34)

(35)

In the above formulae, $R^5$, $R^7$, $R^8$, $R^9$, $R^{10}$, $R^{11}$ and $R^{12}$ are as defined above, and $R^{15}$ and $R^{16}$ are the same or different from each other and each is a lower alkyl group as defined above or an aralkyl group as defined above.

In the Method K, an amino ester compound of formula (30) is prepared by an addition reaction between an unsaturated ester compound of formula (28) and an amine compound of formula (29)(Step 31), then, a diester compound of formula (32) is obtained by a further addition reaction between an unsaturated ester compound of formula (31) and the amino ester compound of formula (30)(Step 32), then the compound of formula (32) is subjected to Dieckmann reaction to give a keto ester compound of formula (33) and/or formula (34) (Step 33), successively the compound of formula (33) and/or the compound of formula (34) is subjected to consecutive hydrolysis and decarboxylation reactions to prepare the desired compound of formula (35) (Step 34).

In Step 34, the reaction is usually carried out in the presence or absence of a solvent with or without the addition of an acid or base.

Examples of suitable solvents include water, or a mixture of water and an organic solvent (examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethyl-acetamide and dimethyl sulfoxide; nitriles such as acetonitrile; and esters such as methyl acetate and ethyl acetate).

The acid to be used is not particularly limited provided that it is one that is usually used as an acid in hydrolysis reactions, and examples thereof include mineral acids such as hydrochloric acid, sulfuric acid and phosphoric acid; carboxylic acids such as formic acid, acetic acid, propionic acid and trifluoroacetic acid; and sulfonic acids such as methanesulfonic acid and ethanesulfonic acid. The hydrolysis reaction is accelerated by the addition of an acid.

The base to be used is not particularly limited provided that it is one that is usually used as a base in hydrolysis reactions, and examples thereof include alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo [5.4.0]undec-7-ene.

The reaction temperature is from -20°C to 150°C, and preferably from 0°C to 100°C.

The time required for the reaction is from 10 minutes to 48 hours, and preferably from 30 minutes to 12 hours is usually sufficient.

The reactions in Steps 33 and 34 can be carried out using the procedures described in detail in J. R. Harrison et al., J. Chem. Soc., Perkin Trans. 1, 1999, 3623 -3631.

Step 35

In this Step, the diester compound of formula (32) is prepared by reacting the unsaturated ester compound of formula (28) with an amino ester compound of formula (36). This is carried out in a similar manner to that described in Step 32 above. This step is preferably employed where the compound of formula (32) to be prepared is one wherein $R^{10}$ and $R^{11}$ together form a lower alkylene group or a lower alkylene group which is substituted with group(s) selected from Substituent group α, Substituent group β, Substituent group γ, Substituent group δ and Substituent group ε.

Each of the reactions in Steps 31 to 35 is described in detail in U. M. Teotino, J. Org. Chem., 27, 1406 (1962).

Method L

In this method, a compound of formula (35a), which is a compound of formula (16) above wherein $R^{10}$ and $R^{11}$ together form a lower alkylene group or a lower alkylene group which is substituted with group(s) selected Substituent group α, Substituent group β, Substituent group γ, Substituent group δ and Substituent group ε can be prepared as shown in below.

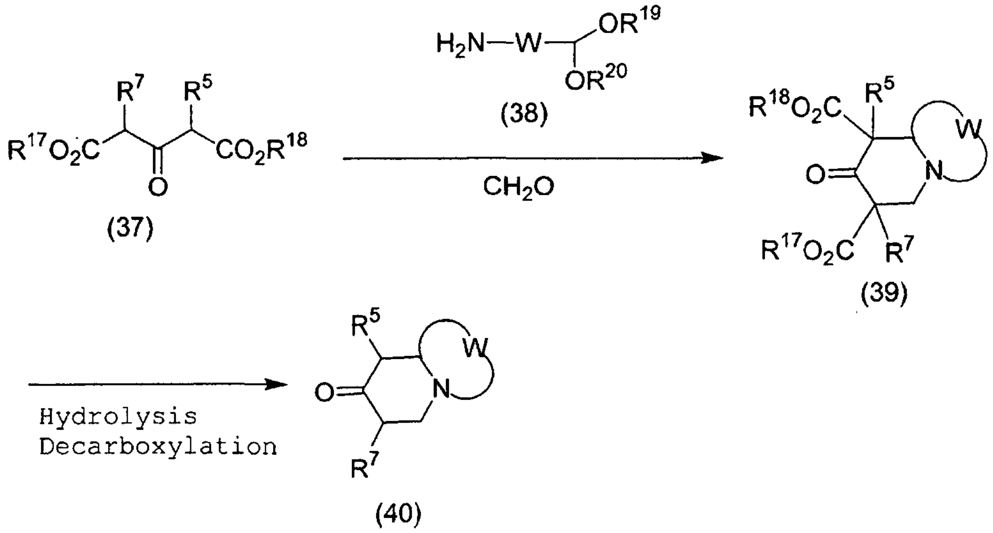


(37) (38) (39) (40)

In the above formulae, $R^5$ and $R^7$ have the same meanings defined above,

$R^{17}$ and $R^{18}$ are the same or different from each other and each represents a lower alkyl group as defined above or an aralkyl group as defined above,

$R^{19}$ and $R^{20}$ are the same or different from each other and each represents a lower alkyl group as defined above or $R^{19}$ and $R^{20}$ together represent a lower alkylene group as defined above, and

W represents a lower alkylene group or a lower alkylene group which is substituted with group(s) selected from Substituent group α, Substituent group β, Substituent group γ, Substituent group δ and Substituent group ε.

The steps of Method L can be carried out according to the methods described in detail in O. Pollet et al., Heterocycles, 43, 1391 (1996) or Anet et al.. Austral. J. Scient. Res., <A>3, 635-640 (1950).

The heterocyclyl ketone compound which is a compound of formula (16) having a group of general formula (IIa) or (IIb) can be prepared according to Method M to Method O.

Method M

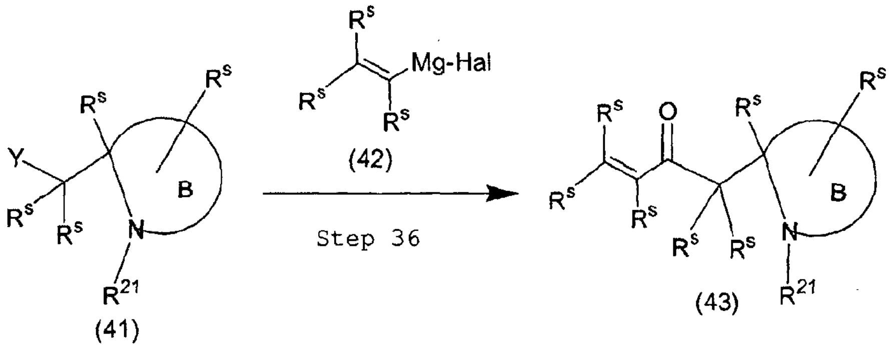


(41) (42) (43)

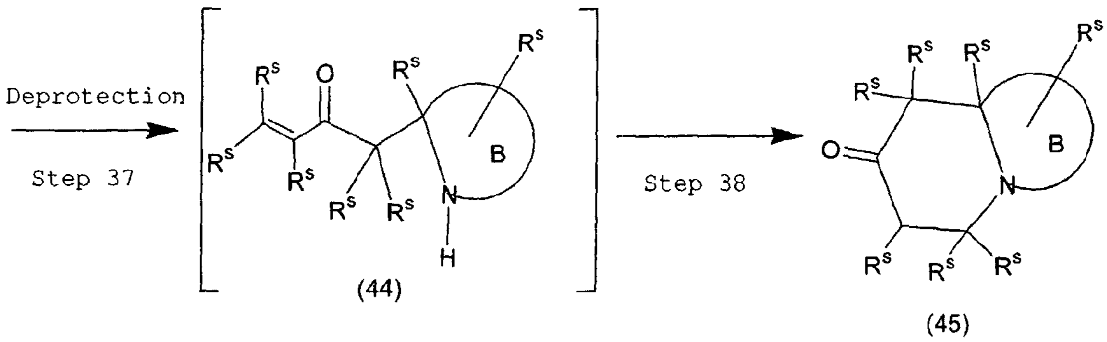


(44) (45)

In the above formulae, B and $R^S$ are as defined above,

$R^{21}$ represents an amino protecting group,

Hal represents a halogen atom (preferably, it is a chlorine atom, bromine atom or iodine atom), and

Y represents a halogenocarbonyl group (for example, -CO-Cl, -CO-Br or -CO-I), a N-(lower alkoxy)-N-(lower alkyl)carbamoyl group (examples of such groups include N-methoxy-N-methylcarbamoyl, N-ethoxy-N-methylcarbamoyl and N-ethyl-N-methoxycarbamoyl groups) or a cyano group.

The amino protecting group in the definition of $R^{21}$ can be any protecting group for an amino group which is commonly used in organic synthesis, examples of which are aliphatic acyl groups as defined above, aromatic acyl groups as defined above, silyl groups as defined above, aralkyl groups as defined above, alkoxycarbonyl groups as defined above, alkenyloxycarbonyl groups as defined above and aralkyloxycarbonyl groups as defined above.

Step 36

In this Step, an α,β-unsaturated ketone derivative of formula (43) is prepared by reacting a cyclic amino acid derivative of formula (41) with a Grignard reagent of an olefin compound of formula (42). Reactions of this type are well known for the preparation of ketones from carboxylic acid derivatives and Grignard reagents, and any such reaction can be employed; for example, it can be carried out using the procedures described in detail in H. R. Snyder et al., Org. Synth., III, 798 (1955); J. Cason et al., J. Org. Chem., 26, 1768 (1961); G. H. Posner et al., J. Am. Chem. Soc., 94, 5106 (1972); and G. H. Posner, Org. React., 19, 1 (1972).

Step 37

In this Step, the nitrogen protecting group ($R^{21}$) in the α,β-unsaturated ketone derivative of formula (43) is removed to afford a deprotected intermediate of formula (44).

Step 38

In this step, the desired cyclic aminoketone compound of formula (45) is prepared by cyclizing the compound of formula (44).

In Step 37, the deprotection reaction employed can be any which is conventionally used in organic synthesis (examples of which are described in T.W.Greene et al., Protective Groups in Organic Synthesis, John Willey & Sons, Inc.). Preferably, the deprotection reaction is conducted under neutral or acidic conditions. After the deprotection reaction, the resulting product of formula (44), which is not isolated, cyclizes immediately to give the desired aminoketone compound of formula (45). The deprotection reaction (Step 37) is more preferably conducted under acidic conditions, and the aminoketone compound of formula (45) is prepared without further reaction by neutralizing the reaction mixture.

Method N

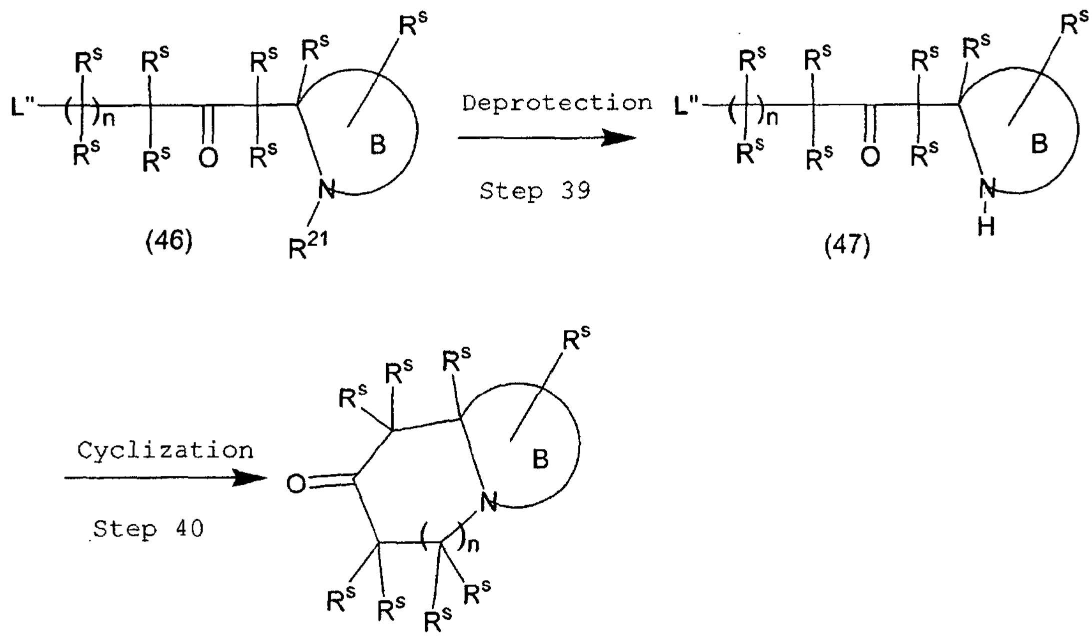


(46) (47)

(48)

In the above formulae, B, $R^s$, $R^{21}$ and m are as defined above, and

L" represents a leaving group as defined for the leaving group L above, a lower alkylsulfonyl group as defined above, an arylsulfonyl group as defined above or a halogeno lower alkylsulfonyl group (examples of said group including trifluoromethanesulfonyl and pentafluoroethanesulfonyl groups).

Steps 39 and 40

Steps 39 and 40 involve first removing the amino protecting group ($R^{21}$) from the ketone compound (46) having the leaving group L" to afford a deprotected intermediate of formula (47), and then cyclizing said intermediate to produce the desired aminoketone compound of formula (48). These steps can be carried out in a manner similar to the reactions described in Steps 37 and 38 above.

The starting compound of formula (46) used as the starting material in this method is either a known compound or it can be prepared from a known compound using known methods [for example, the methods described in S. W. Goldstein et al., J. Org. Chem., 57, 1179-1190 (1992); and B. Achille et al., J. Comb. Chem., 2, 337-340 (2000)].

Method O

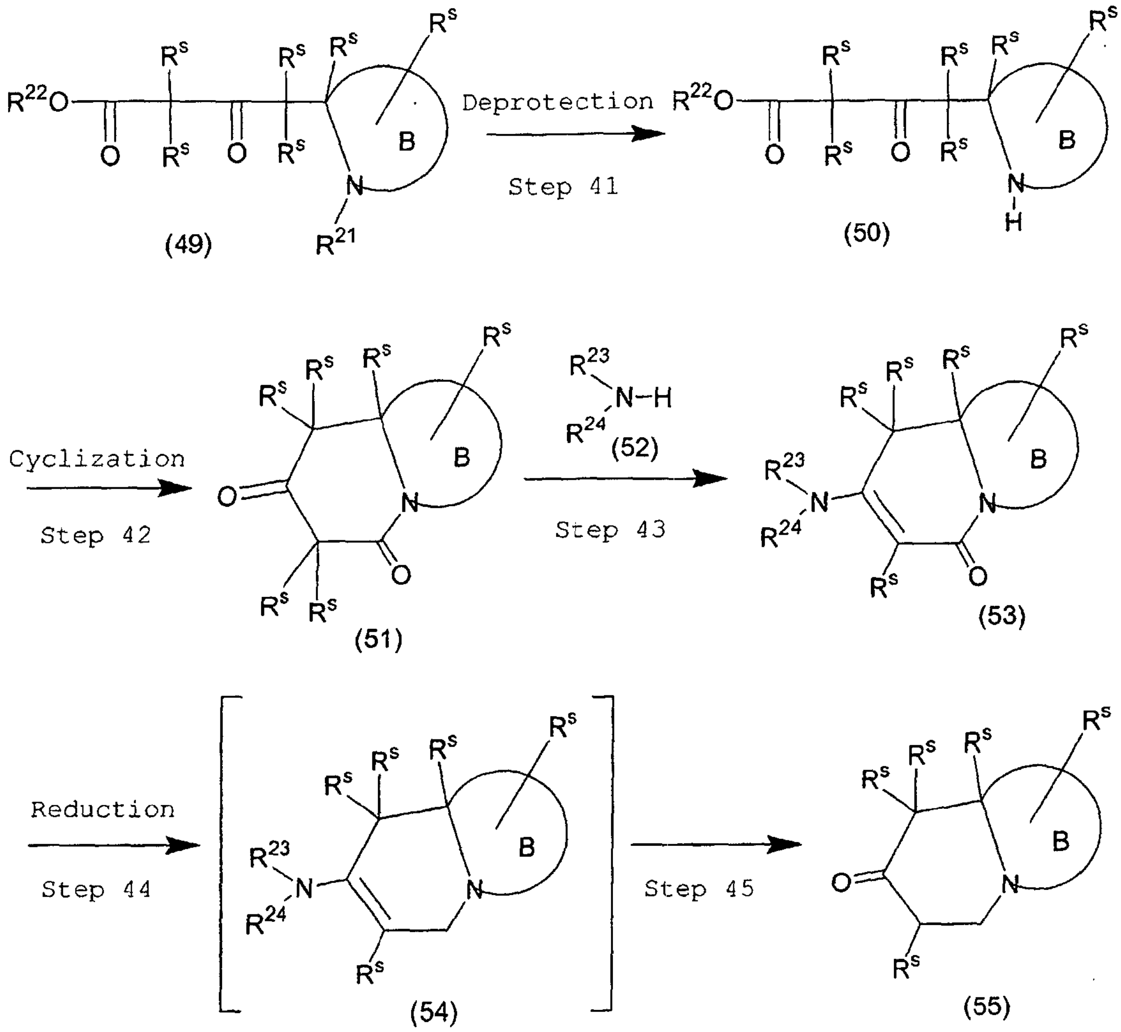


In the above formulae,

$R^S$, $R^{21}$ and B are as defined above,

$R^{22}$ represents a hydrogen atom or a carboxyl protecting group, and

$R^{23}$ and $R^{24}$ are the same or different from each other and each represents a hydrogen atom, a lower alkyl group as defined above or an aralkyl group as defined above, or $R^{23}$ and $R^{24}$, together with the nitrogen atom to which they are attached, form a 5- or 6-membered non-aromatic heterocyclic group which includes one ring nitrogen atom and which may include one further heteroatom selected from oxygen, sulfur and nitrogen atoms (examples of such groups include piperidyl, piperazinyl, morpholinyl and thiomorpholinyl groups).

The carboxyl protecting group in the definition of $R^{22}$ can be any protecting group for a carboxyl group which is commonly used in organic synthesis, examples of which include a lower alkyl group as defined above, a lower alkenyl group as defined above or an aralkyl group as defined above, preferably, it is a lower alkyl group as defined above or an aralkyl group as defined above.

Steps 41 and 42

In these Steps 41 and 42, a ketolactam compound of formula (51) is prepared by first removing the amino protecting group ($R^{21}$) from an α-ketoacid compound of formula (49) to afford a deprotected intermediate of formula (50), and then cyclizing said intermediate. These steps are conducted in a manner similar to that described in Steps 37 and 38 above.

Step 43

In this Step, a cyclic enaminolactam compound of formula (53) is prepared by reacting the ketolactam compound of formula (51) with a secondary amine compound of formula (52). Any of the techniques conventionally used in the field of organic synthetic chemistry for the preparation of enamine derivatives can be employed. For example, the step can be carried out according to the procedure described in G. Stork et al., J. Am, Chem. Soc., 85. 207 (1963).
The reaction is usually carried out in a solvent in the presence or absence of an acid.
The reaction is normally and preferably effected in the presence of a solvent. There is no particular restriction on the nature of the solvent to be employed, provided that it has no adverse effect on the reaction. Examples of suitable solvents include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethyl-acetamide and dimethyl sulfoxide; nitriles such as acetonitrile; and esters such as methyl acetate and ethyl acetate, of which ethers are preferred.
The acid to be used in the reaction is not particularly limited provided that it is one that is usually used in such reactions, and examples thereof include: inorganic acids such as hydrogen chloride, hydrogen bromide, sulfuric acid, perchloric acid and phosphoric acid; and organic acids such as acetic acid, formic acid, oxalic acid, methanesulfonic acid, p-toluenesulfonic acid, trifluoroacetic acid and trifluoromethanesulfonic acid. Of these, sulfuric acid, hydrochloric acid and p-toluenesulfonic acid are preferred.
The reaction of this step can be carried out efficiently by removing water produced during the reaction using molecular sieves or a water separator (for example, a Dean Stark Water Separator which can be obtained from Aldrich).
The reaction temperature is from -20°C to 150°C, and preferably from 0°C to 100°C.
The time required for the reaction is from 10 minutes to 48 hours, and preferably from 30 minutes to 12 hours is usually sufficient.

Step 44

In this Step, a cyclic enamine compound of formula (54) is prepared by reducing the cyclic enaminolactam compound of formula (53). Any of the techniques conventionally used in the field of organic synthetic chemistry for performing reduction reactions can be employed. For example, the reduction can be carried out according to the procedures described in S. Cortes et al., J. Org. Chem., 48, 2246 (1983); Y. Tsuda et al., Synthesis, 652 (1977); H. C. Brown et al., J. Am. Chem. Soc., 86, 3566 (1964) and R. J. Sundberg et al., J. Org. Chem., 46, 3730 (1981)
This reaction is usually carried out in a solvent in the presence of a reducing reagent.
Examples of the reducing reagent to be employed include hydride reagents such as alkali metal borohydrides e.g. sodium borohydride and lithium borohydride, and aluminum hydrides e.g. lithium aluminum hydride and lithium triethoxyalumino-hydride; a combination of a Lewis acid such as aluminum chloride, tin tetrachloride or titanium tetrachloride and a hydride reagent as defined above; and boron compounds such as diborane.
In the reduction reaction, non-polar solvents can be used, preferred examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; and ethers such as diethyl ether, diisopropyl ether, tetrahydrofuran and dioxane.
The reaction temperature is from -20°C to 150°C, and preferably from 0°C to 100°C.
The time required for the reaction is from 10 minutes to 48 hours, and preferably from 30 minutes to 12 hours is usually sufficient.

Step 45

In this Step, the desired cyclic aminoketone compound of formula (55) is obtained by hydrolizing the cyclic enamine compound of formula (54). This reaction is performed by bringing the cyclic enamine compound of formula (54) into contact with water in the presence or absence of a solvent with or without the addition of an acid or base.
Examples of suitable solvents include water, or a mixture of water and an organic solvent (examples of which include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethyl-acetamide and dimethyl sulfoxide; nitriles such as acetonitrile; and esters such as methyl acetate and ethyl acetate).

The acid to be used is not particularly limited provided that it is one that is usually used as an acid in hydrolysis reactions, and examples thereof include mineral acids such as hydrochloric acid, sulfuric acid and phosphoric acid; carboxylic acids such as formic acid, acetic acid, propionic acid and trifluoroacetic acid; and sulfonic acids such as methanesulfonic acid and ethanesulfonic acid. The hydrolysis reaction is accelerated by the addition of an acid.

The base to be used is not particularly limited provided that it is one that is usually used as a base in hydrolysis reactions, and examples thereof include alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo [5.4.0]undec-7-ene.

The reaction temperature is from -20°C to 150°C, and preferably from 0°C to 100°C.

The time required for the reaction is from 10 minutes to 48 hours, and preferably from 30 minutes to 12 hours is usually sufficient.

Method P

The compound of formula (53), which is an intermediate in the preparation of the cyclic aminoketone compound of formula (55), can also be produced by Method P below.

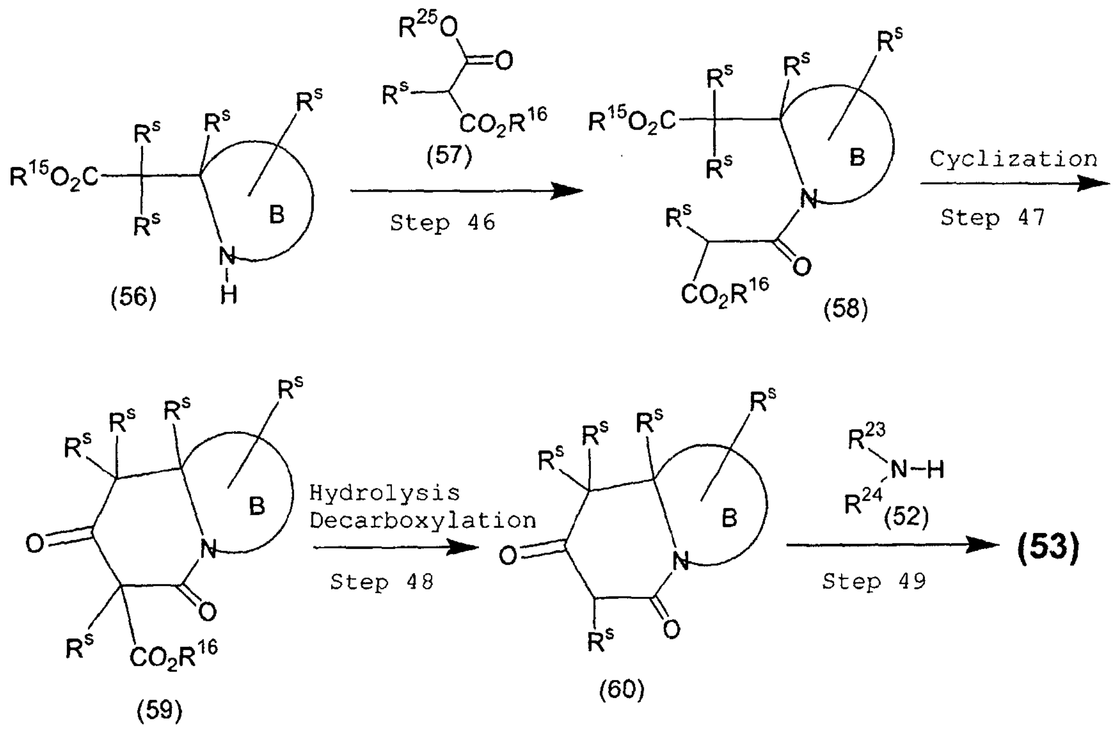


(56) (57) (58) (59) (60) (52) (53)

In the above formulae, B, $R^S$, $R^{15}$, $R^{16}$, $R^{23}$ and $R^{24}$ are as defined above, and

$R^{25}$ represents a hydrogen atom or a carboxyl protecting group.

The carboxyl protecting group in the definition of $R^{25}$ can be any such protecting group conventionally used in the organic chemistry; preferably, it is a lower alkyl group as defined above or an aralkyl group as defined above.

Step 46

In this Step, an aminodiester compound of formula (58) is produced by the reaction of a cyclic amino acid ester compound of formula (56) with a malonic acid derivative of formula (57) or a reactive derivative thereof. Any of the techniques conventionally used in the field of organic synthetic chemistry for amidation reactions can be employed, and this step can, for example, be carried out in the manner described in processes (a), (b) and (c) described below.

(a) When $R^{25}$ is a hydrogen atom, the reaction is conducted in a solvent in the presence of a condensing agent and in the presence or absence of a base.

Examples of the solvent to be employed include: aliphatic hydrocarbons such as pentane, hexane and heptane; aromatic hydrocarbons such as benzene, toluene and xylene; halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethyl-formamide, N,N-dimethylacetamide and dimethyl sulfoxide; nitriles such as acetonitrile; esters such as methyl acetate and ethyl acetate; water; or a mixture of these solvents described above.

Examples of the condensing agent to be used include dicyclohexylcarbodiimide, 1-ethyl-3-(3-dimethylaminopropyl)carbodiimide, N,N'-carbonyldiimidazole and the like.

Examples of the base to be used include alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]undec-7-ene.

The reaction temperature is from -20°C to 150°C, and preferably from 0°C to 100°C.

The time required for the reaction is from 10 minutes to 48 hours, and preferably from 30 minutes to 12 hours is usually sufficient.

Alternatively, where $R^{25}$ is a hydrogen atom, the reaction can also be carried out by converting the compound of formula (57) into a reactive derivative thereof followed by the procedure described in process (c) below.

(b) When $R^{25}$ is a carboxyl protecting group (preferably a lower alkyl group as defined above or an aralkyl group as defined above), the reaction is performed by heating in the presence or absence of a solvent.

When the reaction is conducted in a solvent, the same solvent as that described in process (a) can be used. The temperature for the reaction is between 30°C and 100°C, preferably between the range of ± 5°C of the boiling point of the solvent that is employed. Most preferably, the reaction is carried out by heating the reaction mixture under reflux.

When a solvent is not used in this reaction, the desired compound is prepared by heating a mixture of the compounds of formulae (56) and (57). The reaction temperature is from 30°C to 150°C, and preferably from 50°C to 120°C.

The time required for the reaction is from 10 minutes to 48 hours, and preferably from 30 minutes to 12 hours is usually sufficient.

(c) When a reactive derivative of a compound of formula (57) is used, the reactive derivative can be an acid halide, a mixed acid anhydride, an activated ester, an active amide or the like, and the reaction is conducted in a solvent in the presence of a condensing agent and in the presence or absence of a base.

The acid halide of the compound of formula (57) is prepared by the reaction of a compound of formula (57) wherein $R^{25}$ is a hydrogen atom with a halogenation reagent (for example, thionyl chloride, oxalyl chloride or the like); the mixed acid anhydride is prepared by the reaction of a compound of formula (57) wherein $R^{25}$ is a hydrogen atom with an acid halide (for example, methyl chlorocarbonate, ethyl chlorocarbonate or the like); the activated ester is prepared by the reaction of a compound of formula (57) wherein $R^{25}$ is a hydrogen atom with a compound containing a hydroxyl group (for example, N-hydroxysuccinimide, N-hydroxyphthalimide or the like) in the presence of a condensing agent such as those described in process (a) above; and the active amide (for example, a Weinreb amide) is prepared by the reaction of a compound of formula (57) wherein $R^{25}$ is a hydrogen atom with an N-(lower alkoxy)-N-(lower alkyl)hydroxylamine (for example, N-methoxy-N-methylhydroxylamine or the like) in the presence of a condensing agent such as those described in process (a) above. Each of these reactions described can be conducted under reaction conditions usually used in organic synthetic chemistry for such reactions.

With regard to the solvent, condensing agent and base, the solvents, condensing agents and bases described in process (a) above can be used.

The reaction temperature is from -20°C to 150°C, and preferably from 0°C to 100°C.

The time required for the reaction is from 10 minutes to 48 hours, and preferably from 30 minutes to 12 hours is usually sufficient.

Steps 47 and 48

In Steps 47 and 48, a ketolactam compound of formula (60) is prepared by first executing a Dieckman reaction on the amido diester compound of formula (58) to afford a ketolactam ester compound (59), followed by the performance of hydrolysis and decarboxylation reactions on the product thus obtained. These steps can be conducted in a manner similar to that described in Steps 33 and 34 above.

Step 49

In this step, the target cyclic enaminolactam compound of formula (53) is prepared by the reaction of the ketolactam compound of formula (60) with a secondary amine compound of formula (52), and the reaction is carried out in a manner similar to that described in Step 43 above.

Method Q

The compound of formula (59), which is an intermediate in Method P described above, can also be synthesized by Method Q described below.

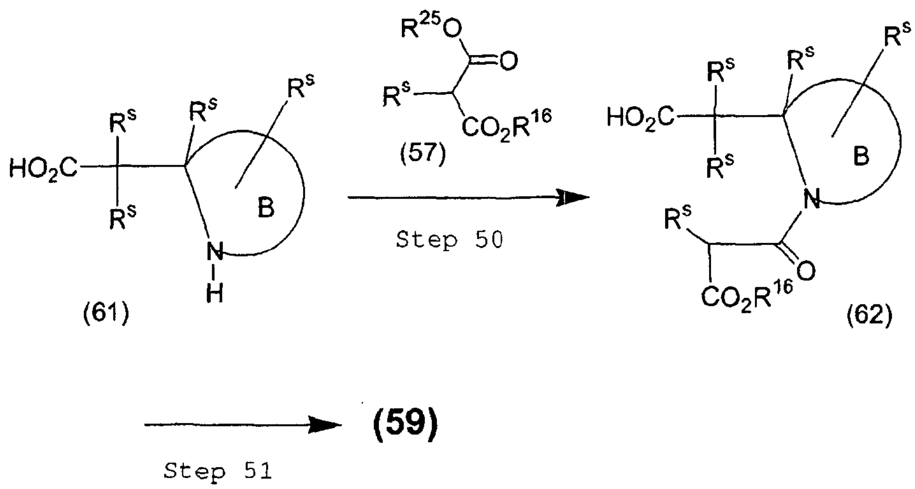


In the above formulae, B, $R^S$, $R^{16}$ and $R^{25}$ are as defined above.

Step 50

In this Step, an amido monoester compound of formula (62) is prepared by the reaction of a cyclic amino acid compound of formula (61) with a malonic acid derivative of formula (57) or a reactive derivative thereof. This step is carried out in a manner similar to that described in processes (a), (b) and (c) of Step 46 above.

Step 51

In this Step, the target ketolactam ester compound of formula (59) is prepared by the intramolecular condensation of a carboxyl group and an active methylene group of the amido monoester compound of formula (62). In this step, the compound of formula (62) is either used in underivatised form or after first being converted into a reactive derivative thereof.

(a) When the compound of formula (62) is used in underivatised form, the reaction is conducted in a solvent in the presence of a condensing agent and with or without a base.

Examples of the solvent to be employed include:

halogenated hydrocarbons such as dichloromethane, chloroform, carbon tetrachloride and dichloroethane; ethers such as diethyl ether, diisopropyl ether, tetrahydrofuran and dioxane; alcohols such as methanol, ethanol, propanol, isopropanol, butanol, s-butanol, isobutanol and t-butanol; aprotic polar solvents such as N,N-dimethylformamide, N,N-dimethylacetamide and dimethyl sulfoxide; nitriles such as acetonitrile; and esters such as methyl acetate and ethyl acetate; water; or a mixture of these solvents described above.

Examples of the condensing agent to be employed include dicyclohexylcarbodiimide, 1-ethyl-3-(3-dimethylaminopropyl)carbodiimide, N,N'-carbonyldiimidazole or the like.

Examples of the base to be used include alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0] undec-7-ene.

The reaction temperature is from -20°C to 150°C, and preferably from 0°C to 100°C.

The time required for the reaction is from 10 minutes to 48 hours, and preferably from 30 minutes to 12 hours is usually sufficient.

(b) When the compound (62) is used after first being converted into a reactive derivative, examples of the reactive derivative include acid halides, mixed acid anhydrides, activated esters, active amides and the like.

The acid halides are prepared by the reaction of the compound of formula (62) with a halogenation reagent (for example, thionyl chloride, oxalyl chloride or the like); the mixed acid anhydride is prepared by the reaction of the compound of formula (62) with an acid halide (for example, methyl chlorocarbonate, ethyl chlorocarbonate or the like); the activated ester is prepared by the reaction of the compound of formula (62) with a compound containing a hydroxyl group (for example, N-hydroxysuccinimide, N-hydroxyphthalimide or the like) in the presence of a condensing agent such as those described in process (a) above; and the active amide (for example, Weinreb amide) is prepared by the reaction of the compound of formula (62) with a N-(lower alkoxy)-N-(lower alkyl)hydroxylamine (for example, N-methoxy-N-methylhydroxylamine or the like) in the presence of a condensing agent such as those described in process (a) above. Each of these reactions described above can be conducted employing reaction conditions conventionally employed in organic synthetic chemistry for such reactions.

The cyclisation of said reactive derivative is usually carried out in a solvent in the presence or absence of a base.

With regard to the solvent, condensing agent and base, the solvents, condensing agents and bases described in process (a) above can be used.

The reaction temperature is from -20°C to 150°C, and preferably from 0°C to 100°C.

The time required for the reaction is from 10 minutes to 48 hours, and preferably from 30 minutes to 12 hours is usually sufficient.

The substituent of general formula (IIa), (IIb) or (IIc), which is one of the components of the compound of general formula (I), can be substituted with various substituents ($R^s$). The substituents $R^s$ can be converted into other substituents in each of the steps described above. The substituent $R^s$ can, for example, be converted as illustrated below employing conventional organic synthetic methods.

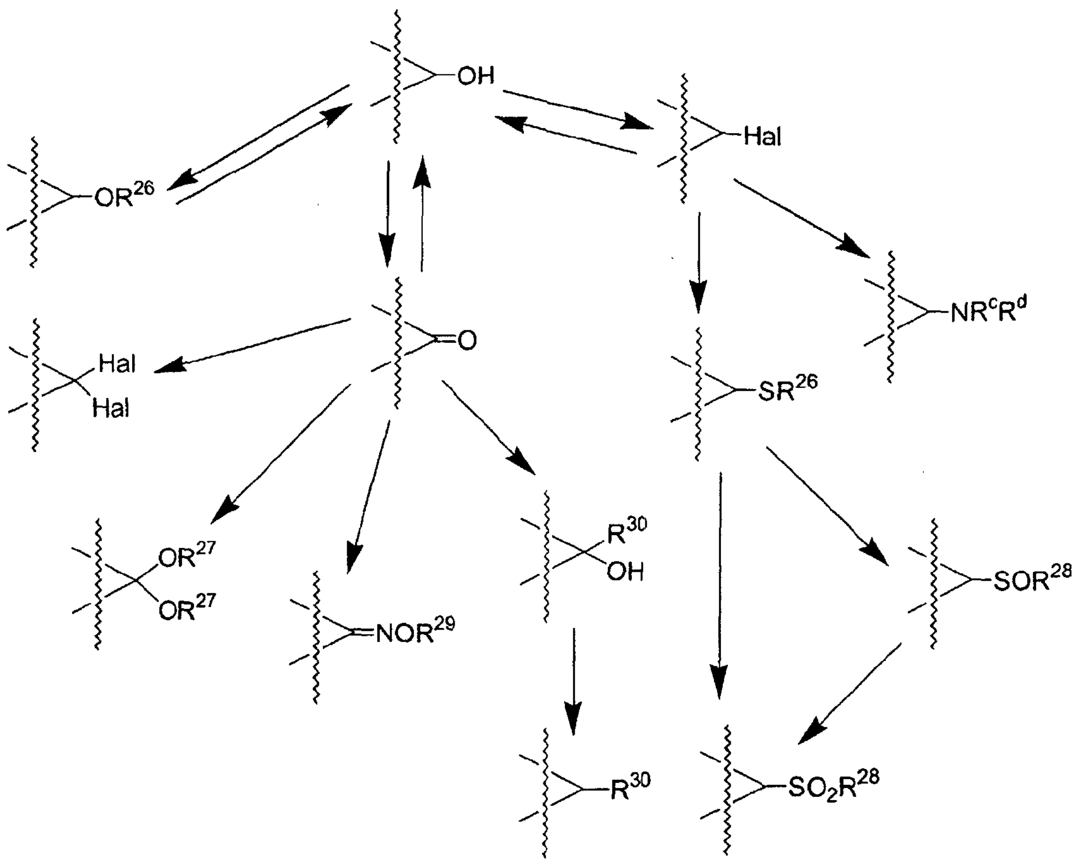

In the above formulae, $R^c$, $R^d$ and Hal have the same meanings as defined above,

$R^{26}$ represents a lower alkyl group as defined above or a halogenated lower alkyl group as defined above,

the groups $R^{27}$ are the same or different, and each represents a lower alkyl group as defined above or a halogenated lower alkyl group as defined above, or the two groups $R^{27}$ can together form a lower alkylene group as defined above,

$R^{28}$ represents a lower alkyl group as defined above,

$R^{29}$ represents a hydrogen atom or a lower alkyl group as defined above, and

$R^{30}$ represents a lower alkyl group, a lower alkenyl group, a lower alkynyl group, an aralkyl group, a cycloalkyl group, a lower alkyl group which is substituted with group(s) selected from Substituent group α, a lower alkenyl group which is substituted with group(s) selected from Substituent group α, or an alkynyl group which is substituted with group(s) selected from Substituent group α, as defined above in the definition of Substituent group β; or an aryl group or an aryl group which is substituted with group(s) selected from Substituent group α and Substituent group β as defined above in the definition of Substituent group γ.

Furthermore, when $R^s$ is a halogen atom, a hydroxyl group, a cyano group or a lower alkylsulfonyl group, $R^s$ can be converted into a hydrogen atom by the formation of a double bond, followed by the reduction of said double bond using conventional methods as illustrated below.

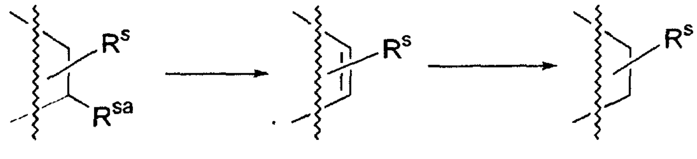

In the above formulae, $R^s$ has the same meaning as defined above, and $R^{sa}$ represents a halogen atom, a hydroxyl group, a cyano group or a lower alkylsulfonyl group.

After completion of each of the reactions described above, the desired compound may be isolated from the

reaction mixture in a conventional manner. For example, it can be obtained by neutralizing the reaction mixture as needed, removing insoluble matters by filtration, if any are present, adding water and an immiscible organic solvent such as ethyl acetate, washing with water or the like, separating the organic layer containing the desired compound, drying it over anhydrous magnesium sulfate or the like and then distilling off the solvent.

If necessary, the desired compound thus obtained can be isolated and purified by using conventional methods such as recrystallization or reprecipitation or by chromatographic methods. Examples of chromatography include adsorption column chromatography using a carrier such as silica gel, alumina or magnesium-silica gel type Florisil, chromatography using a synthetic adsorbent, for example, partition column chromatography using a carrier such as Sephadex LH-20 (product of Pharmacia), Amberlite XAD-11 (product of Rohm & Haas) or Diaion HP-20 (product of Mitsubishi Chemical), ion exchange chromatography and normal-phase·reverse-phase column chromatography (preferably high-performance liquid chromatography) using a silica gel or alkylated silica gel. If necessary, two or more of these techniques can be used in combination to isolate and purify the desired compound.

The compounds of formula (I) and pharmacologically acceptable salts, esters and other derivatives thereof according to the present invention exhibit excellent activity for the prophylaxis or treatment of hepatopathy and can be administered by a number of different routes. Examples of these administration routes include oral administration in the form of tablets, capsules, granules, powders or syrups and parenteral administration in the form of injections or suppositories. Such formulations can be prepared in a known manner by using additives such as an excipients, lubricants, binders, disintegrants, stabilizers, corrigents and diluents.

Examples of suitable excipients include: organic excipients, examples of which include sugar derivatives such as lactose, sucrose, dextrose, mannitol and sorbitol, starch derivatives such as corn starch, potato starch, $\alpha$-starch, dextrin and carboxymethyl starch, cellulose derivatives such as crystalline cellulose, low-substituted hydroxypropylcellulose, hydroxypropylmethylcellulose, carboxymethylcellulose, calcium carboxymethylcellulose and sodium internally-crosslinked carboxymethylcellulose, gum arabic, dextran and pullulan; and inorganic excipients, examples of which include silicate derivatives such as soft silicic acid anhydride, synthetic aluminum silicate and magnesium aluminometasilicate, phosphates such as calcium phosphate, carbonates such as calcium carbonate, and sulfates such as calcium sulfate.

Examples of suitable lubricants include: stearic acid; metal salts of stearic acid such as calcium stearate and magnesium stearate; talc; colloidal silica; waxes such as bee gum and spermaceti; boric acid; adipic acid; sulfates such as sodium sulfate; glycol; fumaric acid; sodium benzoate; DL-leucine; sodium salts of an aliphatic acid; lauryl sulfates such as sodium lauryl sulfate and magnesium lauryl sulfate; silicic acid derivatives such as silicic anhydride and silicic acid hydrate; and starch derivatives exemplified above as examples of suitable excipients.

Examples of suitable binders include polyvinylpyrrolidone, Macrogol and compounds similar to those exemplified above as suitable excipients.

Examples of suitable disintegrants include compounds similar to those exemplified above as suitable excipients and chemically modified starch or cellulose derivatives such as sodium cross carmellose, sodium carboxymethyl starch and crosslinked polyvinylpyrrolidone.

Examples of suitable stabilizers include: paraoxybenzoate esters such as methylparaben and propylparaben; alcohols such as chlorobutanol, benzyl alcohol and phenylethyl alcohol; benzalkonium chloride; phenol derivatives such as phenol and cresol; thimerosal; dehydroacetic acid; and sorbic acid.

Examples of suitable corrigents include sweeteners, acidifiers and flavors commonly employed for this purpose.

The dose of the compound of formula (I) or a pharmacologically acceptable salt, ester or other derivative thereof according to the present invention will vary depending on a variety of factors including the condition to be treated, the age of the patient and the administration route. When administered orally, it is administered to an adult in an amount of 0.1 mg (preferably 0.5 mg) a day as a lower limit and 2000 mg (preferably 500 mg) a day as an upper limit. It can be administered in from one to several portions depending on the condition of the patient. When administered intravenously, it is administered to an adult in an amount of 0.01 mg (preferably 0.05 mg) a day as a lower limit and 200 mg (preferably 50 mg) a day as an upper limit. It can be administered in from one to several portions depending on the condition of the patient.

[Best mode for carrying out the invention]

The following examples, preparative examples, formulation examples and test examples are intended to further illustrate the present invention and are not intended to limit the scope of this invention in any way.

[Examples]

Example 1

4-(3-Aminopropyl)-2-(4-fluorophenyl)-3-(pyridin-4-yl)-1*H*-pyrrole (Compound No. 1-97)

1(i) 4-Ethoxycarbonyl-2-(4-fluorophenyl)-3-(pyridin-4-yl)-1*H*-pyrrole

36 ml (54.7 mmol) of a 1.53 N solution of butyllithium in hexane were added to 240 ml of tetrahydrofuran. A solution of 15.90 g (54.7 mmol) of α-(p-toluene-sulfonyl)-4-fluorobenzylisonitrile in 120 ml of tetrahydrofuran was then added to the resulting solution at -45°C, followed by stirring of the resulting mixture for 10 minutes at the same temperature. At the end of this time, 25.00 g (273 mmol) of 95% lithium bromide were added, the resulting mixture was stirred for 30 minutes and then a solution of 8.73 g (49.2 mmol) of ethyl 3-(4-pyridyl)acrylate in 120 ml of tetrahydrofuran was added. The resulting mixture was stirred at the same temperature for 1 hour and then the cooling bath was removed and the mixture was stirred at room temperature for a further 1 hour. At the end of this time, 500 ml of water were added and the resulting mixture was extracted with ethyl acetate. The organic extract was washed with water and then dried over anhydrous sodium sulfate, after which it was concentrated by evaporation under reduced pressure to afford a solid. The solid was washed with diethyl ether to give 13.61 g (yield 89%) of the title compound as a pale yellow powder. $^1$H-Nuclear magnetic resonance spectrum (500 MHz, $CDCl_3$) δ ppm:

8.84 (1H, broad singlet); 8.51 (2H, doublet, J=7 Hz);
7.58 (1H, doublet, J=3 Hz); 7.21 (2H, doublet, J=6 Hz);
7.11 (2H, doublet of doublets, J=9 Hz, 5 Hz);
6.97 (2H, triplet, J=9 Hz); 4.18 (2H, quartet, J=7 Hz);
1.20 (3H, triplet, J=7 Hz).

1(ii) 2-(4-Fluorophenyl)-4-hydroxymethyl-3-(pyridin-4-yl)-1*H*-pyrrole

121.4 ml (121.4 mmol) of a 1M solution of diisobutylaluminium hydride in toluene were slowly added dropwise to a solution of 12.56 g (40.47 mmol) of 4-ethoxycarbonyl-2-(4-fluorophenyl)-3-(pyridin-4-yl)-1*H*-pyrrole [which was obtained in step (i) above] in 630 ml of tetrahyrofuran with ice-cooling. After completing the addition, the cooling bath was removed and the mixture was stirred at room temperature for 5 hours. At the end of this time, a saturated aqueous solution of ammonium chloride was added to the reaction mixture and the resulting mixture was extracted with ethyl acetate. The organic extract was washed with water, dried over anhydrous magnesium sulfate and then concentrated by evaporation under reduced pressure to give 10.80 g (yield 99%) of the title compound as a pale orange powder. $^1$H-Nuclear magnetic resonance spectrum (500 MHz, $CDCl_3$) δ ppm:

10.52 (1H, broad singlet); 8.46 (2H, doublet, J=6 Hz);
7.31 (2H, doublet, J=6 Hz);
7.26 (2H, doublet of doublets, J=9 Hz, 5 Hz);
6.98 (2H, triplet, J=9 Hz); 6.95 (1H, doublet, J=3 Hz);
4.50 (2H, singlet); 3.49 (1H, broad singlet).

1 (iii) 2-(4-Fluorophenyl)-4-formyl-3-(pyridin-4-yl)-1*H*-pyrrole

118.00 g (440 mmol) of manganese dioxide were added to a solution of 10.73 g (40 mmol) of 2-(4-fluorophenyl)-4-hydroxymethyl-3-(pyridin-4-yl)-1*H*-pyrrole [prepared as described in step (ii) above] in 120 ml of dimethyl sulfoxide, after which the resulting mixture was stirred at 50°C overnight. At the end of this time, the reaction mixture was filtered, water was added to the filtrate, and this was then extracted with ethyl acetate. The organic extract was washed with water, dried over anhydrous magnesium sulfate and then concentrated by evaporation under reduced pressure to afford a solid which was washed with diethyl ether to give 7.35 g (yield 69%) of the title compound as a brown powder. $^1$H-Nuclear magnetic resonance spectrum (500 MHz, $CDCl_3$-$CD_3OD$) δ ppm:

9.76 (1H, singlet); 8.48 (2H, doublet, J=6 Hz) ;
7.59 (1H, singlet); 7.26 (2H, doublet, J=6 Hz);
7.19 (2H, doublet of doublets, J=9 Hz, 6 Hz) ;
7.01 (2H, triplet, J=9 Hz).

1(iv) 3-[2-(4-Fluorophenyl)-3-(pyridin-4-yl)-1*H*-pyrrol-4-yl]acrylonitrile

2.92 ml (18.02 mmol) of diethylphosphonoacetonitrile were added to 16 ml of tetrahydrofuran and the resulting solution was added to a suspension of 786 mg (18.02 mmol) of 55% sodium hydride in 60 ml of tetrahydrofuran with ice-cooling, and the resulting mixture was then stirred at room temperature for 1.5 hours. At the end of this time, a suspension of 4.00 g (15.02 mmol) of 2-(4-fluorophenyl)-4-formyl-3-(pyridin-4-yl)-1*H*-pyrrole [prepared as described in step (iii) above] in 30 ml of tetrahydrofuran was added to this mixture and the resulting mixture was stirred at room temperature for 1 hour. At the end of this time, water was added to the reaction mixture and the resulting mixture was extracted with ethyl acetate. The organic extract was washed with water, dried over anhydrous magnesium sulfate and then concentrated by evaporation under reduced pressure to afford a solid which was washed with diethyl ether to give 2.94 g (yield 68%) of the title compound as a pale brown powder.
$^1$H-Nuclear magnetic resonance spectrum (500 MHz, $CDCl_3$-DMSO-$d_6$) $\delta$ ppm:

11.30 (1H, broad singlet); 8.57 (2H, doublet, J=6 Hz);
7.24 (1H, doublet, J=4 Hz); 7.21 (1H, doublet, J=17 Hz);
7.19 (2H, doublet of doublets, J=9 Hz, 5 Hz);
7.13 (2H, doublet, J=6 Hz); 6.97 (2H, doublet, J=9 Hz);
5.39 (1H, doublet, J=17 Hz).

1(v) 4-(2-Cyanoethyl)-2-(4-fluorophenyl)-3-(pyridin-4-yl)-1*H*-pyrrole

2.94 g (10.16 mmol) of 3-[2-(4-fluorophenyl)-3-(pyridin-4-yl)-1*H*-pyrrol-4-yl]acrylonitrile [prepared as described in step (iv) above] were dissolved in a mixture of 20 ml of tetrahydrofuran and 20 ml of methanol, after which 2.94 g of 10% palladium on carbon were added to the solution. The mixture was stirred under a hydrogen atmosphere at room temperature for 8 hours. At the end of this time, the reaction mixture was filtered and the filtrate was concentrated under reduced pressure. The resulting residue was washed with a mixture of 30 ml of diethyl ether and 30 ml of ethanol to give 1.80 g (yield 61%) of the title compound as a pale orange powder.
1*H*-Nuclear magnetic resonance spectrum (500 MHz, $CDCl_3$) $\delta$ ppm:

8.55 (2H, doublet, J=6 Hz); 8.27 (1H, broad singlet);
7.13 (2H, doublet of doublets, J=9 Hz, 5 Hz);
7.12 (2H, doublet, J=6 Hz); 6.98 (2H, triplet, J=9 Hz);
6.89 (1H, doublet, J=3 Hz); 2.88 (2H, triplet, J=7 Hz);
2.43 (2H, triplet, J=7 Hz).

1 (vi) 4-(3-Aminopropyl)-2-(4-fluorophenyl)-3-(pyridin-4-yl)-1*H*-pyrrole

1.80 g (6.18 mmol) of 4-(2-cyanoethyl)-2-(4-fluorophenyl)-3-(pyridin-4-yl)-1*H*-pyrrole [prepared as described in step (v) above] were added to 100 ml of tetrahydrofuran and the resulting solution was slowly added dropwise with stirring at room temperature to a suspension of 469 mg (12.36 mmol) of lithium aluminum hydride in 150 ml of tetrahydrofuran. This mixture was stirred at 60°C for 30 minutes. At the end of this time, the reaction mixture was cooled to room temperature, 25 ml of water and 0.5 ml of a 15% aqueous solution of sodium hydroxide were slowly added to the reaction mixture and the resulting mixture was then filtered. The filtrate was concentrated under reduced pressure, after which the residue was dehydrated by azeotropic distillation under reduced pressure using toluene. This azeotropic distillation was carried out three times to give 1.71 g (yield 94%) of the title compound as a white powder.
Melting point: 198 - 199°C
$^1$H-Nuclear magnetic resonance spectrum (500 MHz, DMSO-$d_6$) $\delta$ ppm:

11.16 (1H, broad singlet); 8.46 (2H, doublet, J=6 Hz);
7.18 (2H, doublet of doublets, J=9 Hz, 6 Hz);
7.13 (2H, doublet, J=6 Hz); 7.11 (2H, triplet, J=9 Hz);
6.74 (1H, doublet, J=3 Hz); 2.49 (2H, triplet, J=7 Hz);
2.41 (2H, triplet, J=8 Hz); 1.48 (2H, quintet, J=8 Hz).

Example 2

2-(4-Fluorophenyl)-3-(pyridin-4-yl)-4-(3-trifluoroacetylaminopropyl)-1*H*-pyrrole (Compound No. 1-2533)

3.3 ml (23.2 mmol) of trifluoroacetic anhydride were added to a solution of 685 mg (2.32 mmol) of 4-(3-aminopropyl)-2-(4-fluorophenyl)-3-(pyridin-4-yl)-1*H*-pyrrole (which was prepared as described in Example 1 above) in 70 ml of tetrahydrofuran. The resulting mixture was stirred at room temperature for 30 minutes. At the end of this time, 200 ml of a saturated aqueous solution of sodium hydrogencarbonate were added to the reaction mixture, and then this was extracted with ethyl acetate. The organic extract was washed with water and concentrated by evaporation under reduced pressure. The resulting residue was purified by chromatography on a silica gel column using a 1:1 by volume mixture of hexane and ethyl acetate as the eluant to give 280 mg (yield 31%) of the title compound as a white powder.
Melting point: 229 - 230°C
$^1$H-Nuclear magnetic resonance spectrum (500 MHz, $CDCl_3$) δ ppm:

10.09 (1H, broad singlet); 8.49 (2H, doublet, J=6 Hz);
7.93 (1H, broad singlet);
7.18 (2H, doublet of doublets, J=9 Hz, 5 Hz);
7.13 (2H, doublet, J=6 Hz); 6.94 (2H, triplet, J=9 Hz);
6.74 (1H, doublet, J=3 Hz); 3.29 (2H, doublet of doublets, J=13 Hz, 7 Hz); 2.56 (2H, triplet, J=8 Hz);
1.74 (2H, quintet, J=8 Hz).

Example 3

4-(3-Acetylaminopropyl)-2-(4-fluorophenyl)-3-(pyridin-4-yl)-1*H*-pyrrole (Compound No. 1-2528)

In a similar manner to that described in Example 2 above, a reaction was carried out using acetic anhydride instead of trifluoroacetic anhydride to give the title compound (yield 99%) as a pale yellow powder.
Melting point: 218 -220°C
$^1$H-Nuclear magnetic resonance spectrum (400 MHz, DMSO-$d_6$) δ ppm:

11.19 (1H, broad singlet); 8.46 (2H, doublet, J=6 Hz);
7.77 (1H, broad triplet, J=5 Hz);
7.18 (2H, doublet of doublets, J=9 Hz, 5 Hz);
7.15-7.08 (4H, multiplet); 6.77 (1H, d, 2 Hz);
2.99 (2H, doublet of doublets, J=13 Hz, 7 Hz);
2.39 (2H, triplet, J=8 Hz); 1.75 (3H, singlet);
1.53 (2H, quintet, J=7 Hz).

Example 4

2-(4-Fluorophenyl)-4-(3-methylaminopropyl)-3-(pyridin-4-yl)-1*H*-pyrrole (Compound No. 1-101)

4(i) 4-[3-(t-Butoxycarbonylamino)propyl]-2-(4-fluorophenyl)-3-(pyridin-4-yl)-1*H*-pyrrole

In a similar manner to that described in Example 2 above, a reaction was carried out using di-t-butyl dicarbonate instead of trifluoroacetic anhydride and using methanol instead of tetrahydrofuran to give the title compound (yield 68%) as a white powder.
$^1$H-Nuclear magnetic resonance spectrum (400 MHz, $CDCl_3$) δ ppm:

8.51 (2H, doublet, J=6 Hz); 8.26 (1H, broad singlet);
7.15-7.12 (4H, multiplet); 6.96 (2H, triplet, J=9 Hz);
6.75 (1H, doublet, J=3 Hz); 4.45 (1H, broad singlet);
3.12-3.09 (2H, multiplet); 2.54 (2H, triplet, J=8 Hz);
1.67-1.63 (2H, multiplet); 1.43 (9H, singlet).

4 (ii) 2-(4-Fluorophenyl)-4-(3-methylaminopropyl)-3-(pyridin-4-yl)-1*H*-pyrrole

400 mg (1.01 mmol) of 4-[3-(t-butoxycarbonylamino)propyl]-2-(4-fluorophenyl)-3-(pyridin-4-yl)-1*H*-pyrrole [prepared as described in step 4(i) above] were added to a suspension of 154 mg of lithium aluminum hydride in 8 ml of tetrahydrofuran. The resulting mixture was heated under reflux for 2 hours. At the end of this time, the reaction mixture was worked up and then purified in the same manner as that described in Example 1(vi) above to give 300 mg (yield 96%) of the title compound as a white powder.
Melting point: 193 -198°C
$^1$H-Nuclear magnetic resonance spectrum (400 MHz, $CDCl_3$) δ ppm:

8.51 (2H, doublet, J=6 Hz); 8.34 (1H, broad singlet);
7.17-7.12 (4H, multiplet); 6.96 (2H, triplet, J=9 Hz);
6.74 (1H, doublet, J=3 Hz); 2.57 (2H, triplet, J=7 Hz);
2.54 (2H, triplet, J=7 Hz); 2.38 (3H, singlet);
1.69 (2H, quintet, J=7 Hz).

Example 5

4-Aminomethyl-2-(4-fluorophenyl)-3-(pyridin-4-yl)-1*H*-pyrrole dihydrochloride (Dihydrochloride of Compound No. 1-95)

5(i) 5-(4-Pluorophenyl)-4-(pyridin-4-yl)-1*H*-pyrrole-3-carboxaldehyde *O*-methyloxime

0.83 g (9.58 mmol) of *O*-methylhydroxylamine hydrochloride and 1.56 ml (11.2 mmol) of triethylamine were added to a solution of 0.85 mg (3.2 mmol) of 2-(4-fluorophenyl)-4-formyl-3-(pyridin-4-yl)-1*H*-pyrrole [prepared as described in Example 1(iii) above] in 17 ml of methanol. The resulting mixture was heated under reflux for 30 minutes, after which water was added to the reaction mixture which was then extracted with ethyl acetate. The organic extract was washed with water and concentrated by evaporation under reduced pressure. The resulting residue was purified by chromatography on a silica gel column using ethyl acetate as the eluant to afford 728 mg (yield 77%) of the title compound as a pale yellow powder.
Melting point: 242 - 248°C
$^1$H-Nuclear magnetic resonance spectrum (500 MHz, DMSO-$d_6$) δ ppm:

11.88 (1H, broad singlet); 8.54 (0.2H, doublet, J=6 Hz);
8.49 (1.8H, doublet, J=6 Hz); 7.88 (0.9H, singlet);
7.76 (0.1H, singlet); 7.34 (0.9H, singlet);
7.25-7.12 (6H, multiplet); 6.98 (0.1H, singlet);
3.92 (0.4H, singlet); 3.71 (2.6H, singlet).

5(ii) 4-Aminomethyl-2-(4-fluorophenyl)-3-(pyridin-4-yl)-1*H*-pyrrole dihydrochloride

10 ml of 10% hydrochloric acid and 0.50 g of 10% palladium on carbon were added to a solution of 497 mg (1.68 mmol) of 5-(4-fluorophenyl)-4-(pyridin-4-yl)-1*H*-pyrrole-3-carboxaldehyde 0-methyloxime [which was prepared as described in step 5(i) above] in 10 ml of methanol. The mixture was stirred under a hydrogen atmosphere at room temperature for 2 hours. At the end of this time, the reaction mixture was filtered and the filtrate thus obtained was concentrated by evaporation under reduced pressure. The residue was washed with diisopropylether and then with diethylether to give 391 mg (yield 68%) of the title compound as a yellowish orange powder. Melting point: 235 -240°C (decomposition)
$^1$H-Nuclear magnetic resonance spectrum (500 MHz, $CD_3OD$) δ ppm:

11.85 (1H, broad singlet); 8.65 (2H, doublet, J=7 Hz);
7.81 (2H, doublet, J=7 Hz);
7.32 (2H, doublet of doublets, J=9 Hz, 5 Hz);
7.24 (1H, doublet, J=3 Hz); 7.14 (2H, triplet, J=9 Hz);
4.22 (2H, singlet).

Example 6

2-(4-Fluorophenyl)-3-(pyridin-4-yl)-4-(2,4,6-trifluorobenzoylaminomethyl)-1*H*-pyrrole (Compound No. 1-2535)

265 µl (1.905 mmol) of triethylamine and 96 mg (0.493 mmol) of 2,4,6-trifluorobenzoyl chloride were added to a suspension of 162 mg (0.476 mmol) of 4-aminomethyl-2-(4-fluorophenyl)-3-(pyridin-4-yl)-1*H*-pyrrole dihydrochloride [prepared as described in Example 5 above] in 3.3 ml of tetrahydrofuran. The resulting mixture was stirred at room temperature for 4 hours. At the end of this time, water was added to the reaction mixture and then this was extracted with ethyl acetate. The organic extract was washed with water, dried over anhydrous sodium sulfate and then concentrated by evaporation under reduced pressure. The residue thus obtained was washed with diethylether to give 78 mg (yield 38%) of the title compound as a pale yellow powder.
Melting point: 243 -245°C (decomposition)
$^1$H-Nuclear magnetic resonance spectrum (500 MHz, DMSO-$d_6$) δ ppm:

11.41 (1H, broad singlet); 8.99 (1H, broad triplet, J=5 Hz);
8.46 (2H, doublet, J=6 Hz); 7.29-7.19 (4H, multiplet);
7.19-7.13 (4H, multiplet); 6.94 (1H, doublet, J=3 Hz);
4.27 (2H, doublet, J=5 Hz).

Example 7

2-(4-Fluorophenyl)-3-(pyridin-4-yl)-4-(2,3,5,6-tetrafluoropyridin-4-yl)-1*H*-pyrrole (Compound No. 1-2538)

7(i) 2-(4-Fluorophenyl)-3-(pyridin-4-yl)-1*H*-pyrrole

15.00 g (48.3 mmol) of 4-ethoxycarbonyl-2-(4-fluorophenyl)-3-(pyridin-4-yl)-1*H*-pyrrole [prepared as described in Example 1(i) above] were dissolved in a mixture of 90 ml of acetic acid, 30 ml of sulfuric acid and 60 ml of water and the resulting solution was stirred at 100°C for 16 hours. At the end of this time, the reaction mixture was cooled to room temperature and then made alkaline with a 10% aqueous solution of sodium hydroxide. The resulting mixture was extracted with ethyl acetate and the organic extract was washed with water, dried over anhydrous sodium sulfate and then concentrated by evaporation under reduced pressure to give 11.40 g (yield 99%) of the title compound as a pale red powder.
$^1$H-Nuclear magnetic resonance spectrum (500 MHz, $CDCl_3$) δ ppm:

9.78 (1H, broad singlet); 8.42 (2H, doublet, J=7 Hz);
7.37 (2H, doublet of doublets, J=9 Hz, 5 Hz);
7.22 (2H, doublet, J=6 Hz); 7.06 (2H, triplet, J=9 Hz);
6.90 (1H, triplet, J=3 Hz); 6.47 (1H, triplet, J=3 Hz).

7 (ii) 2-(4-Fluorophenyl)-3-(pyridin-4-yl)-1-triisopropylsilyl-1*H*-pyrrole

31 ml (47.4 mmol) of a 1.57 N solution of butyllithium in hexane were added to a solution of 11.30 g (47.4 mmol) of 2-(4-fluorophenyl)-3-(pyridin-4-yl)-1*H*-pyrrole [prepared as described in step 7(i) above] in 300 ml of tetrahydrofuran at - 78°C and the mixture was stirred for 10 minutes. At the end of this time, 13.4 ml (49.8 mmol) of triisopropylsilyl triflate were added to the reaction mixture at the same temperature. After removal of the cooling bath, the mixture was stirred at room temperature for 30 minutes. 200 ml of water and 300 ml of a saturated aqueous solution of sodium hydrogencarbonate were then added to the reaction mixture before extracting with ethyl acetate. The organic extract was washed with water, dried over anhydrous sodium sulfate and then concentrated by evaporation under reduced pressure to give 18.70 g (quantitative yield) of the title compound as a reddish purple oil.
$^1$H-Nuclear magnetic resonance spectrum (500 MHz, DMSO-$d_6$) δ ppm:

8.25 (2H, doublet, J=6 Hz);
7.39 (2H, doublet of doublets, J=9 Hz, 6 Hz);
7.28 (2H, triplet, J=9 Hz); 7.00 (1H, doublet, J=3 Hz);
6.91(2H, doublet, J=7 Hz); 6.71 (1H, doublet, J=3 Hz);
1.15-1.05 (3H, multiplet); 0.98 (18H, doublet, J=8 Hz).

7(iii) 4-Bromo-2-(4-fluorophenyl)-3-(pyridin-4-yl)-1-triisopropylsilyl-1*H*-pyrrole

A suspension of 8.61 g (47.4 mmol) of N-bromosuccinimide in 100 ml of tetrahydrofuran was added to a solution of 18.70 g (47.4 mmol) of 2-(4-fluorophenyl)-3-(pyridin-4-yl)-1-triisopropylsilyl-1*H*-pyrrole [prepared as described in step 7(ii) above] in 300 ml of tetrahydrofuran at -78°C, after which the resulting mixture was stirred at the same temperature for 6 hours. After removal of the cooling bath, the mixture was then stirred at room temperature for a further 1 hour. At the end of this time, 400 ml of hexane was added to the reaction mixture and the insoluble material was removed by filtration. The resulting filtrate was concentrated by evaporation under reduced pressure and the residue thus obtained was purified by chromatography on a silica gel column using a 2:1 by volume mixture of hexane and ethyl acetate as the eluant to give 9.57 g (yield 43%) of the title compound as pale yellow prismatic crystals.
$^1$H-Nuclear magnetic resonance spectrum (500 MHz, DMSO-$d_6$) δ ppm:

8.36 (2H, doublet, J=6 Hz);
7.34 (2H, doublet of doublets, J=9 Hz, 6 Hz);
7.18 (2H, triplet, J=9 Hz); 7.12 (1H, singlet);
7.04 (2H, doublet, J=6 Hz); 1.16-1.08 (3H, multiplet);
0.99 (18H, doublet, J=8 Hz).

7 (iv) 2-(4-Fluorophenyl)-3-(pyridin-4-yl)-4-(2,3,5,6-tetrafluoropyridin-4-yl)-1*H*-pyrrole

1.8 ml (2.75 mmol) of 1.54 N solution of t-butyllithium in pentane were added to a solution of 650 mg (1.37 mmol) of 4-bromo-2-(4-fluorophenyl)-3-(pyridin-4-yl)-1-triisopropylsilyl-1*H*-pyrrole [prepared as described in step 7(iii) above] in 15 ml of tetrahydrofuran at -78°C and the mixture was stirred for 10 minutes. At the end of this time, 165 μl (1.51 mmol) of pentafluoropyridine were added to the reaction mixture. The cooling bath was then removed and the mixture was stirred for 30 minutes. At the end of this time, 70 ml of water and 50 ml of a saturated aqueous solution of sodium hydrogencarbonate were added to the reaction mixture, and then this was extracted with ethyl acetate. The organic extract was washed with water and then concentrated by evaporation under reduced pressure. The residue thus obtained was purified by chromatography on a silica gel column using a 4:1 by volume mixture of hexane and ethyl acetate as the eluant to give 346 mg (yield 65%) of the title compound as a pale yellow powder.
Melting point: >300°
$^1$H-Nuclear magnetic resonance spectrum (500 MHz, $CD_3OD$) δ ppm:

8.34 (2H, doublet, J=8 Hz); 7.32 (1H, singlet);
7.30 (2H, doublet of doublets, J=11 Hz, 7 Hz);
7.09 (2H, triplet, J=11 Hz); 7.06(2H, doublet, J=8 Hz).

Example 8

(±)-[5-(4-Fluorophenyl)-4-(pyridin-4-yl)-1*H*-pyrrol-3-yl]-(pyridin-4-yl)methanol (Compound No. 1-2530)

8(i) (±)-[5-(4-Fluorophenyl)-4-(pyridin-9-yl)-1-triisopropylsilyl-1*H*-pyrrol-3-yl]-(pyridin-4-yl)methanol

In a similar manner to that described in Example 7(iv) above, a reaction was carried out using 4-formylpyridine instead of pentafluoropyridine to give the title compound (yield 56%) as a pale yellow oil.
$^1$H-Nuclear magnetic resonance spectrum (500 MHz, $CD_3OD$) δ ppm:

8.39 (2H, doublet, J=7 Hz); 8.21 (2H, doublet, J=6 Hz);
7.38 (2H, doublet, J=7 Hz);
7.29 (2H, doublet of doublets, J=9 Hz, 6 Hz);
7.17 (2H, doublet, J=6 Hz); 7.03 (2H, triplet, J=9 Hz);
6.67 (1H, singlet); 5.82 (1H, singlet);
1.14-1.05 (3H, multiplet); 1.01 (9H, doublet, J=7 Hz);
1.00 (9H, doublet, J=7 Hz).

8 (ii) (±)-[ 5-(4-Fluorophenyl)-4-(pyridin-4-yl)-1*H*-pyrrol-3-yl]-(pyridin-4-yl)methanol

0.59 ml (0.59 mmol) of a 1 M solution of tetrabutylammonium fluoride in tetrahydrofuran were added to a solution of 148 mg (0.295 mmmol) of [5-(4-fluorophenyl)-4-(pyridin-4-yl)-1-triisopropylsilyl-1*H*-pyrrol-3-yl]-(pyridin-4-yl)methanol [prepared as described in step 8(i) above] in 3 ml of tetrahydrofuran. The resulting mixture was stirred at room temperature for 10 minutes, after which water was added to the reaction mixture and the resulting mixture was acidified with 1 N hydrochloric acid and then extracted with ethyl acetate. The water layer was made alkaline with potassium carbonate and then extracted with ethyl acetate. The organic extract was washed with water, dried over anhydrous sodium sulfate and then concentrated by evaporation under reduced pressure. The residual oil thus obtained was solidified by the addition of isopropanol to give 83 mg (yield 82%) of the title compound as a white powder.
Melting point: 202 - 205°C
$^1$H-Nuclear magnetic resonance spectrum (500 MHz, DMSO-$d_6$) δ ppm:

11.39 (1H, broad singlet); 8.43 (2H, doublet, J=6 Hz);
8.41 (2H, doublet, J=6 Hz); 7.26-7.21 (4H, multiplet);
7.18(2H, doublet of doublets, J=9 Hz, 6 Hz);
7.13 (2H, triplet, J=9 Hz); 6.55 (1H, doublet, J=3 Hz);
5.77 (1H, doublet, J=5 Hz); 5.54 (1H, doublet, J=4 Hz).

Example 9

4-(1-Acetyl-l,2,3,6-tetrahydropyridin-4-yl)-2-(4-fluorophenyl)-3-(pyridin-4-yl)-1*H*-pyrrole (Compound No. 1-2525)

9(i) 4-(1-Benzyl-4-hydroxypiperidin-4-yl)-2-(4-fluorophenyl)-3-(pyridin-4-yl)-1-triisopropylsilyl-1*H*-pyrrole

In a similar manner to that described in Example 7(iv) above, a reaction was carried out using 1-benzylpiperidine-4-one instead of pentafluoropyridine to afford the title compound (yield 61%) as white needle-like crystals.
$^1$H-Nuclear magnetic resonance spectrum (500 MHz, $CD_3OD$) δ ppm:

8.21 (2H, doublet, J=6 Hz); 7.35-7.15 (9H, multiplet);
6.93 (2H, triplet, J=9 Hz); 6.90 (1H, singlet);
3.49 (2H, singlet); 2.58-2.41 (4H, broad multiplet);
2.00-1.91 (2H, broad multiplet);
1.83-1.74 (2H, broad multiplet);
1.20-1.10 (3H, multiplet); 1.06 (18H, doublet, J=7 Hz).

9(ii) 2-(4-Fluorophenyl)-4-(4-hydroxypiperidin-4-yl)-3-(pyridin-4-yl)-1-triisopropylsilyl-1*H*-pyrrole

0.58 g of 10% palladium on carbon were added to a solution of 0.58 g (1.00 mmol) of 4-(1-benzyl-4-hydroxypiperidin-4-yl)-2-(4-fluorophenyl)-3-(pyridin-9-yl)-1-triisopropylsilyl-lH-pyrrole [prepared as described in step 9(i) above] in 15 ml of methanol. The resulting mixture was stirred under a hydrogen atmosphere at room temperature for 5 hours. At the end of this time, the reaction mixture was filtered and the filtrate was concentrated by evaporation under reduced pressure to give 483 mg (yield 98%) of the title compound as a white amorphous solid.
$^1$H-Nuclear magnetic resonance spectrum (500 MHz, $CD_3OD$) δ ppm:

8.22 (2H, doublet, J=7 Hz); 7.27 (2H, doublet, J=6 Hz);
7.21 (2H, doublet of doublets, J=9 Hz, 6 Hz);
6.94 (2H, triplet, J=9 Hz); 6.92 (1H, singlet);
2.98-2.90 (2H, multiplet); 2.76-2.68 (2H, multiplet);
1.93-1.84 (2H, multiplet); 1.77-1.69 (2H, multiplet);
1.20-1.11 (3H, multiplet); 1.07 (18H, doublet, J=7 Hz).

9(iii) 4-(1-Acetyl-1,2,3,6-tetrahydropyridin-4-yl)-2-(4-fluorophenyl)-3-(pyridin-4-yl)-1*H*-pyrrole

1.4 ml (8.70 mmol) of triethylsilane and 2.25 ml (29.0 mmol) of trifluoroacetic acid were added to a solution of 3.58 g (7.25 mmol) of 2-(4-fluorophenyl)-4-(4-hydroxypiperidin-4-yl)-3-(pyridin-4-yl)-1-triisopropylsilyl-1*H*-pyrrole [prepared as described in step 9(ii) above] in 40 ml of dichloromethane, after which the resulting mixture was stirred at room temperature for 5 hours. At the end of this time, a saturated aqueous solution of sodium hydrogencarbonate was added to the reaction mixture and then this was extracted with ethyl acetate. The organic extract was washed with water, dried over anhydrous sodium sulfate and then concentrated by evaporation under reduced pressure to afford 3.90 g of dehydrated product as a yellow amorphous solid.

All of the 3.90 g of dehydrated product thus obtained was dissolved in 80 ml of tetrahydofuran, and then 0.76

ml (8.0 mmol) of acetic anhydride and 2.25 ml (16.0 mmol) of triethylamine were added to the solution. The resulting mixture was stirred at room temperature for 20 minutes, after which 16 ml (16.0 mmol) of a 1 M solution of tetrabutylammonium fluoride in tetrahydrofuran were added before stirring for a further 10 minutes. 150 ml of a saturated aqueous solution of sodium hydrogencarbonate were added to the reaction mixture and then this was extracted with ethyl acetate. After washing the organic extract with water, 1 N hydrochloric acid was added thereto. The water layer was washed with ethyl acetate, made alkaline with potassium carbonate and finally extracted with ethyl acetate. The organic extract thus obtained was washed with water, dried over anhydrous sodium sulfate and then concentrated by evaporation under reduced pressure to give 1.78 g (yield 68%) of the title compound as a pale brown powder.
Melting point: 262 - 264°C (decomposition)
$^1$H-Nuclear magnetic resonance spectrum (500 MHz, DMSO-$d_6$) δ ppm:

11.43 (1H, broad singlet); 8.48-8.44 (2H, multiplet);
7.20-7.09 (6H, multiplet); 6.96 (0.5H, doublet, J=3 Hz);
6.94 (0.5H, doublet, J=3 Hz);
5.32-5.29 (0.5H, broad multiplet);
5.29-5.25 (0.5H, broad multiplet);
3.92-3.88 (1H, multiplet); 3.87-3.84 (1H, multiplet);
3.50 (1H, triplet, J=6 Hz); 3.46 (1H, triplet, J=6 Hz);
2.22-2.17 (1H, broad multiplet);
2.16-2.11 (1H, broad multiplet);
2.00(1.5H, singlet); 1.96 (1.5H, singlet).

Example 10

2-(4-Fluorophenyl)-3-(pyridin-4-yl)-4-(1,2,3,6-tetrahydropyridin-4-yl)-1*H*-pyrrole dihydrochloride (dihydrochloride of Compound No. 1-143)

249 mg (0.689 mmol) of 4-(1-acetyl-1,2,3,6-tetrahydropyridin-4-yl)-2-(4-fluorophenyl)-3-(pyridin-4-yl)-1*H*-pyrrole [prepared as described in Example 9 above] were dissolved in a mixture of 2.5 ml of methanol and 2.5 ml of water, after which 2.43 ml (9.646 mmol) of a 4 N solution of hydrogen chloride in dioxane were added to the solution. The resulting mixture was heated under reflux for 11 hours, after which the reaction mixture was concentrated by evaporation under reduced pressure and the residual solid was washed with hot isopropanol to give 216 mg (yield 80%) of the title compound as a pale yellow powder.
Melting point: 290 - 295°C (decomposition)
$^1$H-Nuclear magnetic resonance spectrum (500 MHz, $CD_3OD$) δ ppm:

11.63 (1H, broad singlet); 8.54 (2H, doublet, J=7 Hz);
7.74 (2H, doublet, J=7 Hz);
7.29 (2H, doublet of doublets, J=9 Hz, 5 Hz);
7.14 (2H, triplet, J=9 Hz); 7.06 (1H, doublet, J=2 Hz);
5.51-5.47 (1H, broad multiplet);
3.72 (2H, doublet, J=3 Hz); 3.42 (2H, triplet, J=6 Hz);
2.66-2.55 (2H, broad multiplet).

Example 11

2-(4-Fluorophenyl)-4-(piperidin-4-yl)-3-(pyridin-4-yl)-1*H*-pyrrole (Compound No. 1-142)

120 mg of 10% palladium on carbon were added to a solution of 120 mg (0.306 mmol) of 2-(4-fluorophenyl)-3-(pyridin-4-yl)-4-(1,2,3,6-tetrahydropyridin-4-yl)-1*H*-pyrrole dihydrochloride [prepared as described in Example 10 above] in 10 ml of methanol. The resulting mixture was stirred under a hydrogen atmosphere at room temperature for 1 hour, after which the reaction mixture was filtered. The filtrate thus obtained was concentrated by evaporation under reduced pressure. Water was added to the resulting residue and then this mixture was made alkaline with a 1 N aqueous solution of sodium hydroxide. The resulting precipitate was collected by filtration to give 84 mg (yield 85%) of the title compound as a pale red powder. Melting point: 265 - 267°C (decomposition)
$^1$H-Nuclear magnetic resonance spectrum (500 MHz, DMSO-$d_6$) δ ppm:

11.17 (1H, broad singlet); 8.48 (2H, doublet, J=6 Hz);

7.20-7.04 (6H, multiplet); 6.73 (1H, doublet, J=3 Hz);
2.88 (2H, doublet, J=12 Hz);
2.46 (1H, triplet of triplets, J=12 Hz, 4 Hz);
2.38 (2H, triplet, J=12 Hz); 1.58 (2H, doublet, J=13 Hz);
1.35 (2H, double doublet of triplets, J=13 Hz, 12 Hz, 4 Hz).

Example 12

1-(1-Benzylpiperidin-4-yl)-3-(4-fluorophenyl)-2-(pyridin-4-yl)-1*H*-pyrrole (Compound No. 2-147)

12(i) 1-Benzyl-4-(pyridin-4-yl)methyleneaminopiperidine

8.57 ml (42.01 mmol) of 4-amino-1-benzylpiperidine were added to a solution of 3.95 ml (42.01 mmol) of 4-formylpyridine in 5 ml of ethanol, and the resulting mixture was then heated under reflux for 1 hour. At the end of this time, the solvent was distilled off from the reaction mixture by evaporation under reduced pressure to afford 11.78 g (quantitative yield) of the title compound as a pale brown powder.
$^1$H-Nuclear magnetic resonance spectrum. (400 MHz, $CDCl_3$) $\delta$ ppm:

8.68 (2H, doublet, J=6 Hz); 8.30 (1H, singlet);
7.60 (2H, doublet, J=6 Hz); 7.38-7.22 (5H, multiplet);
3.56 (2H, singlet); 3.37-3.27 (1H, multiplet);
3.00-2.90 (2H, multiplet); 2.23-2.10 (2H, multiplet);
1.96-1.83 (2H, multiplet); 1.79-1.60 (2H, multiplet).

12 (ii) (±)-α-(1-Benzylpiperidin-4-yl)amino-α-(pyridin-4-yl)acetonitrile

A mixture of 11.73 g (42.00 mmol) of 1-benzyl-4-(pyridin-4-yl)-methyleneaminopiperidine [prepared as described in step 12(i) above] and 9.22 ml (65.67 mmol) of 95% trimethylsilyl cyanide was stirred at 100°C for 3 hours and then it was cooled to room temperature. 100 ml of methanol were added to the reaction mixture and the resulting mixture was then stirred for 30 minutes. At the end of this time, the solvent was distilled off from the reaction mixture by evaporation under reduced pressure to afford 12.87 g (quantitative yield) of the title compound as a brown oil.
$^1$H-Nuclear magnetic resonance spectrum (400 MHz, $CDCl_3$) $\delta$ ppm:

8.67 (2H, doublet, J=6 Hz); 7.50 (2H, doublet, J=6 Hz);
7.37-7.22 (5H, multiplet); 4.85 (1H, singlet);
3.53 (2H, singlet); 2.98-2.77 (4H, multiplet);
2.33-1.44 (5H, multiplet).

12(iii) 1-(1-Benzylpiperidin-4-yl)-3-(4-fluorophenyl)-2-(pyridin-4-yl)-1*H*-pyrrole

14.25 g (46.51 mmol) of α-(1-benzylpiperidin-4-yl)amino-α-(pyridin-4-yl)acetonitrile [prepared as described in step 12(ii) above] and 6.98 g (46.51 mmol) of 3-(4-fluorophenyl)acrolein were dissolved in 145 ml of N,N-dimethylacetamide, 1.29 g (9.30 mmol) of potassium carbonate were added to the solution, and then this was stirred at room temperature for 5 hours. At the end of this time, water was added to the reaction mixture and then this was extracted with ethyl acetate. The organic extract was washed with water and then concentrated by evaporation under reduced pressure. 150 ml of ethylene glycol were added to the residue thus obtained and the resulting mixture was stirred at 180°C for 1 hour. After cooling to room temperature, a saturated aqueous solution of sodium hydrogencarbonate was added to the reaction mixture which was then extracted with ethyl acetate. The organic extract was washed with water and then concentrated by evaporation under reduced pressure. The residue thus obtained was purified by chromatography on a silica gel column using a 1:3 by volume mixture of hexane and ethyl acetate as the eluant to give 10.31 g (yield 49%) of the title compound as a pale brown powder.
Melting point: 132 - 139°C
$^1$H-Nuclear magnetic resonance spectrum (400 MHz, $CDCl_3$) $\delta$ ppm:

8.61 (2H, doublet, J=6 Hz); 7.37-7.23 (5H, multiplet);
7.13 (2H, doublet, J=6 Hz);
7.03 (2H, doublet of doublets, J=9 Hz, 5 Hz);
6.96 (1H, doublet, J=3 Hz); 6.87 (2H, triplet, J=9 Hz);

6.38 (1H, doublet, J=3 Hz); 3.87-3.80 (1H, multiplet);
3.50 (2H, singlet); 2.97 (2H, doublet, J=12 Hz);
2.12-1.56 (6H, multiplet).

Example 13

3-(4-Fluorophenyl)-1-(piperidin-4-yl)-2-(pyridin-4-yl)-1*H*-pyrrole (Compound No. 2-142)

In a similar manner to that described in Example 9 (ii) above, 1-(1-benzylpiperidin-4-yl)-3-(4-fluorophenyl)-2-(pyridin-4-yl)-1*H*-pyrrole (prepared as described in Example 12 above) was debenzylated to give the title compound (yield 30%) as a white powder.
Melting point: 191 - 192°C
$^1$H-Nuclear magnetic resonance spectrum (400 MHz, $CDCl_3$) δ ppm:

8.62 (2H, doublet, J=6 Hz); 7.15 (2H, doublet, J=6 Hz);
7.04 (2H, doublet of doublets, J=9 Hz, 5 Hz);
6.97 (1H, doublet, J=3 Hz); 6.88 (2H, triplet, J=9 Hz);
6.39 (1H, doublet, J=3 Hz); 3.97-3.86 (1H, multiplet);
3.17 (2H, doublet, J=12 Hz);
2.58 (2H, doublet of triplets, J=12 Hz, 3 Hz);
1.98-1.82 (4H, multiplet).

Example 14

3-(4-Fluorophenyl)-1-(1-methylpiperidin-4-yl)-2-(pyridin-4-yl)-1*H*-pyrrole (Compound No. 2-144)

In a similar manner to the procedures described in Examples 4(i) and 4(ii) above, 3-(4-fluorophenyl)-1-(piperidin-4-yl)-2-(pyridin-4-yl)-1*H*-pyrrole (prepared as described in Example 13 above) was subjected consecutively to N-t-butoxycarbonylation and reduction to give the title compound (total yield for the two steps: 55%) as a white powder.
Melting point: 187- 189°C
$^1$H-Nuclear magnetic resonance spectrum (400 MHz, $CDCl_3$) δ ppm:

8.62 (2H, doublet, J=6 Hz); 7.14 (2H, doublet, J=6 Hz);
7.04 (2H, doublet of doublets, J=9 Hz, 5 Hz);
6.96 (1H, doublet, J=3 Hz); 6.87 (2H, triplet, J=9 Hz);
6.39 (1H, doublet, J=3 Hz); 3.87-3.77 (1H, multiplet);
2.93 (2H, doublet, J=12 Hz); 2.29 (3H, singlet);
2.13-1.85 (6H, multiplet).

Example 15

1-(1-Acetylpiperidin-4-yl)-3-(4-fluorophenyl)-2-(pyridin-4-yl)-1*H*-pyrrole (Compound No. 2-2524)

In a similar manner to that described in Example 3 above, a reaction was carried out using 3-(4-fluorophenyl)-1-(piperidin-4-yl)-2-(pyridin-4-yl)-1*H*-pyrrole (prepared as described in Example 13 above) to give the title compound (yield 98%) as a white powder.
Melting point: 223 - 224°C
$^1$H-Nuclear magnetic resonance spectrum (400 MHz, $CDCl_3$) δ ppm:

8.64 (2H, doublet, J=6 Hz); 7.15 (2H, doublet, J=6 Hz);
7.04 (2H, doublet of doublets, J=9 Hz, 5 Hz);
6.88 (2H, triplet, J=9 Hz); 6.87 (1H, doublet, J=3 Hz);
6.41 (1H, doublet, J=3 Hz); 4.84-4.74 (1H, multiplet);
4.05 (1H, doublet of triplets, J=12 Hz, 4 Hz) ;
3.95-3.83 (1H, multiplet); 3.08-2.97 (1H, multiplet);
2.58-2.48 (1H, multiplet); 2.13 (3H, singlet);
2.04-1.78 (4H, multiplet).

Example 16

3-(4-Fluorophenyl)-1-[1-(2-nitroethyl)piperidin-4-yl]-2-(pyridin-4-yl)-1*H*-pyrrole (Compound No. 2-2537)

186 mg (1.40 mmol) of 2-nitroethyl acetate were added to a solution of 300 mg (0.93 mmol) of 3-(4-fluorophenyl)-1-(piperidin-4-yl)-2-(pyridin-4-yl)-1*H*-pyrrole (prepared as described in Example 13 above) in 6 ml of ethanol. The resulting mixture was stirred at room temperature for 30 minutes, after which the solvent was distilled off under reduced pressure. A saturated aqueous solution of sodium hydrogencarbonate was added to the residue and then this was extracted with ethyl acetate. The organic extract was washed with water, dried over anhydrous magnesium sulfate and then concentrated by evaporation under reduced pressure. The residual solid was washed with diethylether to give 280 mg (yield 76%) of the title compound as a white powder.
Melting point: 172- 173°C
$^1$H-Nuclear magnetic resonance spectrum (400 MHz, $CDCl_3$) $\delta$ ppm:

8.61 (2H, doublet, J=6 Hz); 7.13 (2H, doublet, J=6 Hz);
7.04 (2H, doublet of doublets, J=9 Hz, 5 Hz);
6.93 (1H, doublet, J=3 Hz); 6.87 (2H, triplet, J=9 Hz);
6.39 (1H, doublet, J=3 Hz); 4.47 (2H, triplet, J=6 Hz);
3.82 (1H, doublet of triplets, J=12 Hz, 4 Hz);
3.03-2.92 (4H, multiplet); 2.16-1.85 (6H, multiplet).

Example 17

3-(4-Fluorophenyl)-1-[3-(morpholin-1-yl)propyl]-2-(pyridin-4-yl)-1*H*-pyrrole (Compound No. 2-125)

In a similar manner to the procedures described in Examples 12(i), 12(ii) and 12(iii) above, reactions were carried out using 1-(3-aminopropyl)morpholine instead of 4-amino-1-benzylpiperidine to give the title compound (total yield 13%) as pale brown needle-like crystals.
Melting point: 111 - 112°C
$^1$H-Nuclear magnetic resonance spectrum (500 MHz, $CDCl_3$) $\delta$ ppm:

8.59 (2H, doublet, J=6 Hz); 7.18 (2H, doublet, J=6 Hz);
7.06 (2H, doublet of doublets, J=9 Hz, 5 Hz);
6.89 (2H, triplet, J=9 Hz); 6.84 (1H, doublet, J=3 Hz);
6.34 (1H, doublet, J=3 Hz); 3.96 (2H, triplet, J=7 Hz);
3.64 (2H, triplet, J=5 Hz); 2.33-2.22 (4H, multiplet);
2.20 (2H, triplet, J=7 Hz); 1.72 (2H, quintet, J=7 Hz).

Example 18

(±)-3-(4-Fluorophenyl)-2-(pyridin-4-yl)-1-(pyrrolidin-3-yl)-1*H*-pyrrole (Compound No. 2-139)

18(i) (±)-1-[1-(t-Butoxycarbonyl)pyrrolidin-3-yl)-3-(4-fluorophenyl)-2-(pyridin-4-yl)-1*H*-pyrrole

In a similar manner to the procedures described in Examples 12(i), 12(ii) and 12(iii) above, reactions were carried out using (±)-3-amino-1-(t-butoxycarbonyl)pyrrolidine as a starting material instead of 4-amino-1-benzylpiperidine to give the title compound (total yield 31%) as a brown amorphous solid.
$^1$H-Nuclear magnetic resonance spectrum (400 MHz, $CDCl_3$) $\delta$ ppm:

8.63 (2H, doublet, J=6 Hz); 7.17 (2H, doublet, J=6 Hz);
7.03 (2H, doublet of doublets, J=9 Hz, 5 Hz);
6.93-6.84 (3H, multiplet); 6.41 (1H, doublet, J=3 Hz);
4.70-4.55 (1H, multiplet); 3.80-3.36 (4H, multiplet);
2.30-2.12 (2H, multiplet); 1.47 (9H, singlet).

18(ii) (±)-3-(4-Fluorophenyl)-2-(pyridin-4-yl)-1-(pyrrolidin-3-yl)-1*H*-pyrrole

6.01 ml (24.05 mmol) of a 4N solution of hydrogen chloride in dioxane were added to a solution of 1.96 g

(4.81 mmol) of (±)-1-[1-(t-butoxycarbonyl)pyrrolidin-3-yl]-3-(4-fluorophenyl)-2-(pyridin-4-yl)-1*H*-pyrrole [prepared as described in step 18(i) above] in 10 ml of ethanol. The resulting mixture was stirred at 50°C for 30 minutes, after which the solvent was distilled off under reduced pressure. A saturated aqueous solution of sodium hydrogencarbonate was added to the residue thus obtained, and then this was extracted with ethyl acetate. The organic extract was washed with water, dried over anhydrous magnesium sulfate and then concentrated by evaporation under reduced pressure to give 1.29 g (yield 87%) of the title compound as a pale yellow powder.
Melting point: 145 - 147°C
$^1$H-Nuclear magnetic resonance spectrum (500 MHz, $CDCl_3$) δ ppm:

8.61 (2H, doublet, J=6 Hz); 7.16 (2H, doublet, J=6 Hz);
7.05 (2H, doublet of doublets, J=9 Hz, 5 Hz);
7.00 (1H, doublet, J=3 Hz); 6.88 (2H, triplet, J=9 Hz);
6.39 (1H, doublet, J=3 Hz); 4.61-4.53 (1H, multiplet);
3.30-3.17 (2H, multiplet); 3.10-2.97 (2H, multiplet);
2.28-2.18 (2H, multiplet); 2.11-2.01 (2H, multiplet).

Example 19

(±)-3-(4-Pluorophenyl)-1-(piperidin-3-yl)-2-(pyridin-4-yl)-1*H*-pyrrole (Compound No. 2-141)

In a similar manner to the procedures described in Examples 18(i) and 18(ii) above, reactions were carried out using (±)-3-amino-1-(t-butoxycarbonyl)piperidine as a starting material instead of (±)-3-amino-1-(t-butoxycarbonyl) pyrrolidine to give the title compound (total yield 21%) as a white powder.
Melting point 189 - 191°C
$^1$H-Nuclear magnetic resonance spectrum (400 MHz, $CDCl_3$) δ ppm:

8.61 (2H, doublet, J=6 Hz); 7.15 (2H, doublet, J=6 Hz);
7.04 (2H, doublet of doublets, J=9 Hz, 5 Hz);
6.93 (1H, doublet, J=3 Hz); 6.87 (2H, triplet, J=9 Hz);
6.38 (1H, doublet, J=3 Hz);
3.93 (1H, triplet of triplets, J=11 Hz, 4 Hz);
3.20-3.12 (1H, multiplet); 3.05-2.96 (1H, multiplet);
2.83 (1H, triplet, J=11 Hz);
2.58 (1H, doublet of triplets, J=12 Hz, 3 Hz);
2.17-2.08 (1H, multiplet); 1.90-1.76 (2H, multiplet);
1.58-1.43 (1H, multiplet).

Example 20

3-(4-Fluorophenyl)-1-(piperidin-4-yl)methyl-2-(pyridin-4-yl)-1*H*-pyrrole (Compound No. 2-169)

In a similar manner to the procedures described in Examples 18(i) and 18(ii) above, reactions were carried out using 4-aminomethyl-1-(t-butoxycarbonyl)-piperidine as a starting material instead of 3-amino-1-(t-butoxycarbonyl) pyrrolidine to give the title compound (total yield 4%) as a white powder.
Melting point 174 - 176°C
$^1$H-Nuclear magnetic resonance spectrum. (400 MHz, $CDCl_3$) δ ppm:

8.59 (2H, doublet, J=6 Hz); 7.15 (2H, doublet, J=6 Hz);
7.06 (2H, doublet of doublets, J=9 Hz, 5 Hz);
6.88 (2H, triplet, J=9 Hz); 6.79 (1H, doublet, J=3 Hz);
6.34 (1H, doublet, J=3 Hz); 3.75 (2H, doublet, J=7 Hz);
3.05-2.95 (2H, multiplet);
2.45 (2H, doublet of triplets, J=12 Hz, 2 Hz);
1.62-1.52 (1H, multiplet); 1.46-1.38 (2H, multiplet);
1.05-0.93 (2H, multiplet).

Example 21

1-(Azetidin-3-yl)-3-(4-fluorophenyl)-2-(pyridin-4-yl)-1*H*-pyrrole dihydrochloride (Dihydrochloride of Compound No. 2-136)

21(i) 1-(1-Diphenylmethylazetidin-3-yl)-3-(4-fluorophenyl)-2-(pyridin-4-yl)-1*H*-pyrrole

In a similar manner to the procedures in Examples 12(i), 12(ii) and 12(iii) above, reactions were carried out using 3-amino-1-diphenylmethylazetidine as a starting material instead of 4-amino-1-benzylpiperidine to afford the title compound (total yield 20%) as a pale brown powder.
$^1$H-Nuclear magnetic resonance spectrum (400 MHz, $CDCl_3$) $\delta$ ppm:

8.56 (2H, doublet, J=6 Hz); 7.43-7.18 (11H, multiplet);
7.07-7.02 (4H, multiplet); 6.88 (2H, triplet, J=9 Hz);
6.43 (1H, doublet, J=3 Hz); 4.74-4.66 (1H, multiplet);
4.41 (1H, singlet); 3.58-3.52 (2H, multiplet);
3.34-3.28 (2H, multiplet).

21(ii) 1-(Azetidin-3-yl)-3-(4-fluorophenyl)-2-(pyridin-4-yl)-1*H*-pyrrole dihydrochloride

0.45 ml of concentrated hydrochloric acid and 0.12g of 20% palladium hydroxide on carbon were added to a solution of 1.20 g (2.61 mmol) of 1-(1-diphenylmethylazetidin-3-yl)-3-(4-fluorophenyl)-2-(pyridin-4-yl)-1*H*-pyrrole [prepared as described in step 21(i) above] in 12 ml of ethanol. The resulting mixture was stirred under a hydrogen atmosphere at 50°C for 20 hours. After cooling to room temperature, the reaction mixture was then filtered and the filtrate was concentrated by evaporaration under reduced pressure. The residual solid was washed with diethylether to give 0.91 g (yield 95%) of the title compound as a pale yellow amorphous solid.
$^1$H-Nuclear magnetic resonance spectrum (400 MHz, DMSO-$d_6$) $\delta$ ppm:

9.84-9.64 (1H, broad singlet); 9.58-9.37 (1H, broad singlet);
9.80 (2H, doublet, J=6 Hz); 7.94 (1H, doublet, J=3 Hz);
7.64 (2H, doublet, J=6 Hz); 7.25 (2H, triplet, J=9 Hz);
7.20-7.07 (4H, multiplet); 7.62 (1H, doublet, J=3 Hz);
5.19-5.08 (1H, multiplet); 4.43-4.23 (4H, multiplet).

Example 22

1-(3-Aminopropyl)-3-(4-fluorophenyl)-2-(pyridin-4-yl)-1*H*-pyrrole (Compound No. 2-97)

22(i) 1-(3-Benzyloxypropyl)-3-(4-fluorophenyl)-2-(pyridin-4-yl)-1*H*-pyrrole

In a similar manner to the procedures described in Examples 12(i), 12(ii) and 12(iii), reactions were carried out using 3-benzyloxypropylamine as a starting material instead of 4-amino-1-benzylpiperidine to give the title compound (total yield 34%) as a brown oil.
$^1$H-Nuclear magnetic resonance spectrum (400 MHz, $CDCl_3$) $\delta$ ppm:

8.55 (2H, doublet, J=6 Hz); 7.38-7.23 (5H, multiplet);
7.16 (2H, doublet, J=6 Hz);
7.07 (2H, doublet of doublets, J=9 Hz, 5 Hz);
6.89 (2H, triplet, J=9 Hz); 6.81 (1H, doublet, J=3 Hz);
6.34 (1H, doublet, J=3 Hz); 4.41 (2H, singlet);
4.03 (2H, triplet, J=7 Hz); 3.36 (2H, triplet, J=7 Hz);
1.86 (2H, quintet, J=7 Hz).

22(ii) 3-(4-Fluorophenyl)-1-(3-hydroxypropyl)-2-(pyridin-4-yl)-1*H*-pyrrole

In a similar manner to that described in Example 9(ii) above, 1-(3-benzyloxypropyl)-3-(4-fluorophenyl)-2-(pyridin-4-yl)-1*H*-pyrrole [prepared as described in step 22(i) above] was debenzylated to afford the title compound (yield 55%) as a white powder.

$^1$H-Nuclear magnetic resonance spectrum (400 MHz, $CDCl_3$) δ ppm:

8.59 (2H, doublet, J=6 Hz); 7.19 (2H, doublet, J=6 Hz);
7.05 (2H, doublet of doublets, J=9 Hz, 5 Hz);
6.89 (2H, triplet, J=9 Hz); 6.87 (1H, doublet, J=3 Hz);
6.36 (1H, doublet, J=3 Hz); 4.05 (2H, triplet, J=7 Hz);
3.55 (2H, triplet, J=7 Hz); 1.80 (2H, quintet, J=7 Hz).

22(iii) 3-(4-Fluorophenyl)-2-(pyridin-4-yl)-1-[3-(p-toluenesulfonyloxy)propyl]-1*H*-pyrrole

1.99 g (6.09 mmol) of p-toluenesulfonic anhydride and 0.85ml (6.09 mmol) of triethylamine were added to a solution of 1.64 g (5.53 mmol) of 3-(4-fluorophenyl)-1-(3-hydroxypropyl)-2-(pyridin-4-yl)-1*H*-pyrrole [prepared as described in step 22(ii) above] in 16 ml of dichloromethane. The resulting mixture was stirred at room temperature for 2 hours, after which the solvent was distilled off by evaporation under reduced pressure. The resulting residue was purified by chromatography on a silica gel column using a 1:1 by volume mixture of hexane and ethyl acetate as the eluant to afford 1.33 g (yield 53%) of the title compound as a yellow powder.
$^1$H-Nuclear magnetic resonance spectrum (400 MHz, $CDCl_3$) δ ppm:

8.57 (2H, doublet, J=6 Hz); 7.74 (2H, doublet, J=8 Hz);
7.35 (2H, doublet, J=8 Hz); 7.10 (2H, doublet, J=6 Hz);
7.03 (2H, doublet of doublets, J=9 Hz, 5 Hz);
6.88 (2H, triplet, J=9 Hz); 6.71 (1H, doublet, J=3 Hz);
6.29 (1H, doublet, J=3 Hz); 3.98 (2H, triplet, J=7 Hz);
3.89 (2H, triplet, J=7 Hz); 2.46 (3H, singlet);
1.84 (2H, quintet, J=7 Hz).

22(iv) 1-(3-Azidopropyl)-3-(4-fluorophenyl)-2-(pyridin-4-yl)-1*H*-pyrrole

0.38 g (5.90 mmol) of sodium azide were added to a solution of 1.33 g (2.95 mmol) of 3-(4-fluorophenyl)-2-(pyridin-4-yl)-1-[3-(p-toluenesulfonyloxy)propyl]-1*H*-pyrrole [prepared as described in step 22(iii) above] in 13 ml of dimethylsulfoxide. The resulting mixture was stirred at 70°C for 1 hour. At the end of this time water was added to the reaction mixture and then this was extracted with dichloromethane. The organic extract was washed with water and then concentrated under reduced pressure. The residue thus obtained was purified by chromatography on a silica gel column using a 1:1 by volume mixture of hexane and ethylacetate as the eluant to afford 100 mg (yield 11%) of the title compound as a pale brown powder.
$^1$H-Nuclear magnetic resonance spectrum (400 MHz, $CDCl_3$) δ ppm:

8.16 (2H, doublet, J=6 Hz); 7.17 (2H, doublet, J=6 Hz);
7.07 (2H, doublet of doublets, J=9 Hz, 5 Hz);
6.89 (2H, triplet, J=9 Hz); 6.83 (1H, doublet, J=3 Hz);
6.37 (1H, doublet, J=3 Hz); 4.00 (2H, triplet, J=7 Hz);
3.18 (2H, triplet, J=7 Hz); 1.78 (2H, quintet, J=7 Hz).

22(v) 1-(3-Aminopropyl)-3-(4-fluorophenyl)-2-(pyridin-4-yl)-1*H*-pyrrole

20 mg of 10% palladium on carbon were added to a solution of 100 mg (0.31 mmol) of 1-(3-azidopropyl)-3-(4-fluoropenyl)-2-(pyridin-4-yl)-1*H*-pyrrole [prepared as described in Step 22(iv) above] in 2 ml of ethanol. The resulting mixture was stirred under a hydrogen atmosphere at room temperature for 1 hour, after which the reaction mixture was filtered and the filtrate was concentrated by evaporation under reduced pressure. The residual solid was washed with diethylether to give 43 mg (yield 47%) of the title compound as a pale yellow powder.
Melting point: 219 - 222°C
$^1$H-Nuclear magnetic resonance spectrum (400 MHz, $CDCl_3$) δ ppm:

8.61 (2H, doublet, J=6 Hz); 7.74-7.40 (2H, broad singlet);
7.27 (2H, doublet, J=6 Hz); 7.12-7.00 (5H, multiplet);
6.38 (1H, doublet, J=3 Hz); 3.97 (2H, triplet, J=7 Hz);
2.60 (2H, triplet, J=7 Hz); 1.73 (2H, quintet, J=7 Hz).

Example 23

2-(4-Fluorophenyl)-3-(pyridin-4-yl)-4-[3-(thiomorpholin-1-yl)propyl]-1*H*-pyrrole (Compound No. 1-129)

23(i) Ethyl 3-[2-(4-fluorophenyl)-3-(pyridin-4-yl)-1*H*-pyrrol-4-yl]acrylate

In a similar manner to that described in Example 1(iv) above, a reaction was carried out using ethyl diethylphosphonoacetate instead of diethylphosphonoacetonitrile to afford the title compound (yield 60%) as a brown powder.
$^1$H-Nuclear magnetic resonance spectrum (400 MHz, $CDCl_3$) δ ppm:

8.64 (1H, broad singlet); 8.56 (2H, doublet, J=6 Hz);
7.54 (1H, doublet, J=16 Hz); 7.16 (2H, doublet, J=6 Hz);
7.16-7.13 (3H, multiplet); 6.99 (2H, triplet, J=9 Hz);
6.07 (1H, doublet, J=16 Hz); 4.20 (2H, quartet, J=7 Hz);
1.28 (3H, triplet, J=7 Hz).

23(ii) 4-(2-Ethoxycarbonylethyl)-2-(4-fluorophenyl)-3-(pyridin-4-yl)-1*H*-pyrrole

In a similar manner to that described in Example 1(v) above, ethyl 3-[2-(4-fluorophenyl)-3-(pyridin-4-yl)-1*H*-pyrrol-4-yl]acrylate [prepared as described in step 23 (i) above] was reduced to afford the title compound (yield 73%) as a pale brown powder.
$^1$H-Nuclear magnetic resonance spectrum (400 MHz, $CDCl_3$) δ ppm:

8.51 (2H, doublet, J=4 Hz); 8.33 (1H, broad singlet);
7.15 (2H, doublet, J=4 Hz); 7.16-7.12 (2H, multiplet);
6.96 (2H, triplet, J=9 Hz); 6.74 (1H, doublet, J=3 Hz);
4.11 (2H, quartet, J=7 Hz); 2.85 (2H, triplet, J=8 Hz);
2.50 (2H, triplet, J=8 Hz); 1.23 (3H, triplet, J=7 Hz).

23 (iii) 2-(4-Fluorophenyl)-4-(3-hydroxypropyl)-3-(pyridin-4-yl)-1*H*-pyrrole

In a similar manner to that described in Example 1(vi) above, 4-(2-ethoxycarbonylethyl)-2-(4-fluorophenyl)-3-(pyridin-4-yl)-1*H*-pyrrole [prepared as described in step 23 (ii) above] was reduced using lithium aluminum hydride to afford the title compound (yield 91%) as a pale brown powder.
$^1$H-Nuclear magnetic resonance spectrum (400 MHz, $CDCl_3$) δ ppm:

8.51 (2H, doublet, J=5 Hz); 8.23 (1H, broad singlet);
7.17-7.12 (4H, multiplet); 7.00 (2H, triplet, J=9 Hz);
6.75 (1H, doublet, J=3 Hz); 3.65 (2H, triplet, J=6 Hz);
2.61 (2H, triplet, J=8 Hz); 1.79-1.72 (2H, multiplet);
1.61 (1H, broad singlet).

23 (iv) 2-(4-Fluorophenyl)-3-(pyridin-4-yl)-4-[3-(p-toluenesulfonyloxy)propyl]-1*H*-pyrrole

In a similar manner to that described in Example 22(iii) above, p-toluenesulfonylation was carried out using 2-(4-fluorophenyl)-4-(3-hydroxypropyl)-3-(pyridin-4-yl)-1*H*-pyrrole [prepared as described in step 23(iii) above] to afford the title compound (yield 71%) as a pale yellow powder.
$^1$H-Nuclear magnetic resonance spectrum (400 MHz, $CDCl_3$) δ ppm:

8.49 (2H, doublet, J=6 Hz); 8.20 (1H, broad singlet);
7.59 (2H, doublet, J=8 Hz); 7.33 (2H, doublet, J=8 Hz);
7.12 (2H, doublet of doublets, J=9 Hz, 5 Hz) ;
7.08 (2H, doublet, J=6 Hz); 6.96 (2H, triplet, J=9 Hz);
6.69 (1H, doublet, J=3 Hz); 4.00 (2H, triplet, J=6 Hz);
2.56 (2H, triplet, J=8 Hz); 2.44 (3H, singlet);
1.81-1.74 (2H, multiplet).

23(v) 2-(4-Fluorophenyl)-3-(pyridin-4-yl)-4-[3-(thiomorpholin-1-yl)propyl]-1*H*-pyrrole

163 mg (1.18 mmol) of potassium carbonate and 122μl (1.29 mmol) of thiomorpholine were added to a solution of 450 mg (1.07 mmol) of 2-(4-fluorophenyl)-3-(pyridin-4-yl)-4-[3-(p-toluenesulfonyloxy)propyl]-1*H*-pyrrole [prepared as described in step 23(iv) above] in 50 ml of acetonitrile. The resulting mixture was heated under reflux overnight. After cooling the reaction mixture to room temperature, water was added and then this was extracted with ethyl acetate. The organic extract was washed with water and then concentrated by evaporation under reduced pressure. The residue thus obtained was purified by chromatography on a silica gel column using a 95:5 by volume mixture of ethyl acetate and methanol as the eluant to give 340 mg (yield 83%) of the title compound as a pale yellow powder.
Melting point: 205 - 207°C
$^1$H-Nuclear magnetic resonance spectrum (400 MHz, $CDCl_3$) δ ppm:

8.51 (2H, doublet, J=6 Hz); 8.20 (1H, broad singlet);
7.14 (2H, doublet of doublets, J=9 Hz, 5 Hz);
7.13 (2H, doublet, J=6 Hz); 6.96 (2H, triplet, J=9 Hz);
6.72 (1H, doublet, J=3 Hz); 2.65 (8H, singlet);
2.50 (2H, triplet, J=8 Hz); 2.35 (2H, triplet, J=8 Hz);
1.66 (2H, quintet, J=8 Hz).

Example 24

4-[3-(1-Benzylpiperazin-4-yl)propyl]-2-(4-fluorophenyl)-3-(pyridin-4-yl)-1*H*-pyrrole (Compound No. 1-135)

In a similar manner to that described in Example 23(v) above, a reaction was carried out using 1-benzylpiperazine instead of thiomorpholine to give the title compound (yield 52%) as a white powder.
Melting point: 153 - 154°C
$^1$H-Nuclear magnetic resonance spectrum (400 MHz, $CDCl_3$) δ ppm:

8.50 (2H, doublet, J=5 Hz); 8.28 (1H, broad singlet);
7.31 (4H, doublet, J=4 Hz); 7.28-7.22 (1H, multiplet);
7.13 (2H, doublet of doublets, J=9 Hz, 5 Hz);
7.13 (2H, doublet, J=5 Hz); 6.96 (2H, triplet, J=9 Hz);
6.72 (1H, doublet, J=2 Hz); 3.50 (2H, singlet);
2.50 (2H, triplet, J=8 Hz); 2.44 (8H, broad singlet);
2.34 (2H, triplet, J=8 Hz); 1.69 (2H, quintet, J=8 Hz).

Example 25

2-(4-Pluorophenyl)-4-[3-(piperazin-1-yl)propyl]-3-(pyridin-4-yl)-1*H*-pyrrole (Compound No. 1-133)

In a similar manner to that described in Example 9(ii) above, 4-[3-(1-benzylpiperazin-4-yl)propyl]-2-(4-fluorophenyl)-3-(pyridin-4-yl)-1*H*-pyrrole (prepared as described in Example 24 above) was debenzylated to give the title compound (yield 98%) as a white powder.
Melting point: 162 - 164°C
$^1$H-Nuclear magnetic resonance spectrum (400 MHz, $CDCl_3$) δ ppm:

8.51 (2H, doublet, J=6 Hz); 8.20 (1H, broad singlet);
7.14 (2H, doublet of doublets, J=9 Hz, 5 Hz);
7.14 (2H, doublet, J=6 Hz); 6.96 (2H, triplet, J=9 Hz);
6.73 (1H, doublet, J=2 Hz); 2.87 (4H, triplet, J=5 Hz);
2.51 (2H, triplet, J=8 Hz); 2.36 (4H, broad singlet);
2.32 (2H, triplet, J=8 Hz); 1.70 (2H, quintet, J=8 Hz).

Example 26

4-(3-Dimethylaminopropyl)-2-(4-fluorophenyl)-3-(pyridin-4-yl)-1*H*-pyrrole (Compound No. 1-109)

In a similar manner to that described in Example 23(v) above, a reaction was carried out using dimethylamine

instead of thiomorpholine to give the title compound (yield 66%) as a white powder.
Melting point: 177 - 179°C
$^1$H-Nuclear magnetic resonance spectrum (500 MHz, $CDCl_3$) δ ppm:

8.51 (2H, doublet, J=5 Hz); 8.24 (1H, broad singlet);
7.15-7.13 (2H,multiplet); 7.14 (2H, doublet, J=5 Hz);
6.96 (2H, triplet, J=9 Hz); 6.74 (1H, doublet, J=2 Hz);
2.52 (2H, triplet, J=8 Hz); 2.28 (2H, triplet, J=8 Hz);
2.19 (6H, singlet); 1.74-1.59 (2H, multiplet).

Example 27

2-(4-Fluorophenyl)-4-[3-(morpholin-1-yl)propyl]-3-(pyridin-4-yl)-1*H*-pyrrole (Compound No. 1-125)

In a similar manner to that described in Example 23(v) above, a reaction was carried out using morpholine instead of thiomorpholine to give the title compound (yield 45%) as a pale yellow powder.
Melting point: 182 - 183°C
$^1$H-Nuclear magnetic resonance spectrum (400 MHz, $CDCl_3$) δ ppm:

8.62 (1H, broad singlet); 8.50 (2H, doublet, J=5 Hz);
7.17-7.12 (2H, multiplet); 7.14 (2H, doublet, J=5 Hz) ;
6.96 (2H, triplet, J=9 Hz); 6.73 (1H, doublet, J=3 Hz);
3.70-3.68 (4H, multiplet); 2.53 (2H, triplet, J=8 Hz);
2.41-2.34 (4H, multiplet); 2.33 (2H, triplet, J=8 Hz);
1.68 (2H, quintet, J=8 Hz).

Example 28

2-(4-Fluorophenyl)-4-[3-(piperidin-1-yl)propyl]-3-(pyridin-4-yl)-1*H*-pyrrole (Compound No. 1-121)

In a similar manner to that described in Example 23(v) above, a reaction was carried out using piperidine instead of thiomorpholine to give the title compound (yield 63%) as a pale yellow powder.
Melting point: 195 - 197°C
$^1$H-Nuclear magnetic resonance spectrum (500 MHz, $CDCl_3$) δ ppm:

8.50 (2H, doublet, J=6 Hz); 8.19 (1H, broad singlet);
7.14 (2H, doublet of doublets, J=9 Hz, 6 Hz);
6.96 (2H, triplet, J=9 Hz); 6.74 (1H, doublet, J=2 Hz);
2.50 (2H, triplet, J=8 Hz); 2.40-2.30 (4H, multiplet);
1.75-1.55 (8H, multiplet) ; 1.43-1.42 (2H, multiplet).

Example 29

2-(4-Fluorophenyl)-4-[3-(1-methylpiperazin-4-yl)propyl]-3-(pyridin-4-yl)-1*H*-pyrrole (Compound No. 1-134)

In a similar manner to that described in Example 23(v) above, a reaction was carried out using 1-methylpiperazine instead of thiomorpholine to give the title compound (yield 64%) as a pale yellow powder.
Melting point: 190 - 192°C
$^1$H-Nuclear magnetic resonance spectrum (400 MHz, $CDCl_3$) δ ppm:

8.65 (1H, broad singlet); 8.49 (2H, doublet, J=6 Hz);
7.16-7.12 (2H, multiplet); 7.14 (2H, doublet, J=6 Hz);
6.95 (2H, triplet, J=9 Hz); 6.73 (1H, doublet, J=3 Hz);
2.52 (2H, triplet, J=8 Hz); 2.53-2.36 (8H, multiplet);
2.36 (2H, triplet, J=8 Hz); 2.28 (3H, singlet);
1.70 (2H, quintet, J=8 Hz).

Example 30

1-(3-Dimethylaminopropyl)-3-(4-fluorophenyl)-2-(pyridin-4-yl)-1*H*-pyrrole (Compound No. 2-109)

In a similar manner to the procedures described in Examples 12(i), 12(ii) and 12(iii) above, reactions were carried out using 3-dimethylaminopropylamine as a starting material instead of 4-amino-1-benzylpiperidine to give the title compound (total yield 9%) as a white powder.
Melting point: 93 - 95°C
$^1$H-Nuclear magnetic resonance spectrum (400 MHz, $CDCl_3$) δ ppm:

8.59 (2H, doublet, J=6 Hz); 7.18 (2H, doublet, J=6 Hz);
7.07 (2H, doublet of doublets, J=9 Hz, 6 Hz);
6.89 (2H, triplet, J=9 Hz); 6.84 (1H, doublet, J=3 Hz);
6.35 (1H. doublet. J=3 Hz): 3.93 (2H, triplet, J=7 Hz);
2.14 (2H, triplet, J=7 Hz); 2.11 (6H, singlet);
1.71 (2H, quintet, J=7 Hz).

Example 31

2- (4-Pluorophenyl)-3,4-bis(pyridin-4-yl)-1*H*-pyrrole (Compound No. 1-159)

450 mg (1.15 mmol) of 2-(4-fluorophenyl)-3-(pyridin-4-yl)-4-(1,2,3,6-tetrahydropyridin-4-yl)-1*H*-pyrrole dihydrochloride [prepared as described in Example 10 above] were suspended in a mixture of 25 ml of xylene and 2 ml of methanol and 450 mg of 10% palladium on carbon and 320 mg (2.30 mmol) of sodium carbonate were added to the suspension. The resulting mixture was heated under reflux for 4 hours. At the end of this time, the reaction mixture was filtered and the resulting filtrate was concentrated by evaporation under reduced pressure. The residue thus obtained was purified by chromatography on a silica gel column using a 10:1:1 by volume mixture of ethyl acetate, methanol and isopropylamine as the eluant to give 192 mg (yield 53%) of the title compound as a pale yellow powder.
Melting point: 325 - 330°C (decomposition)
$^1$H-Nuclear magnetic resonance spectrum (500 MHz, DMSO-$d_6$) δ ppm:

11.87 (1H, broad singlet); 8.47 (2H, doublet, J=6 Hz);
8.35 (2H, doublet, J=6 Hz); 7.39 (1H, doublet, J=3 Hz);
7.21 (2H, doublet of doublets, J=9 Hz, 5 Hz);
7.16 (2H, triplet, J=9 Hz); 7.08 (2H, doublet, J=6 Hz);
7.03 (2H, doublet, J=6 Hz).

Example 32

2-(4-Fluorophenyl)-4-(1-methyl-1,2,3,6-tetrahydropyridin-4-yl)-3-(pyridin-4-yl)-1*H*-pyrrole (Compound No. 1-145)

32(i) 2-(4-Fluorophenyl)-4-(4-hydroxy-1-methylpiperidin-4-yl)-3-(pyridin-4-yl)-1-triisopropylsilyl-1*H*-pyrrole

In a similar manner to that described in Example 7(iv) above, a reaction was carried out using 1-methylpiperidine-4-one instead of pentafluoropyridine to afford the title compound (yield 47%) as a pale brown powder.
$^1$H-Nuclear magnetic resonance spectrum (500 MHz, $CD_3OD$) δ ppm:

8.22 (2H, doublet, J=6 Hz); 7.27 (2H, doublet, J=6 Hz);
7.21 (2H, doublet of doublets, J=9 Hz, 5 Hz);
6.94 (2H, triplet, J=9 Hz); 6.92 (1H, singlet);
2.56-2.42 (4H, broad multiplet); 2.22 (3H, singlet);
2.00-1.91 (2H, broad multiplet);
1.84-1.78 (2H, broad multiplet);
1.20-1.11 (3H, multiplet); 1.06 (18H, doublet, J=7 Hz).

32(ii) 2-(4-Fluorophenyl)-4-(1-methyl-1,2,3,6-tetrahydropyridin-4-yl)-3-(pyridin-4-yl)-1*H*-pyrrole

In a similar manner to that described in Example 9(iii) above, using 2-(4-fluorophenyl)-4-(4-hydroxy-1-methylpiperidin-4-yl)-3-(pyridin-4-yl)-1-triisopropylsilyl-1*H*-pyrrole [prepared as described in step 32(i) above], dehydration with triethylsilane/trifluoroacetic acid and desilylation with tetrabutylammonium fluoride were carried out to give the title compound (yield 91%) as a pale yellow powder.
Melting point: 230 - 232°C (decomposition)
$^1$H-Nuclear magnetic resonance spectrum (500 MHz, DMSO-$d_6$) δ ppm:

11.36 (1H, broad singlet); 8.45 (2H, doublet, J=6 Hz);
7.19-7.14 (6H, multiplet); 6.90 (1H, doublet, J=3 Hz);
5.24-5.21 (1H, broad multiplet);
2.78-2.75 (2H, broad multiplet);
2.40 (2H, triplet, J=5 Hz); 2.18 (3H, singlet);
2.20-2.13 (2H, broad multiplet).

Example 33

2-(4-Fluorophenyl)-4-(1-methylpiperidin-4-yl)-3-(pyridin-4-yl)-1*H*-pyrrole (Compound No. 1-144)

In a similar manner to that described in Example 11 above, 2-(4-fluorophenyl)-4-(1-methyl-1,2,3,6-tetrahydropyridin-4-yl)-3-(pyridin-4-yl)-1*H*-pyrrole [prepared as described in Example 32 above] was reduced to give the title compound (yield 85%) as a white powder.
Melting point: 284 - 285°C (decomposition)
$^1$H-Nuclear magnetic resonance spectrum (500 MHz, DMSO-$d_6$) δ ppm:

11.20 (1H, broad singlet); 8.48 (2H, doublet, J=6 Hz);
7.19-7.16 (4H, multiplet); 7.10 (2H, triplet, J=9 Hz);
6.76 (1H, doublet, J=2 Hz); 2.73 (2H, doublet, J=12 Hz);
2.35 (1H, triplet of triplets, J=12 Hz, 4 Hz);
2.12 (3H, singlet); 1.77 (2H, triplet, J=12 Hz);
1.61 (2H, doublet, J=12 Hz);
1.51 (2H, double doublet of triplets, J=13 Hz, 12 Hz, 4 Hz).

Example 34

3-(4-Fluorophenyl)-2-(pyridin-4-yl)-1-(2,2,6,6-tetramethylpiperidin-4-yl)-1*H*-pyrrole (Compound No. 2-2523)

In a similar manner to the procedures described in Examples 12(i), 12(ii) and 12(iii) above, reactions were carried out using 4-amino-2,2,6,6-tetramethylpiperidine as a starting material instead of 4-amino-1-benzylpiperidine to give the title compound (total yield 12%) as a white powder.
Melting point: 118 - 119°C
$^1$H-Nuclear magnetic resonance spectrum (500 MHz, $CDCl_3$) δ ppm:

8.62 (2H, doublet, J=6 Hz); 7.18 (2H, doublet, J=6 Hz);
7.07 (2H, doublet of doublets, J=9 Hz, 6 Hz);
6.89 (1H, doublet, J=3 Hz); 6.88 (2H, triplet, J=9 Hz);
6.39 (1H, doublet, J=3 Hz); 4.43-4.38 (1H, multiplet);
1.86 (2H, doublet of doublets, J=13 Hz, 3 Hz);
1.55-1.38 (3H, multiplet); 1.15 (6H, singlet);
1.05 (6H, singlet).

Example 35

2-(4-Fluorophenyl)-3-(pyridin-4-yl)-4-(quinuclidin-2-en-3-yl)-1*H*-pyrrole (Compound No. 1-2540)

35(i) (±)-2-(4-Fluorophenyl)-4-(3-hydroxyquinuclidin-3-yl)-3-(pyridin-4-yl)-1-triisopropylsilyl-1*H*-pyrrole

In a similar manner to that described in Example 7(iv) above, a reaction was carried out using quinuclidine-3-one instead of pentafluoropyridine to afford the title compound (yield 37%) as a pale brown powder.
$^1$H-Nuclear magnetic resonance spectrum (500 MHz, DMSO-$d_6$) δ ppm:

8.25 (2H, doublet, J=6 Hz); 7.25 (2H, doublet, J=6 Hz);
7.26-7.16 (2H, broad multiplet); 7.08 (2H, triplet, J=9 Hz);
6.80 (1H, singlet); 4.82 (1H, singlet);
2.81 (1H, doublet, J=14 Hz); 2.72-2.64 (1H, broad multiplet);
2.59 (1H, doublet, J=15 Hz); 2.60-2.50 (2H, broad multiplet);
2.46-2.37 (1H, broad multiplet);
2.03-1.91 (2H, broad multiplet);
1.49-1.37 (2H, broad multiplet);
1.26-1.17 (1H, broad multiplet);
1.15-1.05 (3H, multiplet); 1.03-0.95 (18H, multiplet).

35(ii) 2-(4-Fluorophenyl)-3-(pyridin-4-yl)-4-(quinuclidin-2-en-3-yl)-1*H*-pyrrole

24 ml of formic acid were added to 1.20 g (2.31 mmol) of (±)-2-(4-fluorophenyl)-4-(3-hydroxyquinuclidin-3-yl)-3-(pyridin-4-yl)-1-triisopropylsilyl-1*H*-pyrrole [prepared as described in step 35(i) above]. The resulting mixture was stirred at 90°C for 3 hours. After cooling to room temperature, water was added to the reaction mixture. The pH of the mixture was adjusted to greater than 10 through the addition of a 10% aqueous solution of sodium hydroxide and then the resulting mixture was extracted with ethyl acetate. The organic extract was washed with water, dried over anhydrous sodium sulfate and then concentrated by evaporation under reduced pressure. The residual solid was washed with a 1:1 by volume mixture of ethyl acetate and diethyl ether to give 568 mg (yield 71%) of the title compound as a pale brown powder.
Melting point: 246 - 248 (decomposition)
$^1$H-Nuclear magnetic resonance spectrum (500 MHz, DMSO-$d_6$) δ ppm:

11.48 (1H, broad singlet); 8.47 (2H, doublet, J=6 Hz);
7.19-7.14 (4H, multiplet); 7.11 (2H, triplet, J=9 Hz);
7.00 (1H, doublet, J=2 Hz); 5.86 (1H, singlet);
2.77 (2H, double doublet of doublets, J=13 Hz, 9 Hz, 5 Hz);
2.61-2.56 (1H, broad multiplet); 2.40-2.31 (2H, multiplet);
1.58-1.49 (2H, broad multiplet);
1.43-1.34 (2H, broad multiplet).

Example 36

(±)-2-(4-Fluorophenyl)-3-(pyridin-4-yl)-4-(quinuclidin-3-yl)-1*H*-pyrrole (Compound No. 1-2539)

In a similar manner to that described in Example 11 above, 2-(4-fluorophenyl)-3-(pyridin-4-yl)-4-(quinuclidin-2-en-3-yl)-1*H*-pyrrole (prepared as described in Example 35 above) was reduced to give the title compound (yield 82%) as a white powder.
Melting point: 239 - 241°C (decomposition)
$^1$H-Nuclear magnetic resonance spectrum (500 MHz, DMSO-$d_6$) δ ppm:

11.25 (1H, broad singlet); 8.47 (2H, doublet, J=6 Hz);
7.18-7.07 (6H, multiplet); 6.93 (1H, doublet, J=2 Hz);
2.98-2.90 (1H, multiplet); 2.89-2.76 (2H, broad multiplet);
2.74-2.58 (4H, broad multiplet);
1.73-1.64 (1H, broad multiplet);
1.54-1.46 (2H, broad multiplet);
1.42-1.33 (1H, broad multiplet);
1.26-1.17 (1H, broad multiplet).

Example 37

2-(4-Fluorophenyl)-4-(4-hydroxypiperidin-4-yl)-3-(pyridin-4-yl)-1*H*-pyrrole (Compound No. 1-2526)

2.73 ml (2.73 mmol) of a 1M solution of tetrabutylammonium fluoride in tetrahydrofuran were added to a solution of 675 mg (1.37 mmol) of 2-(4-fluorophenyl)-4-(4-hydroxypiperidin-4-yl)-3-(pyridin-4-yl)-1-triisopropylsilyl-1*H*-pyrrole [prepared as described in Example 9(iii) above] in 14 ml of tetrahydrofuran and the resulting mixture was stirred

at room temperature for 30 minutes. At the end of this time, the solvent was distilled off under reduced pressure. Water was added to the resulting residue and the precipitate thus obtained was collected by filtration and then washed with ethyl acetate to give 363 mg (yield 79%) of the title compound as a white powder.
Melting point: 197 - 199°C
$^1$H-Nuclear magnetic resonance spectrum (500 MHz, DMSO-$d_6$) δ ppm:

11.16 (1H, broad singlet); 8.42 (2H, doublet, J=6 Hz);
7.32 (2H, doublet, J=6 Hz); 7.04-7.01 (4H, multiplet);
6.79 (1H, doublet, J=2 Hz); 4.45 (1H, singlet);
2.78-2.70 (2H, multiplet);
2.55-2.45 (2H, these overlaped with protons derived from remaining DMSO-$d_6$); 1.63-1.48 (4H, multiplet).

Example 38

2-(4-Fluorophenyl)-4-(3-methanesulfonylaminopropyl)-3-(pyridin-4-yl)-1*H*-pyrrole (Compound No. 1-2536)

188 mg (1.08 mmol) of methanesulfonic anhydride were added to a solution of 290 mg (0.982 mmol) of 4-(3-aminopropyl)-2-(4-fluorophenyl)-3-(pyridin-4-yl)-1*H*-pyrrole (prepared as described in Example 1 above) in 5 ml of pyridine and the resulting mixture was allowed to stand overnight. At the end of this time, water was added to the reaction mixture, giving a precipitate which was collected by filtration and washed with ethyl acetate to give 208 mg (yield 57%) of the title compound as a pale brown powder.
Melting point: 210 - 214°C
$^1$H-Nuclear magnetic resonance spectrum (400 MHz, $CDCl_3$) δ ppm:

8.52 (2H, doublet, J=6 Hz); 8.23 (1H, broad singlet);
7.15-7.12 (4H, multiplet); 6.97 (2H, triplet, J=9 Hz);
6.76 (1H, doublet, J=2 Hz); 4.15 (1H, broad singlet);
3.13-3.09 (2H, multiplet); 2.89 (3H, singlet);
2.61 (2H, triplet, J=7 Hz); 1.72 (2H, quintet, J=7 Hz).

Example 39

(±)-3-(4-Fluorophenyl)-2-(pyridin-4-yl)-1-(quinuclidin-3-yl)-1*H*-pyrrole (Compound No. 2-2539)

In a similar manner to the procedures described in Examples 12(i), 12(ii) and 12(iii) above, reactions were carried out using (±)-3-aminoquinuclidine as a starting material instead of 4-amino-1-benzylpiperidine to give the title compound (total yield 25%) as a pale brown powder.
Melting point: 229 - 230°C
$^1$H-Nuclear magnetic resonance spectrum (400 MHz, $CDCl_3$) δ ppm:

8.61 (2H, doublet, J-6 Hz); 7.16 (2H, doublet, J=6 Hz);
7.13 (1H, doublet, J=3 Hz);
7.04 (2H, doublet of doublets, J=9 Hz, 5 Hz);
6.87 (2H, triplet, J=9 Hz); 6.42 (1H, doublet, J=3 Hz);
4.24-4.15 (1H, multiplet); 3.33-3.23 (1H, multiplet);
3.22-3.05 (2H, multiplet); 2.98-2.79 (2H, multiplet);
2.78-2.67 (1H, multiplet); 1.95-1.81 (2H, multiplet);
1.71-1.58 (1H, multiplet); 1.56-1.37 (2H, multiplet).

Example 40

(+)-3-(4-Fluorophenyl)-1-(piperidin-3-yl)methyl-2-(pyridin-4-yl)-1*H*-pyrrole (Compound No. 2-2520)

In a similar manner to the procedures described in Examples 18(i) and 18(ii) above, reactions were carried out using (±)-3-aminomethyl-(1-t-butoxycarbonyl)piperidine as a starting material instead of (±)-3-amino-1-(t-butoxycarbonyl)pyrrolidine to give the title compound (total yield 7%) as a white powder.
Melting point 134 - 135°C
$^1$H-Nuclear magnetic resonance spectrum (400 MHz, $CDCl_3$) δ ppm:

8.59 (2H, doublet, J=6 Hz); 7.16 (2H, doublet, J=6 Hz);
7.06 (2H, doublet of doublets, J=9 Hz, 5 Hz);
6.88 (2H, triplet, J=9 Hz); 6.79 (1H, doublet, J=3 Hz);
6.34 (1H, doublet, J=3 Hz); 3.74 (2H, doublet, J=8 Hz);.
2.94-2.87 (1H, multiplet); 2.83-2.73 (1H, multiplet);
2.46 (1H, doublet of doublets, J=12 Hz, 3 Hz);
2.13 (1H, doublet of doublets, J=12 Hz, 10 Hz);
1.75-1.51 (3H, multiplet); 1.40-1.28 (1H, multiplet);
0.98-0.85 (1H, multiplet).

Example 41

cis-1-(4-Aminocyclohexyl)-3-(4-fluorophenyl)-2-(pyridin-4-yl)-1*H*-pyrrole (Compound No. 2-2529)

In a similar manner to the procedures described in Examples 18 (i) and 18 (ii) above, reactions were carried out using cis-4-(t-butoxycarbonylamino)-cyclohexylamine as a starting material instead of (±)-3-amino-1-(t-butoxycarbonyl)-pyrrolidine to give the title compound (total yield 10%) as a pale brown powder.
Melting point 163 - 164°C
$^1$H-Nuclear magnetic resonance spectrum (400 MHz, $CDCl_3$) δ ppm:

8.61 (2H, doublet, J=6 Hz); 7.14 (2H, doublet, J=6 Hz);
7.05 (2H, doublet of doublets, J=9 Hz, 5 Hz) ;
7.03 (1H, doublet, J=3 Hz); 6.87 (2H, triplet, J=9 Hz);
6.83 (1H, doublet, J=3 Hz);
3.82 (1H, triplet of triplets, J=12 Hz, 4 Hz);
3.29-3.22 (1H, multiplet); 2.22-2.07 (2H, multiplet);
1.82-1.41 (6H, multiplet).

Example 42

3-(4-Fluorophenyl)-2-(2-methylaminopyrimidin-4-yl)-1-(piperidin-4-yl)-1*H*-pyrrole (Compound No. 2-1072)

42(i) 1-(1-Benzylpiperidin-4-yl)-3-(4-fluorophenyl)-2-(2-methylthiopyrimidin-4-yl)-1*H*-pyrrole

In a similar manner to the procedures described in Examples 12(i), 12(it) and 12(iii) above, reactions were carried out using 4-formyl-2-methylthiopyrimidine as a starting material instead of 4-formylpyridine to give the title compound (total yield 34%) as a brown oil.
$^1$H-Nuclear magnetic resonance spectrum (400 MHz, $CDCl_3$) δ ppm:

8.21 (1H, doublet, J=5 Hz); 7.39-7.24 (5H, multiplet);
7.16 (2H, doublet of doublets, J=9 Hz, 5 Hz);
7.06 (1H, doublet, J=3 Hz); 6.96 (2H, triplet, J=9 Hz);
6.57 (1H, doublet, J=5 Hz); 6.27 (1H, doublet, J=3 Hz);
4.83-4.73 (1H, multiplet); 3.54 (2H, singlet);
3.02 (2H, doublet, J=9 Hz); 2.75 (2H, triplet, J=6 Hz);
2.46 (2H, triplet, J=6 Hz); 2.13-1.96 (2H, multiplet).

42(ii) 1-(1-benzylpiperidin-4-yl)-3-(4-fluorophenyl)-2-(2-methanesulfonylpyrimidin-4-yl)-1*H*-pyrrole

3.51 g (15.26 mmol) of 69-75 wt.% of m-chloroperbenzoic acid were added in small portions to a solution of 3.50 g (7.63 mmol) of 1-(1-benzylpiperidin-4-yl)-3-(4-fluorophenyl)-2- (2-methylthiopyrimidin-4-yl)-1*H*-pyrrole [prepared as described in step 42(i) above] in 35 ml of ethyl acetate with ice-cooling. The resulting mixture was stirred at room temperature 2 days, after which a 10% aqueous solution of sodium thiosulfate was added to the reaction mixture followed by extraction with ethyl acetate. The organic extract was washed with water and then concentrated by evaporation under reduced pressure. The residue thus obtained was purified by chromatography on a silica gel column using a 9:1 by volume mixture of dichloromethane and methanol as the eluant to give 2.48 g (yield 66%) of the title compound as a pale brown amorphous solid.
$^1$H-Nuclear magnetic resonance spectrum (400 MHz, $CDCl_3$) δ ppm:

8.35 (1H, doublet, J=5 Hz); 7.63-7.56 (2H, multiplet);
7.47-7.38 (3H, multiplet); 7.29-7.24 (1H, multiplet);
7.22 (2H, doublet of doublets, J=9 Hz, 5 Hz) ;
7.05 (2H, triplet, J=9 Hz); 6.90 (2H, doublet, J=5 Hz);
6.29 (1H, doublet, J=3 Hz); 5.43-5.33 (1H, multiplet);
4.46 (2H, singlet); 3.56-3.27 (4H, multiplet);
3.03-2.86 (2H, multiplet); 2.93 (3H, singlet);
2.26-2.14 (1H, multiplet); 2.10-2.01 (1H, multiplet).

42(iii) 1-(1-Benzylpiperidin-4-yl)-3-(4-fluorophenyl)-2-(2-methylaminopyrimidin-4-yl)-1*H*-pyrrole

A mixture of 1.19 g (2.43 mmol) of 1-(1-benzylpiperidin-4-yl)-3-(4-fluorophenyl)-2-(2-methanesulfonylpyrimidin-4-yl)-1*H*-pyrrole [prepared as described in Step 42(ii) above] and 13 ml of a 2M solution of methylamine in tetrahydrofuran was stirred at 100°C for 30 minutes in a sealed tube. At the end of this time, the solvent was distilled off under reduced pressure. The resulting residue was purified by chromatography on a silica gel column using a 1:2 by volume mixture of hexane and ethyl acetate as the eluant to give 260 mg (yield 24%) of the title compound as a yellow amorphous solid.
$^1$H-Nuclear magnetic resonance spectrum (500 MHz, $CDCl_3$) δ ppm:

8.03 (1H, doublet, J=5 Hz); 7.42-7.23 (5H, multiplet);
7.18 (2H, doublet of doublets, J=9 Hz, 5 Hz);
7.00 (1H, doublet, J=3 Hz); 6.94 (2H, triplet, J=9 Hz);
6.26 (1H, doublet, J=3 Hz); 6.23 (1H, doublet, J=5 Hz);
5.14-5.04 (1H, multiplet); 4.82-4.68 (1H, multiplet);
3.53 (2H, singlet); 3.10-2.95 (5H, multiplet);
2.12-1.96 (6H, multiplet).

42 (iv) 3-(4-Fluorophenyl)-2-(2-methylaminopyrimidin-4-yl)-1-(piperidin-4-yl)-1*H*-pyrrole

In a similar manner to that described in Example 9(ii) above, 1-(1-benzylpiperidin-4-yl)-3-(4-fluorophenyl)-2-(2-methylaminopyrimidin-4-yl)-1*H*-pyrrole [prepared as described in step 42(iii) above] was debenzylated to give the title compound (yield 39%) as a pale yellow powder.
Melting point: 189- 191°C
$^1$H-Nuclear magnetic resonance spectrum (500 MHz, $CDCl_3$) δ ppm:

8.05 (1H, doublet, J=5 Hz);
7.19 (2H, doublet of doublets, J=9 Hz, 5 Hz);
7.00 (1H, doublet, J=3 Hz); 6.95 (2H, triplet, J=9 Hz);
6.28 (1H, doublet, J=3 Hz); 6.24 (1H, doublet, J=5 Hz);
5.14-5.04 (1H, multiplet); 4.96-4.81 (1H, multiplet);
3.24 (2H, doublet, J=12 Hz); 3.04 (3H, doublet, J=5 Hz);
2.70 (2H, doublet of quartets, J=12 Hz, 2 Hz);
2.14 (2H, doublet, J=12 Hz);
1.91 (2H, doublet of quartets, J=12 Hz, 4 Hz).

Example 43

(±)-2-(4-Fluorophenyl)-4-(8-methyl-8-azabicyclo[3.2.1]oct-2-en-3-yl)-3-(pyridin-4-yl)-1*H*-pyrrole (Compound No. 1-2544)

43(i) 2-(4-Fluorophenyl)-4-(3-hydroxy-8-methyl-8-azabicyclo [3.2.1] octan-3-yl)-3-(pyridin-4-yl)-1-triisopropylsilyl-1*H*-pyrrole

In a similar manner to that described in Example 7(iv) above, a reaction was carried out using 8-methyl-8-azabicyclo[3.2.1]octane-3-one instead of pentafluoropyridine to give a mixture of isomers of the title compound (yield 49%) as a pale brown powder.
From the mixture thus obtained, isomer A (Rf value = 0.45, pale brown powder) and isomer B (Rf value = 0.40, pale brown powder) were separated by chromatography on an alumina column using a 10:1 by volume mixture

of ethyl acetate and methanol as the eluant (the ratio of isomer A:isomer B was 85:90 by weight).
isomer A
$^1$H-Nuclear magnetic resonance spectrum (500 MHz, $CD_3OD$) δ ppm:

8.21 (2H, doublet, J=6 Hz); 7.26 (2H, doublet, J=6 Hz);
7.17 (2H, doublet of doublets, J=9 Hz, 5 Hz);
6.92 (2H, triplet, J=9 Hz); 6.84 (1H, singlet);
3.14-3.06 (1H, broad multiplet); 2.28-2.15 (4H, multiplet);
2.20 (3H, singlet); 1.95-1.84 (4H, broad multiplet);
1.19-1.09 (3H, multiplet); 1.05 (18H, doublet, J=7 Hz).

isomer B
$^1$H-Nuclear magnetic resonance spectrum (500 MHz, $CD_3OD$) δ ppm:

8.22 (2H, doublet, J=6 Hz); 7.27 (2H, doublet, J=6 Hz);
7.18 (2H, doublet of doublets, J=9 Hz, 5 Hz);
6.93 (2H, triplet, J=9 Hz); 6.85 (1H, singlet);
3.38 (1H, broad singlet); 2.41 (3H, singlet);
2.38-2.27 (4H, multiplet); 2.06-1.95 (4H, broad multiplet);
1.20-1.10 (3H, multiplet); 1.06 (18H, doublet, J=7 Hz).

43(ii) (±)-2-(4-Fluorophenyl)-4-(8-methyl-8-azabicyclo[3.2.1]oct-2-en-3-yl)-3-(pyridin-4-yl)-1*H*-pyrrole

In a similar manner to that described in Example 9(iii) above, 2-(4-fluorophenyl)-4-(3-hydroxy-8-methyl-8-azabicyclo[3.2.1]octan-3-yl)-3-(pyridin-4-yl)-1-triisopropylsilyl-1*H*-pyrrole (a mixture of isomers A and B, prepared as described in step 43(i) above] was subjected to dehydration using triethylsilane and trifluoroacetic acid followed by desilylation (deprotection) with tetrabutylammonium fluoride to give the title compound (yield 83%) as a white powder.
Melting point: 243 - 245°C
$^1$H-Nuclear magnetic resonance spectrum (500 MHz, $CD_3OD$) δ ppm:

8.39 (2H, doublet, J=7 Hz); 7.25 (2H, doublet, J=6 Hz);
7.16 (2H, doublet of doublets, J=9 Hz, 5 Hz);
6.99 (2H, triplet, J=9 Hz); 6.83 (1H, singlet);
5.45 (1H, broad doublet, J=6 Hz);
3.28-3.24 (1H, broad multiplet);
3.22 (1H, triplet, J=6 Hz); 2.68 (1H, broad doublet, J=16 Hz);
2.37 (3H, singlet); 2.20-2.11 (1H, broad multiplet);
2.09-1.99 (1H, multiplet); 1.90-1.80 (2H, broad multiplet);
1.69-1.61 (1H, multiplet).

Example 44

2- (4-Fluorophenyl)-4-(8-methyl-8-azablcyclo[3.2.1]octan-3-yl)-3-(pyridin-4-yl)-1*H*-pyrrole (Compound No. 1-2542)

In a similar manner to that described in Example 11 above, (±)-2-(4-fluorophenyl)-4-(8-methyl-8-azabicyclo [3.2.1]oct-2-en-3-yl)-3-(pyridin-4-yl)-1*H*-pyrrole [prepared as described in Example 43 above] was reduced to give the title compound (yield 57%) as a white powder.
Melting point: 233 - 234°C (decomposition)
$^1$H-Nuclear magnetic resonance spectrum (500 MHz, DMSO-$d_6$) δ ppm:

11.13 (1H, broad singlet); 8.49 (2H, doublet, J=6 Hz);
7.14-7.05 (6H, multiplet); 6.76 (1H, doublet, J=3 Hz);
3.03-2.85 (3H, broad multiplet); 2.16-2.07 (2H, multiplet);
2.04 (3H, singlet); 1.98-1.90 (2H, multiplet);
1.45-1.36 (2H, multiplet); 1.24-1.14 (2H, multiplet).

Example 45

(±)-4-(8-Azabicyclo[3.2.1]oct-2-en-3-yl)-2-(4-fluorophenyl)-3-(pyridin-4-yl)-1*H*-pyrrole (Compound No. 1-2543)

In a similar manner to the procedures described in Examples 9(i), 9(ii) and 9(iii) above, coupling, debenzylation, dehydration and desilylation reactions were carried out using 8-benzyl-8-azabicyclo[3.2.1]octane-3-one as a starting material instead of 1-benzylpiperidine-4-one to give the title compound (yield 35%) as a pale yellow powder.
Melting point: 212 - 213°C
$^1$H-Nuclear magnetic resonance spectrum (500 MHz, DMSO-$d_6$) δ ppm:

11.32 (1H, broad singlet); 8.45 (2H, doublet, J=6 Hz);
7.17-7.07 (6H, multiplet); 6.81 (1H, doublet, J=3 Hz);
5.45 (1H, doublet, J=6 Hz); 3.53-3.46 (1H, multiplet);
3.38-3.32 (1H, multiplet); 2.55-2.48 (1H, broad multiplet);
1.86-1.69 (3H, multiplet); 1.66-1.55 (1H, multiplet);
1.52-1.44 (1H, multiplet).

Example 46

4-(8-Azabicyclo[3.2.1]octan-3-yl)-2-(4-fluorophenyl)-3-(pyridin-4-yl)-1*H*-pyrrole (Compound No. 1-2541)

In a similar manner to that described in Example 11 above, (±)-4-(8-azabicy*clo[3.2.1]oct-2-en-3-yl)-2-(4-fluorophenyl)-3-(pyridin-4-yl)-1H*-pyrrole [prepared as described in Example 45 above] was reduced to give the title compound (yield 74%) as a white powder.
Melting point: 235 - 237°C (decomposition)
$^1$H-Nuclear magnetic resonance spectrum (500 MHz, DMSO-$d_6$) δ ppm:

11.14 (1H, broad singlet); 8.48 (2H, doublet, J=6 Hz);
7.15-7.05 (6H, multiplet); 6.73 (1H, doublet, J=2 Hz);
3.18-3.15 (2H, multiplet); 2.86-2.78 (1H, multiplet);
2.05-1.96 (2H, multiplet); 1.63-1.56 (2H, multiplet);
1.50-1.43 (2H, multiplet); 1.17-1.08 (2H, multiplet).

Example 47

2-(4-Fluorophenyl)-3-(pyridin-4-yl)-4-(1,2,3,6-tetrahydro-2,2,6,6-tetramethylpyridin-4-yl)-1*H*-pyrrole (Compound No. 1-2650)

In a similar manner to the procedures described in Examples 9(i) and 9(iii) above, coupling, dehydration and desilylation reactions were carried out using 2,2,6,6-tetramethylpiperidine-4-one as a starting material instead of 1-benzylpiperidine-4-one to give the title compound (yield 50%) as a white powder.
Melting point: 258 - 259°C
$^1$H-Nuclear magnetic resonance spectrum (500 MHz, DMSO-$d_6$) δ ppm:

11.37 (1H, broad singlet); 8.42 (2H, doublet, J=6 Hz);
7.19-7.09 (6H, multiplet); 6.86 (1H, doublet, J=2 Hz);
5.04-5.02 (1H, broad multiplet); 1.93 (2H, singlet);
1.25-1.15 (1H, broad singlet); 1.04 (6H, singlet);
0.94 (6H, singlet).

Example 48

2-(4-Fluorophenyl)-3-(pyridin-4-yl)-4-(2,2,6,6-tetramethylpiperidin-4-yl)-1*H*-pyrrole (Compound No. 1-2523)

In a similar manner to that described in Example 11 above, 2-(4-fluorophenyl)-3-(pyridin-4-yl)-4-(1,2,3,6-tetrahydro-2,2,6,6-tetramethylpyridin-4-yl)-1*H*-pyrrole [prepared as described in Example 47 above] was reduced to give the title compound (yield 77%) as a white powder.
Melting point: 242 - 243°C

$^1$H-Nuclear magnetic resonance spectrum (500 MHz, DMSO-$d_6$) δ ppm:

11.18 (1H, broad singlet); 8.47 (2H, doublet, J=6 Hz);
7.21-7.15 (4H, multiplet); 7.11 (2H, triplet, J=9 Hz);
6.70 (1H, doublet, J=2 Hz);
2.95 (1H, triplet of triplets, J=13 Hz, 3 Hz) ;
1.50 (2H, doublet of doublets, J=13 Hz, 3 Hz);
1.09 (2H, triplet, J=13 Hz); 1.01 (6H, singlet);
0.96 (6H, singlet).

Example 49

(±)-2-(4-Fluorophenyl)-4-(2-methyl-1,2,3, 6-tetrahydropyridin-4-yl)-3-(pyridin-4-yl)-1*H*-pyrrole (Compound No. 1-2707)

In a similar manner to the procedures described in Examples 9(i), 9(ii) and 9(iii) above, coupling, debenzylation, dehydration and desilylation reactions were carried out using (±)-1-benzyl-2-methylpiperidine-4-one as a starting material instead of 1-benzylpiperidine-4-one to afford a mixture of the title compound and the (6-methyl-1,2,3,6-tetrahydropyridin-4-yl) isomer thereof (which is the title compound of Example 50). The mixture was separated by chromatography on a silica gel column using a 25:1 by volume mixture of ethyl acetate and isopropylamine as the eluant to give the title compound (yield 3%, Rf value= 0.50) as a white powder.
Melting point: 178 - 180°C (decomposition)
$^1$H-Nuclear magnetic resonance spectrum (500 MHz, $CD_3OD$) δ ppm:

8.36 (2H, doublet, J=6 Hz); 7.24 (2H, doublet, J=6 Hz);
7.17 (2H, doublet of doublets, J=9 Hz, 6 Hz);
7.00 (2H, triplet, J=9 Hz); 6.85 (1H, singlet);
5.40-5.35 (1H, broad multiplet); 3.46-3.38 (1H, multiplet);
3.36-3.28 (1H, multiplet); 2.96-2.88 (1H, multiplet);
2.23-2.15 (1H, multiplet); 2.06-1.97 (1H, multiplet);
1.14 (3H, doublet, J=6 Hz).

Example 50

(±)-2-(4-Fluorophenyl)-4-(6-methyl-1,2,3,6-tetrahydropyridin-4-yl)-3-(pyridin-4-yl)-1*H*-pyrrole (Compound No. 1-2665)

The title compound (yield 5%, Rf value = 0.45) was obtained as a white powder during the chromatography performed in Example 49 above.
Melting point: 201 - 204°C (decomposition)
$^1$H-Nuclear magnetic resonance spectrum (500 MHz, $CD_3OD$) δ ppm:

8.36 (2H, doublet, J=6 Hz); 7.24 (2H, doublet, J=6 Hz) ;
7.18 (2H, doublet of doublets, J=9 Hz, 6 Hz);
7.00 (2H, triplet, J=9 Hz); 6.86 (1H, singlet);
5.24-5.20 (1H, multiplet); 3.48-3.40 (1H, multiplet);
3.16-3.09 (1H, multiplet); 2.90-2.82 (1H, multiplet);
2.37-2.27 (1H, multiplet); 2.18-2.10 (1H, multiplet);
1.05 (3H, doublet, J=7 Hz).

Example 51

(±)-2-(4-Fluorophenyl)-4-(2-methylpiperidin-4-yl)-3-(pyridin-4-yl)-1*H*-pyrrole (Compound No. 1-2522)

In a similar manner to the procedure in Example 11 above, (±)-2-(4-fluorophenyl)-4-(6-methyl-1,2,3,6-tetrahydropyridin-4-yl)-3-(pyridin-4-yl)-1*H*-pyrrole [prepared as described in Example 50 above] was reduced to give the title compound (yield 83%) as a pale yellow powder.
Melting point: 198 - 205°C
$^1$H-Nuclear magnetic resonance spectrum (500 MHz, $CDCl_3$) δ ppm:

8.53 (2H, doublet, J=4 Hz); 8.33 (0.5H, broad singlet);
8.31 (0.5H, broad singlet); 7.14 (2H, doublet, J=4 Hz);
7.11 (2H, doublet of doublets, J=9 Hz, 5 Hz);
6.95 (2H, triplet, J=9 Hz); 6.79 (0.5H, doublet, J=3 Hz);
6.74 (0.5H, doublet, J=3 Hz); 3.25-2.63 (4H, multiplet);
1.74-1.43 (5H, multiplet); 1.11 (1.5H, doublet, J=7 Hz);
1.07 (1.5H, doublet, J=7 Hz).

Example 52

4- (1-Ethyl-1,2,3,6-tetrahydropyridin-4-yl)-2-(4-fluorophenyl)-3-(pyridin-4-yl)-1*H*-pyrrole (Compound No. 1-2629)

In a similar manner to the procedures described in Examples 9(i) and 9(iii) above, coupling, dehydration and desilylation reactions were carried out using 1-ethylpiperidine-4-one as a starting material instead of 1-benzylpiperidine-4-one to give the title compound (yield 55%) as a pale yellow powder.
Melting point: 234 - 236°C (decomposition)
$^1$H-Nuclear magnetic resonance spectrum (500 MHz, DMSO-$d_6$) δ ppm:

11.36 (1H, broad singlet); 8.45 (2H, doublet, J=6 Hz);
7.18-7.08 (6H, multiplet); 6.90 (1H, doublet, J=3 Hz);
5.26-5.22 (1H, broad multiplet);
2.84-2.79 (2H, broad multiplet) ;
2.45 (2H, triplet, J=6 Hz); 2.34 (2H, quartet, J=8 Hz);
2.18-2.12 (2H, broad multiplet); 0.99 (3H, triplet, J=8 Hz).

Example 53

4-(1-Ethylpiperidin-4-yl)-2-(4-fluorophenyl)-3-(pyridin-4-yl)-1*H*-pyrrole (Compound No. 1-146)

In a similar manner to the procedure described in Example 11 above, 4-(1-ethyl-1,2,3,6-tetrahydropyridin-4-yl)-2-(4-fluorophenyl)-3-(pyridin-4-yl)-1*H*-pyrrole [prepared as described in Example 52 above] was reduced to give the title compound (yield 95%) as a white powder.
Melting point: 262 - 263°C (decomposition)
$^1$H-Nuclear magnetic resonance spectrum (500 MHz, DMSO-$d_6$) δ ppm:

11.19 (1H, broad singlet); 8.48 (2H, doublet, J=6 Hz);
7.18-7.07 (6H, multiplet); 6.76 (1H, doublet, J=2 Hz);
2.87-2.79 (2H, broad multiplet);
2.37 (1H, triplet of triplets, J=12 Hz, 4 Hz);
2.26 (2H, quartet, J=7 Hz); 1.80-1.71 (2H, broad multiplet);
1.66-1.59 (2H, broad multiplet); 1.55-1.43 (2H, multiplet);
0.96 (3H, triplet, J=7 Hz).

Example 54

2-(4-Fluorophenyl)-4-(1-isopropyl-1,2,3,6-tetrahydropyridin-4-yl)-3-(pyridin-4-yl)-1*H*-pyrrole (Compound No. 1-2632)

In a similar manner to the procedures described in Examples 9(i) and 9(iii) above, coupling, dehydration and desilylation reactions were carried out using 1-isopropylpiperidine-4-one as a starting material instead of 1-benzylpiperidine-4-one to give the title compound (yield 42%) as a pale yellow powder.
Melting point: 237 - 240°C (decomposition)
$^1$H-Nuclear magnetic resonance spectrum (500 MHz, DMSO-$d_6$) δ ppm:

11.35 (1H, broad singlet); 8.45 (2H, doublet, J=6 Hz);
7.18-7.08 (6H, multiplet); 6.88 (1H, doublet, J=3 Hz);
5.28-5.24 (1H, broad multiplet);
2.94-2.89 (2H, broad multiplet);
2.64 (1H, septet, J=6 Hz); 2.49 (2H, triplet, J=6 Hz);

2.15-2.09 (2H, broad multiplet); 0.96 (6H, doublet, J=6 Hz).

Example 55

2-(4-Fluorophenyl)-4-(1-isopropylpiperidin-4-yl)-3-(pyridin-4 *-yl)-1H*-pyrrole (Compound No. 1-2633)

In a similar manner to that described in Example 11 above, 2-(4-fluorophenyl)-4-(1-isopropyl-1,2,3,6-tetrahydropyridin-4-yl)-3-(pyridin-4-yl)-1H-pyrrole (prepared as described in Example 54 above) was reduced to give the title compound (yield 80%) as white needle-like crystals.
Melting point: 264 - 266°C (decomposition)
$^1$H-Nuclear magnetic resonance spectrum (500 MHz, DMSO-$d_6$) δ ppm:

11.18 (1H, broad singlet); 8.48 (2H, doublet, J=6 Hz);
7.17-7.06 (6H, multiplet); 6.76 (1H, singlet);
2.77-2.70 (2H, broad multiplet); 2.63 (1H, septet, J=6 Hz);
2.35 (1H, triplet of triplets, J=12 Hz, 4 Hz);
2.06-1.97 (2H, multiplet); 1.67-1.60 (2H, broad multiplet);
1.52-1.40 (2H, multiplet); 0.92 (6H, doublet, J=6 Hz).

Example 56

2-(4-Fluorophenyl)-4-(1-propyl-1,2,3,6-tetrahydropyridin-4-yl)-3-(pyridin-4-yl)-1*H*-pyrrole (Compound No. 1-2630)

In a similar manner to the procedures described in Examples 9(i) and 9(iii) above, coupling, dehydration and desilylation reactions were carried out using 1-propylpiperidine-4-one as a starting material instead of 1-benzylpiperidine-4-one to give the title compound (yield 46%) as a white powder.
Melting point: 236 - 238°C (decomposition)
$^1$H-Nuclear magnetic resonance spectrum (500 MHz, DMSO-$d_6$) δ ppm:

11.36 (1H, broad singlet); 8.45 (2H, doublet, J=6 Hz);
7.18-7.08 (6H, multiplet); 6.90 (1H, doublet, J=3 Hz);
5.26-5.23 (1H, multiplet); 2.84-2.79 (2H, multiplet);
2.44 (2H, triplet, J=5 Hz); 2.25 (2H, triplet, J=8 Hz);
2.17-2.11 (2H, broad multiplet);
1.43 (2H, triplet of quartets, J=8 Hz, 8 Hz);
0.84 (3H, triplet, J=8 Hz).

Example 57

4- (1-Benzyl-1,2,3,6-tetrahydropyridin-4-yl)-2-(4-fluorophenyl)-3-(pyridin-4-yl)-1*H*-pyrrole (Compound No. 1-2644)

In a similar manner to that described in Example 9(iii) above, dehydration and desilylation reactions were carried out using 4-(1-benzyl-4-hydroxypiperidin-4-yl)-2-(4-fluorophenyl)-3-(pyridin-4-yl)-1-triisopropylsilyl-1*H*-pyrrole [prepared as described in Example 9(i) above] to give the title compound (yield 88%) as a pale yellow powder.
Melting point: 217 - 219°C (decomposition)
$^1$H-Nuclear magnetic resonance spectrum (500 MHz, DMSO-$d_6$) δ ppm:

11.37 (1H, broad singlet); 8.45 (2H, doublet, J=6 Hz);
7.34-7.21 (5H, multiplet); 7.18-7.08 (6H, multiplet);
6.90 (1H, doublet, J=3 Hz); 5.23-5.21 (1H, broad multiplet);
3.50 (2H, singlet); 2.86-2.81 (2H, broad multiplet)
2.48 (2H, triplet, J=6 Hz); 2.18-2.12 (2H, broad multiplet).

Example 58

2-(4-Fluorophenyl)-4-(1-phenethyl-1,2,3,6-tetrahydropyridin-4-yl)-3-(pyridin-4-yl)-*1H*-pyrrole (Compound No. 1-2645)

In a similar manner to the procedures described in Examples 9(i) and 9(iii) above, coupling, dehydration and

desilylation reactions were carried out using 1-phenethylpiperidine-4-one as a starting material instead of 1-benzylpiperidine-4-one to give the title compound (yield 46%) as a pale brown powder.
Melting point: 219 - 221°C (decomposition)
$^1$H-Nuclear magnetic resonance spectrum (500 MHz, DMSO-$d_6$) δ ppm:

11.37 (1H, broad singlet); 8.45 (2H, doublet, J=6 Hz);
7.29-7.09 (11H, multiplet); 6.90 (1H, doublet, J=3 Hz);
5.28-5.24 (1H, broad multiplet);
2.95-2.89 (2H, broad multiplet);
2.76-2.69 (2H, multiplet); 2.57-2.51 (4H, multiplet);
2.19-2.12 (2H, broad multiplet).

Example 59

(±)-2-(4-Fluorophenyl)-4-(1,2,3,5,8, 8a-hexahydroindolizin-7-yl)-3-(pyridin-4-yl)-1*H*-pyrrole (Compound No. 1-2654)

In a similar manner to the procedures described in Examples 9(i) and 9(iii) above, coupling, dehydration and desilylation reactions were carried out using (±)-1,2,3,5,6,7,8,8a-octahydroindolizine-7-one as a starting material instead of 1-benzylpiperidine-4-one to give a mixture of the title compound and the (1,2,3,5,6,8a-hexahydroindolizin-7-yl) isomer thereof (which is the compound of Example 60 below). The mixture was separated by chromatography on a silica gel column using a 10:1:1 by volume mixture of ethyl acetate, methanol and isopropylamine as the eluant to give the title compound (yield 24%, Rf value = 0.50) as a white powder.
Melting point: 220 - 222°C (decomposition)
$^1$H-Nuclear magnetic resonance spectrum (500 MHz, $CD_3OD$) δ ppm:

8.36 (2H, doublet, J=6 Hz); 7.24 (2H, doublet, J=6 Hz);
7.17 (2H, doublet of doublets, J=9 Hz, 6 Hz);
7.00 (2H, triplet, J=9 Hz); 6.85 (1H, singlet);
5.41-5.37 (1H, multiplet); 3.47-3.40 (1H, multiplet);
3.18-3.12 (1H, multiplet); 2.84-2.77 (1H, multiplet);
2.40-2.26 (2H, multiplet); 2.23-2.11 (2H, multiplet);
2.05-1.97 (1H, multiplet); 1.90-1.75 (2H, multiplet);
1.46-1.36 (1H, multiplet).

Example 60

(±)-2-(4-Fluorophenyl)-9-(1,2,3,5,6,8a-hexahydroindolizin-7-yl)-3-(pyridin-4-yl)-1*H*-pyrrole (Compound No. 1-2653)

The title compound (yield 27%, Rf value = 0.40) was obtained as a pale yellow powder during the chromatography performed in Example 59 above.
Melting point: 188 - 190°C (decomposition)
$^1$H-Nuclear magnetic resonance spectrum (500 MHz, $CD_3OD$) δ ppm:

8.37 (2H, doublet, J=6 Hz); 7.23 (2H, doublet, J=6 Hz);
7.18 (2H, doublet of doublets, J=9 Hz, 5 Hz);
7.00 (2H, triplet, J=9 Hz); 6.86 (1H, singlet);
5.34-5.30 (1H, broad multiplet); 3.38-3.30 (1H, multiplet);
2.99-2.86 (2H, multiplet); 2.82-2.73 (2H, multiplet);
2.40-2.31 (1H, multiplet); 2.25-2.16 (1H, multiplet);
1.97-1.86 (2H, multiplet); 1.84-1.73 (1H, multiplet);
1.48-1.38 (1H, multiplet).

Example 61

(±)-4-(1,6-Dimethyl-1,2,3,6-tetrahydropyridin-4-yl)-2-(4-fluorophenyl)-3-(pyridin-4-yl)-1*H*-pyrrole (Compound No. 1-2666)

In a similar manner to the procedures described in Examples 9(i) and 9(iii) above, coupling, dehydration and

desilylation reactions were carried out using (±)-1,2-dimethylpiperidine-4-one as a starting material instead of 1-benzylpiperidine-4-one to give a mixture of the title compound and the (1,2-dimethyl-1,2,3,6-tetrahydropyridin-4-yl) isomer thereof (which is the compound of Example 62 below). The mixture was separated by chromatography on a silica gel column using a 99:1 by volume mixture of ethyl acetate and isopropylamine as the eluant to give the title compound (yield 10%, Rf value = 0.40) as a pale yellow powder.
Melting point: 208 - 209°C (decomposition)
$^1$H-Nuclear magnetic resonance spectrum (500 MHz, $CDCl_3$) δ ppm:

8.47 (2H, doublet, J=6 Hz); 8.29-8.10 (1H, broad singlet);
7.17 (2H, doublet, J=6 Hz);
7.13 (2H, doublet of doublets, J=9 Hz, 5 Hz);
6.97 (2H, triplet, J=9 Hz); 6.83 (1H, doublet, J=3 Hz);
5.19 (1H, singlet); 2.90-2.83 (1H, multiplet);
2.55-2.32 (2H, multiplet); 2.37 (3H, singlet);
2.17-2.07 (2H, multiplet); 1.05 (3H, doublet, J=7 Hz).

Example 62

(±)-3-(1,2-Dimethyl-1,2,3,6-tetrahydropyridin-4-yl)-2-(4-fluorophenyl)-3-(pyridin-4-yl)-1*H*-pyrrole (Compound No. 1-2708)

The title compound (yield 14%, Rf value = 0.30) was obtained as a pale yellow powder during the chromatography performed in Example 61 above.
Melting point: 198 - 201°C
$^1$H-Nuclear magnetic resonance spectrum (400 MHz, $CDCl_3$) δ ppm:

8.47 (2H, doublet, J=6 Hz); 8.33 (1H, broad singlet);
7.17 (2H, doublet, J=6 Hz) ;
7.12 (2H, doublet of doublets, J=9 Hz, 5 Hz);
6.97 (2H, triplet, J=9 Hz); 6.82 (1H, doublet, J=3 Hz);
5.40 (1H, singlet); 3.22-3.18 (1H, multiplet);
2.95-2.91 (1H, multiplet); 2.57-2.46 (1H, multiplet);
2.34 (3H, singlet); 2.27-2.23 (1H, multiplet);
2.11-2.09 (1H, multiplet); 1.08 (3H, doublet, J=6 Hz).

Example 63

2-(4-Fluorophenyl)-4-(1,2,2,6,6-pentamethyl-1,2,3,6-tetrahydropyridin-4-yl)-3-(pyridin-4-yl)-1*H*-pyrrole (Compound No. 1-2651)

In a similar manner to the procedures described in Example 9(i) and 9(iii) above, coupling, dehydration and desilylation reactions were carried out using 1,2,2,6,6-pentamethylpiperidine-4-one as a starting material instead of 1-benzylpiperidine-4-one to give the title compound (yield 69%) as a white powder.
Melting point: 264 - 268°C (decomposition)
$^1$H-Nuclear magnetic resonance spectrum (500 MHz, DMSO-$d_6$) δ ppm:

11.38 (1H, broad singlet); 8.43 (2H, doublet, J=6 Hz);
7.20-7:09 (6H, multiplet); 6.88 (1H, doublet, J=3 Hz);
4.88 (1H, singlet); 2.18 (3H, singlet);
2.07 (2H, singlet); 1.01 (6H, singlet);
0.90 (6H, singlet).

Example 64

2-(4-Fluorophenyl)-4-(1,2,2,6,6-pentamethylpiperidin-4-yl)-3-(pyridin-4-yl)-1*H*-pyrrole (Compound No. 1-2652)

In a similar manner to that described in Example 11 above, 2- (4-fluorophenyl)-4-(1,2,2,6,6-pentamethyl-1,2,3,6-tetrahydropyridin-4-yl)-3-(pyridin-4-yl)-1*H*-pyrrole (prepared as described in Example 63 above) was reduced

to give the title compound (yield 82%) as a white powder.
Melting point: 248 - 250°C (decomposition)
$^1$H-Nuclear magnetic resonance spectrum (500 MHz, DMSO-$d_6$) δ ppm:

11.19 (1H, broad singlet); 8.47 (2H, doublet, J=6 Hz); 7.21-7.15 (4H, multiplet); 7.11 (2H, triplet, J=9 Hz); 6.73 (1H, doublet, J=2 Hz); 2.91-2.82 (1H, multiplet); 2.14 (3H, singlet); 1.54-1.47 (2H, multiplet); 1.36 (2H, triplet, J=13 Hz); 1.01 (6H, singlet); 0.86 (6H, singlet).

Example 65

2-(4-Fluorophenyl)-3-(pyridin-4-yl)-4-(1,2,3,6-tetrahydropyridin-5-yl)-1*H*-pyrrole (Compound No. 1-2627)

In a similar manner to the procedures described in Example 9(i), 9(ii) and 9(iii) above, coupling, debenzylation, dehydration and desilylation reactions were carried out using 1-benzylpiperidine-3-one as a starting material instead of 1-benzyl-piperidine-4-one to give the title compound (yield 12%) as a pale brown powder.
Melting point: 230 - 233°C (decomposition)
$^1$H-Nuclear magnetic resonance spectrum (500 MHz, $CD_3OD$) δ ppm:

8.36 (2H, doublet, J=6 Hz); 7.24 (2H, doublet, J=6 Hz); 7.18 (2H, doublet of doublets, J=9 Hz, 6 Hz); 7.01 (2H, triplet, J=9 Hz); 6.82 (1H, singlet); 5.55-5.50 (1H, broad multiplet); 3.36-3.30 (2H, multiplet); 2.90 (2H, triplet, J=6 Hz); 2.16-2.09 (2H, broad multiplet).

Example 66

4-(3-Dimethylamino-1-propen-1-yl)-2-(4-fluorophenyl)-3-(pyridin-4-yl)-1*H*-pyrrole (Compound No. 1-2519)

66(i) 2-(4-Fluorophenyl)-4-(3-hydroxy-1-propen-1-yl)-3-(pyridin-4-yl)-1*H*-pyrrole

27.6 ml (27.6 mmol) of a 1M solution of diisobutylaluminum hydride in dichloromethane were added dropwise to a solution of 3.88 g (12.5 mmol) of ethyl 3-[2-(4-fluorophenyl)-3-(pyridin-4-yl)-1*H*-pyrrol-4-yl]acrylate in 120 ml of dichloromethane at -78°C with stirring. The resulting mixture was stirred at the same temperature for 3 hours. At the end of this time, the reaction mixture was added to a saturated aqueous solution of sodium hydrogencarbonate and the resulting mixture was filtered. The filtrate was extracted with dichloromethane and the organic extract was washed with water, dried over anhydrous sodium sulfate and then concentrated by evaporation under reduced pressure. The residue thus obtained was purified by chromatography on a silica gel column using a 9:1 by volume mixture of hexane and ethyl acetate as the eluant to afford 3.42 g (yield 93%) of the title compound as a pale brown powder.
$^1$H-Nuclear magnetic resonance spectrum (400 MHz, $CD_3OD$) δ ppm:

8.41 (2H, doublet, J=6 Hz); 7.23 (2H, doublet, J=6 Hz); 7.20 (2H, doublet of doublets, J=9 Hz, 5 Hz); 7.08 (1H, singlet); 7.01 (2H, triplet, J=9 Hz); 6.39 (1H, doublet, J=16 Hz); 5.98 (1H, doublet of triplets, J=16 Hz, 6 Hz); 4.09 (2H, doublet of doublets, J=6 Hz, 1 Hz).

66 (ii) 3-[2-(4-Fluorophenyl)-3-(pyridin-4-yl)-1*H*-pyrrol-4-yl]acrylaldehyde

16.32 g of activated manganese oxide were added to a solution of 1.63 g (5.54 mmol) of 2-(4-fluorophenyl)-4-(3-hydroxy-1-propen-1-yl)-3-(pyridin-4-yl)-1*H*-pyrrole [prepared as described in step 66 (i) above] in 80 ml of dimethylsulfoxide. The resulting mixture was stirred at room temperature for 24 hours. At the end of this time, ethyl acetate was added to the reaction mixture and then this was filtered. Water was added to the resulting filtrate and then this was extracted with ethyl acetate. The organic extract was washed with water, dried over anhydrous magnesium sulfate and then concentrated by evaporation under reduced pressure. The residue thus obtained was washed with dichloromethane to afford 1.36 g (yield 84%) of the title compound as a pale brown powder.
$^1$H-Nuclear magnetic resonance spectrum (400 MHz, $CD_3OD$-$CDCl_3$) δ ppm:

9.42 (1H, doublet, J=8 Hz); 8.51 (2H, doublet, J=6 Hz);
7.49 (1H, singlet); 7.45 (1H, doublet, J=16 Hz) ;
7.27 (2H, doublet, J=6 Hz);
7.22 (2H, doublet of doublets, J=9 Hz, 5 Hz);
7.01 (2H, triplet, J=9 Hz);
6.39 (1H, doublet of doublets, J=16 Hz, 8 Hz).

66(iii) 4-(3-Dimethylamino-1-propen-1-yl)-2-(4-fluorophenyl)-3-(pyridin-4-yl)-1*H*-pyrrole

700 mg (2.38 mmol) of 3-[2-(4-fluorophenyl)-3-(pyridin-4-yl)-1*H*-pyrrol-4-yl]acrylaldehyde [prepared as described in step 66 (i) above] and 1.95 g (23.8 mmol) of dimethylamine hydrochloride were dissolved in 110 ml of a 1:1 by volume mixture of methanol and tetrahydrofuran, after which 226 mg (3.59 mmol) of sodium cyanotrihydridoborate were added to the solution. The resulting mixture was stirred at room temperature for 19 hours. At the end of this time, the reaction mixture was added to a saturated aqueous solution of sodium hydrogencarbonate and then this was extracted with ethyl acetate. The organic extract was washed with water, dried over anhydrous magnesium sulfate and then concentrated by evaporation under reduced pressure. The residue thus obtained was purified by chromatography on a silica gel column using a 20:1:1 by volume mixture of ethyl acetate, methanol and isopropylamine as the eluant to give 429 mg (yield 56%) of the title compound as a white powder.
Melting point: 196 - 198°C (decomposition)
$^1$H-Nuclear magnetic resonance spectrum (400 MHz, $CD_3OD$-$CDCl_3$) δ ppm:

8.42 (2H, doublet, J=6 Hz); 7.23 (2H, doublet, J=6 Hz);
7.20 (2H, doublet of doublets, J=9 Hz, 5 Hz);
7.09 (1H, singlet); 6.98 (2H, triplet, J=9 Hz);
6.35 (1H, doublet, J=16 Hz);
5.88 (1H, doublet of triplets, J=16 Hz, 7 Hz);
3.04 (2H, doublet, J=7 Hz); 2.27 (6H, singlet).

Example 67

4-(4-Aminobutyl)-2-(4-fluorophenyl)-3-(pyridin-4-yl)-1*H*-pyrrole (Compound No. 1-98)

67(i) 4-(3-Cyanopropyl)-2-(4-fluorophenyl)-3-(pyridin-4-yl)-1*H*-pyrrole

568 mg (8.72 mmol) of potassium cyanide were added to a solution of 3.04 g (7.26 mmol) of 2-(4-fluorophenyl)-3-(pyridin-4-yl)-4-[3-(p-toluene-sulfonyloxy)propyl)]-1*H*-pyrrole [prepared as described in Example 23(iv) above] in 60 ml of N,N-dimethylformamide and the resulting mixture was stirred at 100°C for 2 hours. After cooling to room temperature, a saturated aqueous solution of sodium hydrogencarbonate was added to the reaction mixture and then this was extracted with ethyl acetate. The organic extract was washed with water, dried over anhydrous magnesium sulfate and then concentrated by evaporation under reduced pressure. The residue thus obtained was purified by chromatography on a silica gel column using a 2:1 by volume mixture of hexane and ethyl acetate as the eluant to give 0.94 g (yield 42%) of the title compound as a pale yellow powder.
$^1$H-Nuclear magnetic resonance spectrum (500 MHz, $CDCl_3$) δ ppm:

8.53 (2H, doublet, J=6 Hz); 8.25 (1H, broad singlet);
7.13 (2H, doublet of doublets, J=9 Hz, 5 Hz);
7.12 (2H, doublet, J=6 Hz); 6.97 (2H, triplet, J=9 Hz);
6.75 (1H, doublet, J=3 Hz); 2.70 (2H, triplet, J=7 Hz);
2.28 (2H, triplet, J=7 Hz); 1.76 (2H, quintet, J=7 Hz).

67(ii) 4-(4-Aminobutyl)-2-(4-fluorophenyl)-3-(pyridin-4-yl)-1*H*-pyrrole

A solution of 0.94 g (3.08 mmol) of 4-(3-cyanopropyl)-2-(4-fluorophenyl)-3-(pyridin-4-yl)-1H-pyrrole [prepared as described in step 67(i) above] in 50 ml of tetrahydrofuran was added dropwise to a suspension of 468 mg (12.32 mmol) of lithium aluminum hydride in 100 ml of tetrahydrofuran at room temperature with stirring. The resulting mixture

was stirred at 60°C for 3 hours. After cooling to room temperature,0.47 ml of a 15% aqueous solution of sodium hydroxide and 20 ml of water were successively added gradually to the reaction mixture. The resulting mixture was filtered and the filtrate thus obtained was concentrated by evaporation under reduced pressure. The resulting residue was purified by chromatography on a silica gel column using a 25:1 by volume mixture of ethyl acetate and isopropylamine as the eluant to give 410 mg (yield 43%) of the title compound as a pale red powder.
Melting point: 185 - 187°C
$^1$H-Nuclear magnetic resonance spectrum (400 MHz, $CDCl_3$) δ ppm:

10.02 (1H, broad singlet); 8.49 (2H, doublet, J=4 Hz);
7.19 (2H, doublet of doublets, J=9 Hz, 5 Hz);
7.14 (2H, doublet, J=4 Hz); 6.94 (2H, triplet, J=9 Hz);
6.72 (1H, doublet, J=2 Hz); 2.65 (2H, triplet, J=7 Hz);
2.51 (2H, doublet, J=7 Hz); 2.46-2.08 (2H, multiplet);
1.52-1.46 (4H, multiplet).

Example 68

1-(2-Dimethylaminoethyl)-3-(4-fluorophenyl)-2-(pyridin-4-yl)-1*H*-pyrrole (Compound No. 2-108)

In a similar manner to the procedures described in Examples 12(i), 12(ii) and 12(iii) above, reactions were carried out using 2-dimethylaminoethylamine as a starting material instead of 4-amino-1-benzylpiperidine to give the title compound (yield 8%) as a pale brown powder.
Melting point: 70-73°C
$^1$H-Nuclear magnetic resonance spectrum (400 MHz, $CDCl_3$) δ ppm:

8.60 (2H, doublet, J=6 $H_z$); 7.19 (2H, doublet, J=6 Hz);
7.07 (2H, doublet of doublets, J=9 Hz, 5 Hz);
6.89 (2H, triplet, J=9 Hz); 6.88 (1H, doublet, J=3 Hz);
6.36 (1H, doublet, J=3 Hz); 3.96 (2H, triplet, J=7 Hz);
2.48 (2H, triplet, J=7 Hz); 2.15 (6H, singlet).

Example 69

1-(2,2-Dimethyl-3-dimethylaminopropyl)-3-(4-fluorophenyl)-2-(pyridin-4-yl)-1*H*-pyrrole (Compound No. 2-2626)

In a similar manner to the procedures described in Examples 12(i), 12 (ii) and 12(iii) above, reactions were carried out using 2,2-dimethyl-3-dimethylamino-propylamine as a starting material instead of 4-amino-1-benzylpiperidine to give the title compound (yield 21%) as a white powder. Melting point: 101 - 103°C
$^1$H-Nuclear magnetic resonance spectrum (400 MHz, $CDCl_3$) δ ppm:

8.58 (2H, doublet, J=6 Hz); 7.14 (2H, doublet, J=6 Hz);
7.04 (2H, doublet of doublets, J=9 Hz, 5 Hz);
6.88 (2H, triplet, J=9 Hz); 6.84 (1H, doublet, J=3 Hz);
6.32 (1H, doublet, J=3 Hz); 3.90 (2H, singlet);
2.22 (6H, singlet); 2.00 (2H, singlet); 0.64 (6H, singlet).

Example 70

1-(8-Azabicyclo[3.2.1]octan-3-yl)-3-(4-fluorophenyl)-2-(pyridin-4-yl)-1*H*-pyrrole (Compound No. 2-2541)

In a similar manner to the procedures described in Examples 12(i), 12(ii) and 12(iii) above, reactions were carried out using 3-amino-8-benzyl-8-azabicyclo[3.2.1]octane as a starting material instead of 4-amino-1-benzylpiperidine to afford the N-benzyl derivative of the title compound. The N-benzyl derivative was then debenzylated in a similar manner to that described in Example 9(ii) above to give the title compound (yield 90%) as a white powder.
Melting point: 204- 205°C
$^1$H-Nuclear magnetic resonance spectrum (500 MHz, $CDCl_3$) δ ppm:

8.60 (2H, doublet, J=6 Hz); 7.14 (2H, doublet, J=6 Hz);

7.02 (2H, doublet of doublets, J=9 Hz, 5 Hz);
6.88 (1H, doublet, J=3 Hz); 6.86 (2H, triplet, J=9 Hz);
6.38 (1H, doublet, J=3 Hz); 4.36-4.25 (1H, multiplet);
3.67-3.53 (2H, multiplet); 2.51-2.39 (2H, multiplet);
1.90-1.67 (4H, multiplet); 1.57-1.47 (2H, multiplet).

Example 71

(±)-3-(4-Fluorophenyl)-1-(2-methylpiperidin-4-yl)-2-(pyridin-4-yl)-1*H*-pyrrole (Compound No. 2-2522)

In a similar manner to the procedures described in Examples 12(i), 12(ii) and 12(iii) above, reactions were carried out using (±)-4-amino-1-benzyl-2-methyl-piperidine as a starting material instead of 4-amino-1-benzylpiperidine to afford the N-benzyl derivative of the title compound. The N-benzyl derivative was then debenzylated in a similar manner to that described in Example 9(ii) above to give the title compound (yield 24%) as a white powder.
Melting point: 173 - 175°C
$^1$H-Nuclear magnetic resonance spectrum (500 MHz, $CDCl_3$) δ ppm:

8.61 (2H, doublet, J=5 Hz); 7.15 (1H, doublet, J=5 Hz);
7.14 (1H, doublet, J=5 Hz); 7.07-7.03 (2H, multiplet);
7.00 (0.5H, doublet, J=3 Hz); 6.95 (0.5H, doublet, J=3 Hz);
6.88 (2H, triplet, J=9 Hz); 6.38 (1H, doublet, J=3 Hz);
4.30-4.26 (0.5H, multiplet); 4.00-3.91 (0.5H, multiplet);
3.47-3.39 (0.5H, multiplet); 3.17-3.15 (0.5H, multiplet);
2.98-2.88 (1H, multiplet); 2.66-2.61 (1H, multiplet);
2.06-1.76 (4H, multiplet); 1.11 (1.5H, doublet, J=7 Hz);
1.08 (1.5H, doublet, J=7 Hz).

Example 72

3-(4-Fluorophenyl)-2-(2-methylaminopyridin-4-yl)-1-(piperidin-4-yl)-1*H*-pyrrole (Compound No. 2-2042)

72(i) 1-(1-Benzylpiperidin-4-yl)-3-(4-fluorophenyl)-2-(2-fluoropyridin-4-yl)-1*H*-pyrrole

In a similar manner to the procedures described in Examples 12(i), 12(ii) and 12(iii) above, reactions were carried out using 2-fluoropyridin-4-carboxyaldehyde as a starting material instead of pyridine-4-carboxyaldehyde to give the title compound (yield 49%) as a pale brown powder.
$^1$H-Nuclear magnetic resonance spectrum (400 MHz, $CDCl_3$) δ ppm:

8.20 (1H, doublet, J=5 Hz); 7.37-7.24 (5H, multiplet);
7.04 (2H, doublet of doublets, J=9 Hz, 5 Hz);
7.02-6.96 (2H, multiplet); 6.90 (2H, triplet, J=9 Hz);
6.75 (1H, singlet); 6.37 (1H, doublet, J=3 Hz);
3.89-3.78 (1H, multiplet); 3.52 (2H, singlet);
3.01-2.96 (2H, multiplet); 2.12-1.96 (4H, multiplet);
1.94-1.86 (2H, multiplet).

72(ii) 1-(1-Benzylpiperidin-4-yl)-3-(4-fluorophenyl)-2-(2-methylaminopyridin-4-yl)-1*H*-pyrrole

A mixture of a solution of 1.50 g (3.49 mmol) of 1-(1-benzylpiperidin-4-yl)-3-(4-fluorophenyl)-2-(2-fluoropyridin-4-yl)-1*H*-pyrrole [prepared as described in step 72(i) above] in 30 ml of tetrahydrofuran and 50 ml (100 mmol) of a 2M solution of methylamine in tetrahydrofuran was stirred at 150°C in an autoclave for 28 hours. At the end of this time, the solvent was distilled off from the reaction mixture under reduced pressure. Ethyl acetate and water were added to the residue, and the organic layer was separated, dried over anhydrous magnesium sulfate and then concentrated by evaporation under reduced pressure. The residue thus obtained was purified by chromatography on a silica gel column using a 20:1 by volume mixture of dichloromethane and methanol as the eluant to give 1.83 g (yield 60%) of the title compound as a pale brown amorphous solid.
$^1$H-Nuclear magnetic resonance spectrum (400 MHz, $CDCl_3$) δ ppm:

8.10 (1H, doublet, J=5 Hz); 7.35-7.23 (5H, multiplet);
7.12 (2H, doublet of doublets, J=9 Hz, 5 Hz);
6.92 (1H, doublet, J=3 Hz); 6.88 (2H, triplet, J=9 Hz);
6.48 (1H, doublet, J=5 Hz); 6.37 (1H, doublet, J=3 Hz);
6.20 (1H, singlet); 4.66-4.57 (1H, multiplet);
3.93-3.84 (1H, multiplet); 3.51 (2H, singlet);
2.99-2.91 (2H, multiplet); 2.84 (3H, doublet, J=5 Hz);
2.06-1.85 (6H, multiplet).

72 (iii) 3-(4-Fluorophenyl)-2-(2-methylaminopyridin-4-yl)-1-(piperidin-4-yl)-1*H*-pyrrole

In a similar manner to that described in Example 9(ii) above, 1-(1-benzyl-piperidin-4-yl)-3-(4-fluorophenyl)-2-(2-methyl-aminopyridin-4-yl)-1*H*-pyrrole [prepared as described in step 72(ii) above] was debenzylated to give the title compound (yield 52%) as a white powder.
Melting point: 165 - 167°C
$^1$H-Nuclear magnetic resonance spectrum (400 MHz, $CDCl_3$) δ ppm:

8.12 (1H, doublet, J=5 Hz);
7.13 (2H, doublet of doublets, J=9 Hz, 5 Hz);
6.93 (1H, doublet, J=3 Hz); 6.88 (2H, triplet, J=9 Hz);
6.49 (1H, doublet of doublets, J=5 Hz, 1 Hz) ;
6.38 (1H, doublet, J=3 Hz); 6.21 (1H, doublet, J=1 Hz);
4.65-4.55 (1H, multiplet); 4.02-3.97 (1H, multiplet);
3.16 (2H, broad doublet, J=12 Hz);
2.85 (3H, doublet, J=5 Hz);
2.60 (2H, doublet of triplets, J=12 Hz, 3 Hz);
1.99-1.81 (4H, multiplet).

Example 73

2-(4-Fluorophenyl)-3-[2-(1(S)-phenylethylamino)pyridin-4-yl]-4-(1,2,3,6-tetrahydropyridin-4-yl)-1*H*-pyrrole (Compound No. 1-2600)

73(i) 4-Bromo-2-(4-fluorophenyl)-3-(2-fluoropyridin-4-yl)-1-triisopropylsilyl-*1H*-pyrrole

In a similar manner to the procedures described in Examples 1(i), 7(i), 7(ii) and 7(iii) above, a pyrrole ring-forming reaction, a decarboxylation reaction, a silylation reaction and a bromination reaction were carried out using ethyl 3-(2-fluoropyridin-4-yl)acrylate as a starting material instead of ethyl 3-(4-pyridyl)acrylate to give the title compound (yield 48%) as a pale yellow powder.
$^1$H-Nuclear magnetic resonance spectrum (400 MHz, DMSO-$d_6$) δ ppm:

8.05 (1H, doublet, J=5 Hz);
7.38 (2H, doublet of doublets, J=9 Hz, 5 Hz);
7.20 (2H, triplet, J=9 Hz); 7.15 (1H, singlet);
7.01-6.97 (1H, multiplet); 6.78 (1H, singlet);
1.20-1.07 (3H, multiplet); 0.99 (18H, doublet, J=7 Hz).

73(ii) 4-(1-Allyloxycarbonyl-1,2,3,6-tetrahydropyridin-4-yl)-2-(4-fluorophenyl)-3-(2-fluoropyridin-4-yl)-1*H*-pyrrole

In a similar manner to the procedures described in Examples 9(i) and 9 (iii) above, coupling, dehydration and desilylation reactions were carried out using 4-bromo-2-(4-fluorophenyl)-3-(2-fluoropyridin-4-yl)-1-triisopropylsilyl-1*H*-pyrrole [prepared as described in step 73 (i) above] instead of 4-bromo-2-(4-fluorophenyl)-3-(pyridin-4-yl)-1-triisopropylsilyl-1*H*-pyrrole and using 1-allyloxycarbonylpiperidine-4-one instead of 1-benzylpiperidine-4-one to give the title compound (yield 6%) as a white powder.
$^1$H-Nuclear magnetic resonance spectrum (500 MHz, $CDCl_3$) δ ppm:

8.42-8.31 (1H, broad singlet); 8.09 (1H, doublet, J=5 Hz);
7.15 (2H, doublet of doublets, J=9 Hz, 5 Hz);

7.04-6.97 (3H, multiplet); 6.82 (1H, singlet);
6.77 (1H, singlet); 6.00-5.89 (1H, multiplet);
5.63-5.39 (1H, multiplet); 5.31 (1H, doublet, J=16 Hz);
5.21 (1H, doublet, J=11 Hz); 4.62 (2H, doublet, J=5 Hz);
4.04-3.96 (2H, broad singlet); 3.63-3.52 (2H, broad singlet);
2.29-2.12 (2H, multiplet).

73 (iii) 4-(1-Allyloxycarbonyl-1,2,3,6-tetrahydropyridin-4-yl)-2-(4-fluorophenyl)-3-[2-(1(S)-phenylethylamino)pyridin-4-yl]-1*H*-pyrrole

A mixture of 360 mg (0.85 mmol) of 4-(1-allyloxycarbonyl-1,2,3,6-tetrahydropyridin-4-yl)-2-(4-fluorophenyl)-3-(2-fluoropyridin-4-yl)-1*H*-pyrrole [prepared as described in step 73 (ii) above], 3.6 ml (28.3 mmol) of 1(S)-phenylethylamine and 0.36 ml of concentrated hydrochloric acid was stirred at 150°C for 10 hours. At the end of this time, a saturated aqueous solution of sodium hydrogencarbonate was added to the reaction mixture and then this was extracted with ethyl acetate. The organic extract was washed with water, dried over anhydrous magnesium sulfate and then concentrated by evaporation under reduced pressure. The residue thus obtained was purified by chromatography on a silica gel column using a 1:1 by volume mixture of hexane and ethyl acetate as the eluant to give 229 mg (yield 49%) of the title compound as a brown amorphous solid.
$^1$H-Nuclear magnetic resonance spectrum (500 MHz, $CDCl_3$) δ ppm:

8.20-8.12 (1H, broad singlet); 7.96 (1H, doublet, J=5 Hz);
7.25 (4H, singlet); 7.22-7.16 (1H, multiplet);
7.04 (2H, doublet of doublets, J=9 Hz, 5 Hz);
6.89 (2H, triplet, J=9 Hz); 6.74 (1H, singlet);
6.44 (1H, doublet, J=4 Hz); 6.10 (1H, singlet);
6.00-5.89 (1H, multiplet); 5.54-5.33 (1H, multiplet);
5.32 (1H, doublet, J=16 Hz); 5.22 (1H, doublet, J=11 Hz);
5.05 (1H, broad doublet, J=6 Hz); 4.62 (2H, doublet, J=5 Hz);
4.54-4.46 (1H, multiplet); 3.93-3.83 (2H, multiplet);
3.50-3.38 (2H, multiplet); 2.20-2.05 (2H, multiplet);
1.48 (3H, doublet, J=7 Hz).

73(iv) 2-(4-Fluorophenyl)-3-[2-(1(S)-phenylethylamino)pyridin-4-yl]-4-(1,2,3,6-tetrahydropyridin-4-yl)-1*H*-pyrrole

A mixture of 229 mg (0.44 mmol) of 4-(1-allyloxycarbonyl-1,2,3,6-tetrahydropyridin-4-yl)-2-(4-fluorophenyl)-3-[2-(1(S)-phenylethylamino)pyridin-4-yl]-1*H*-pyrrole [prepared as described in step 73(iii) above] in 2.5 ml of a 10:1 by volume mixture of dioxane and water, 55 μl (0.66 mmol) of pyrrolidine and 5 mg (0.0044 mmol) of tetrakis(triphenylphosphine)palladium (0) was stirred at 0°C for 10 minutes. At the end of this time, water was added to the reaction mixture and then this was extracted with ethyl acetate. The organic extract was washed with water and then concentrated by evaporation under reduced pressure. The residue thus obtained was purified by chromatography on a silica gel column using a 10:1:1 by volume mixture of ethyl acetate, methanol and isopropylamine as the eluant to give 41 mg (yield 21%) of the title compound as a pale brown amorphous solid.
$^1$H-Nuclear magnetic resonance spectrum (500 MHz, $CDCl_3$) δ ppm:

8.19-8.01 (1H, broad singlet); 7.96 (1H, doublet, J=5 Hz);
7.26 (4H, singlet); 7.24-7.18 (1H, multiplet);
7.06 (2H, doublet of doublets, J=9 Hz, 5 Hz);
6.89 (2H, triplet, J=9 Hz); 6.74 (1H, singlet);
6.47 (1H, doublet, J=4 Hz); 6.15 (1H, singlet);
5.47 (1H, doublet, J=3 Hz); 5.05 (1H, broad doublet, J=6 Hz);
4.60-4.52 (1H, multiplet); 3.31-3.26 (2H, multiplet);
2.94-2.84 (2H, multiplet); 2.13-2.07 (2H, multiplet);
1.48 (3H, doublet, J=7 Hz).

Example 74

4-(1-t-Butyl-1,2,3,6-tetrahydropyridin-4-yl)-2-(4-fluorophenyl)-3-(pyridin-4-yl)-1*H*-pyrrole (Compound No. 1-2635)

In a similar manner to the procedures described in Examples 9(i), 9(ii) and 9(iii) above, coupling, dehydration and desilylation reactions were carried out using 1-t-butylpiperidine-4-one as a starting material instead of 1-benzylpiperidine-4-one to give the title compound (yield 20%) as a pale yellow powder.
Melting point: 242 - 244°C
$^1$H-Nuclear magnetic resonance spectrum (400 MHz, $CDCl_3$-DMSO-$d_6$) δ ppm:

9.72 (1H, broad singlet); 8.46 (2H, doublet, J=6 Hz);
7.19 (2H, doublet, J=6 Hz);
7.16 (2H, doublet of doublets, J=9 Hz, 5 Hz);
6.94 (2H, triplet, J=9 Hz); 6.81 (1H, doublet, J=3 Hz);
5.46 (1H, singlet); 3.19-3.11 (2H, multiplet);
2.66-2.58 (2H, multiplet); 2.26-2.18 (2H, multiplet);
1.10 (9H, singlet).

Example 75

2-(4-Fluorophenyl)-4-(1-octyl-1,2,3,6-tetrahydropyridin-4-yl)-3-(pyridin-4-yl)-1*H*-pyrrole (Compound No. 1-2639)

In a similar manner to the procedures described in Examples 9(i) and 9(iii) above, coupling, dehydration and desilylation reactions were carried out using 1-octylpiperidine-4-one as a starting material instead of 1-benzylpiperidine-4-one to give the title compound (yield 26%) as a white powder.
Melting point: 173 - 175°C
$^1$H-Nuclear magnetic resonance spectrum (400 MHz, $CDCl_3$) δ ppm:

8.47 (2H, doublet, J=6 Hz); 8.29 (1H, broad singlet);
7.17 (2H, doublet, J=6 Hz);
7.12 (2H, doublet of doublets, J=9 Hz, 5 Hz) ;
6.96 (2H, triplet, J=9 Hz); 6.82 (1H, doublet, J=3 Hz);
5.45 (1H, singlet); 2.99 (2H, broad doublet, J=3 Hz) ;
2.56 (2H, triplet, J=6 Hz); 2.39 (2H, triplet, J=8 Hz);
2.28-2.23 (2H, multiplet); 1.55-1.48 (2H, multiplet);
1.31-1.22 (10H, multiplet); 0.88 (3H, triplet, J=7 Hz).

Example 76

(±)-4- (1-Ethyl-6-methyl-1,2,3,6-tetrahydropyridin-4-yl)-2-(4-fluorophenyl)-3-(pyridin-4-yl)-1*H*-pyrrole (Compound No. 1-2667)

In a similar manner to the procedures described in Examples 9(i) and 9(iii) above, coupling, dehydration and desilylation reactions were carried out using (±)-1-ethyl-2-methylpiperidine-4-one as a starting material instead of 1-benzylpiperidine-4-one to afford a mixture of the title compound and the (1-ethyl-2-methyl-1,2,3,6-tetrahydropyridin-4-yl) isomer thereof (which is the compound of Example 77 below). The mixture was separated by chromatography on a silica gel column using a 50:1 by volume mixture of ethyl acetate and isopropylamine as the eluant to give the title compound (yield 33%, Rf value = 0.20) as a pale yellow powder. Melting point: 210 - 212°C (decomposition)
$^1$H-Nuclear magnetic resonance spectrum (400 MHz, $CDCl_3$) δ ppm:

8.47 (2H, doublet, J=6 Hz); 8.31 (1H, broad singlet);
7.17 (2H, doublet, J=6 Hz);
7.13 (2H, doublet of doublets, J=9 Hz, 5 Hz);
6.97 (2H, triplet, J=9 Hz); 6.82 (1H, singlet);
5.27 (1H, singlet); 3.09-3.00 (1H, multiplet);
2.97-2.88 (1H, multiplet); 2.84-2.73 (1H, multiplet);
2.48-2.24 (3H, multiplet); 2.22-2.11 (1H, multiplet);
1.09 (3H, triplet, J=7 Hz); 1.04 (3H, doublet, J=7 Hz).

Example 77

(±)-4-(1-Ethyl-2-methyl-1,2,3,6-tetrahydropyridin-4-yl)-2-(4-fluorophenyl)-3-(pyridin-4-yl)-1*H*-pyrrole (Compound No. 1-2709)

The title compound (yield 46%, Rf value = 0.15) was obtained as an orange powder during the chromatography performed in Example 76.
Melting point: 218 - 220°C (decomposition)
$^1$H-Nuclear magnetic resonance spectrum (400 MHz, $CDCl_3$) δ ppm:

8.47 (2H, doublet, J=6 Hz); 8.32 (1H, broad singlet);
7.17 (2H, doublet, J=6 Hz);
7.12 (2H, doublet of doublets, J=9 Hz, 5 Hz);
6.96 (2H, triplet, J=9 Hz); 6.82 (1H, singlet);
5.44 (1H, singlet); 3.16-2.99 (2H, multiplet);
2.90-2.79 (1H, multiplet); 2.76-2.64 (1H, multiplet);
2.51-2.32 (2H, multiplet); 2.03-1.98 (1H, multiplet);
1.08 (3H, triplet, J=7 Hz); 1.01 (3H, doublet, J=6 Hz).

Example 78

(±)-2-(4-Fluorophenyl)-4-(2,2,3,5,6,7,8,8a-octahydroindolizin-7-yl)-3-(pyridin-4-yl)-1*H*-pyrrole (Compound No. 1-2655)

In a similar manner to that described in Example 11 above, (±)-2-(4-fluorophenyl)-4-(1,2,3,5,8,8a-hexahydroindolizin-7-yl)-3-(pyridin-4-yl)-1*H*-pyrrole (prepared as described in Example 59 above) was reduced to give the title compound (yield 52%) as a white powder.
Melting point : 263 - 265°C
$^1$H-Nuclear magnetic resonance spectrum (500 MHz, DMSO-$d_6$) δ ppm:

11.20 (1H, broad singlet); 8.48 (2H, doublet, J=6 Hz);
7.18-7.12 (4H, multiplet); 7.09 (2H, triplet, J=9 Hz);
6.76 (1H, doublet, J=2 Hz); 2.98-2.92 (1H, multiplet);
2.92-2.86 (1H, broad triplet, J=8 Hz);
2.46 (1H, triplet of triplets, J=13Hz, 3 Hz);
1.98-1.45 (9H, multiplet); 1.30-1.14 (2H, multiplet).

Example 79

(±)-4-(6-Allyl-1-methyl-1,2,3,5-tetrahydropyridin-4-yl)-2-(4-fluorophenyl)-3-(pyridin-4-yl)-1*H*-pyrrole (Compound No. 1-2690)

In a similar manner to the procedures described in Examples 9(i) and 9(iii) above, coupling, dehydration and desilylation reactions were carried out using (±)-2-allyl-1-methylpiperidine-4-one as a starting material instead of 1-benzylpiperidine-4-one to afford a mixture of the title compound and the (2-allyl-1-methyl-1,2,3,6-tetrahydropyridin-4-yl) isomer thereof (which is the compound of Example 80 below). The mixture was separated by chromatography on a silica gel column using a 100:1 by volume mixture of ethyl acetate and isopropylamine as the eluant to give the title compound (yield 20%, Rf value = 0.40) as a pale yellow powder. Melting point : 218 - 220°C
$^1$H-Nuclear magnetic resonance spectrum (500 MHz, $CDCl_3$) δ ppm:

8.48 (2H, doublet, J=6 Hz); 8.39 (1H, broad singlet);
7.17 (2H, doublet, J=6 Hz);
7.11 (2H, doublet of doublets, J=9Hz, 5 Hz);
6.96 (2H, triplet, J=9 Hz); 6.83 (1H, doublet, J=3 Hz);
5.66-5.58 (1H, multiplet); 5.34 (1H, singlet);
4.96 (1H, doublet, J=10 Hz); 4.90 (1H, doublet, J=17 Hz);
2.89-2.87 (1H, multiplet); 2.71-2.67 (1H, multiplet);
2.46-2.43 (2H, multiplet); 2.37 (3H, singlet);

2.35-2.32 (1H, multiplet); 2.12-2.07 (2H, multiplet).

Example 80

(±)-4-(2-Allyl-1-methyl-1,2,3,6-tetrahydropyridin-4-yl)-2-(4-fluorophenyl)-3-(pyridin-4-yl)-1*H*-pyrrole (Compound No. 1-2732)

The title compound (yield 18%, Rf value = 0.35) was obtained as a pale yellow powder during the chromatography performed in Example 79 above.
Melting point : 178 - 180°C
$^1$H-Nuclear magnetic resonance spectrum (500 MHz, $CDCl_3$) δ ppm:

8.47 (2H, doublet, J=6 Hz); 8.38 (1H, broad singlet);
7.17 (2H, doublet, J=6 Hz);
7.12 (2H, doublet of doublets, J=9Hz, 5 Hz);
6.96 (2H, triplet, J=9 Hz); 6.82 (1H, doublet, J=3 Hz);
5.77-5.69 (1H, multiplet); 5.39 (1H, singlet);
5.03 (1H, doublet, J=4 Hz); 5.00 (1H, singlet);
3.19-3.15 (1H, multiplet); 2.99-2.95 (1H, multiplet);
2.57-2.41 (1H, multiplet); 2.36 (3H, singlet);
2.36-2.31 (1H, multiplet); 2.24-2.09 (3H, multiplet).

Example 81

(+)-4-(6-Benzyl-1-methyl-1,2,3,6-tetrahydropyridin-4-yl)-2-(4-fluorophenyl)-3-(pyridin-4-yl)-1*H*-pyrrole (Compound No. 1-2696)

In a similar manner to the procedures described in Examples 9(i) and 9(iii) above, coupling, dehydration and desilylation reactions were carried out using (±)-2-benzyl-1-methylpiperidine-4-one as a starting material instead of 1-benzylpiperidine-4-one to afford a mixture of the title compound and the (2-benzyl-1-methyl-1,2,3,6-tetrahydropyridin-4-yl) isomer thereof (which is the compound of Example 82 below). The mixture was separated by chromatography on a silica gel column using a 100:1 by volume mixture of ethyl acetate and isopropylamine as the eluant to give the title compound (yield 11%, Rf value = 0.38) as a pale yellow powder. Melting point : 198 - 200°C
$^1$H-Nuclear magnetic resonance spectrum (500 MHz, $CDCl_3$) δ ppm:

8.45 (2H, doublet, J=6 Hz); 8.28 (1H, broad singlet);
7.20-7.12 (3H, multiplet); 7.09 (2H, doublet, J=6 Hz);
7.10-7.07 (2H, multiplet); 6.97-6.91 (4H, multiplet);
6.76 (1H, doublet, J=3 Hz); 5.24 (1H, singlet);
3.13-3.10 (1H, multiplet); 2.94-2.89 (2H, multiplet);
2.51 (3H, singlet); 2.49-2.35 (3H, multiplet);
2.11-2.05 (1H, multiplet).

Example 82

(±)-4-(2-benzyl-1-methyl-1,2,3,6-tetrahydropyridin-4-yl)-2-(4-fluorophenyl)-3-(pyridin-4-yl)-1*H*-pyrrole (Compound No. 1-2738)

The title compound (yield 4%, Rf value = 0.33) was obtained as a pale yellow powder during the chromatography performed in Example 81 above.
Melting point: 198 - 199°C
$^1$H-Nuclear magnetic resonance spectrum (500 MHz, $CDCl_3$) δ ppm:

8.55 (1H, broad singlet); 8.43 (2H, doublet, J=6 Hz);
7.23 (2H, triplet, J=7 Hz); 7.16 (2H, triplet, J=7 Hz);
7.12 (2H, doublet, J=6 Hz); 7.10-7.05 (4H, multiplet);
6.94 (2H, triplet, J=9 Hz); 6.72 (1H, doublet, J=3 Hz);
5.40 (1H, singlet); 3.20-3.17 (1H, multiplet);

3.08-3.04 (2H, multiplet); 2.81-2.78 (1H, multiplet);
2.46 (3H, singlet); 2.43-2.38 (1H, multiplet);
2.10-2.00 (2H, multiplet).

Example 83

(-)-2-(4-Fluorophenyl)-4-(1,2,3,5,6,8a-hexahydroindolizin-7-yl)-3-(pyridin-4-yl)-1*H*-pyrrole (Compound No. 1-2653)

(±)-2-(4-Fluorophenyl)-4-(1,2,3,5,6,8a-hexahydroindolizin-7-yl)-3-(pyridin-4-yl)-1*H*-pyrrole (prepared as described in Example 60 above) was subjected to High Performance Liquid Chromatography using a chiral column and employing the conditions described below to give the title compound (retention time: 4.51 minutes, yield 94%) as a pale yellow powder.
Melting point: 204 - 205°C (Decomposition)
$[\alpha]^{27}_{D}$ -50.67° (c=0.975, methanol)
<HPLC>
Column: CHIRALPAK AD (product of Daicel Chemical Industries, Ltd.)
Eluant: n-hexane : ethanol = 80:20
Flow rate: 1.0 ml/minute
Temperature: 40°C
Detection: 254nm (UV).

Example 84

(+)-2-(4-Fluorophenyl)-4-(1,2,3,5,6,8a-hexahydroindolizin-7-yl)-3-(pyridin-4-yl)-1*H*-pyrrole (Compound No. 1-2653)

The title compound (retention time: 6.23 minutes, yield 95%) was obtained as a pale yellow powder during the High Performance Liquid Chromatography performed in Example 83 above.
Melting Point: 207-210°C (Decomposition) $[\alpha]^{24}_{D}$ +46.59° (c=0.920, methanol)
<HPLC>
The same as described in Example 83.

Example 85

(±)-2-(4-Fluorophenyl)-4-(6-methyl-1-propyl-1,2,3,6-tetrahydropyridin-4-yl)-3-(pyridin-4-yl)-1*H*-pyrrole (Compound No. 1-2668)

In a similar manner to the procedures described in Examples 9(i) and 9(iii) above, coupling, dehydration and desilylation reactions were carried out using (±)-2-methyl-1-propylpiperidine-4-one as a starting material instead of 1-benzylpiperidine-4-one to afford a mixture of the title compound and the (2-methyl-1-propyl-1,2,3,6-tetrahydropyridin-4-yl) isomer thereof (which is the compound of Example 86 below). The mixture was separated by chromatography on a silica gel column using a 100:1 by volume mixture of ethyl acetate and isopropylamine as the eluant to give the title compound (yield 6%, Rf value = 0.35) as a white powder. Melting point : 214 - 215°C (decomposition)
$^{1}$H-Nuclear magnetic resonance spectrum (400 MHz, $CDCl_3$) δ ppm:

8.47 (2H, doublet, J=6 Hz); 8.22 (1H, broad singlet);
7.17 (2H, doublet, J=6 Hz);
7.13 (2H, doublet of doublets, J=9Hz, 5 Hz);
6.97 (2H, triplet, J=9 Hz); 6.83 (1H, singlet);
5.27 (1H, singlet); 3.07-2.98 (1H, multiplet);
2.97-2.89 (1H, multiplet); 2.68-2.56 (1H, multiplet);
2.47-2.39 (1H, multiplet); 2.37-2.23 (2H, multiplet);
2.20-2.10 (1H, multiplet); 1.62-1.43 (2H, multiplet);
1.04 (3H, doublet, J=7 Hz); 0.90 (3H, triplet, J=7 Hz).

Example 86

(±) -2-(4-Fluorophenyl)-4-(2-methyl-1-propyl-1,2,3,6-tetrahydropyridin-4-yl)-3-(pyridin-4-yl)-1*H*-pyrrole (Compound No. 1-2710)

The title compound (yield 8%, Rf value = 0.32) was obtained as a white powder during the chromatography performed in Example 85 above.
Melting point : 210 - 218°C (decomposition)
$^1$H-Nuclear magnetic resonance spectrum (400 MHz, $CDCl_3$) δ ppm:

8.47 (2H, doublet, J=6 Hz); 8.23 (1H, broad singlet);
7.17 (2H, doublet, J=6 Hz);
7.12 (2H, doublet of doublets, J=9Hz, 5 Hz);
6.96 (2H, triplet, J=9 Hz); 6.82 (1H, singlet);
5.43 (1H, singlet); 3.16-3.01 (2H, multiplet);
2.89-2.82 (1H, multiplet); 2.58-2.50 (1H, multiplet);
2.42-2.30 (2H, multiplet); 2.00-1.92 (1H, multiplet);
1.57-1.45 (2H, multiplet); 1.01 (3H, doublet, J=6 Hz);
0.90 (3H, triplet, J=7 Hz).

Example 87

(±)-2-(4-Fluorophenyl)-4-(1,3,4,6,7,9a-hexahydro-2*H*-quinolizin-8-yl)-3-(pyridin-4-yl)-1*H*-pyrrole (Compound No. 1-2656)

In a similar manner to the procedures described in Examples 9(i) and 9(iii) above, coupling, dehydration and desilylation reactions were carried out using (±)-octahydro-2H-quinolizine-2-one as a starting material instead of 1-benzylpiperidine-4-one to afford a mixture of the title compound and the (1,3,4,6,9,9a-hexahydro-2H-quinolizin-8-yl) isomer thereof (which is the compound of Example 88 below). The mixture was separated by chromatography on a silica gel column using a 100:1 by volume mixture of ethyl acetate and isopropylamine as the eluant to give the title compound (yield 6%, Rf value = 0.35) as a pale yellow powder.
Melting point : 223 - 224°C
$^1$H-Nuclear magnetic resonance spectrum (500 MHz, $CDCl_3$) δ ppm:

8.47 (2H, doublet, J=6 Hz); 8.30 (1H, broad singlet);
7.17 (2H, doublet, J=6 Hz);
7.12 (2H, doublet of doublets, J=9Hz, 5 Hz);
6.96 (2H, triplet, J=9 Hz); 6.83 (1H, doublet, J=3 Hz);
5.13 (1H, singlet); 2.88-2.81 (2H, multiplet);
2.67-2.56 (1H, multiplet); 2.45-2.43 (1H, multiplet);
2.40-2.35 (1H, multiplet); 2.17-2.07 (2H, multiplet);
1.76-1.73 (1H, multiplet); 1.69-1.64 (2H, multiplet);
1.44-1.41 (1H, multiplet); 1.34-1.18 (2H, multiplet).

Example 88

(±)-2-(4-Fluorophenyl)-4-(1,3,4,6,9,9a-hexahydro-2H-quinolizin-8-yl)-3-(pyridin-4-yl)-1*H*-pyrrole (Compound No. 1-2657)

The title compound (yield 19%, Rf value = 0.30) was obtained as a pale yellow powder during the chromatography performed in Example 87 above.
Melting point : 228 - 234°C (decomposition)
$^1$H-Nuclear magnetic resonance spectrum (500 MHz, $CDCl_3$) δ ppm:

8.47 (2H, doublet, J=6 Hz); 8.28 (1H, broad singlet);
7.17 (2H, doublet, J=6 Hz);
7.12 (2H, doublet of doublets, J=9Hz, 5 Hz);
6.96 (2H, triplet, J=9 Hz); 6.81 (1H, doublet, J=3 Hz);

5.42 (1H, singlet); 3.30-3.25 (1H, multiplet);
2.98-2.95 (1H, multiplet); 2.77-2.73 (1H, multiplet);
2.14-1.99 (5H, multiplet); 1.74-1.65 (3H, multiplet);
1.31-1.21 (2H, multiplet).

Example 89

(±)-2-(4-Fluorophenyl)-4-(1,2,3,5,8,8a-hexahydroindolizin-7-yl)-3-(2-methylaminopyrimidin-4-yl)-1*H*-pyrrole (Compound No. 1-2906)

In a similar manner to the procedures described in Examples 9(i) and 9(iii) above, coupling, dehydration and desilylation reactions were carried out using 4-bromo-2-(4-fluorophenyl)-3-(2-methylaminopyrimidin-4-yl)-1-triisopropylsilyl-1*H*-pyrrole (prepared as described in Preparative Example 1 below) instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 90 below). The mixture was separated by chromatography on a silica gel column using a 200:20:1 by volume mixture of ethyl acetate, methanol and isopropylamine as the eluant to give the title compound (yield 15%, Rf value = 0.55) as a pale brown amorphous solid.
$^1$H-Nuclear magnetic resonance spectrum (400 MHz, $CD_3OD$) δ ppm:

8.08 (1H, doublet, J=5 Hz);
7.30 (2H, doublet of doublets, J=9Hz, 5 Hz);
7.02 (2H, triplet, J=9 Hz); 6.82 (1H, singlet);
6.49 (1H, doublet, J=5 Hz); 5.57-5.51 (1H, multiplet);
3.53-3.45 (1H, multiplet); 3.21-3.11 (1H, multiplet);
2.90-2.81 (1H, multiplet); 2.80 (3H, singlet);
2.47-2.28 (2H, multiplet); 2.26-2.13 (2H, multiplet);
2.07-1.96 (1H, multiplet); 1.91-1.73 (2H, multiplet);
1.49-1.37 (1H, multiplet).

Example 90

(±)-2-(4-Fluorophenyl)-4-(1,2,3,5,6,8a-hexahydroindolizin-7-yl)-3-(2-methylaminopyrimidin-4-yl)-1*H*-pyrrole (Compound No. 1-2905)

The title compound (yield 12%, Rf value = 0.50) was obtained as a pale brown amorphous solid during the chromatography performed in Example 89 above.
$^1$H-Nuclear magnetic resonance spectrum (400 MHz, $CD_3OD$) δ ppm:

8.09 (1H, doublet, J=5 Hz);
7.30 (2H, doublet of doublets, J=9Hz, 5 Hz);
7.02 (2H, triplet, J=9 Hz); 6.83 (1H, singlet);
6.46 (1H, doublet, J=5 Hz); 5.48-5.42 (1H, multiplet);
3.47-3.38 (1H, multiplet); 3.03-2.75 (4H, multiplet);
2.81 (3H, singlet); 2.46-2.36 (1H, multiplet);
2.29-2.20 (1H, multiplet); 2.03-1.87 (2H, multiplet);
1.83-1.75 (1H, multiplet); 1.54-1.43 (1H, multiplet).

Example 91

(±)-2-(3,4-Difluorophenyl)-4-(1,2,3,5,8,8a-hexahydroindolizin-7-yl)-3-(pyridin-4-yl)-1*H*-pyrrole (Compound No. 1-3260)

In a similar manner to the procedures described in Examples 9(i) and 9(iii) above, coupling, dehydration and desilylation reactions were carried out using 4-bromo-2-(3,4-difluorophenyl)-3-(pyridin-4-yl)-1-triisopropylsilyl-1*H*-pyrrole (prepared as described in Preparative Example 5 below), instead of 4-bromo-2-(4-fluorophenyl)-3-(pyridin-4-yl)-1-triisopropylsilyl-1*H*-pyrrole, and (+)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer

thereof (which is the compound of Example 92 below). The mixture was separated by chromatography on a silica gel column using a 100:10:1 by volume mixture of ethyl acetate, methanol and isopropylamine as the eluant to give the title compound (yield 29%, Rf value = 0.66) as a pale pink powder.
Melting point : 215 - 217°C (decomposition)
$^1$H-Nuclear magnetic resonance spectrum (400 MHz, DMSO-$d_6$) δ ppm:

11.48 (1H, broad singlet); 8.48 (2H, doublet, J=6 Hz);
7.37-7.28 (1H, multiplet); 7.21-7.10 (1H, multiplet);
7.14 (2H, doublet, J=6 Hz); 6.96 (1H, doublet, J=3 Hz);
6.93-6.86 (1H, multiplet); 5.27-5.20 (1H, multiplet);
3.33-3.23 (1H, multiplet); 3.05-2.97 (1H, multiplet);
2.65-2.56 (1H, multiplet); 2.30-2.22 (1H, multiplet);
2.12-1.93 (3H, multiplet); 1.91-1.81 (1H, multiplet);
1.75-1.57 (2H, multiplet); 1.33-1.20 (1H, multiplet).

Example 92

(+)-2-(3,4-Difluorophenyl)-4-(1,2,3,5,6,8a-hexahydroindolizin-7-yl)-3-(pyridin-4-yl)-1*H*-pyrrole (Compound No. 1-3259)

The title compound (yield 23%, Rf value = 0.31) was obtained as a pale pink powder during the chromatography performed in Example 91 above.
Melting point : 187 - 190°C (decomposition)
$^1$H-Nuclear magnetic resonance spectrum (400 MHz, DMSO-$d_6$) δ ppm:

11.48 (1H, broad singlet); 8.47 (2H, doublet, J=6 Hz);
7.39-7.29 (1H, multiplet); 7.21-7.10 (1H, multiplet);
7.14 (2H, doublet, J=6 Hz); 6.96 (1H, doublet, J=3 Hz);
6.94-6.87 (1H, multiplet); 5.18-5.13 (1H, multiplet);
3.10-3.02 (1H, multiplet); 2.89-2.82 (1H, multiplet);
2.79-2.71 (1H, multiplet); 2.63-2.45 (2H, multiplet);
2.30-2.20 (1H, multiplet); 2.06-1.97 (1H, multiplet);
1.78-1.53 (3H, multiplet); 1.22-1.10 (1H, multiplet).

Example 93

(±)-4-(1,2,3,5,8,8a-Hexahydroindolizin-7-yl)-3-(pyridin-4-yl)-2-(3-trifluoromethylphenyl)-1*H*-pyrrole (Compound No. 1-3262)

In a similar manner to the procedures described in Examples 9(i) and 9(iii) above, coupling, dehydration and desilylation reactions were carried out using 4-bromo-3-(pyridin-4-yl)-2-(3-trifluoromethylphenyl)-1-triisopropylsilyl-1*H*-pyrrole (prepared as described in Preparative Example 6 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 94 below). The mixture was separated by chromatography on a silica gel column using a 100:10:1 by volume mixture of ethyl acetate, methanol and isoprppylamine as the eluant to give the title compound (yield 26%, Rf value = 0.65) as a pale brown powder.
Melting point : 220 - 223°C (decomposition)
$^1$H-Nuclear magnetic resonance spectrum (400 MHz, DMSO-$d_6$) δ ppm:

11.62 (1H, broad singlet); 8.49 (2H, doublet, J=6 Hz);
7.54-7.35 (4H, multiplet); 7.16 (2H, doublet, J=6 Hz);
7.02 (1H, doublet, J=3 Hz); 5.29-5.21 (1H, multiplet);
3.35-3.24 (1H, multiplet); 3.06-2.98 (1H, multiplet);
2.65-2.56 (1H, multiplet); 2.34-2.24 (1H, multiplet);
2.13-1.94 (3H, multiplet); 1.92-1.82 (1H, multiplet);
1.75-1.58 (2H, multiplet); 1.34-1.21 (1H, multiplet).

Example 94

(±)-4-(1,2,3,5,6,8a-Hexahydroindolizin-7-yl)-3-(pyridin-4-yl)-2-(3-trifluoromethylphenyl)-1*H*-pyrrole (Compound No. 1-3261)

The title compound (yield 17%, Rf value = 0.31) was obtained as a pale brown powder during the chromatography performed in Example 93 above.
Melting point : 189 - 191°C (decomposition)
$^1$H-Nuclear magnetic resonance spectrum (400 MHz, DMSO-$d_6$) δ ppm:

11.63 (1H, broad singlet); 8.48 (2H, doublet, J=6 Hz); 7.55-7.37 (4H, multiplet); 7.15 (2H, doublet, J=6 Hz) ; 7.02 (1H, doublet, J=3 Hz); 5.19-5.14 (1H, multiplet); 3.12-3.03 (1H, multiplet); 2.91-2.82 (1H, multiplet); 2.80-2.71 (1H, multiplet); 2.64-2.45 (2H, multiplet); 2.33-2.21 (1H, multiplet); 2.09-1.98 (1H, multiplet); 1.78-1.53 (3H, multiplet); 1.21-1.10 (1H, multiplet).

Example 95

(+)-2-(3-Fluorophenyl)-4-(1,2,3,5,8,8a-hexahydroindolizin-7-yl)-3-(pyridin-4-yl)-1*H*-pyrrole (Compound No. 1-3256)

In a similar manner to the procedures described in Examples 9(i) and 9(iii) above, coupling, dehydration and desilylation reactions were carried out using 4-bromo-2-(3-fluorophenyl)-3-(pyridin-4-yl)-1-triisopropylsilyl-1*H*-pyrrole (prepared as described in Preparative Example 4 below), instead of 4-bromo-2-(4-fluorophenyl)-3-(pyridin-4-yl)-1-triisopropylsilyl-1*H*-pyrrole, and (+)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 96 below). The mixture was separated by chromatography on a silica gel column using a 30:1 by volume mixture of ethyl acetate and isopropylamine as the eluant to give the title compound (yield 17%, Rf value = 0.25) as a pale pink powder.
Melting point : 199 - 203°C (decomposition)
$^1$H-Nuclear magnetic resonance spectrum (400 MHz, $CDCl_3$) δ ppm:

8.49 (2H, doublet, J=6 Hz); 8.30 (1H, broad singlet); 7.23-7.18 (3H, multiplet); 6.96-6.82 (4H, multiplet); 5.50-5.47 (1H, multiplet); 3.53-2.96 (1H, multiplet); 3.20 (1H, triplet, J=9 Hz); 2.79 (1H, doublet, J=17 Hz); 2.32-2.08 (4H, multiplet); 2.00-1.67 (3H, multiplet); 1.48-1.33 (1H, multiplet).

Example 96

(±)-2-(3-Fluorophenyl)-4-(1,2,3,5,6,8a-hexahydroindolizin-7-yl)-3-(pyridin-4-yl)-1*H*-pyrrole (Compound No. 1-3255)

The title compound (yield 12%, Rf value = 0.10) was obtained as a pale brown powder during the chromatography performed in Example 95 above.
Melting point : 178 - 181°C (decomposition)
$^1$H-Nuclear magnetic resonance spectrum (400 MHz, $CDCl_3$) δ ppm:

8.50 (2H, doublet, J=6 Hz); 8.34 (1H, broad singlet) ; 7.26-7.18 (3H, multiplet); 6.95-6.84 (4H, multiplet); 5.42 (1H, singlet); 3.23-3.17 (1H, multiplet); 3.02-2.82 (2H, multiplet); 2.78-2.61 (2H, multiplet); 2.42-2.30 (1H, multiplet); 2.16-2.04 (1H, multiplet); 1.95-1.68 (3H, multiplet); 1.44-1.32 (1H, multiplet).

Example 97

(±)-2-(3-Chlorophenyl)-4-(1,2,3,5,8,8a-hexahydroindolizin-7-yl)-3-(pyridin-4-yl)-1*H*-pyrrole (Compound No. 1-3258)

In a similar manner to the procedures described in Examples 9(i) and 9(iii) above, coupling, dehydration and desilylation reactions were carried out using 4-bromo-2-(3-chlorophenyl)-3-(pyridin-4-yl)-1-triisopropylsilyl-1*H*-pyrrole (prepared as described in Preparative Example 3 below), instead of 4-bromo-2-(4-fluorophenyl)-3-(pyridin-4-yl)-1-triisopropylsilyl-1*H*-pyrrole, and (±)-1,2,3,5,6,7,8, 8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 98 below). The mixture was separated by chromatography on a silica gel column using a 30:1 by volume mixture of ethyl acetate and isopropylamine as the eluant to give the title compound (yield 18%, Rf value = 0.25) as a pale pink powder.
Melting point : 197 - 201°C (decomposition)
$^1$H-Nuclear magnetic resonance spectrum (400 MHz, $CDCl_3$) δ ppm:

8.49 (2H, doublet, J=6 Hz); 8.32 (1H, broad singlet);
7.25-7.13 (5H, multiplet); 6.96 (1H, doublet, J=7 Hz);
6.85 (1H, doublet, J=3 Hz); 5.50-5.47 (1H, multiplet);
3.54-3.45 (1H, multiplet); 3.20 (1H, triplet, J=9 Hz) ;
2.79 (1H, doublet, J=16 Hz); 2.31-2.07 (4H, multiplet);
2.00-1.67 (3H, multiplet); 1.48-1.33 (1H, multiplet).

Example 98

(±)-2-(3-Chlorophenyl)-4-(1,2,3,5,6,8a-hexahydroindolizin-7-yl)-3-(pyridin-4-yl)-1*H*-pyrrole (Compound No. 1-3257)

The title compound (yield 16%, Rf value = 0.10) was obtained as a pale brown powder during the chromatography performed in Example 97 above.
Melting point : 193 - 195°C (decomposition)
$^1$H-Nuclear magnetic resonance spectrum (400 MHz, $CDCl_3$) δ ppm:

8.50 (2H, doublet, J=6 Hz); 8.42 (1H, broad singlet);
7.25-7.13 (5H, multiplet); 6.96 (1H, doublet, J=7 Hz);
6.85 (1H, doublet, J=3 Hz); 5.42 (1H, singlet);
3.26-3.17 (1H, multiplet); 3.02-2.82 (2H, multiplet);
2.78-2.60 (2H, multiplet); 2.42-2.30 (1H, multiplet);
2.16-2.03 (1H, multiplet); 1.95-1.65 (3H, multiplet);
1.45-1.32 (1H, multiplet).

Example 99

(±)-4-(1,2,3,5,8,8a-Hexahydroindolizin-7-yl)-2-phenyl-3-(pyridin-4-yl)-1*H*-pyrrole (Compound No. 1-3254)

In a similar manner to the procedures described in Examples 9(i) and 9(iii) above, coupling, dehydration and desilylation reactions were carried out using 4-bromo-2-phenyl-3-(pyridin-4-yl)-1-triisopropylsilyl-1*H*-pyrrole (prepared as described in Preparative Example 2 below), instead of 4-bromo-2-(4-fluorophenyl)-3-(pyridin-4-yl)-1-triisopropylsilyl-1*H*-pyrrole, and (±)-1,2,3,5,6,7,8,8a-octahydroindolizine-7-one, instead of 1-benzylpiperidine-4-one, as starting materials to afford a mixture of the title compound and the (1,2,3,5,6,8a-hexahydroindolizin-7-yl) isomer thereof (which is the compound of Example 100 below). The mixture was separated by chromatography on a silica gel column using a 30:1 by volume mixture of ethyl acetate and isopropylamine as the eluant to give the title compound (yield 14%, Rf value = 0.24) as a pale brown powder.
Melting point : 201 - 204°C (decomposition)
$^1$H-Nuclear magnetic resonance spectrum (400 MHz, $CDCl_3$) δ ppm:

8.46 (2H, doublet, J=6 Hz); 8.28 (1H, broad singlet);
7.30-7.13 (7H, multiplet); 6.84 (1H, doublet, J=3 Hz);
5.50-5.47 (1H, multiplet); 3.54-3.45 (1H, multiplet);
3.50 (1H, triplet, J=9 Hz); 2.79 (1H, doublet, J=17 Hz);

2.32-2.07 (4H, multiplet); 2.00-1.67 (3H, multiplet);
1.48-1.33 (1H, multiplet).

Example 100

(±)-4-(1,2,3,5,6,8a-Hexahydroindolizin-7-yl)-2-phenyl-3-(pyridin-4-yl)-1*H*-pyrrole (Compound No. 1-3253)

The title compound (yield 10%, Rf value = 0.11) was obtained as a pale brown powder during the chromatography performed in Example 99 above.
Melting point : 180 - 183°C (decomposition)
$^1$H-Nuclear magnetic resonance spectrum (400 MHz, $CDCl_3$) δ ppm:

8.47 (2H, doublet, J=6 Hz); 8.34 (1H, broad singlet);
7.30-7.13 (7H, multiplet); 6.84 (1H, doublet, J=3 Hz);
5.44 (1H, singlet); 3.27-3.18 (1H, multiplet);
3.02-2.82 (2H, multiplet); 2.78-2.60 (2H, multiplet);
2.43-2.30 (1H, multiplet); 2.17-2.06 (1H, multiplet);
1.95-1.65 (3H, multiplet); 1.45-1.32 (1H, multiplet).

[Preparative Examples]

Preparative Example 1

4-Bromo-2-(4-fluorophenyl)-3-(2-methylaminopyrimidin-4-yl)-1-triisopropylsilyl-1*H*-pyrrole

1(i) 2-(4-Fluorophenyl)-3-(2-methylthiopyrimidin-4-yl)-1*H*-pyrrole

In a similar manner to the procedures described in Example 1(i) above (pyrrole ring forming reaction) and Example 7(i) above (decarboxylation), reactions were carried out using ethyl 3-(2-methylthiopyrimidin-4-yl)acrylate instead of ethyl 3-(4-pyridyl)acrylate as a starting material to afford the title compound (yield 37%) as a white powder.
$^1$H-Nuclear magnetic resonance spectrum (400 MHz, $CDCl_3$) δ ppm:

8.41 (1H, broad singlet); 8.24 (1H, doublet, J=5 Hz);
7.42 (1H, doublet of doublets, J=9Hz, 5 Hz);
7.10 (2H, triplet, J=9 Hz); 6.88 (1H, triplet, J=3 Hz);
6.84 (1H, doublet, J=5 Hz); 6.81 (1H, triplet, J=3 Hz);
2.33 (3H, singlet).

1(ii) 2-(4-Fluorophenyl)-3-(2-methanesulfonylpyrimidin-4-yl)-1*H*-pyrrole

In a similar manner to that described in Example 42(ii) above, 2-(4-fluorophenyl)-3-(2-methylthiopyrimidin-4-yl)-1*H*-pyrrole [which was prepared as described in step 1(i) above] was oxidized to afford the title compound (yield 81%) as a pale yellow powder.
$^1$H-Nuclear magnetic resonance spectrum (400 MHz, $CDCl_3$-$CD_3OD$) δ ppm:

8.58 (1H, doublet, J=6 Hz);
7.50 (2H, doublet of doublets, J=9Hz, 6 Hz);
7.47 (1H, doublet, J=6 Hz); 7.16 (2H, triplet, J=9 Hz);
6.90 (1H, doublet, J=3 Hz); 6.84 (1H, doublet, J=3 Hz);
3.05 (3H, singlet).

1(iii) 2-(4-Fluorophenyl)-3-(2-methylaminopyrimidin-4-yl)-1*H*-pyrrole

In a similar manner to that described in Example 42(iii) above, 2-(4-fluorophenyl)-3-(2-methanesulfonylpyrimidin-4-yl)-1*H*-pyrrole [prepared as described in step 1(ii) above] was reacted with methylamine to afford the title compound (quantitative yield) as a pale brown amorphous solid.
$^1$H-Nuclear magnetic resonance spectrum (400 MHz, $CDCl_3$) δ ppm:

8.37 (1H, broad singlet); 8.08 (1H, doublet, J=5 Hz);
7.44 (2H, doublet of doublets, J=9Hz, 6 Hz) ;
7.08 (2H, triplet, J=9 Hz); 6.85 (1H, triplet, J=3 Hz);
6.75 (1H, triplet, J=3 Hz); 6.47 (1H, doublet, J=5 Hz);
4.90 (1H, broad quartet, J=5 Hz); 2.87 (3H, doublet, J=5 Hz).

1(iv) 2-(4-Fluorophenyl)-3-(2-methylaminopyrimidin-4-yl)-1-triisopropylsilyl-1*H*-pyrrole

In a similar manner to that described in Example 7(ii) above, 2-(4-fluorophenyl)-3-(2-methylaminopyrimidin-4-yl)-1*H*-pyrrole [prepared as described in step 1(iii) above] was reacted with triisopropylsilyl triflate, to afford the title compound (yield 82%) as a white powder.
$^1$H-Nuclear magnetic resonance spectrum (400 MHz, $CDCl_3$) $\delta$ ppm:

7.92 (1H, doublet, J=5 Hz);
7.36 (2H, doublet of doublets, J=9Hz, 6 Hz);
7.06 (2H, triplet, J=9 Hz); 6.90 (1H, doublet, J=3 Hz);
6.89 (1H, doublet, J=3 Hz); 5.93 (1H, doublet, J=5 Hz);
4.80 (1H, broad quartet, J=5 Hz); 2.83 (3H, doublet, J=5 Hz);
1.15-0.92 (21H, multiplet).

1(v) 3-[2-(N-t-Butoxycarbonyl-N-methylamino)pyrimidin-4-yl]-2- (4-fluorophenyl)-1-triisopropylsilyl-1*H*-pyrrole

In a similar manner to that described in Example 4(i) above, 2-(4-fluorophenyl)-3-(2-methylaminopyrimidin-4-yl)-1-trisopropylsilyl-1*H*-pyrrole [prepared as described in step 1(iv) above] was reacted with di-t-butyl dicarbonate to afford the title compound (yield 90%) as colorless oil.
$^1$H-Nuclear magnetic resonance spectrum (400 MHz, $CDCl_3$) $\delta$ ppm:

8.24 (1H, doublet, J=5 Hz);
7.36 (2H, doublet of doublets, J=9Hz, 5 Hz);
7.09 (2H, triplet, J=9 Hz); 6.95 (1H, doublet, J=3 Hz);
6.90 (1H, doublet, J=3 Hz); 6.28 (1H, doublet, J=5 Hz);
3.20 (3H, singlet); 1.51 (9H, singlet);
1.15-0.94 (21H, multiplet).

1(vi) 4-Bromo-3-[2-(N-t-butoxycarbonyl-N-methylamino)pyrimidin-4-yl]-2-(4-fluorophenyl)-1-triisopropylsilyl-1*H*-pyrrole

In a similar manner to that described in Example 7(iii) above, 3-[2-(N-t-butoxycarbonyl-N-methylamino)pyrimidin-4-yl]-2-(4-fluorophenyl)-1-triisopropylsilyl-1*H*-pyrrole [prepared as described in step 1(v) above] was brominated to afford the title compound (yield 79%) as a pale brown amorphous solid. $^1$H-Nuclear magnetic resonance spectrum (400 MHz, $CDCl_3$) $\delta$ ppm:

8.42 (1H, doublet, J=5 Hz);
7.28 (2H, doublet of doublets, J=9Hz, 5 Hz);
7.00 (2H, triplet, J=9 Hz); 6.93 (1H, singlet);
6.78 (1H, doublet, J=5 Hz); 3.13 (3H, singlet);
1.49 (9H, singlet); 1.15-0.93 (21H, multiplet).

1(vii) 4-Bromo-2-(4-fluorophenyl)-3-(2-methylaminopyrimidin-9-yl)-1*H*-pyrrole

472 mg (0.782 mmol) of 4-bromo-3-[2-(N-t-butoxycarbonyl-N-methylamino)pyrimidin-4-yl]-2-(4-fluorophenyl)-1-triisopropylsilyl-1*H*-pyrrole [prepared as described in step (vi) above] were dissolved in 10 of ml tetrahydrofuran, 0.98 ml (3.91 mmol) of a 4N solution of hydrogen chloride in dioxane were added to the solution, and the resulting mixture was stirred for 3 hours at 50 °C. At the end of this time, water was added to the reaction mixture and then it was neutralized with a saturated aqueous solution of sodium hydrogencarbonate. This was extracted with ethyl acetate, and the organic extract was concentrated under reduced pressure. The resulting residue was purified by chromatography on a silica gel column using a 7:3 by volume mixture of hexane and ethyl acetate as the eluant to afford 202 mg (yield 74%) of the title compound as a pale brown amorphous solid.

$^1$H-Nuclear magnetic resonance spectrum (400 MHz, $CDCl_3$) δ ppm:

8.66 (1H, broad singlet); 8.21(1H, doublet, J=5 Hz);
7.26 (2H, doublet of doublets, J=9Hz, 5 Hz);
7.01 (2H, triplet, J=9 Hz); 6.88 (1H, doublet, J=3 Hz);
6.61 (1H, doublet, J=5 Hz); 4.96 (1H, broad quartet, J=5 Hz);
2.86 (3H, doublet, J=5 Hz).

1(viii) 4-Bromo-2-(4-fluorophenyl)-3-(2-methylaminopyrimidin-4-yl)-1-triisopropylsilyl-1*H*-pyrrole

In a similar manner to the procedures described in Example 7(ii) above, 4-bromo-2-(4-fluorophenyl)-3-(2-methylaminopyrimidin-4-yl)-1*H*-pyrrole [prepared as described in step 1(vii) above] was reacted with triisopropylsilyl triflate to afford the title compound (yield 67%) as a pale brown amorphous solid.
$^1$H-Nuclear magnetic resonance spectrum (400 MHz, $CDCl_3$) δ ppm:

8.04 (1H, doublet, J=5 Hz);
7.30 (2H, doublet of doublets, J=9Hz, 6 Hz);
6.98 (2H, triplet, J=9 Hz); 6.91 (1H, singlet);
6.32 (1H, broad singlet); 4.85 (1H, broad quartet, J=4 Hz);
2.82 (3H, doublet, J=4 Hz); 1.15-0.92 (21H, multiplet).

Preparative Example 2

4-Bromo-2-phenyl-3-(pyridin-4-yl)-1-triisopropylsilyl-1*H*-pyrrole

In a similar manner to the procedures described in Example 1(i) above (pyrrole ring forming reaction), Example 7(i) above (decarboxylation), Example 7(ii) above (reaction with triisopropylsilyl triflate) and Example 7(iii) above (bromination), reactions were carried out using α-(p-toluenesulfonyl)benzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.35 (2H, doublet, J=6 Hz); 7.36-7.30 (1H, multiplet);
7.28-7.21 (4H, multiplet); 7.03 (2H, doublet, J=6 Hz);
6.94 (1H, singlet); 1.15-1.02 (21H, multiplet).

Preparative Example 3

4-Bromo-2-(3-chlorophenyl)-3-(pyridin-4-yl)-1-triisopropylsilyl-1*H*-pyrrole

In a similar manner to the procedures described in Example 1(i) above (pyrrole ring forming reaction), Example 7(i) above (decarboxylation), Example 7(ii) above (reaction with triisopropysilyl triflate) and Example 7(iii) above (bromination), reactions were carried out using α-(p-toluenesulfonyl)-3-chlorobenzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.40 (2H, doublet, J=6 Hz); 7.33-7.29 (2H, multiplet);
7.18 (1H, triplet, J=8 Hz); 7.09 (1H, doublet, J=8 Hz);
7.03 (2H, doublet, J=6 Hz); 6.95 (1H, singlet);
1.12-0.99 (21H, multiplet).

Preparative Example 4

4-Bromo-2-(3-fluorophenyl)-3-(pyridin-4-yl)-1-triisopropylsilyl-1*H*-pyrrole

In a similar manner to the procedures described in Example 1(i) above (pyrrole ring forming reaction), Example 7(i) above (decarboxylation), Example 7(ii) above (reaction with triisopropysilyl triflate) and Example 7(iii) above (bromination), reactions were carried out using α-(p-toluenesulfonyl)-3-fluorobenzylisonitrile, instead of α-(p-toluenesulfonyl)-4-fluorobenzylisonitrile, as a starting material to afford the title compound as a pale purple powder.

$^1$H-Nuclear magnetic resonance spectrum (400 MHz, $CDCl_3$) δ ppm:

8.39 (2H, doublet, J=6 Hz);
7.22 (1H, doublet of doublets, J=8Hz, 6 Hz);
7.07-7.01 (4H, multiplet); 6.98-6.94 (2H, multiplet);
1.14-0.98 (21H, multiplet).

Preparative Example 5

4-Bromo-2-(3,4-difluorophenyl)-3-(pyridin-4-yl)-1-triisopropylsilyl-1*H*-pyrrole

In a similar manner to the procedures described in Example 1(i) above (pyrrole ring forming reaction), Example 7(i) above (decarboxylation), Example 7(ii) above (reaction with triisopropysilyl triflate) and Example 7(iii) above (bromination), reactions were carried out using α-(p-toluenesulfonyl)-3,4-difluorobenzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.42 (2H, doublet, J=6 Hz); 7.12-6.93 (3H, multiplet);
7.02 (2H, doublet, J=6 Hz); 6.94 (1H, singlet);
1.18-0.94 (21H, multiplet).

Preparative Example 6

4-Bromo-2-(3-trifluoromethylphenyl)-3-(pyridin-4-yl)-1-triisopropylsilyl-1*H*-pyrrole

In a similar manner to the procedures described in Example 1(i) above (pyrrole ring forming reaction), Example 7(i) above (decarboxylation), Example 7(ii) above (reaction with triisopropysilyl triflate) and Example 7(iii) above (bromination), reactions were carried out using α-(p-toluenesulfonyl)-3-trifluoromethyl-benzylisonitrile, instead of α-(p-toluenesulfonyl)-4-fluorobenzylisonitrile, as a starting material to afford the title compound as a pale purple powder.
$^1$H-Nuclear magnetic resonance spectrum (400 MHz, $CDCl_3$) δ ppm:

8.39 (2H, doublet, J=6 Hz); 7.64-7.57 (2H, multiplet);
7.40-7.33 (2H, multiplet); 7.00 (2H, doublet, J=6 Hz);
6.98 (1H, singlet); 1.15-0.94 (21H, multiplet).

[Formulation Examples]

A pharmaceutical preparation containing a compound of the present invention having the above formula (I), or a pharmacologically acceptable salt, ester or other derivative thereof as its active ingredient, can be produced according to, for example, the following methods.

Formulation Example 1 Powder

5 g of the compound of Example 2, 895 g of lactose and 100 g of corn starch were mixed in a blender to provide the desired powder.

Formulation Example 2 Granules

5 g of the compound of Example 5, 865 g of lactose and 100 g of low- substituted hydroxypropylcellulose were mixed, 300 g of a 10% aqueous hydroxypropyl cellulose solution were added to the resulting mixture, and this was then kneaded. The product thus obtained was then granulated using an extrusion granulating machine and dried to provide the desired granules.

Preparation Example 3 Capsules

5 g of the compound of Example 6, 115 g of lactose, 58 g of corn starch and 2 g of magnesium stearate were mixed using a V-shaped mixer, no. 3 capsules were chosen and then each of said no. 3 capsules was filled with 180 mg of the resulting mixture to provide the desired capsules.

Preparation Example 4 Tablets

5 g of the compound of Example 83, 90 g of lactose, 34 g of corn starch, 20 g of crystalline cellulose and 1 g of magnesium stearate were mixed in a blender, and the resulting mixture was then formed into tablets with a tabletting machine to provide the desired tablets.

[Test Examples]

Test Example 1

Concanavalin A-induced hepatopathy inhibitory activity test (Preventive effects)

Groups of 5 Balb/c mice (male, 6-8 weeks old) per group were used. Hepatopathy was induced in the mice by injection of concanavalin A (ConA) dissolved in physiologic saline (1.5 mg/ml) into the tail vein at a volume of 10 ml/kg (F. Gantner et al., Hepatology, 21, 190-198 (1995)).
The test compound was suspended at a concentration of 3 mg/ml in distilled water containing 0.5% tragacantha powder. This suspended solution was orally administered to each mouse at an administration volume of 10 ml/kg 30 min prior to injection of ConA (group A).
Besides this group, other groups were prepared, namely a group in which distilled water containing tragacantha powder (0.5%) was orally administered to each mouse, instead of the suspended solution of the test compound (group B), and a group in which physiologic saline not containing ConA, instead of physiologic saline containing ConA, was injected, and distilled water containing tragacantha powder (0.5%), instead of the suspended solution of the test compound, was orally administered to each mouse (group C).
Six hours after the injection of ConA (or physiologic saline), blood was collected from the abdominal portion of the vena cava of the mouse and the serum was isolated. Activities of glutamate oxaloacetate transaminase (AST) and glutamate pyruvate transaminase (ALT) were determined according to methods described by Nagakawa et al. (J. Nagakawa et al., J. Pharmacol. Exp. Ther., 264, 496-500 (1993)).
Inhibition rates of ConA-induced increases in AST and ALT activities caused by the test compound were calculated by insertion of the determined values into the following equation:

$$\text{Inhibition Rate (\%)}=\{1-[(A-C) / (B-C)]\} \times 100$$

A: average AST or ALT activity in group A
B: average AST or ALT activity in group B
C: average AST or ALT activity in group C

The results are summarized in Table 3.

Table 3

| Inhibition rate of the test compound on hepatopathy (Preventive effects) | | |
|---|---|---|
| Test Compound | Inhibition rate (%) | |
| | AST | ALT |
| Test Compound 83 | 82 | 96 |

As clearly shown in Table 3, compounds having general formula (I) described above inhibited ConA-induced increases in AST and ALT activities (i.e., hepatopathy).

Test Example 2

Concanavalin A-induced hepatopathy inhibitory activity test (Therapeutic effects)

As described in Test Example 1, hepatopathy was induced in Balb/c mice (male, 6-8 weeks old) by injection of ConA.
The test compound was administered to the mice 30 min after injection of ConA (group A). In addition, group B and group C were prepared as described in Test Example 1. Blood was collected from each mouse, serum was

isolated, and AST as well as ALT activities were determined. The present study was conducted according to Test Example 1, except for the timing of administration of the test compound (or distilled water containing tragacantha (0.5%)).

Inhibition rates of ConA-induced increases in AST and ALT activities due to the test compound were calculated according to the equation shown in the Test Example 1.

Results are summarized in Table 4.

Table 4

| Inhibition rate of the test compound on hepatopathy (Therapeutic effects) | | |
|---|---|---|
| Test Compound | Inhibition rate (%) | |
| | AST | ALT |
| Test Compound 83 | 91 | 99 |

As clearly shown in Table 4, compounds having general formula (I) described above inhibited ConA-induced increases in AST and ALT activities (i.e., hepatopathy).

Test Example 3

Galactosamine/lipopolysaccharide-induced hepatopathy inhibitory activity test (Preventive effects)

Groups of 10 C57BL/6 mice (female, 7 weeks old) per group were used. Hepatopathy was induced in each mouse by intraperitoneal injection of physiologic saline containing D-galactosamine (60 mg/ml, hereinafter expressed as "GalN") and lipopolysaccharide (1 µg/ml, hereinafter expressed as "LPS") at an administration volume of 10 ml/kg (M. Niehorster et al., Biochem. Pharmacol., 40, 1601-1603 (1990)).

The test compound was suspended in physiologic saline containing carboxymethylcellulose sodium powder (hereinafter expressed as "CMC") at a concentration of 0.5%, making a 1-mg/ml solution. This suspended solution was orally administered to each mouse at an administration volume of 10 ml/kg 30 min prior to the injection of GalN/LPS (group A).

Besides this group, other groups were prepared, namely a group in which physiologic saline containing CMC powder (0.5%) was orally administered to each mouse, instead of the suspended solution of the test compound (group B), and a group in which physiologic saline, instead of physiologic saline containing GalN/LPS, was injected, and distilled water containing CMC powder (0.5%), instead of the suspended solution of the test compound, was orally administered to each mouse (group C).

Six hours after the injection of GalN/LPS (or physiologic saline), blood was collected from the abdominal portion of the vena cava of the mouse and plasma was isolated after addition of heparin. Activities of glutamate oxaloacetate transaminase (AST) and glutamate pyruvate transaminase (ALT) in the plasma were determined as described in Test Example 1.

From the values determined, the inhibition rates of GalN plus LPS-induced increases in AST and ALT activities following administration of the test compounds were calculated according to the equation described in Test Example 1.

Compounds of the present invention exerted excellent inhibition activities against hepatopathy in the present study.

The results are summarized in Table 5.

Table 5

| Inhibition rate of the test compound on hepatopathy (Preventive effects) | | |
|---|---|---|
| Test Compound | Inhibition rate (%) | |
| | AST | ALT |
| Test Compound 83 | 95 | 96 |

As clearly demonstrated in Table 5, compounds having general formula (I) inhibited increases in AST and ALT activities caused by galactosamine and lipopolysaccharide (i.e., hepatopathy).

[Industrial applicability]

Compounds of the present invention having general formula (I) exerted inhibitory activities toward increases in AST and ALT activities, which are indicators of hepatopathy, in animal models of hepatopathy, when they were administered prior to induction of hepatopathy or even after the induction of hepatopathy. Thus compounds of the present invention may useful as preventive or therapeutic agents in hepatopathy.

## Claims

**1.** A composition containing as an active ingredient a compound of formula (I), or a pharmacologically acceptable salt, ester or other derivative thereof for the prophylaxis or treatment of hepatopathy:

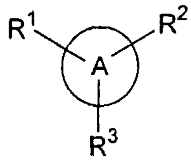

(I)

wherein:

A represents a pyrrole ring;

$R^1$ represents an aryl group; an aryl group which is substituted with group(s) selected from Substituent group α, Substituent group β, Substituent group γ and Substituent group δ; a heteroaryl group; or a heteroaryl group which is substituted with group(s) selected from Substituent group α, Substituent group β, Substituent group γ and Substituent group δ;

$R^2$ represents a heteroaryl group having at least one ring nitrogen atom; or a heteroaryl group having at least one ring nitrogen atom, wherein said heteroaryl group is substituted with group(s) selected from Substituent group α, Substituent group β, Substituent group γ and Substituent group δ;

$R^3$ represents a group of the formula -X-$R^4$,

wherein:

X represents a single bond; a lower alkylene group; a lower alkylene group which is substituted with group(s) selected from Substituent group α; a lower alkenylene group; a lower alkenylene group which is substituted with group(s) selected from Substituent group α; a lower alkynylene group; or a lower alkynylene group which is substituted with group(s) selected from Substituent group α; and
$R^4$ represents a cycloalkyl group which may be substituted with group(s) selected from Substituent group α and Substituent group δ and which is substituted with group(s) selected from Substituent group β and Substituent group γ; an aryl group which may be substituted with group(s) selected from Substituent group α and Substituent group δ and which is substituted with group(s) selected from Substituent group β and Substituent group γ; a heterocyclyl group which may be substituted with group(s) selected from Substituent group α and Substituent group δ and which is substituted with group(s) selected from Substituent group β and Substituent group γ; a heterocyclyl group having at least one nitrogen atom, said heterocyclyl group being optionally substituted with group(s) selected from Substituent group α, Substituent group δ and Substituent group ε; a heteroaryl group which may be substituted with group(s) selected from Substituent group α and Substituent group δ and which is substituted with group(s) selected from Substituent group β and Substituent group γ; a heteroaryl group having at least one nitrogen atom, said heteroaryl group being optionally substituted with group(s) selected from Substituent group α and Substituent group δ; or a group of formula -$NR^aR^b$ (wherein $R^a$ and $R^b$ are the same or different from each other and each represents a hydrogen atom, a lower alkyl group, a lower alkenyl group, a lower alkynyl group, an aralkyl group or a lower alkylsulfonyl group); PROVIDED THAT said substituents $R^1$ and $R^3$ are bonded to the atoms of said pyrrole ring which are adjacent to the atom of the pyrrole ring to which said substituent $R^2$ is bonded;

[Substituent group α]
a hydroxyl group, a nitro group, a cyano group, a halogen atom, a lower alkoxy group, a halogeno lower alkoxy group, an alkylthio group and a halogeno lower alkylthio group;
[Substituent group β]
a group of formula -$NR^cR^d$ (wherein $R^c$ and $R^d$ are the same or different from each other and each represents a hydrogen atom, a lower alkyl group, a lower alkenyl group, a lower alkynyl group, an aralkyl group or a lower alkylsulfonyl group, or $R^c$ and $R^d$, together with the nitrogen atom to which $R^c$ and $R^d$ are bonded, form a heterocyclyl group);
[Substituent group γ]
a lower alkyl group which is substituted with a group of formula -$NR^cR^d$ (wherein $R^c$ and $R^d$ are as defined above) ;
[Substituent group δ]
a lower alkyl group, a lower alkenyl group, a lower alkynyl group, an aralkyl group, a cycloalkyl group, a lower alkyl group which is substituted with group(s) selected from Substituent group α, a lower alkenyl group which is substituted with group(s) selected from Substituent group α, and an alkynyl group which is substituted with group(s) selected from Substituent group α; and
[Substituent group ε]
an oxo group, a hydroxyimino group, a lower alkoxyimino group, a lower alkylene group, a lower alkylenedioxy group, a lower alkylsulfinyl group, a lower alkylsulfonyl group, an aryl group and an aryl group which is substituted with group(s) selected from Substituent group α, Substituent group β, Substituent group γ and Substituent group δ.

**2.** A composition according to claim 1 comprising a compound of formula (I) or a pharmacologically acceptable salt, ester or other derivative thereof, wherein $R^1$ is an aryl group; or an aryl group which is substituted with group(s) selected from Substituent group a, Substituent group β, Substituent group γ and Substituent group δ.

**3.** A composition according to claim 1 comprising a compound of formula (I) or a pharmacologically acceptable salt, ester or other derivative thereof, 1 wherein $R^1$ is a phenyl or naphthyl group; or a phenyl or naphthyl group which is substituted with group(s) selected from Substituent group α, Substituent group β, Substituent group γ and Substituent group δ.

**4.** A composition according to claim 1 comprising a compound of formula (I) or a pharmacologically acceptable salt, ester or other derivative thereof, wherein $R^1$ is a phenyl group; or a phenyl group which is substituted with group (s) selected from Substituent group $\alpha^1$, Substituent group $\beta^1$, Substituent group $\gamma^1$ and Substituent group $\delta^1$,
[Substituent group $\alpha^1$]
a halogen atom, a lower alkoxy group and a halogeno lower alkoxy group;
[Substituent group $\beta^1$]
a group of formula -$NR^cR^d$ (wherein one of $R^c$ and $R^d$ is a hydrogen atom or a lower alkyl group and the other is a hydrogen atom, a lower alkyl group or an aralkyl group);
[Substituent group $\gamma^1$]
an amino lower alkyl group, a lower alkyl-amino lower alkyl group, di(lower alkyl)-amino lower alkyl group and an aralkyl-amino lower alkyl group; and
[Substituent group $\delta^1$]
a lower alkyl group, a halogeno lower alkyl group, a hydroxy lower alkyl group and a nitro lower alkyl group.

**5.** A composition according to claim 1 comprising a compound of formula (I) or a pharmacologically acceptable salt, ester or other derivative thereof, wherein $R^1$ is a phenyl group; or a phenyl group which is substituted with group (s) selected from the substituent groups below, (the substituent groups: a halogen atom, a halogeno lower alkyl group and a halogeno lower alkoxy group).

**6.** A composition according to claim 1 comprising a compound of formula (I) or a pharmacologically acceptable salt, ester or other derivative thereof, wherein $R^1$ is a phenyl, 4-fluorophenyl, 3-fluorophenyl, 3-chlorophenyl, 3,4-difluorophenyl, 3,4,5-trifluorophenyl, 3-chloro-4-fluorophenyl, 3-difluoromethoxyphenyl or 3-trifluoromethylphenyl group.

**7.** A composition according to any one of claims 1 to 6 comprising a compound of formula (I) or a pharmacologically acceptable salt, ester or other derivative thereof, wherein $R^2$ is a 5- or 6-membered aromatic heterocyclic group containing one or two nitrogen atoms; or a 5- or 6-membered aromatic heterocyclic group containing one or two nitrogen atoms, said group being substituted with group(s) selected from Substituent group α, Substituent group β, Substituent group γ and Substituent group δ.

**8.** A composition according to any one of claims 1 to 6 comprising a compound of formula (I) or a pharmacologically acceptable salt, ester or other derivative thereof, wherein $R^2$ is a pyridyl or pyrimidinyl group; or a pyridyl or pyrimidinyl group which is substituted with group(s) selected from Substituent group α, Substituent group β, Substituent group γ and Substituent group δ.

**9.** A composition according to any one of claims 1 to 6 comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted with group(s) selected from Substituent group α, Substituent group β, Substituent group γ and Substituent group δ.

**10.** A composition according to any one of claims 1 to 6 comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted at the 2-position thereof with a group selected from Substituent group α, Substituent group β, Substituent group γ and Substituent group δ.

**11.** A composition according to any one of claims 1 to 6 comprising a compound of formula (I) or a pharmacologically acceptable salt, ester or other derivative thereof, wherein $R^2$ is a 4-pyridyl or 4-pyrimidinyl group; or a 4-pyridyl or 4-pyrimidinyl group which is substituted at the 2-position thereof with a group selected from the substituent groups below,
(the substituent groups: methoxy, amino, methylamino, benzylamino and α-methylbenzylamino).

**12.** A composition according to any one of claims 1 to 11 comprising a compound of formula (I) or a pharmacologically acceptable salt, ester or other derivative thereof, wherein $R^4$ is a cycloalkyl group having from 3 to 7 carbon atoms, which may be substituted with group(s) selected from Substituent group α and Substituent group δ and which is substituted with group(s) selected from Substituent group β and Substituent group γ; a phenyl or naphthyl group which may be substituted with group(s) selected from Substituent group α and Substituent group δ and which is substituted with group(s) selected from Substituent group β and Substituent group γ; a 4-to 12-membered non-aromatic heterocyclic group containing one or two heteroatom(s) selected from the group consisting of sulfur atoms, oxygen atoms and nitrogen atoms, which may be substituted with group(s) selected from Substituent group α and Substituent group δ and which is substituted with group(s) selected from Substituent group β and Substituent group γ; a 4-to 12-membered non-aromatic heterocyclic group containing one nitrogen atom and optionally one further heteroatom selected from the group consisting of oxygen atom, sulfur atom and nitrogen atom, which may be substituted with group(s) selected from Substituent group α, Substituent group δ and Substituent group ε; a 5- or 6-membered aromatic heterocyclic group containing one or two heteroatom(s) selected from the group consisting of sulfur atoms, oxygen atoms and nitrogen atoms, which may be substituted with group(s) selected from Substituent group α and Substituent group δ and which is substituted with group(s) selected from Substituent group β and Substituent group γ; a 5- or 6-membered aromatic heterocyclic group containing one or two nitrogen atoms, said group being optionally substituted with group(s) selected from Substituent group α and Substituent group δ; and a group of formula $-NR^aR^b$ wherein $R^a$ and $R^b$ are as defined above.

**13.** A composition according to any one of claims 1 to 12 comprising a compound of formula (I) or a pharmacologically acceptable salt, ester or other derivative thereof, wherein X is a single bond; an alkylene group having from 1 to 6 carbon atoms; an alkylene group having from 1 to 6 carbon atoms which is substituted with group(s) selected from Substituent group α; an alkenylene group having from 2 to 6 carbon atoms; an alkenylene group having from 2 to 6 carbon atoms which is substituted with group(s) selected from Substituent group α; an alkynylene group having from 2 to 6 carbon atoms; or an alkynylene group having from 2 to 6 carbon atoms which is substituted with group(s) selected from Substituent group α.

**14.** A composition according to any one of claims 1 to 12 comprising a compound of formula (I) or a pharmacologically acceptable salt, ester or other derivative thereof, wherein X is a single bond; an alkylene group having from 1 to 4 carbon atoms; an alkylene group having from 1 to 4 carbon atoms which is substituted with group(s) selected from Substituent group α; an alkenylene group having from 2 to 4 carbon atoms; an alkenylene group having from

2 to 4 carbon atoms which is substituted with group(s) selected from Substituent group α; an alkynylene group having from 2 to 4 carbon atoms; or an alkynylene group having from 2 to 4 carbon atoms which is substituted with group(s) selected from Substituent group α.

**15.** A composition according to any one of claims 1 to 12 comprising a compound of formula (I) or a pharmacologically acceptable salt, ester or other derivative thereof, wherein X is a single bond; an alkenylene group having from 2 to 4 carbon atoms; or an alkenylene group having from 2 to 4 carbon atoms which is substituted with group(s) selected from Substituent group α.

**16.** A composition according to any one of claims 1 to 15 comprising a compound of formula (I) or a pharmacologically acceptable salt, ester or other derivative thereof, wherein $R^3$ is a group of the following formula (II):

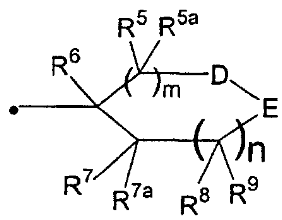
(II)

wherein:

m represents 0 or 1;
n represents 1 or 2;
$R^5$, $R^{5a}$, $R^6$, $R^7$, $R^{7a}$, $R^8$ and $R^9$ are the same or different from each other and each represents a group selected from a hydrogen atom, Substituent group α, Substituent group β, Substituent group γ, Substituent group δ and Substituent group ε;
one of D and E represents a group of formula >$NR^{10}$ (wherein $R^{10}$ is selected from a hydrogen atom, Substituent group γ and Substituent group δ), and the other represents a group of formula >$CR^{11}R^{12}$ (wherein $R^{11}$ and $R^{12}$ are the same or different from each other and each is selected from a hydrogen atom, Substituent group α, Substituent group β, Substituent group γ and Substituent group δ), or
$R^6$ may form, together with $R^5$ or $R^7$, a single bond, and/or $R^{10}$ and $R^{11}$ together may form a lower alkylene group; or a lower alkylene group which is substituted with group(s) selected from Substituent group α, Substituent group β, Substituent group γ, Substituent group δ and Substituent group ε;

provided that
when one of $R^5$ and $R^{5a}$ represents an oxo group, a hydroxyimino group, a lower alkoxyimino group, a lower alkylene group or a lower alkylenedioxy group, the other is absent;
when one of $R^7$ and $R^{7a}$ represents an oxo group, a hydroxyimino group, a lower alkoxyimino group, a lower alkylene group or a lower alkylenedioxy group, the other is absent;
when one of $R^8$ and $R^9$ represents an oxo group, a hydroxyimino group, a lower alkoxyimino group, a lower alkylene group or a lower alkylenedioxy group, the other is absent;

**17.** A composition according to claim 16 comprising a compound of formula (II) or a pharmacologically acceptable salt, ester or other derivative thereof, wherein m is 1 and n is 1.

**18.** A composition according to claim 16 or claim 17 comprising a compound of formula (II) or claim 17 or a pharmacologically acceptable salt, ester or other derivative thereof, wherein E represents a group of formula >$NR^{10}$ and D represents a group of formula >$CR^{11}R^{12}$.

**19.** A composition according to any one of claims 16 to 18 comprising a compound of formula (II) or a pharmacologically acceptable salt, ester or other derivative thereof, wherein one of $R^5$ and $R^7$ together with $R^6$ represents a single bond and the other represents a group selected from a hydrogen atom, Substituent group α, Substituent group γ, Substituent group δ and Substituent group ε,
$R^{5a}$, $R^{7a}$, $R^8$ and $R^9$ are the same or different from each other and each represents a group selected from a

hydrogen atom, Substituent group α, Substituent group γ, Substituent group δ and Substituent group ε, $R^{12}$ may be the same or different from each other and each represents a group selected from a hydrogen atom, Substituent group α, Substituent group γ and Substituent group δ, and $R^{10}$ and $R^{11}$ are the same or different from each other and each represents a group selected from a hydrogen atom, Substituent group α, Substituent group γ and Substituent group δ, or $R^{10}$ and $R^{11}$ together form a lower alkylene group or a lower alkylene group which is substituted with group(s) selected from Substituent group α, Substituent group β, Substituent group γ, Substituent group δ and Substituent group ε.

**20.** A composition according to any one of claims 16 to 19 comprising a compound of formula (II) or a pharmacologically acceptable salt, ester or other derivative thereof, wherein $R^{10}$ and $R^{11}$ together form a straight chain alkylene group having 3 or 4 carbon atoms or a straight chain alkylene group having 3 or 4 carbon atoms which is substituted with group(s) selected from Substituent group α, Substituent group β, Substituent group γ, Substituent group δ and Substituent group ε.

**21.** A composition according to any one of claims 16 to 20 comprising a compound of formula (II) or a pharmacologically acceptable salt, ester or other derivative thereof, wherein each of $R^{5a}$, $R^7$, $R^{7a}$, $R^8$ and $R^9$ is a hydrogen atom, and $R^{10}$ and $R^{11}$ together form a straight chain alkylene group having 3 or 4 carbon atoms or a straight chain alkylene group having 3 or 4 carbon atoms which is substituted with group(s) selected from Substituent group α, Substituent group β, Substituent group γ, Substituent group δ and Substituent group ε.

**22.** A composition according to any one of claims 1 to 21 comprising a compound of formula (I) or a pharmacologically acceptable salt, ester or other derivative thereof, wherein said compound of formula (I) is a compound of formula (I-1) or (I-3) below:

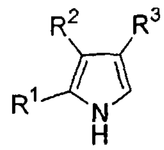

**(I-1)**

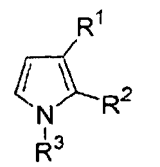

**(I-3)**

**23.** A composition according to claim 1 comprising a compound of formula (I) selected from the following group of compounds, or a pharmacologically acceptable salt, ester or other derivative thereof:

4-(3-aminopropyly-2-(4-fluorophenyl)-3-(pyridin-4-yl)-1*H*-pyrrole,
4-(3-acetylaminopropyl)-2-(4-fluorophenyl)-3-(pyridin-4-yl)-1*H*-pyrrole,
2-(4-fluorophenyl)-4-(3-methylaminopropyl) -3-(pyridin-4-yl)-1*H*-pyrrole,
[5-(4-fluorophenyl)-4-(pyridin-4-yl)-1*H*-pyrrol-3-yl]-(pyridin-4-yl)methanol,
4-(1-acetyl-1,2,3,6-tetrahydropyridin-4-yl)-2-(4-fluorophenyl)-3-(pyridin-4-yl)-1*H*-pyrrole,
2-(4-fluorophenyl)-3-(pyridin-4-yl)-4-(1,2,3,6-tetrahydropyridin-4-yl)-1*H*-pyrrole,
2-(4-fluorophenyl)-4-(piperidin-4-yl)-3-(pyridin-4-yl)-1*H*-pyrrole,
3-(4-fluorophenyl)-1-(piperidin-4-yl)-2-(pyridin-4-yl)-1*H*-pyrrole,
1-(1-acetylpiperidin-4-yl)-3-(4-fluorophenyl)-2-(pyridin-4-yl)-1*H*-pyrrole,
3-(4-fluorophenyl)-1-[1-(2-nitroethyl)piperidin-4-yl]-2-(pyridin-4-yl)-1*H*-pyrrole,
3-(4-fluorophenyl)-1-[3-(morpholin-1-yl)propyl]-2-(pyridin-4-yl)-1*H*-pyrrole,
3-(4-fluorophenyl)-1-(piperidin-3-yl)-2-(pyridin-4-yl)-1*H*-pyrrole,
1-(azetidin-3-yl)-3-(4-fluorophenyl)-2-(pyridin-4-yl)-1*H*-pyrrole,
4-(3-dimethylaminopropyl)-2-(4-fluorophenyl)-3-(pyridin-4-yl)-1*H*-pyrrole,
2-(4-fluorophenyl)-4-[3-(piperidin-1-yl)propyl]-3-(pyridin-4-yl)-1*H*-pyrrole,
2-(4-fluorophenyl)-4-[3-(1-methylpiperazin-4-yl)propyl]-3-(pyridin-4-yl)-1*H*-pyrrole,
2-(4-fluorophenyl)-4-(1-methyl-1,2,3,6-tetrahydropyridin-4-yl)-3-(pyridin-4-yl)-1*H*-pyrrole,
2-(4-fluorophenyl)-4-(1-methylpiperidin-4-yl)-3-(pyridin-4-yl)-1*H*-pyrrole,
3-(4-fluorophenyl)-2-(pyridin-4-yl)-1-(2,2,6,6-tetramethylpiperidin-4-yl)-1*H*-pyrrole,
2-(4-fluorophenyl)-3-(pyridin-4-yl)-4-(quinuclidin-2-en-3-yl)-1*H*-pyrrole,

2-(4-fluorophenyl)-3-(pyridin-4-yl)-4-(quinuclidin-3-yl)-1*H*-pyrrole,
2-(4-fluorophenyl)-4-(4-hydroxypiperidin-4-yl)-3-(pyridin-4-yl)-1*H*-pyrrole,
3-(4-fluorophenyl)-2-(pyridin-4-yl)-1-(quinuclidin-3-yl)-1*H*-pyrrole,
1-(4-aminocyclohexyl)-3-(4-fluorophenyl)-2-(pyridin-4-yl)-1*H*-pyrrole,
3-(4-fluorophenyl)-2-(2-methylaminopyrimidin-4-yl)-1-(piperidin-4-yl)-1*H*-pyrrole,
2-(4-fluorophenyl)-4-(8-methyl-8-azabicyclo[3.2.1]oct-2-en-3-yl)-3-(pyridin-4-yl)-1*H*-pyrrole,
2-(4-fluorophenyl)-4-(8-methyl-8-azabicyclo[3.2.1]octan-3-yl)-3-(pyridin-4-yl)-1*H*-pyrrole,
4-(8-azabicyclo[3,2,1]oct-2-en-3-yl)-2-(4-fluorophenyl)-3-(pyridin-4-yl)-1*H*-pyrrole,
2-(4-fluorophenyl)-3-(pyridin-4-yl)-4-(1,2,3,6-tetrahydro-2,2,6,6-tetramethylpyridin-4-yl)-1*H*-pyrrole,
2-(4-fluorophenyl)-3-(pyridin-4-yl)-4-(2,2,6,6-tetramethylpiperidin-4 -yl)-1*H*-pyrrole,
2-(4-fluorophenyl)-4-(2-methyl-1,2,3,6-tetrahydropyridin-4-yl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,
4-(1-ethylpiperidin-4-yl)-2-(4-fluorophenyl)-3-(pyridin-4-yl)-1*H*-pyrrole,
2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4-(1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,8,8a-hexahydroindolizin-7-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
2-(4-fluorophenyl)-4-(1,2,2,6,6-pentamethyl-1,2,3,6-tetrahydropyridin-4-yl)-3-(pyridin-4-yl)-1*H*-pyrrole,
4-(3-dimethylamino-1-propen-1-yl)-2-(4-fluorophenyl)-3-(pyridin-4-yl)-1*H*-pyrrole,
4-(4-aminobutyl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
1-(3-dimethylaminopropyl)-3-(4-fluorophenyl)-2-(pyridin-4-yl)-1*H*-pyrrole,
2-(4-fluorophenyl)-4-(1,2,3,5,6,7,8,8a-octahydroindolizin-7-yl)-3-(pyridin-4-yl)-1*H*-pyrrole,
4-(6-allyl-1-methyl-1,2,3,6-tetrahydropyridin-4-yl)-2-(4-fluorophenyl)-3-(pyridin-4-yl)-1*H*-pyrrole,
4-(2-allyl-1-methyl-1,2,3,6-tetrahydropyridin-4-yl)-2-(4-fluorophenyl)-3-(pyridin-4-yl)-1*H*-pyrrole,
4-(6-benzyl-1-methyl-1,2,3,6-tetrahydropyridin-4-yl)-2-(4-fluorophenyl)-3-(pyridin-4-yl)-1*H*-pyrrole,
4-(2-benzyl-1-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(4-fluorophenyl)-4-(1,3,4,6,9,9a-hexahydro-2*H*-quinolizin-8-yl)-3-(pyridin-4-yl)-1*H*-pyrrole,
2-(4-fluorophenyl)-4-(1,2,3,5,8,8a-hexahydroindolizin-7-yl)-3-(2-methylaminopyrimidin-4-yl)-1*H*-pyrrole,
2-(4-fluorophenyl)-4-(1,2,3,5,6,8a-hexahydroindolizin-7-yl)-3-(2-methylaminopyrimidin-4-yl)-1*H*-pyrrole,
2-(3,4-difluorophenyl)-4-(1,2,3,5,8,8a-hexahydroindolizin-7-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-3-(pyridin-4-yl)-2-(3-trifluoromethylphenyl)-1*H*-pyrrole,
4-(1,2,3,5,6,8a-hexahydroindolizin-7-yl)-3-(pyridin-4-yl)-2-(3-trifluoromethylphenyl)-1*H*-pyrrole,
2-(3-fluorophenyl)-4-(1,2,3,5,8,8a-hexahydroindolizin-7-yl)-3-(pyridin-4-yl)-1*H*-pyrrole,
2-(3-fluorophenyl)-4-(1,2,3,5,6,8a-hexahydroindolizin-7-yl)-3-(pyridin-4-yl)-1*H*-pyrrole,
2-(3-chlorophenyl)-4-(1,2,3,5,8,8a-hexahydroindolizin-7-yl)-3-(pyridin-4-yl)-1*H*-pyrrole,
2-(3-chlorophenyl)-4-(1,2,3,5,6,8a-hexahydroindolizin-7-yl)-3-(pyridin-4-yl)-1*H*-pyrrole,
4-(1,2,3,5,8,8a-hexahydroindolizin-7-yl)-2-phenyl-3-(pyridin-4-yl)-1*H*-pyrrole, and
4-(1,2,3,5,6,8a-hexahydroindolizin-7-yl)-2-phenyl-3-(pyridin-4-yl)-1*H*-pyrrole.

**24.** A composition according to claim 1 comprising a compound of formula (I) selected from the following group of compounds, or a pharmacologically acceptable salt, ester or other derivative thereof:

2-(4-fluorophenyl)-3-(pyridin-4-yl)-4-(1,2,3,6-tetrahydropyridin-4-yl)-1*H*-pyrrole,
3-(4-fluorophenyl)-1-(piperidin-4-yl)-2-(pyridin-4-yl)-1*H*-pyrrole,
3-(4-fluorophenyl)-1-[3-(morpholin-1-yl)propyl]-2-(pyridin-4-yl)-1*H*-pyrrole,
4-(8-azabicyclo[3.2.1]oct-2-en-3-yl)-2-(4-fluorophenyl)-3-(pyridin-4-yl)-1*H*-pyrrole,
2-(4-fluorophenyl)-4-(6-methyl-1,2,3,6-tetrahydropyridin-4-yl)-3-(pyridin-4-yl)-1*H*-pyrrole,
4-(1-ethyl-1,2,3,6-tetrahydropyridin-4-yl)-2-(4-fluorophenyl)-3-(pyridin-4-yl)-1*H*-pyrrole,

2-(4-fluorophenyl)-4-(1-isopropyl-1,2,3,6-tetrahydropyridin-4-yl)-3-(pyridin-4-yl)-1*H*-pyrrole,
2-(4-fluorophenyl)-4- (1-propyl-1,2,3,6-tetrahydropyridin-4-yl)-3-(pyridin-4-yl)-1*H*-pyrrole,
2-(4-fluorophenyl)-4-(1-phenethyl-1,2,3,6-tetrahydropyridin-4-yl)-3-(pyridin-4-yl)-1*H*-pyrrole,
2-(4-fluorophenyl)-4-(1,2,3,5,6,8a-hexahydroindolizin-7-yl)-3-(pyridin-4-yl)-1*H*-pyrrole,
4-(1,6-dimethyl-1,2,3,6-tetrahydropyridin-4-yl)-2-(4-fluorophenyl)-3-(pyridin-4-yl)-1*H*-pyrrole,
4-(1,2-dimethyl-1,2,3,6-tetrahydropyridin-4-yl)-2-(4-fluorophenyl)-3-(pyridin-4-yl)-1*H*-pyrrole,
4-(1-ethyl-6-methyl-1,2,3,6-tetrahydropyridin-4-yl)-2-(4-fluorophenyl)-3-(pyridin-4-yl)-1*H*-pyrrole,
4-(1-ethyl-2-methyl-1,2,3,6-tetrahydropyridin-4-yl)-2-(4-fluorophenyl)-3-(pyridin-4-yl)-1*H*-pyrrole,
2-(4-fluorophenyl)-4-(6-methyl-1-propyl-1,2,3,6-tetrahydropyridin-4-yl)-3-(pyridin-4-yl)-1*H*-pyrrole,
2-(4-fluorophenyl)-4-(2-methyl-1-propyl-1,2,3,6-tetrahydropyridin-4-yl)-3-(pyridin-4-yl)-1*H*-pyrrole,
2-(4-fluorophenyl)-4-(1,3,4,6,7,9a-hexahydro-2*H*-quinolizin-8-yl)-3-(pyridin-4-yl)-1*H*-pyrrole,
2-(3,4-difluorophenyl)-4-(1,2,3,5,6,8a-hexahydroindolizin-7-yl)-3-(pyridin-4-yl)-1*H*-pyrrole, and
4-(1,2,3,5,6,8a-hexahydroindolizin-7-yl)-3-(pyridin-4-yl)-2-(3-trifluoromethylphenyl)-1*H*-pyrrole.

**25.** A method for the prophylaxis or treatment of hepatopathy which comprises administering to a warm blooded animal suffering therefrom an effective amount of a compound of formula (I) according to any one of claims 1 to 24 or a pharmacologically acceptable salt, ester or other derivative thereof.

**26.** The use of a compound of formula (I) according to any one of claims 1 to 24 or a pharmacologically acceptable salt, ester or other derivative thereof in the manufacture of a medicament for the prophylaxis or treatment of hepatopathy.

## INTERNATIONAL SEARCH REPORT

International application No.
PCT/JP02/00290

A. CLASSIFICATION OF SUBJECT MATTER
Int.Cl[7] C07D401/04, 401/14, 451/02, 453/02,471/04, A61K31/437, 4375, 439, 4439, 444, 4545, 496, 506, 5377, 541, A61P1/16
According to International Patent Classification (IPC) or to both national classification and IPC

B. FIELDS SEARCHED
Minimum documentation searched (classification system followed by classification symbols)
Int.Cl[7] C07D401/04, 401/14, 451/02, 453/02, 471/04, A61K31/437, 4375, 439, 4439, 444, 4545, 496, 506, 5377, 541, A61P1/16

Documentation searched other than minimum documentation to the extent that such documents are included in the fields searched

Electronic data base consulted during the international search (name of data base and, where practicable, search terms used)
CAPLUS, REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | EP, 1070711, A2 (Sankyo Co., Ltd.), 24 January, 2001 (24.01.01), & NO 2000003734 A & BR 2000004534 A & CN 1295069 A & JP 2001-247564 A | 1-24,26 |
| X | WO, 97/5877, A1 (Merck & Co., Inc.), 20 February, 1997 (20.02.97), & CA 2228136 A & AU 9667689 A & EP 863757 A1 & JP 11-510510 A | 1-24,26 |
| P,X | JP, 2001-181187, A (Sankyo Co., Ltd.), 03 July, 2001 (03.07.01), (Family: none) | 1-24,26 |
| X | WO, 00/66124, A1 (Sankyo Co., Ltd.), 09 November, 2000 (09.11.00), & JP 2001-10957 A | 1-24,26 |
| X | WO, 00/01688, A1 (Sankyo Co., Ltd.), 13 January, 2000 (13.01.00), & AU 9943953 A & JP 2000-80094 A | 1-24,26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 March, 2002 (22.03.02) | Date of mailing of the international search report<br>02 April, 2002 (02.04.02) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/00290

C (Continuation). DOCUMENTS CONSIDERED TO BE RELEVANT

| Category* | Citation of document, with indication, where appropriate, of the relevant passages | Relevant to claim No. |
|---|---|---|
| X | WO, 97/16442, A1 (Merck & Co., Inc.), 09 May, 1997 (09.05.97), & AU 9711208 A & EP 859771 A1 & JP 11-514651 A | 1-24,26 |
| X | WO, 97/05878, A1 (Merck & Co., Inc.), 20 February, 1997 (20.02.97), & CA 2228050 A & AU 9667691 A & EP 871444 A1 & JP 11-510511 A | 1-24,26 |

Form PCT/ISA/210 (continuation of second sheet) (July 1998)

## INTERNATIONAL SEARCH REPORT

International application No.
PCT/JP02/00290

**Box I Observations where certain claims were found unsearchable (Continuation of item 1 of first sheet)**

This international search report has not been established in respect of certain claims under Article 17(2)(a) for the following reasons:

1. [X] Claims Nos.: 25
   because they relate to subject matter not required to be searched by this Authority, namely:
   Claim 25 pertains to a method for treatment of the human body by therapy.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)